(19) Europäisches Patentamt / European Patent Office / Office européen des brevets

(11) **EP 4 365 178 A1**

(12) **EUROPEAN PATENT APPLICATION**
published in accordance with Art. 153(4) EPC


(43) Date of publication:
**08.05.2024 Bulletin 2024/19**

(21) Application number: **22832136.0**

(22) Date of filing: **29.06.2022**

(51) International Patent Classification (IPC):
***C07D 519/00*** (2006.01) ***C07F 7/18*** (2006.01)
***C07D 471/04*** (2006.01) ***C07C 53/18*** (2006.01)
***C07C 51/41*** (2006.01) ***A61K 31/519*** (2006.01)
***A61P 35/00*** (2006.01) ***A61P 35/02*** (2006.01)

(52) Cooperative Patent Classification (CPC):
**A61K 31/519; A61P 35/00; A61P 35/02; C07C 51/41; C07C 53/18; C07D 471/04; C07D 519/00; C07F 7/18**

(86) International application number:
**PCT/CN2022/102497**

(87) International publication number:
**WO 2023/274324 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2021 CN 202110736463**
**20.08.2021 CN 202110959764**
**11.01.2022 CN 202210029060**



(71) Applicant: **Shanghai Allist Pharmaceuticals Co., Ltd.**
**Shanghai 201318 (CN)**

(72) Inventors:
- **LUO, Huibing**
  **Shanghai 201318 (CN)**
- **JIANG, Jiajun**
  **Shanghai 201318 (CN)**
- **WANG, Jiapo**
  **Shanghai 201318 (CN)**
- **ZHOU, Huayong**
  **Shanghai 201318 (CN)**

(74) Representative: **HGF**
**HGF Europe LLP**
**Neumarkter Straße 18**
**81673 München (DE)**


(54) **NITROGEN-CONTAINING HETEROCYCLIC COMPOUND, AND PREPARATION METHOD THEREFOR, INTERMEDIATE THEREOF, AND APPLICATION THEREOF**


(57) Disclosed in the present invention are a nitrogen-containing heterocyclic compound, and a preparation method therefor, an intermediate thereof, and an application thereof. The present invention provides a nitrogen-containing heterocyclic compound as represented by formula (I), a stereoisomer thereof, or a pharmaceutically acceptable salt thereof. The nitrogen-containing heterocyclic compound as represented by formula (I), the stereoisomer thereof, or the pharmaceutically acceptable salt thereof provided by the present invention has the activity of inhibiting proliferation of Ba/F3 KRAS-G12D cells, AGS cells, Ba/F3 KRAS-G12V cells expressing KRAS G12D and/or KRAS G12V mutant proteins, and also shows good in vivo tumor suppression activity; the present invention is expected to treat and/or prevent a variety of diseases mediated by KRAS G12D and/or KRAS G12V.


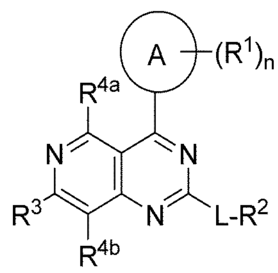

I




Processed by Luminess, 75001 PARIS (FR)

## Description

The present application claims the right of the priorities of Chinese patent application 202110736463.4 filed on June 30, 2021; Chinese patent application 202110959764.3 filed on August 20, 2021; and Chinese patent application 202210029060.0 filed on January 11, 2022. The contents of the above Chinese patent applications are incorporated herein by reference in their entireties.

TECHNICAL FIELD

The present disclosure relates to a nitrogen-containing heterocyclic compound, a preparation method therefor, an intermediate thereof, and a use thereof.

BACKGROUND

RAS protein is a guanosine triphosphate (GTP) binding protein located on the cell membrane, with a molecular weight of 21 kDa and composed of 188 or 189 amino acids. The active state of RAS protein has an impact on cell growth, differentiation, cytoskeleton, protein transport, protein secretion, etc., and the activity of RAS protein is regulated by binding with GTP or guanosine diphosphate (GDP). When RAS protein binds with GDP, it is in an "inactive" state; when stimulated by specific upstream cell growth factors, the guanine nucleotide exchange factor (GEF) catalyzes the RAS protein to release GDP and bind with GTP, putting it in an "activated" state. RAS protein bound with GTP can activate downstream proteins and activate downstream signaling pathways. RAS protein itself has weak GTPase activity, which can hydrolyze GTP to GDP, thus achieving the transformation from the activated state to the inactive state. In this hydrolysis process, GTPase-activating protein (GAP) is also required, which can interact with RAS protein and greatly promote its ability to hydrolyze GTP to GDP. Any mutation in the RAS protein that affects its own GTPase activity, its interaction with GAP, or its ability to hydrolyze GTP to GDP, will cause the RAS protein to be in a prolonged activated state. The prolonged activation of RAS protein continues to give growth signals to downstream proteins, leading to continuous cell growth and differentiation, which may eventually lead to cancer. There are three members of RAS gene family: KRAS, NRAS, and HRAS.

KRAS mutation is the most common oncogenic driver, present in various tumors: lung adenocarcinoma (32%), colorectal cancer (41%), and pancreatic cancer (86%). G12 mutation at codon 12 is the most common mutation in KRAS. For example, in KRAS-mutated lung adenocarcinoma, colorectal cancer, and pancreatic cancer, G12 mutations account for 85%, 68%, and 91% respectively; G12 mutations include G12C, G12D, G12V, G12R, and other mutation forms. Among patients with KRAS G12-mutated lung adenocarcinoma, colorectal cancer, and pancreatic cancer, the proportion of patients with KRAS G12D mutation is 17%, 45%, and 45% respectively, and the proportion of patients with KRAS G12V mutation is 23%, 30%, and 35% respectively (for example, see Moore, A.R. et al. Nat Rev Drug Discov 19, 533 (2020)).

MRTX1133, a KRAS G12D inhibitor developed by Mitati Therapeutics, Inc., has been disclosed in WO2021041671 and has the following structure, with currently disclosed preclinical data.

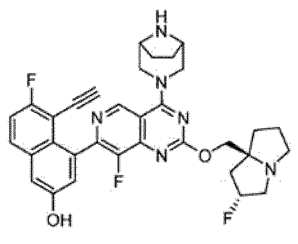

MRTX1133

Although some progress has been made in this field, there are currently no approved treatments for KRAS G12D and/or KRAS G12V mutations, so there is still a need to continue to develop effective, stable, and safe small molecule KRAS G12D and/or KRAS G12V inhibitors for the treatment of diseases mediated by KRAS G12D and/or KRAS G12V mutations, such as cancer.

CONTENT OF THE PRESENT INVENTION

The technical problem to be solved by the present disclosure is the single structure of KRAS G12D and KRAS G12V inhibitors in the prior art, and a nitrogen-containing heterocyclic compound, a preparation method therefor, an

intermediate thereof, and a use thereof are provided. The nitrogen-containing heterocyclic compound has the activity of inhibiting the proliferation of Ba/F3 KRAS-G12D cells, AGS cells, and Ba/F3 KRAS-G12V cells expressing KRAS G12D and/or KRAS G12V mutant proteins, and also shows good *in vivo* tumor inhibitory activity; and is expected to treat and/or prevent multiple diseases mediated by KRAS G12D and/or KRAS G12V.

The present disclosure solves the above technical problem by the following technical solutions.

The present disclosure provides a nitrogen-containing heterocyclic compound of formula I, a stereoisomer thereof, or a pharmaceutically acceptable salt thereof

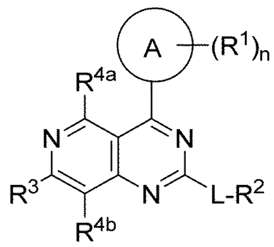

I

wherein ring A is 3- to 7-membered cycloalkyl, 4- to 10-membered heterocycloalkyl, or 4- to 10-membered heterocycloalkenyl; the number of heteroatoms of the 4- to 10-membered heterocycloalkyl and the 4- to 10-membered heterocycloalkenyl is 1 or 2, wherein the heteroatom is selected from one or two of N, O, and S;

n is 0, 1, 2, 3, 4, 5, 6, 7, 8, 9, or 10;

each $R^1$ is independently deuterium, halogen, -CN, -OH, $-N(R^5)_2$, $C_1$-$C_6$ alkyl, $C_1$-$C_6$ alkyl substituted by one or more than one $R^{1a}$, $C_1$-$C_6$ alkyl-O-, $C_1$-$C_6$ alkyl-O- substituted by one or more than one $R^{1b}$, $C_2$-$C_6$ alkenyl, $C_2$-$C_6$ alkynyl, $C_2$-$C_6$ alkenyl substituted by one or more than one $R^{1c}$, $C_2$-$C_6$ alkynyl substituted by one or more than one $R^{1d}$, -C(=O)H, $-CO_2R^5$, $-C(=O)N(R^5)_2$, 5- to 6-membered heteroaryl, or two $R^1$ on the same ring atom form an oxo group; the heteroatom of the 5- to 6-membered heteroaryl is selected from one or more than one of N, O, and S, and the number of heteroatoms is 1, 2, or 3; when there is more than one substituent, the substituents are the same or different;

$R^{1a}$, $R^{1b}$, $R^{1c}$, and $R^{1d}$ are each independently deuterium, -CN, halogen, or -OH;

L is $-(CR^{6a}R^{6b})_{n1}-$, $-O-(CR^{6a}R^{6b})_{n2}-$, $-S-(CR^{6a}R^{6b})_{n3}-$, or $-N(R^5)(CR^{6a}R^{6b})_{n4}-$;

$R^2$ is H, -COOH, $-N(R^5)_2$, $C_1$-$C_6$ alkyl, $C_1$-$C_6$ alkyl-O-, 3- to 7-membered cycloalkyl, 4- to 10-membered heterocycloalkyl, $C_6$-$C_{10}$ aryl, 5- to 10-membered heteroaryl, - $NHC(=NH)NH_2$, $-C(O)N(R^5)_2$, $-(CH_2OR^5)(CH_2)_{n5}OR^5$, $-NR^5C(=O)$-$C_6$-$C_{10}$ aryl, -C(=O)O-$C_1$-$C_6$ alkyl, 3- to 7-membered cycloalkyl substituted by one or more than one $R^{2a}$, 4- to 10-membered heterocycloalkyl substituted by one or more than one $R^{2b}$, $C_6$-$C_{10}$ aryl-C(=O)$NR^5$-substituted by one or more than one $R^{2c}$, $C_6$-$C_{10}$ aryl substituted by one or more than one $R^{2d}$, or 5- to 10-membered heteroaryl substituted by one or more than one $R^{2e}$; the heteroatom of the 4- to 10-membered heterocycloalkyl is selected from one or more than one of N, O, and S, and the number of heteroatoms is 1, 2, or 3; the heteroatom of the 5- to 10-membered heteroaryl is selected from one or more than one of N, O, and S, and the number of heteroatoms is 1, 2, or 3; when there is more than one substituent, the substituents are the same or different;

$R^{2a}$, $R^{2b}$, and $R^{2c}$ are each independently halogen, -OH, deuterium, -CN, -C(=O)H, Ci-$C_4$ alkyl, $C_2$-$C_4$ alkenyl, $C_2$-$C_4$ alkynyl, $C_1$-$C_4$ alkyl substituted by one or more than one $R^{2-a}$, $C_1$-$C_4$ alkyl-O-, phenyl-Q-, FOzS-phenylene-Q-, phenyl-C(=O)NH-, pyrazolyl substituted by one or more than one $C_1$-$C_4$ alkyl, $-N(R^5)_2$, ($C_1$-$C_4$ alkyl)-O-$C_1$-$C_4$ alkyl, ($C_1$-$C_4$ alkyl)-C(=O)-, =O, ($C_1$-$C_4$ alkyl substituted by one or more than one halogen)-C(=O)-, -SOzF, ($C_1$-$C_4$ alkyl)-$SO_2$-, ($C_1$-$C_4$ alkyl)-O-($C_1$-$C_4$ alkyl)-O-, $-CH_2OC(=O)N(R^5)_2$, ($C_1$-$C_4$ alkyl)-O-C(=O)-$NHCH_2$-, $-CH_2NHC(=O)N(R^5)_2$, ($C_1$-$C_4$ alkyl)-C(=O)$NHCH_2$-, (pyrazolyl)-$CH_2$-, ($C_1$-$C_4$ alkyl)-$SO_2$-$NHCH_2$-, (4- to 10-membered heterocycloalkyl)-C(=O)-$OCH_2$-, $(R^5)_2N$-C(=O)-O-, ($C_1$-$C_4$ alkyl)-O-($C_1$-$C_4$ alkyl)-NH-C(=O)-O-, phenyl-($C_1$-$C_4$ alkyl)-NH-C(=O)-O-, (4- to 10-membered heterocycloalkyl)-C(=O)-O-, or (4- to 10-membered heterocycloalkyl)-$CH_2$-; phenyl in the phenyl-C(=O)NH- and phenyl-($C_1$-$C_4$ alkyl)-NH-C(=O)-O- is optionally substituted by -C(=O)H, halogen, CN, or OH; 4- to 10-membered heterocycloalkyl in the (4-to 10-membered heterocycloalkyl)-C(=O)-$OCH_2$-, (4- to 10-membered heterocycloalkyl)-C(=O)-O-, and (4- to 10-membered heterocycloalkyl)-$CH_2$- is optionally substituted by =O; the heteroatom of the 4- to 10-membered heterocycloalkyl in the (4- to 10-membered heterocycloalkyl)-C(=O)-$OCH_2$-, (4- to 10-membered heterocycloalkyl)-C(=O)-O-, and (4- to 10-membered heterocycloalkyl)-$CH_2$- is selected from one or more than one of N, O, and S, and the number of heteroatoms is 1, 2, or 3; when there is more than one substituent, the substituents are the same or different;

Q is independently a linker bond or -O-;

each $R^{2-a}$ is independently deuterium, -CN, halogen, or -OH;

$R^{2d}$ and $R^{2e}$ are each independently halogen, -OH, -C(=O)H, $C_1$-$C_4$ alkyl, $C_2$-$C_4$ alkenyl, $C_2$-$C_4$ alkynyl, $C_1$-$C_4$ alkyl-O-, $C_1$-$C_4$ alkyl substituted by one or more than one halogen or OH, or -N($R^5$)$_2$; when there is more than one substituent, the substituents are the same or different;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and the 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, -CN, $C_1$-$C_6$ alkyl, $C_1$-$C_6$ alkyl-O-, $C_1$-$C_6$ alkyl-S-, $C_1$-$C_6$ alkyl substituted by one or more than one $R^{3\text{-}a}$, $C_1$-$C_6$ alkyl-O-substituted by one or more than one $R^{3\text{-}b}$, $C_1$-$C_6$ alkyl-S- substituted by one or more than one $R^{3\text{-}c}$, $C_2$-$C_6$ alkenyl, $C_2$-$C_6$ alkynyl, $C_2$-$C_6$ alkenyl substituted by one or more than one $R^{3\text{-}d}$, Cz-$C_6$ alkynyl substituted by one or more than one $R^{3\text{-}e}$, -N($R^5$)$_2$, -($CH_2$)-C(=O)N($R^5$)$_2$, 3- to 6-membered cycloalkyl, 3- to 6-membered cycloalkyl substituted by one or more than one $R^{3\text{-}f}$, or triazolyl; when there is more than one substituent, the substituents are the same or different;

$R^{3\text{-}a}$, $R^{3\text{-}b}$, $R^{3\text{-}c}$, $R^{3\text{-}d}$, $R^{3\text{-}e}$, and $R^{3\text{-}f}$ are each independently deuterium, halogen, -CN, - OH, $C_1$-$C_4$ alkyl, $C_1$-$C_4$ alkyl-O-, or 3- to 6-membered cycloalkyl;

$R^{4a}$ and $R^{4b}$ are each independently H, deuterium, -N($R^5$)$_2$, halogen, -OH, $C_1$-$C_6$ alkyl, -CN, halo-$C_1$-$C_6$ alkyl, $C_1$-$C_6$ alkyl-O-, deuterated $C_1$-$C_6$ alkyl, $C_2$-$C_6$ alkenyl, or $C_2$-$C_6$ alkynyl; when there is more than one substituent, the substituents are the same or different;

n1, n2, n3, n4, or n5 are each independently 0, 1, 2, or 3; (when being 0, it indicates a linker bond)

each $R^5$ is independently H or $C_1$-$C_6$ alkyl;

$R^{6a}$ and $R^{6b}$ are each independently H, deuterium, halogen, -CN, -OH, $C_1$-$C_4$ alkyl, or deuterated $C_1$-$C_4$ alkyl;

and, when ring A comprises two heteroatoms, $R^{4a}$ is not H.

In a certain embodiment, some groups in the nitrogen-containing heterocyclic compound of formula I, the stereoisomer thereof, or the pharmaceutically acceptable salt thereof are defined as follows, and unmentioned groups are as defined in any one embodiment of the present disclosure (the content of this paragraph is hereinafter referred to as "in a certain embodiment"):

the nitrogen-containing heterocyclic compound of formula I satisfies 1 or 2 of the following conditions:

(1) ring A is 3- to 7-membered cycloalkyl, 4- to 10-membered heterocycloalkyl, or 4- to 10-membered heterocycloalkenyl; the number of heteroatoms of the 4- to 10-membered heterocycloalkyl and the 4- to 10-membered heterocycloalkenyl is 1, wherein the heteroatom is selected from one of N, O, and S;

(2) $R^{4a}$ is deuterium, -N($R^5$)$_2$, halogen, -OH, $C_1$-$C_6$ alkyl, -CN, halo-$C_1$-$C_6$ alkyl, $C_1$-$C_6$ alkyl-O-, deuterated Ci-Ce alkyl, $C_2$-$C_6$ alkenyl, or $C_2$-$C_6$ alkynyl.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is selected from one of N, O, and S.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is selected from one of N, O, and S; n is 1, 2, 3, 4, 5, 6, 7, 8, 9, or 10, and at least one of $R^1$ is -N($R^5$)$_2$.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is selected from one of N, O, and S; n is 1, 2, 3, 4, 5, 6, 7, 8, 9, or 10, and only one of $R^1$ is - N($R^5$)$_2$.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is N.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is N; n is 1, 2, 3, 4, 5, 6, 7, 8, 9, or 10, and at least one of $R^1$ is -N($R^5$)$_2$.

In a certain embodiment, ring A is 4- to 10-membered heterocycloalkyl or 4- to 10-membered heterocycloalkenyl; the number of heteroatoms of the 4- to 10-membered heterocycloalkyl and the 4- to 10-membered heterocycloalkenyl is 1, wherein the heteroatom is N; n is 1, 2, 3, 4, 5, 6, 7, 8, 9, or 10, and only one of $R^1$ is -N($R^5$)$_2$.

In a certain embodiment, $R^{4a}$ is deuterium, -N($R^5$)$_2$, halogen, -OH, $C_1$-$C_6$ alkyl, -CN, halo-$C_1$-$C_6$ alkyl, $C_1$-$C_6$ alkyl-O-, deuterated $C_1$-$C_6$ alkyl, $C_2$-$C_6$ alkenyl, or $C_2$-$C_6$ alkynyl.

In a certain embodiment, $R^{4a}$ is $-N(R^5)_2$ or halogen.
In a certain embodiment, $R^{4a}$ is halogen.
In a certain embodiment, $R^{4a}$ is fluorine or chlorine.
In a certain embodiment, when ring A is 4- to 10-membered heterocycloalkyl, the 4-to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl.
In a certain embodiment, when ring A is 4- to 10-membered heterocycloalkyl, the number of heteroatoms of the 4- to 10-membered heterocycloalkyl is 1, wherein the heteroatom is N.
In a certain embodiment, when ring A is 4- to 10-membered heterocycloalkyl, the number of heteroatoms of the 4- to 10-membered heterocycloalkyl is 2, wherein the heteroatoms are N.
In a certain embodiment, when ring A is 4- to 10-membered heterocycloalkyl, the 4-to 10-membered heterocycloalkyl is connected to the pyrimidine ring shown in formula I through an N atom.
In a certain embodiment, when ring A is 4- to 10-membered heterocycloalkyl, the 4-to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

the number of heteroatoms of the 5- to 7-membered monocyclic heterocycloalkyl is 1 or 2, wherein the heteroatom is N;
the number of heteroatoms of the 6- to 8-membered fused heterocycloalkyl is 1 or 2, wherein the heteroatom is N;
the number of heteroatoms of the 6- to 8-membered bridged heterocycloalkyl is 1, wherein the heteroatom is N;
the number of heteroatoms of the 7- to 10-membered spiro heterocycloalkyl is 1 or 2, wherein the heteroatom is N.

In a certain embodiment, in ring A, the 5- to 7-membered monocyclic heterocycloalkyl may be

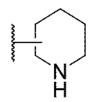

(for example,

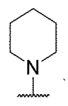

),

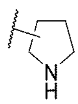

(for example,

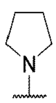 or 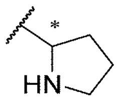,

for another example,

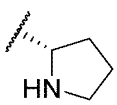

),

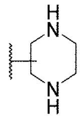

(for example,

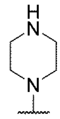

), or

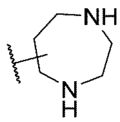

(for example,

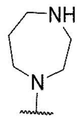

); "*" means that when a carbon atom with "*" is a chiral carbon atom, it is in an R configuration, an S configuration, or a mixture thereof.

In a certain embodiment, in ring A, the 5- to 7-membered monocyclic heterocycloalkyl may be

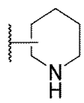

(for example,

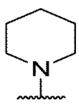

),

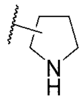

(for example,

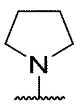

),

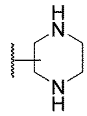

(for example,

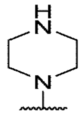

), or

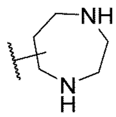

(for example,

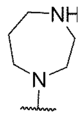

).

In a certain embodiment, in ring A, the 6- to 8-membered fused heterocycloalkyl may be

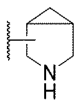

(for example,

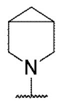

) or

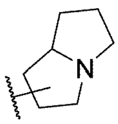

(for example,

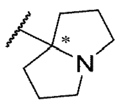

,

for another example,

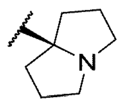

); "*" means that when a carbon atom with "*" is a chiral carbon atom, it is in an R configuration, an S configuration, or a mixture thereof.

In a certain embodiment, in ring A, the 6- to 8-membered fused heterocycloalkyl may be

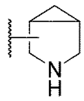

(for example,

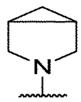

).

In a certain embodiment, in ring A, the 6- to 8-membered bridged heterocycloalkyl may be

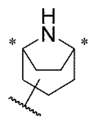

(for example,

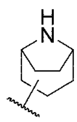

,

for another example,

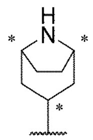

or

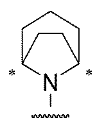

,

for yet another example,

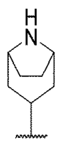

,

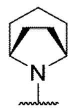

,

or

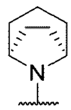

),

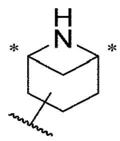

(for example,

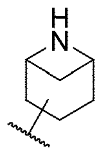

,

for another example,

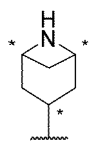

,

for yet another example,

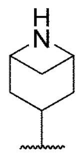

),

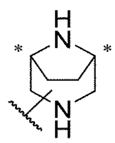

(for example,

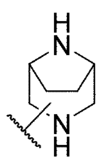

,

for another example,

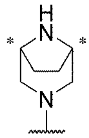,

for yet another example,

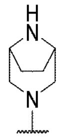

), or

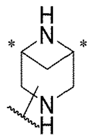

(for example,

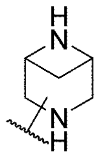,

for another example,

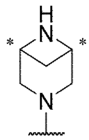,

for yet another example,

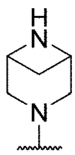

), "*" means that when a carbon atom with "*" is a chiral carbon atom, it is in an R configuration, an S configuration, or a mixture thereof.

In a certain embodiment, in ring A, the 6- to 8-membered bridged heterocycloalkyl may be

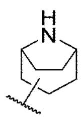

(for example,

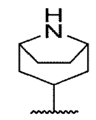

),

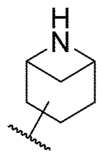

(for example,

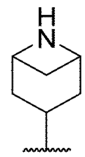

),

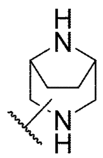

(for example,

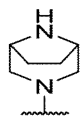

), or

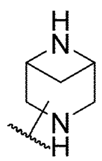

(for example,

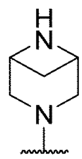

).

In a certain embodiment, in ring A, the 7- to 10-membered spiro heterocycloalkyl may be

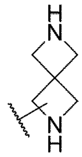

(for example,

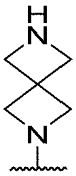

),

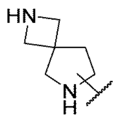

(for example,

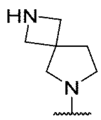

),

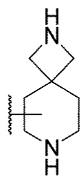

(for example,

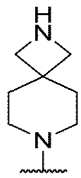

),

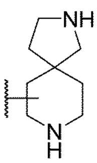

(for example,

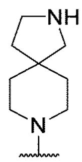

),

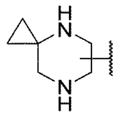

(for example,

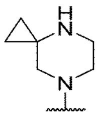

),

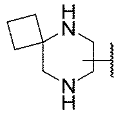

(for example,

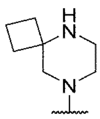

), or

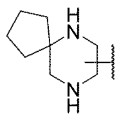

(for example,

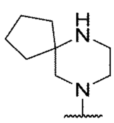

).

In a certain embodiment, when ring A is 4- to 10-membered heterocycloalkenyl, the 4- to 10-membered heterocycloalkenyl is 5- to 7-membered monocyclic heterocycloalkenyl, 6-to 8-membered fused heterocycloalkenyl, 6- to 8-membered bridged heterocycloalkenyl, or 7-to 10-membered spiro heterocycloalkenyl.

In a certain embodiment, when ring A is 4- to 10-membered heterocycloalkenyl, the number of heteroatoms of the 4- to 10-membered heterocycloalkenyl is 1, wherein the heteroatom is N.

In a certain embodiment, when ring A is 4- to 10-membered heterocycloalkenyl, the number of heteroatoms of the 4- to 10-membered heterocycloalkenyl is 2, wherein the heteroatoms are N.

In a certain embodiment, when ring A is 4- to 10-membered heterocycloalkenyl, the 4- to 10-membered heterocycloalkenyl is connected to the pyrimidine ring shown in formula I through a C atom.

In a certain embodiment, when ring A is 4- to 10-membered heterocycloalkenyl, the 4- to 10-membered heterocycloalkenyl is connected to the pyrimidine ring shown in formula I through an N atom.

In a certain embodiment, in ring A, the 6- to 8-membered bridged heterocycloalkenyl may be

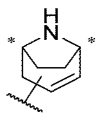 or 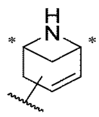,

for example,

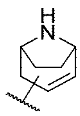 or 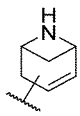,

for another example,

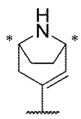

or

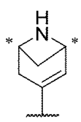,

for yet another example,

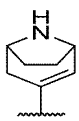 or 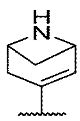;

"*" means that when a carbon atom with "*" is a chiral carbon atom, it is in an R configuration, an S configuration, or a mixture thereof.

In a certain embodiment, in ring A, the 6- to 8-membered bridged heterocycloalkenyl may be

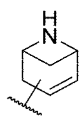

(for example,

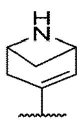

) or

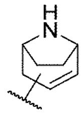

(for example,

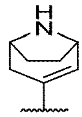

).

In a certain embodiment, when ring A is 4- to 10-membered heterocycloalkyl, the 4-to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

the 5- to 7-membered monocyclic heterocycloalkyl may be

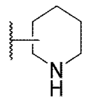

(for example,

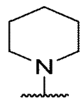

),

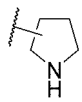

(for example,

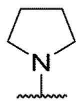

),

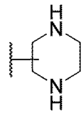

(for example,

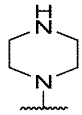

), or

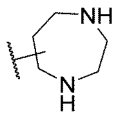

(for example,

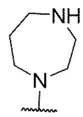

);

the 6- to 8-membered fused heterocycloalkyl may be

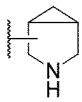

(for example,

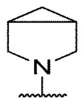

);

the 6- to 8-membered bridged heterocycloalkyl may be

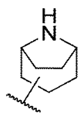

(for example,

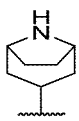

),

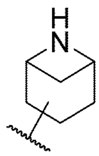

(for example,

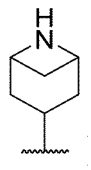

),

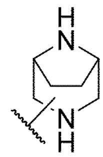

(for example,

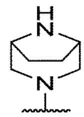

), or

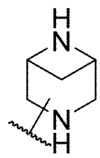

(for example,

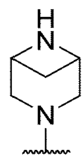

);

the 7- to 10-membered spiro heterocycloalkyl may be

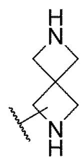

(for example,

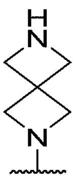

),

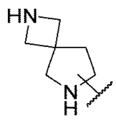

(for example,

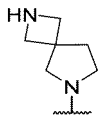

),

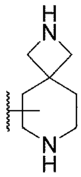

(for example,

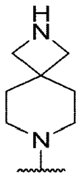

),

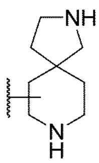

(for example,

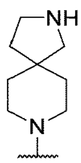

),

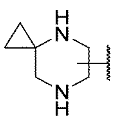

(for example,

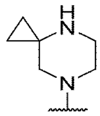

),

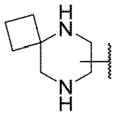

(for example,

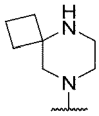

), or

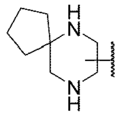

(for example,

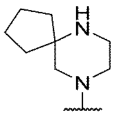

).

In a certain embodiment,

when ring A is 4- to 10-membered heterocycloalkyl, the 4- to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

the 5- to 7-membered monocyclic heterocycloalkyl may be

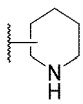

(for example,

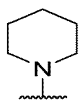

),

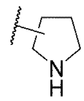

(for example,

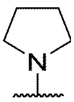

),

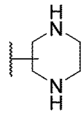

(for example,

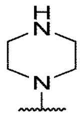

), or

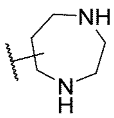

(for example,

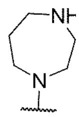

);

the 6- to 8-membered fused heterocycloalkyl may be

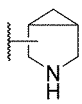

(for example,

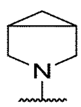

);

the 6- to 8-membered bridged heterocycloalkyl may be

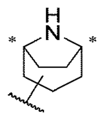

(for example,

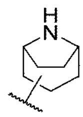,

for another example,

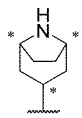 or 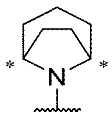,

for yet another example,

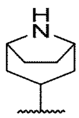, 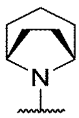, or 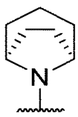

),

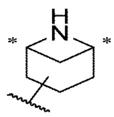

(for example,

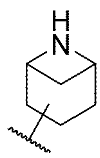,

for another example,

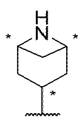,

for yet another example,

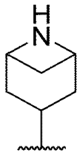

),

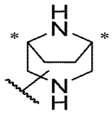

(for example,

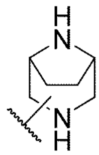

,

for another example,

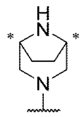

,

for yet another example,

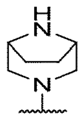

), or

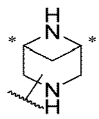

(for example,

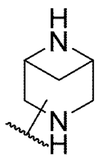

,

for another example,

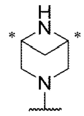,

for yet another example,

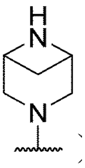

), "*" means that when a carbon atom with "*" is a chiral carbon atom, it is in an R configuration, an S configuration, or a mixture thereof;

the 7- to 10-membered spiro heterocycloalkyl may be

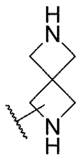

(for example,

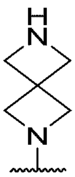

),

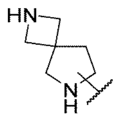

(for example,

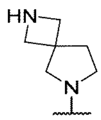

),

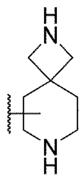

(for example,

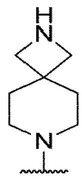

),

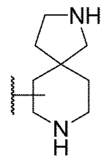

(for example,

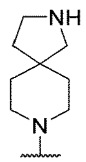

),

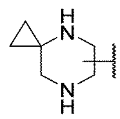

(for example,

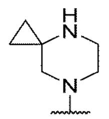

),

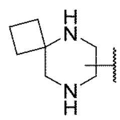

(for example,

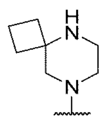

), or

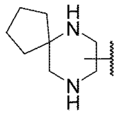

(for example,

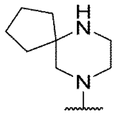

).

In a certain embodiment,

when ring A is 4- to 10-membered heterocycloalkenyl, the 4- to 10-membered heterocycloalkenyl is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl;

the 6- to 8-membered bridged heterocycloalkenyl may be

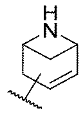

(for example,

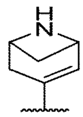

) or

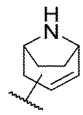

(for example,

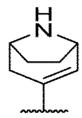

).

In a certain embodiment,

when ring A is 4- to 10-membered heterocycloalkenyl, the 4- to 10-membered heterocycloalkenyl is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl;

the 6- to 8-membered bridged heterocycloalkenyl may be

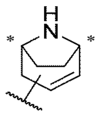 or 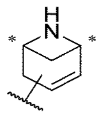 ,

for example,

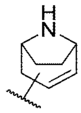 or 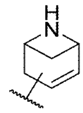 ,

for another example,

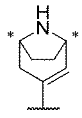 or 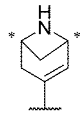 ,

for yet another example,

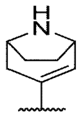 or 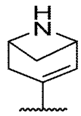 ;

"*" means that when a carbon atom with "*" is a chiral carbon atom, it is in an R configuration, an S configuration, or a mixture thereof.

In a certain embodiment,
when $R^1$ is halogen, the halogen is fluorine, chlorine, bromine, or iodine, such as fluorine.

In a certain embodiment,
when $R^2$ is 4- to 10-membered heterocycloalkyl or 4- to 10-membered heterocycloalkyl substituted by one or more than one $R^{2b}$, 4- to 10-membered heterocycloalkyl in the 4- to 10-membered heterocycloalkyl and the 4- to 10-membered heterocycloalkyl substituted by one or more than one $R^{2b}$ is 5- to 7-membered monocyclic heterocycloalkyl, 6-to 8-membered fused heterocycloalkyl, 6- to 8-membered bridged heterocycloalkyl, or 7- to 10-membered spiro heterocycloalkyl.

In a certain embodiment,
when $R^2$ is 4- to 10-membered heterocycloalkyl or 4- to 10-membered heterocycloalkyl substituted by one or more than one $R^{2b}$, 4- to 10-membered heterocycloalkyl in the 4- to 10-membered heterocycloalkyl and the 4- to 10-membered heterocycloalkyl substituted by one or more than one $R^{2b}$ is 6- to 8-membered fused heterocycloalkyl.

In a certain embodiment,
in $R^2$, the 5- to 7-membered monocyclic heterocycloalkyl may be

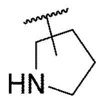 ,

for example,

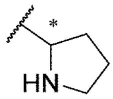 ,

"*" means that when a carbon atom with "*" is a chiral carbon atom, it is in an R configuration, an S configuration, or a mixture thereof; for another example,

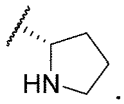

In a certain embodiment,
in $R^2$, the 6- to 8-membered fused heterocycloalkyl may be

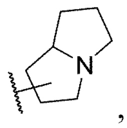

for example,

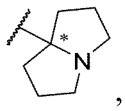

"*" means that when a carbon atom with "*" is a chiral carbon atom, it is in an R configuration, an S configuration, or a mixture thereof; for another example,

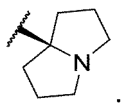

In a certain embodiment,

when $R^2$ is 4- to 10-membered heterocycloalkyl or 4- to 10-membered heterocycloalkyl substituted by one or more than one $R^{2b}$, 4- to 10-membered heterocycloalkyl in the 4- to 10-membered heterocycloalkyl and the 4- to 10-membered heterocycloalkyl substituted by one or more than one $R^{2b}$ is 5- to 7-membered monocyclic heterocycloalkyl, 6-to 8-membered fused heterocycloalkyl, 6- to 8-membered bridged heterocycloalkyl, or 7- to 10-membered spiro heterocycloalkyl;

the 5- to 7-membered monocyclic heterocycloalkyl may be

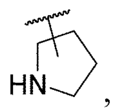

for example,

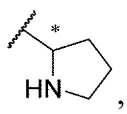

"*" means that when a carbon atom with "*" is a chiral carbon atom, it is in an R configuration, an S configuration, or a mixture thereof; for another example,

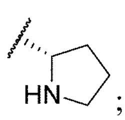

the 6- to 8-membered fused heterocycloalkyl may be

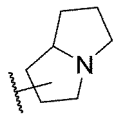

,

for example,

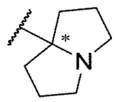

,

"*" means that when a carbon atom with "*" is a chiral carbon atom, it is in an R configuration, an S configuration, or a mixture thereof; for another example,

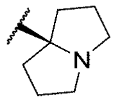

.

In a certain embodiment,
when $R^{2a}$, $R^{2b}$, $R^{2c}$, $R^{2d}$, and $R^{2e}$ are each independently halogen, the halogen is fluorine, chlorine, bromine, or iodine, such as fluorine.

In a certain embodiment,
when $R^3$ is $C_6$-$C_{10}$ aryl or $C_6$-$C_{10}$ aryl substituted by one or more than one $R^{3a}$, $C_6$-$C_{10}$ aryl in the $C_6$-$C_{10}$ aryl and the $C_6$-$C_{10}$ aryl substituted by one or more than one $R^{3a}$ is phenyl or naphthyl, such as

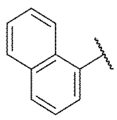

.

In a certain embodiment,
when $R^3$ is $C_6$-$C_{10}$ aryl or $C_6$-$C_{10}$ aryl substituted by one or more than one $R^{3a}$, $C_6$-$C_{10}$ aryl in the $C_6$-$C_{10}$ aryl and the $C_6$-$C_{10}$ aryl substituted by one or more than one $R^{3a}$ is not fused with other rings (such as cycloalkyl, cycloalkenyl, heterocycloalkyl, or heterocycloalkenyl).

In a certain embodiment,
when $R^3$ is 5- to 14-membered heteroaryl or 5- to 14-membered heteroaryl substituted by one or more than one $R^{3b}$, 5- to 14-membered heteroaryl in the 5- to 14-membered heteroaryl and the 5- to 14-membered heteroaryl substituted by one or more than one $R^{3b}$ is 5- to 10-membered heteroaryl, for example, pyridyl, pyrimidinyl, quinolinyl, quinazolinyl, benzothienyl, benzothiazolyl, or indazolyl, and for another example,

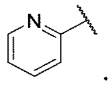

.

In a certain embodiment,
when $R^3$ is 5- to 14-membered heteroaryl or 5- to 14-membered heteroaryl substituted by one or more than one $R^{3b}$, 5- to 14-membered heteroaryl in the 5- to 14-membered heteroaryl and 5- to 14-membered heteroaryl substituted by one or more than one $R^{3b}$ is not fused with other rings (such as cycloalkyl, cycloalkenyl, heterocycloalkyl, or heterocycloalkenyl).

In a certain embodiment,
when $R^{3a}$ and $R^{3b}$ are independently halogen, the halogen is fluorine, chlorine, bromine, or iodine; for example, fluorine or chlorine.

In a certain embodiment,

when $R^{3a}$ and $R^{3b}$ are independently $C_1$-$C_6$ alkyl, $C_1$-$C_6$ alkyl-O-, $C_1$-$C_6$ alkyl-S-, Ci-$C_6$ alkyl substituted by one or more than one $R^{3a}$, $C_1$-$C_6$ alkyl-O- substituted by one or more than one $R^{3-b}$, or $C_1$-$C_6$ alkyl-S- substituted by one or more than one $R^{3-c}$, $C_1$-$C_6$ alkyl in the Ci-$C_6$ alkyl, $C_1$-$C_6$ alkyl-O-, $C_1$-$C_6$ alkyl-S-, $C_1$-$C_6$ alkyl substituted by one or more than one $R^{3-a}$, $C_1$-$C_6$ alkyl-O- substituted by one or more than one $R^{3-b}$, and $C_1$-$C_6$ alkyl-S- substituted by one or more than one $R^{3-c}$ is independently $C_1$-$C_4$ alkyl, for example, methyl or ethyl.

In a certain embodiment,
when $R^{3a}$ and $R^{3b}$ are independently $C_2$-$C_6$ alkynyl or $C_2$-$C_6$ alkynyl substituted by one or more than one $R^{3-e}$, $C_2$-$C_6$ alkynyl in the $C_2$-$C_6$ alkynyl and $C_2$-$C_6$ alkynyl substituted by one or more than one $R^{3-e}$ is $C_2$-$C_4$ alkynyl, for example, ethynyl.

In a certain embodiment,
when $R^{3-a}$, $R^{3-b}$, $R^{3-c}$, $R^{3-d}$, $R^{3-e}$, and $R^{3-f}$ are independently halogen, the halogen is fluorine, chlorine, bromine, or iodine; for example, fluorine or chlorine.

In a certain embodiment,
when $R^{3a}$ and $R^{3b}$ are independently $C_1$-$C_6$ alkyl substituted by one or more than one $R^{3-a}$, $C_1$-$C_6$ alkyl-O- substituted by one or more than one $R^{3-b}$, $C_1$-$C_6$ alkyl-S- substituted by one or more than one $R^{3-c}$, $C_2$-$C_6$ alkenyl substituted by one or more than one $R^{3-d}$, $C_2$-$C_6$ alkynyl substituted by one or more than one $R^{3-e}$, or 3- to 6-membered cycloalkyl substituted by one or more than one $R^{3-f}$, the number of substituents is 1, 2, 3, or 4; for example, 3.

In a certain embodiment,
when $R^{4a}$ and $R^{4b}$ are halogen, the halogen is fluorine, chlorine, bromine, or iodine; for example, fluorine or chlorine.

In a certain embodiment,
$R^5$ is independently H.

In a certain embodiment,
$R^{6a}$ and $R^{6b}$ are independently H.

In a certain embodiment,
n is 0, 1, 2, or 3; for example, n is 1, 2, or 3.

In a certain embodiment,
n1, n2, n3, n4, or n5 are each independently 1.

In a certain embodiment,
each $R^1$ is independently halogen, -OH, or -$NH_2$; for example, halogen or -$NH_2$.

In a certain embodiment, all atoms in the nitrogen-containing heterocyclic compound of formula I, the stereoisomer thereof, or the pharmaceutically acceptable salt thereof have atomic masses typically found in nature, that is, the all atoms are atoms comprising naturally proportional isotopes.

In a certain embodiment,
ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1 or 2, and the heteroatom is N.

In a certain embodiment,
ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, the heteroatom is N, and the N is connected to the pyrimidine ring shown in formula I (i.e.,

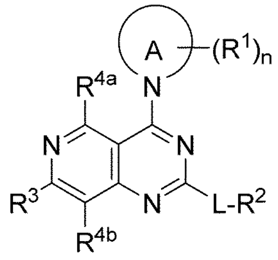

**I-1**

); preferably, n is 1, 2, or 3; $R^1$ is independently halogen, -OH, or -$NH_2$ and at least one $R^1$ is -$NH_2$.

In a certain embodiment,
ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, and the heteroatom is N; and the atom in ring A connected to the pyrimidine ring shown in formula I is a carbon atom (for example, CH or C) (i.e.,

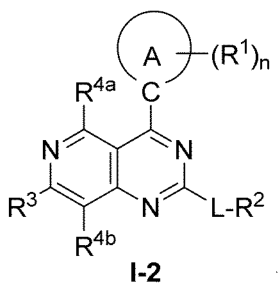

I-2

); preferably, n is 0.

In a certain embodiment,

ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 2, the heteroatoms are N, and one of the N is connected to the pyrimidine ring shown in formula I.

In a certain embodiment,

when L is -O-$(CR^{6a}R^{6b})_{n2}$-, -S-$(CR^{6a}R^{6b})_{n3}$-, or -N($R^5$)$(CR^{6a}R^{6b})_{n4}$, $R^2$ is bonded to the end of $(CR^{6a}R^{6b})_{n2}$, $(CR^{6a}R^{6b})_{n3}$, or $(CR^{6a}R^{6b})_{n4}$ in the L group, or $R^2$ is bonded to O, S, or N atom in the L group; preferably, $R^2$ is bonded to the end of $(CR^{6a}R^{6b})_{n2}$, $(CR^{6a}R^{6b})_{n3}$, or $(CR^{6a}R^{6b})_{n4}$ in the L group.

In a certain embodiment,

L is -O-$(CR^{6a}R^{6b})_{n2}$-.

In a certain embodiment,

$R^5$ is independently H.

In a certain embodiment,

$R^{6a}$ and $R^{6b}$ are independently H or deuterium.

In a certain embodiment,

$R^{6a}$ and $R^{6b}$ are independently H.

In a certain embodiment,

$R^2$ is 4- to 10-membered heterocycloalkyl substituted by one or more than one $R^{2b}$.

In a certain embodiment,

$R^{2b}$ is halogen or $C_1$-$C_4$ alkyl, for example, F or methyl.

In a certain embodiment,

$R^3$ is $C_6$-$C_{10}$ aryl substituted by one or more than one $R^{3a}$ or 5- to 14-membered heteroaryl substituted by one or more than one $R^{3b}$.

In a certain embodiment,

$R^{3a}$ and $R^{3b}$ are each independently halogen, -OH, $C_1$-$C_6$ alkyl, Ci-Ce alkyl substituted by one or more than one $R^{3\text{-}a}$, $C_2$-$C_6$ alkynyl, $C_2$-$C_6$ alkynyl substituted by one or more than one $R^{3\text{-}e}$, or -N$(R^5)_2$; for example, $R^{3a}$ and $R^{3b}$ are each independently halogen, -OH, $C_1$-$C_4$ alkyl, $C_1$-$C_4$ alkyl substituted by one or more than one $R^{3\text{-}a}$, $C_2$-$C_4$ alkynyl, $C_2$-$C_4$ alkynyl substituted by one or more than one $R^{3\text{-}e}$, or -N$(R^5)_2$.

In a certain embodiment,

$R^{3a}$ and $R^{3b}$ are each independently halogen, -OH, $C_1$-$C_6$ alkyl, $C_1$-$C_6$ alkyl substituted by one or more than one $R^{3\text{-}a}$, $C_2$-$C_6$ alkynyl, or -N$(R^5)_2$; for example, $R^{3a}$ and $R^{3b}$ are each independently halogen, -OH, $C_1$-$C_4$ alkyl, $C_1$-$C_4$ alkyl substituted by one or more than one $R^{3\text{-}a}$, $C_2$-$C_4$ alkynyl, or -N$(R^5)_2$.

In a certain embodiment,

$R^{3\text{-}a}$ is halogen, for example, F.

In a certain embodiment,

$R^{3\text{-}e}$ is deuterium.

In a certain embodiment,

$R^{4a}$ and $R^{4b}$ are independently H, -N$(R^5)_2$, or halogen.

In a certain embodiment, ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S.

In a certain embodiment, n is 0.

In a certain embodiment, L is -O-$(CR^{6a}R^{6b})_{n2}$-; n2 is 1; $R^{6a}$ and $R^{6b}$ are each independently H or deuterium.

In a certain embodiment, $R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one

or more than one of N, O, and S, and the number of heteroatoms is 1, 2, or 3; each $R^{2b}$ is independently halogen or $C_1$-$C_4$ alkyl.

In a certain embodiment, $R^2$ is 4- to 10-membered heterocycloalkyl substituted by one or more than one $R^{2b}$; in 4- to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3; each $R^{2b}$ is independently halogen.

In a certain embodiment, $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5-to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, $C_1$-$C_6$ alkyl, $C_1$-$C_6$ alkyl substituted by one or more than one $R^{3-a}$, - CN, $C_2$-$C_6$ alkynyl, $C_2$-$C_6$ alkynyl substituted by one or more than one $R^{3-e}$, -$N(R^5)_2$, or triazolyl; when there is more than one substituent, the substituents are the same or different; each $R^{3-a}$ is independently halogen; $R^{3-e}$ is deuterium; each $R^5$ is H or $C_1$-$C_6$ alkyl.

In a certain embodiment, $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5-to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, $C_1$-$C_6$ alkyl, $C_1$-$C_6$ alkyl substituted by one or more than one $R^{3-a}$, - CN, $C_2$-$C_6$ alkynyl, -$N(R^5)_2$, or triazolyl; when there is more than one substituent, the substituents are the same or different; each $R^{3-a}$ is independently halogen; each $R^5$ is H or Ci-$C_6$ alkyl.

In a certain embodiment, $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5-to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, -CN, $C_2$-$C_6$ alkynyl, $C_2$-$C_6$ alkynyl substituted by one or more than one $R^{3-e}$, -$N(R^5)_2$, or triazolyl; $R^{3-e}$ is deuterium; when there is more than one substituent, the substituents are the same or different; each $R^5$ is H.

In a certain embodiment, $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5-to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, -CN, $C_2$-$C_6$ alkynyl, -$N(R^5)_2$, or triazolyl; when there is more than one substituent, the substituents are the same or different; each $R^5$ is H.

In a certain embodiment, $R^{4a}$ and $R^{4b}$ are each independently halogen.

In a certain embodiment,
ring A is

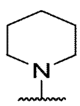, 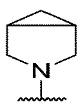, 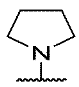, 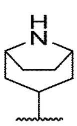, 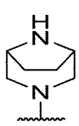, 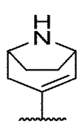, 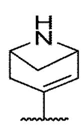, 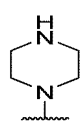, 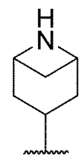,

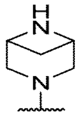, 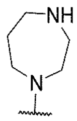, 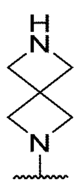, 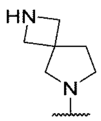, 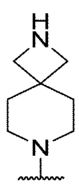, 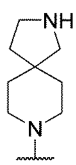, 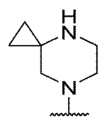, 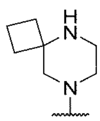, or 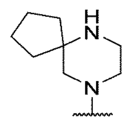.

In a certain embodiment,
ring A is

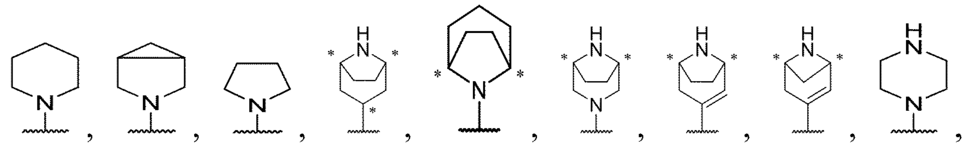

, , , , , , , , ,

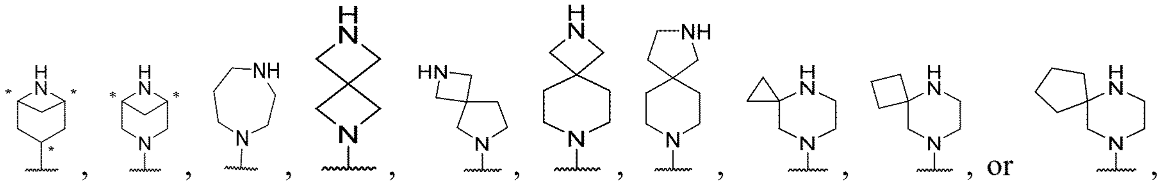

, , , , , , , , , or ,

for example,

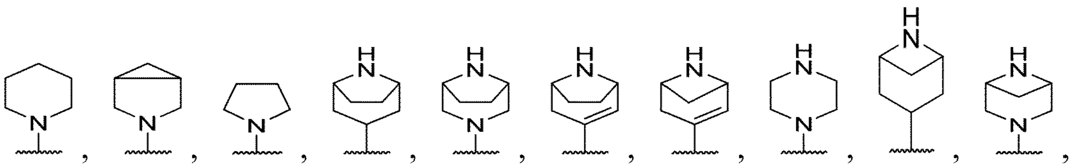

, , , , , , , , , , ,

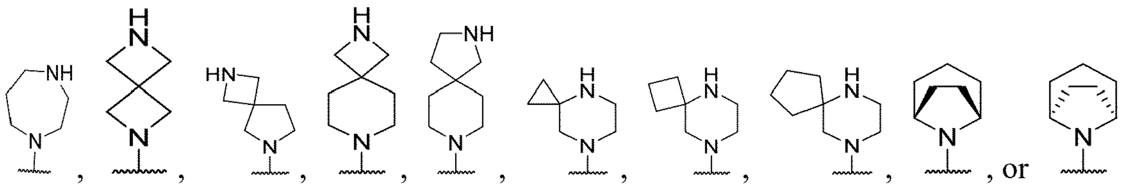

, , , , , , , , , , or .

In a certain embodiment,
$R^1$ is independently -OH, F, or $-NH_2$.

In a certain embodiment,
L is $-O-(CH_2)-$ or $-O-(CD_2)-$.

In a certain embodiment,
L is $-O-(CH_2)-$.

In a certain embodiment,
$R^2$ is

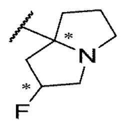

or

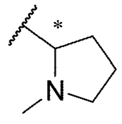

,

for example,

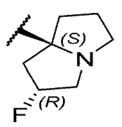

or

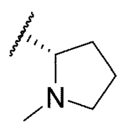

.

In a certain embodiment,
$R^{3a}$ and $R^{3b}$ are each independently -OH, fluorine, $-CH_3$, $-CF_3$, ethyl, ethynyl,

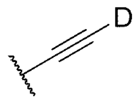,

or $-NH_2$.

In a certain embodiment,

$R^{3a}$ and $R^{3b}$ are each independently -OH, fluorine, $-CH_3$, $-CF_3$, ethyl, ethynyl, or $-NH_2$.

In a certain embodiment,

$R^{4a}$ and $R^{4b}$ are independently H, F, Cl, or $-NH_2$.

In a certain embodiment,

$R^3$ is

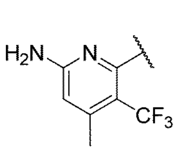, 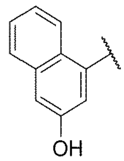, 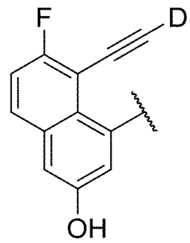, 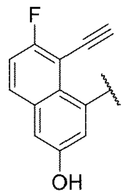, 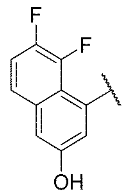, or 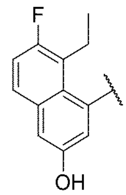.

In a certain embodiment,

$R^3$ is

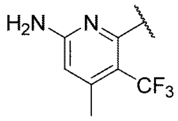, 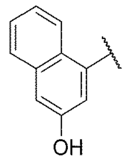, 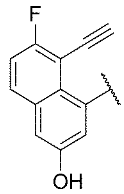, 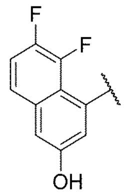, or 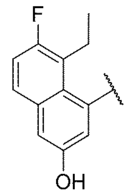.

In a certain embodiment,

$R^3$ is

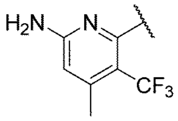, 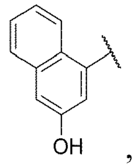, 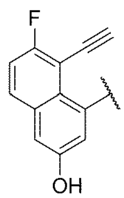, or 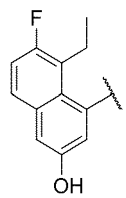.

In a certain embodiment,

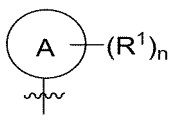

is

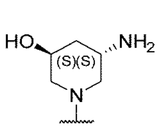, 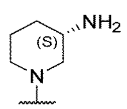, 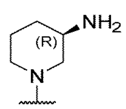, 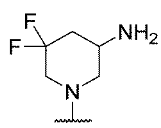, 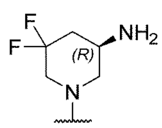,

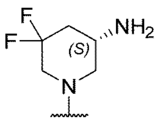, 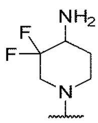, 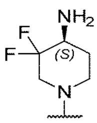, 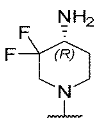, 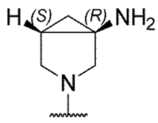, 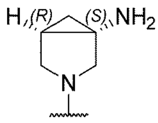, 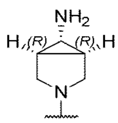, 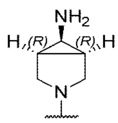,

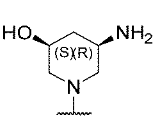, 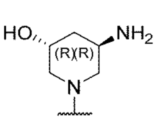, 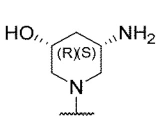, 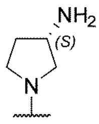, 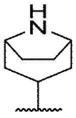, 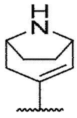, or 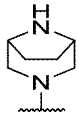.

In a certain embodiment,

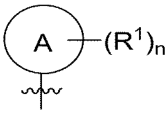

is

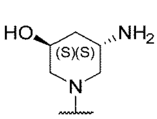, 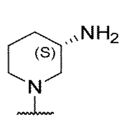, 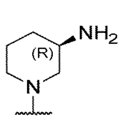, 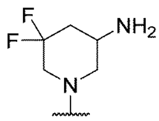, 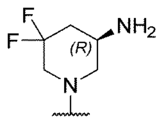,

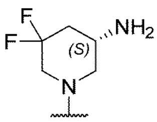, 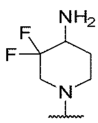, 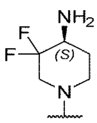, 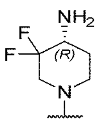, 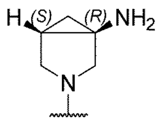, 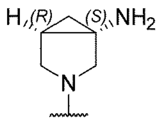, 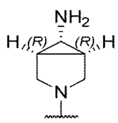, 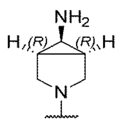,

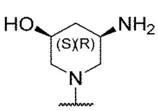, 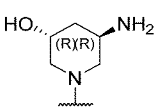, 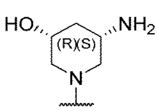, 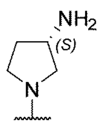, 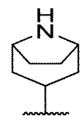, 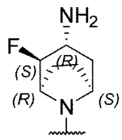, 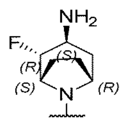, 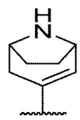,

or

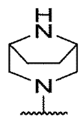.

In a certain embodiment, -L-$R^2$ is

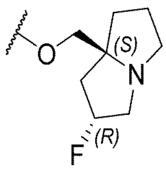 or 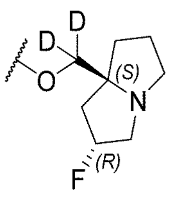.

In a certain embodiment,

$-L-R^2$ is

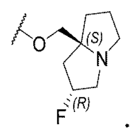

.

In a certain embodiment,

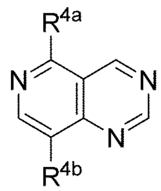

is

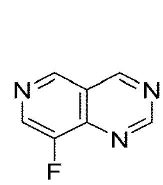
,
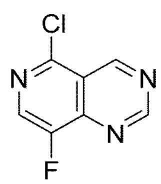
,
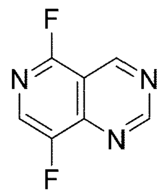
, or
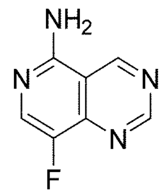
.

In a certain embodiment,

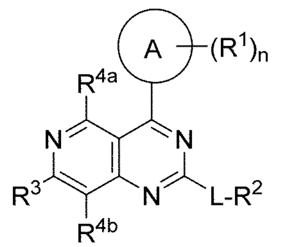

I

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})_{n2}-$;

$R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen or $C_1$-$C_4$ alkyl;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, $C_1$-$C_6$ alkyl, $C_1$-$C_6$ alkyl substituted by one or more than one $R^{3a}$, -CN, $C_2$-$C_6$ alkynyl, $C_2$-$C_6$ alkynyl substituted by one or more than one $R^{3-e}$, $-N(R^5)_2$, or triazolyl;

when there is more than one substituent, the substituents are the same or different;
each $R^{3-a}$ is independently halogen; $R^{3-e}$ is deuterium;
$R^{4a}$ and $R^{4b}$ are each independently halogen;
n2 is 1;
each $R^5$ is H or $C_1$-$C_6$ alkyl;
$R^{6a}$ and $R^{6b}$ are each independently H or deuterium;
the nitrogen-containing heterocyclic compound of formula I is not any one of the following compounds:

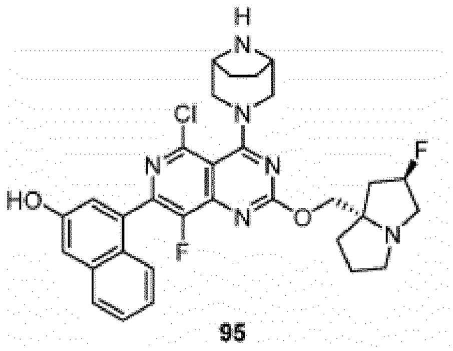
95
,

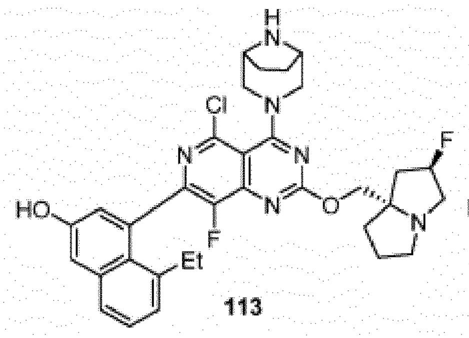
113

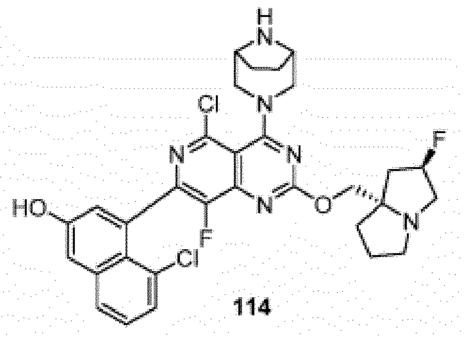
114
,

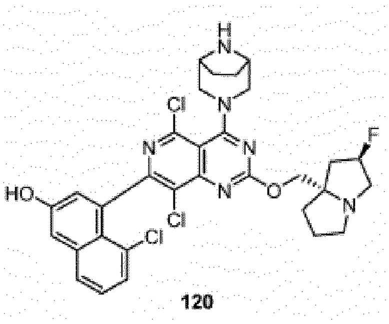
120
,

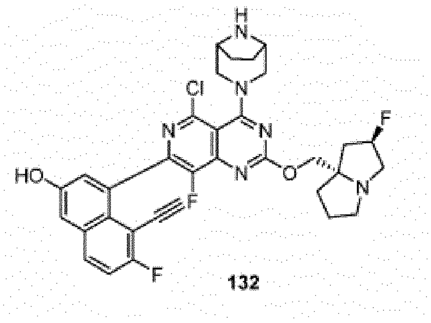
132
,

and

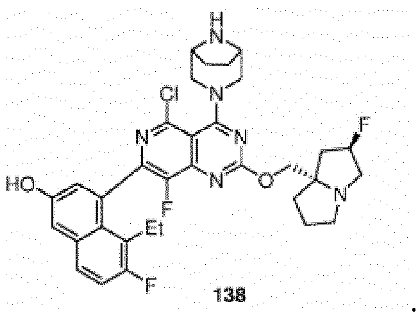
138
.

In a certain embodiment,

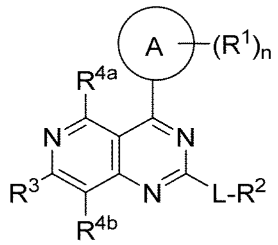

I

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})_{n2}-$;

$R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen or $C_1$-$C_4$ alkyl;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, $C_1$-$C_6$ alkyl, $C_1$-$C_6$ alkyl substituted by one or more than one $R^{3-a}$, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different;

each $R^{3-a}$ is independently halogen; $R^{3-e}$ is deuterium;

$R^{4a}$ and $R^{4b}$ are each independently halogen;

n2 is 1;

each $R^5$ is H or $C_1$-$C_6$ alkyl;

$R^{6a}$ and $R^{6b}$ are each independently H or deuterium;

the nitrogen-containing heterocyclic compound of formula I is not any one of the following compounds:

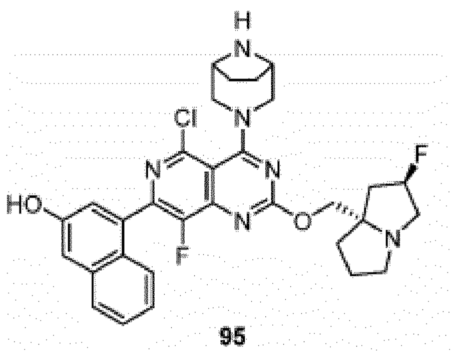

95

,

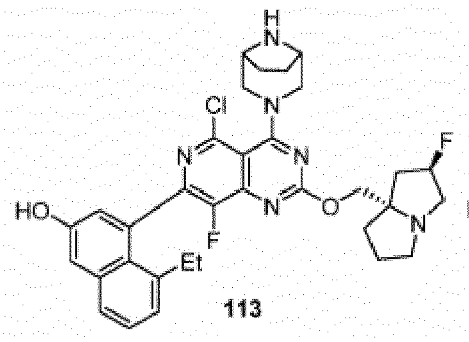
113

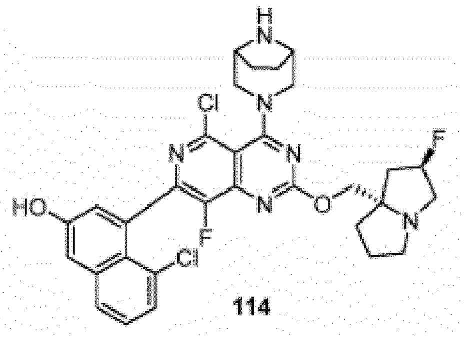
114

,

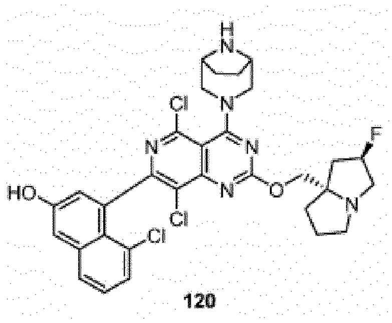
120

,

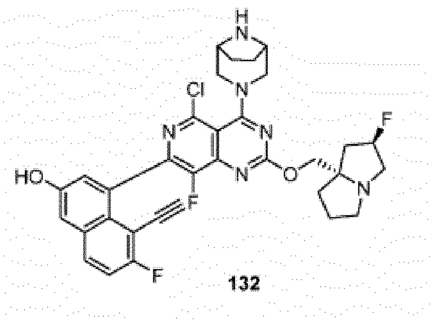
132

,

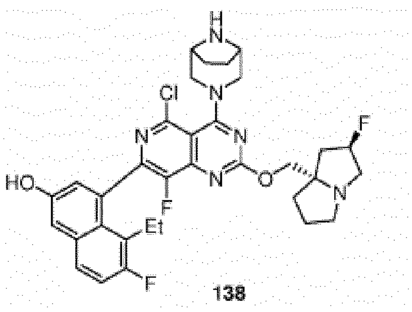
138

,

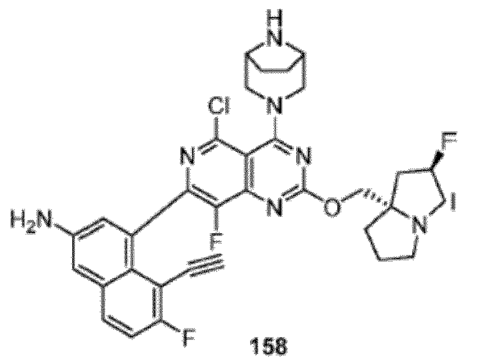
158

,

and

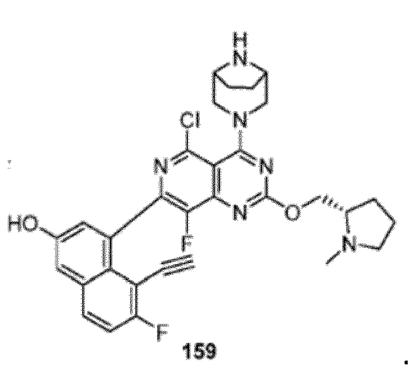
159

.

In a certain embodiment,

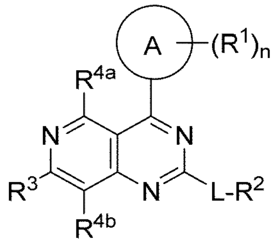

I

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})_{n2}-$;

$R^2$ is 4- to 10-membered heterocycloalkyl substituted by one or more than one $R^{2b}$; in 4- to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different; $R^{3-e}$ is deuterium;

$R^{4a}$ and $R^{4b}$ are each independently halogen;

n2 is 1;

each $R^5$ is H;

$R^{6a}$ and $R^{6b}$ are each independently H or deuterium;

the nitrogen-containing heterocyclic compound of formula I is not any one of the following compounds:

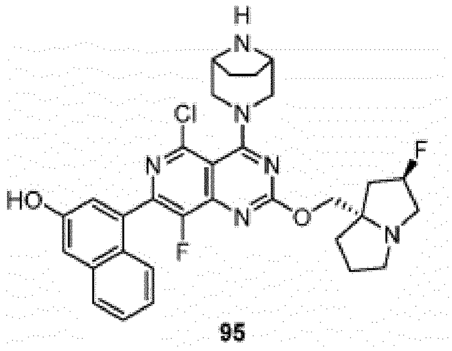

95

,

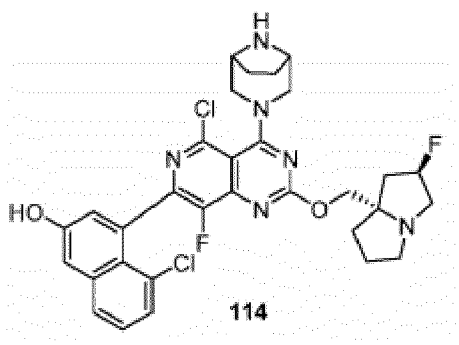

114

,

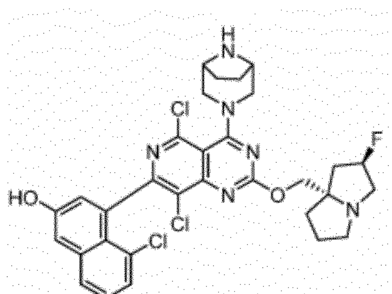

120

, and

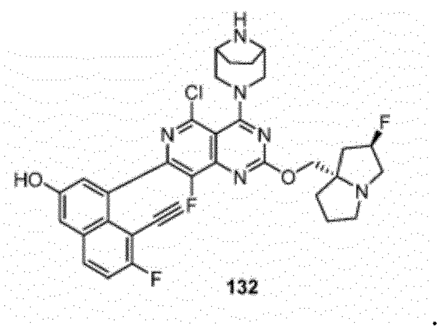

132

.

In a certain embodiment,

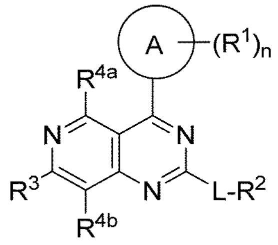

I

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})_{n2}-$;

$R^2$ is 4- to 10-membered heterocycloalkyl substituted by one or more than one $R^{2b}$; in 4- to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different; $R^{3-e}$ is deuterium;

$R^{4a}$ and $R^{4b}$ are each independently halogen;

n2 is 1;

each $R^5$ is H;

$R^{6a}$ and $R^{6b}$ are each independently H or deuterium;

the nitrogen-containing heterocyclic compound of formula I is not any one of the following compounds:

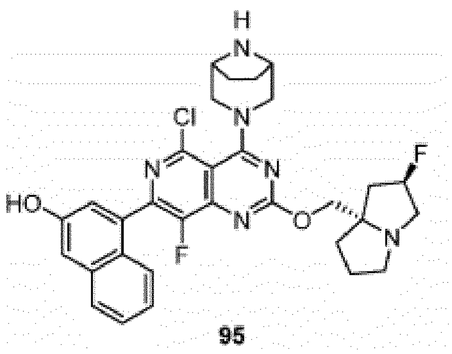

95

,

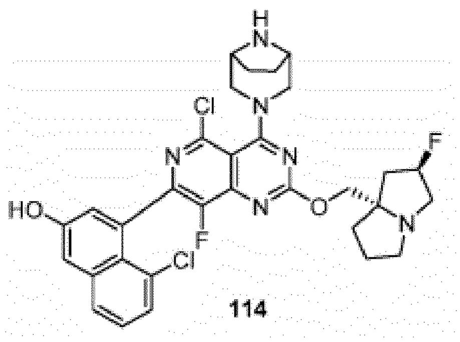

114

,

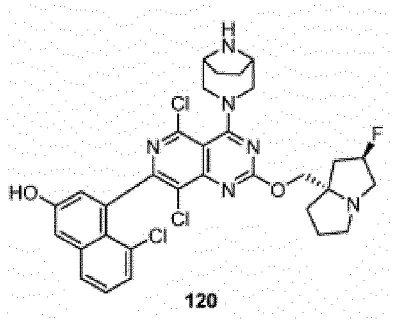

120

,

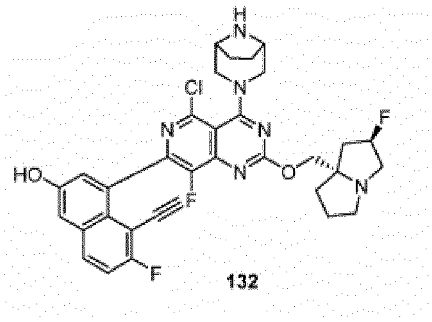

132

,

and

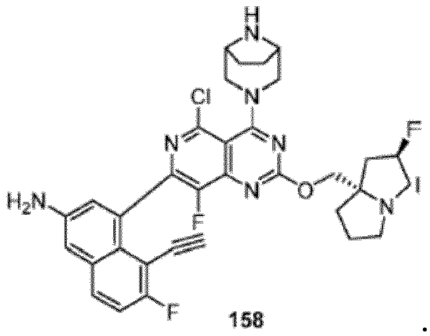

158

.

In a certain embodiment,

wherein ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1 or 2, and the heteroatom is N;
n is 0, 1, 2, or 3;
$R^1$ is independently halogen, -OH, or $-N(R^5)_2$;
L is $-O-(CR^{6a}R^{6b})_{n2}-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$;
$R^{3a}$ and $R^{3b}$ are independently halogen, -OH, $C_1$-$C_6$ alkyl, $C_1$-$C_6$ alkyl substituted by one or more than one $R^{3-a}$, $C_2$-$C_6$ alkynyl, or $-N(R^5)_2$;
$R^{3-a}$ is independently halogen;
$R^{4a}$ and $R^{4b}$ are independently H, $-N(R^5)_2$, or halogen;
$R^5$ is independently hydrogen;
$R^{6a}$ and $R^{6b}$ are H;
n2 is 1;
and, when ring A comprises two heteroatoms, $R^{4a}$ is not H.

In a certain embodiment, the nitrogen-containing heterocyclic compound of formula I is shown in formula 1-1;

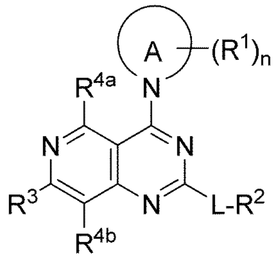

I-1

wherein ring A is 4- to 10-membered heterocycloalkyl; the number of heteroatoms of the 4- to 10-membered heterocycloalkyl is 1;
n is 1, 2, or 3;
each $R^1$ is independently halogen, -OH, or $-N(R^5)_2$;
L is $-O-(CR^{6a}R^{6b})_{n2}-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is

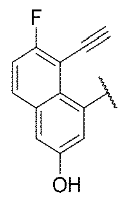

;

$R^{4a}$ and $R^{4b}$ are independently H, $-N(R^5)_2$, or halogen;
$R^5$ is independently hydrogen;
$R^{6a}$ and $R^{6b}$ are H;
n2 is 1.

In a certain embodiment, the nitrogen-containing heterocyclic compound of formula I is shown in formula 1-1;

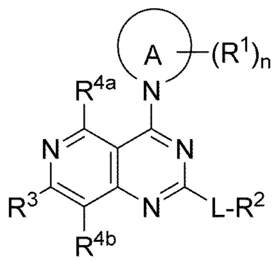

I-1

wherein ring A is 4- to 10-membered heterocycloalkyl; the number of heteroatoms of the 4- to 10-membered heterocycloalkyl is 2, and the heteroatoms are N; and one of the N is connected to the pyrimidine ring shown in formula I;
n is 0;
L is $-O-(CR^{6a}R^{6b})_{n2}-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is

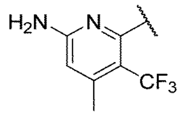

;

$R^{4a}$ and $R^{4b}$ are independently $-N(R^5)_2$ or halogen;
$R^5$ is independently hydrogen;
$R^{6a}$ and $R^{6b}$ are H;
n2 is 1.

In a certain embodiment, the nitrogen-containing heterocyclic compound of formula I is shown in formula 1-1;

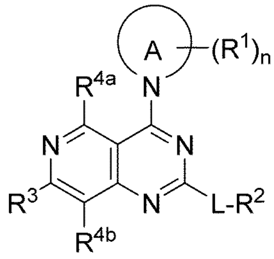

**I-1**

wherein ring A is 4- to 10-membered heterocycloalkyl; the number of heteroatoms of the 4- to 10-membered heterocycloalkyl is 2, and the heteroatoms are N; and one of the N is connected to the pyrimidine ring shown in formula 1;
n is 0;
L is $-O-(CR^{6a}R^{6b})_{n2}-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is

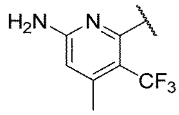

or

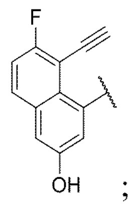

;

$R^{4a}$ and $R^{4b}$ are independently $-N(R^5)_2$ or halogen;
$R^5$ is independently hydrogen;
$R^{6a}$ and $R^{6b}$ are H;
n2 is 1.

In a certain embodiment, the nitrogen-containing heterocyclic compound of formula I is shown in formula 1-2';

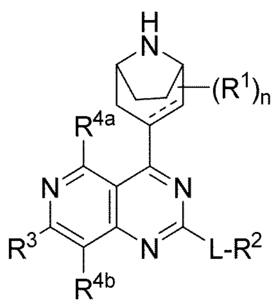

**I-2'**

wherein $\overline{---}$ represents a single bond or a double bond.

In a certain embodiment, the nitrogen-containing heterocyclic compound of formula I is shown in formula I-3;

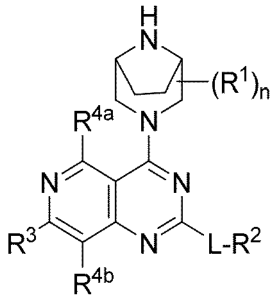

**I-3** ,

wherein $R^{4a}$ is halogen or $-N(R^5)_2$, and $R^5$ is independently hydrogen.

In a certain embodiment, the 3- to 7-membered cycloalkyl may be cyclopropyl, cyclobutyl, cyclopentyl, cyclohexyl, or cycloheptyl.

In a certain embodiment, the $C_1$-$C_6$ alkyl may be methyl, ethyl, n-propyl, isopropyl, n-butyl, isobutyl, sec-butyl, or tert-butyl.

In a certain embodiment, the $C_1$-$C_4$ alkyl may be methyl, ethyl, *n*-propyl, isopropyl, *n*-butyl, isobutyl, *sec*-butyl, or *tert*-butyl.

In a certain embodiment, the halogen or "halo" is fluorine, chlorine, bromine, or iodine; for example, fluorine or chlorine.

In a certain embodiment, the $C_2$-$C_6$ alkenyl is vinyl,

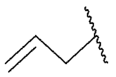 , 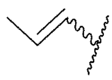 ,

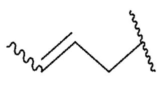 , 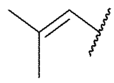 , or 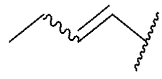 .

In a certain embodiment, the $C_2$-$C_4$ alkenyl is vinyl,

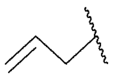 , 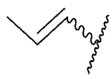 ,

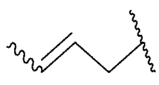 , 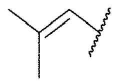 , or 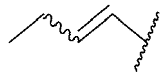 .

In a certain embodiment, the $C_2$-$C_6$ alkynyl is ethynyl or

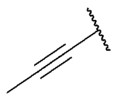 .

In a certain embodiment, the $C_2$-$C_4$ alkynyl is ethynyl or

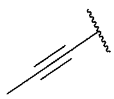 .

In a certain embodiment, the $C_6$-$C_{10}$ aryl is phenyl or naphthyl.

In a certain embodiment, the $C_1$-$C_6$ alkyl substituted by one or more than one halogen or halo-$C_1$-$C_6$ alkyl is $-CF_3$.

In a certain embodiment, the $C_1$-$C_4$ alkyl substituted by one or more than one halogen or halo-$C_1$-$C_4$ alkyl is $-CF_3$.

In a certain embodiment, the 4- to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

the 5- to 7-membered monocyclic heterocycloalkyl may be

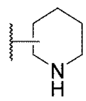

(for example,

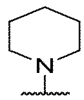

),

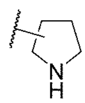

(for example,

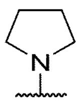 or 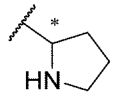,

for another example,

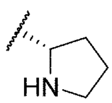

),

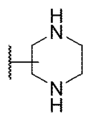

(for example,

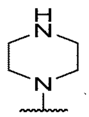

), or

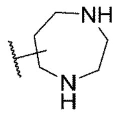

(for example,

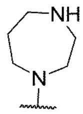

); "*" means that when a carbon atom with "*" is a chiral carbon atom, it is in an R configuration, an S configuration, or a mixture thereof;

the 6- to 8-membered fused heterocycloalkyl may be

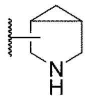

(for example,

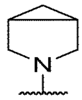

) or

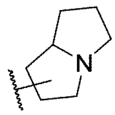

(for example,

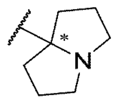

,

for another example,

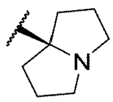

); "*" means that when a carbon atom with "*" is a chiral carbon atom, it is in an R configuration, an S configuration, or a mixture thereof;

the 6- to 8-membered bridged heterocycloalkyl may be

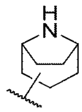

(for example,

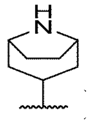

),

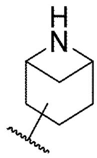

(for example,

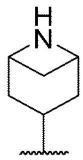

),

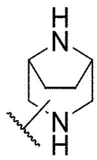

(for example,

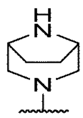

), or

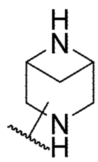

(for example,

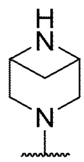

);

the 7- to 10-membered spiro heterocycloalkyl may be

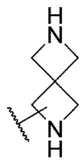

(for example,

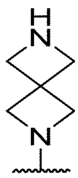

),

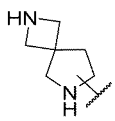

(for example,

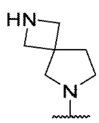

),

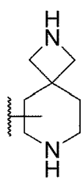

(for example,

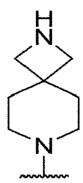

),

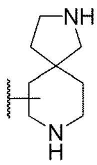

(for example,

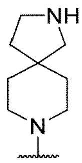

),

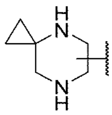

(for example,

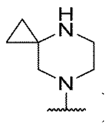

),

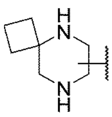

(for example,

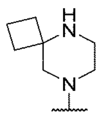

), or

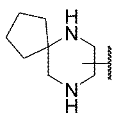

(for example,

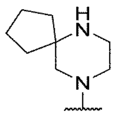

).

In a certain embodiment, the 4- to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

the 5- to 7-membered monocyclic heterocycloalkyl may be

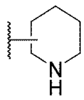

(for example,

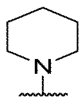

),

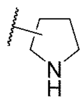

(for example,

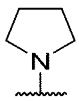

or

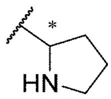

,

for another example,

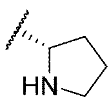

),

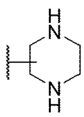

(for example,

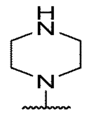

), or

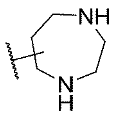

(for example,

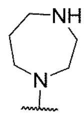

); "*" means that when a carbon atom with "*" is a chiral carbon atom, it is in an R configuration, an S configuration, or a mixture thereof;

the 6- to 8-membered fused heterocycloalkyl may be

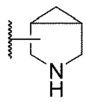

(for example,

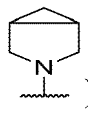

) or

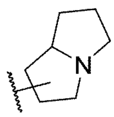

(for example,

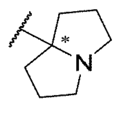

, for another example,

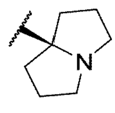

); "*" means that when a carbon atom with "*" is a chiral carbon atom, it is in an R configuration, an S configuration,

or a mixture thereof;

the 6- to 8-membered bridged heterocycloalkyl may be

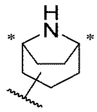

(for example,

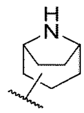,

for another example,

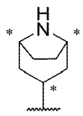 or 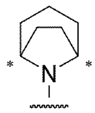,

for yet another example,

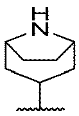, 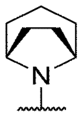, or 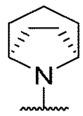

),

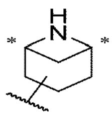

(for example,

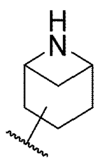,

for another example,

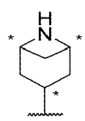,

for yet another example,

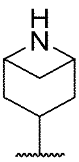

),

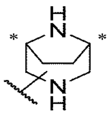

(for example,

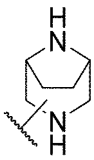

,

for another example,

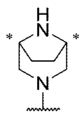

,

for yet another example,

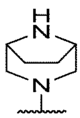

), or

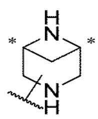

(for example,

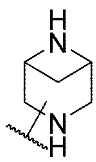

,

for another example,

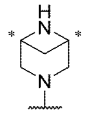

, for yet another example,

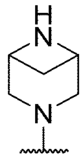

), "*" means that when a carbon atom with "*" is a chiral carbon atom, it is in an R configuration, an S configuration, or a mixture thereof;

the 7- to 10-membered spiro heterocycloalkyl may be

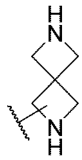

(for example,

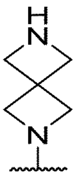

),

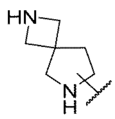

(for example,

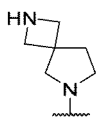

),

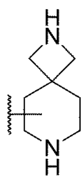

(for example,

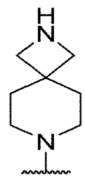

),

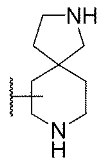

(for example,

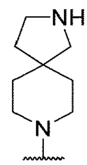

),

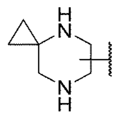

(for example,

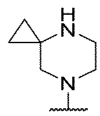

),

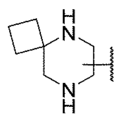

(for example,

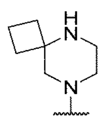

), or

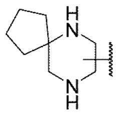

(for example,

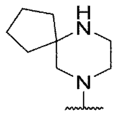

).

In a certain embodiment, the 4- to 10-membered heterocycloalkenyl is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl;

the 6- to 8-membered bridged heterocycloalkenyl may be

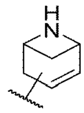

(for example,

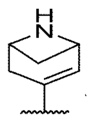

) or

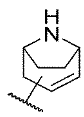

(for example,

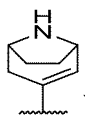

).

In a certain embodiment, the 4- to 10-membered heterocycloalkenyl is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl;

the 6- to 8-membered bridged heterocycloalkenyl may be

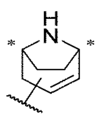

or

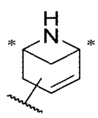

,

for example,

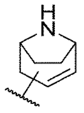 or 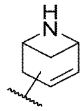 ,

for another example,

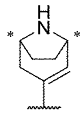 or 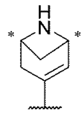 ,

for yet another example,

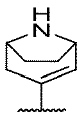 or 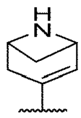 ;

"*" means that when a carbon atom with "*" is a chiral carbon atom, it is in an R configuration, an S configuration, or a mixture thereof.

In a certain embodiment, the 5- to 14-membered heteroaryl is 5- to 10-membered heteroaryl, for example, pyridyl, pyrimidinyl, quinolinyl, quinazolinyl, or indazolyl, and for another example,

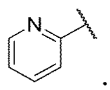 .

In a certain embodiment, the nitrogen-containing heterocyclic compound of formula I is not any one of the following compounds:

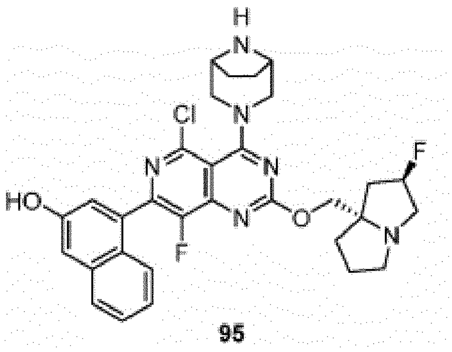

95 ,

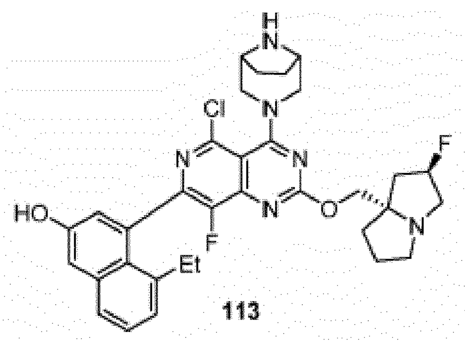

113

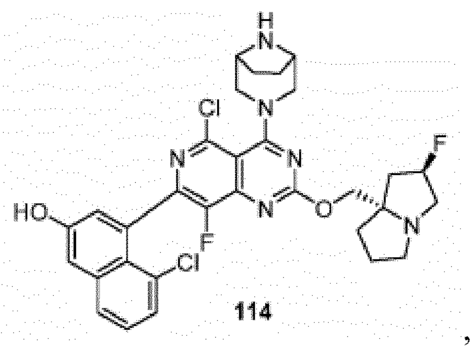

114 ,

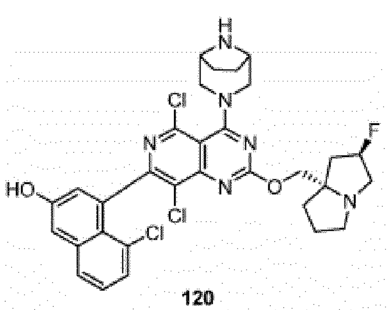
120
,
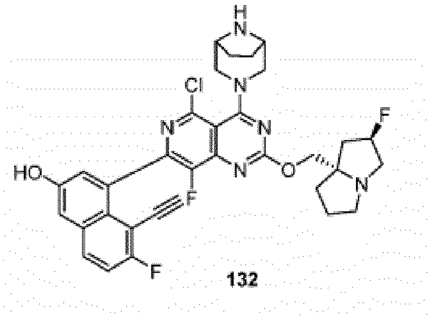
132
,

, and

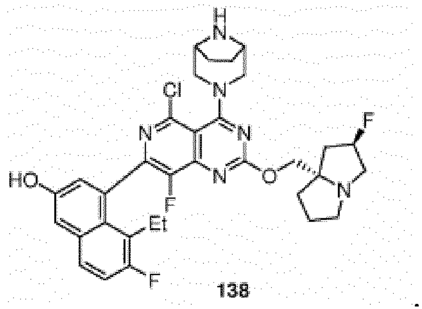
138
.

In a certain embodiment, the nitrogen-containing heterocyclic compound of formula I is not any one of the following compounds:

:
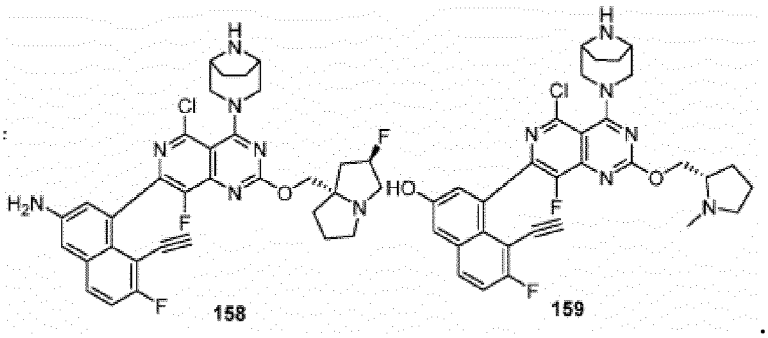
158
159
.

In a certain embodiment, the nitrogen-containing heterocyclic compound of formula I is not any one of the following compounds:

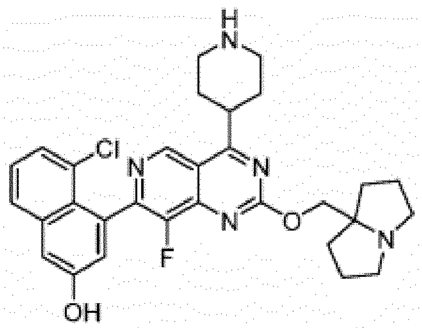
55
,
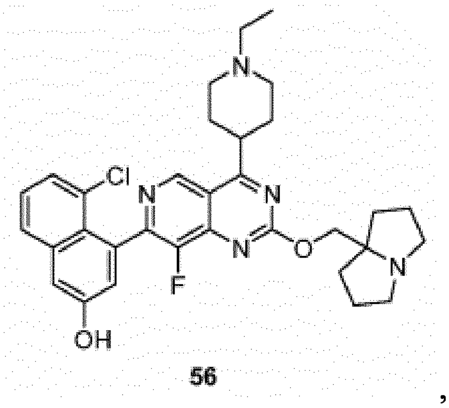
56
,

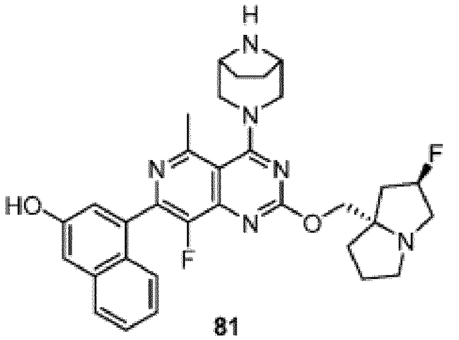

81 ,

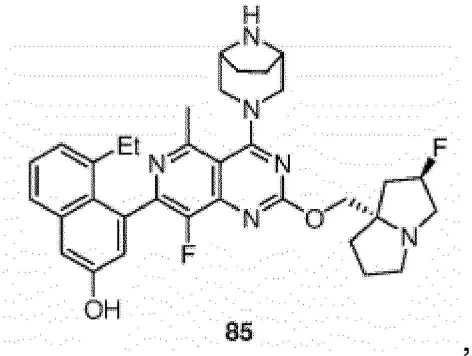

85 ,

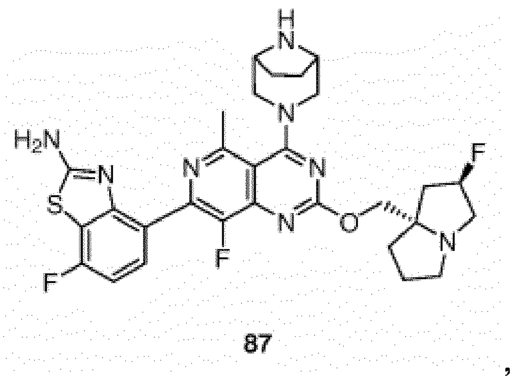

87 ,

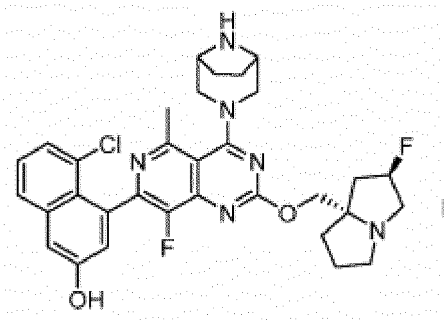

89 ,

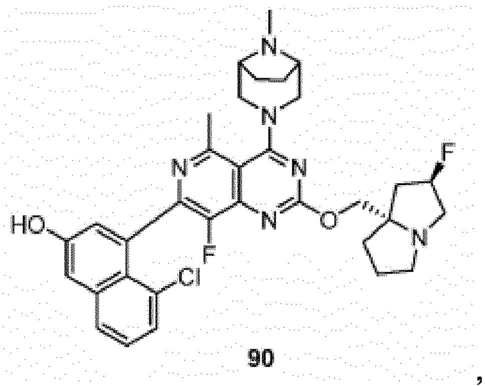

90 ,

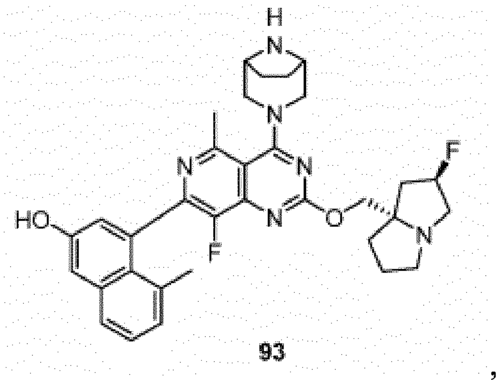

93 ,

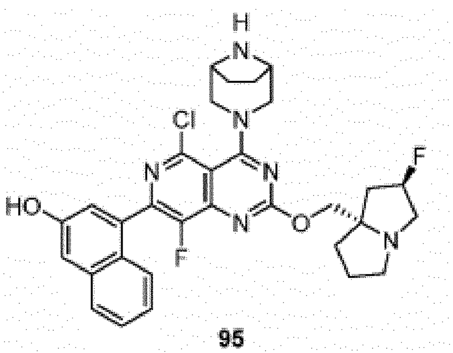

95 ,

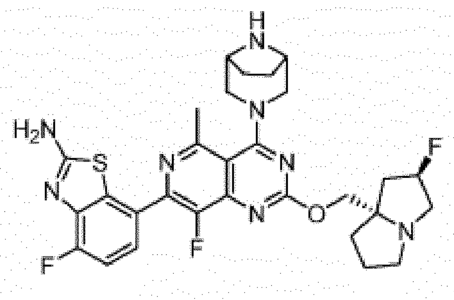

99 ,

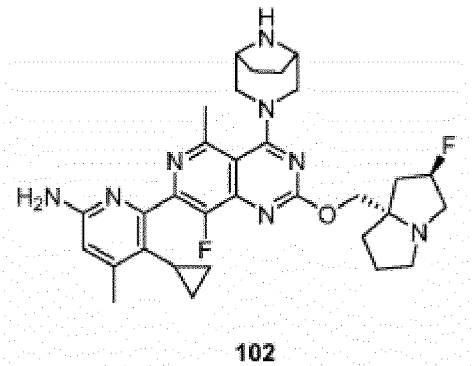

102

,

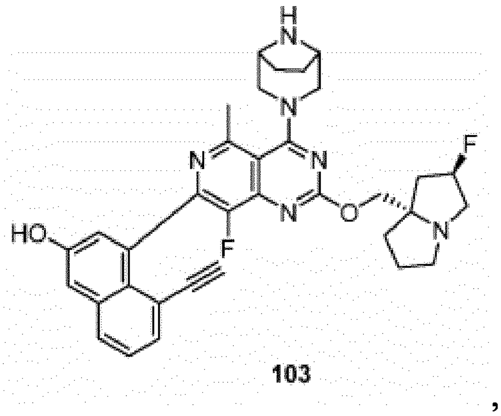

103

,

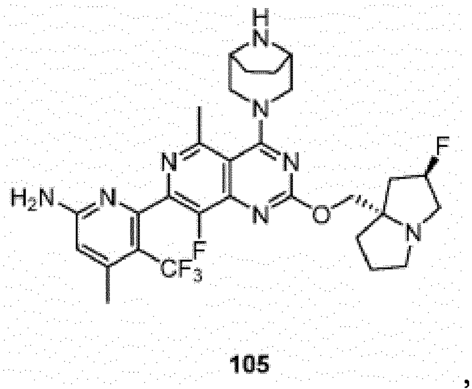

105

,

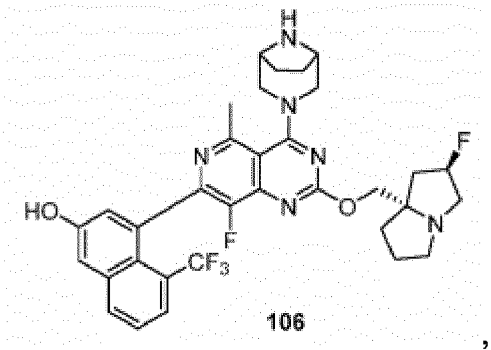

106

,

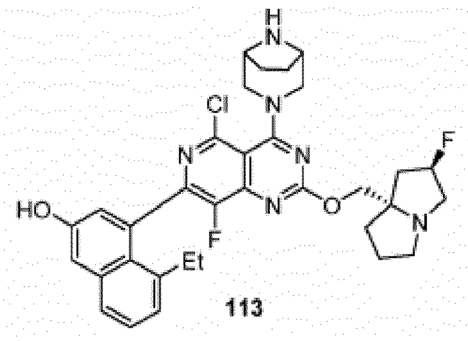

113

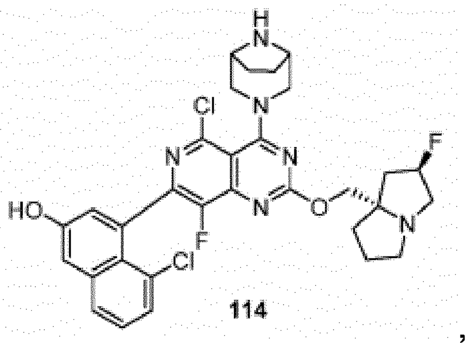

114

,

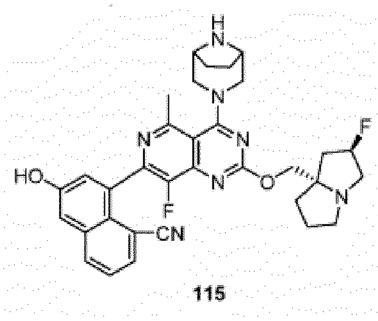

115

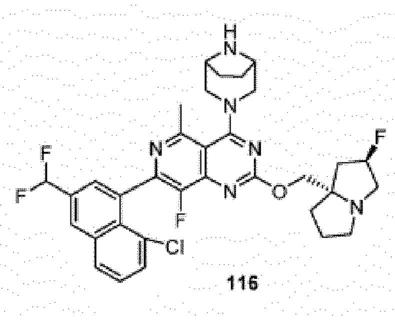

116

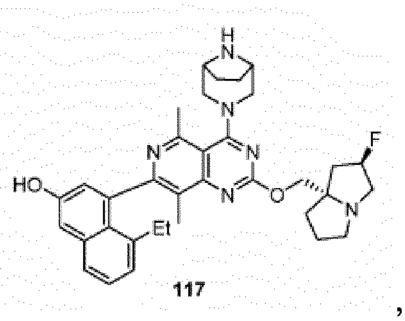

117

,

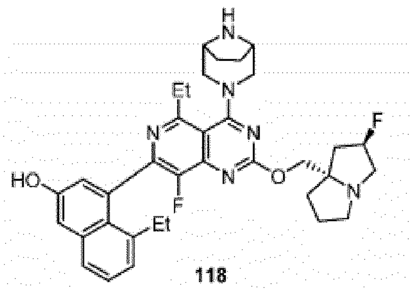
118

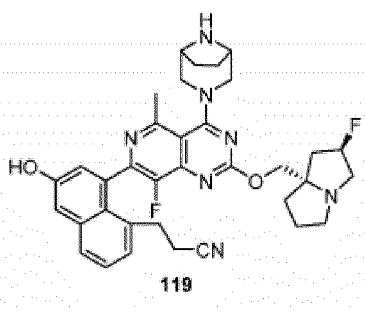
119

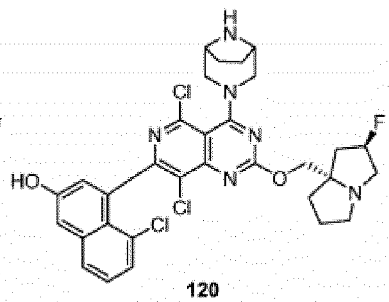
120
,

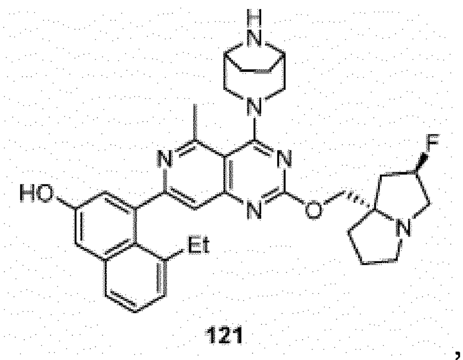
121
,

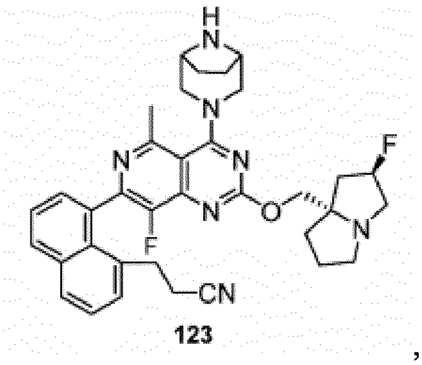
123
,

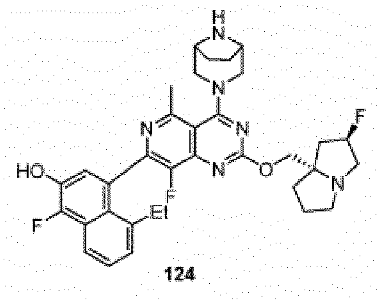
124

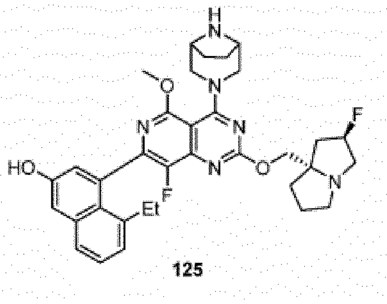
125

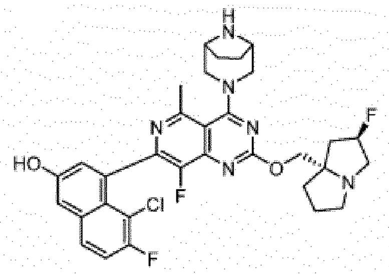
126
,

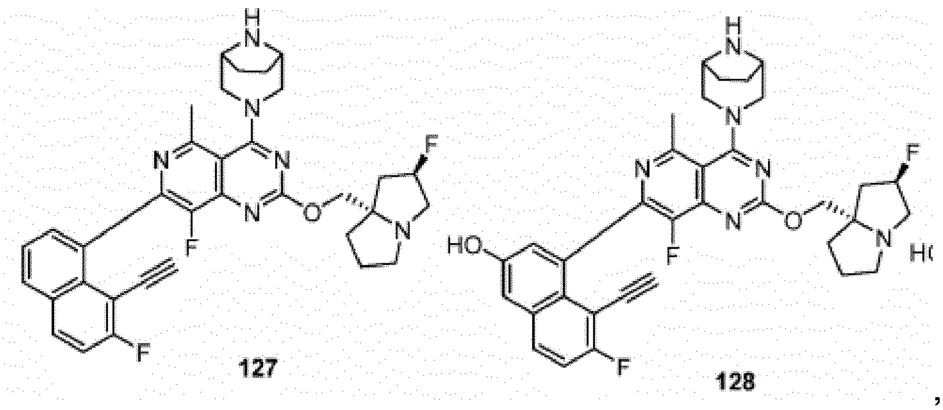
127

128
,

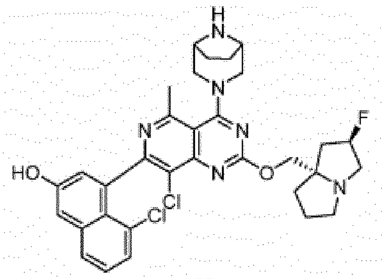
130
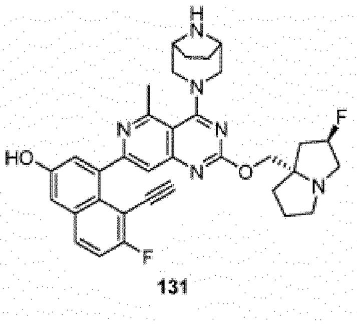
131
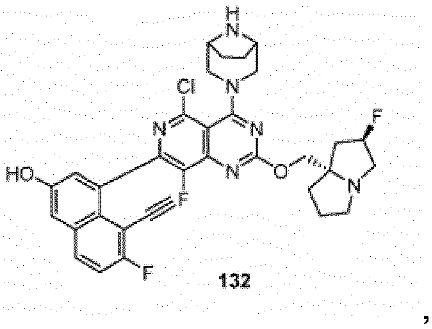
132
,

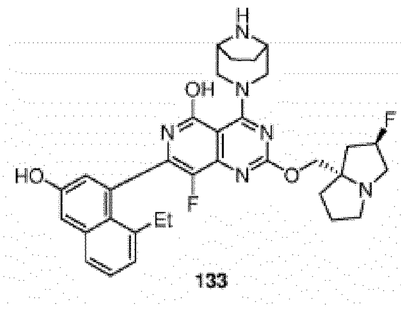
133
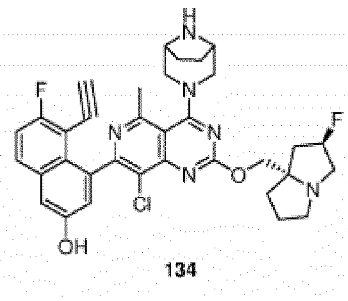
134
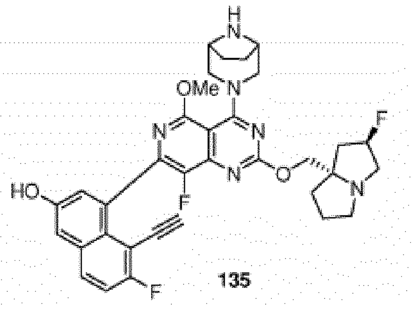
135

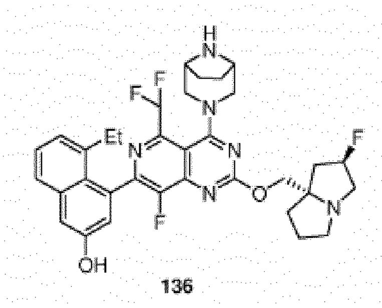
136
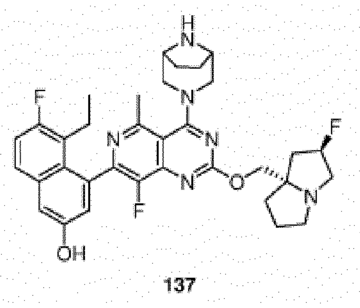
137
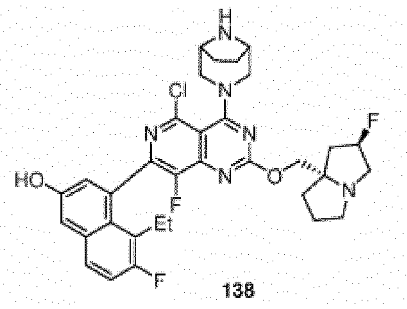
138
,

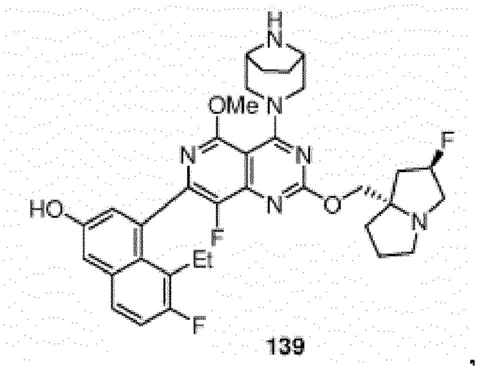
139
, and
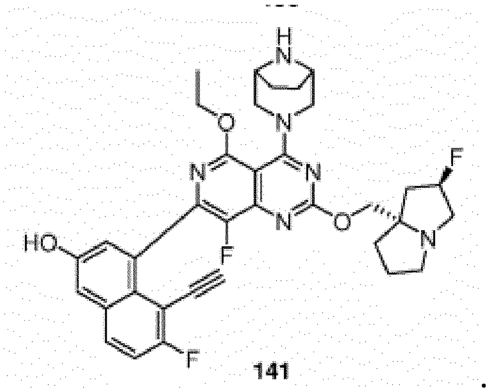
141
.

In a certain embodiment, the nitrogen-containing heterocyclic compound of formula I is not any one of the following compounds:

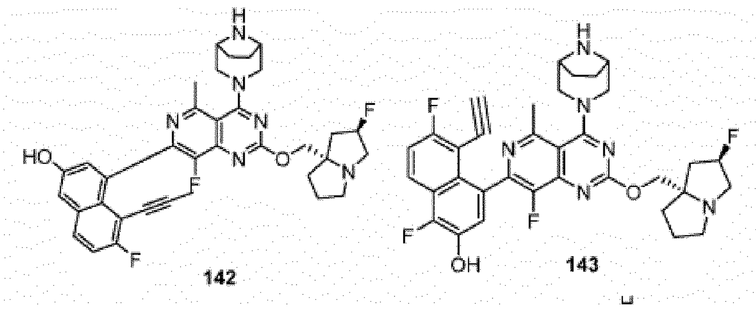

142 143

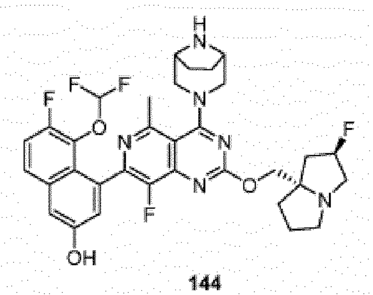

144 ,

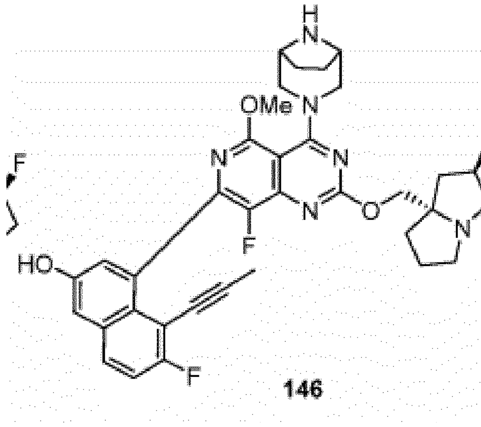

146

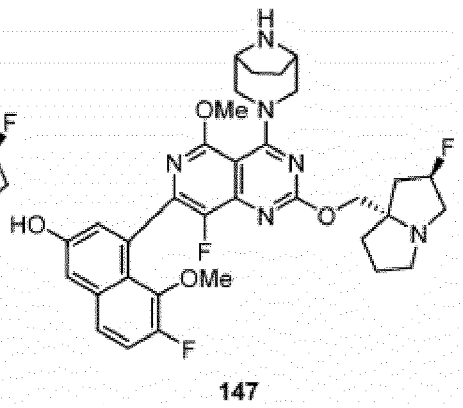

147 ,

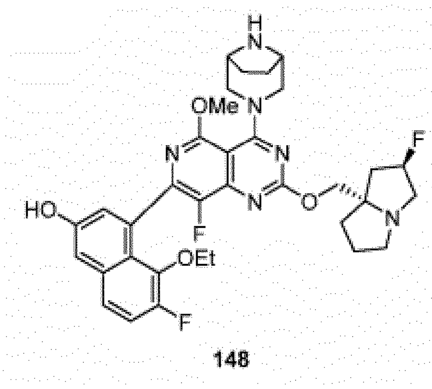

148

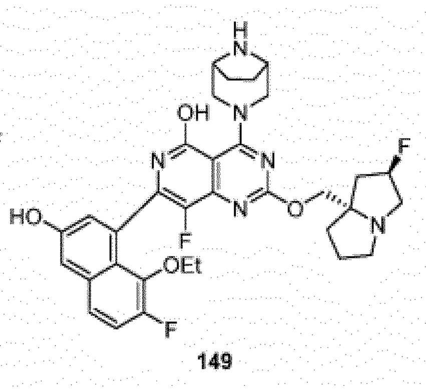

149

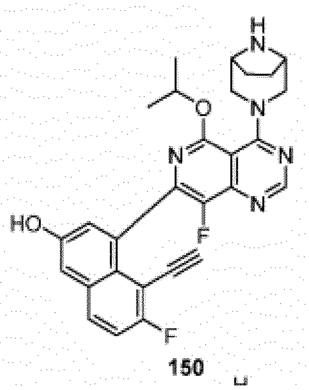

150 ,

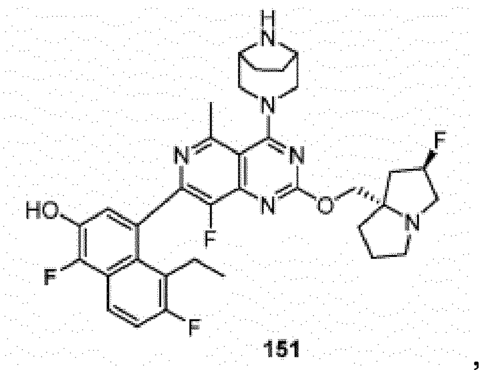

151 ,

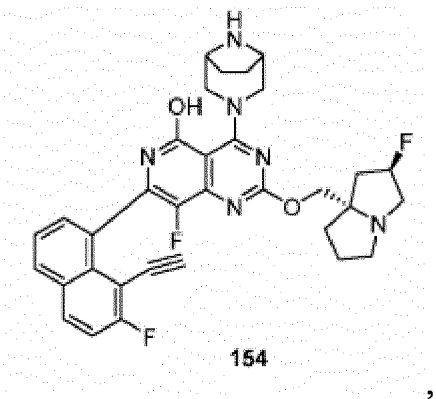

154 ,

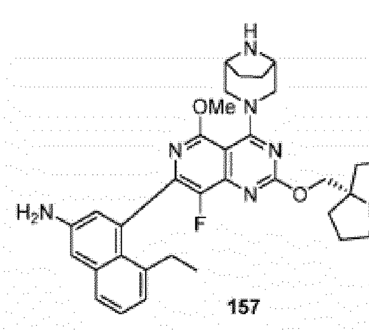

157

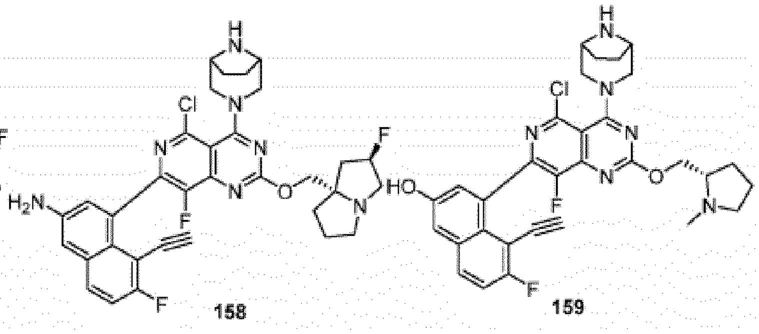

158 159

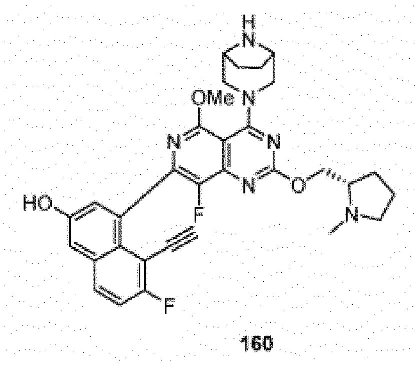

160

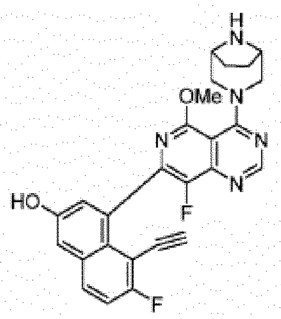

161

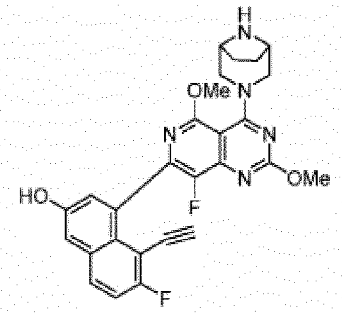

162

,

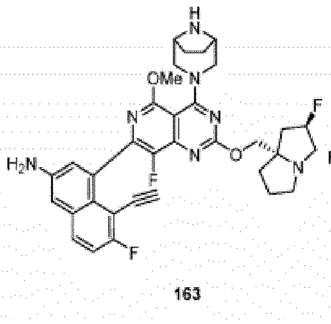

163

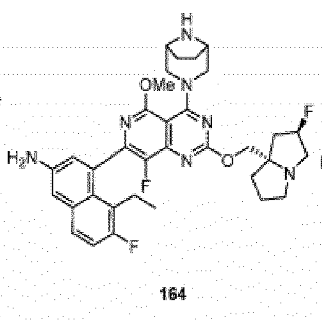

164

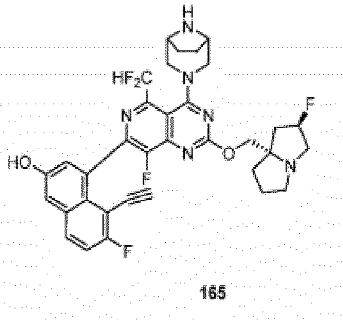

165

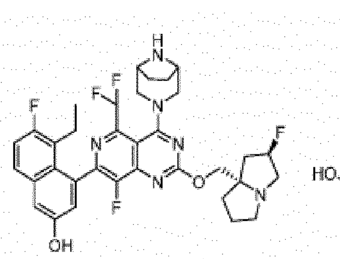

166

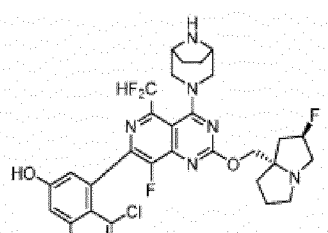

167

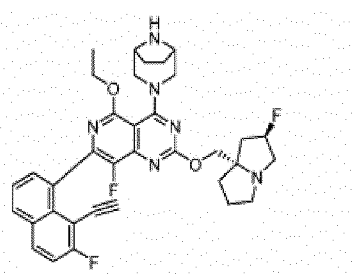

168

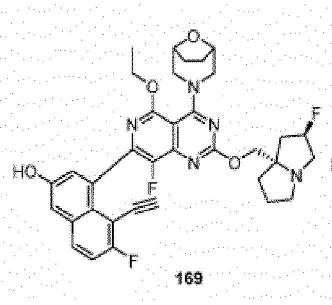

169

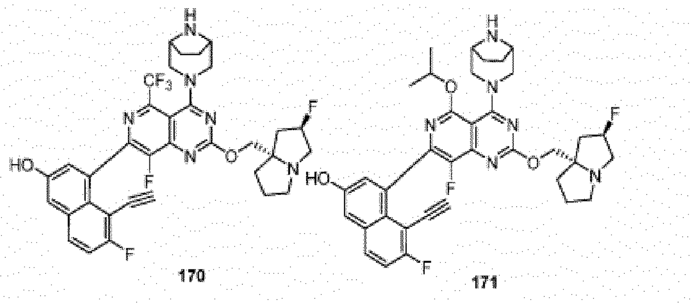

170 171

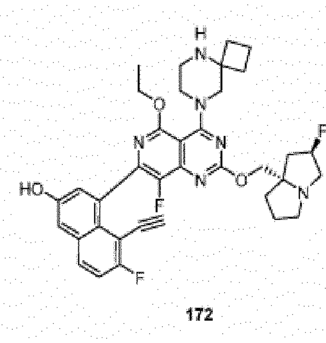

172

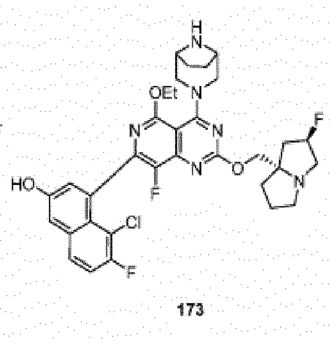

173

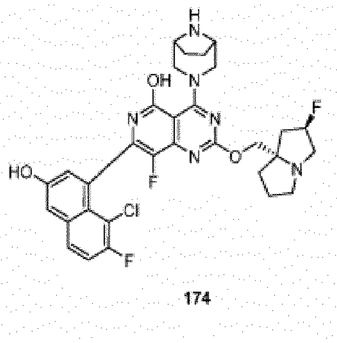

174

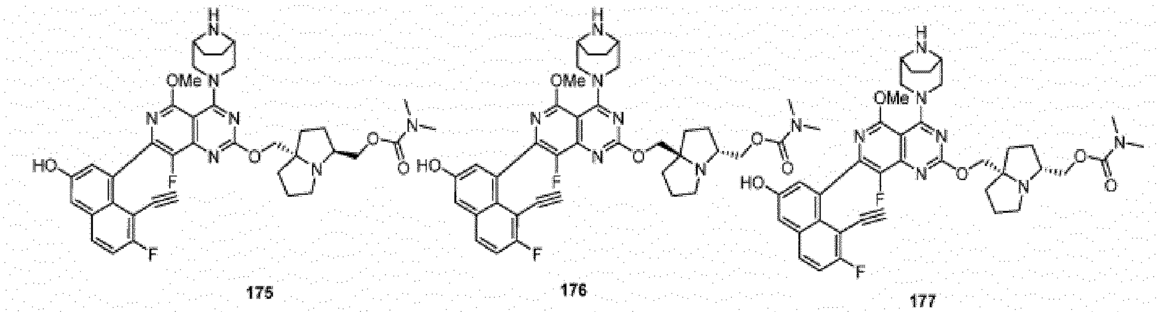

175 176 177

,

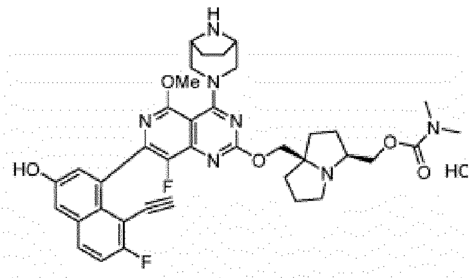

178

,

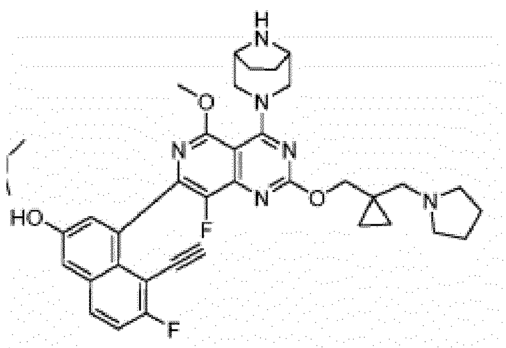

183

,

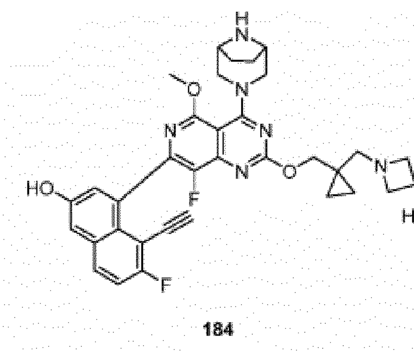

184

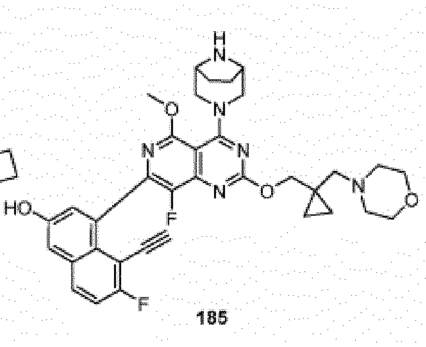

185

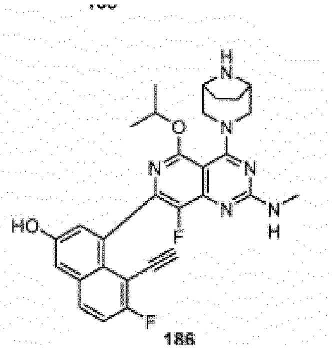

186

,

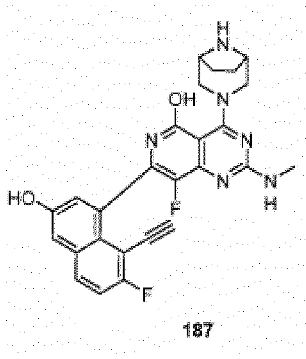

187

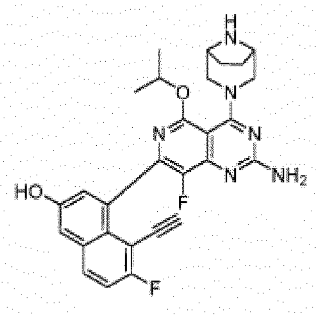

188

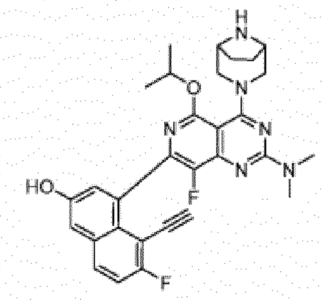

189

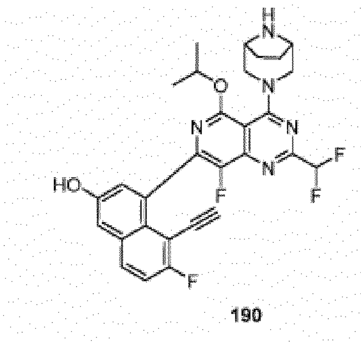

190

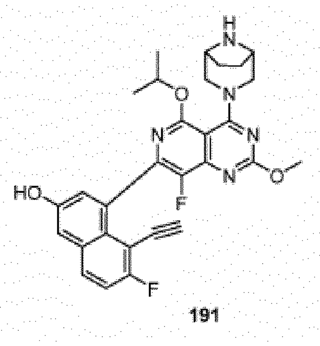

191

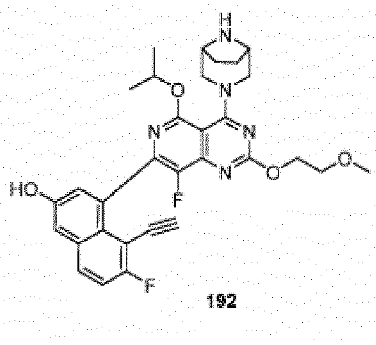

192

,

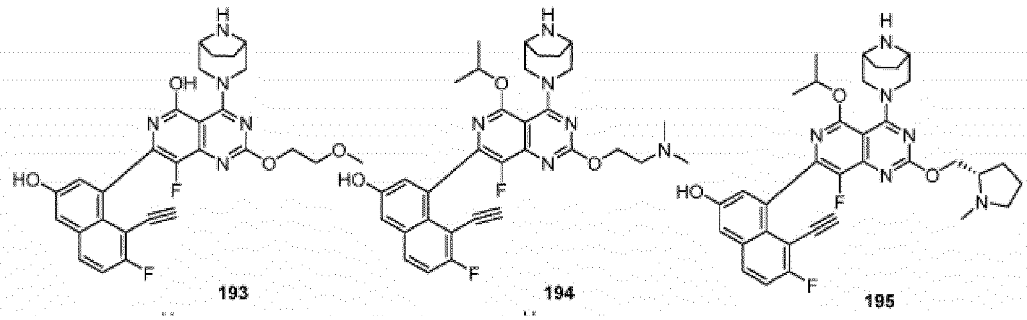

193

194

195

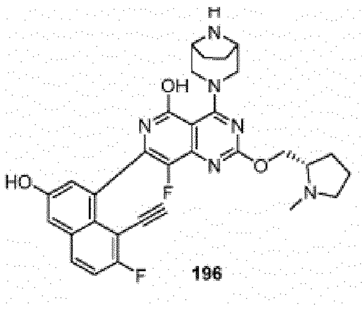

196

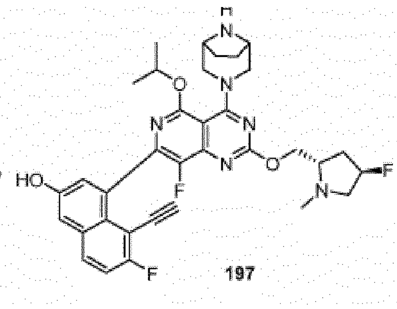

197

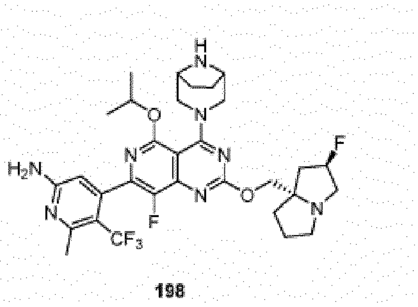

198

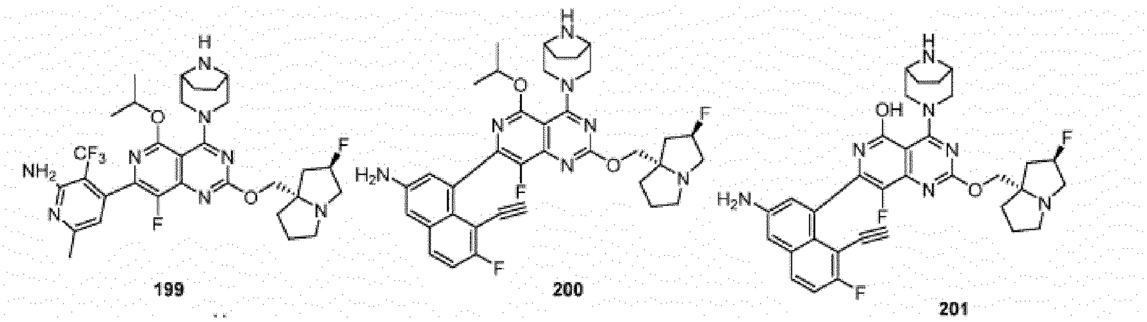

199 200 201

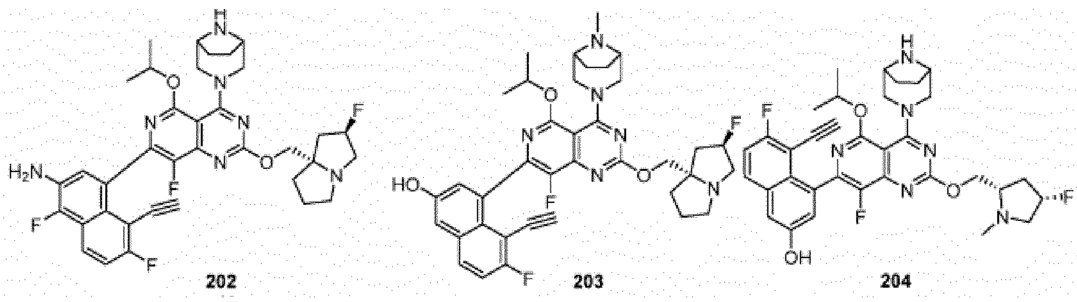

202 203 204

,

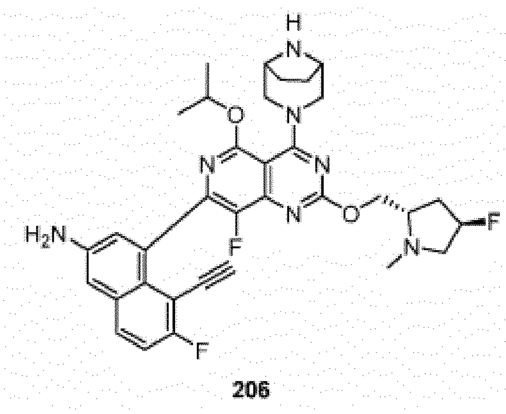

206

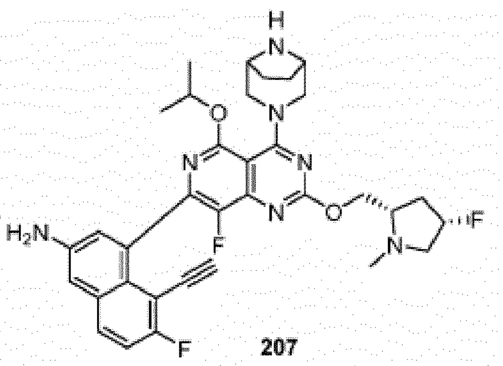

207

,

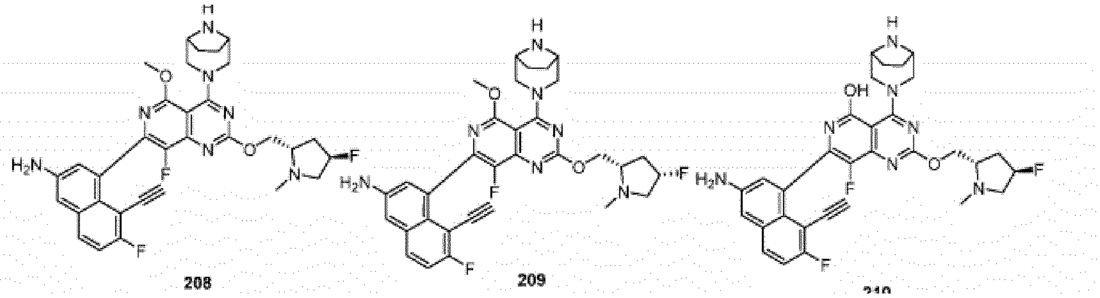

208 209 210

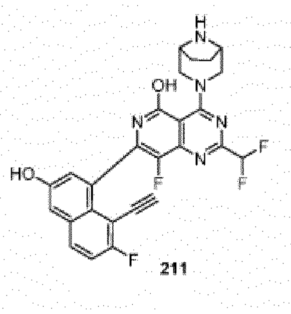
211

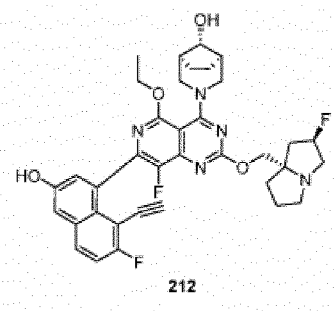
212

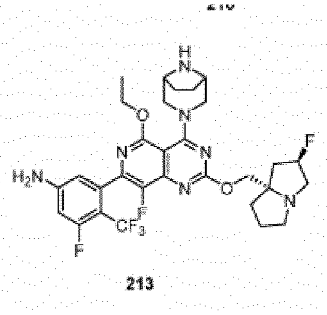
213

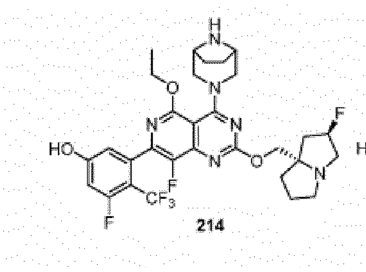
214

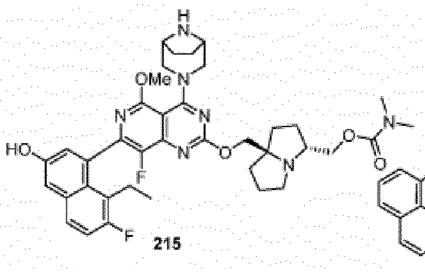
215

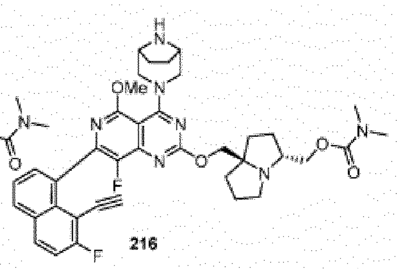
216

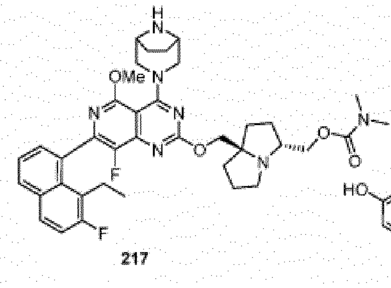
217

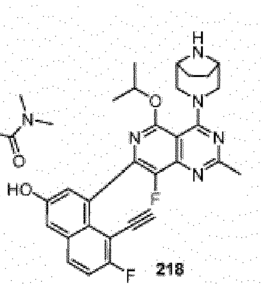
218

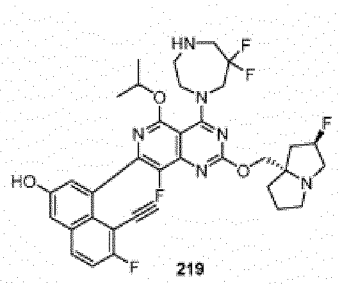
219

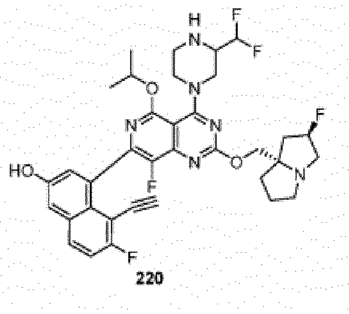
220

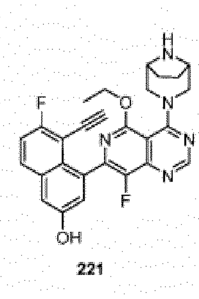
221

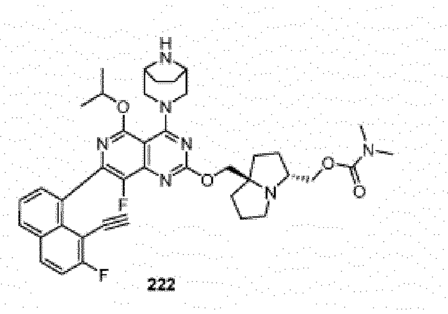
222

,

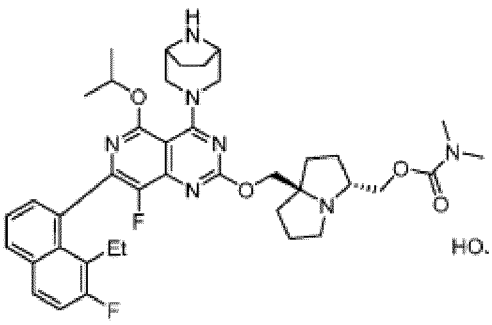
223

,

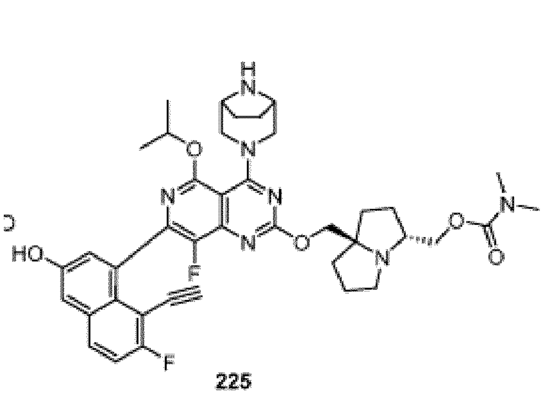
225

,

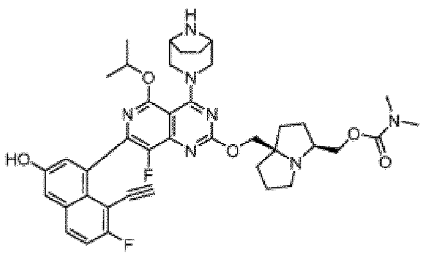

226

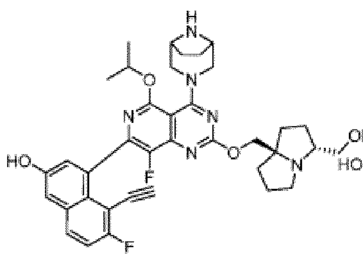

227

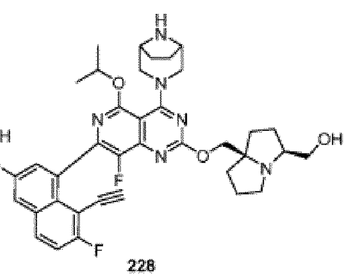

228

,

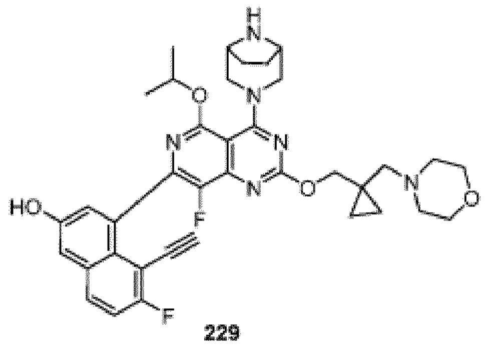

229

,

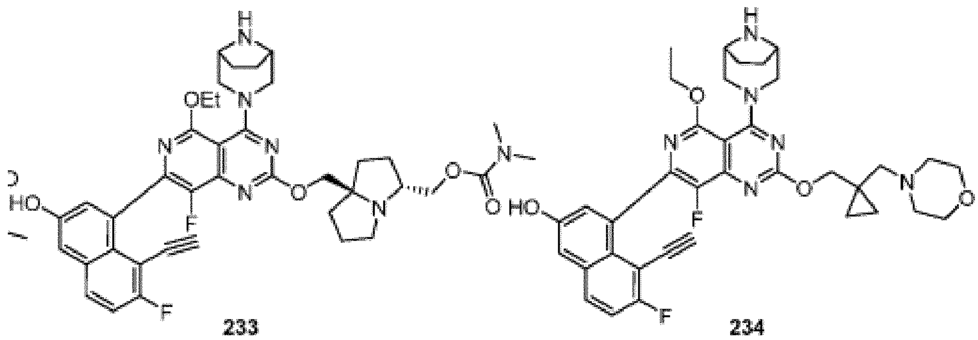

233

234

,

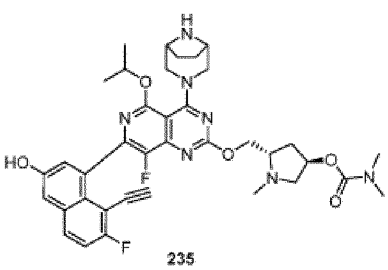

235

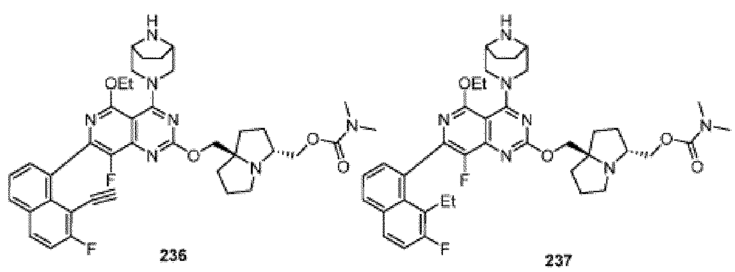

236

237

,

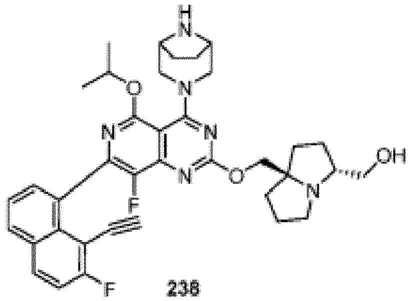
238
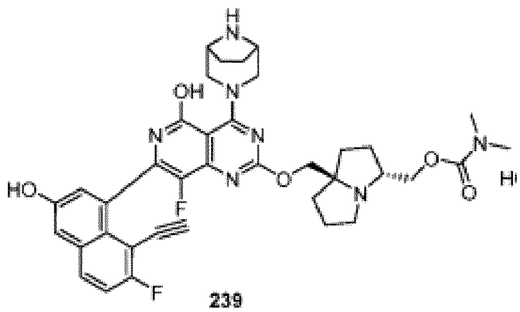
239
,

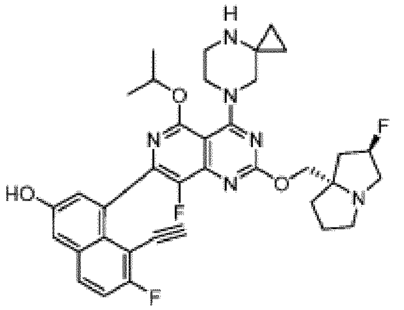
241
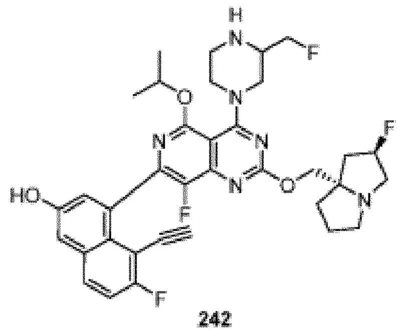
242
,

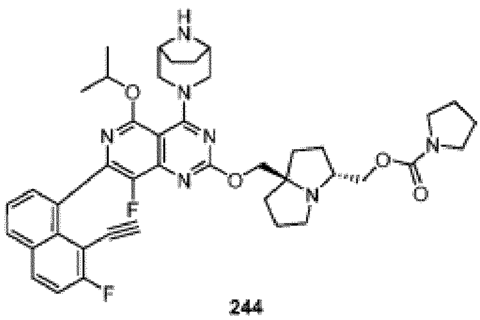
244
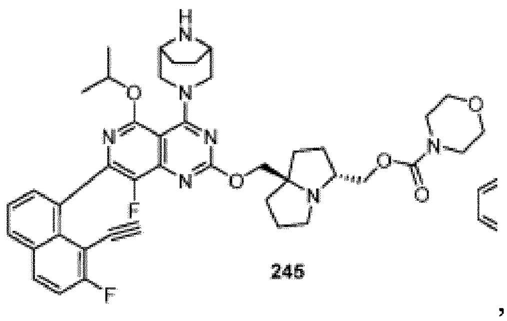
245
,

and

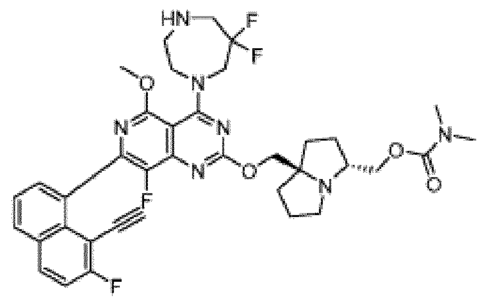
247
.

In a certain embodiment, the nitrogen-containing heterocyclic compound of formula I is not any one of the following compounds:

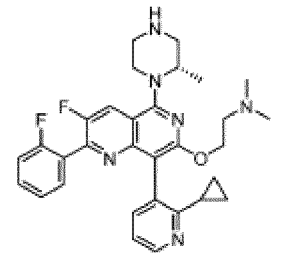

1

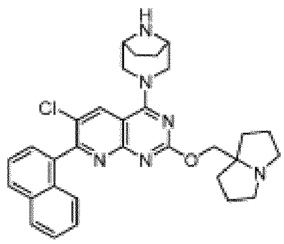

2

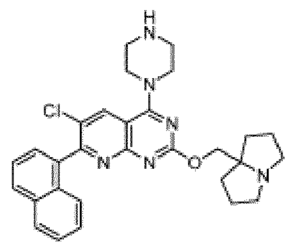

3

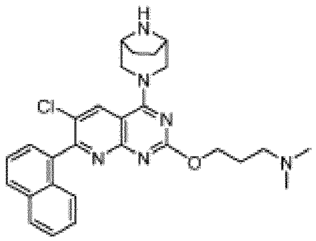

4

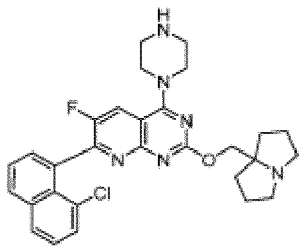

5

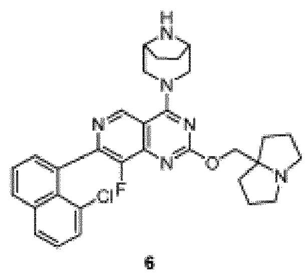

6

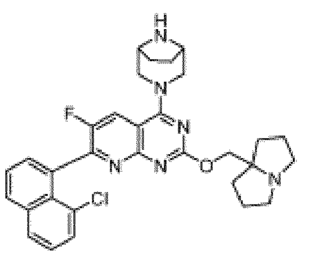

7

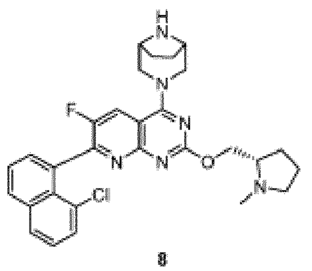

8

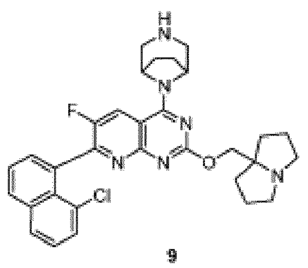

9

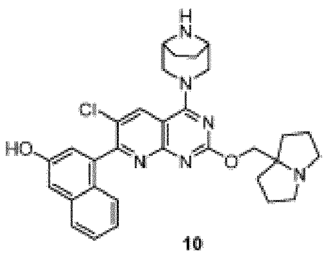

10

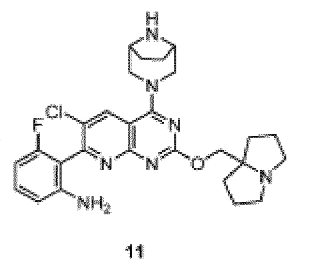

11

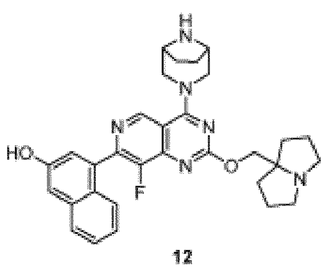

12

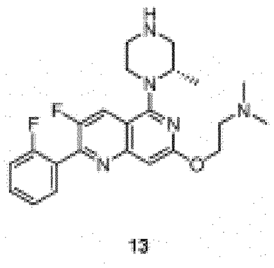

13

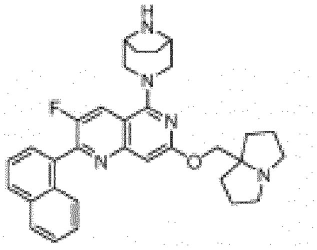

14

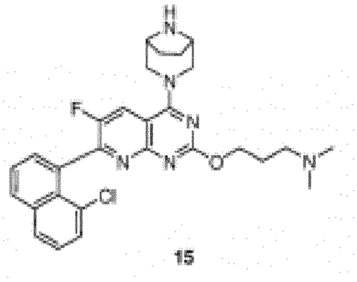

15

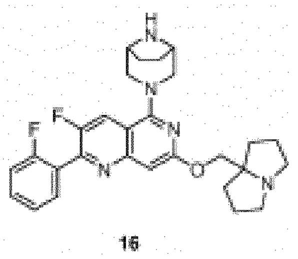

16

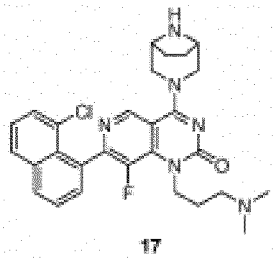

17

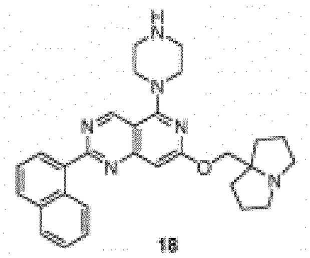

18

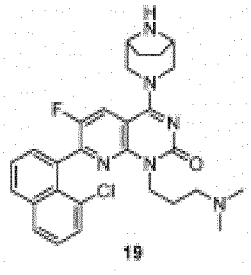

19

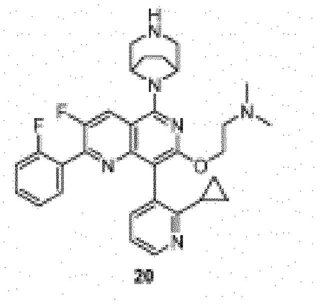

20

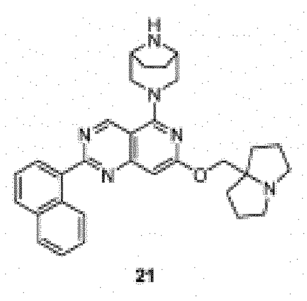

21

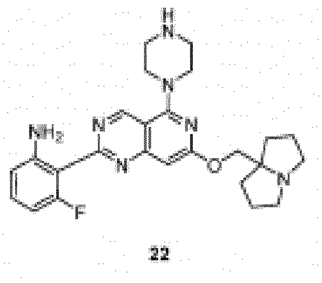

22

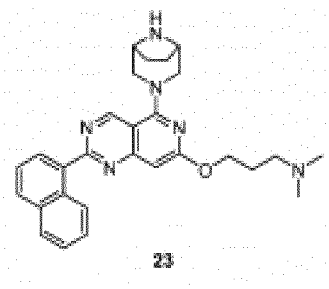

23

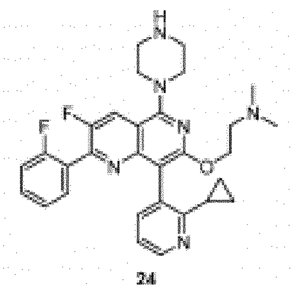

24

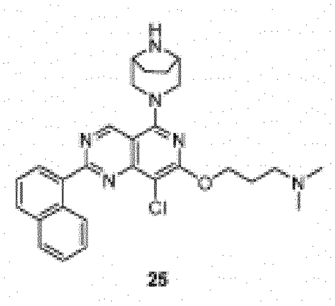

25

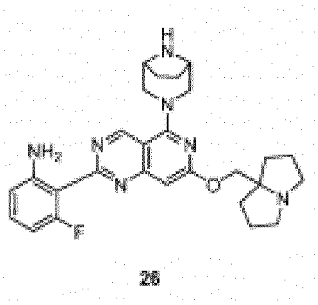

26

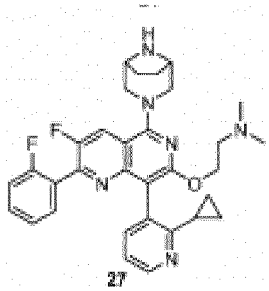

27

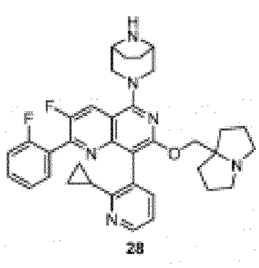

28

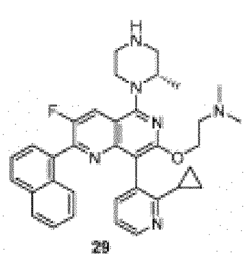

29

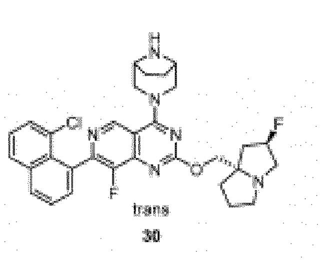

trans

30

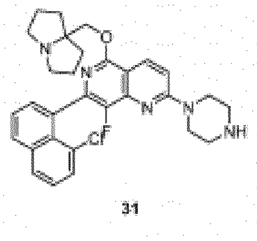
31

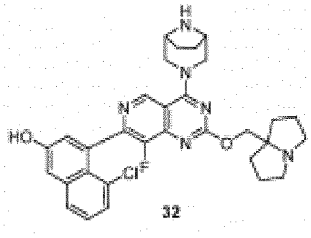
32

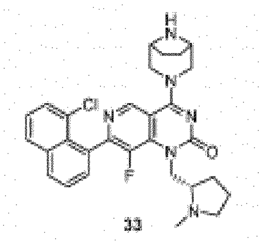
33

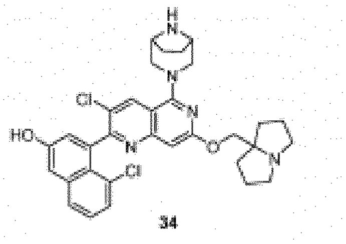
34

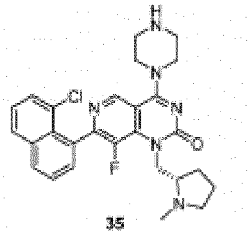
35

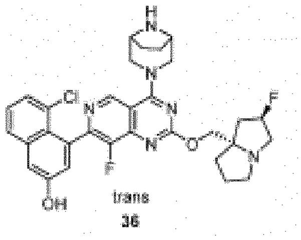
trans
36

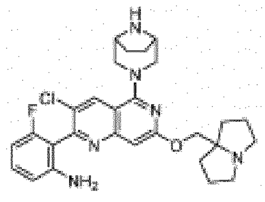
37

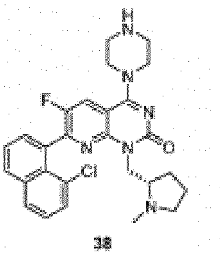
38

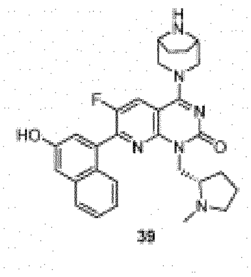
39

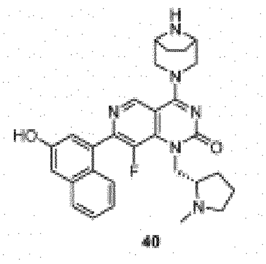
40

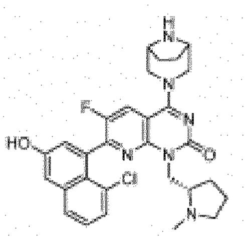
41

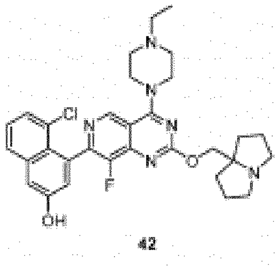
42

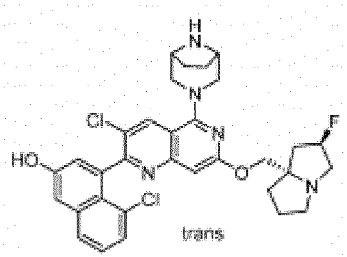
trans
43

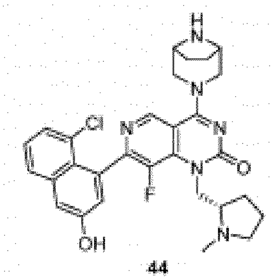
44

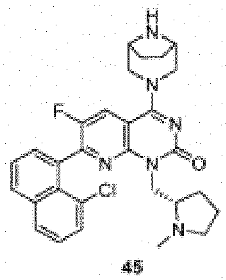
45

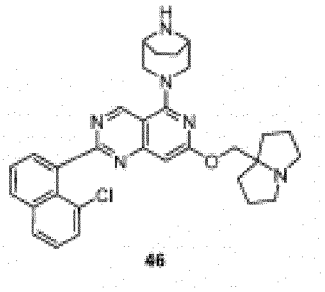
46

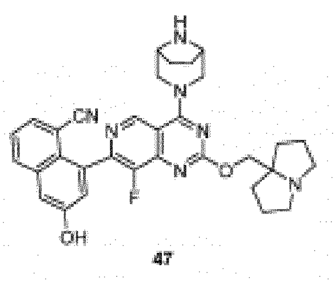
47

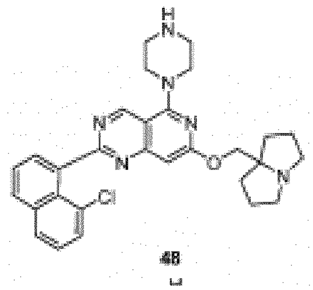
48

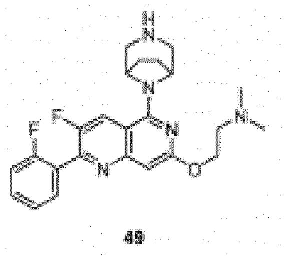
49

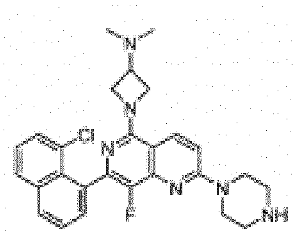
50

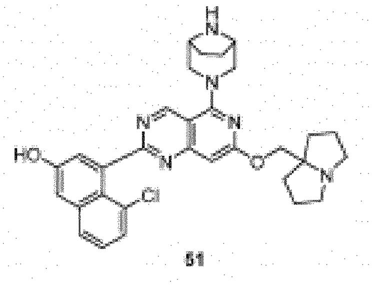
51

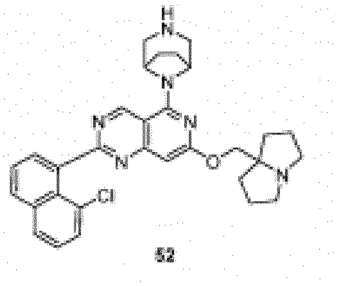
52

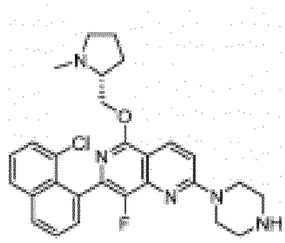
53

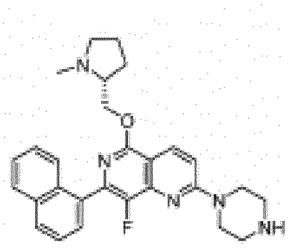
54

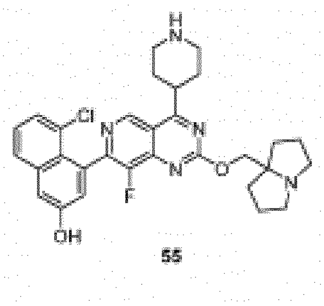
55

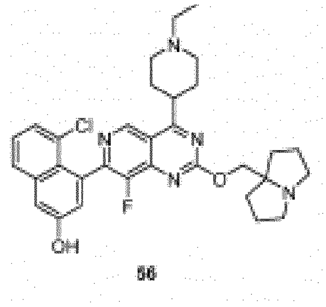
56

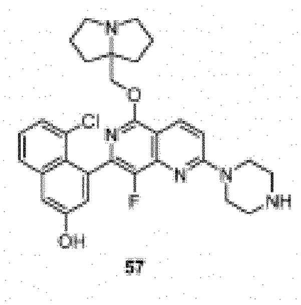
57

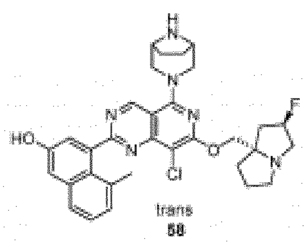
trans
58

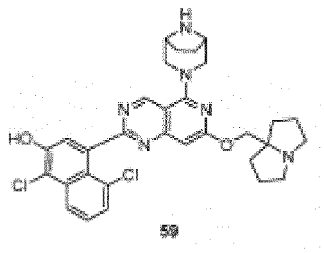
59

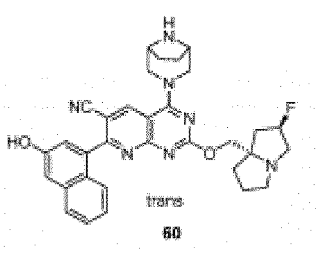
trans
60

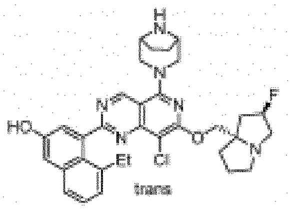
trans
61

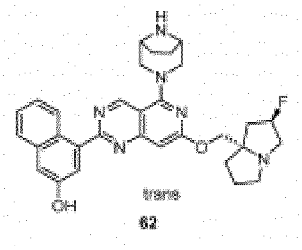
trans
62

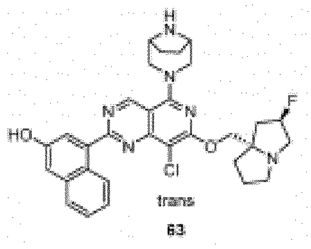
trans
63

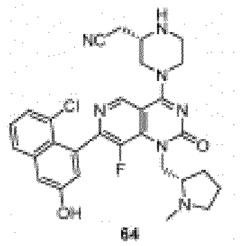
64

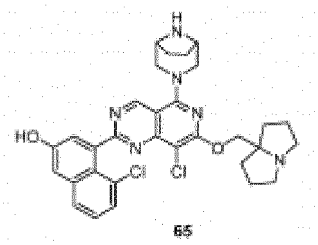
65

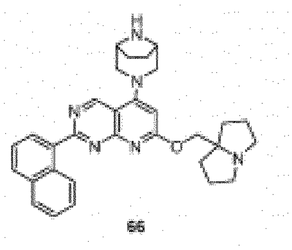
66

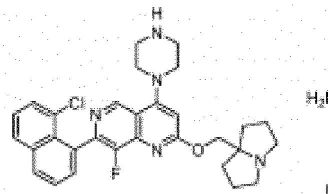
67

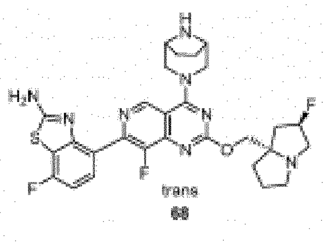
trans
68

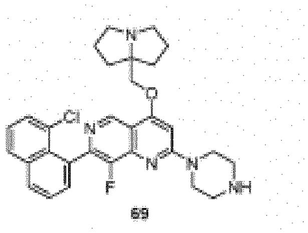
69

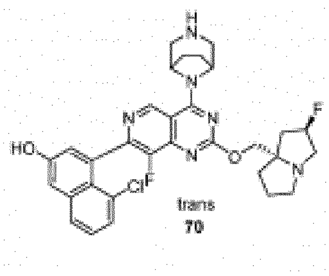
trans
70

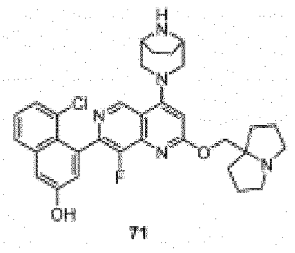
71

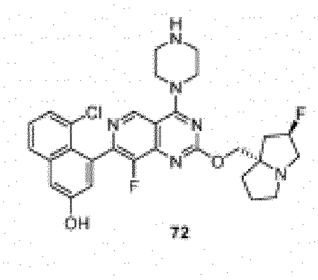
72

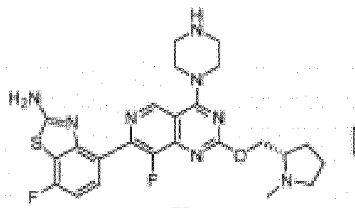
73

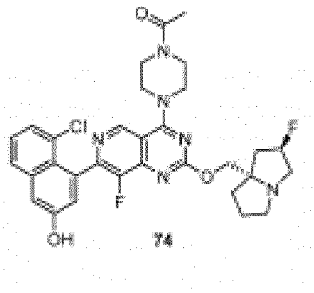
74

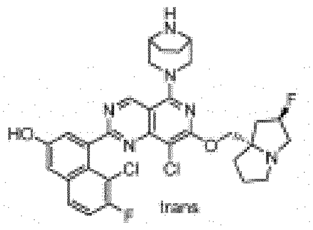
trans
75

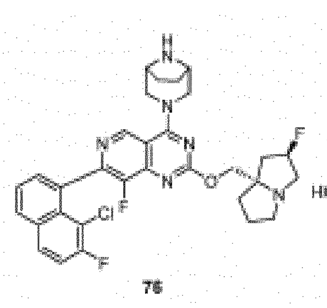
76

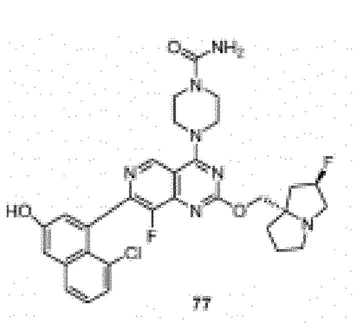
77

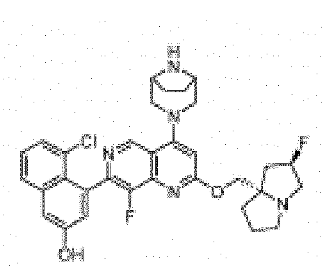
78

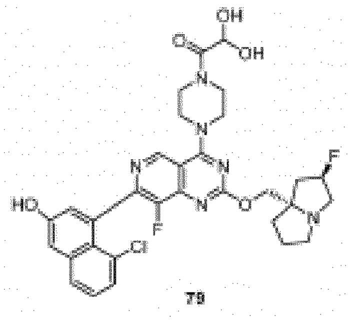

79

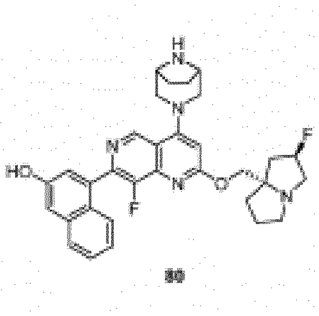

80

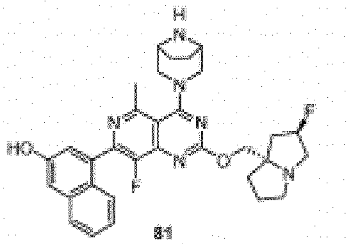

81

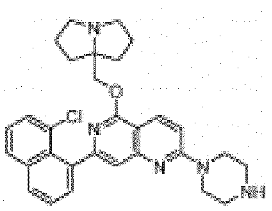

82

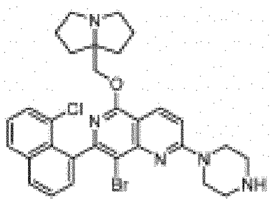

83

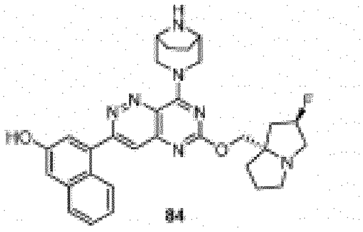

84

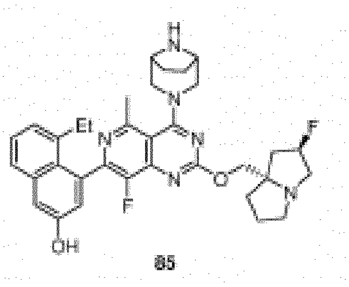

85

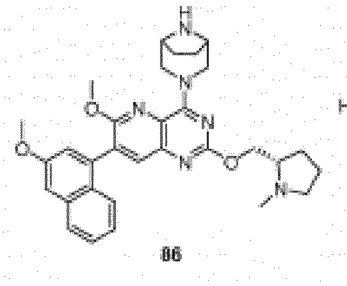

86

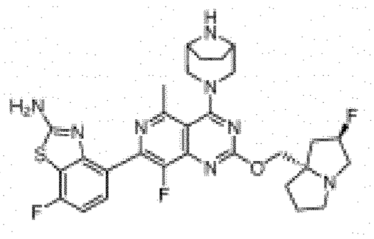

87

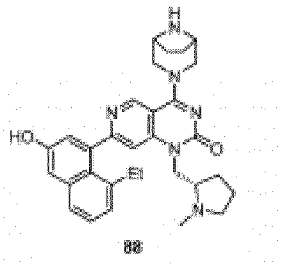

88

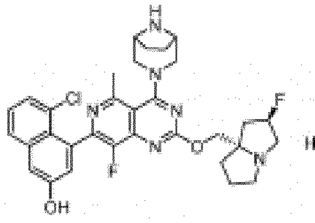

89

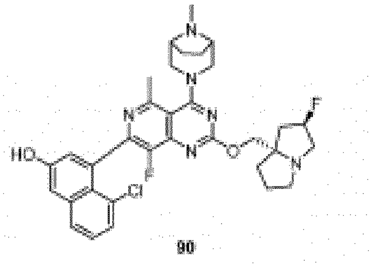

90

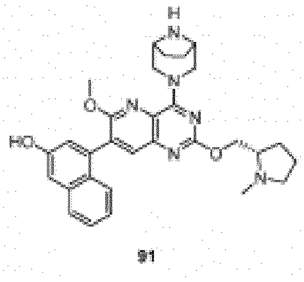

91

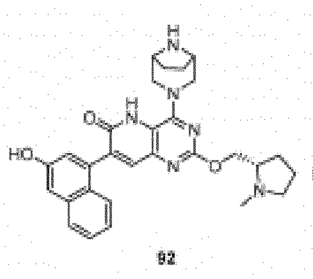

92

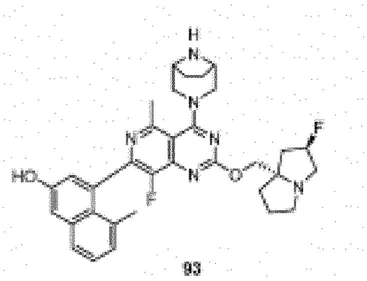

93

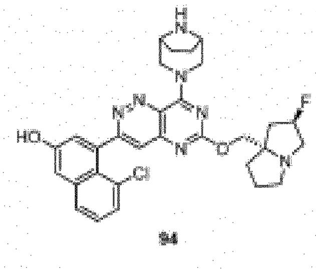

94

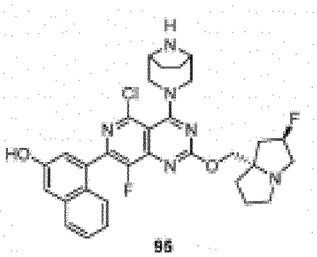

95

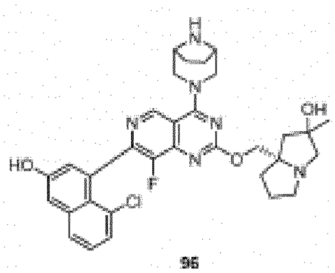

96

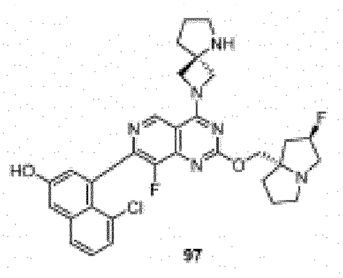

97

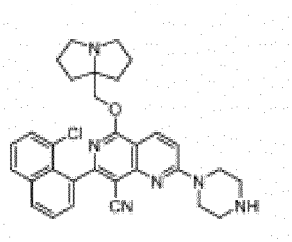

98

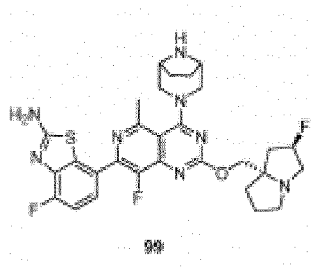

99

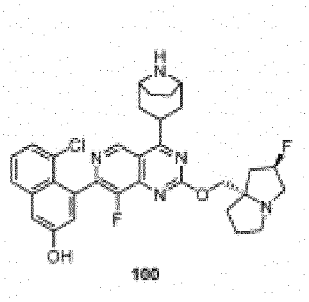

100

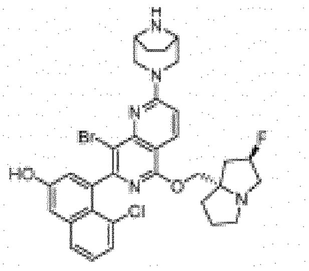

101

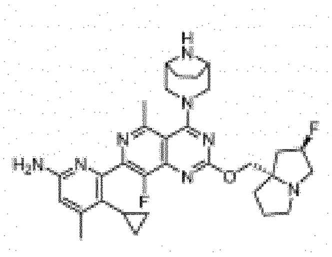

102

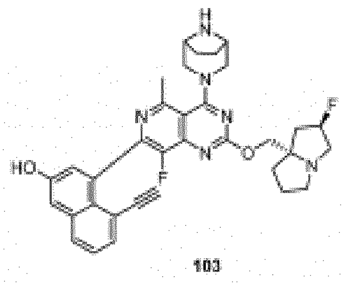

103

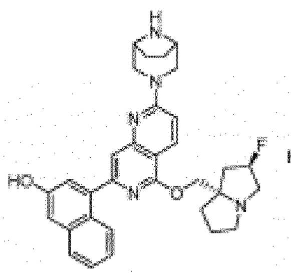

104

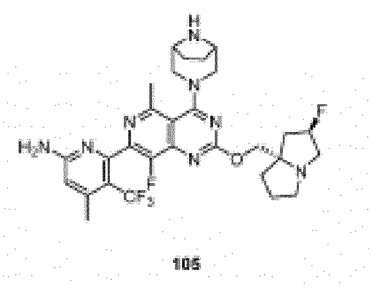

105

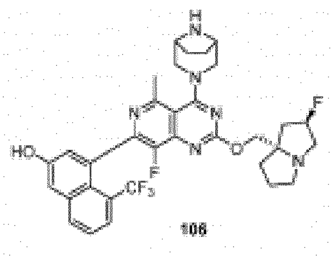

106

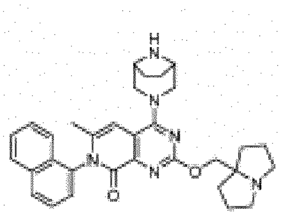

107

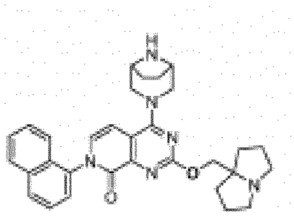

108

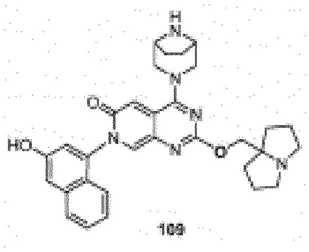
109

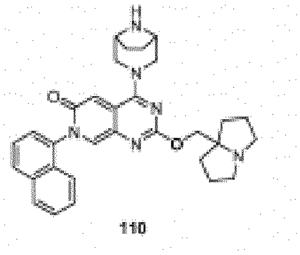
110

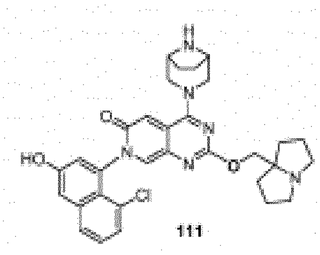
111

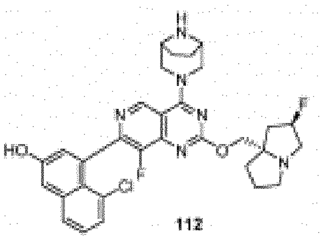
112

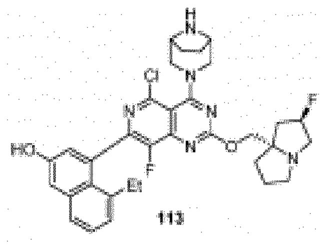
113

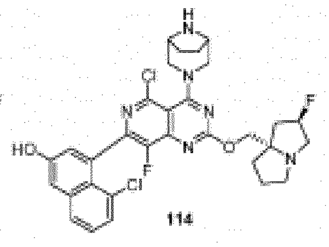
114

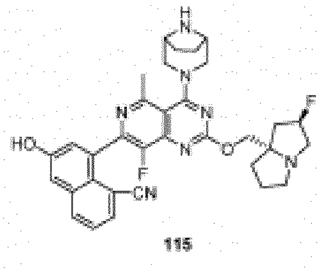
115

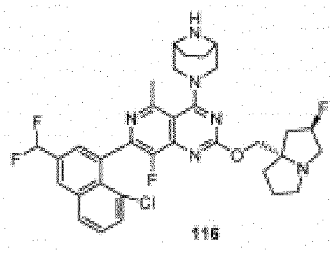
116

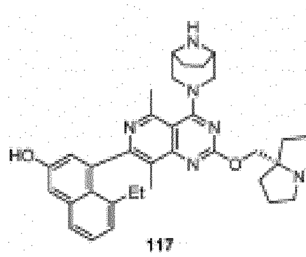
117

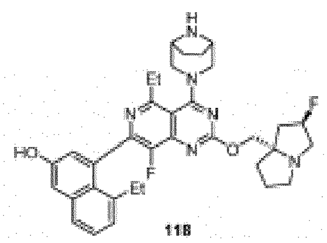
118

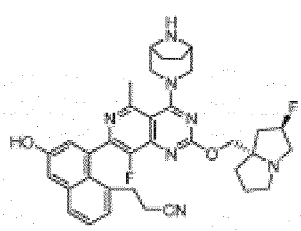
119

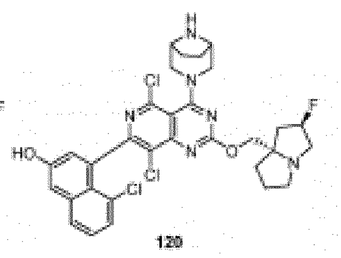
120

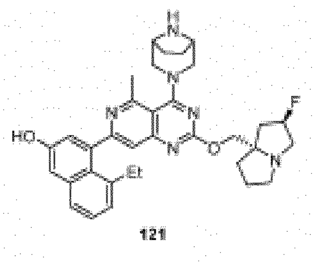
121

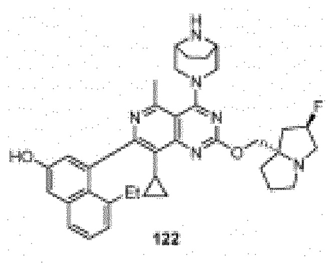
122

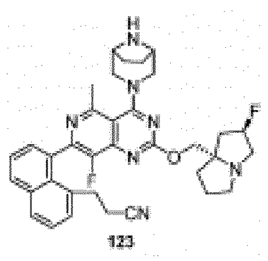
123

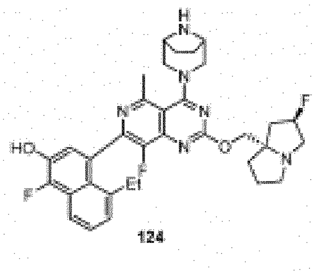
124

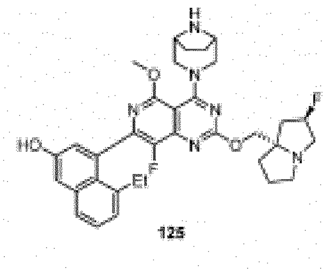
125

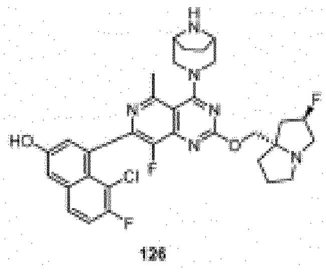
126

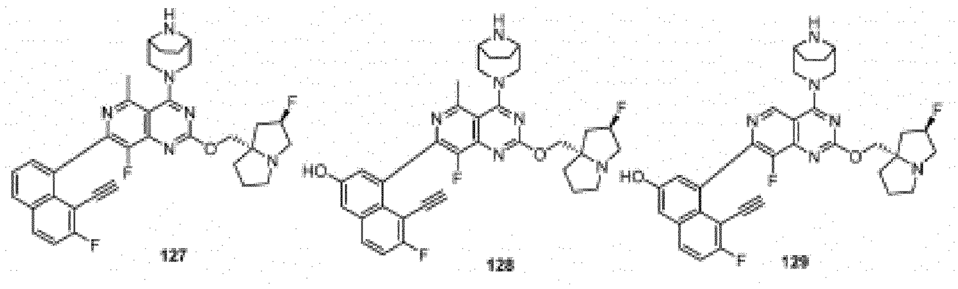

127 128 129

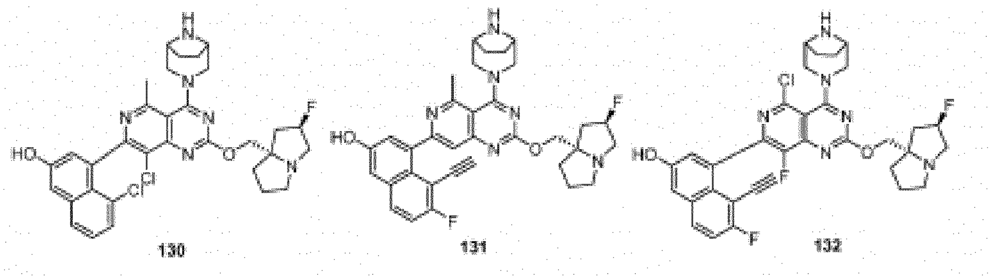

130 131 132

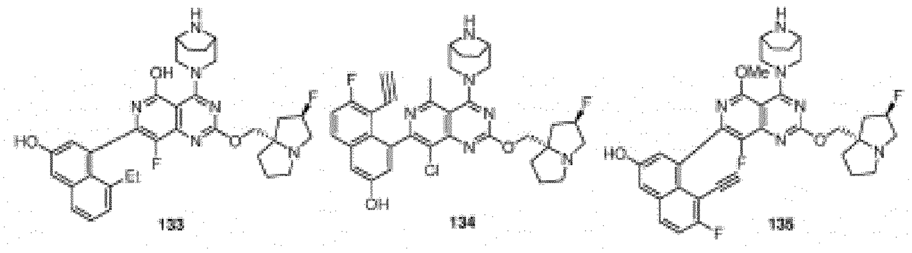

133 134 135

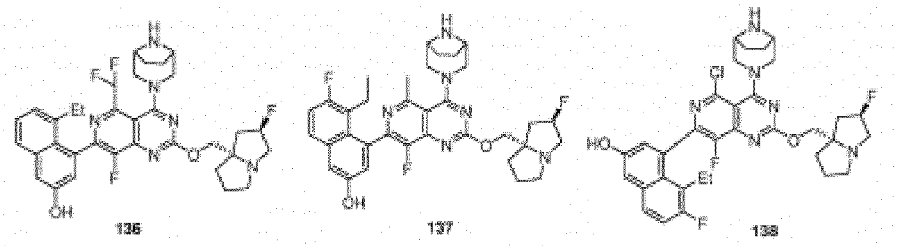

136 137 138

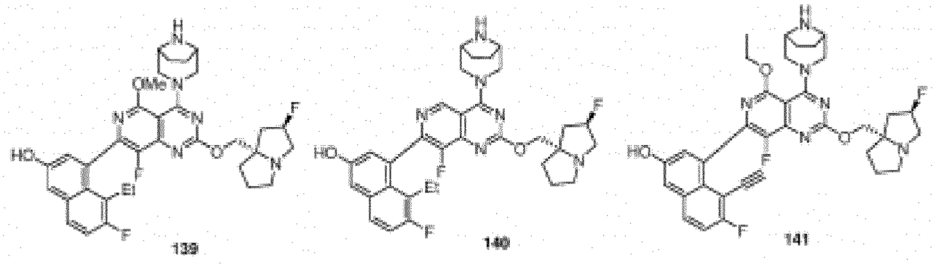

139 140 141

In a certain embodiment, the nitrogen-containing heterocyclic compound of formula I is not any one of the following compounds:

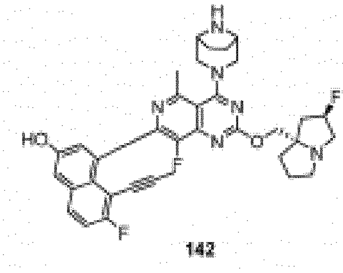

142

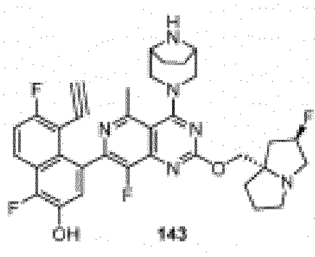

143

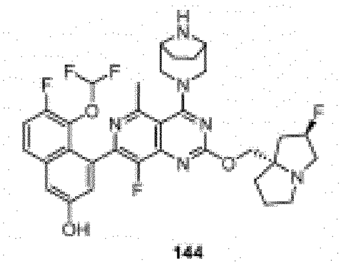

144

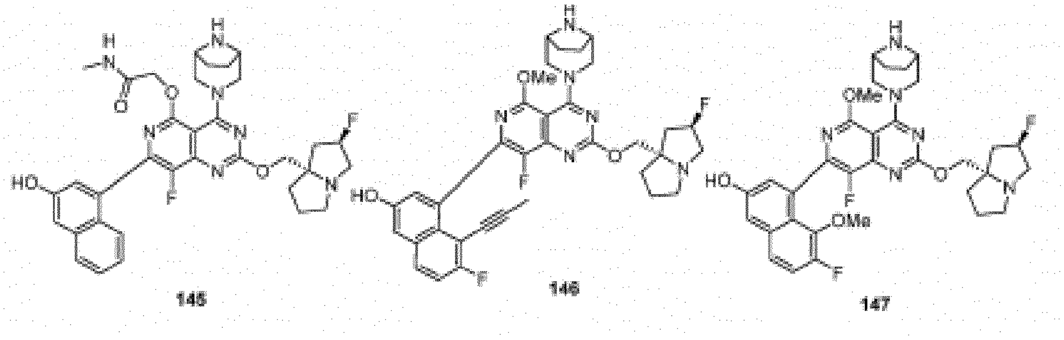

145 146 147

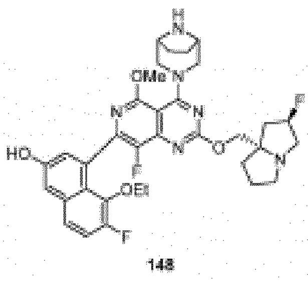

148

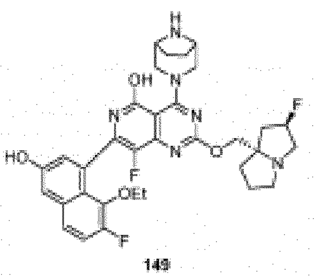

149

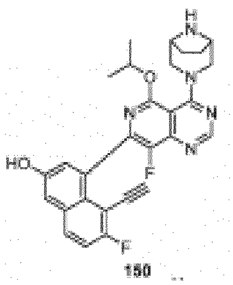

150

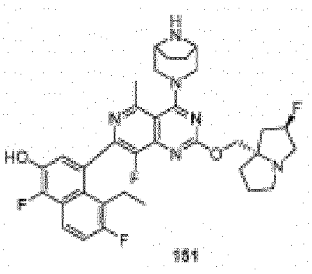

151

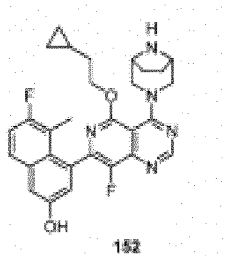

152

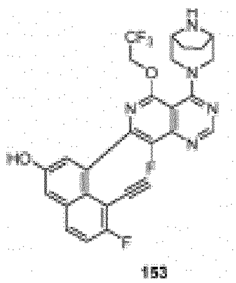

153

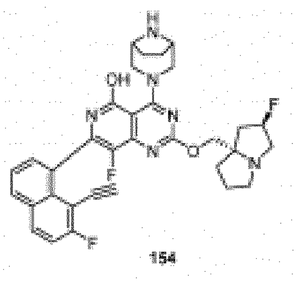

154

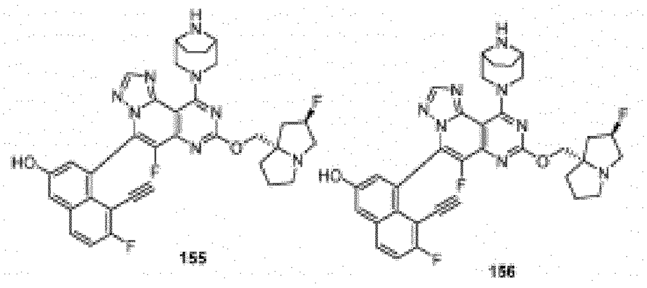

155 156

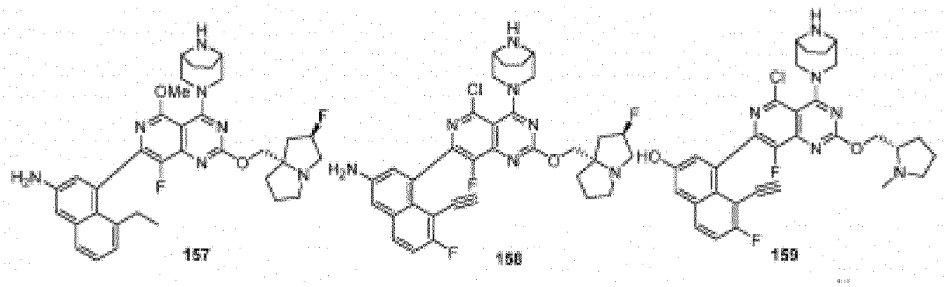

157 158 159

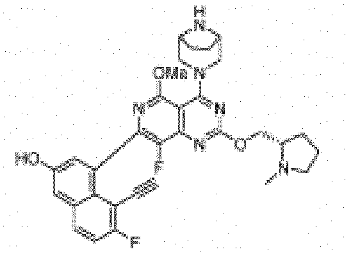

160

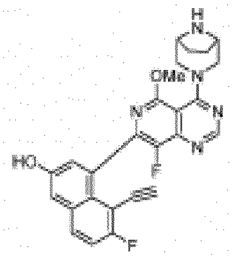

161

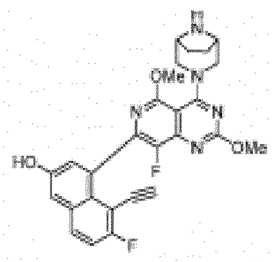

162

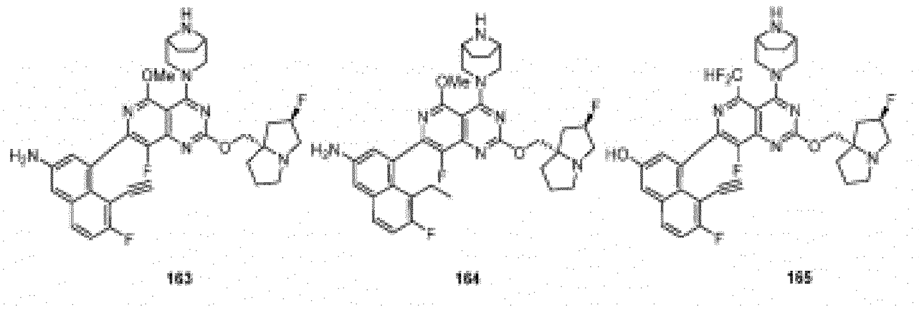

163 164 165

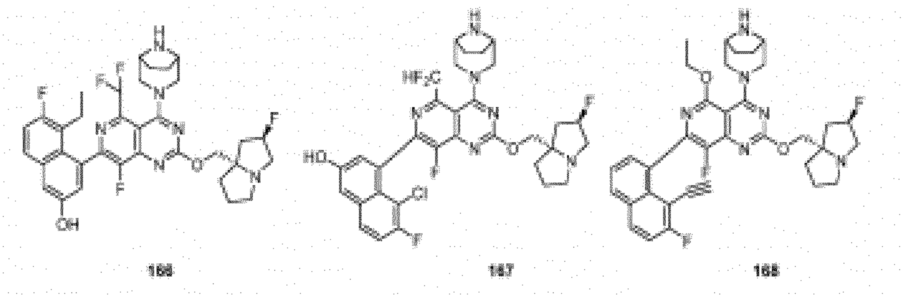

166 167 168

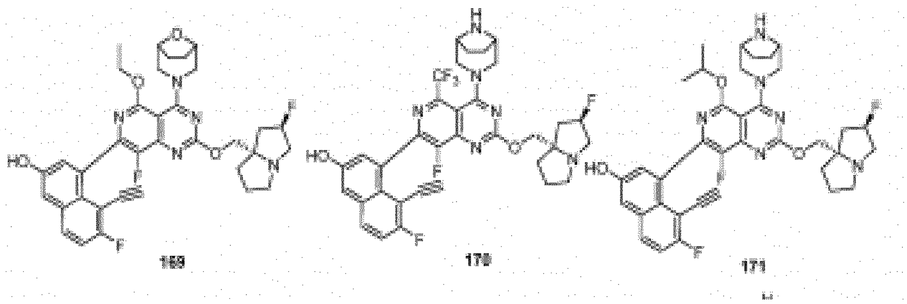

169 170 171

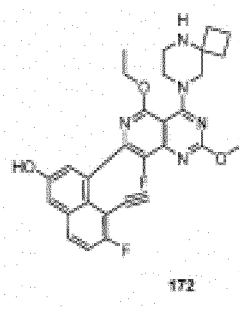

172

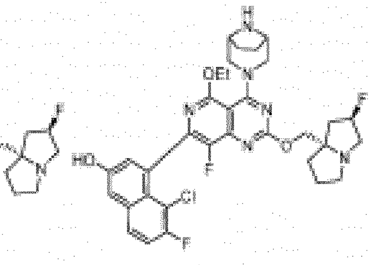

173

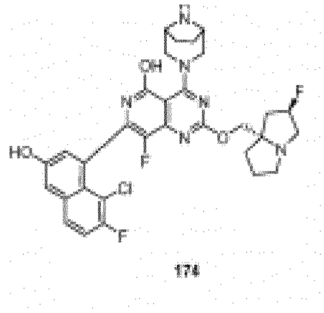

174

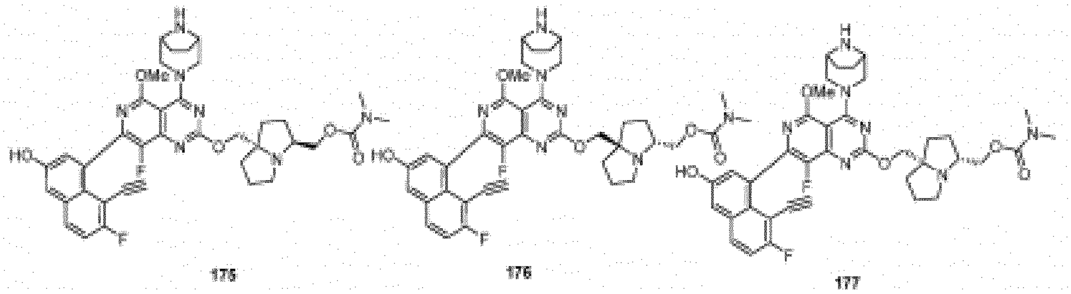

175

176

177

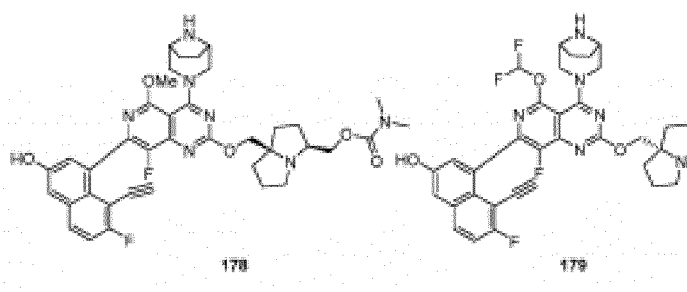

178

179

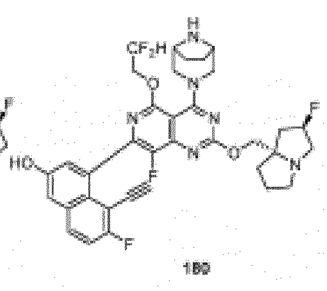

180

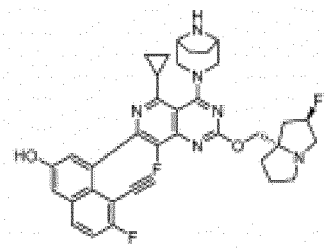

181

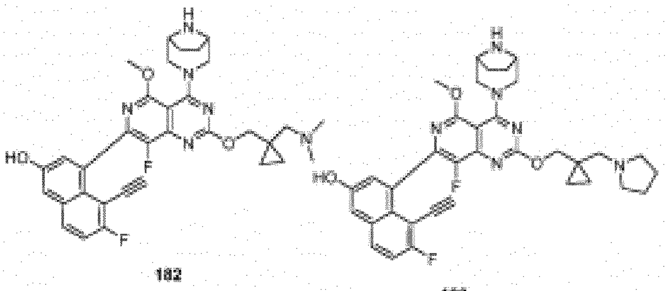

182

183

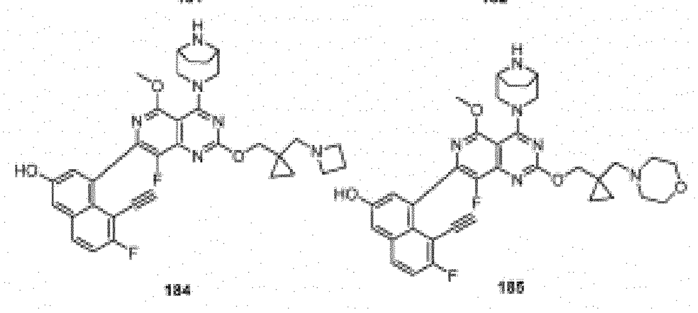

184

185

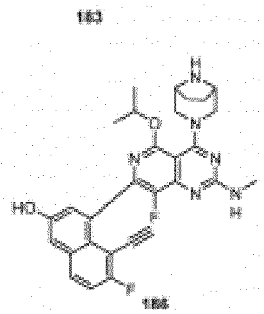

186

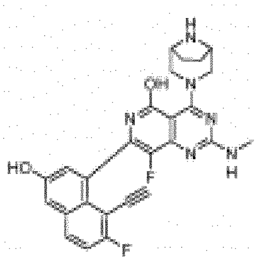

187

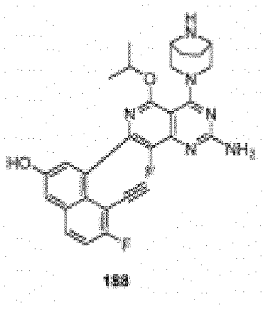

188

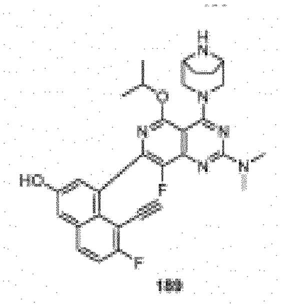

189

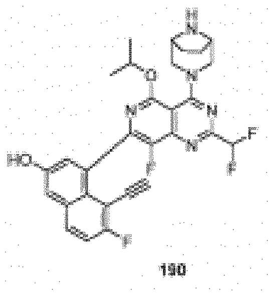

190

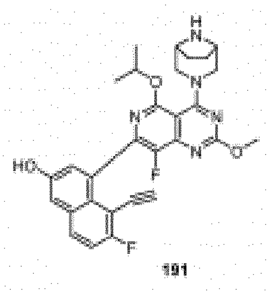

191

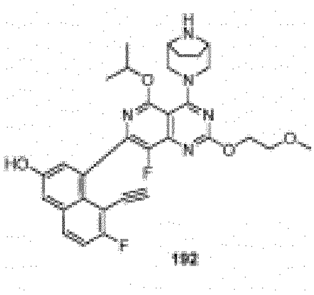

192

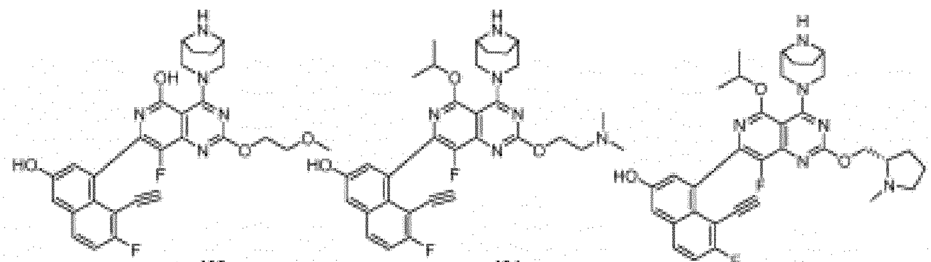

193

194

195

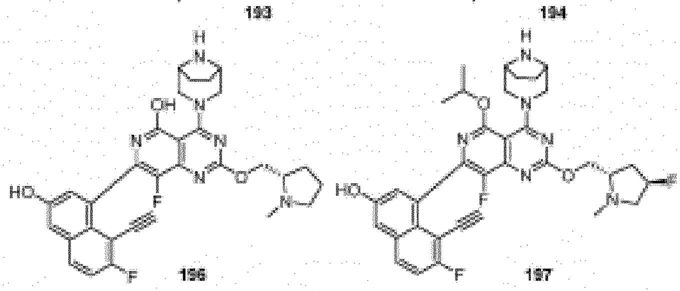

196

197

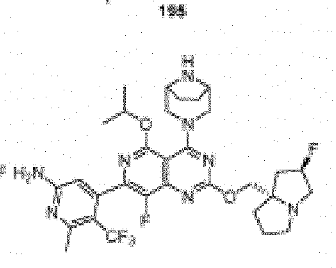

198

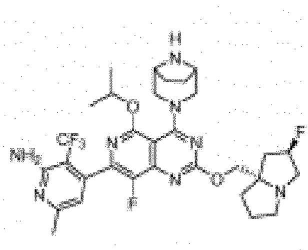

199

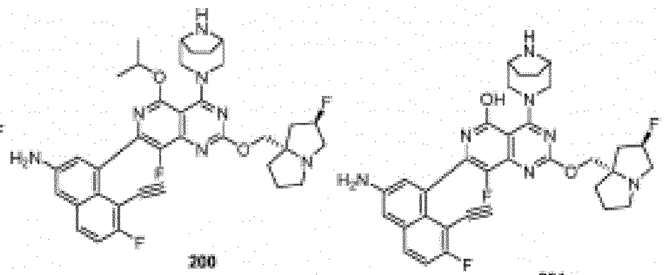

200

201

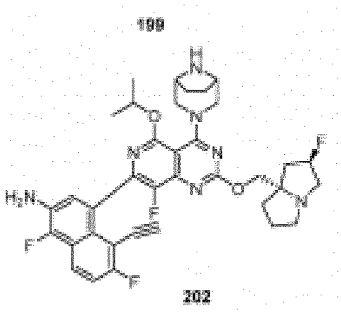

202

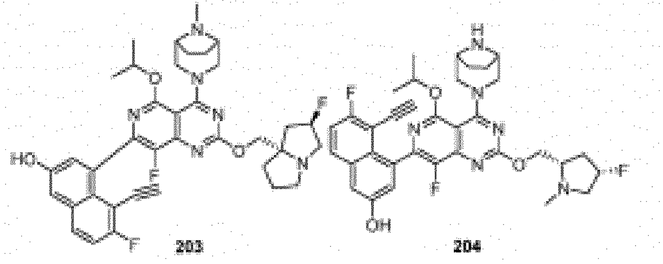

203

204

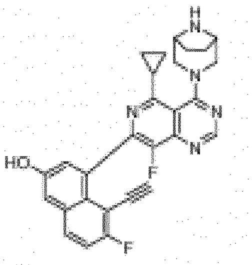

205

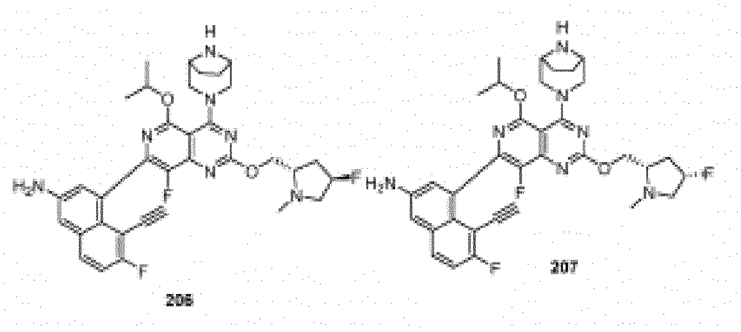

206

207

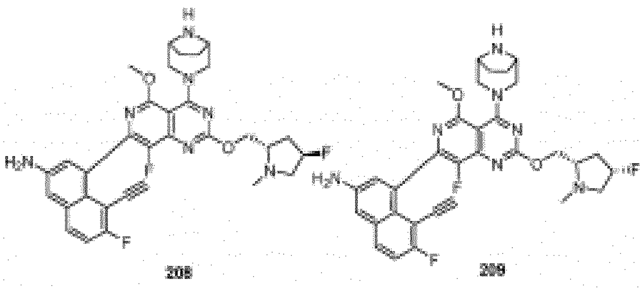

208

209

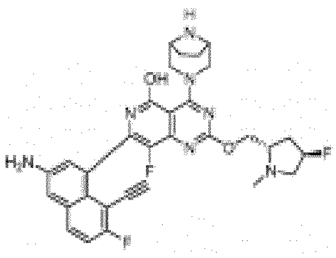

210

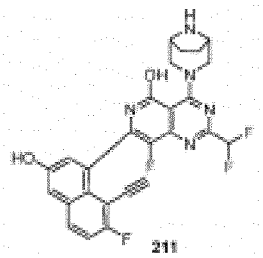

211

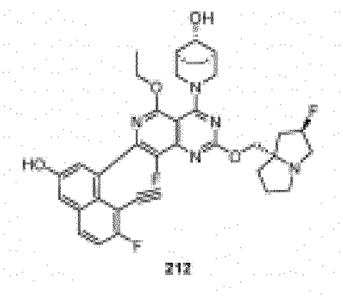

212

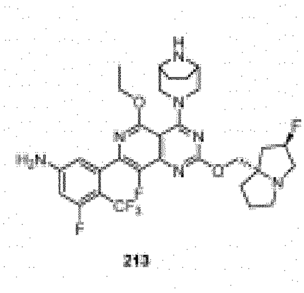

213

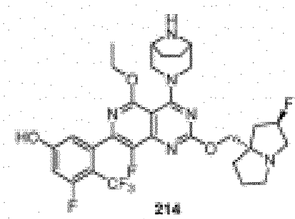

214

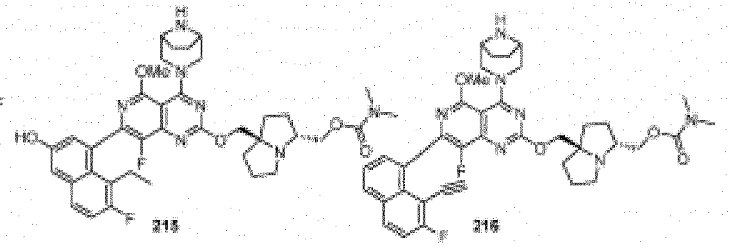

215 216

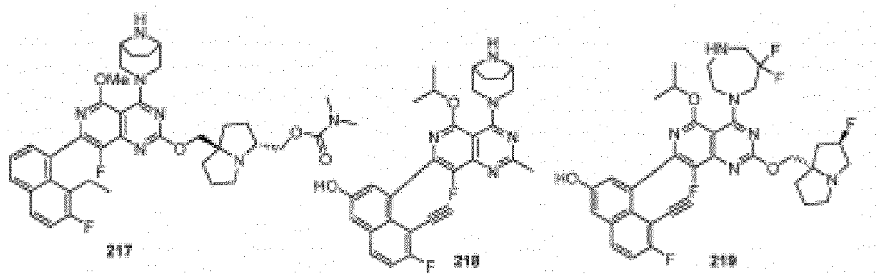

217 218 219

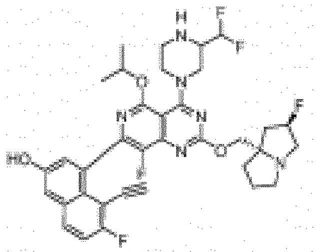

220

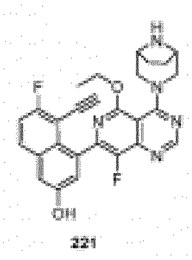

221

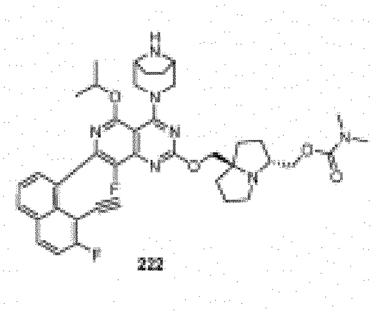

222

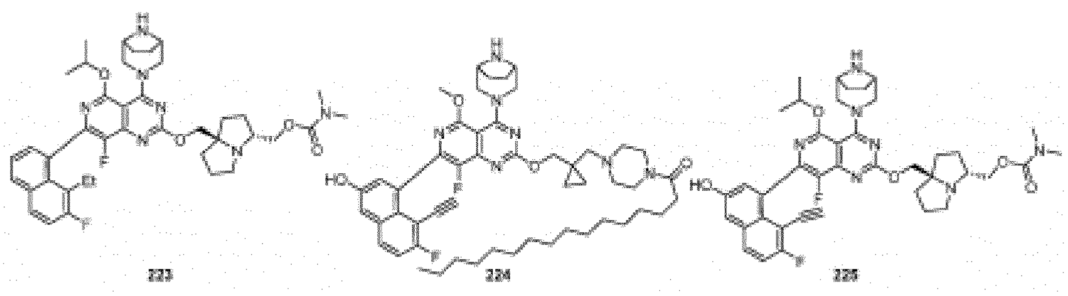

223 224 225

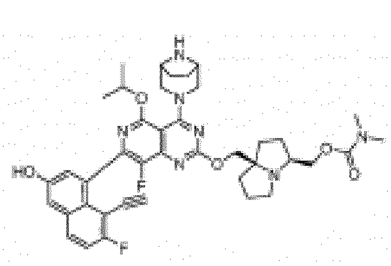

226

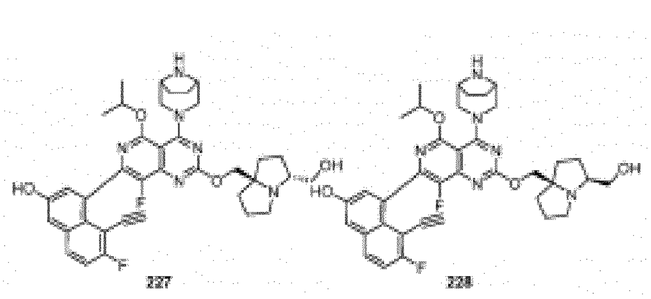

227 228

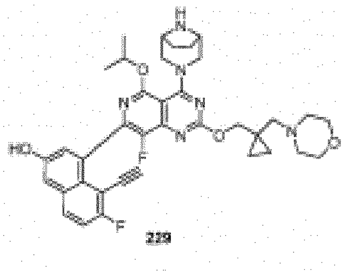

229

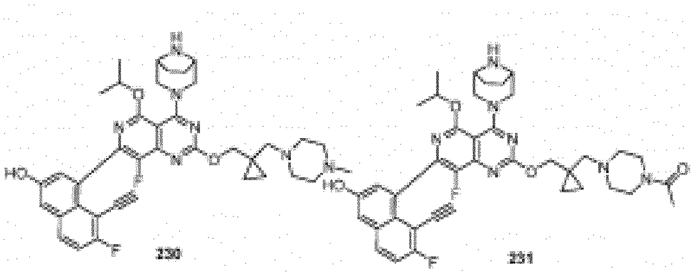

230

231

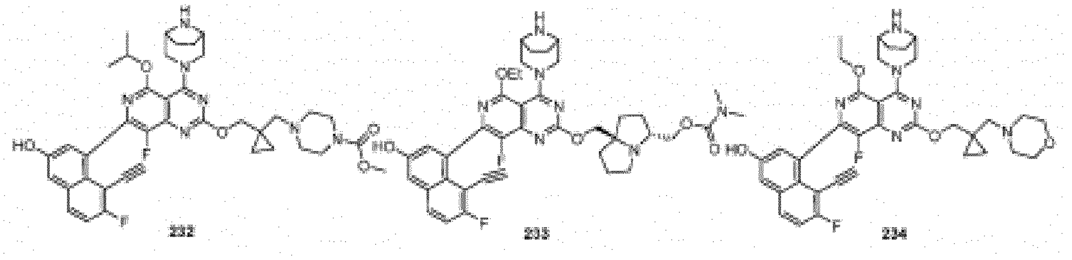

232

233

234

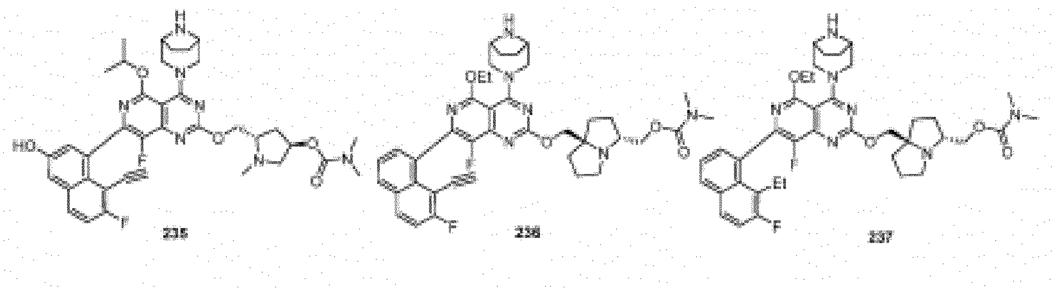

235

236

237

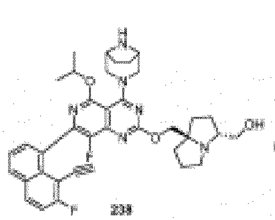

238

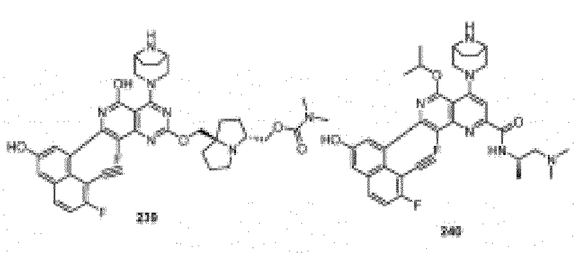

239

240

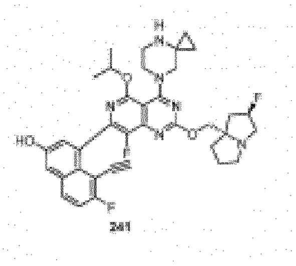

241

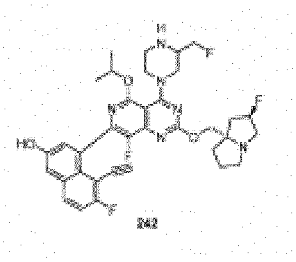

242

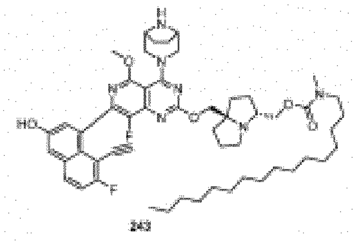

243

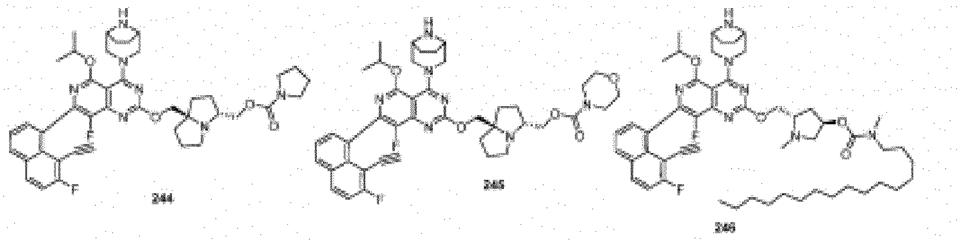

244

245

246

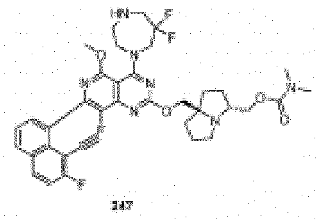

247

In a certain embodiment, the nitrogen-containing heterocyclic compound of formula I is not any one of the following compounds:

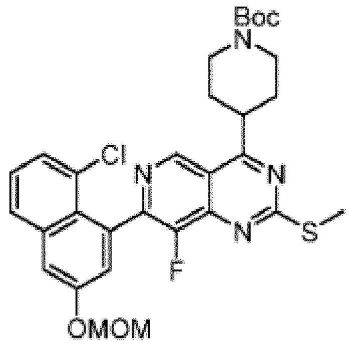

56-6 ,

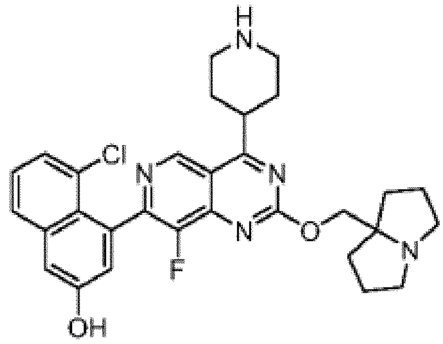

56-9 ,

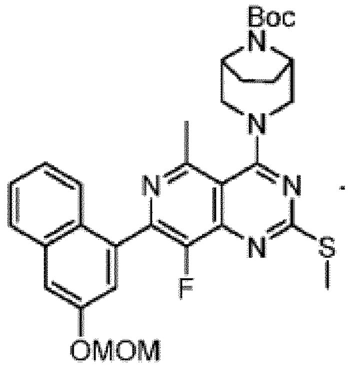

81-9 ,

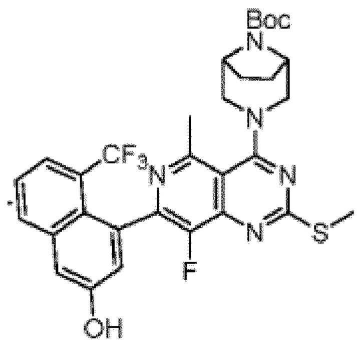

106-4 ,

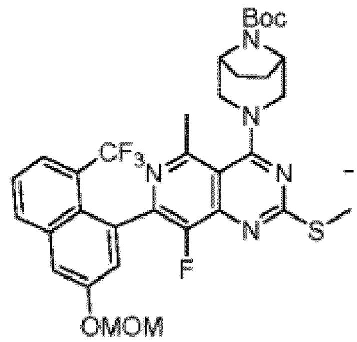

106-5 ,

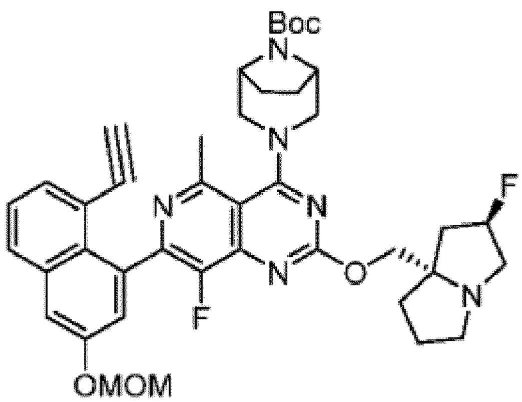

103-8 ,

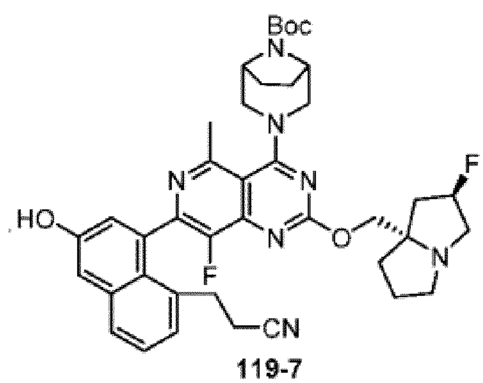

119-7 ,

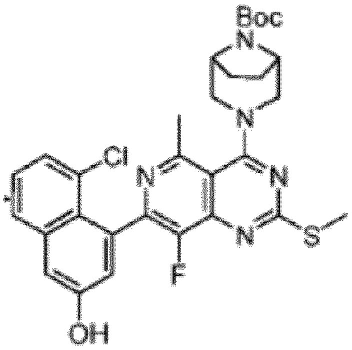

116-1 ,

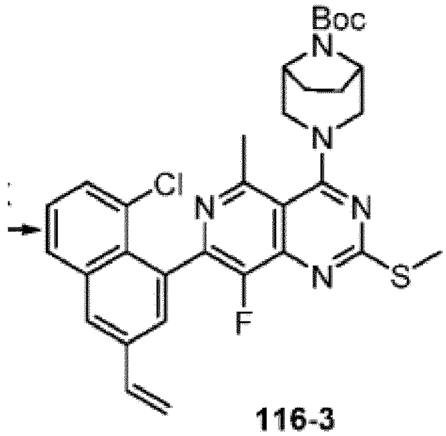

116-3 ,

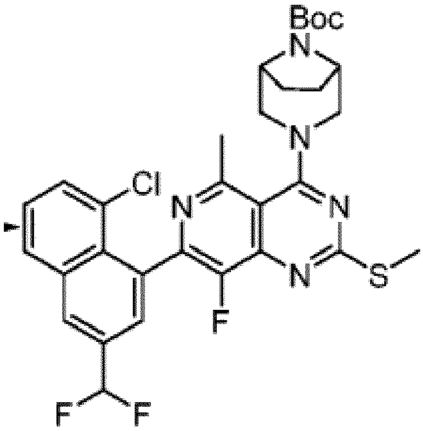

116-5 ,

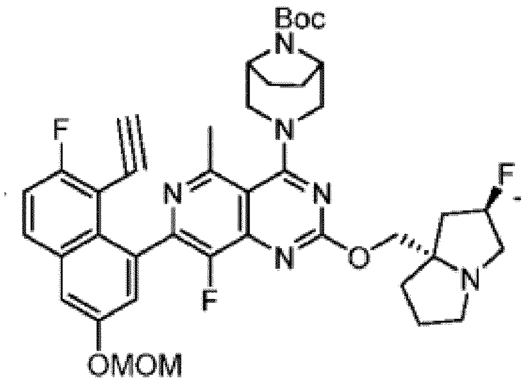

128-15 ,

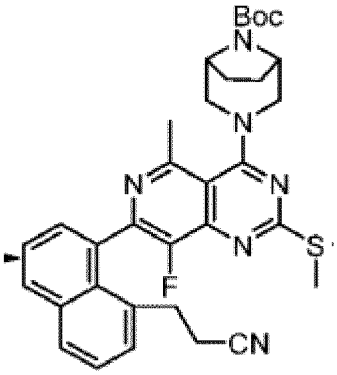

123-5 ,

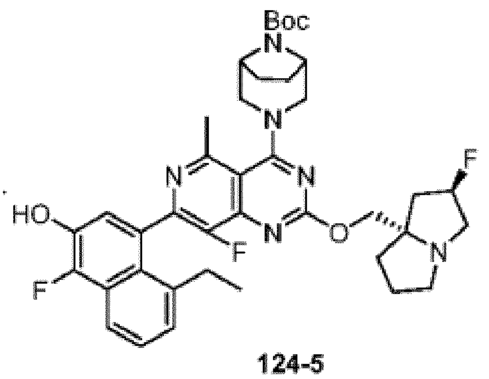

124-5 ,

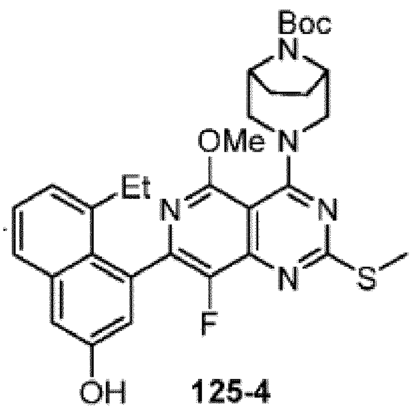

125-4,

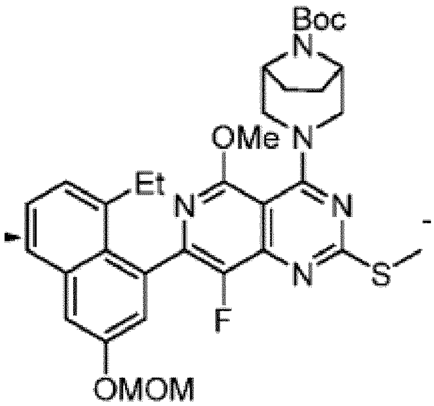

125-5,

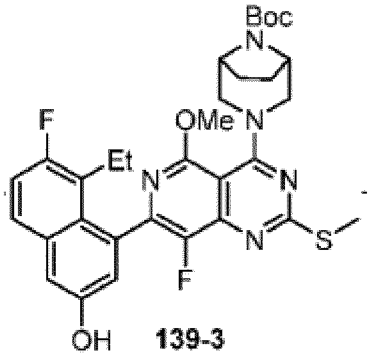

139-3,

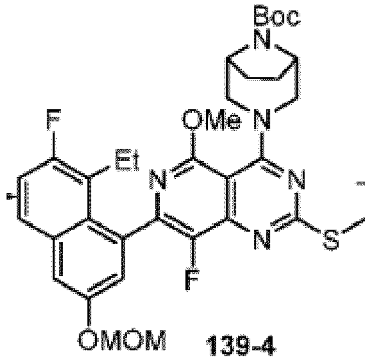

139-4,

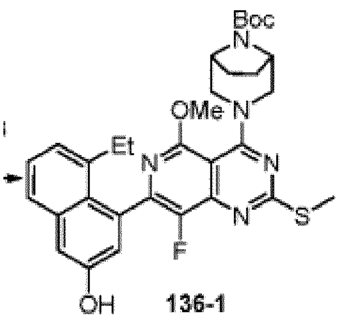

136-1,

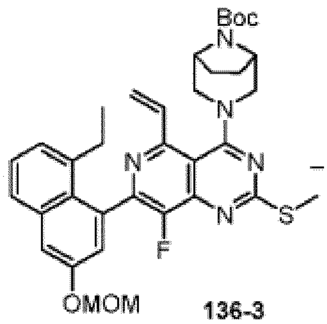

136-3,

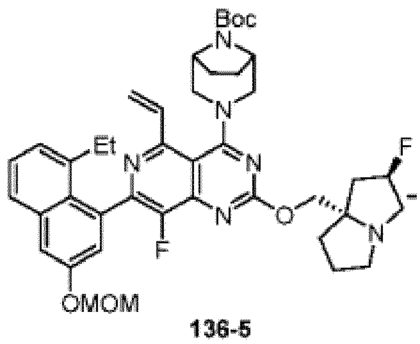

136-5,

and

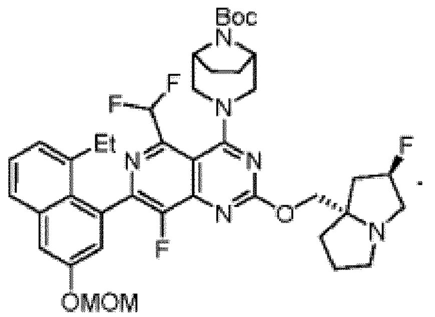

136-7.

In a certain embodiment, the nitrogen-containing heterocyclic compound of formula I is not any one of the following compounds:

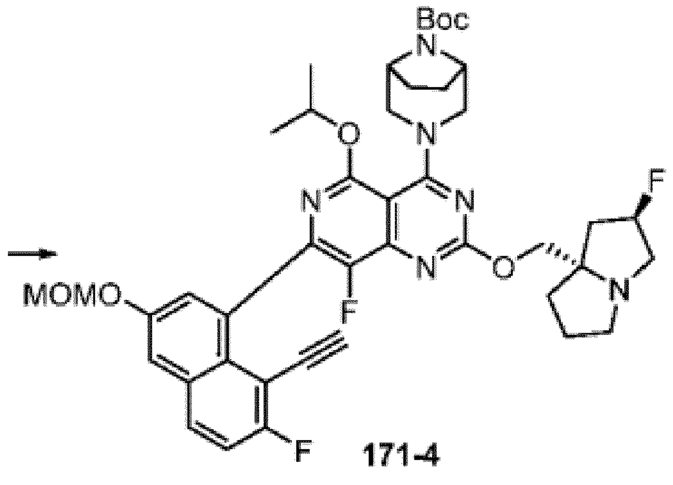

171-4

,

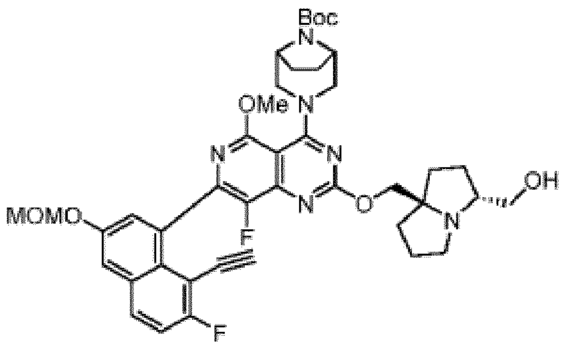

176-8

,

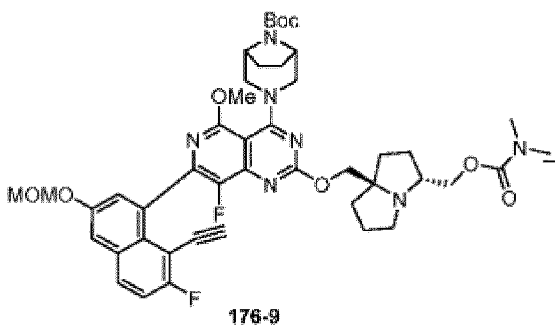

176-9

,

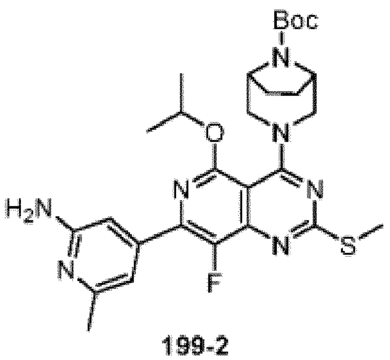

199-2

,

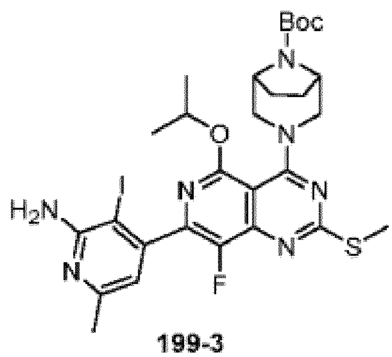

199-3

,

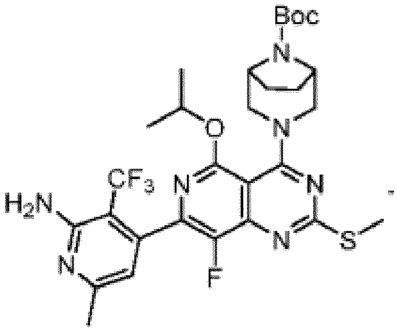

199-4 ,

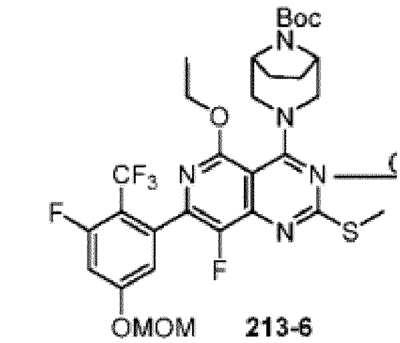

213-6 ,

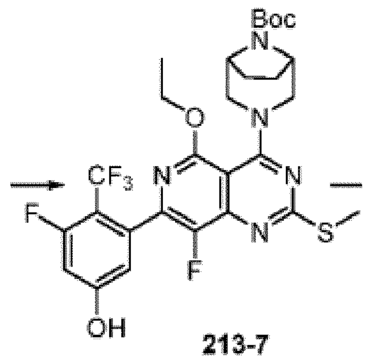

213-7 ,

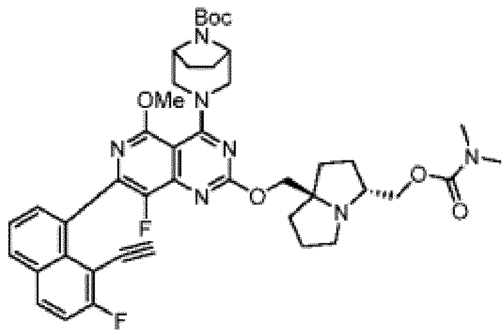

217-3 ,

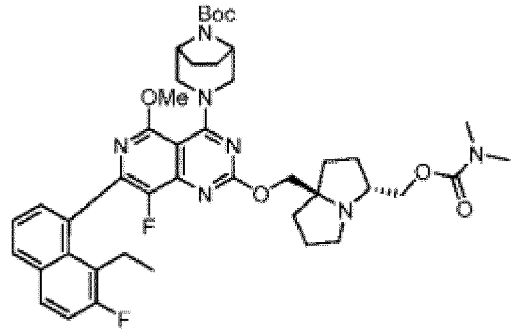

217-4 ,

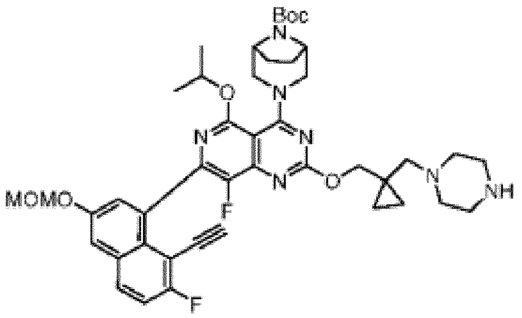

231-3 ,

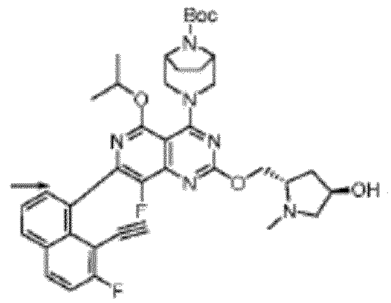

246-7 ,

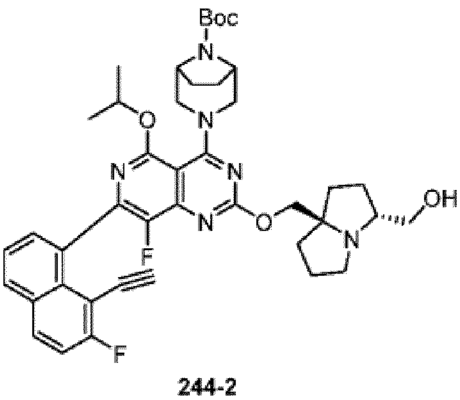

244-2 , and

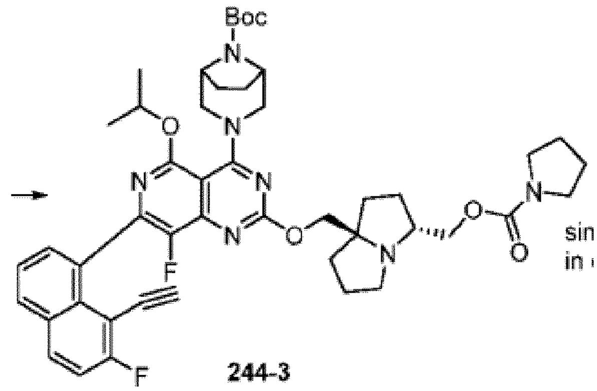

sin
in

244-3 .

In a certain embodiment, the nitrogen-containing heterocyclic compound of formula I is not any one of the

following compounds:

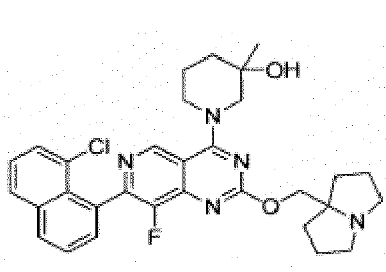,

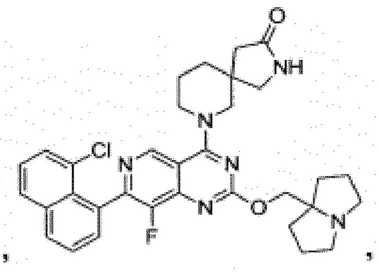,

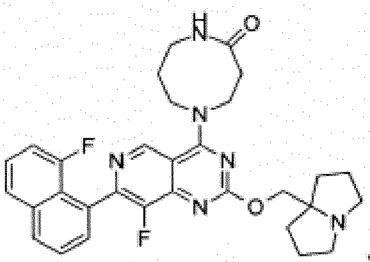,

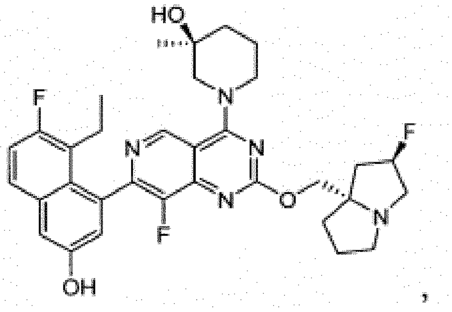,

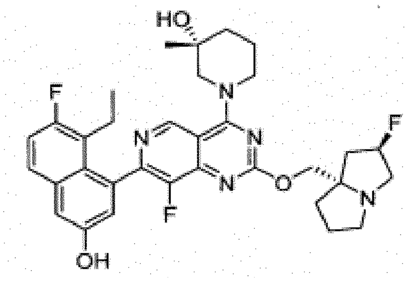,

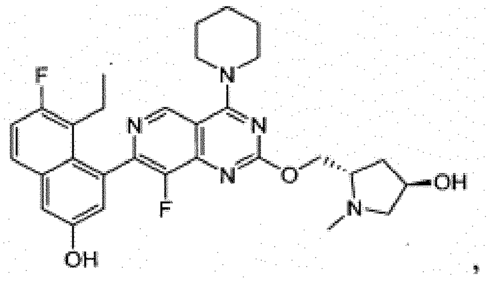,

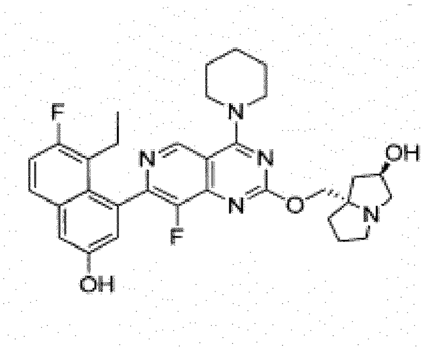,

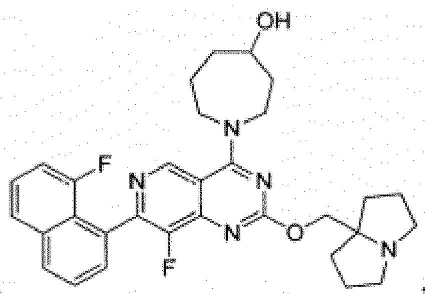,

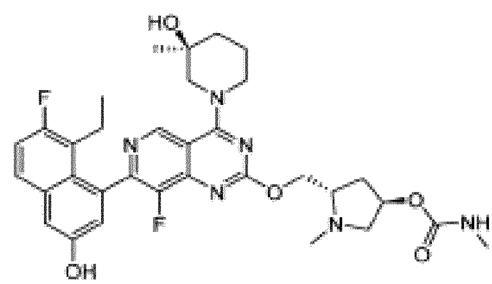,

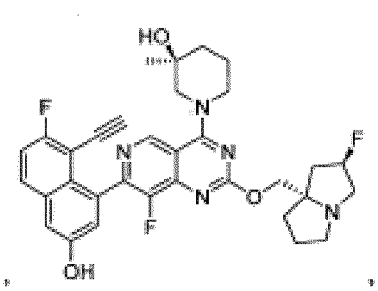,

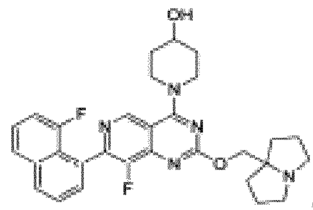, 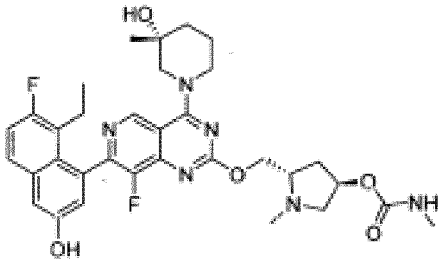,

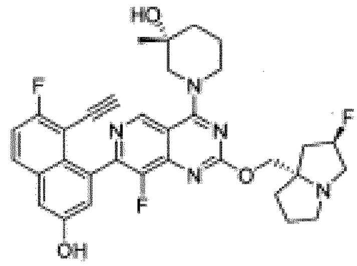, 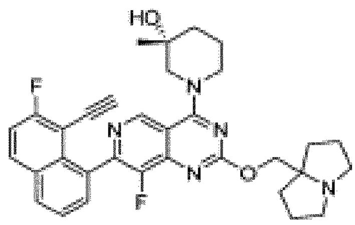,

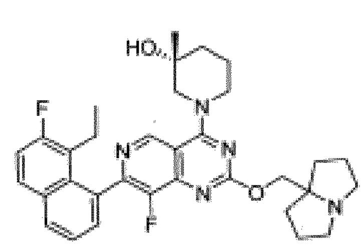, 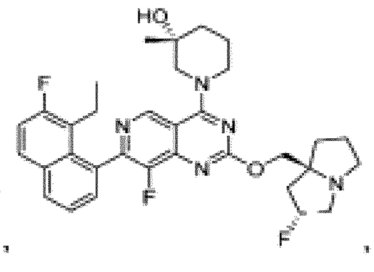,

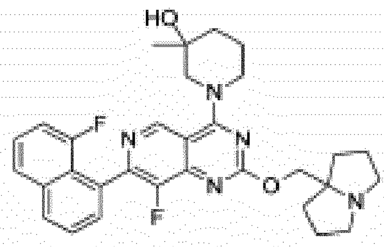, 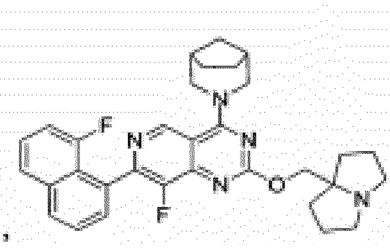,

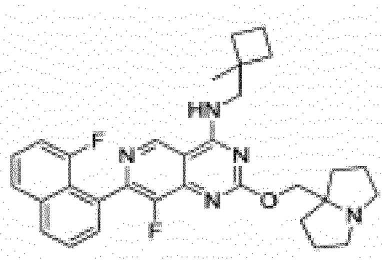, 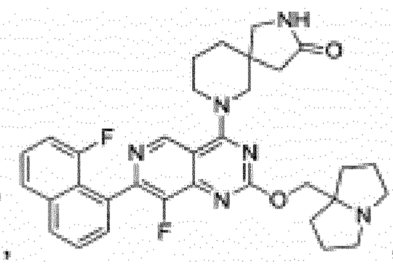,

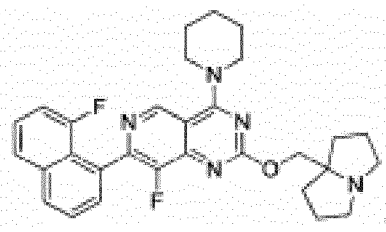, 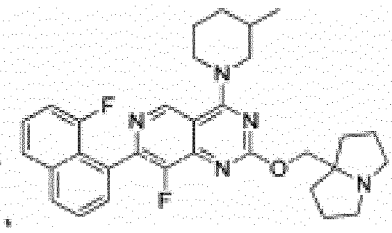,

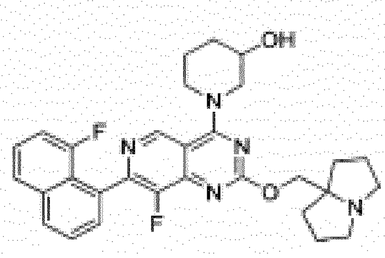,
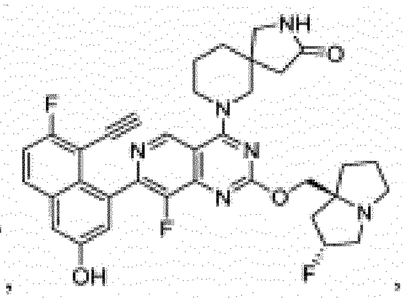,

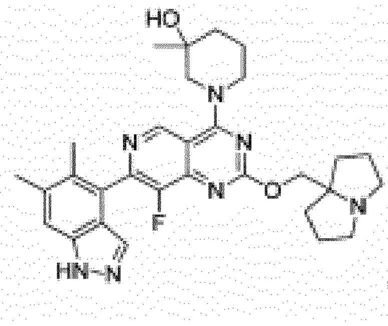,
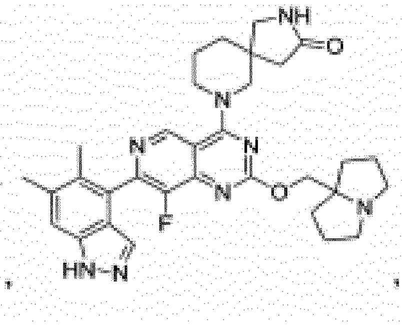,

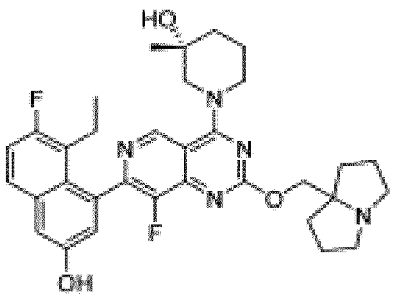,
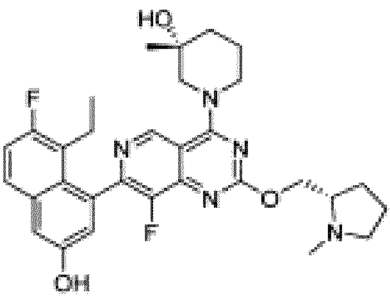,

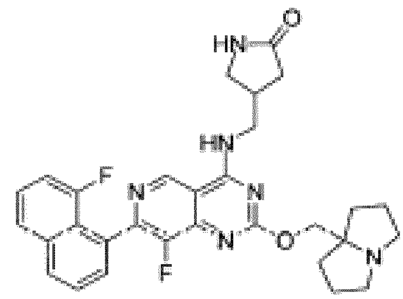,
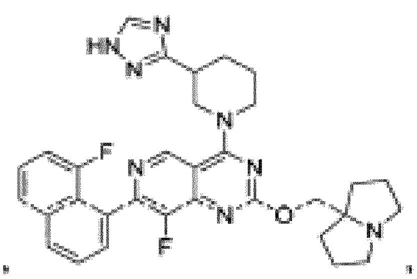,

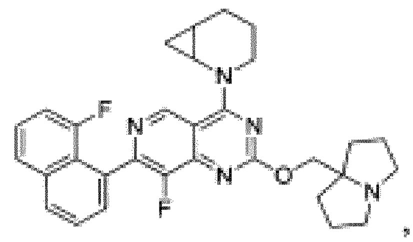,
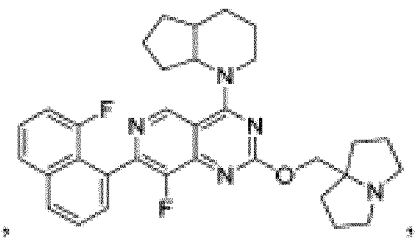,

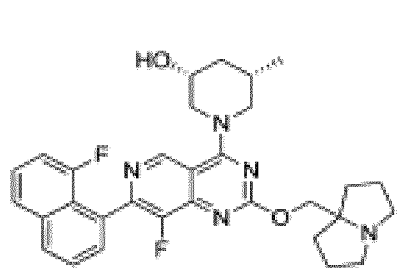, 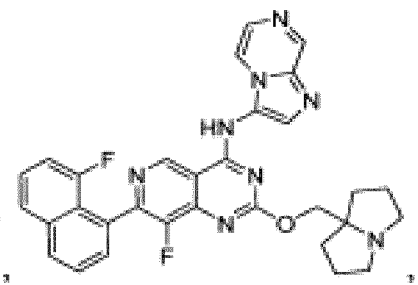,

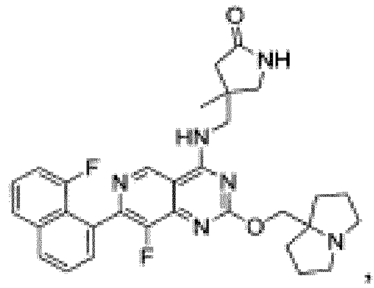, 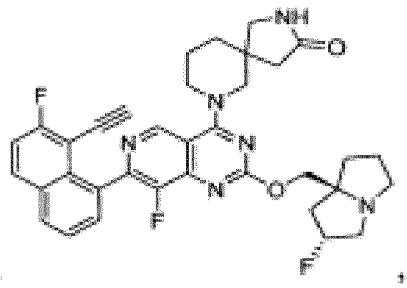,

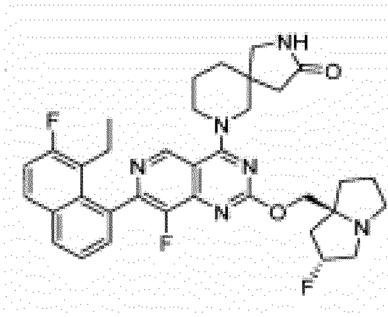, 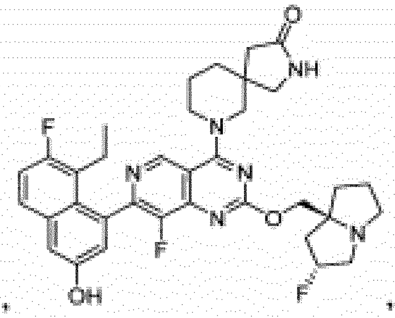,

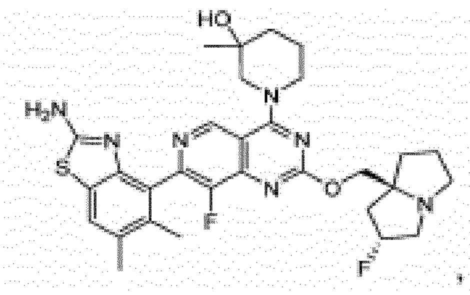, 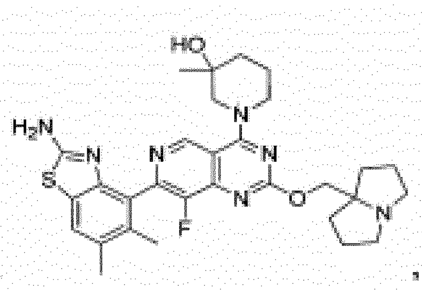,

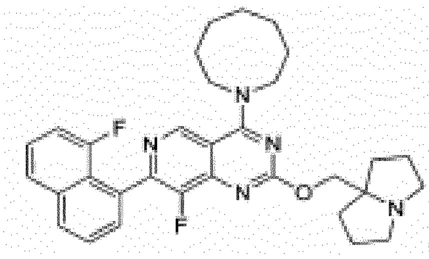, 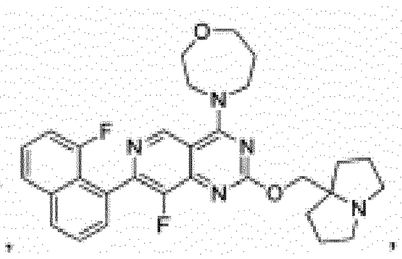,

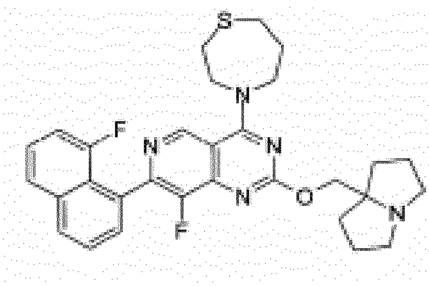,
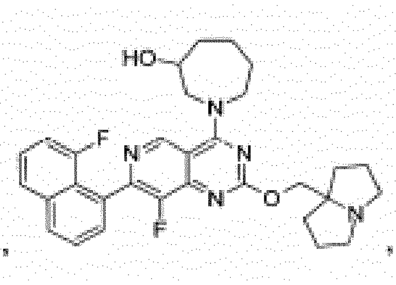,

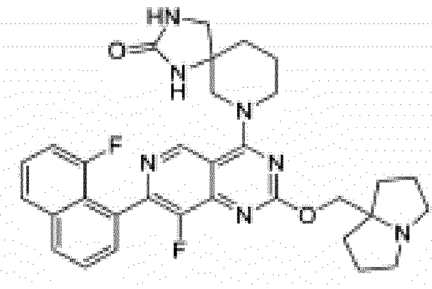,
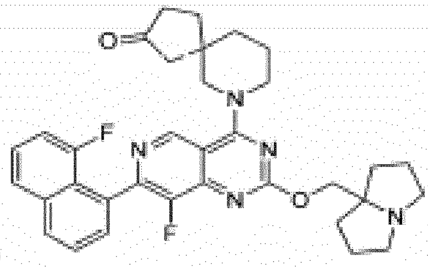,

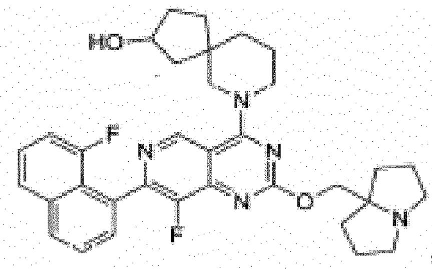,
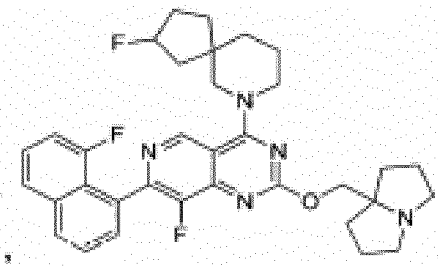,

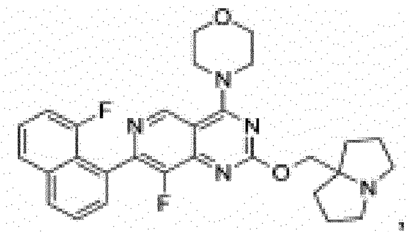,
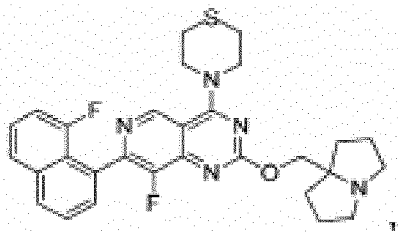,

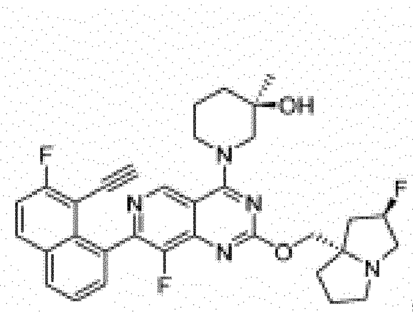,
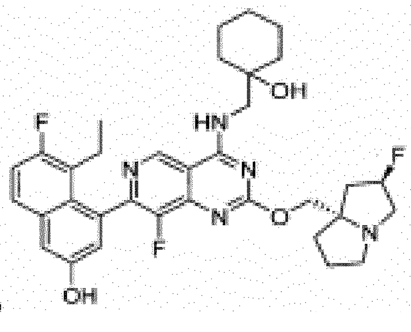,

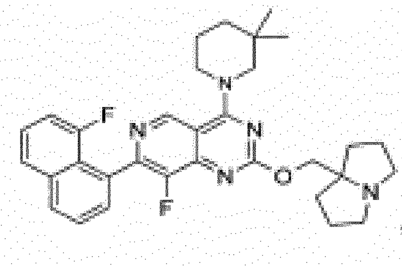,
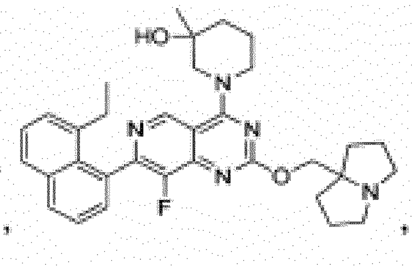,

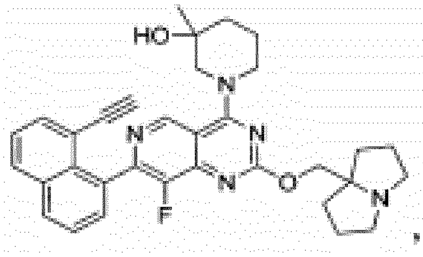,
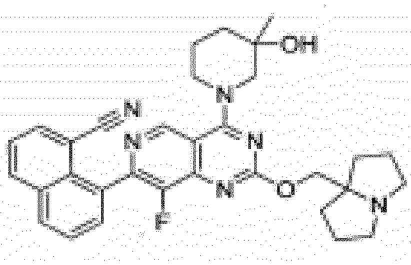,

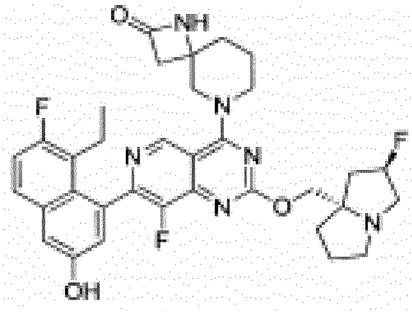,
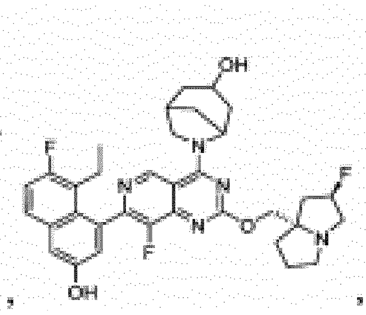,

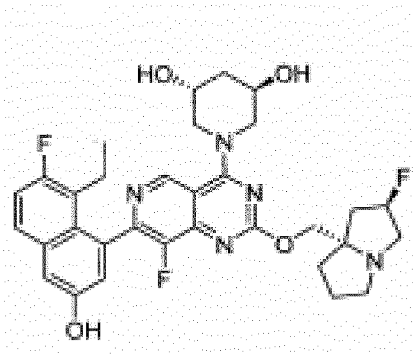,
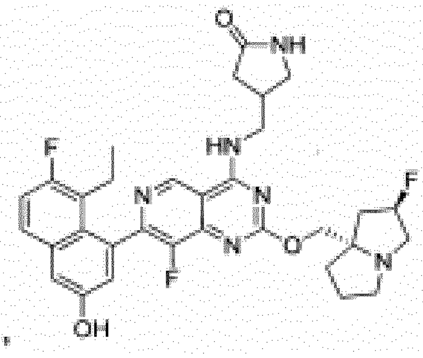,

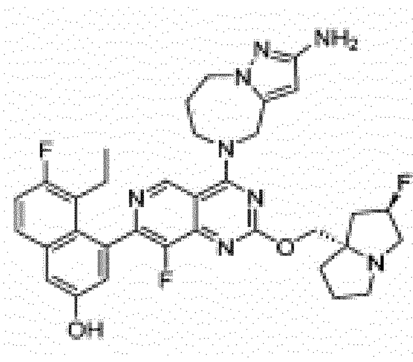,
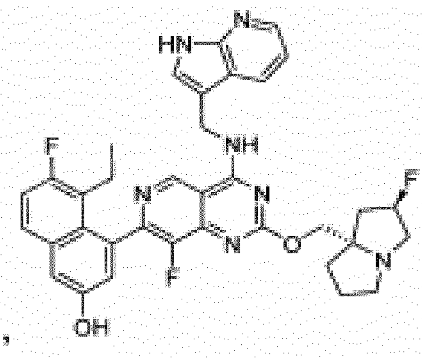,

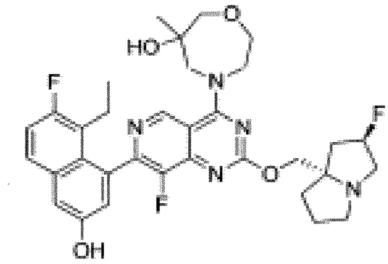,
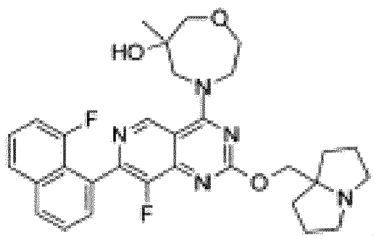,

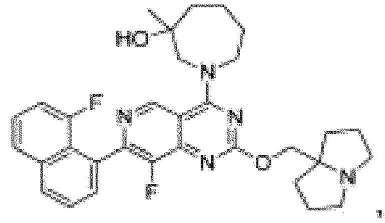,
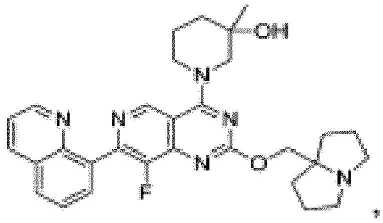,

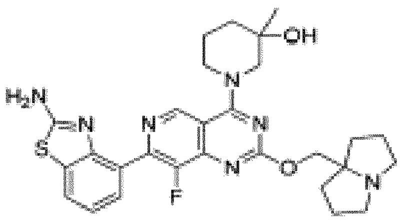,
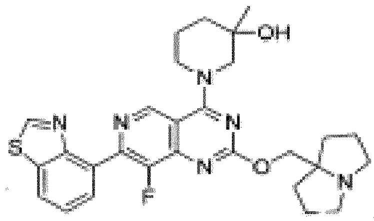,

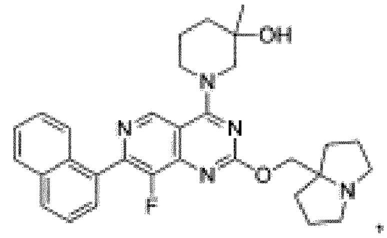,
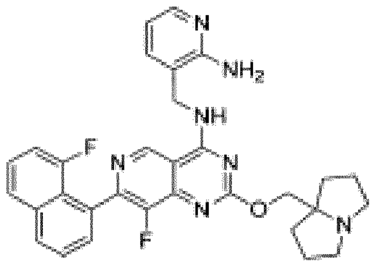,

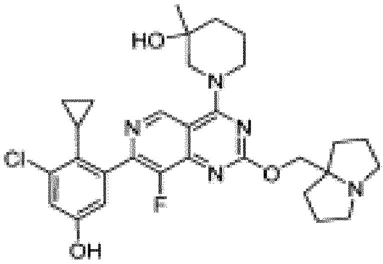,
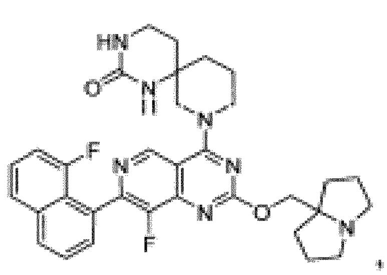,

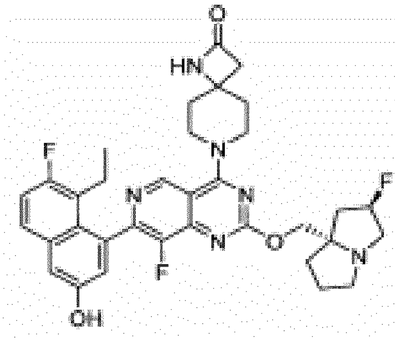,
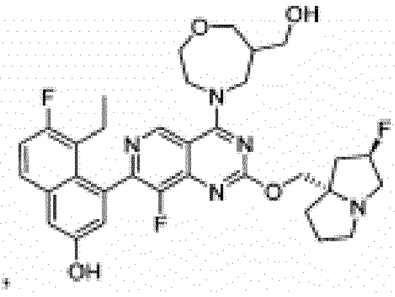,

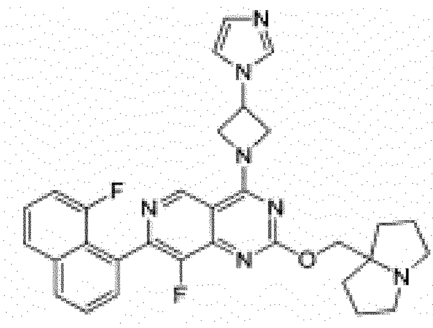,
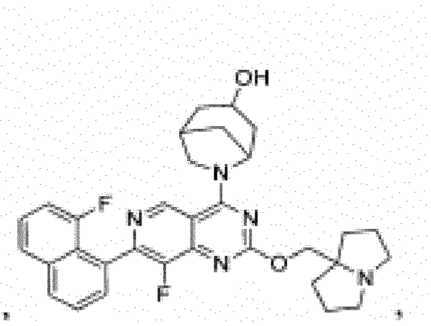,

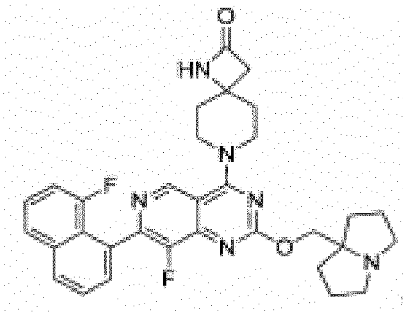,
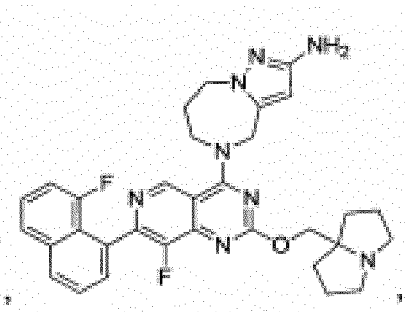,

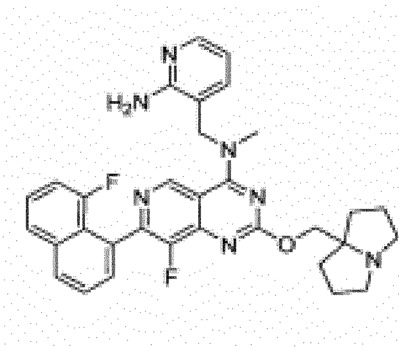,
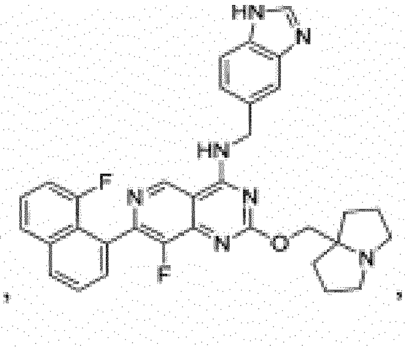,

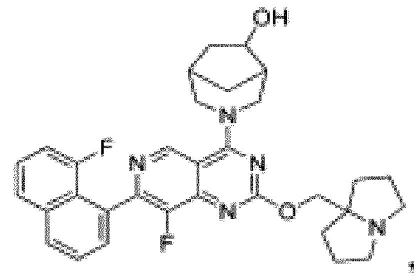,
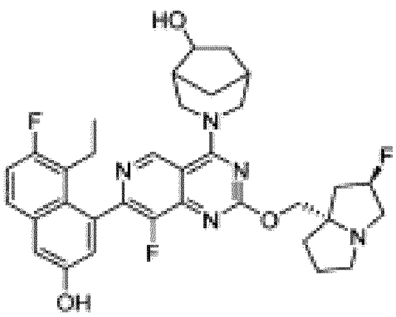,

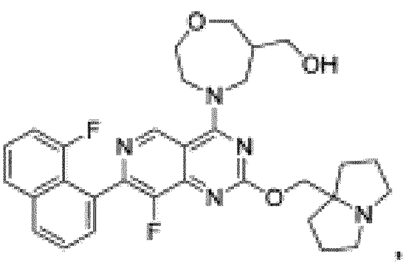,
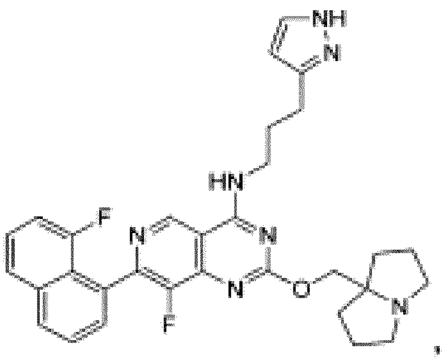,

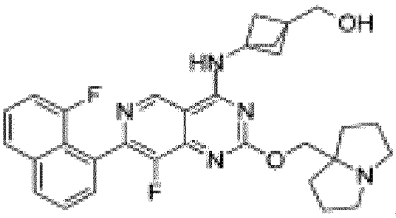,
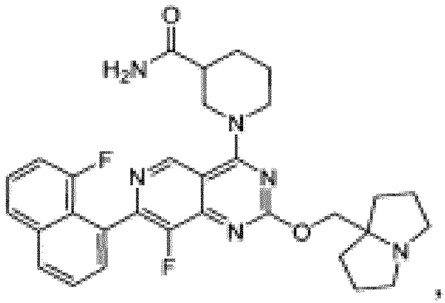,

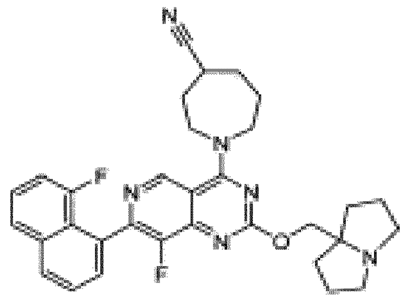,
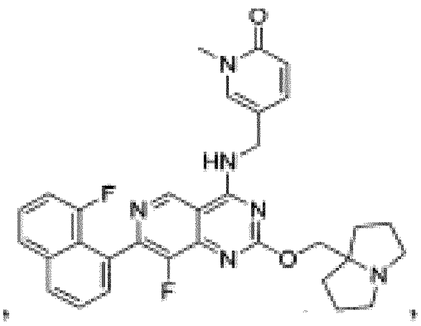,

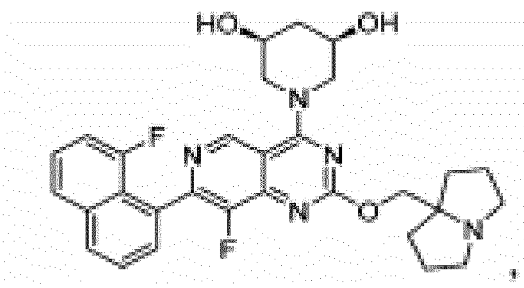,
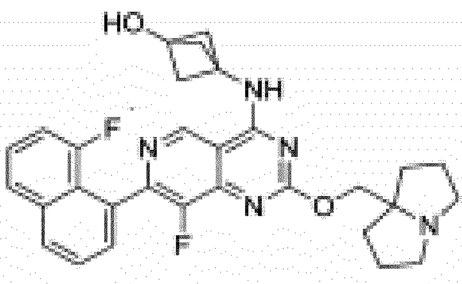,

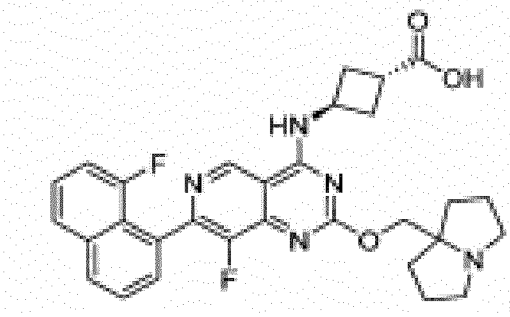,
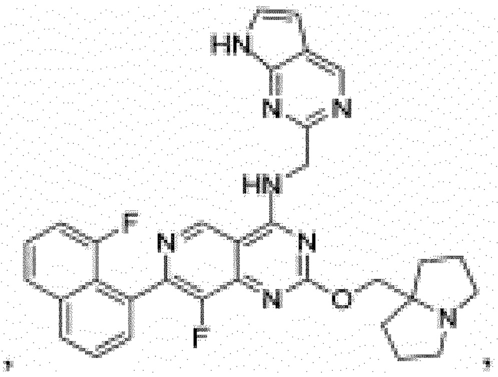,

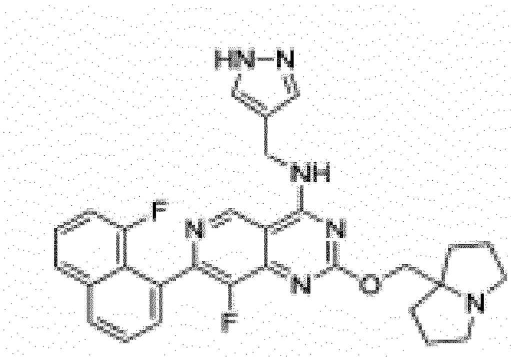,
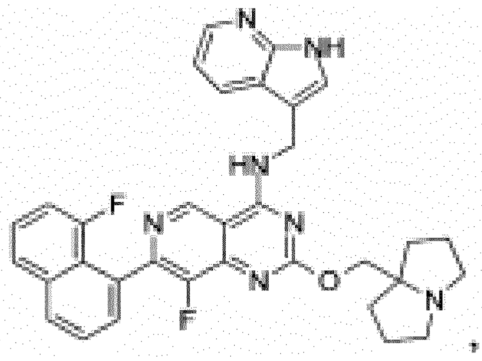,

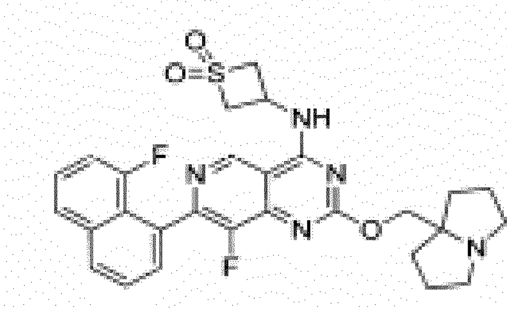,
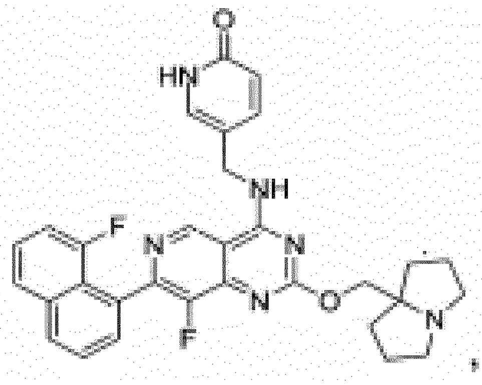,

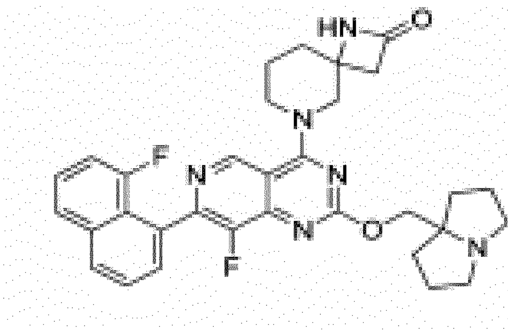,
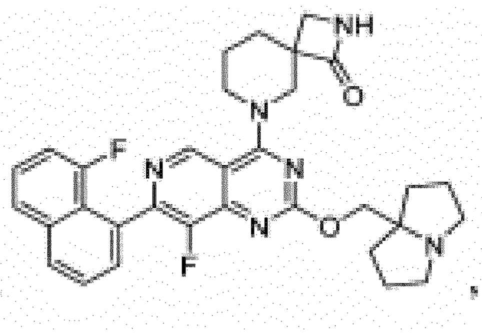,

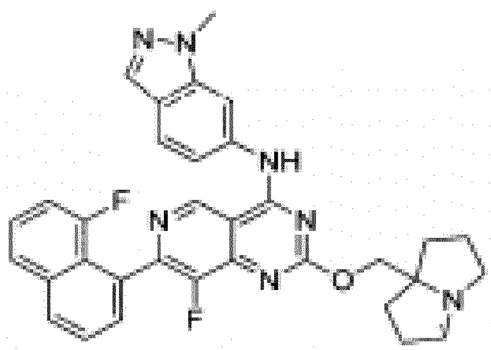,
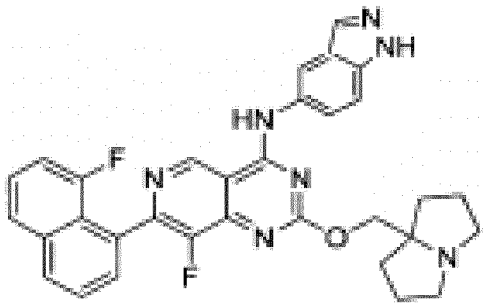,

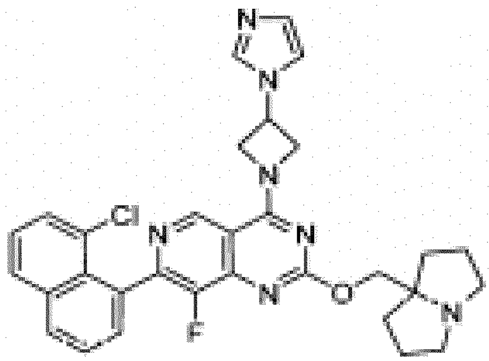,
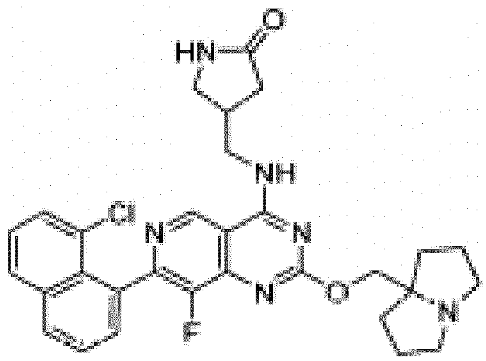,

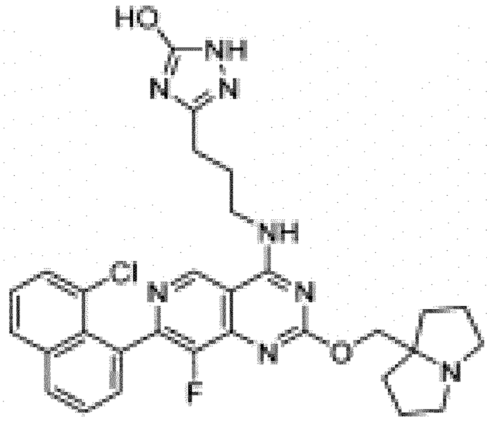,
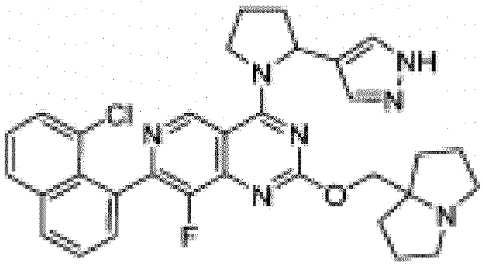,

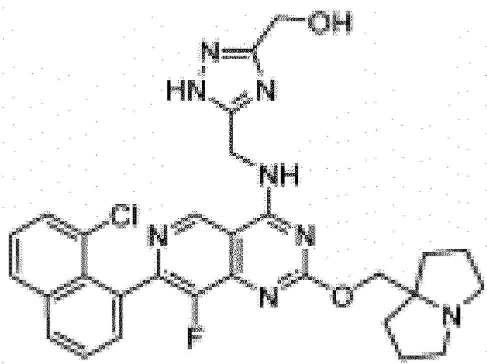,
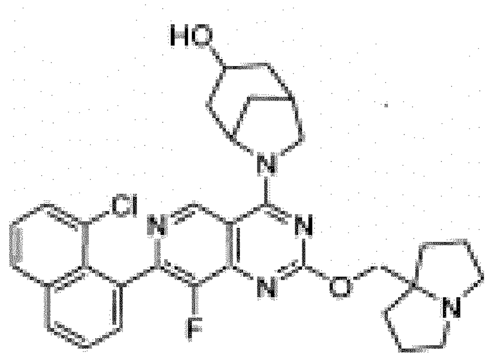,

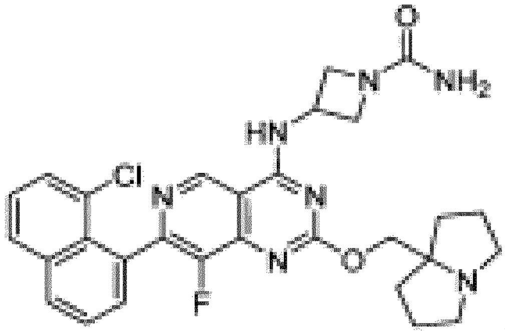,

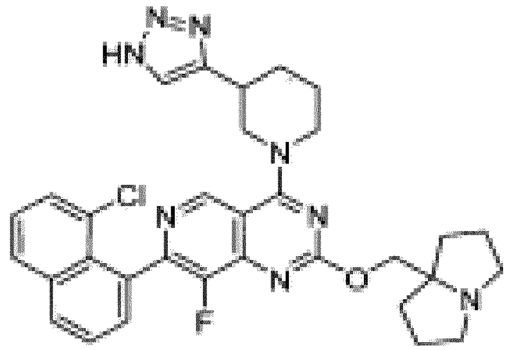,

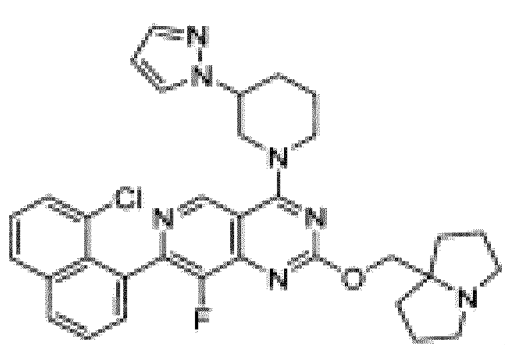,

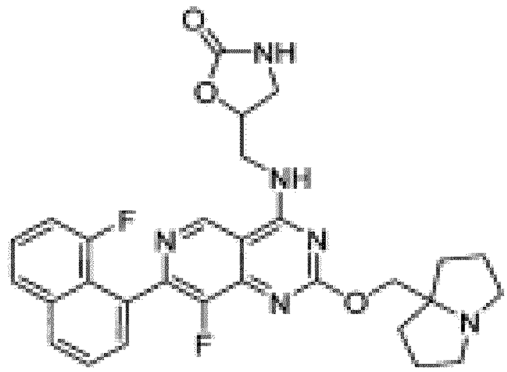,

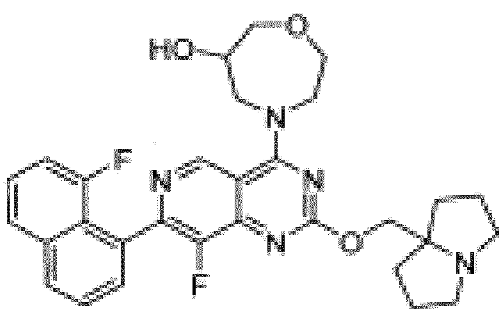,

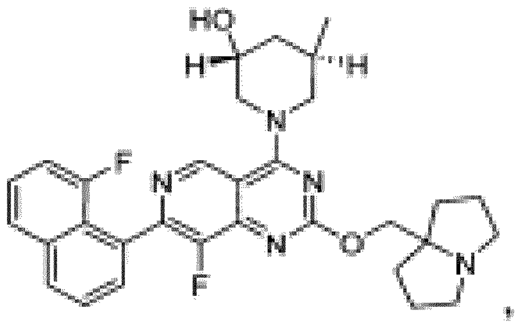,

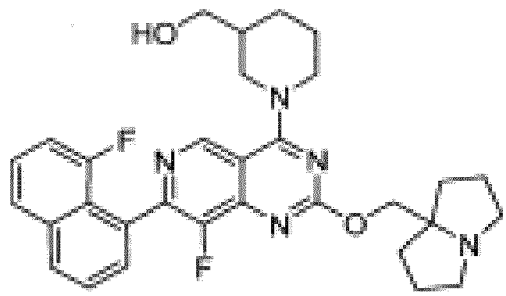,

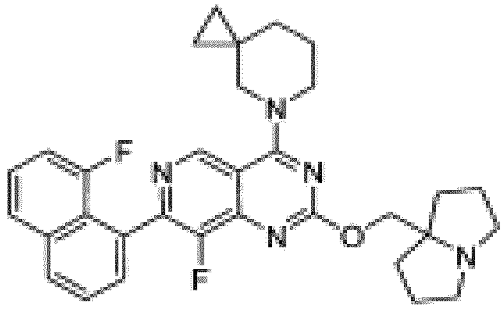,

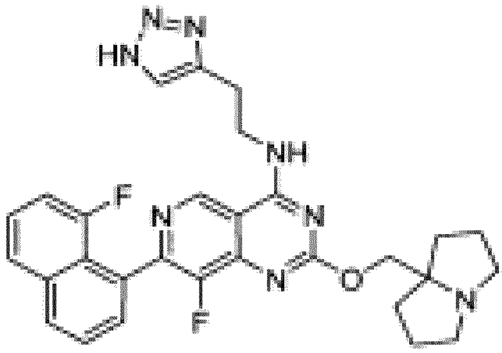,
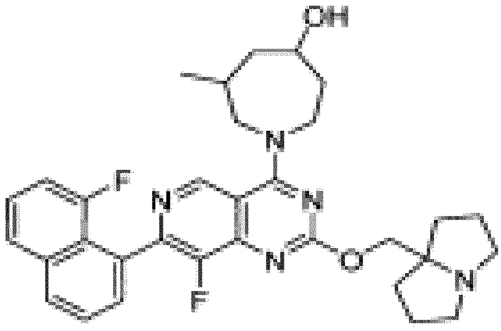,

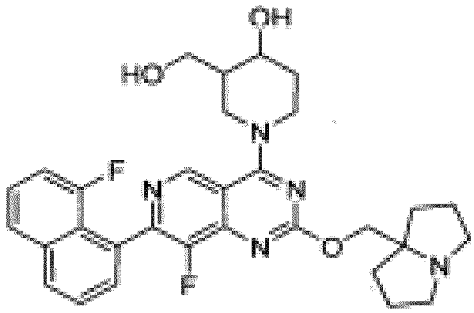,
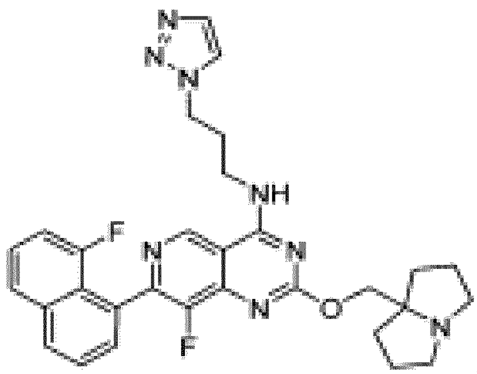,

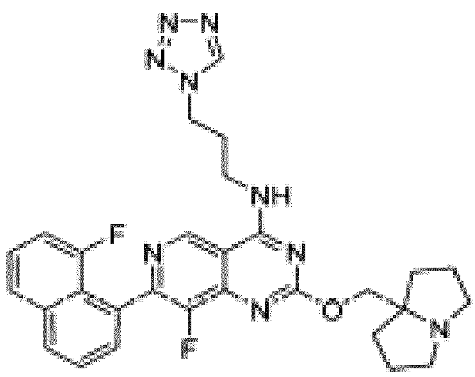,
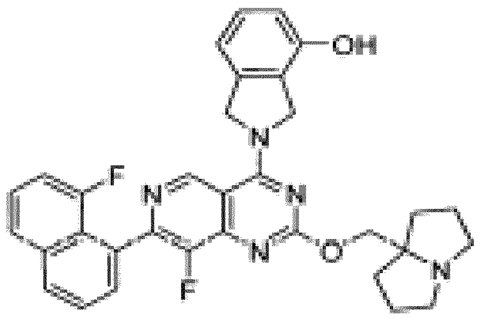,

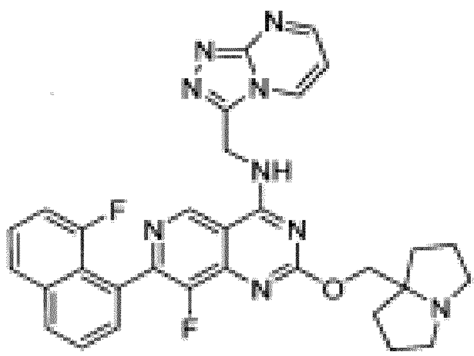,
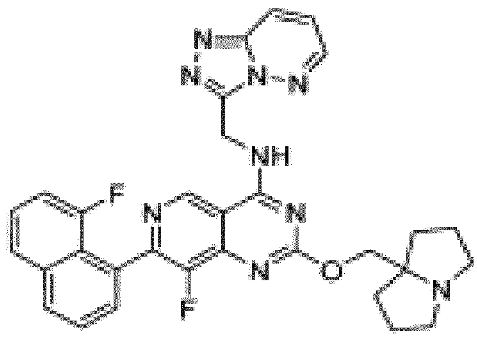,

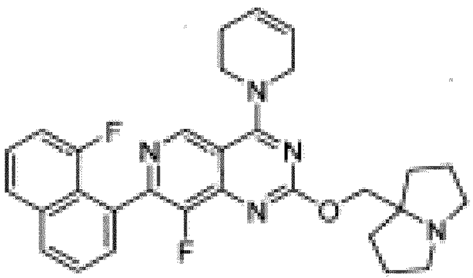,

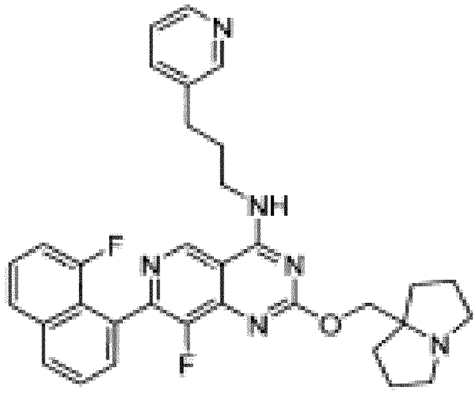,

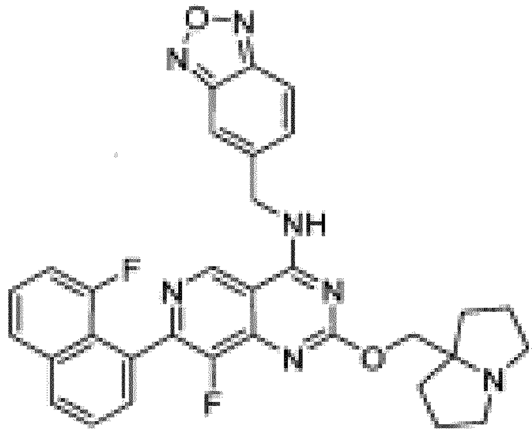,

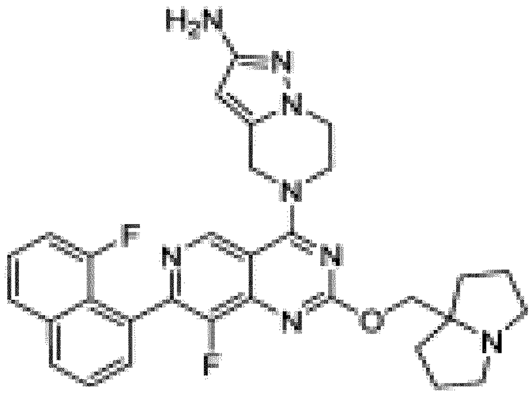,

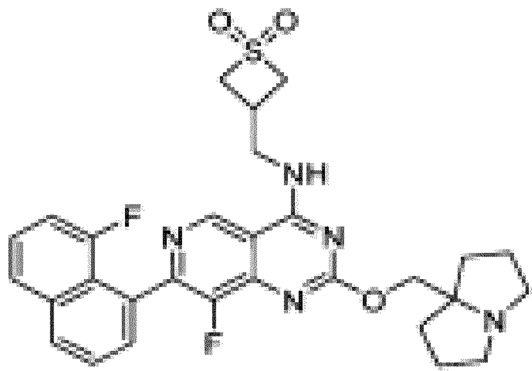,

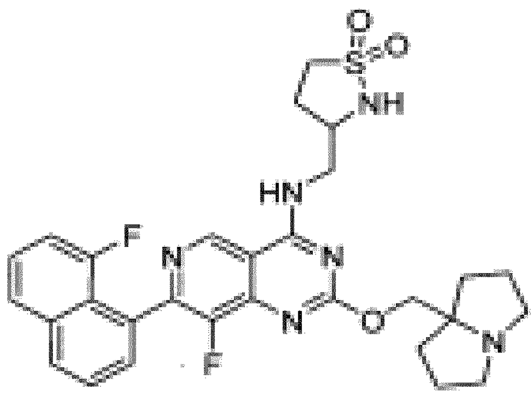,

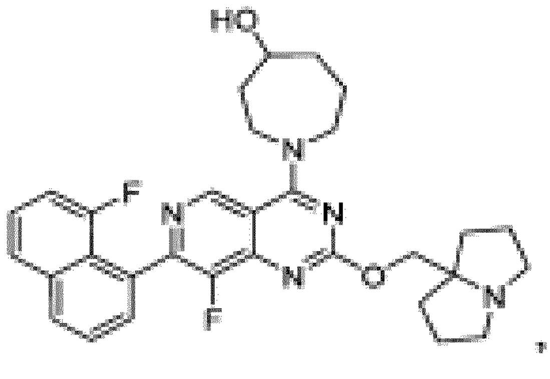,

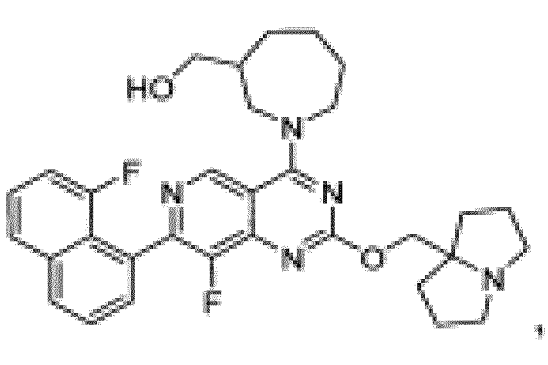,

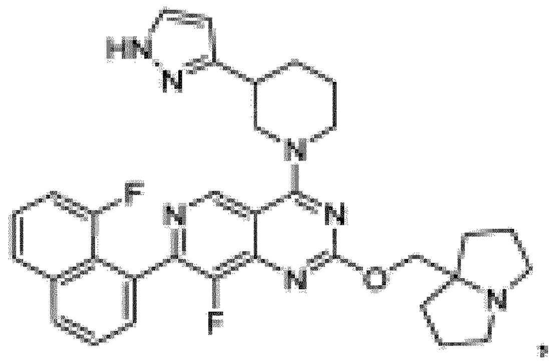,

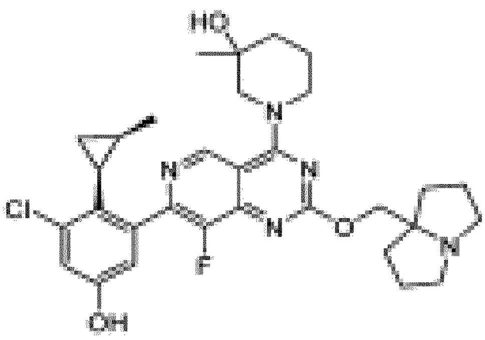,

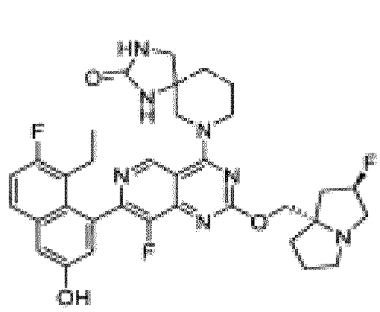,

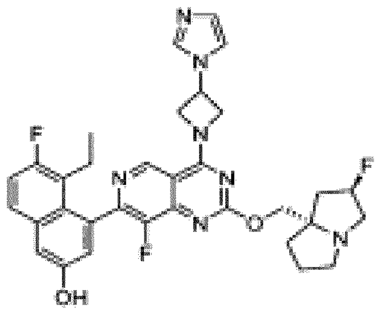,

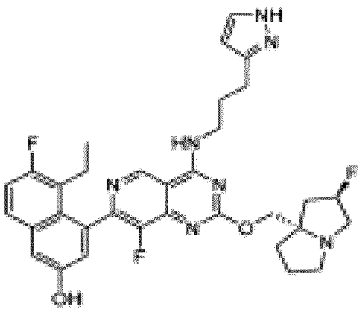,

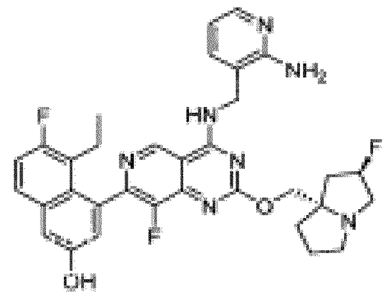,

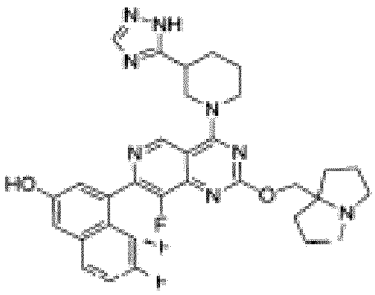,

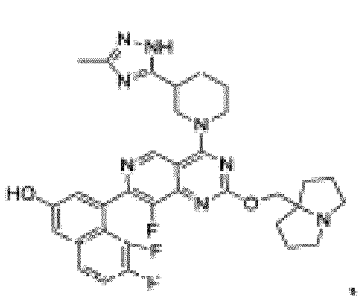,

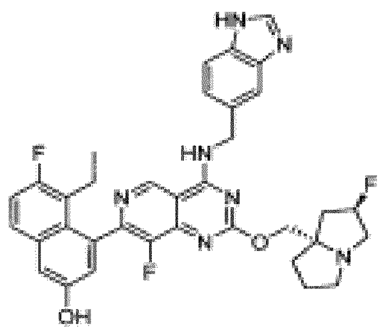,

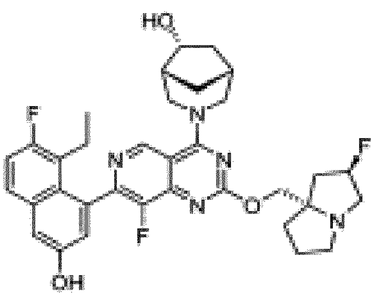,

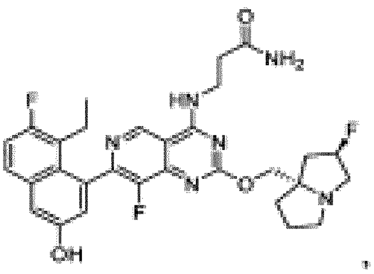,

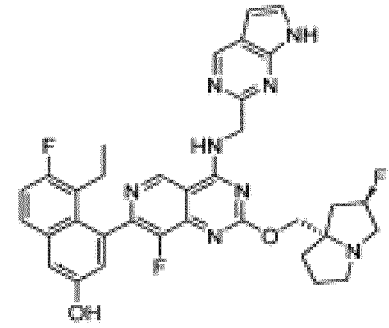,
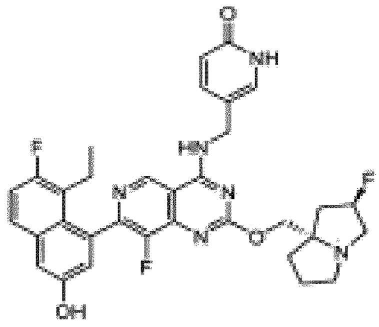,

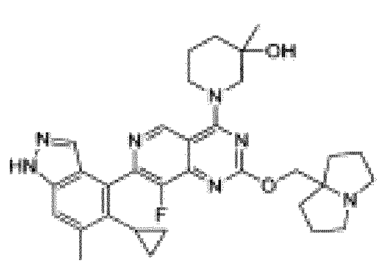,
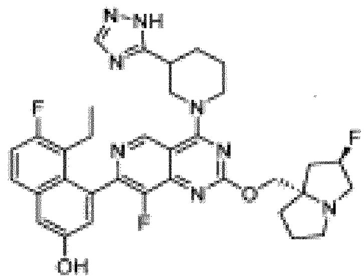,

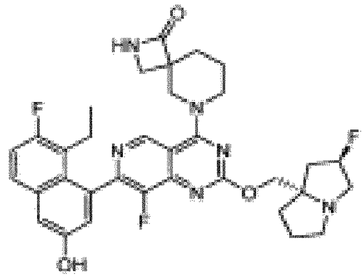,
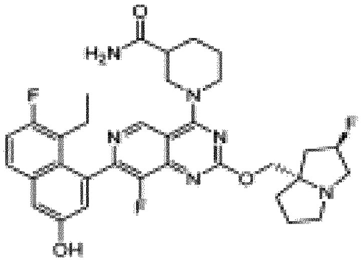,
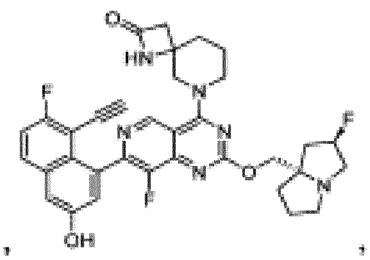,

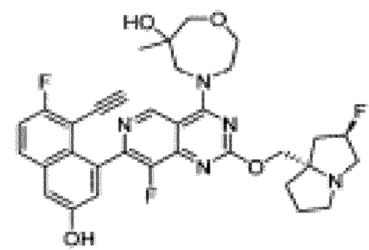,
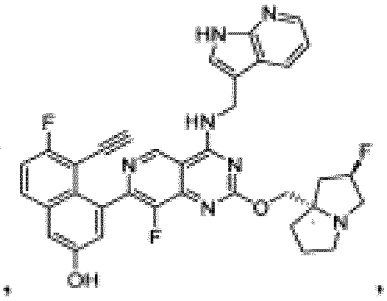,
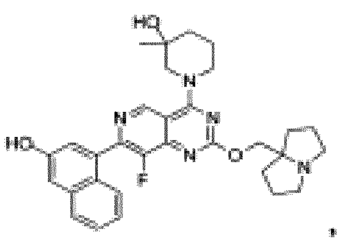,

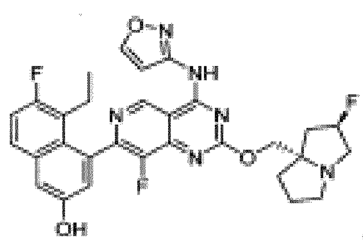,
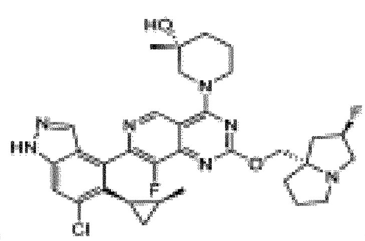,
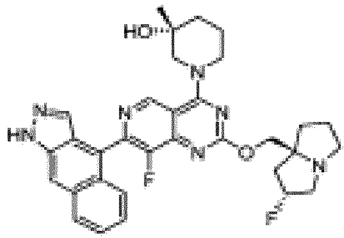,

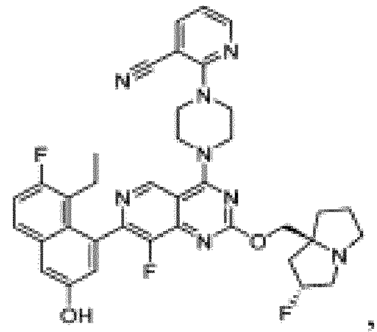, 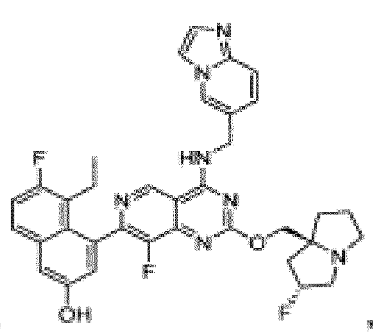, 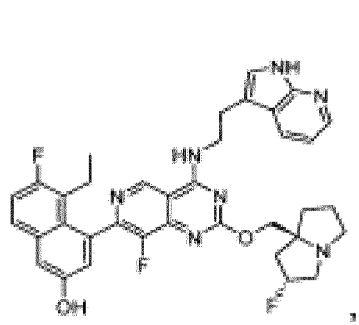,

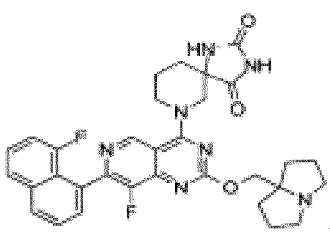, 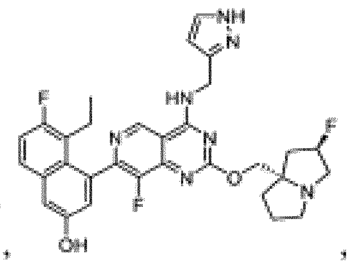, 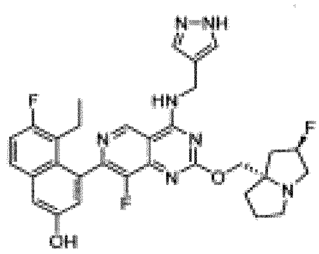,

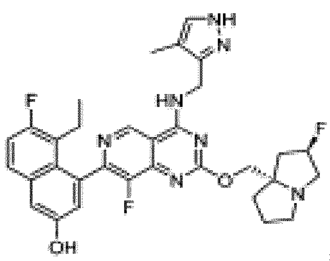, 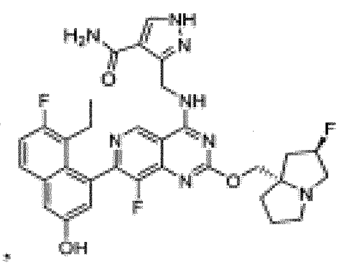, 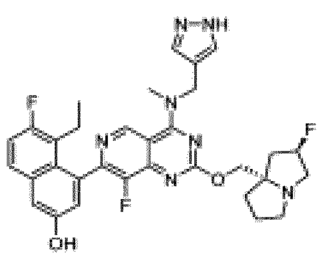,

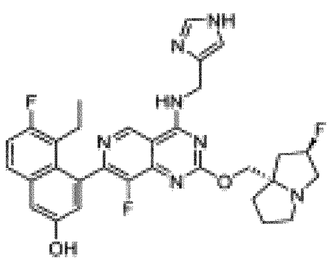, 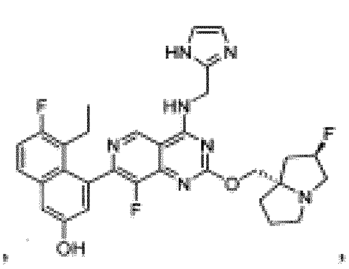, 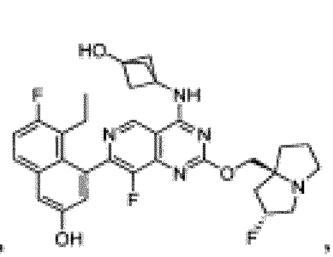,

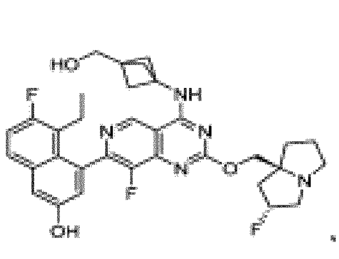, 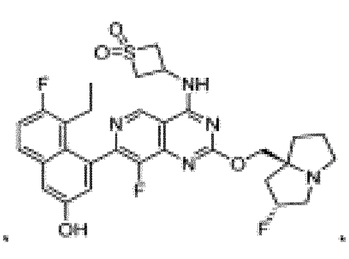, 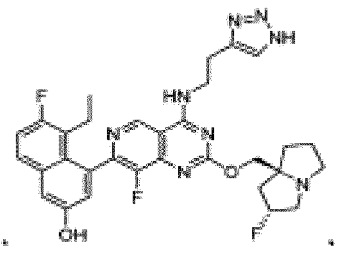,

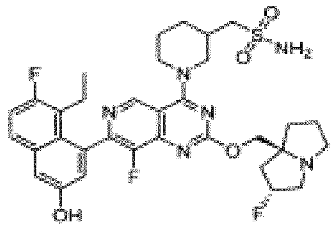,
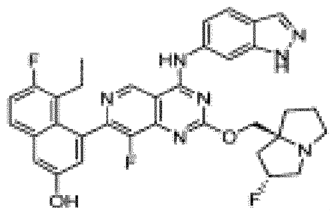,
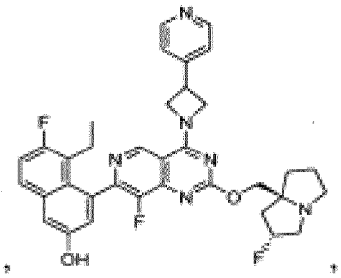,
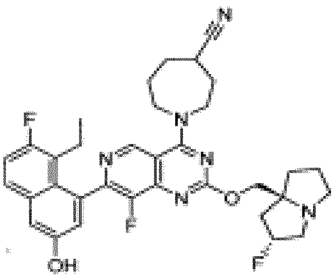,
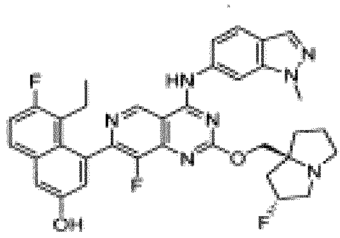,
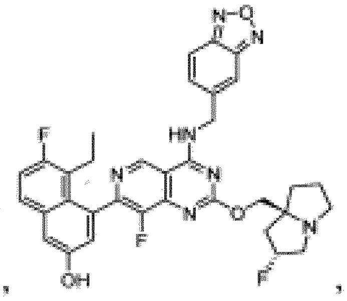,
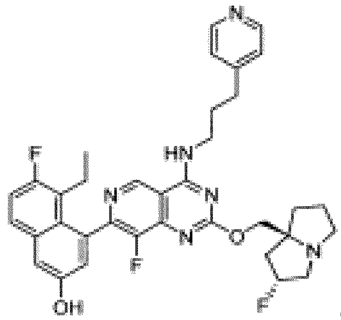,
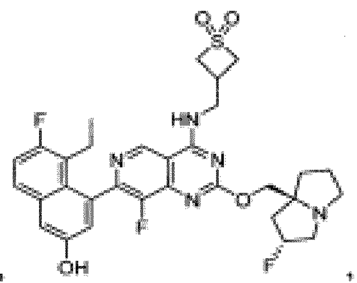,
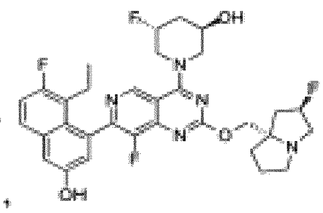,
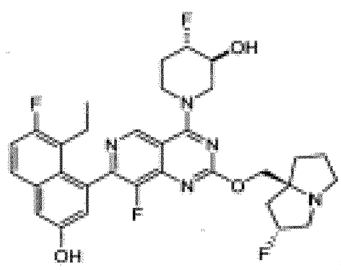,
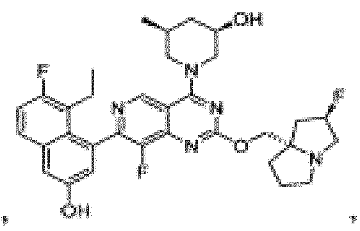,
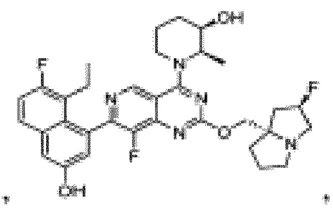,
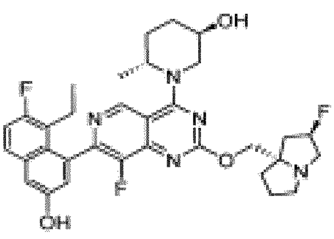,
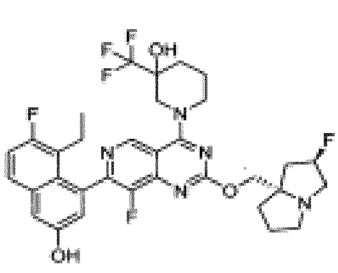,
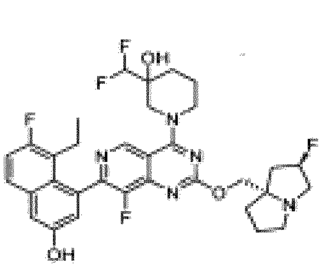,

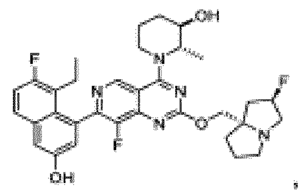 , 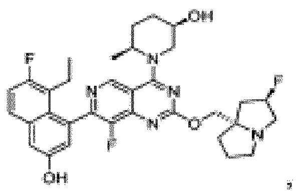 , 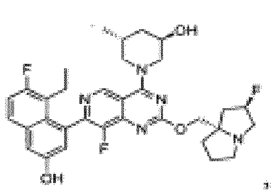 ,

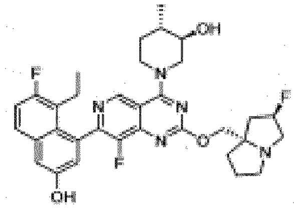 , 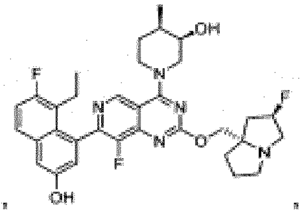 , 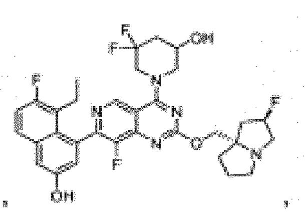 ,

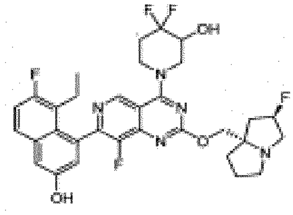 , 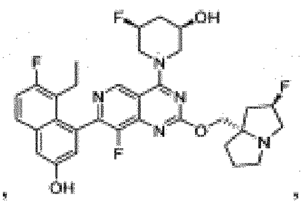 , 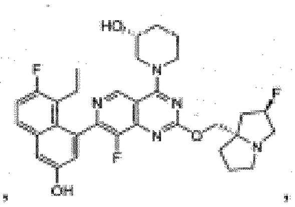 ,

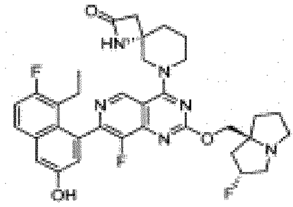 , 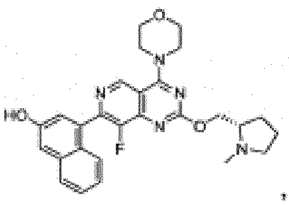 , 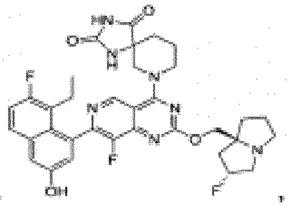 ,

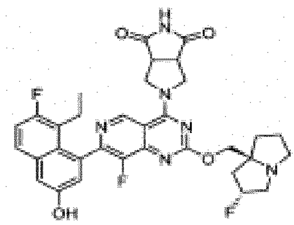 , 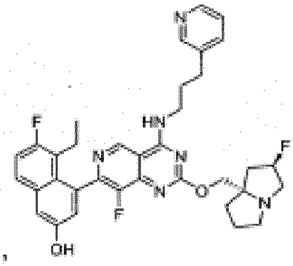 , 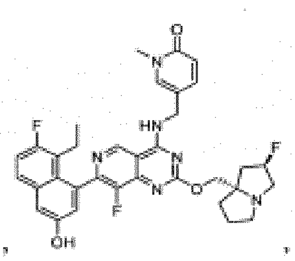 ,

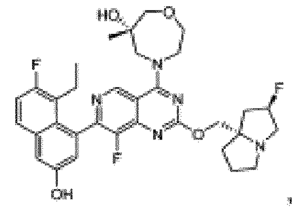 , 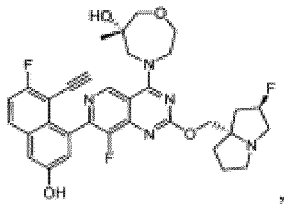 ,

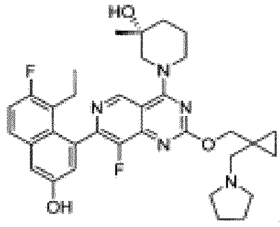 , 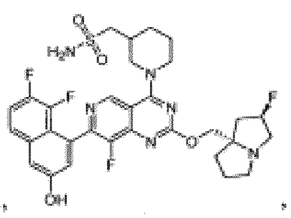 , 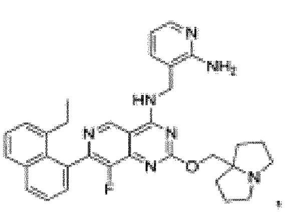 ,

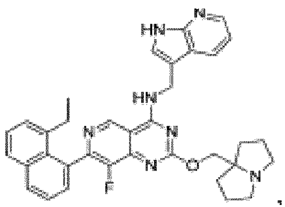,
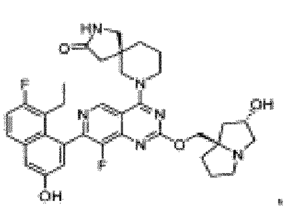,

In a certain embodiment, the nitrogen-containing heterocyclic compound of formula I is not any one of the following compounds:

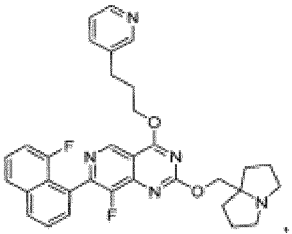,
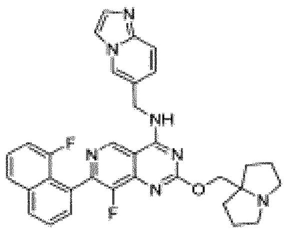,

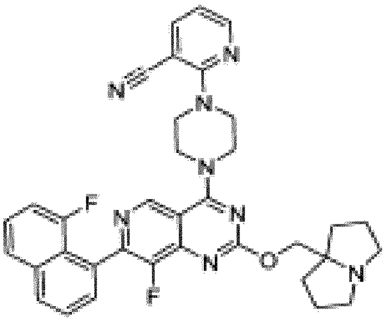,
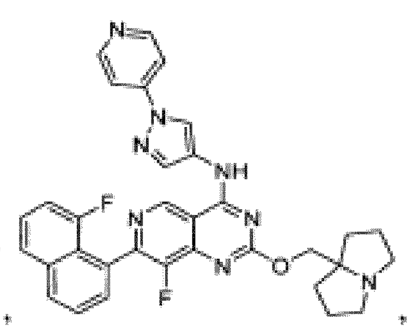,

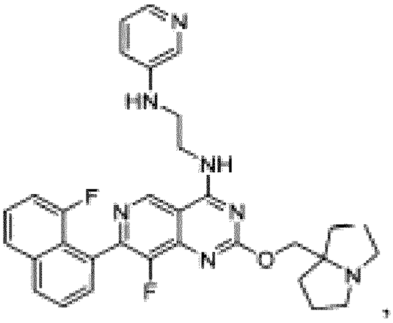,
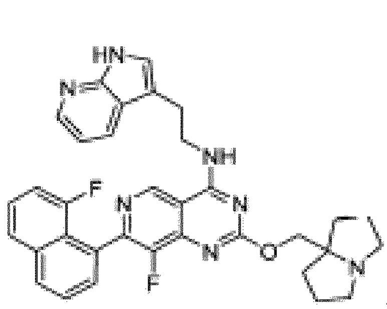,

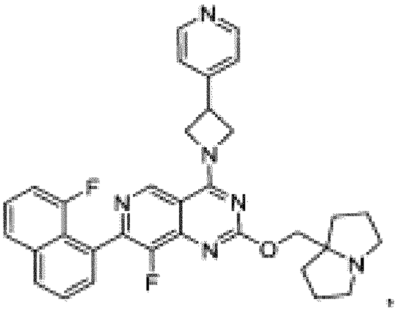,
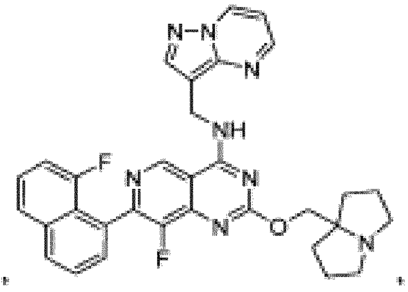,

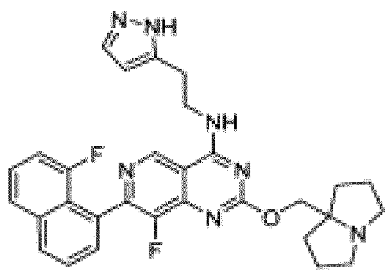 , 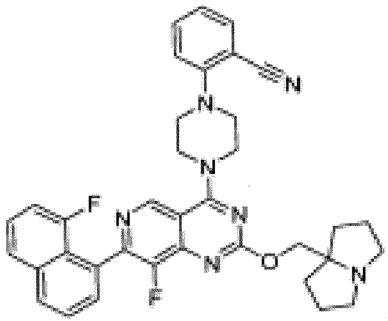 ,

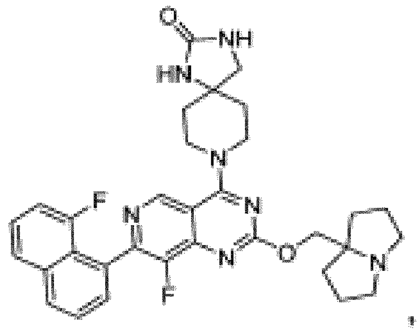 , 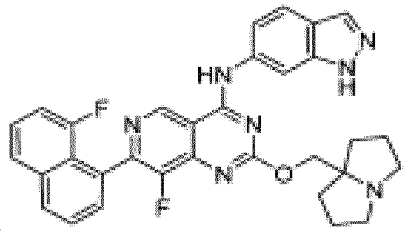 ,

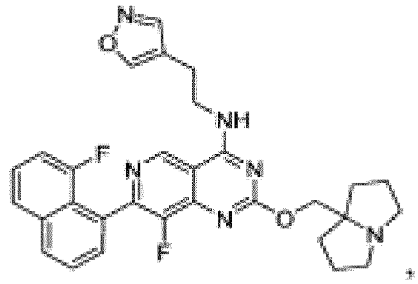 , 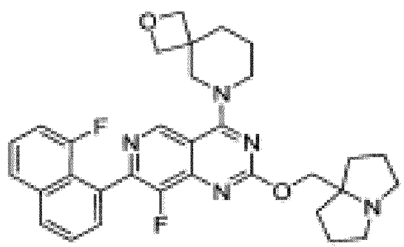 ,

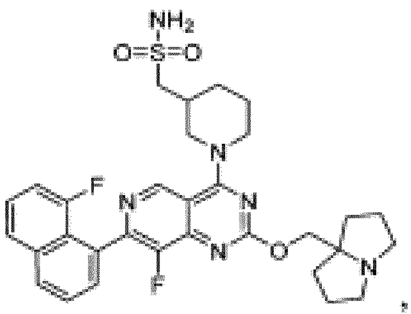 , 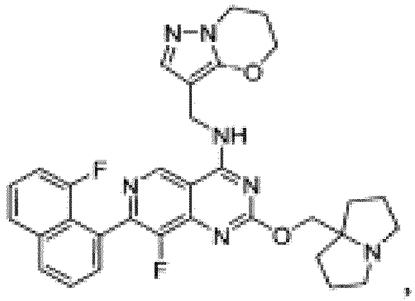 ,

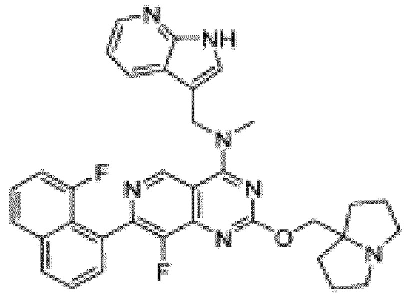 , 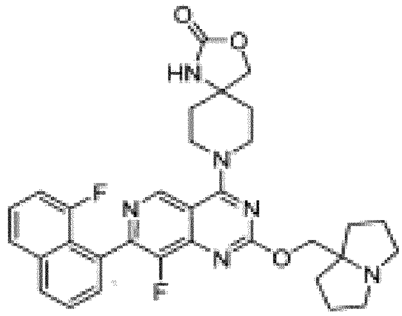 ,

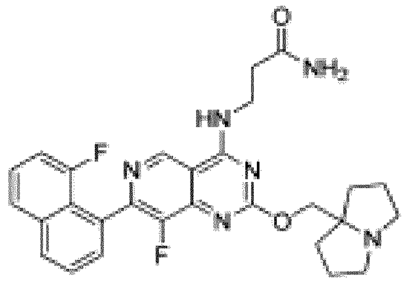,
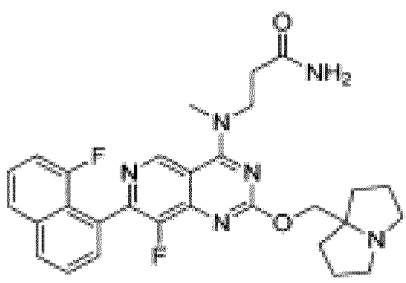,

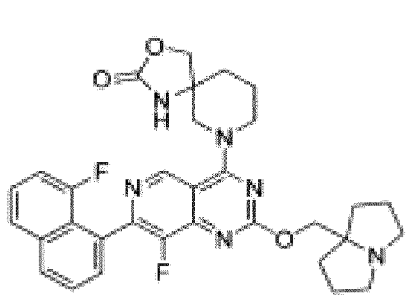,
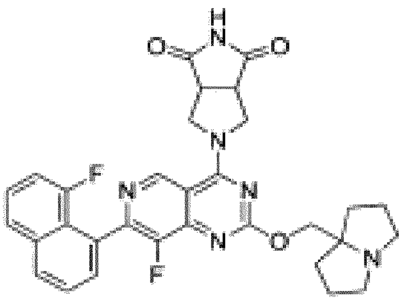,

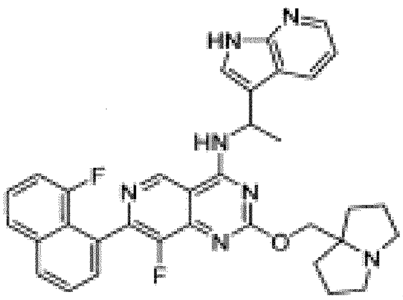,
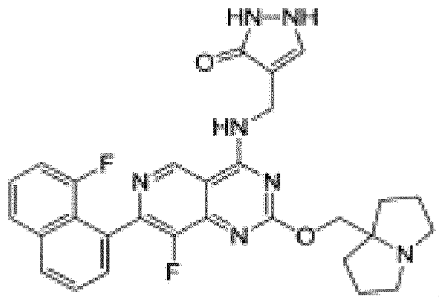,

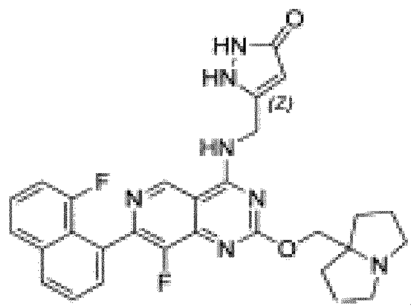,
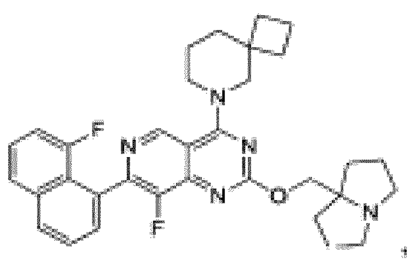,

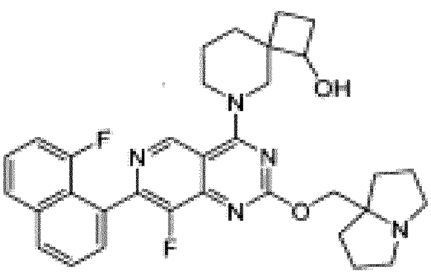,
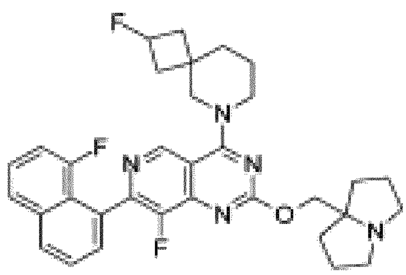,

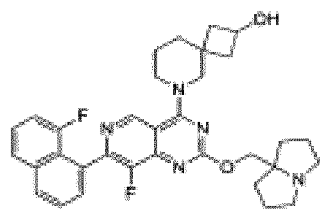,

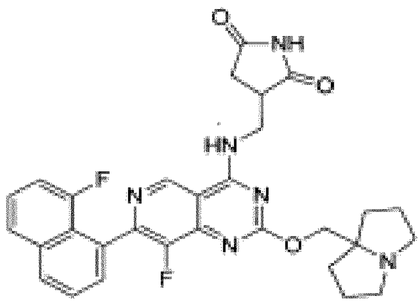,

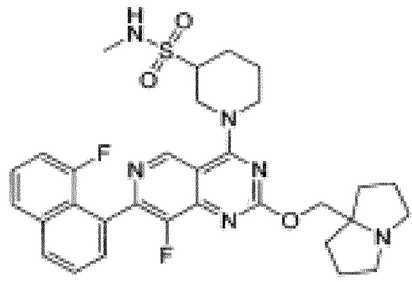,

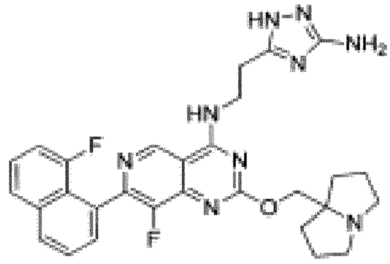,

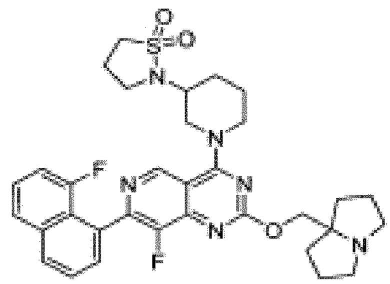,

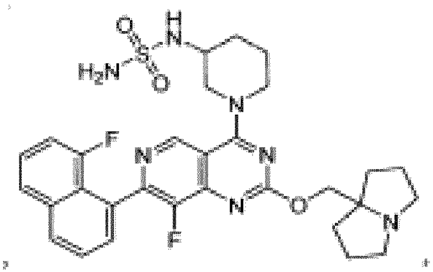,

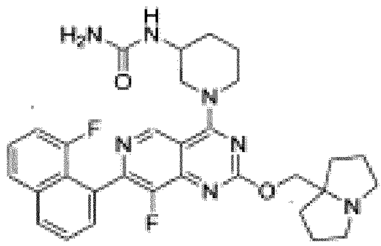,

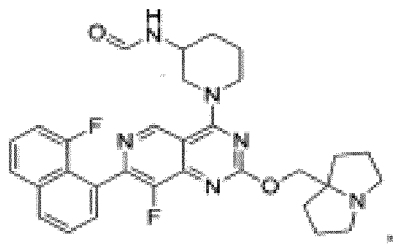,

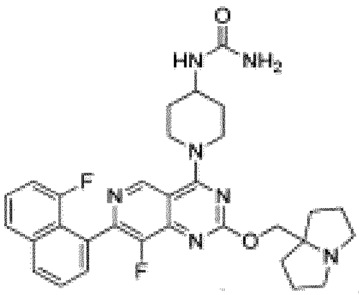,

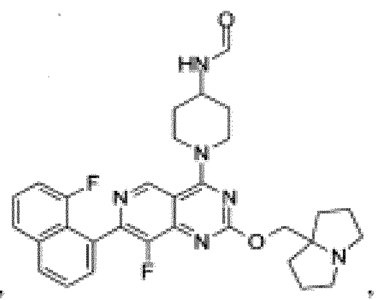,

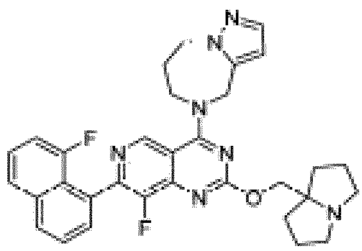,
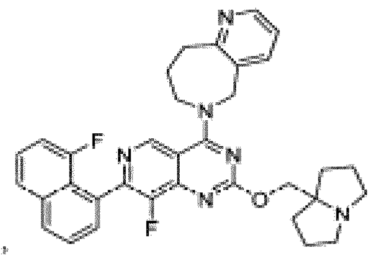,

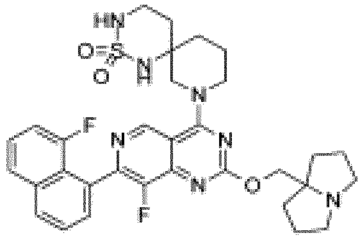,
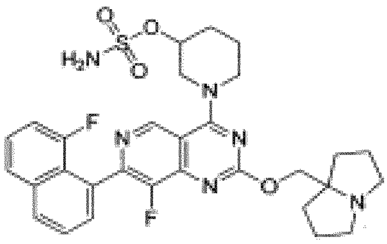,

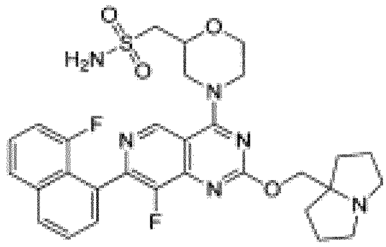,
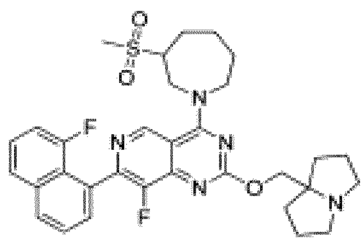,

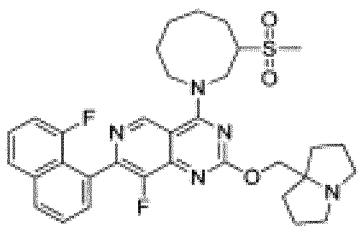,
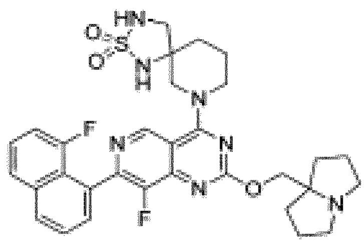,

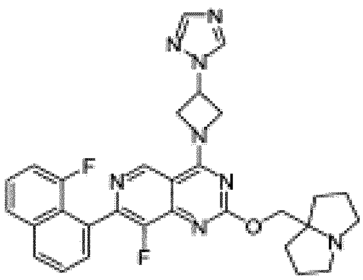,
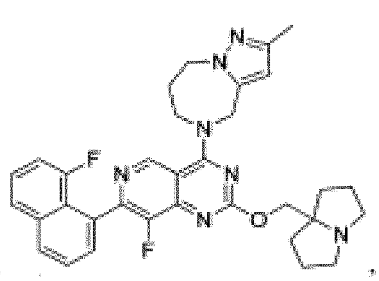,

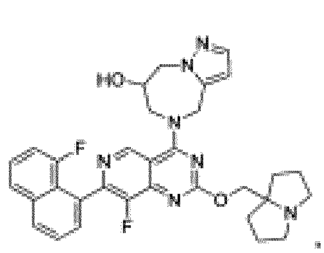,
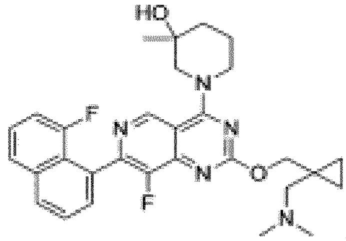,
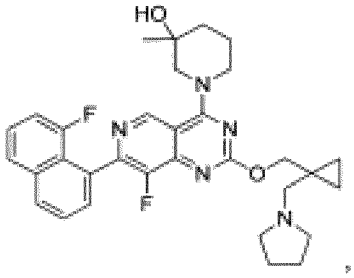,

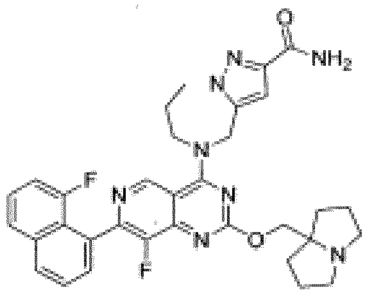,
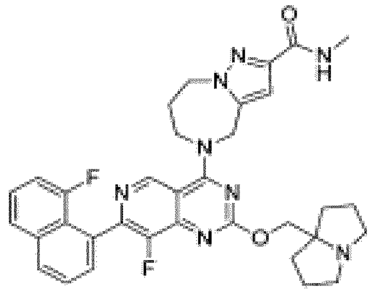,
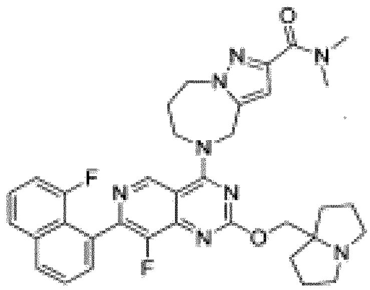,
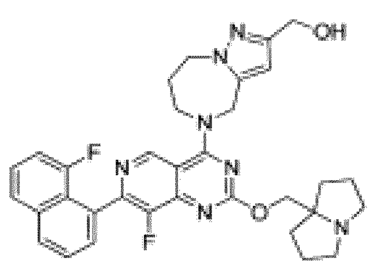,
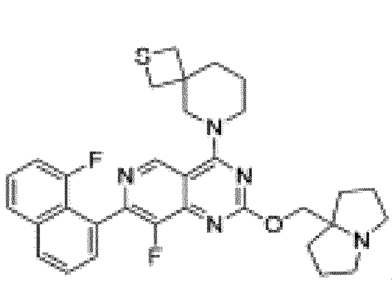,
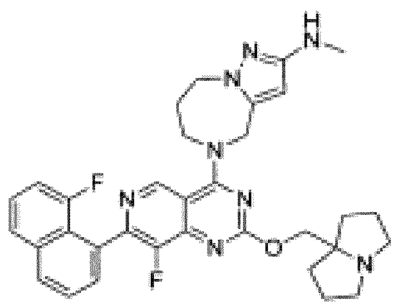,
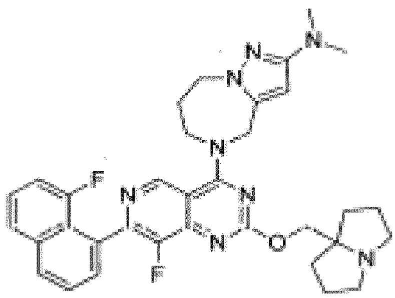,
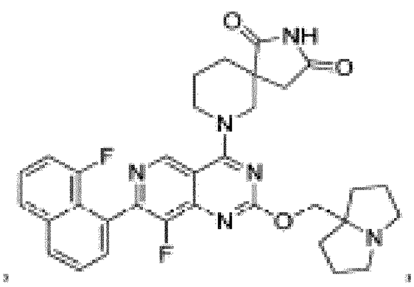,
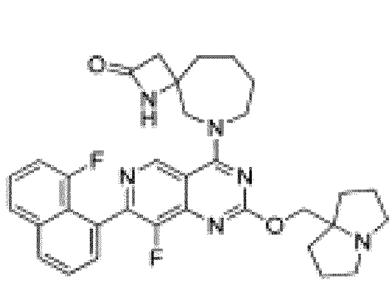,
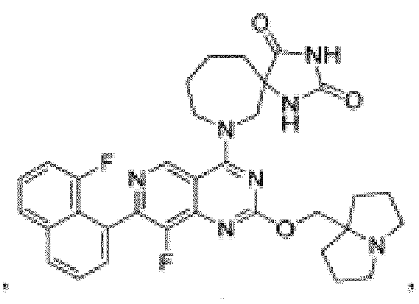,

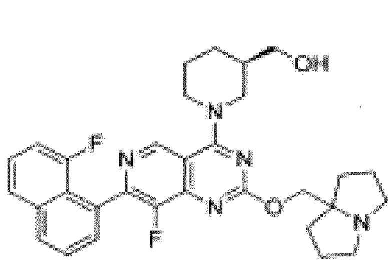 , 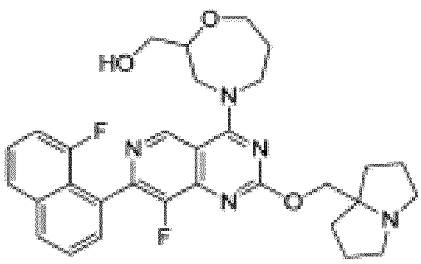 ,

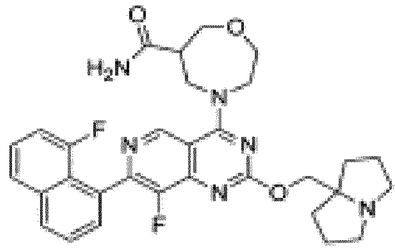 , 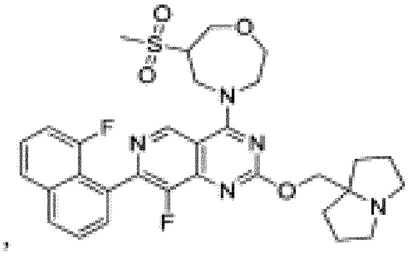 ,

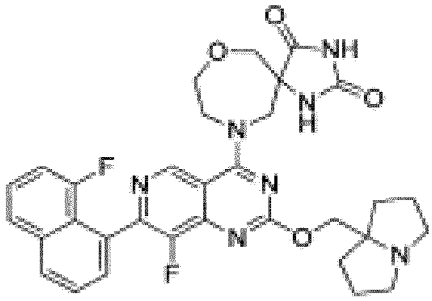 , 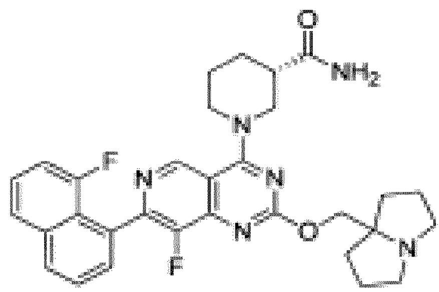 ,

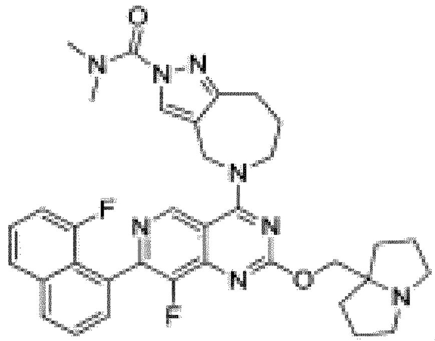 , 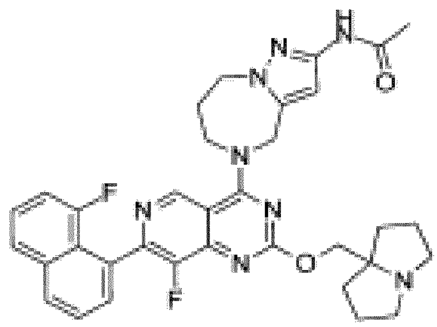 ,

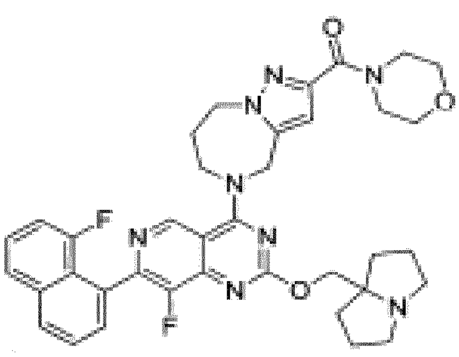 , 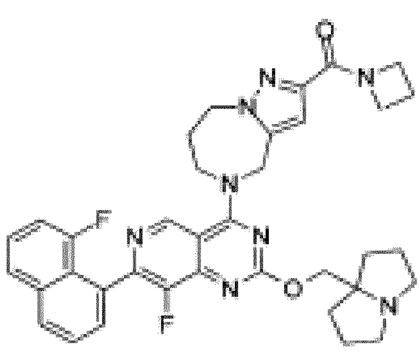 ,

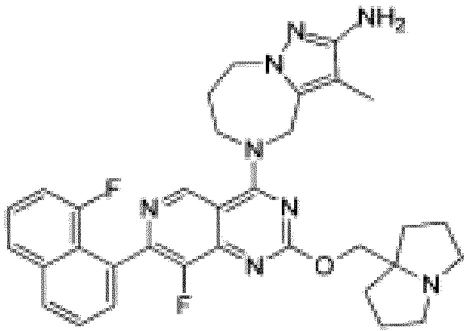
,
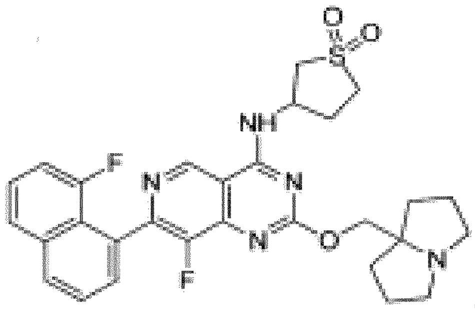
,

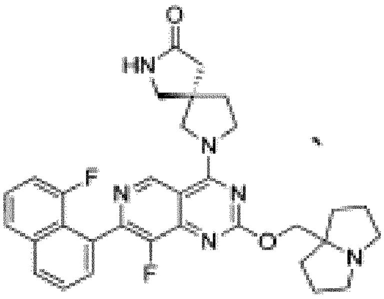
,
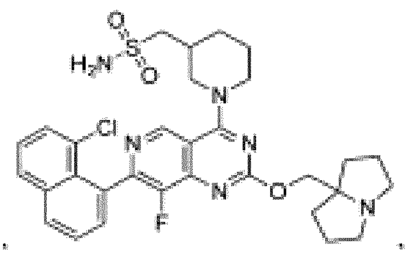
,

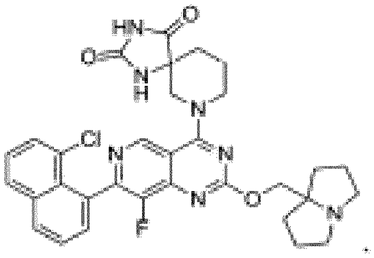
,
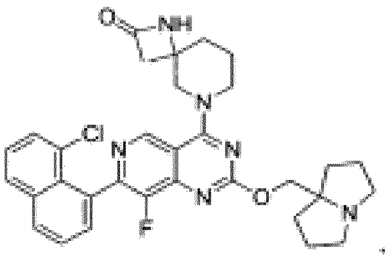
,

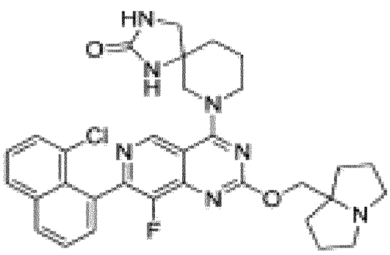
,
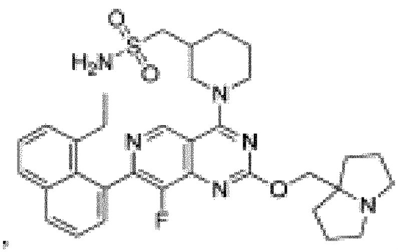
,

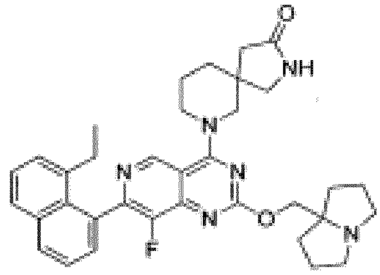
,
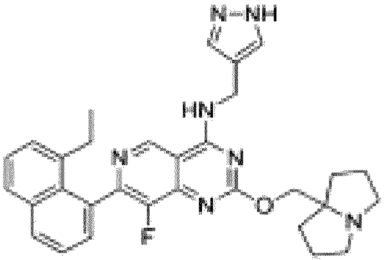
,

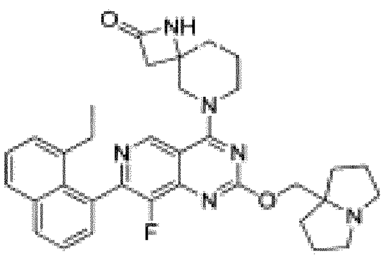,
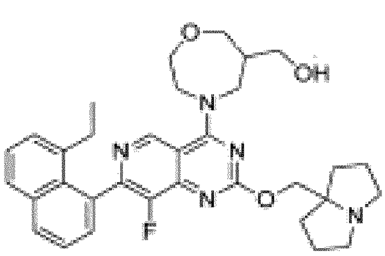,

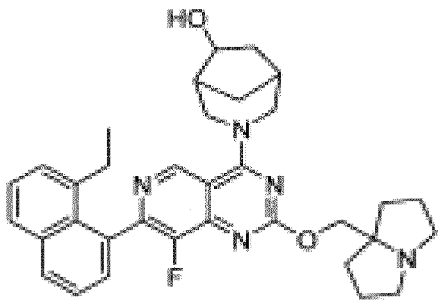,
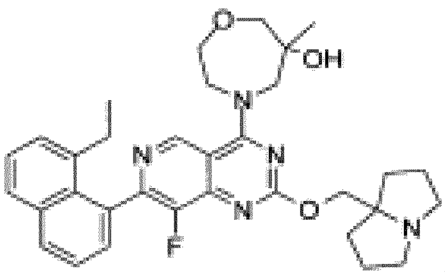,

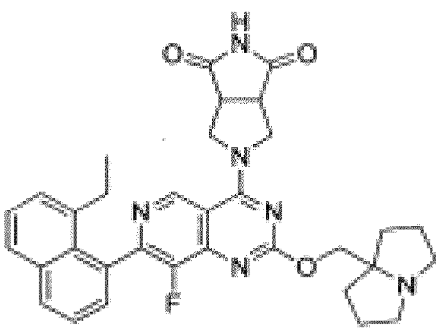,
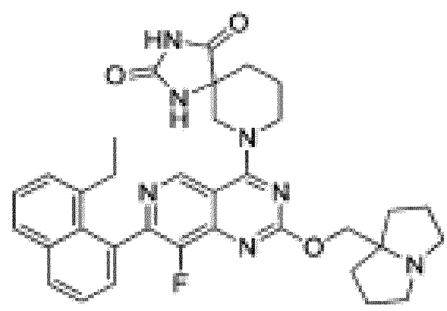,

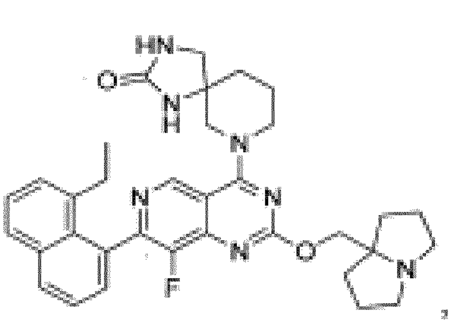,
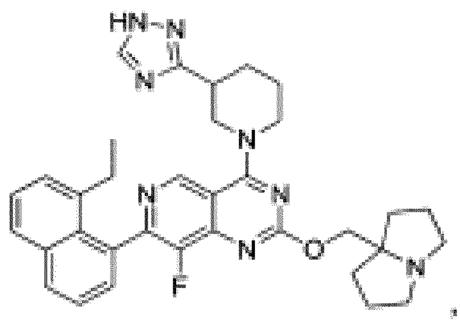,

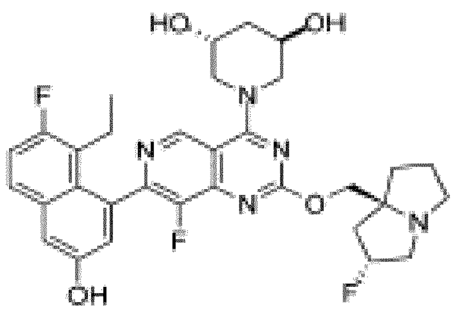,
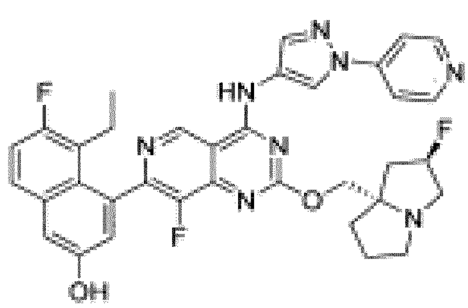,

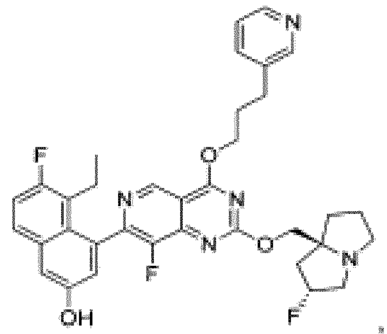,
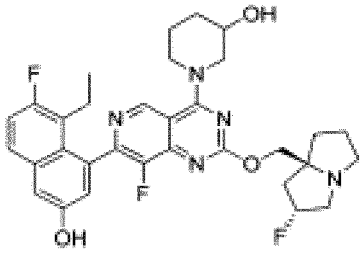,

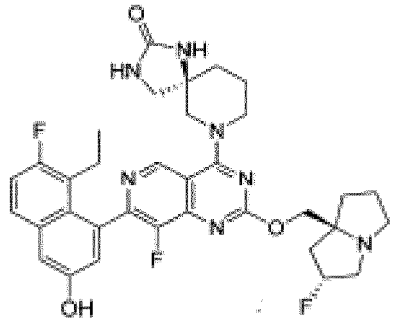,
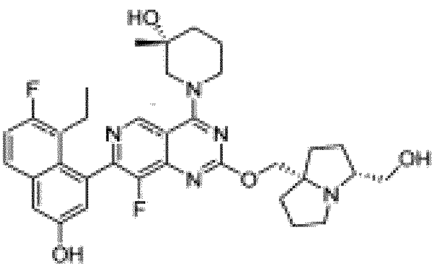,

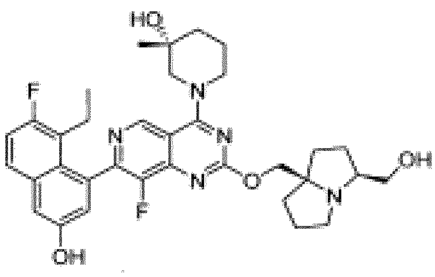,
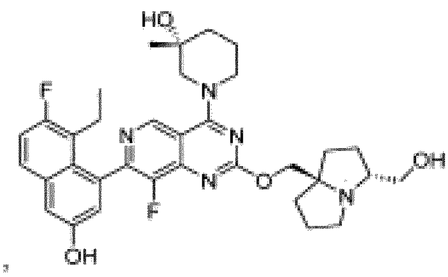,

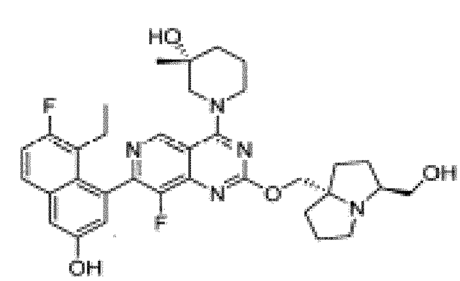,
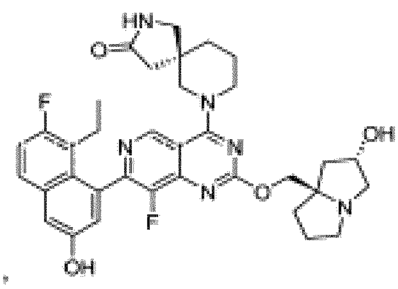,

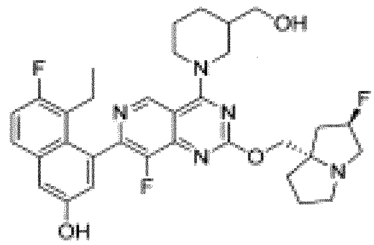,
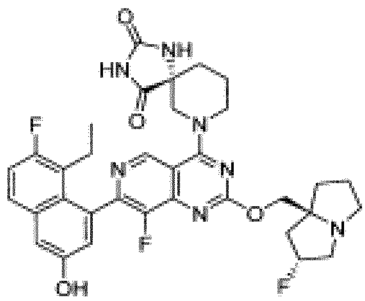,

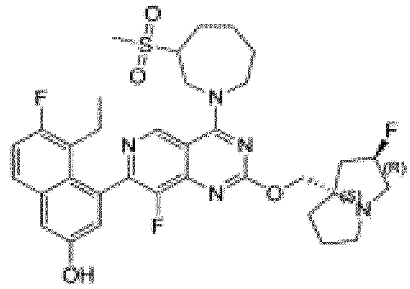 , 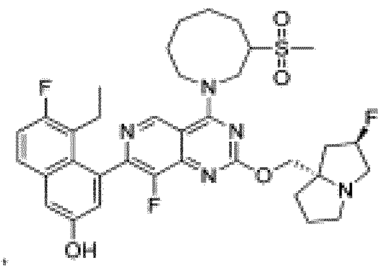 ,

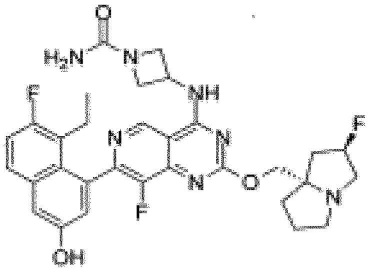 , 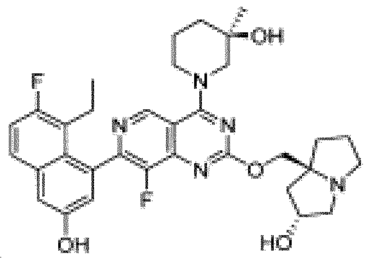 ,

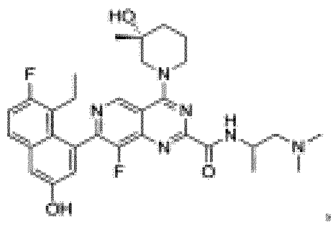 , 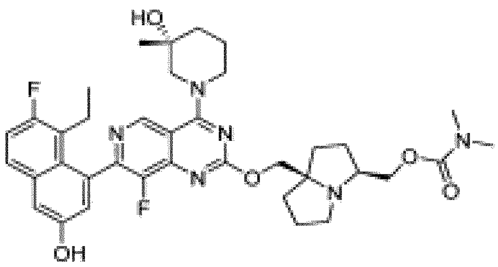 ,

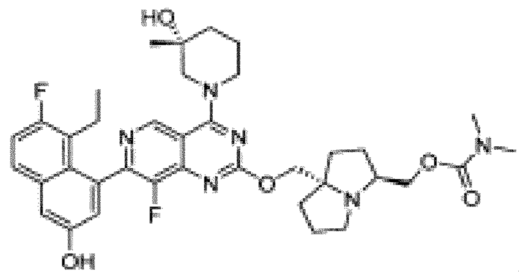 , 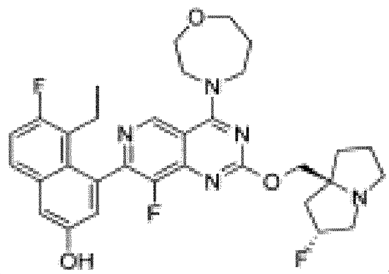 ,

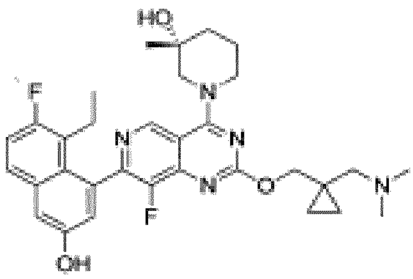 , 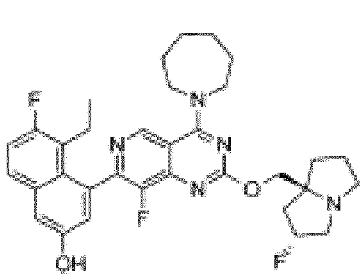 ,

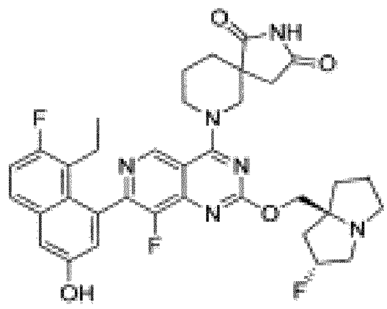,
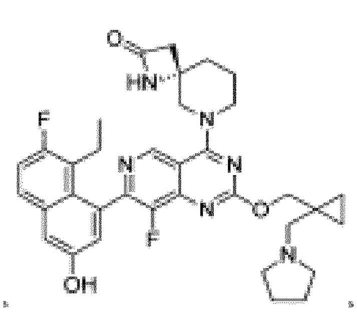,

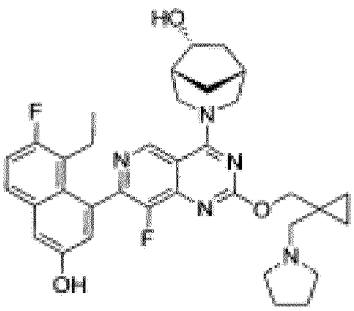,
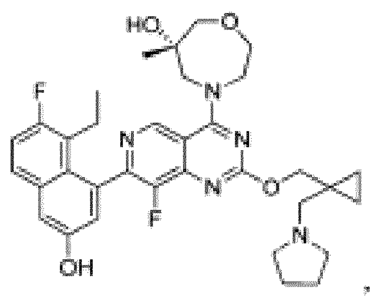,

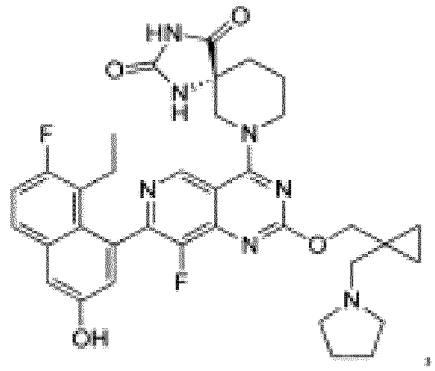,
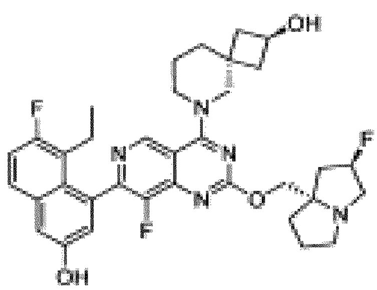,

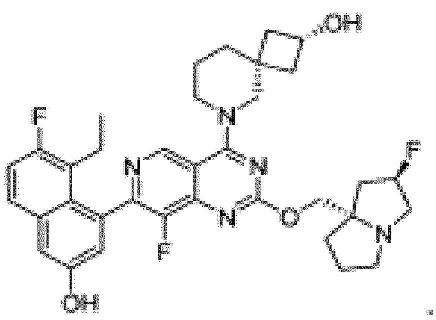,
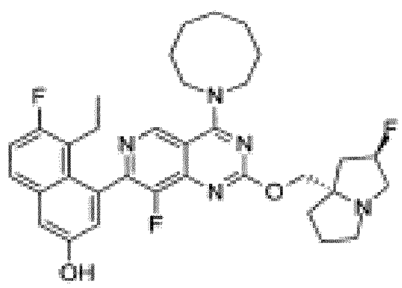,

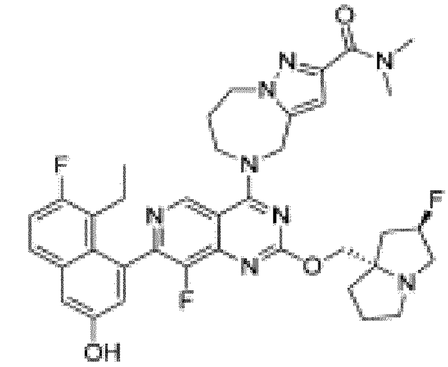
,
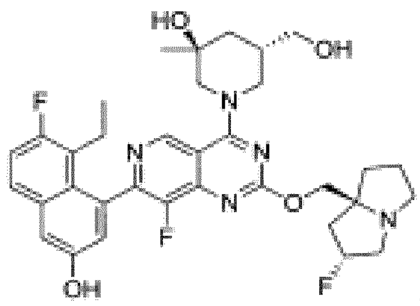
,
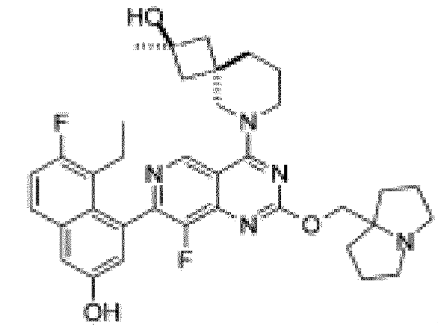
,
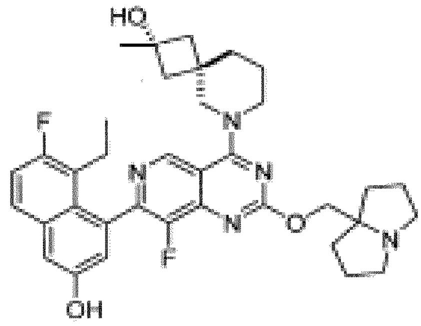
,
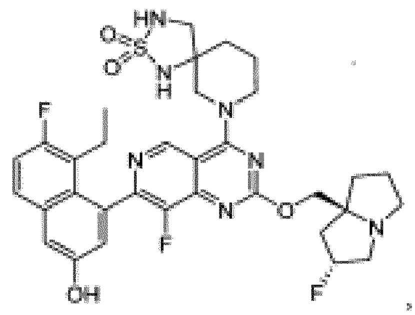
,
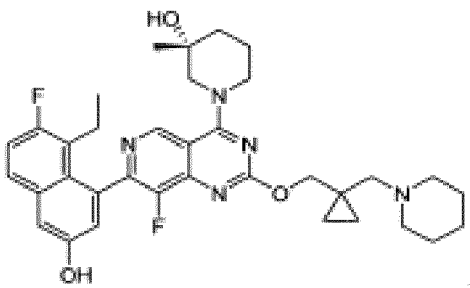
,
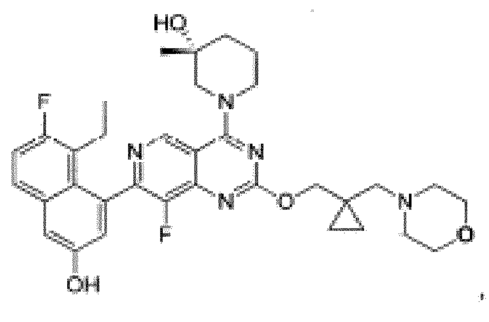
,
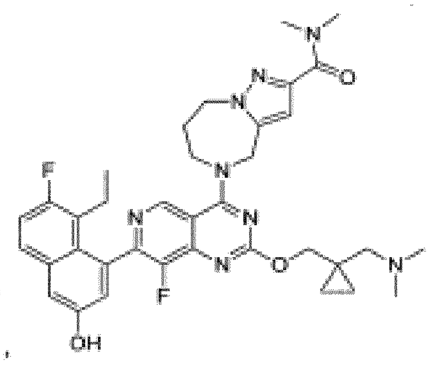
,

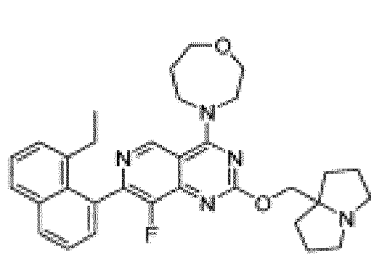,
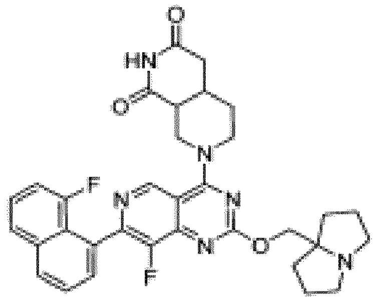,

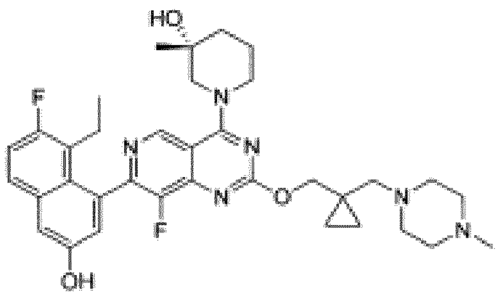,
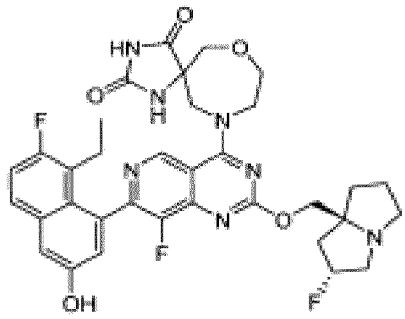,

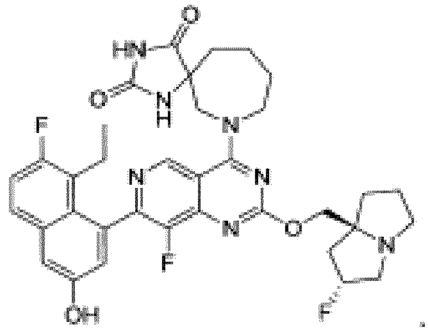,
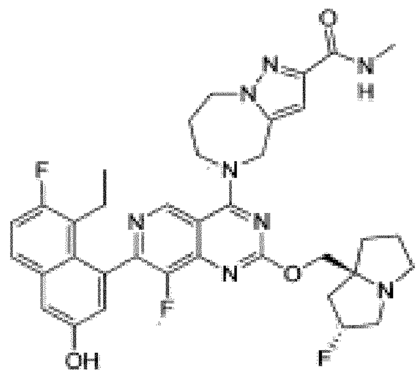,

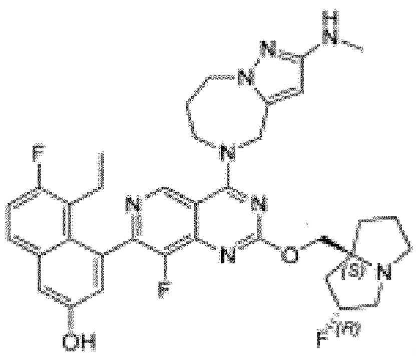,
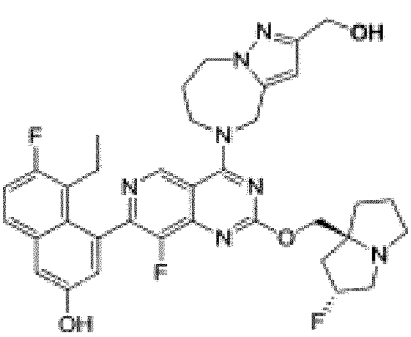,

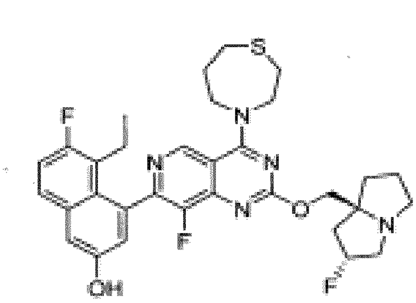,
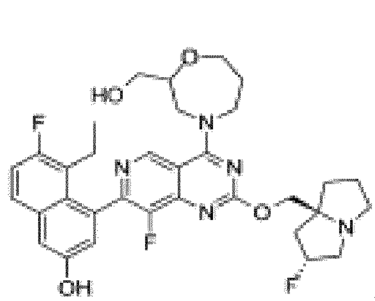,

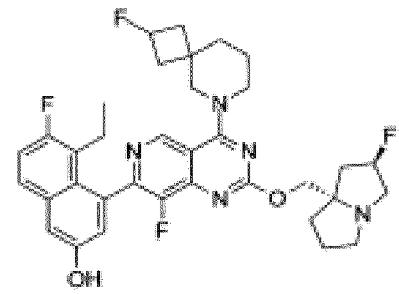 ,

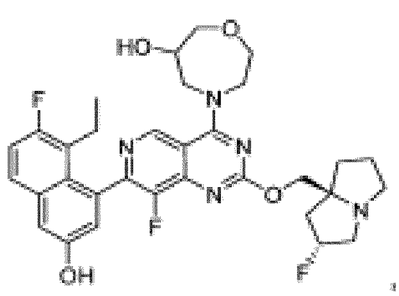 ,

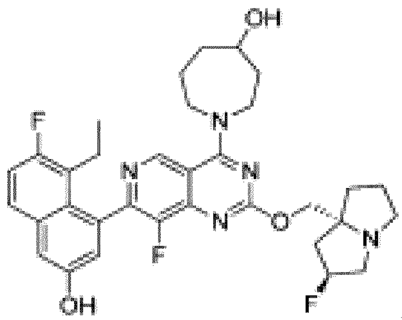 ,

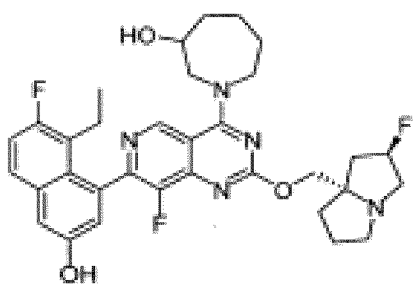 ,

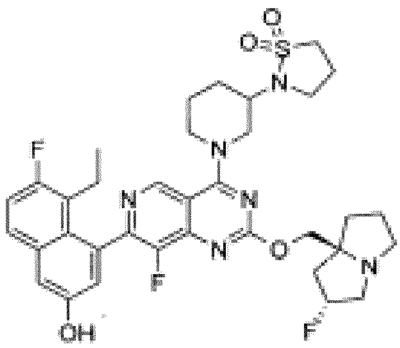 ,

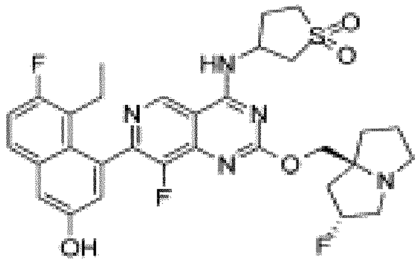 ,

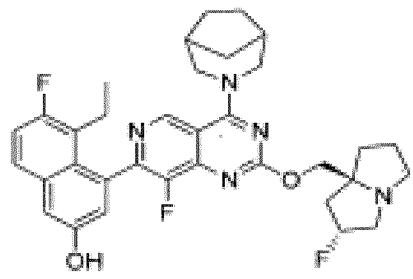 ,

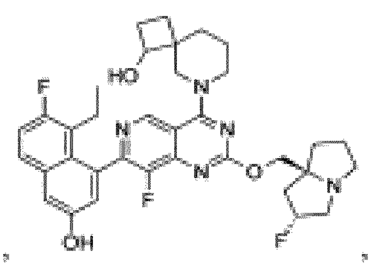 ,

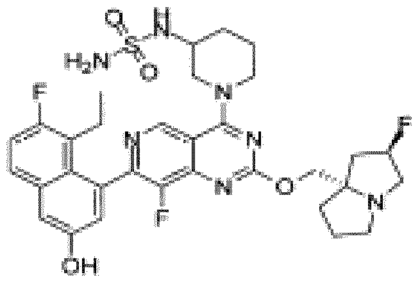
,
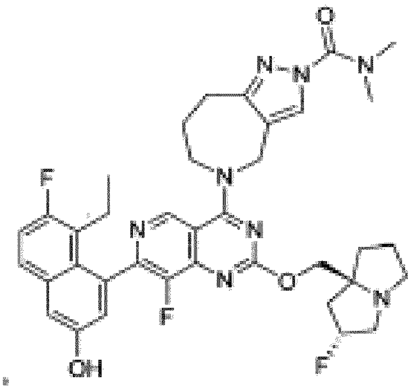
,

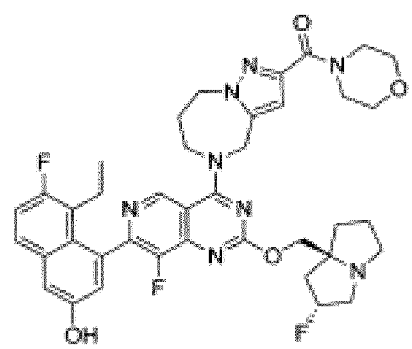
,
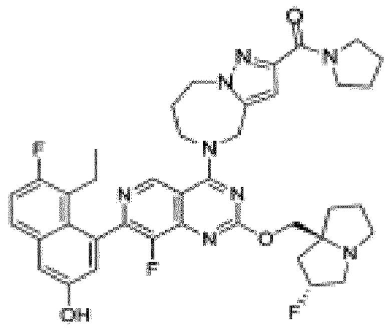
,

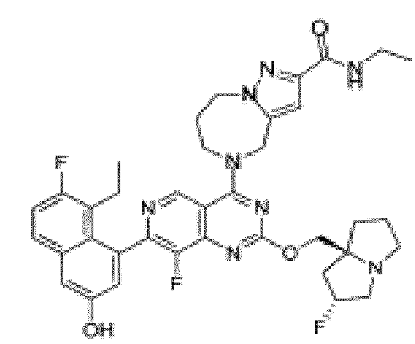
,
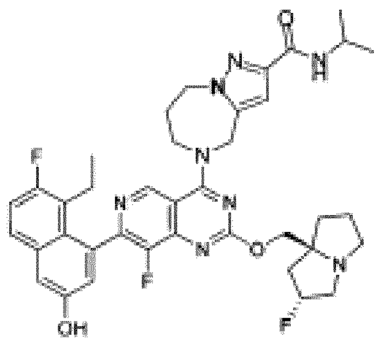
,

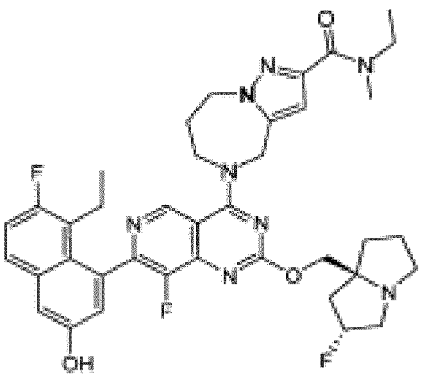
,
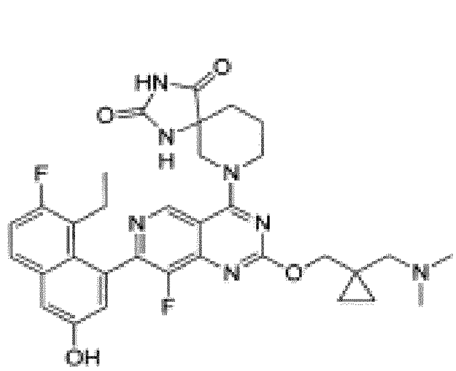
,

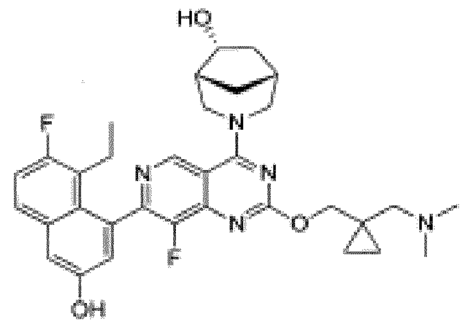,
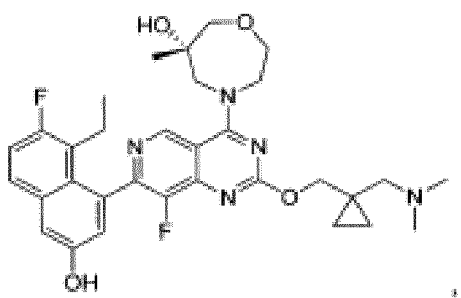,

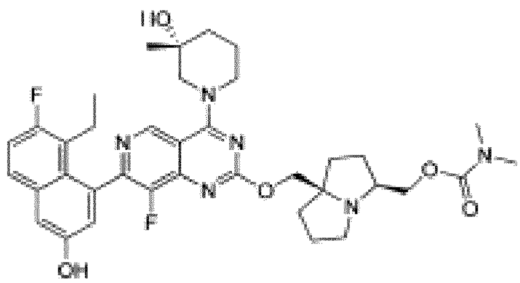,
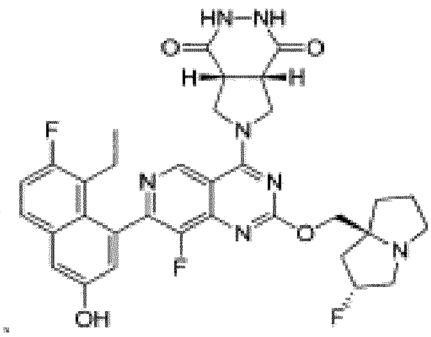,

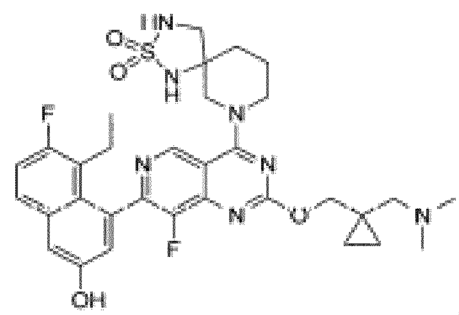,
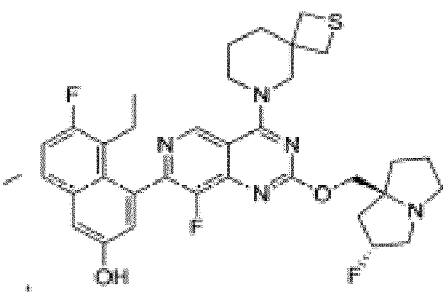,

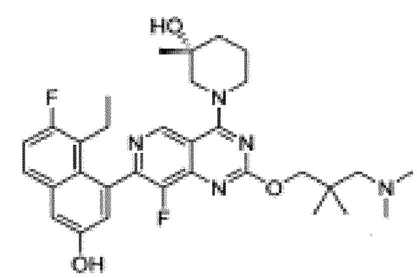,
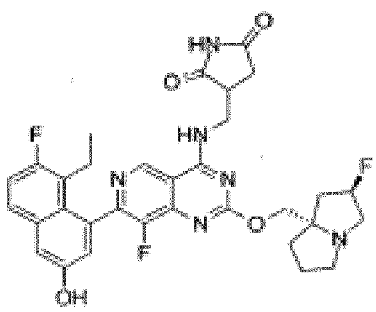,

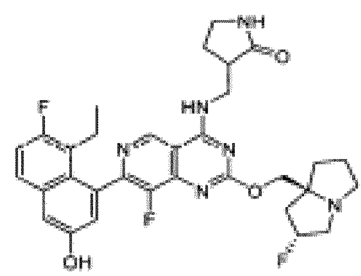,
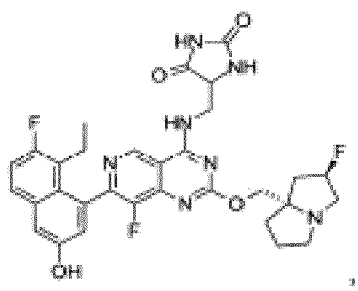,

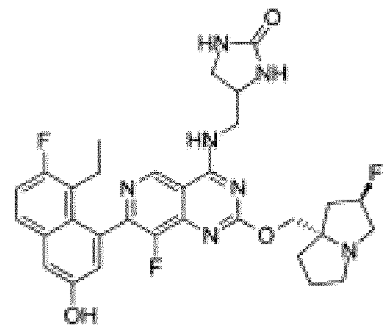
,
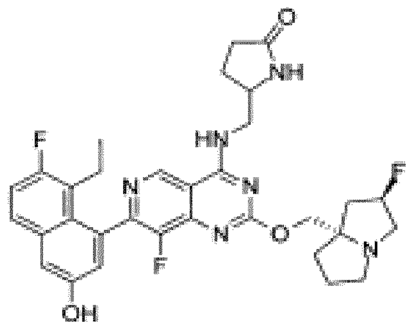
,

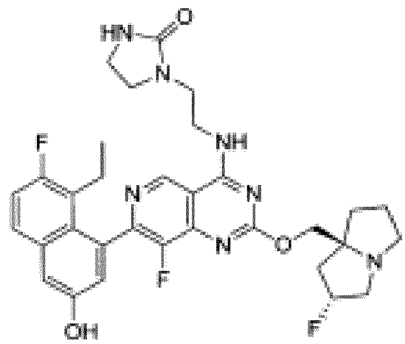
,
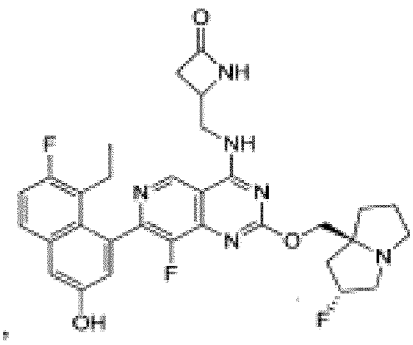
,

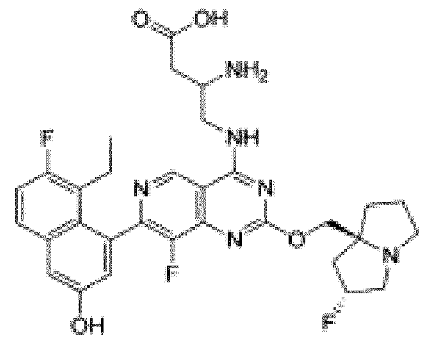
,
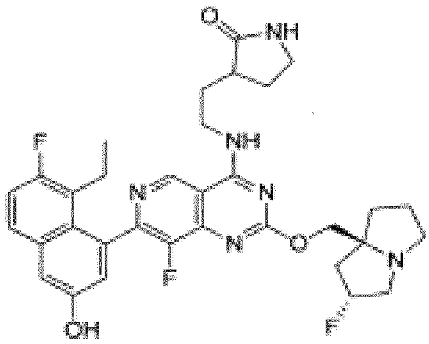
,

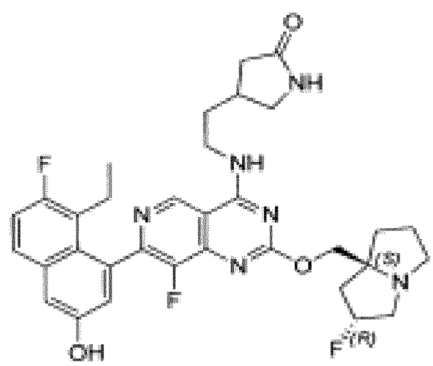
,
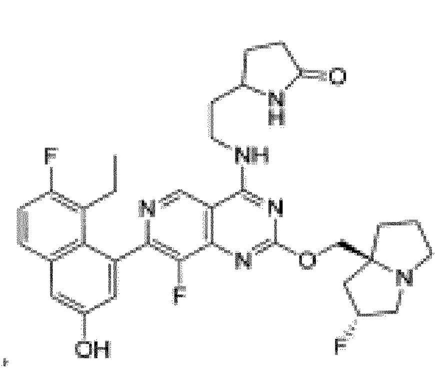
,

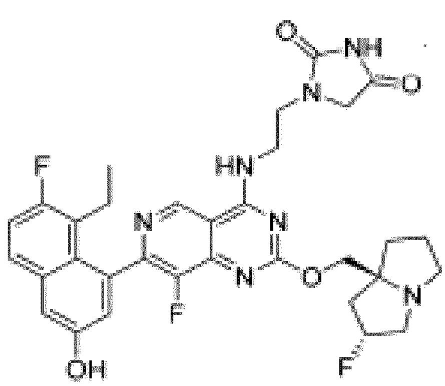
,
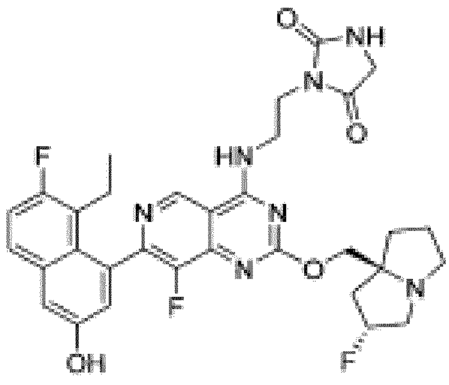
,

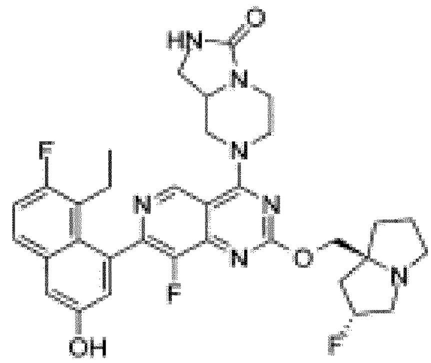
,
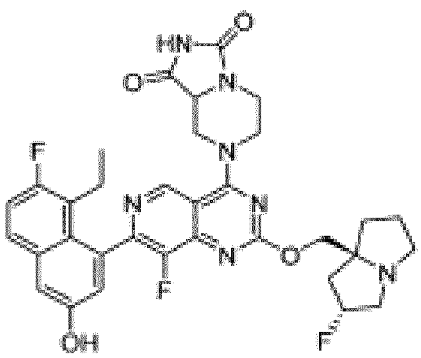
,

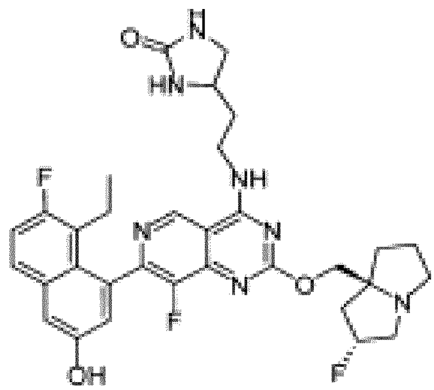
,
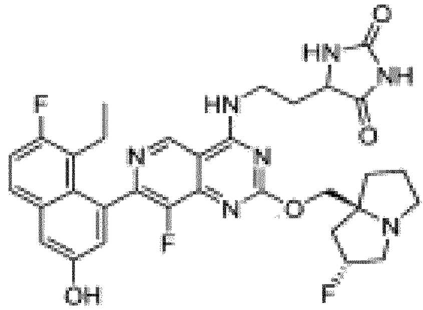
,

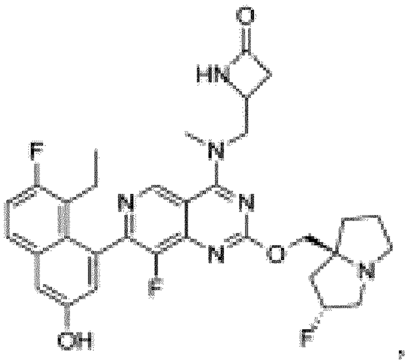
,
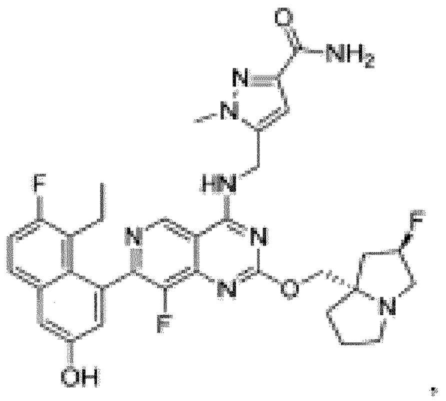
,

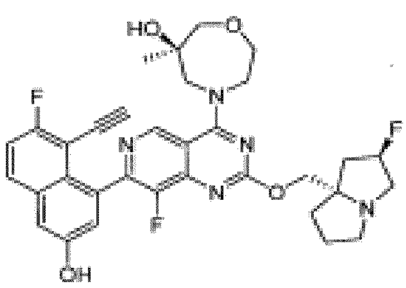 , 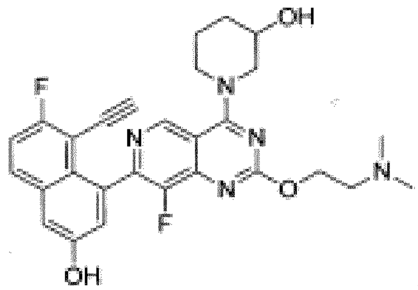 ,

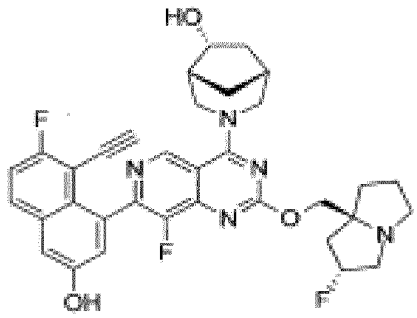 , 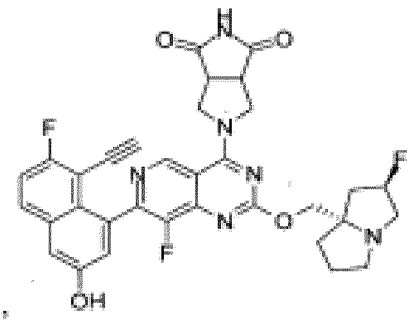 ,

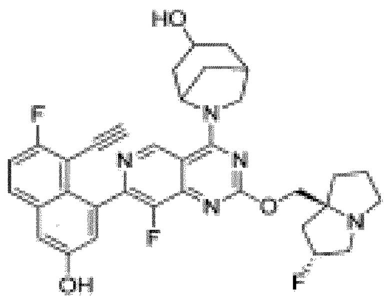 , 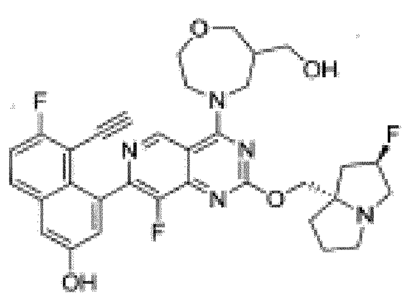 ,

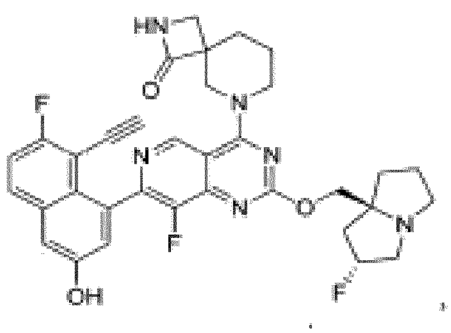 , 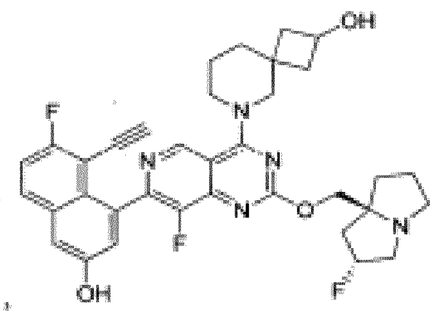 ,

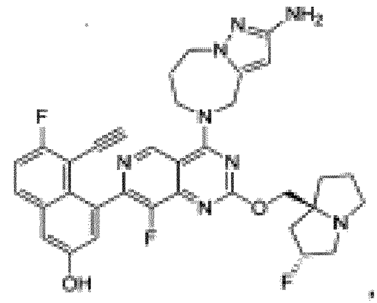 , 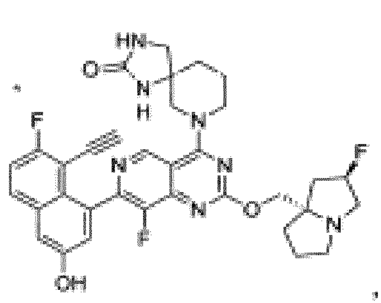 ,

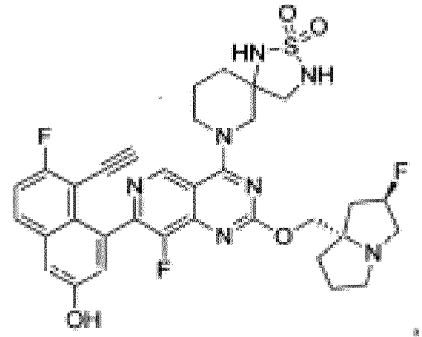 , 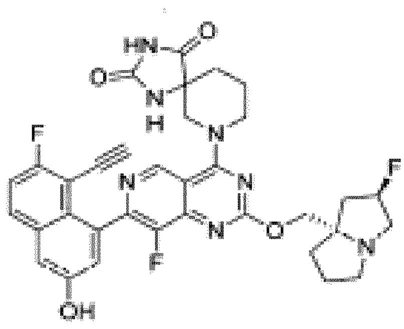 ,

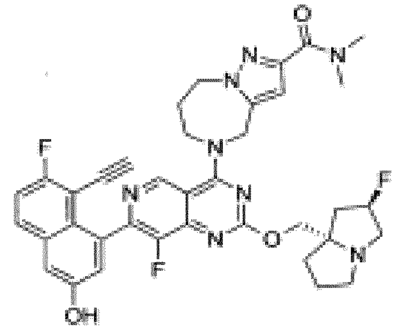 , 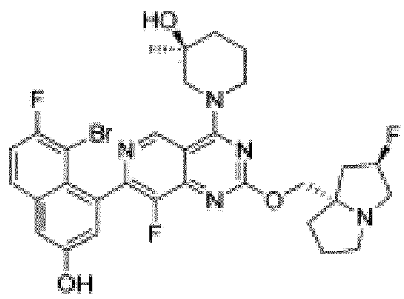 ,

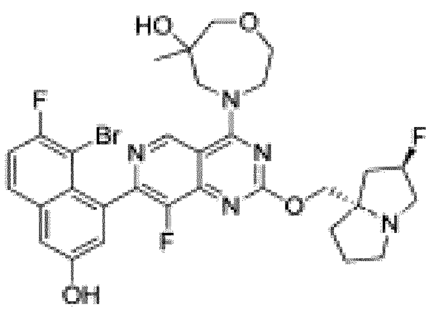 , 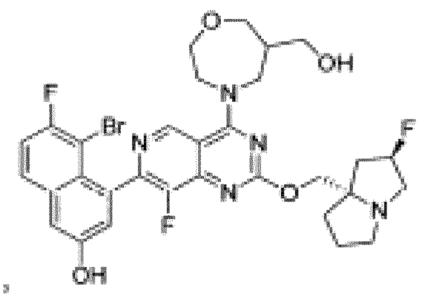 ,

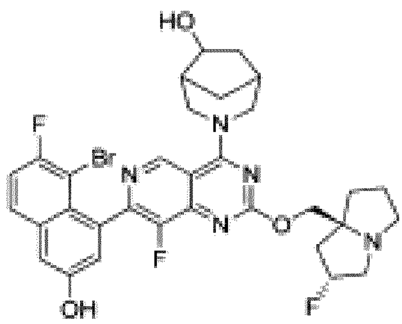 , 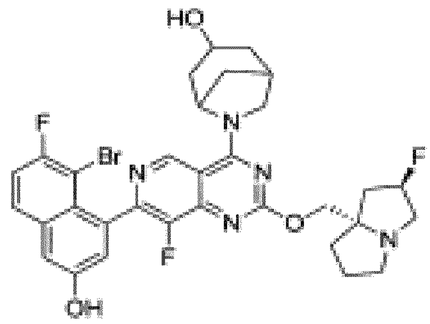 ,

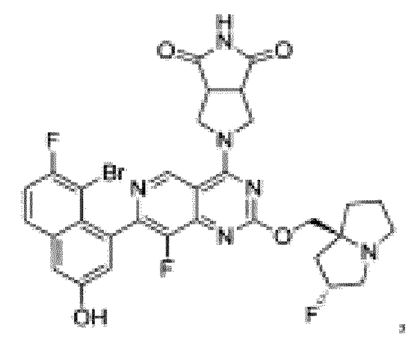 , 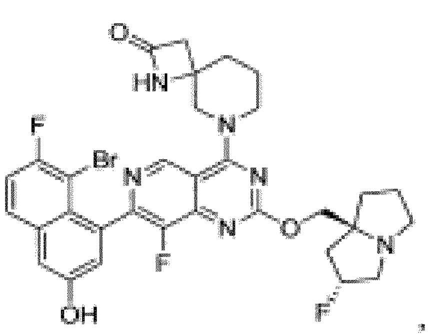 ,

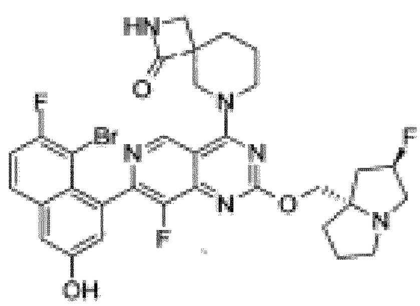 , 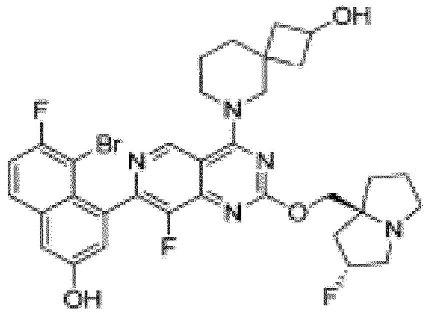 ,

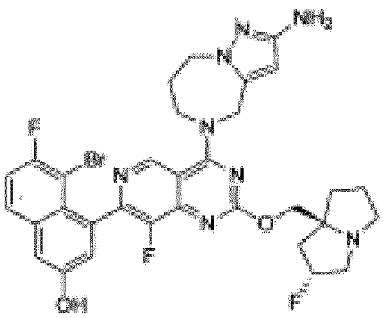 , 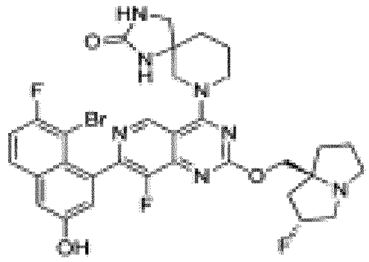 ,

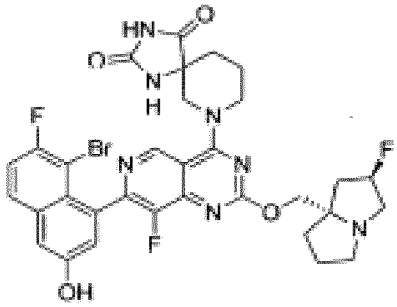 , 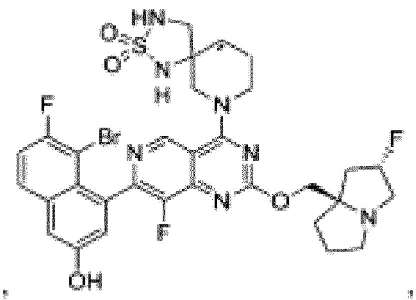 ,

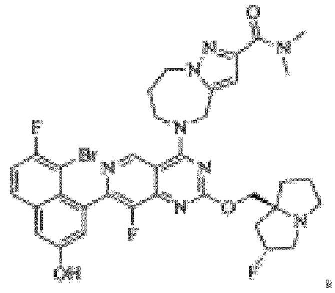 , 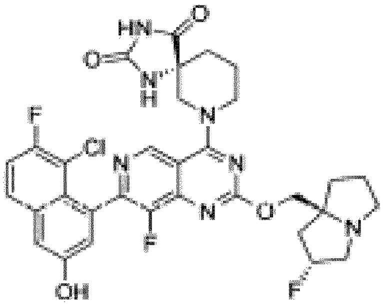 ,

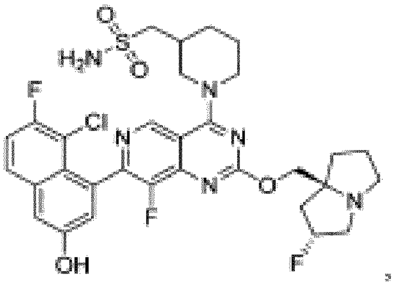 , 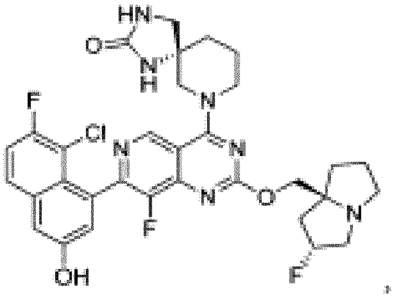 ,

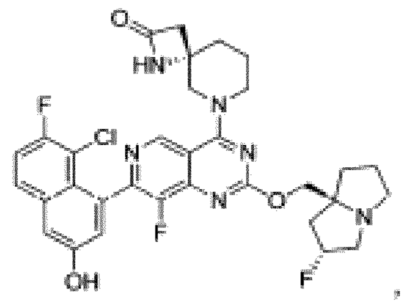 , 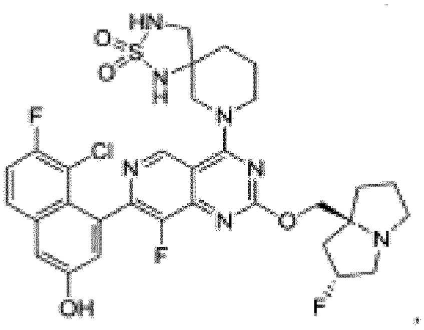 ,

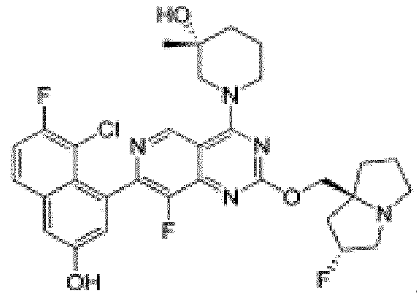 , 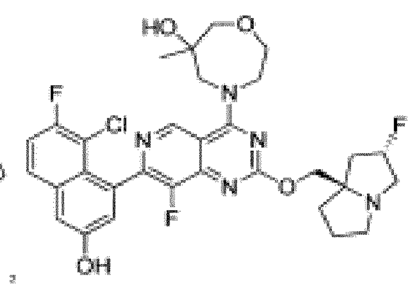 ,

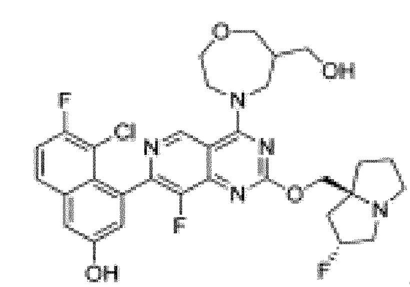 , 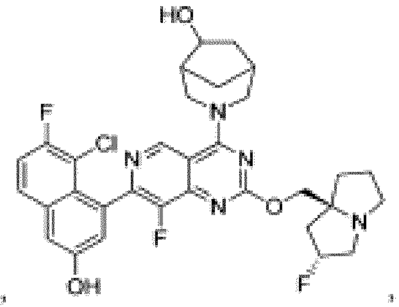 ,

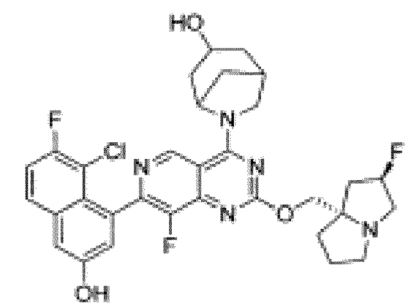 , 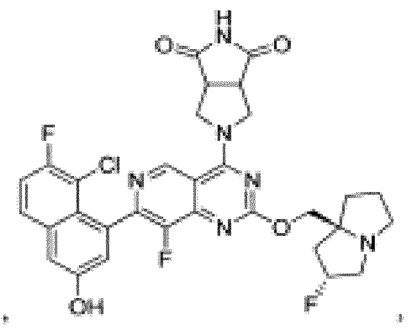 ,

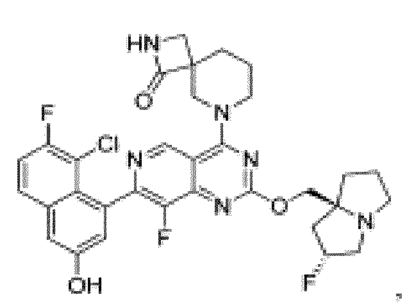 , 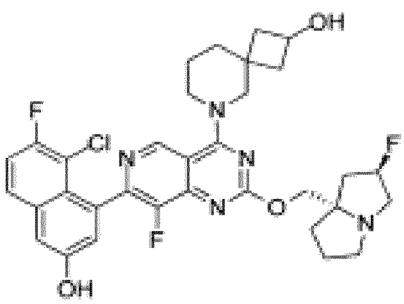 ,

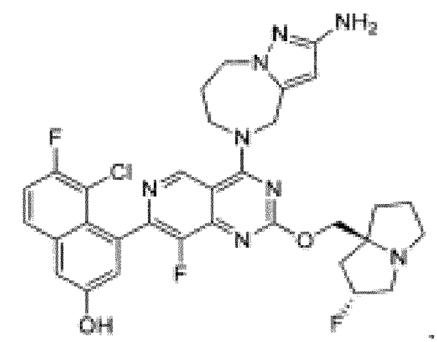,
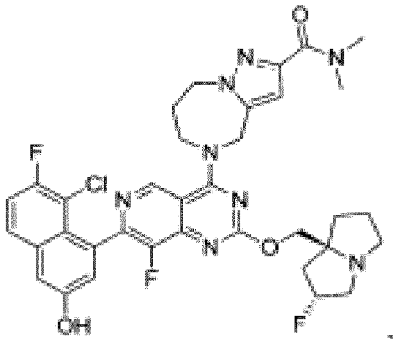,

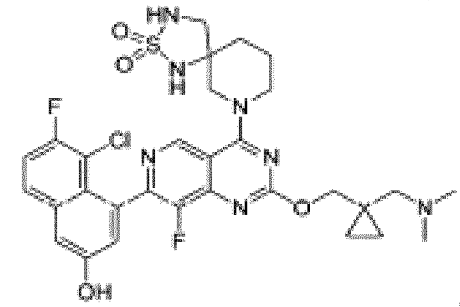,
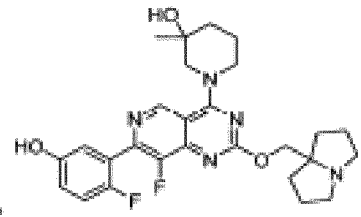,

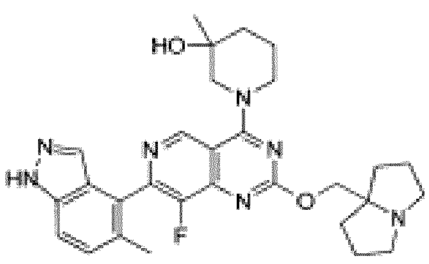,
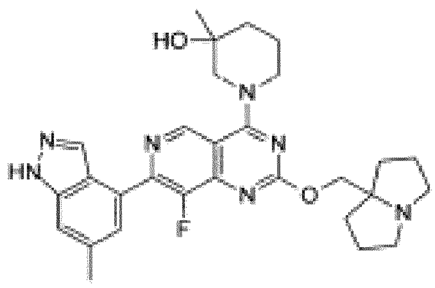,

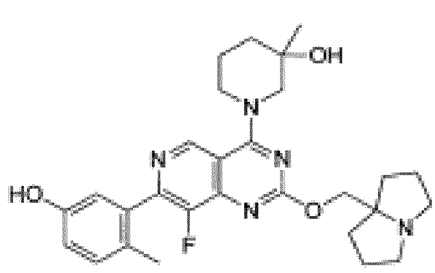,
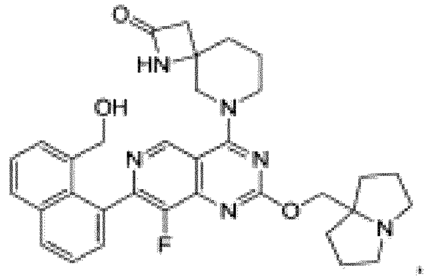,

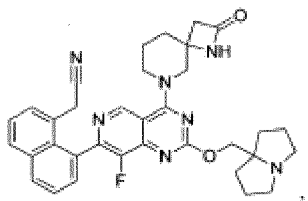,
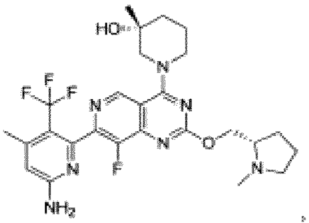,

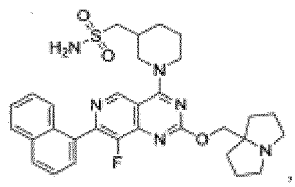,
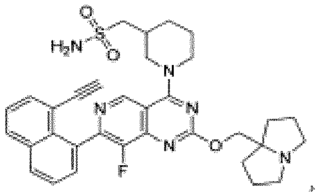,

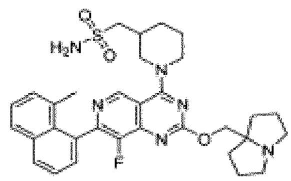,
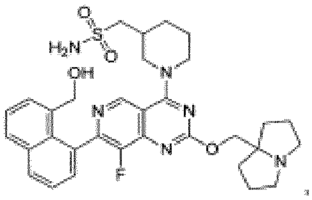,

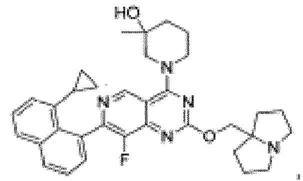,
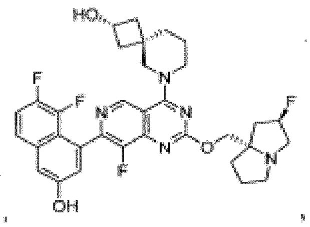,

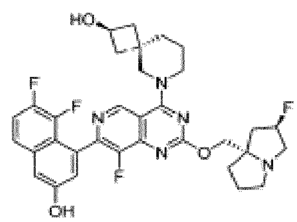,
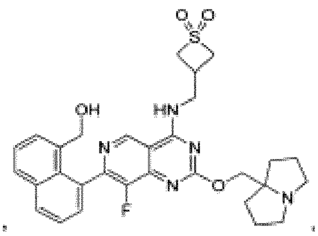,

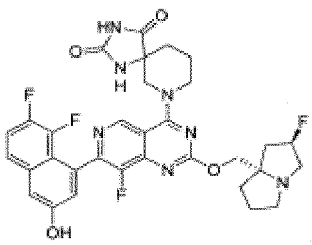,
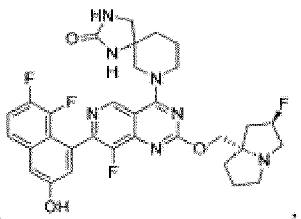,

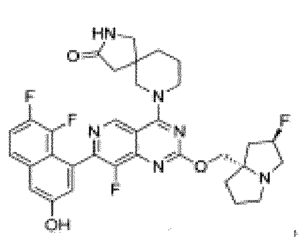,
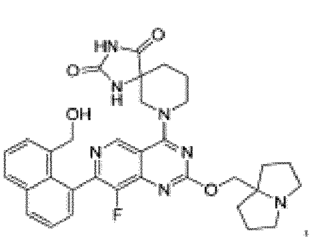,

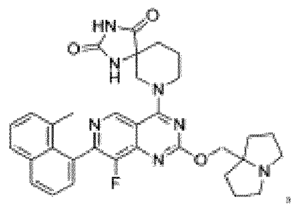,
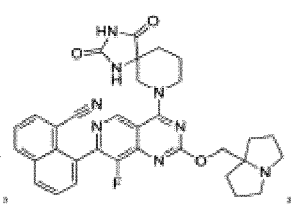,

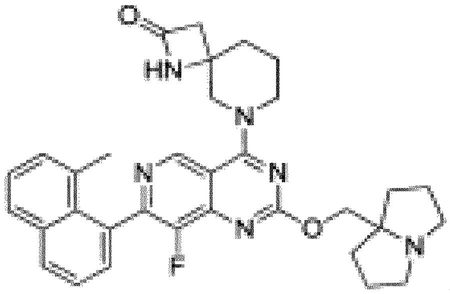,
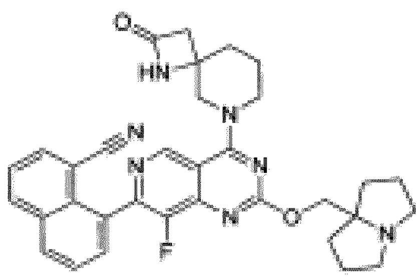,

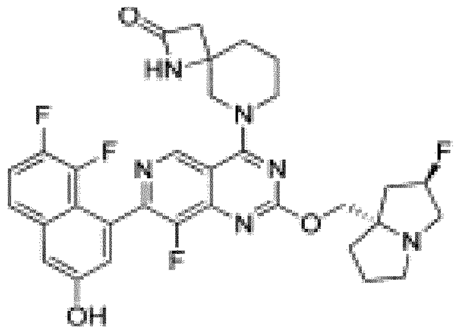,
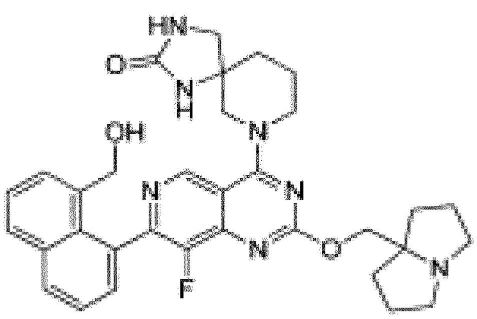,

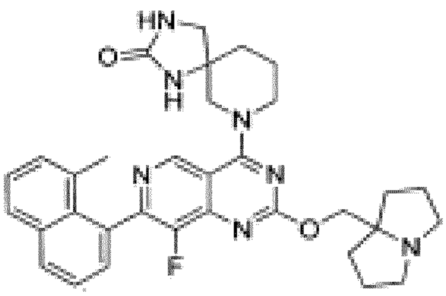,
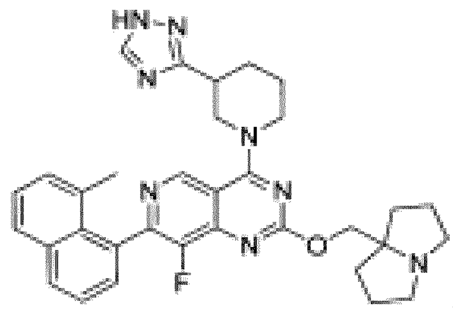,

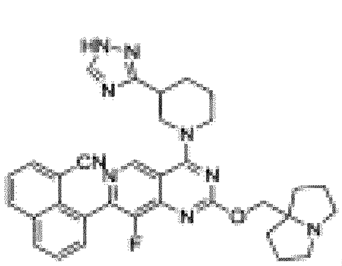,
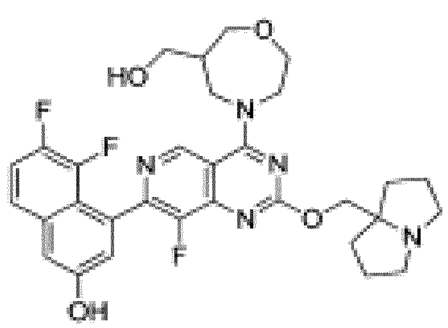,

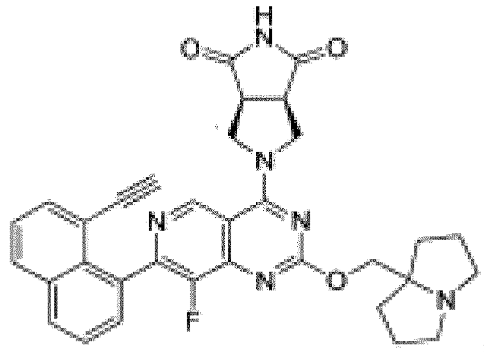,
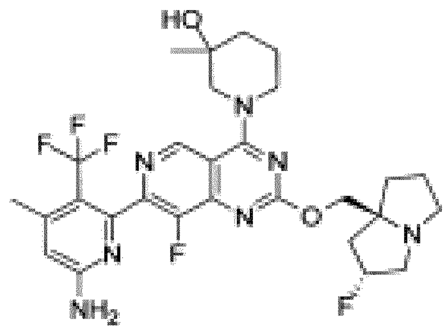,

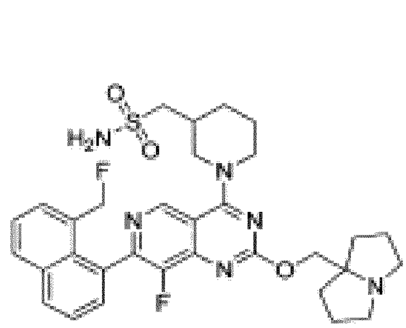,
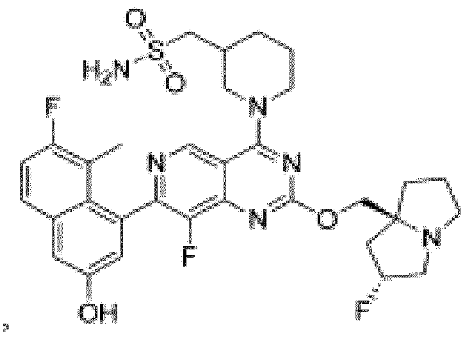,

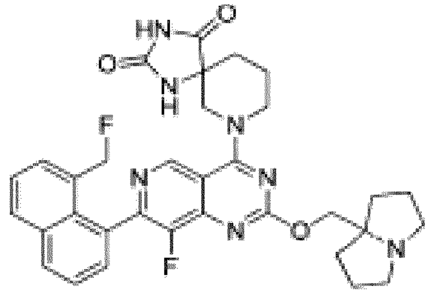,
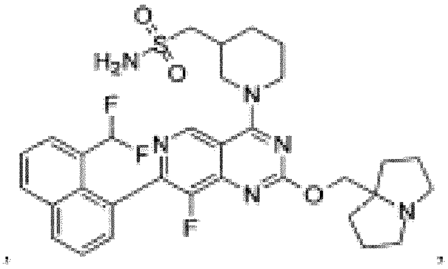,

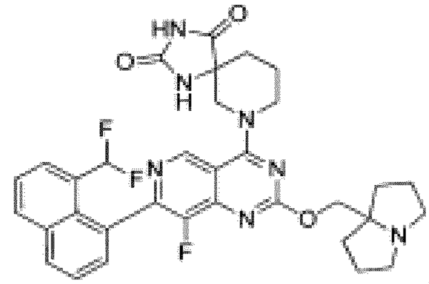,
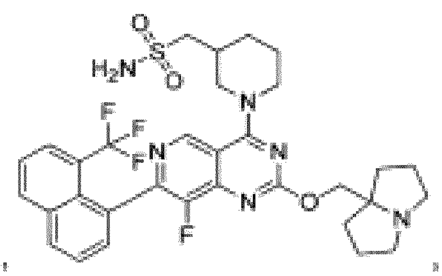,

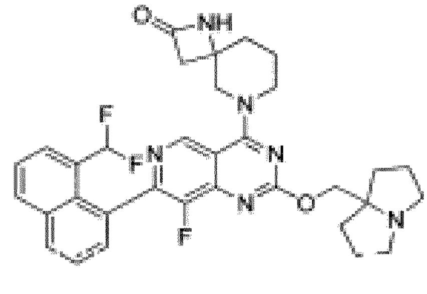,
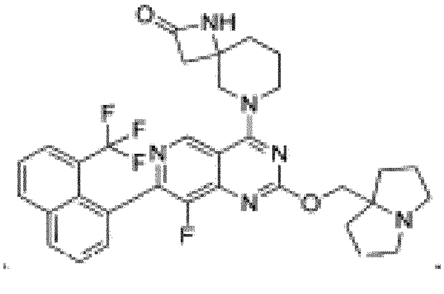.

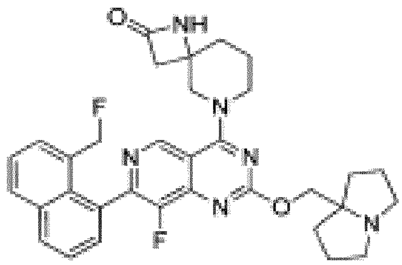,
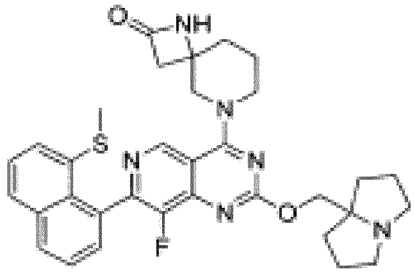,

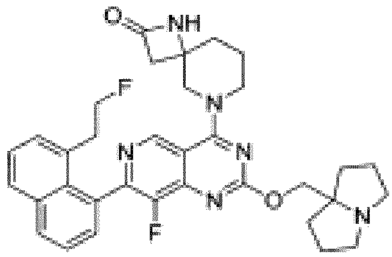,
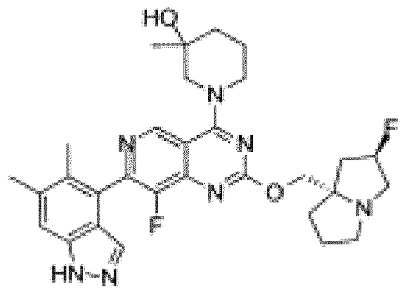,

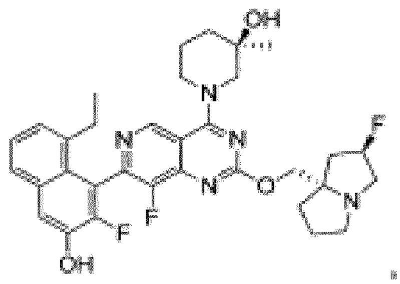,
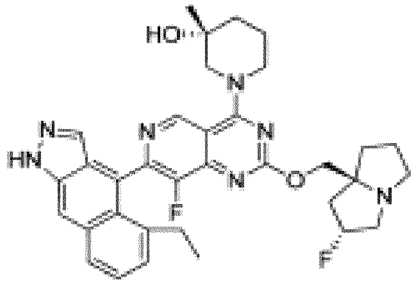,

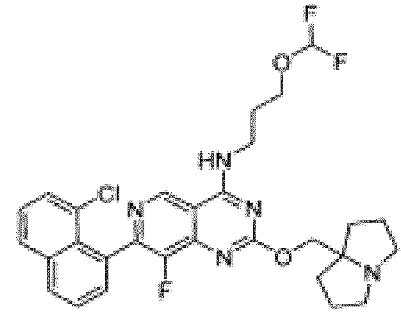,
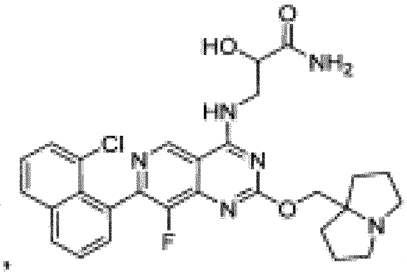,

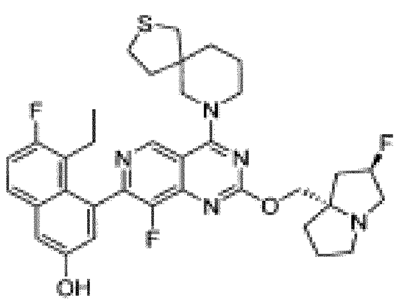,
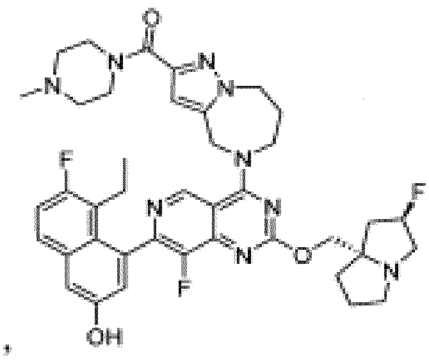,

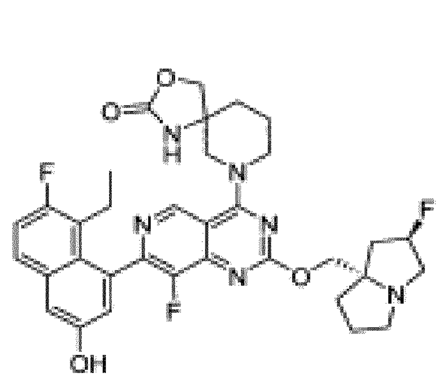,

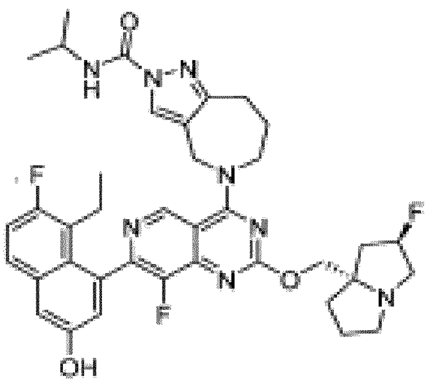,

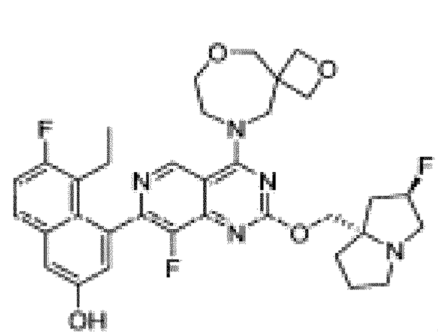,

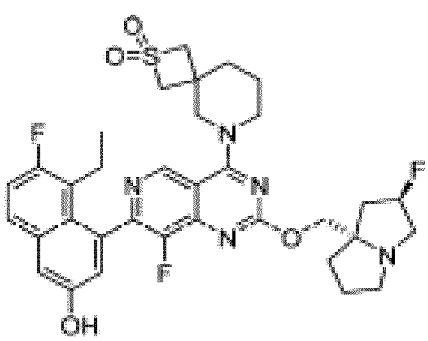,

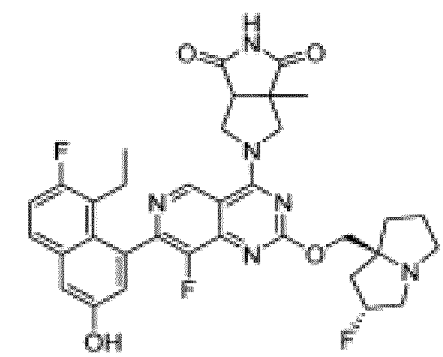,

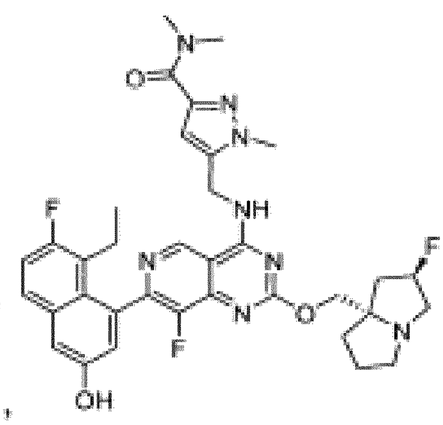,

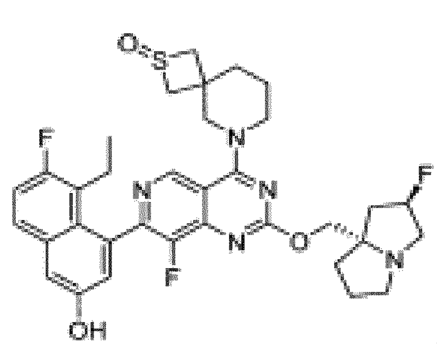,

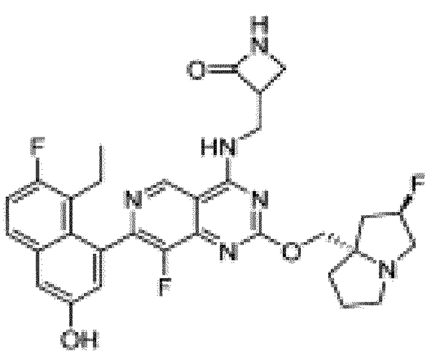,

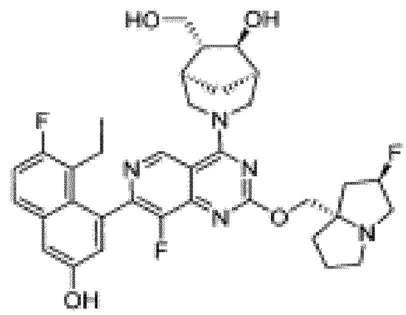,
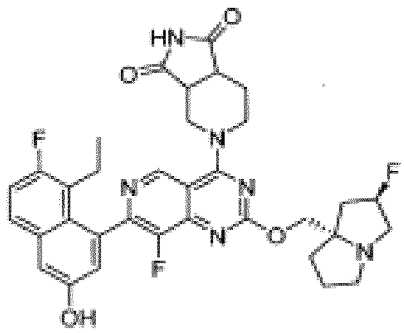,

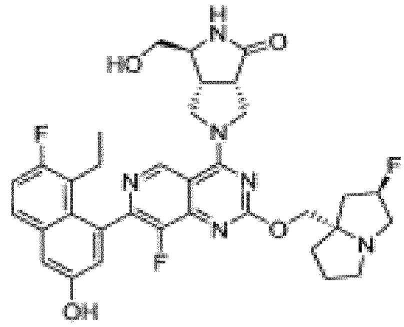,
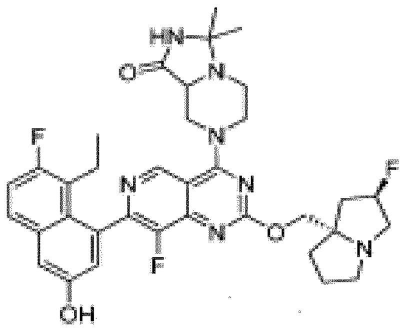,

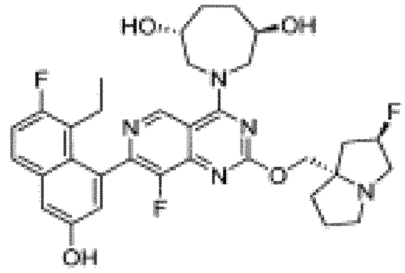,
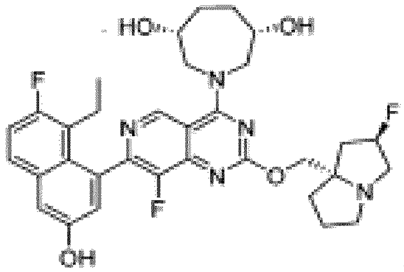,

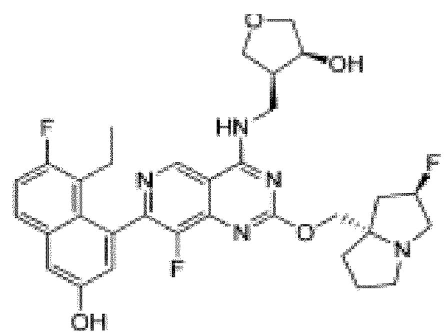,
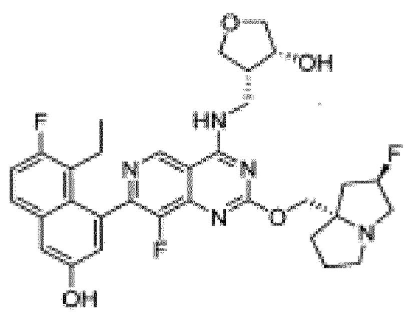,

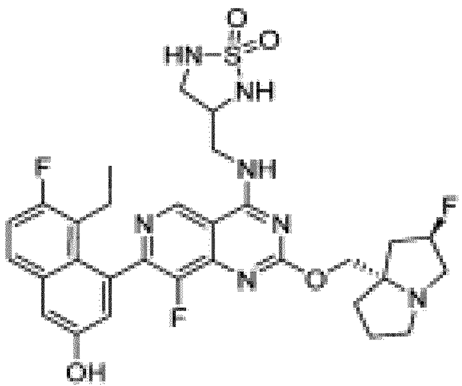
,
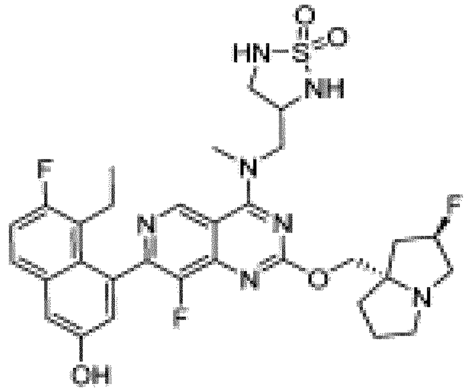
,

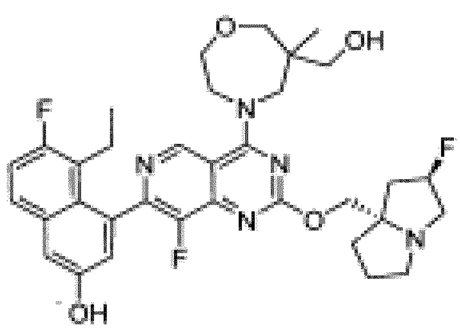
,
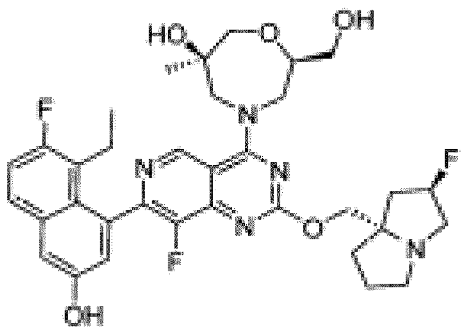
,

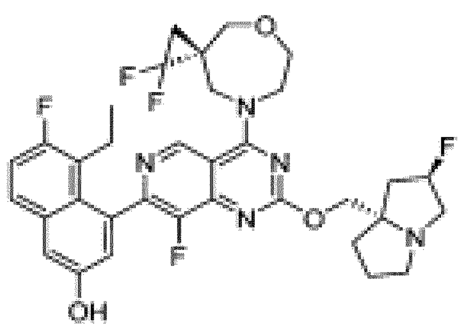
,
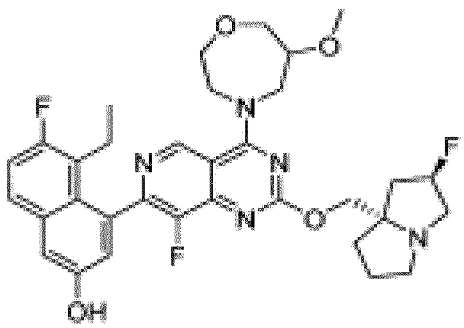
,

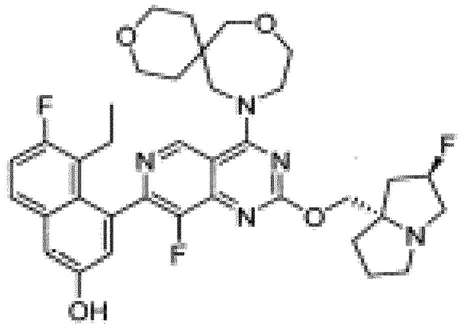
,
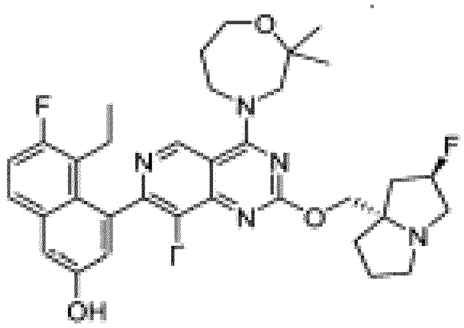
,

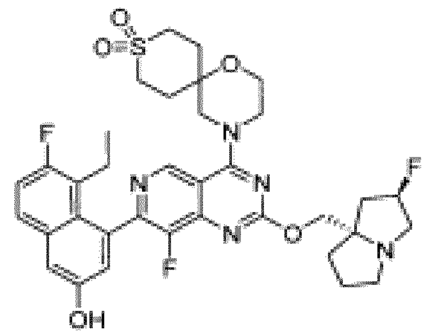
,
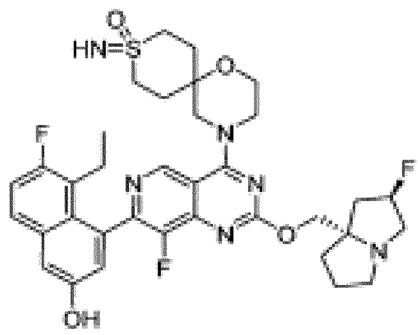
,

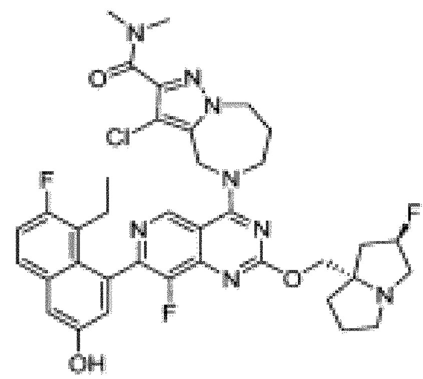
,
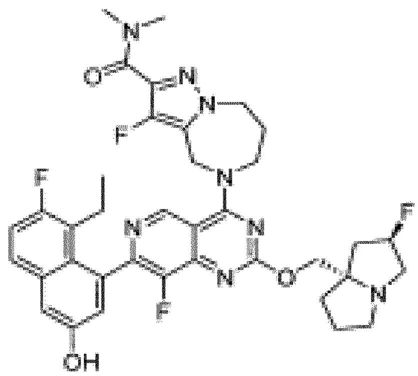
,

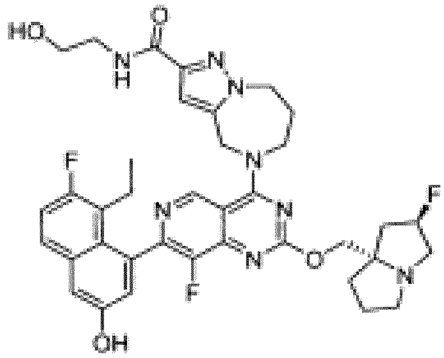
,
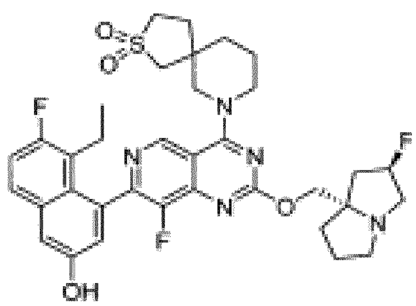

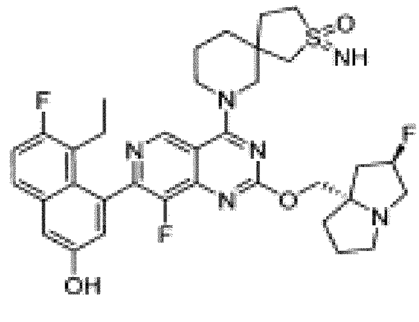
,
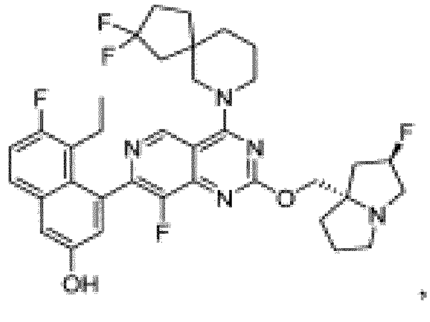
,

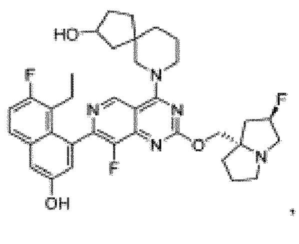
,
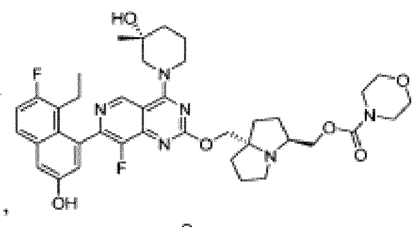
,

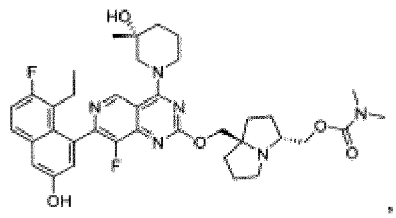, 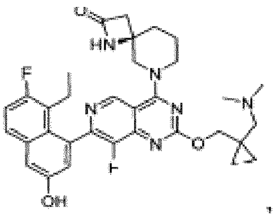,

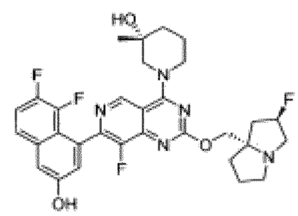, 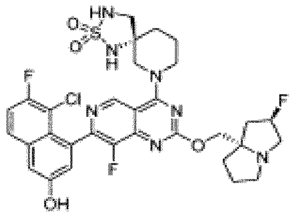,

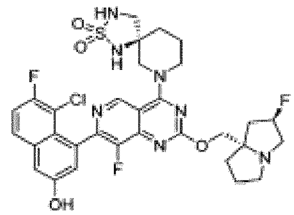, 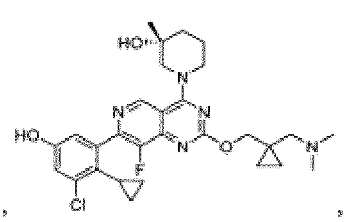,

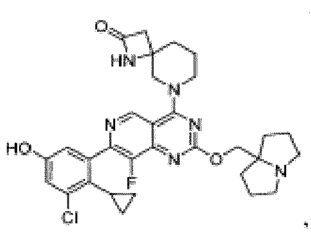, 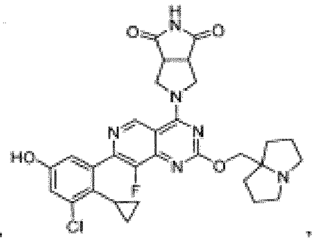,

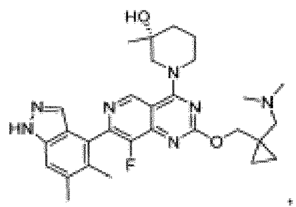, 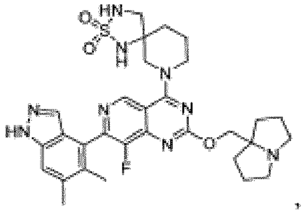,

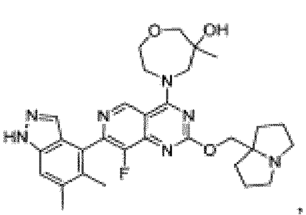, 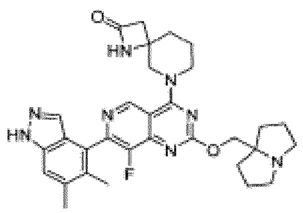.

In a certain embodiment, the nitrogen-containing heterocyclic compound of formula I is not any one of the following compounds:

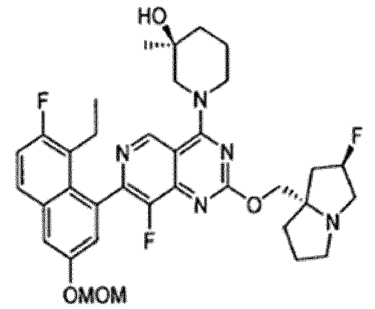,

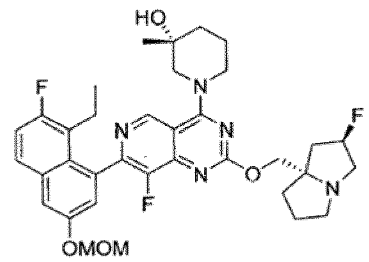,

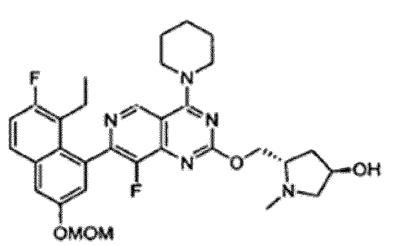,

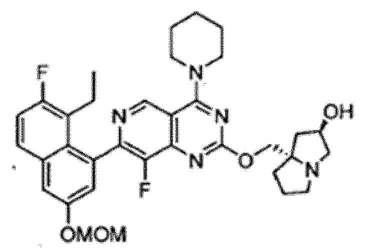,

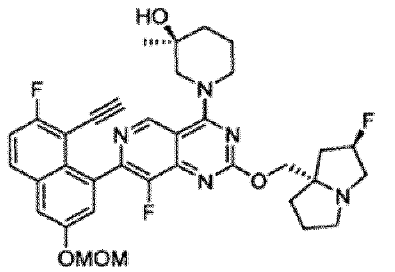,

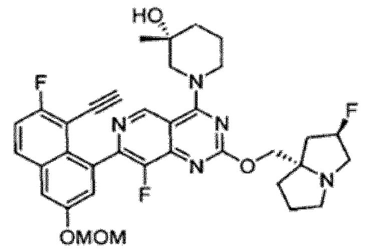,

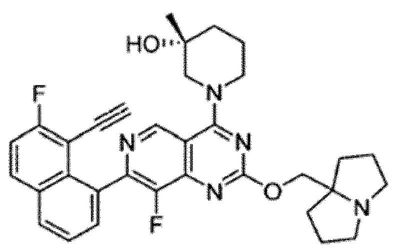,

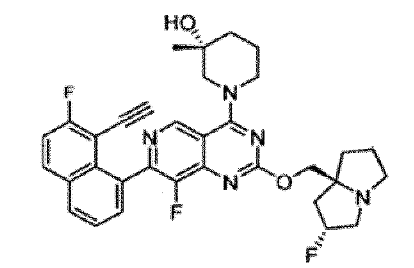,

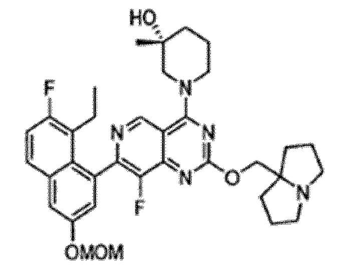,

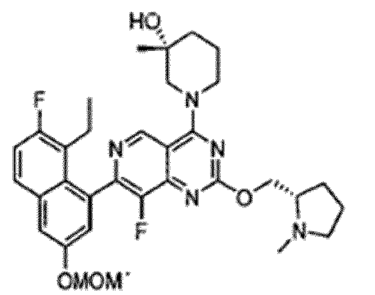,

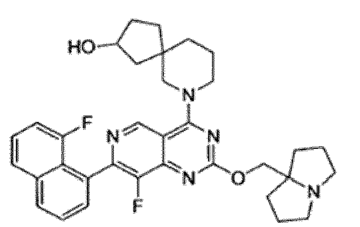,

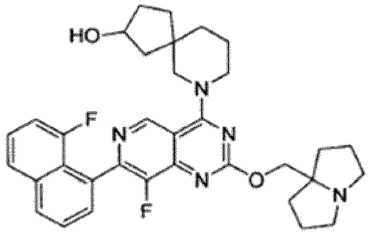,

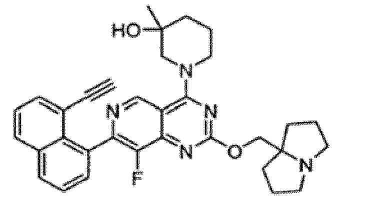,

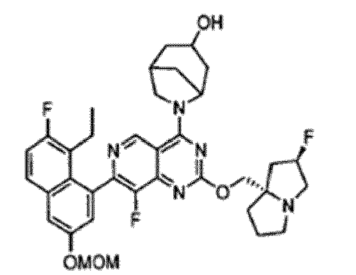,

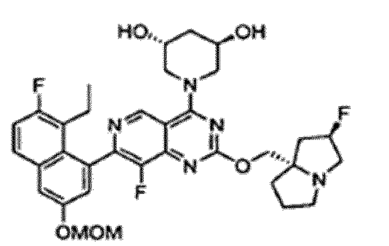,

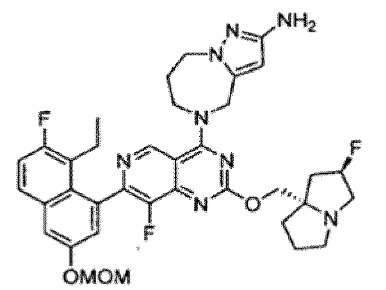,
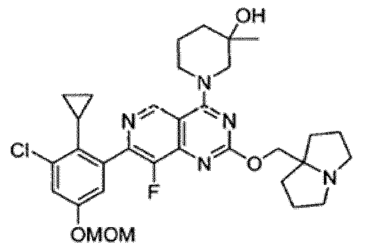,
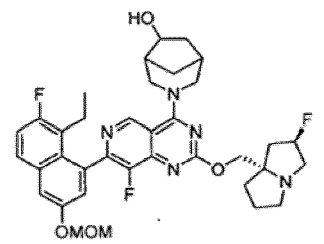,

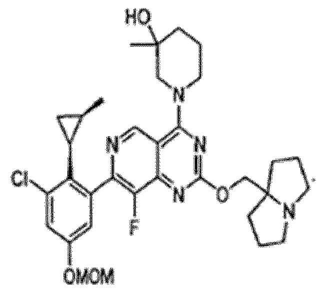,
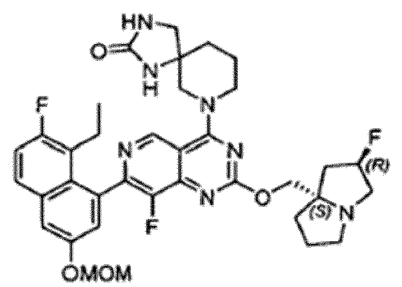,
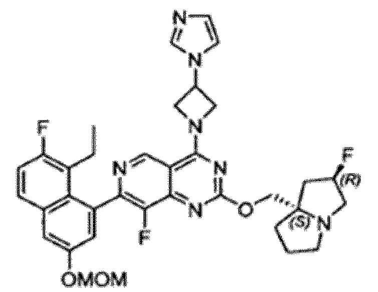,

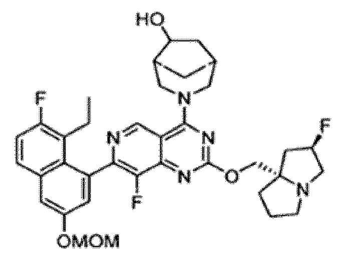,
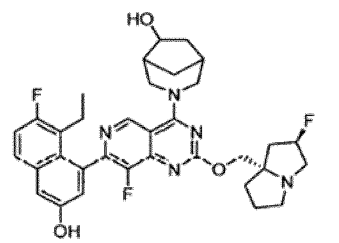,
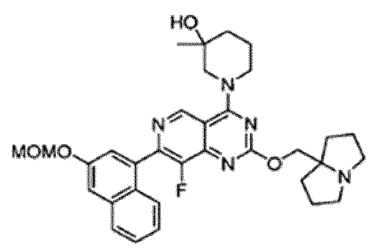,

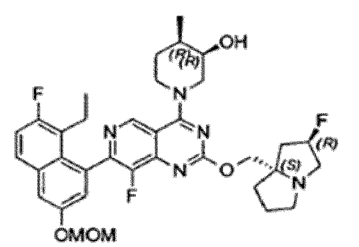,
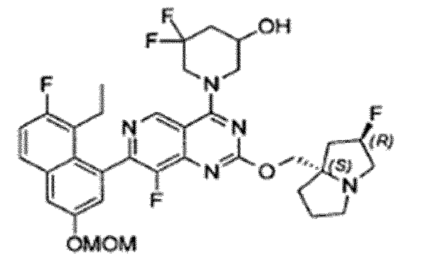,

and

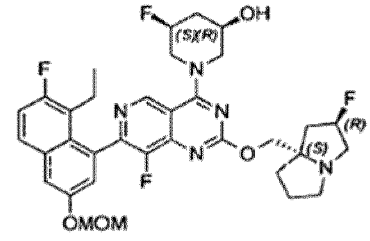.

In a certain embodiment, the nitrogen-containing heterocyclic compound of formula I is not any one of the following compounds:

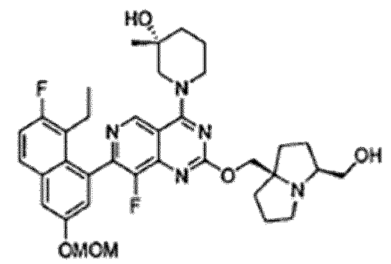

,

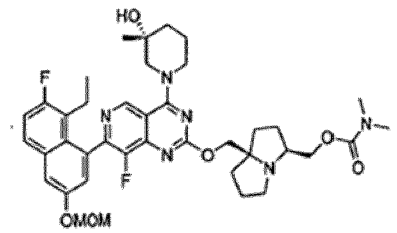

,

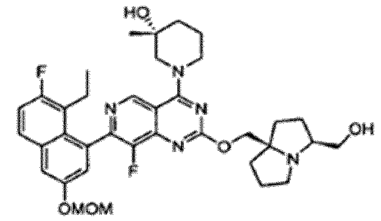

,

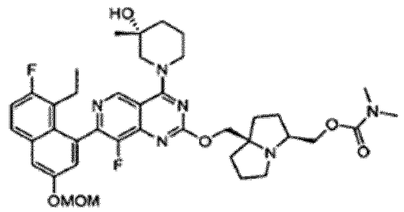

,

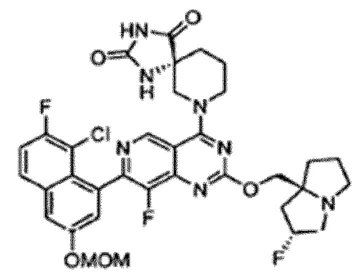

,

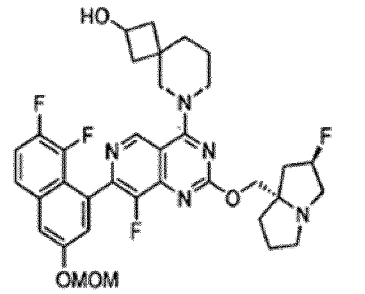

, and

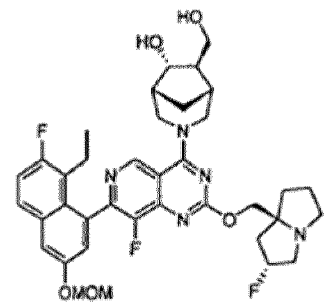

.

In a certain embodiment, the nitrogen-containing heterocyclic compound of formula I is selected from any one of the following compounds:

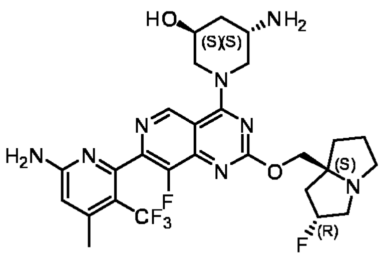

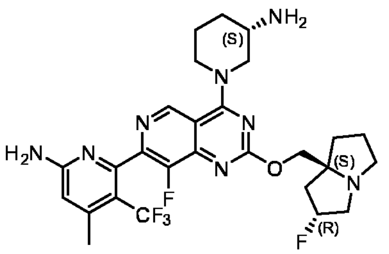

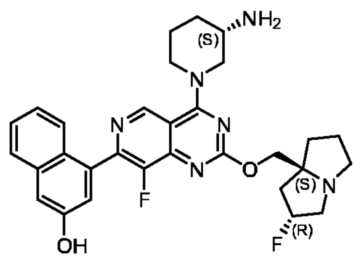

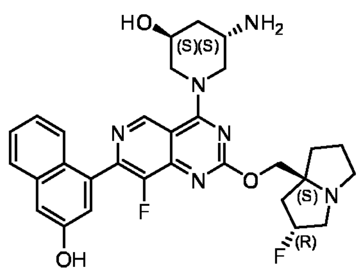

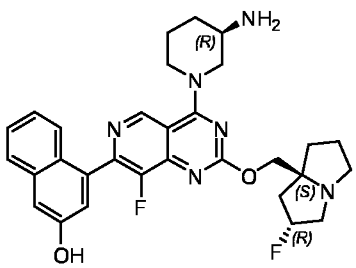

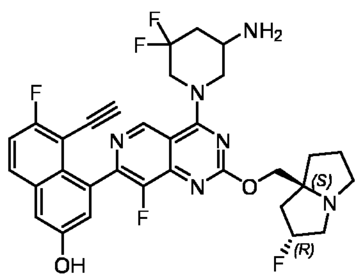

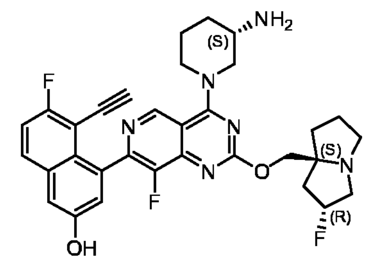
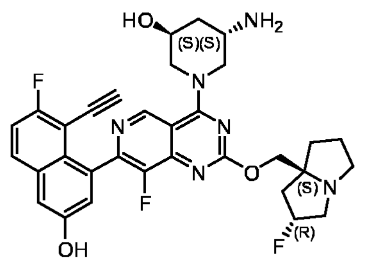
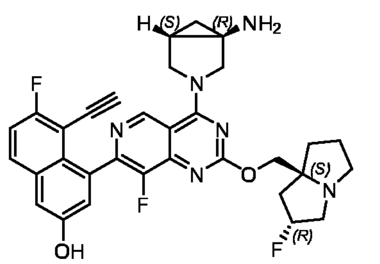

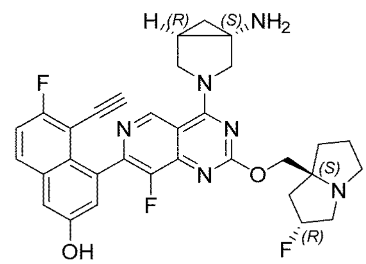
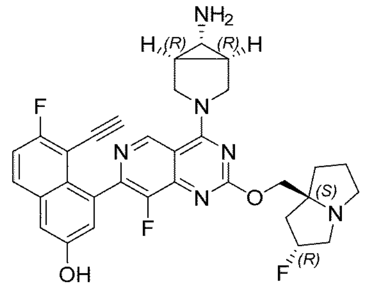
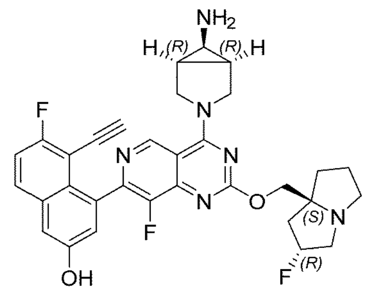

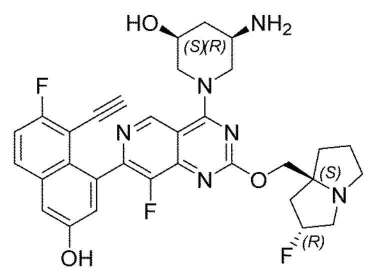
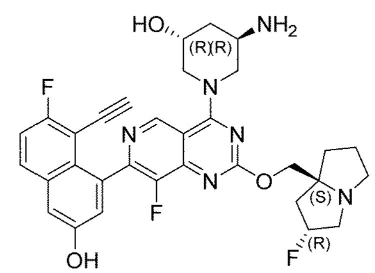
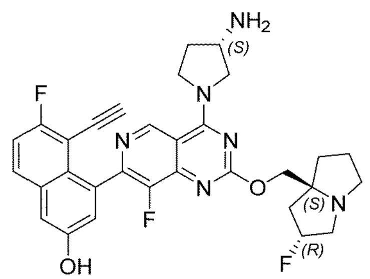

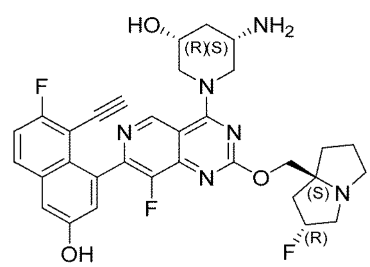
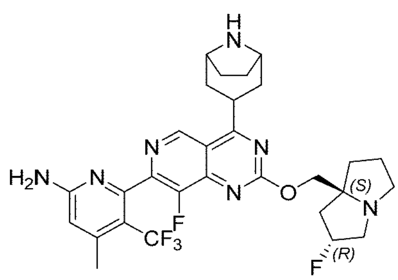
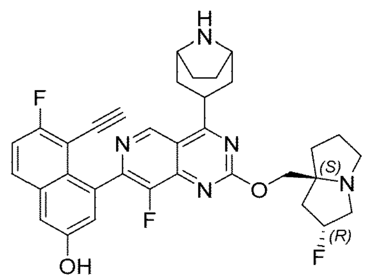

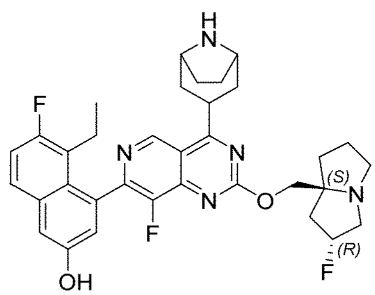
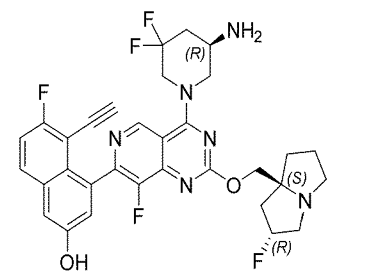
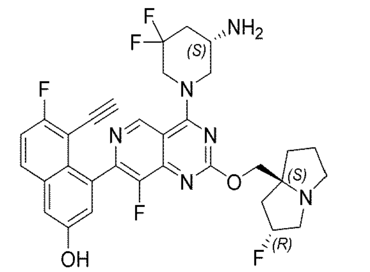

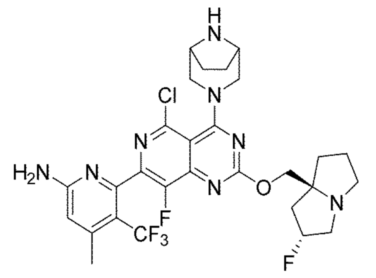

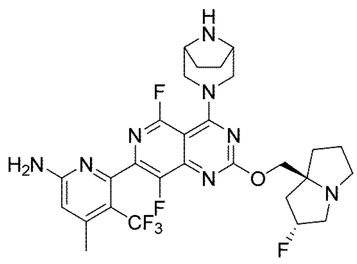

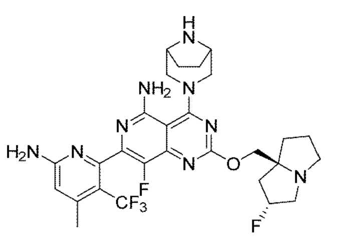

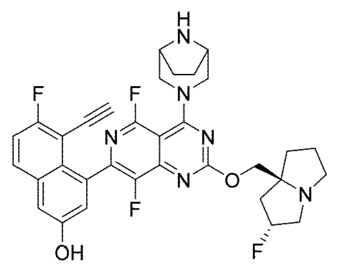

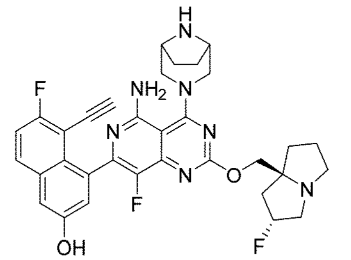

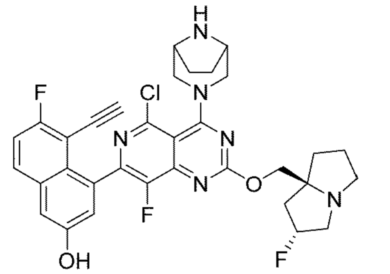

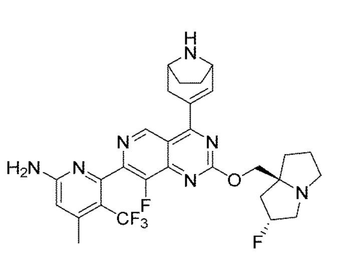

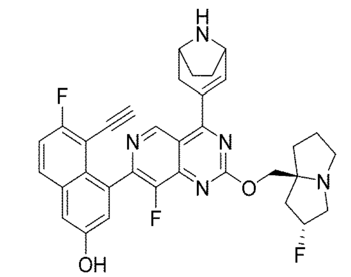

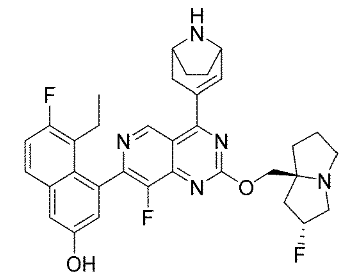

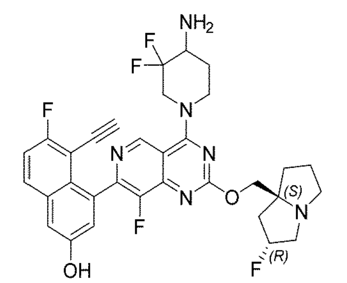

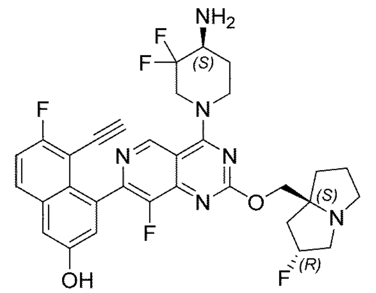

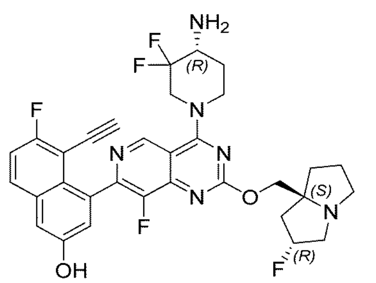

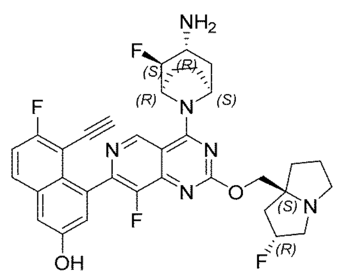

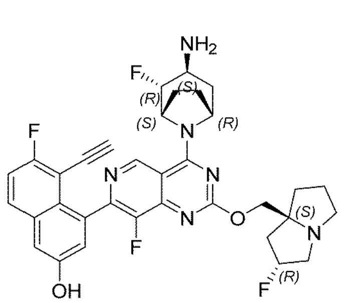

,

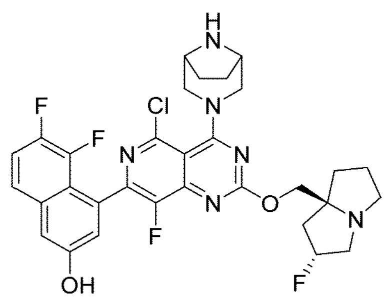

,

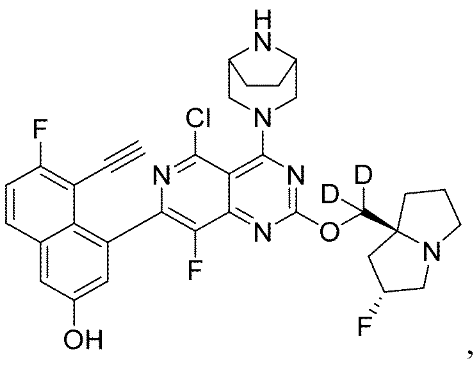

, and

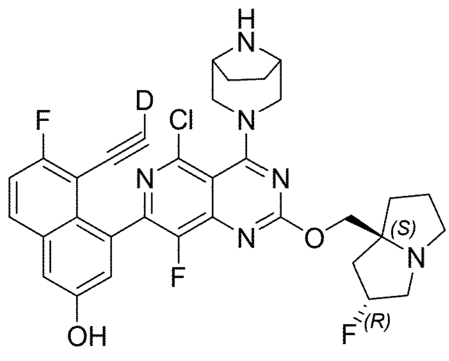

.

In a certain embodiment, the pharmaceutically acceptable salt of the nitrogen-containing heterocyclic compound of formula I may be any one of the following structures:

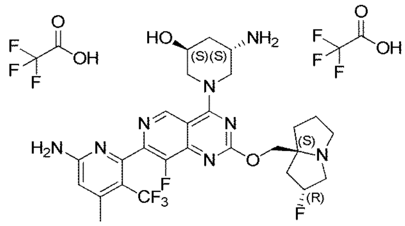

,

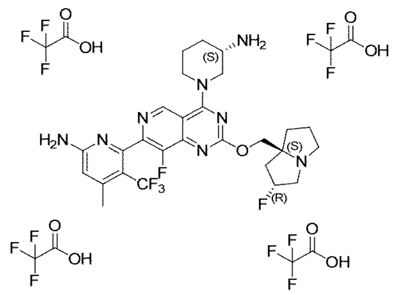

,

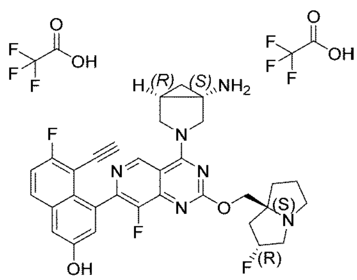

,

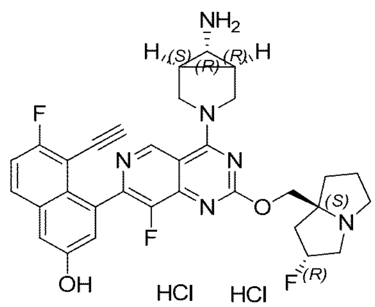

,

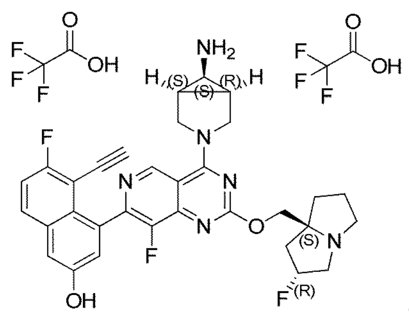

,

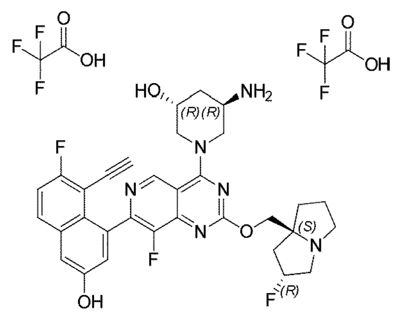

,

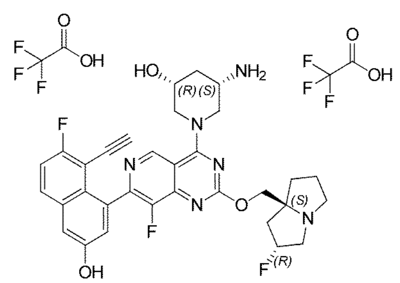

,

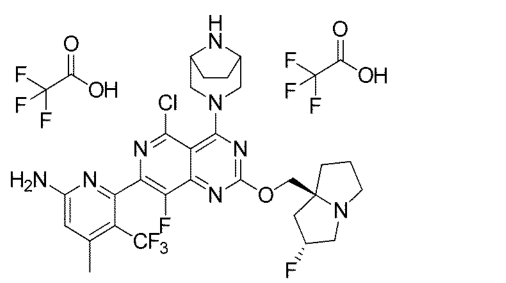

,

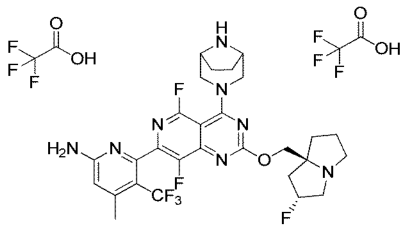

, and

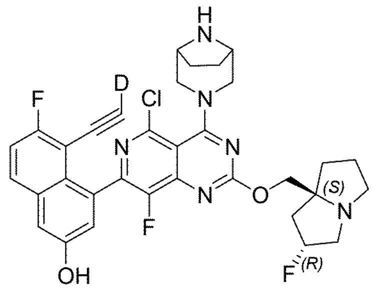

hydrochloride.

In the present disclosure, the nitrogen-containing heterocyclic compound of formula I, the stereoisomer thereof, or the pharmaceutically acceptable salt thereof can be synthesized by methods similar to those known in the chemical field, and the steps and conditions can refer to the steps and conditions of similar reactions in the art, especially according to the description herein. Starting materials are usually from commercial sources or can be readily prepared using methods well known to those skilled in the art (available through SciFinder, Reaxys online database).

In the present disclosure, for the nitrogen-containing heterocyclic compound of formula I, the stereoisomer thereof, or the pharmaceutically acceptable salt thereof, other said nitrogen-containing heterocyclic compound of formula I, the stereoisomer thereof, or the pharmaceutically acceptable salt thereof can also be obtained by employing the conventional methods of the art through peripheral modification of the nitrogen-containing heterocyclic compound of formula I, the stereoisomer thereof, or the pharmaceutically acceptable salt thereof which have already been prepared.

Necessary raw materials or reagents for preparing the nitrogen-containing heterocyclic compound of formula I, the stereoisomer thereof, or the pharmaceutically acceptable salt thereof can be commercially available or prepared by synthetic methods known in the art. According to the method described in the following experimental section, the free base of the compound or the salt of the compound in the present disclosure formed by adding acid can be prepared. The term pharmaceutically acceptable salt refers to the pharmaceutically acceptable salt as defined herein and which has all the effects of the parent compound. The pharmaceutically acceptable salt can be prepared by adding a corresponding acid into an appropriate organic solvent of an organic base and treating according to a conventional method.

The present disclosure also provides a method for preparing the nitrogen-containing heterocyclic compound of formula I, the stereoisomer thereof, or the pharmaceutically acceptable salt thereof, which may be the following routes:

route 1

when

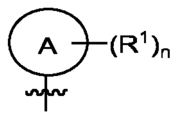

is

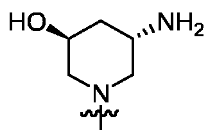

,

$R^3$ is

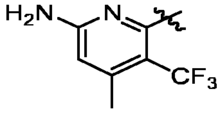

,

the corresponding nitrogen-containing heterocyclic compound of formula I, the stereoisomer thereof, or the pharmaceutically acceptable salt thereof may be prepared by the following method:

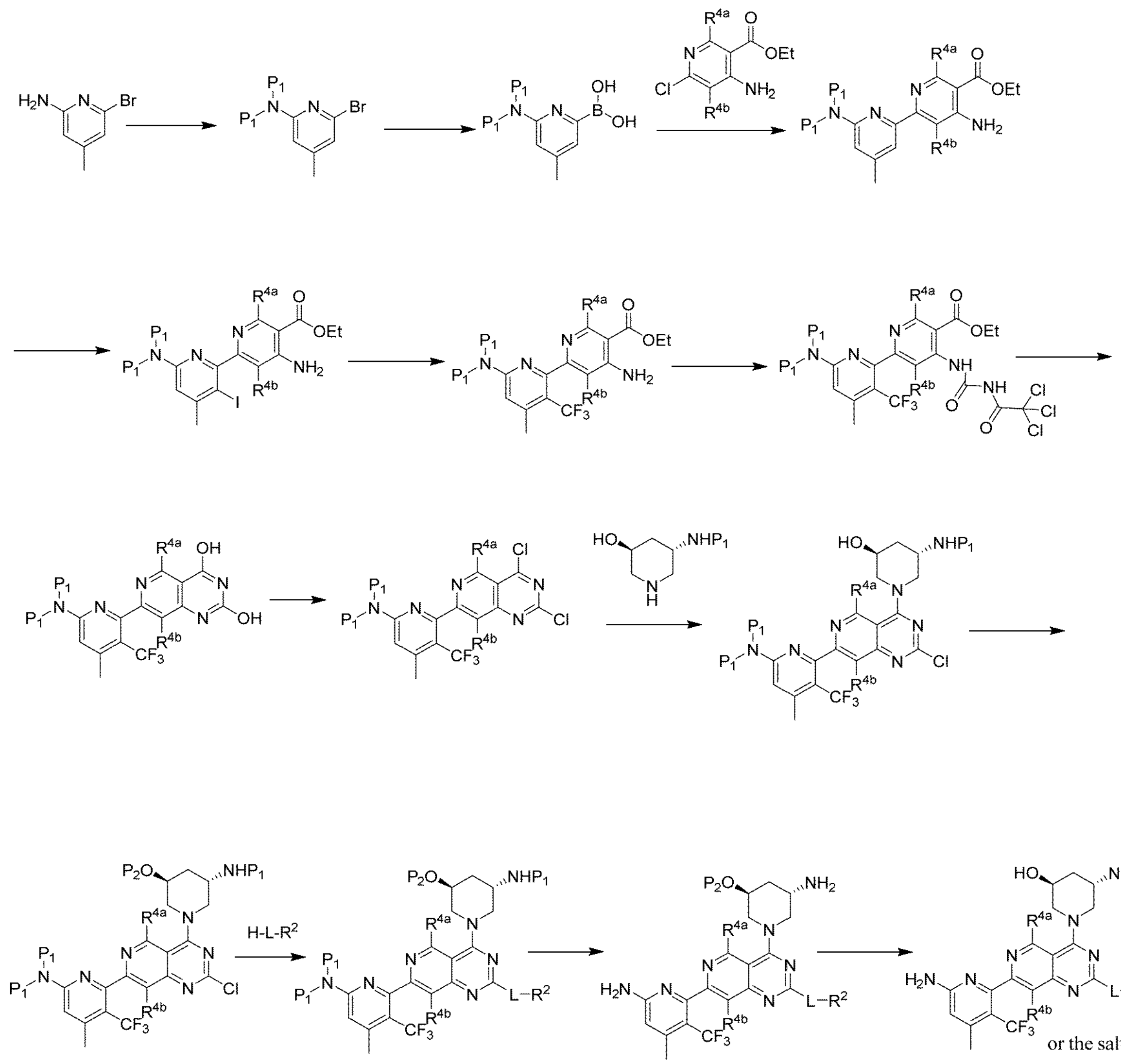


or the salt thereof

wherein $P_1$ is an amino protecting group, such as Boc, PMB, Bn, Cbz, Fmoc; each $P_1$ may be the same or different; $P_2$ is a hydroxyl protecting group, such as TBS, TIPS, TMS;

$R^{4a}$, $R^{4b}$, L, and $R^2$ are defined as in any one of the above;

step 1: nucleophilic substitution under an alkaline condition;

step 2: carrying out a borylation reaction under a palladium catalysis condition;

step 3: Suzuki coupling reaction;

step 4: iodination reaction;

step 5: carrying out a coupling reaction in the presence of a copper catalyst;

step 6: substitution reaction;

step 7: amination ring-closing reaction;

step 8: chlorination reaction;

step 9: substitution reaction under an alkaline condition;

step 10: carrying out a hydroxyl protection reaction in the presence of a silicon-containing reagent (such as TBSCl, TIPSCl, TMSCl);

step 11: substitution reaction under an alkaline condition;

step 12: removing an amino protecting group under an acidic condition;

step 13: removing a hydroxyl protecting group in the presence of tetrabutylammonium fluoride, tetramethylammonium fluoride, potassium fluoride, or CsF;

route 2

when $R^3$ is

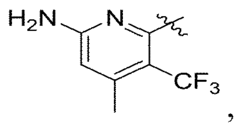

one $R^1$ is $-NH_2$, and n is 1, 2, 3, 4, 5, 6, 7, 8, 9, 10, the corresponding nitrogen-containing heterocyclic compound of formula I, the stereoisomer thereof, or the pharmaceutically acceptable salt thereof may be prepared by the following method:

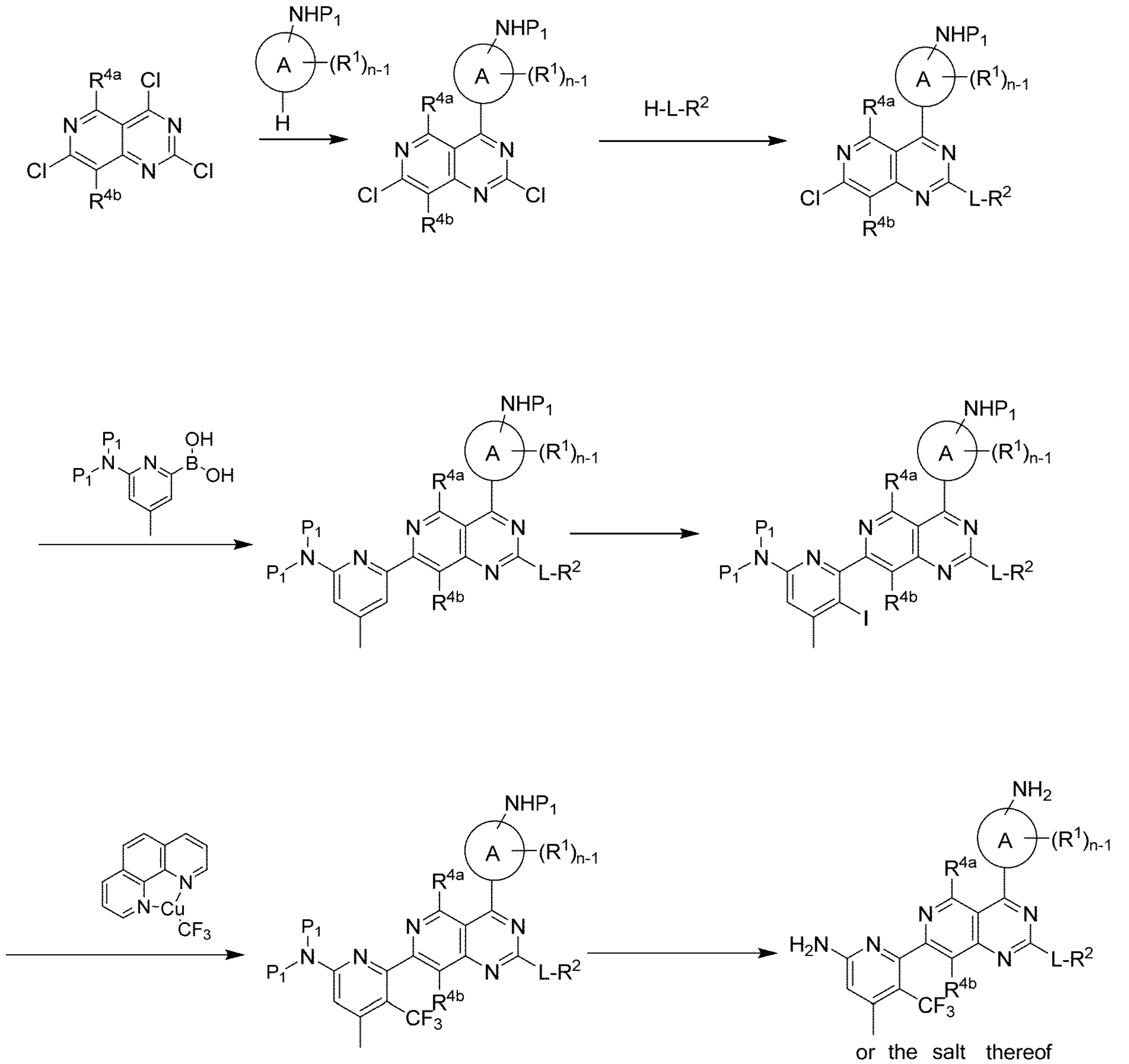


or the salt thereof

wherein $P_1$ is an amino protecting group, such as Boc, PMB, Bn, Cbz, Fmoc; each $P_1$ may be the same or different; $R^1$, $R^{4a}$, $R^{4b}$, L, and $R^2$ are defined as in any one of the above;

step 1: substitution reaction under an alkaline condition;

step 2: substitution reaction under an alkaline condition;

step 3: Suzuki coupling reaction;

step 4: iodination reaction;

step 5: carrying out a coupling reaction in the presence of a copper catalyst;

step 6: removing an amino protecting group;

route 3

when one $R^1$ is $-NH_2$, and n is 1, 2, 3, 4, 5, 6, 7, 8, 9, 10, the corresponding nitrogen-containing heterocyclic compound of formula I, the stereoisomer thereof, or the pharmaceutically acceptable salt thereof may be prepared by the following method:

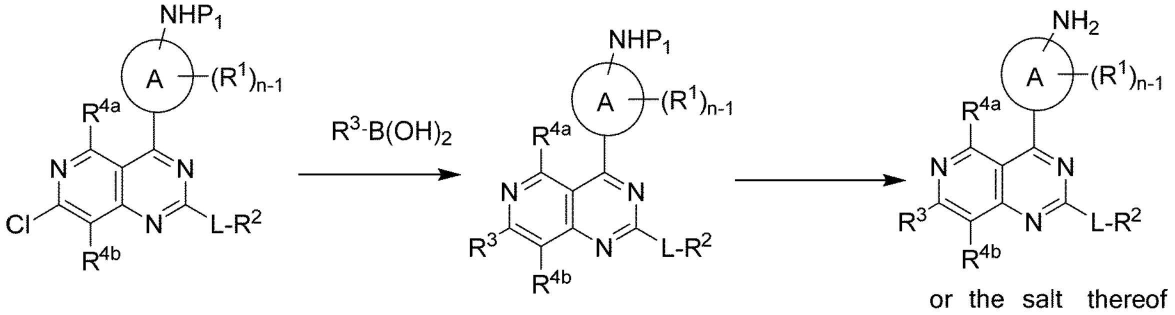


wherein $R^1$, $R^3$, $R^{4a}$, $R^{4b}$, L, and $R^2$ are defined as in any one of the above;
step 1: Suzuki coupling reaction;
step 2: deprotection reaction [if the substituents $R^{3a}$ and $R^{3b}$ (such as -OH, $-NH_2$, $C_2$-$C_6$ alkynyl) in $R^3$ are protected by protecting groups, the deprotection reaction can be carried out to obtain the compound of general formula I];

route 4

when

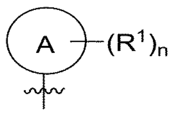

is

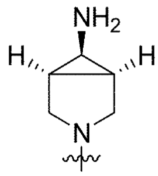

,

$R^3$ is

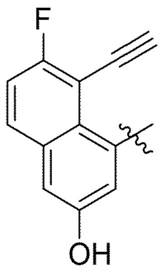

,

the corresponding nitrogen-containing heterocyclic compound of formula I, the stereoisomer thereof, or the pharmaceutically acceptable salt thereof may be prepared by the following method:

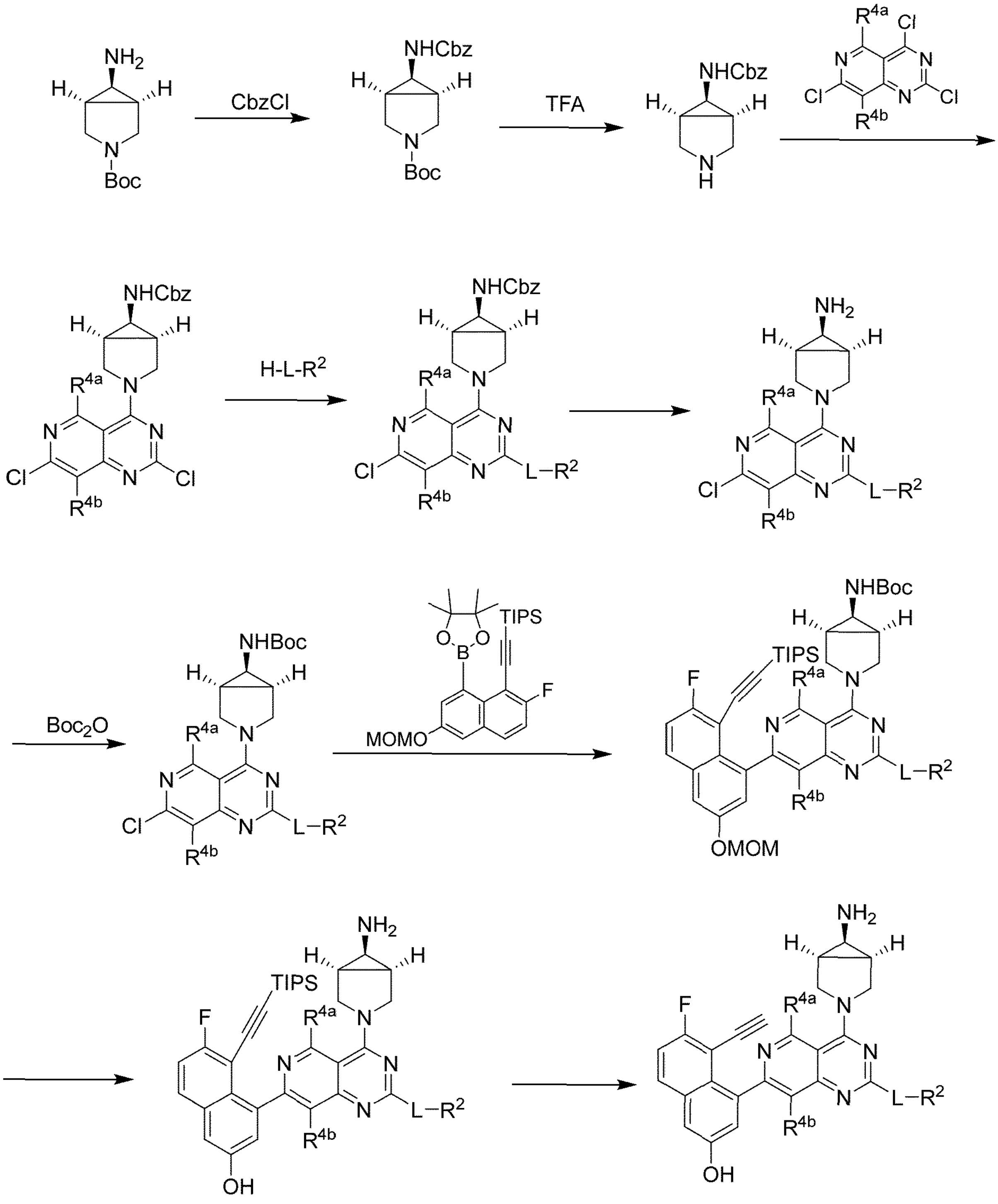


or the salt thereof

wherein $R^{4a}$, $R^{4b}$, L, and $R^2$ are defined as in any one of the above;

step 1: amino protection reaction;

step 2: removing Boc protection;

step 3: substitution reaction under an alkaline condition;

step 4: substitution reaction under an alkaline condition;

step 5: removing Cbz protection;

step 6: amino protection;

step 7: Suzuki coupling reaction;

step 8: carrying out a deamination protecting group and dehydroxyl protecting group reaction under an acidic

condition;
step 9: removing TIPS protection in the presence of tetrabutylammonium fluoride, tetramethylammonium fluoride, or CsF;

route 5

when

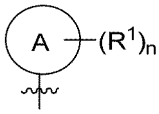

is

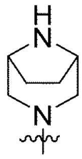

,

$R^3$ is

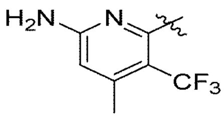

,

and $R^{4a}$ is halogen or amino, the corresponding nitrogen-containing heterocyclic compound of formula I, the stereoisomer thereof, or the pharmaceutically acceptable salt thereof may be prepared by the following method:

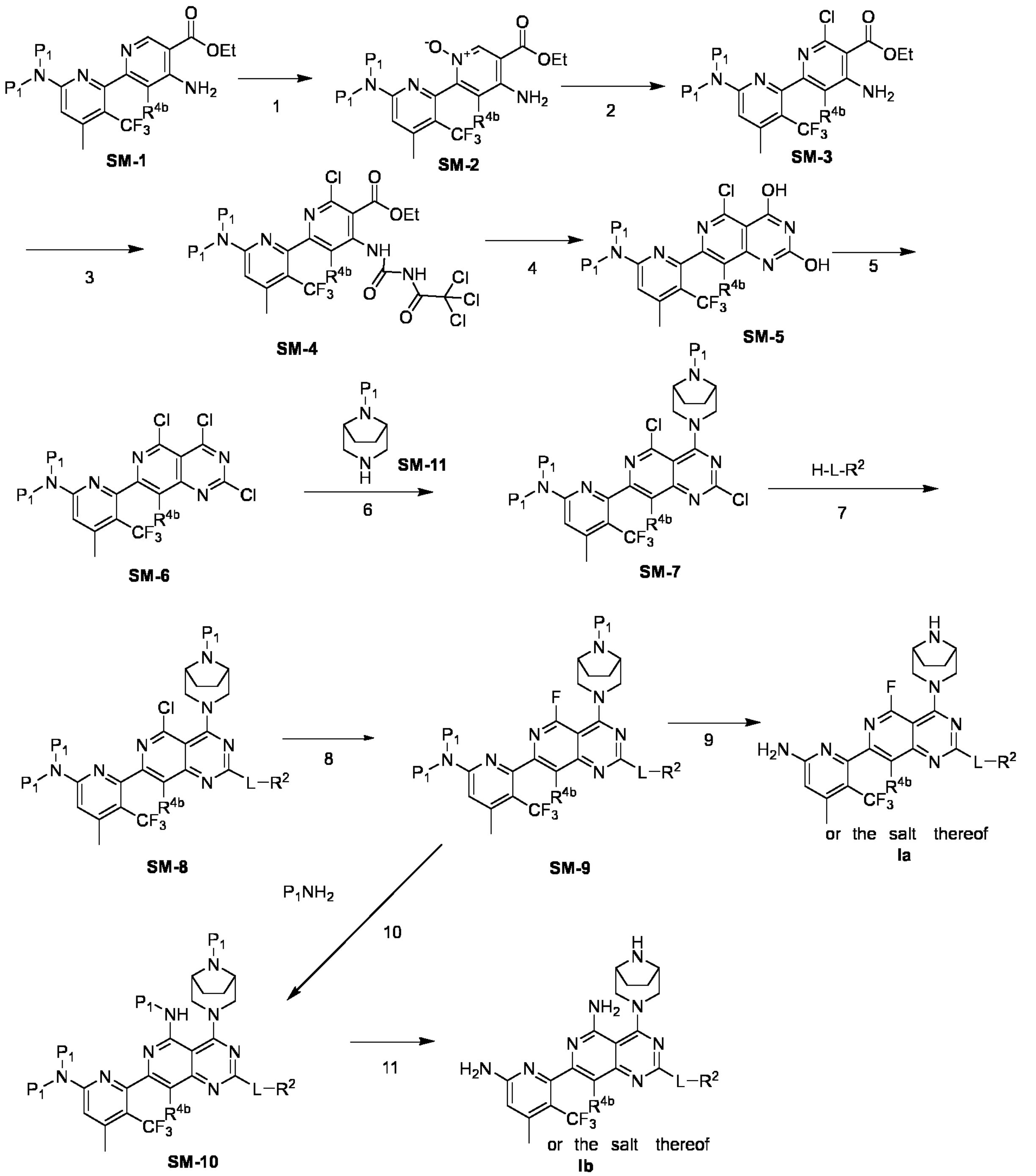


SM-1 SM-2 SM-3 SM-4 SM-5 SM-11 SM-6 SM-7 SM-8 SM-9 Ia SM-10 Ib

wherein $P_1$ is an amino protecting group, such as Boc, PMB, Bn, Cbz, Fmoc; each $P_1$ may be the same or different; $R^{4b}$, L, and $R^2$ are defined as in any one of the above;

step 1: carrying out an oxidation reaction with a compound of formula SM-1 in a solvent in the presence of an oxidant to obtain a compound of formula SM-2;

the operations and conditions of the oxidation reaction may be conventional operations and conditions for such reactions in the art; for example, the oxidant may be *m*-chloroperoxybenzoic acid, hydrogen peroxide, etc.;

step 2: carrying out a chlorination reaction with the compound of formula SM-2 in the presence of a chlorinating reagent to obtain a compound of formula SM-3;

the operations and conditions of the chlorination reaction may be conventional operations and conditions for such reactions in the art; for example, the chlorinating reagent may be phosphorus oxychloride, thionyl chloride, etc.;

step 3: carrying out a substitution reaction with the compound of formula SM-3 and trichloroacetyl isocyanate

in a solvent to obtain a compound of formula SM-4; the operations and conditions of the substitution reaction may be conventional operations and conditions for such reactions in the art;

step 4: carrying out an amination ring-closing reaction with the compound of formula SM-4 in a solvent to obtain a compound of formula SM-5; the operations and conditions of the amination ring-closing reaction may be conventional operations and conditions for such reactions in the art; for example, the solvent may be an ammonia-methanol solution;

step 5: carrying out a chlorination reaction with the compound of formula SM-5 in the presence of a chlorinating reagent to obtain a compound of formula SM-6;

the operations and conditions of the chlorination reaction may be conventional operations and conditions for such reactions in the art; for example, the chlorinating reagent may be phosphorus oxychloride, thionyl chloride, etc.;

step 6: carrying out a substitution reaction with the compound of formula SM-6 and a compound of formula SM-11 in a solvent under an alkaline condition to obtain a compound of formula SM-7; the operations and conditions of the substitution reaction may be conventional operations and conditions for such reactions in the art; for example, the alkaline condition may be in the presence of *N,N*-diisopropylethylamine;

step 7: carrying out a substitution reaction with the compound of formula SM-7 and a compound of formula H-L-$R^2$ in a solvent in the presence of a base to obtain a compound of formula SM-8; the operations and conditions of the substitution reaction may be conventional operations and conditions for such reactions in the art; for example, the base may be sodium tert-butoxide;

step 8: carrying out a substitution reaction with the compound of formula SM-8 in a solvent in the presence of a fluorinating reagent to obtain a compound of formula SM-9; the operations and conditions of the substitution reaction may be conventional operations and conditions for such reactions in the art; for example, the fluorinating reagent may be cesium fluoride, potassium fluoride, tetrabutylammonium fluoride, tetramethylammonium fluoride, etc.;

step 9: carrying out a deamination protecting group reaction with the compound of formula SM-9 under an acidic condition to obtain a compound of formula Ia or a pharmaceutically acceptable salt thereof; the operations and conditions of the deamination protecting group reaction may be conventional operations and conditions for such reactions in the art; for example, the acidic condition may be in the presence of trifluoroacetic acid;

step 10: carrying out a substitution reaction with the compound of formula SM-9 and a compound of formula $P_1$-$NH_2$ in a solvent to obtain a compound of formula SM-10; the operations and conditions of the substitution reaction may be conventional operations and conditions for such reactions in the art; for example, the substitution reaction is carried out under microwave conditions;

step 11: carrying out a deamination protecting group reaction with the compound of formula SM-10 under an acidic condition to obtain a compound of formula Ib or a pharmaceutically acceptable salt thereof; the operations and conditions of the deamination protecting group reaction may be conventional operations and conditions for such reactions in the art; for example, the acidic condition may be in the presence of trifluoroacetic acid;

route 6

when

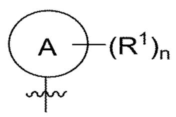

is

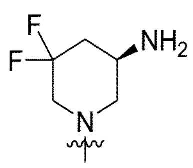 , 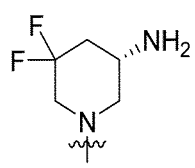 ,

the corresponding nitrogen-containing heterocyclic compound of formula I, the stereoisomer thereof, or the pharmaceutically acceptable salt thereof may be prepared by the following method:

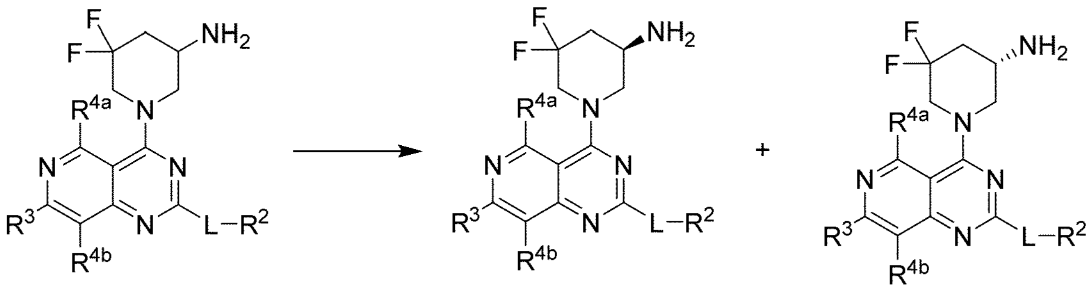

wherein $R^3$, $R^{4a}$, $R^{4b}$, L, and $R^2$ are defined as in any one of the above;
step 1: chiral column resolution; SFC separation conditions:
column: Phenomenex-Cellulose-2 (250 mm * 30 mm, 10 μm);
mobile phase: [0.1% $NH_3H_2O$ MEOH]; B%: 40% to 40%;

route 7

when

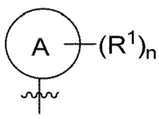

is

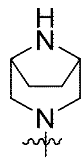

,

$R^3$ is

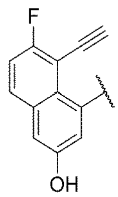

,

and $R^{4a}$ is halogen, the corresponding nitrogen-containing heterocyclic compound of formula I, the stereoisomer thereof, or the pharmaceutically acceptable salt thereof may be prepared by the following method:

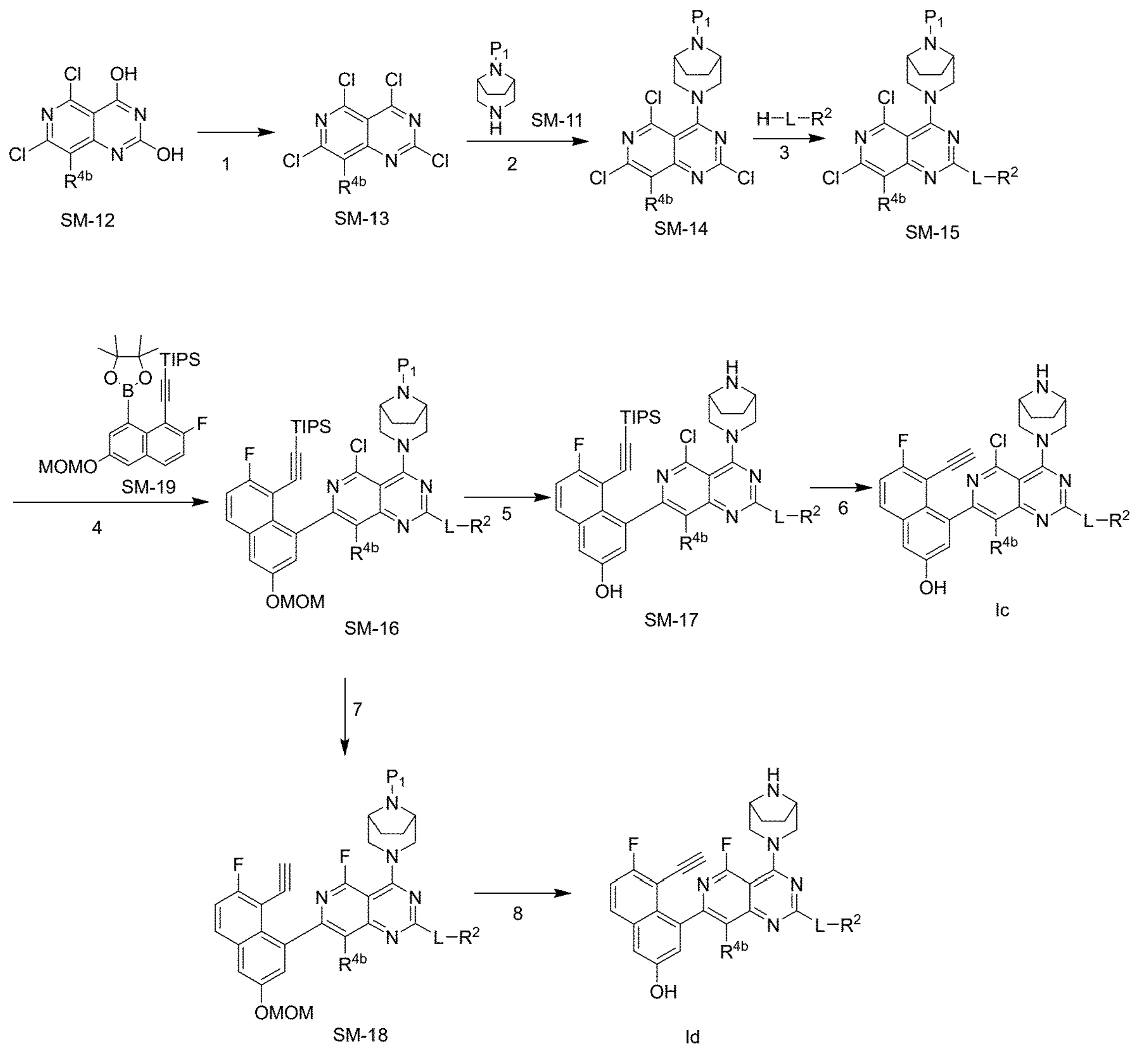


SM-12, SM-13, SM-14, SM-15, SM-19, SM-16, SM-17, Ic, SM-18, Id

wherein Pt is an amino protecting group, such as Boc, PMB, Bn, Cbz, Fmoc;

$R^{4b}$, L, and $R^2$ are defined as in any one of the above;

step 1: carrying out a chlorination reaction with a compound of formula SM-12 in the presence of a chlorinating reagent to obtain a compound of formula SM-13;

the operations and conditions of the chlorination reaction may be conventional operations and conditions for such reactions in the art; for example, the chlorinating reagent may be phosphorus oxychloride, thionyl chloride, etc.;

step 2: carrying out a substitution reaction with the compound of formula SM-13 and a compound of formula SM-11 in a solvent under an alkaline condition to obtain a compound of formula SM-14;

the operations and conditions of the substitution reaction may be conventional operations and conditions for such reactions in the art; for example, the alkaline condition may be in the presence of *N,N*-diisopropylethylamine or triethylamine, etc.;

step 3: carrying out a substitution reaction with the compound of formula SM-14 and a compound of formula H-L-$R^2$ in a solvent in the presence of a base to obtain a compound of formula SM-15;

the operations and conditions of the substitution reaction may be conventional operations and conditions for such reactions in the art; for example, the base may be sodium tert-butoxide, potassium *tert*-butoxide, NaH, etc.;

step 4: carrying out a Suzuki coupling reaction with the compound of formula SM-15 and a compound of formula SM-19 in the presence of a catalyst to obtain a compound of formula SM-16;

the operations and conditions of the Suzuki coupling reaction may be conventional operations and conditions for such reactions in the art; for example, the catalyst may be Ruphos-Pd-$G_3$/Ruphos [methanesulfonato(2-dicyclohexylphosphino-2,6-diisopropoxy-1,1-biphenyl)(2-amino-1,1-biphenyl-2-yl)palladium(II)/2-dicyclohexylphosphino-2,6-diisopropoxy-1,1-biphenyl], tetrakis(triphenylphosphine)palladium, etc.;

step 5: carrying out a deamination protecting group and dehydroxyl protecting group reaction with the compound

of formula SM-16 under an acidic condition to obtain a compound of formula SM-17;

the operations and conditions of the deamination protecting group and dehydroxyl protecting group reaction may be conventional operations and conditions for such reactions in the art; for example, the acidic condition may be in the presence of HCl/dioxane;

step 6: carrying out a dealkynyl protecting group reaction with the compound of formula SM-17 in the presence of tetrabutylammonium fluoride, tetramethylammonium fluoride, or CsF to obtain a compound of formula Ic or a pharmaceutically acceptable salt thereof;

the operations and conditions of the dealkynyl protecting group reaction may be conventional operations and conditions for such reactions in the art;

step 7: carrying out a dealkynyl protecting group reaction and a substitution reaction with the compound of formula SM-16 in the presence of tetrabutylammonium fluoride, tetramethylammonium fluoride, or CsF to obtain a compound of formula SM-18;

the operations and conditions of the dealkynyl protecting group reaction and the substitution reaction may be conventional operations and conditions for such reactions in the art;

step 8: carrying out a deamination protecting group and dehydroxyl protecting group reaction with the compound of formula SM-18 under an acidic condition to obtain a compound of formula Id or a pharmaceutically acceptable salt;

the operations and conditions of the deamination protecting group and dehydroxyl protecting group reaction may be conventional operations and conditions for such reactions in the art; for example, the acidic condition may be in the presence of HCl/dioxane;

In route 7, for the compound of formula SM-12, when $R^{4b}$ is F, the corresponding compound of formula SM-12 may be further prepared by the following method:

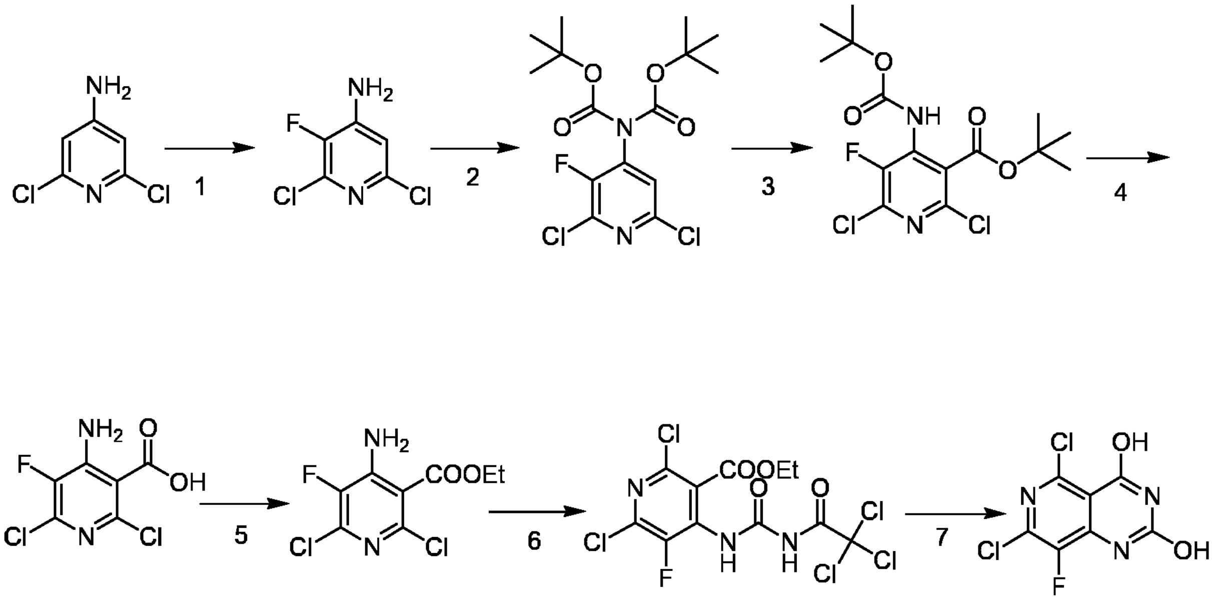


SM-12

step 1: carrying out a fluorination reaction with 2,6-dichloropyridin-4-amine and a fluorinating reagent; the fluorinating reagent is, for example, Selectfluoro(1-chloromethyl-4-fluoro-1,4-diazoniabicyclo[2.2.2]octane bis(tetrafluoroborate));

step 2: carrying out an amino protection reaction under an alkaline condition;

step 3: reacting in the presence of a strong base to obtain a product; the strong base is lithium diisopropylamide, lithium bis(trimethylsilyl)amide, sodium bis(trimethylsilyl)amide, etc.;

step 4: carrying out a deamination protecting group reaction and a hydrolysis reaction under an acidic condition;

step 5: carrying out an esterification reaction under an acidic condition;

step 6: substitution reaction;

step 7: amination ring-closing reaction;

route 8

when

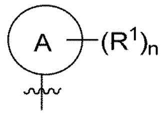

is

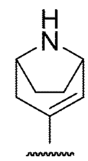

,

$R^3$ is

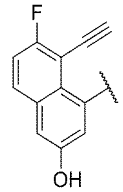

,

the corresponding nitrogen-containing heterocyclic compound of formula I, the stereoisomer thereof, or the pharmaceutically acceptable salt thereof may be prepared by the following method:

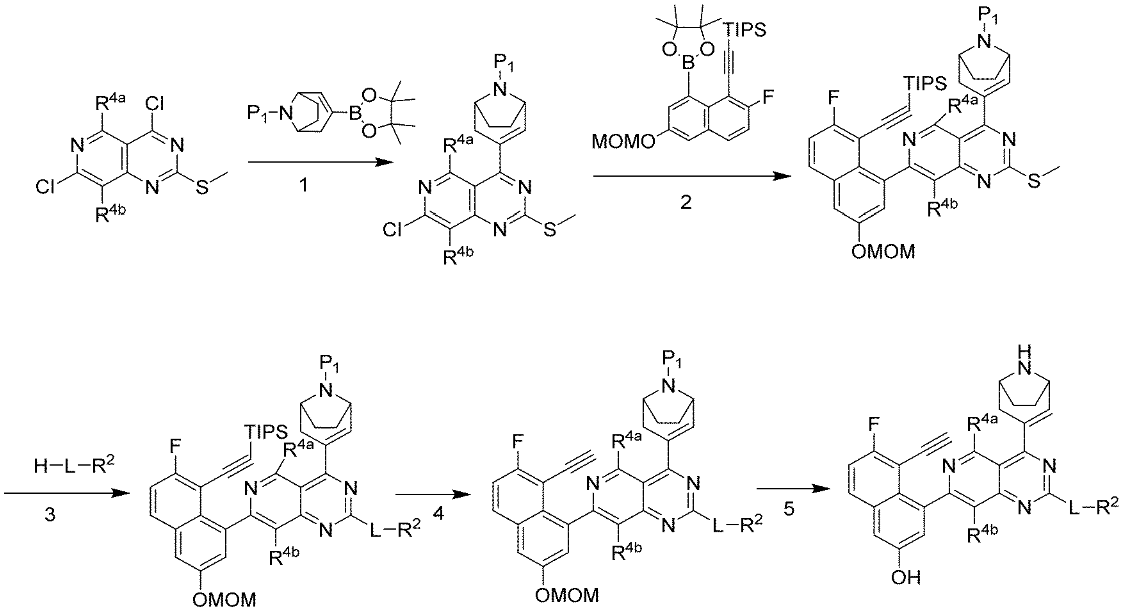


wherein Pt is an amino protecting group, such as Boc, PMB, Bn, Cbz, Fmoc;
$R^{4a}$, $R^{4b}$, L, and $R^2$ are defined as in any one of the above;
step 1: Suzuki coupling reaction;
step 2: Suzuki coupling reaction;
step 3: substitution reaction;
step 4: carrying out a dealkynyl protecting group reaction in the presence of tetrabutylammonium fluoride, tetramethylammonium fluoride, or CsF;
step 5: carrying out a deamination protecting group and dehydroxyl protecting group reaction under an acidic condition.

In the method for preparing the nitrogen-containing heterocyclic compound of formula I, the stereoisomer thereof, or the pharmaceutically acceptable salt thereof, the conditions and operations of each step of the reactions may be conventional operations and conditions for such reactions in the art.

The present disclosure also provides a compound as shown below,

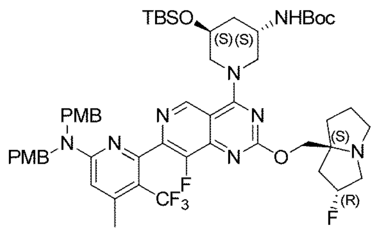
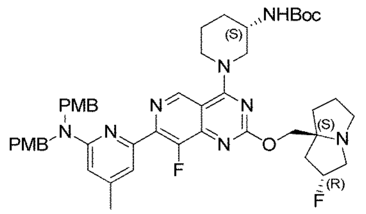
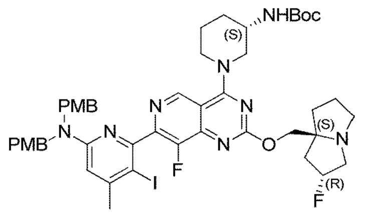
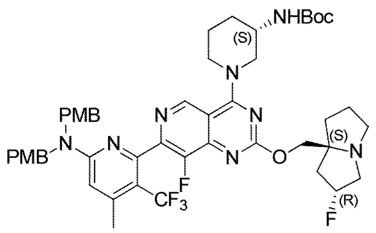
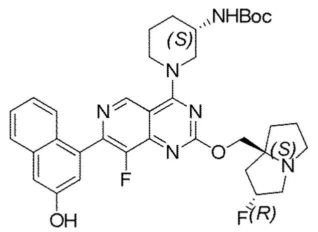
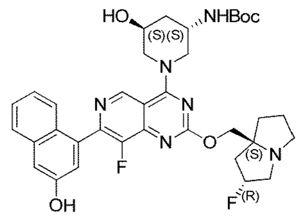
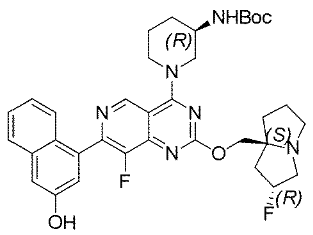
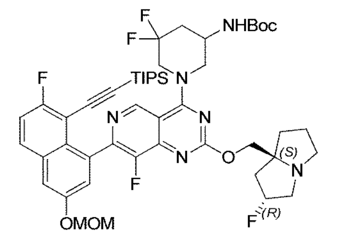
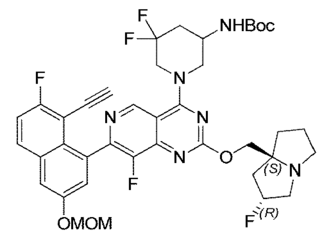
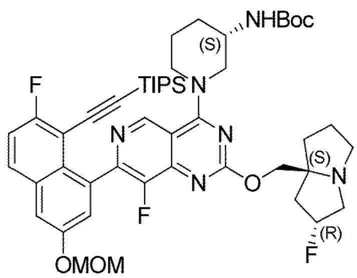
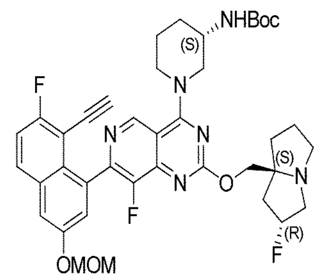
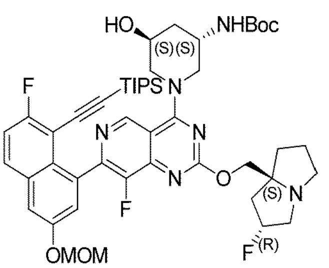
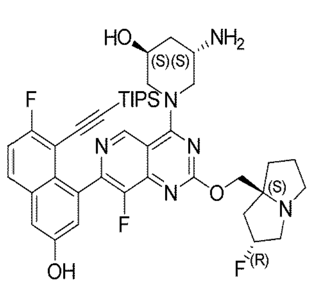
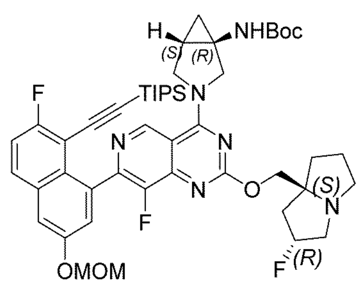
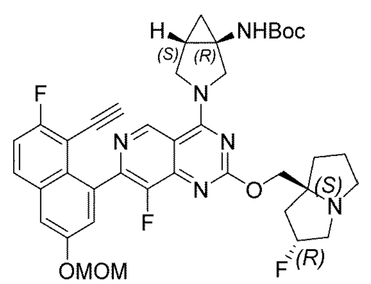

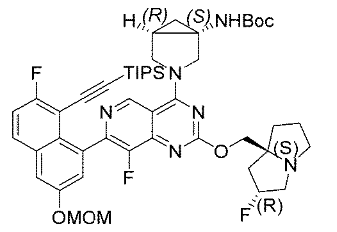

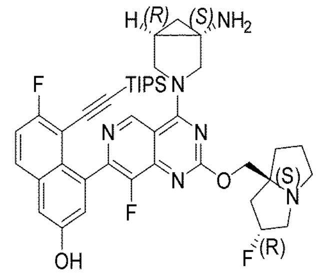

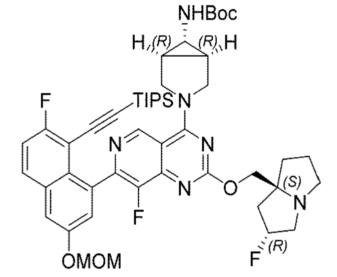

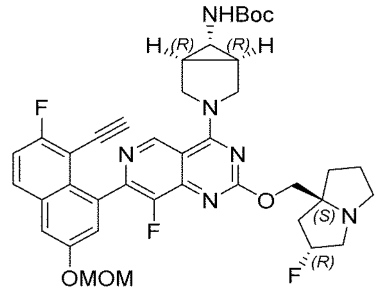

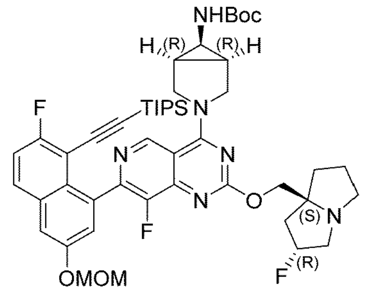

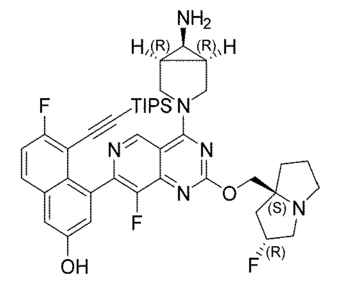

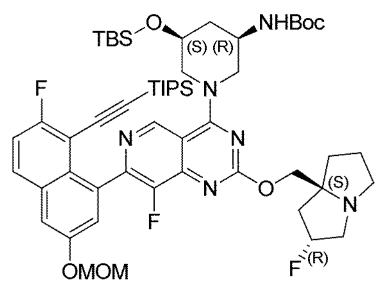

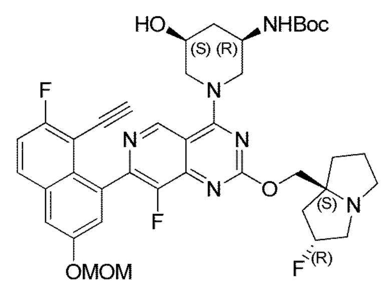

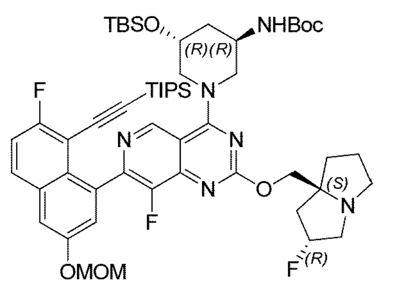

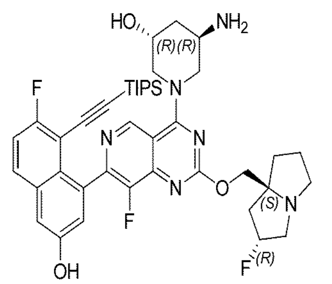

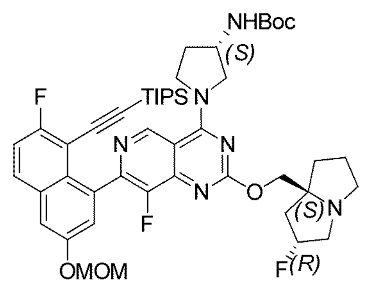

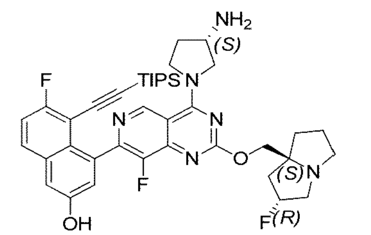

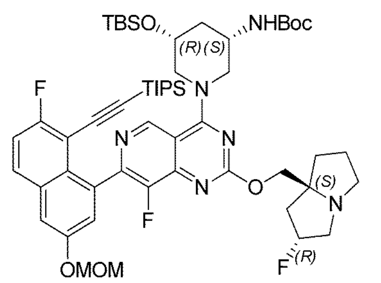

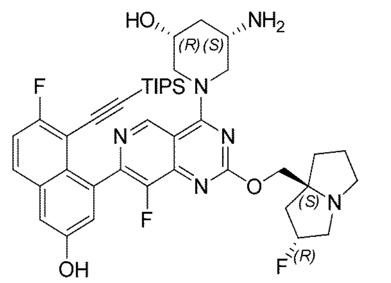

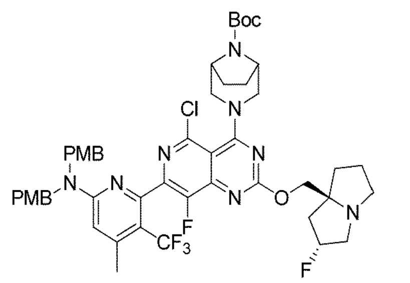

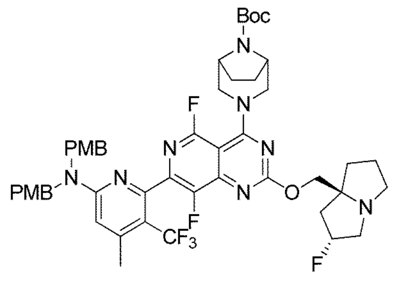

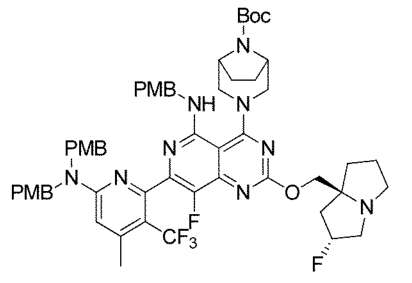

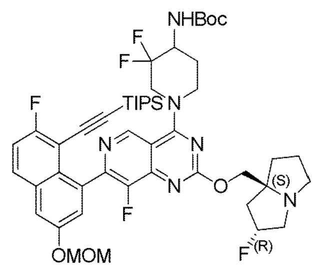

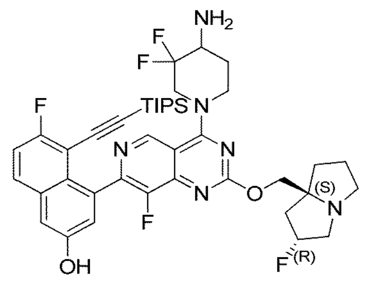

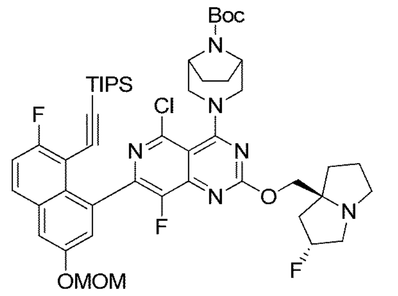

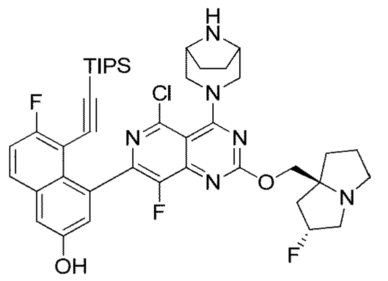

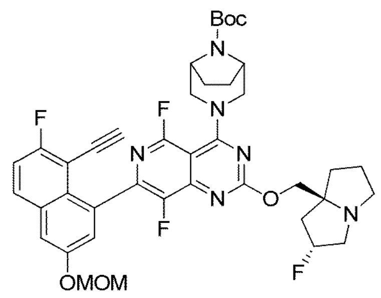

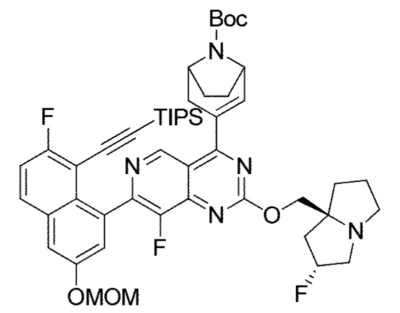

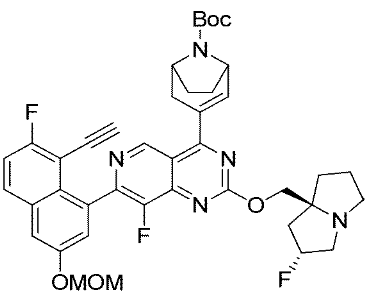

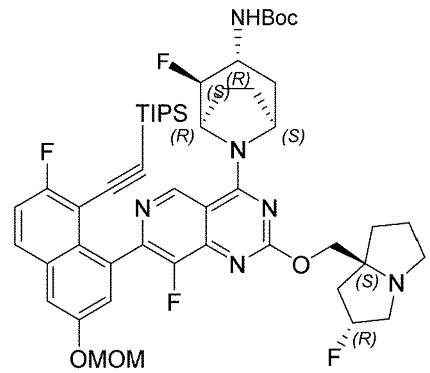

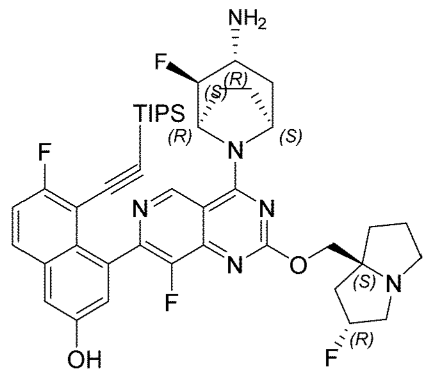

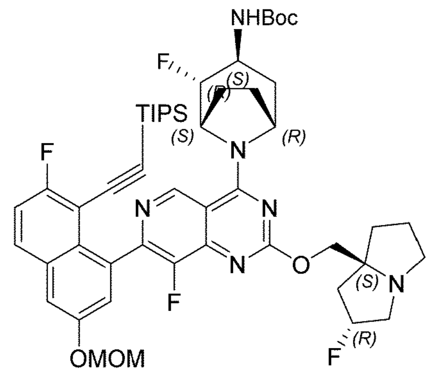

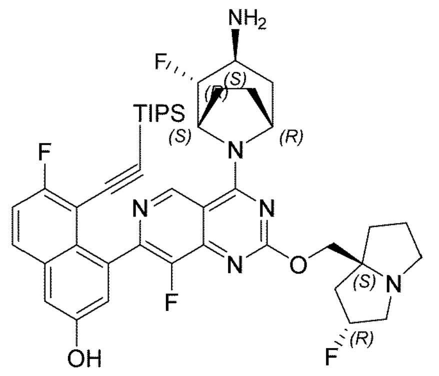

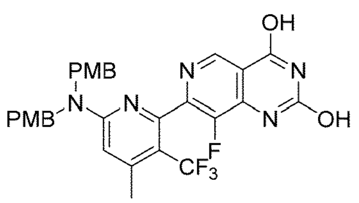
,
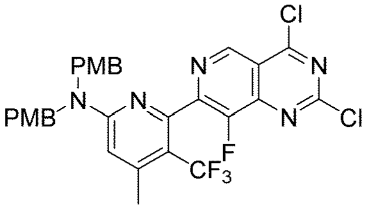
,
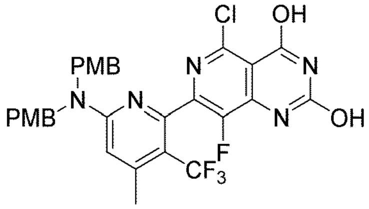
,
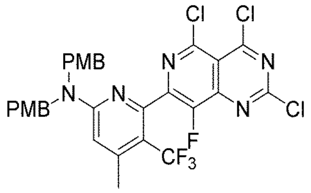
,
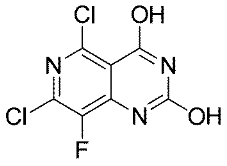
,
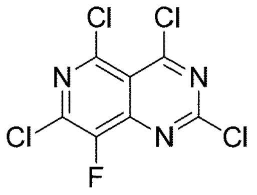
,
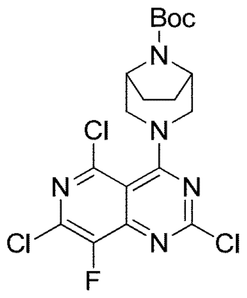
,
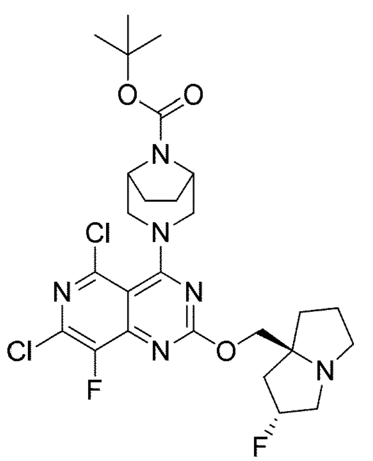
,
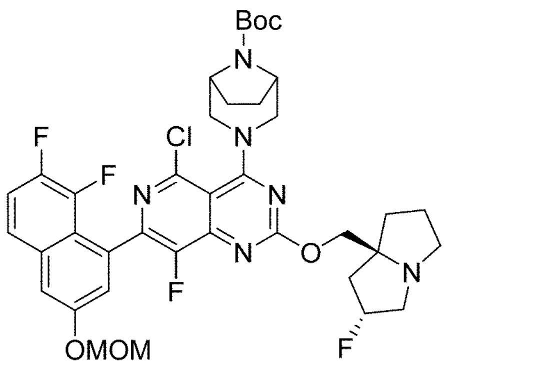
,
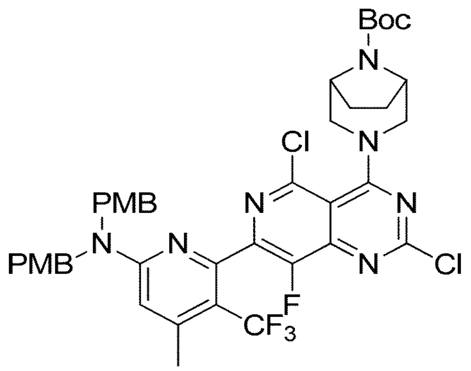
,
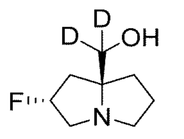
,
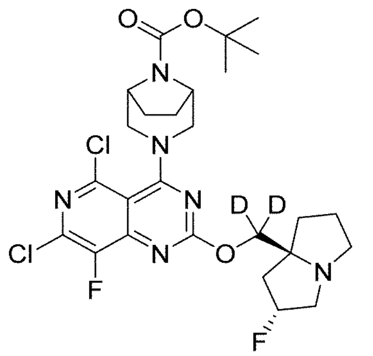
,

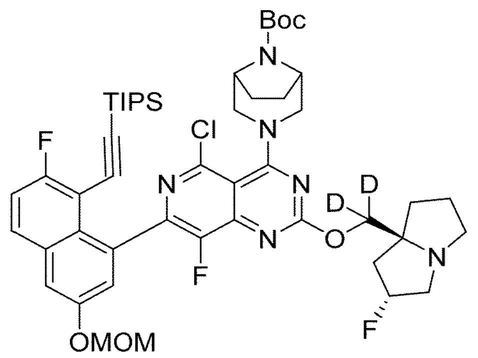

,

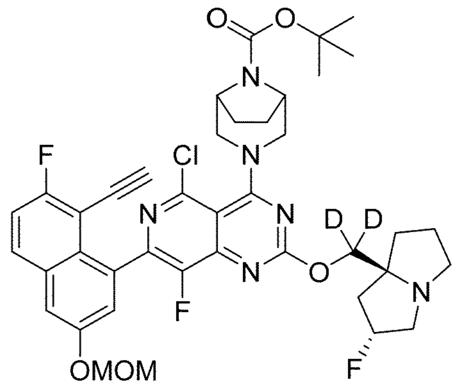

,

or

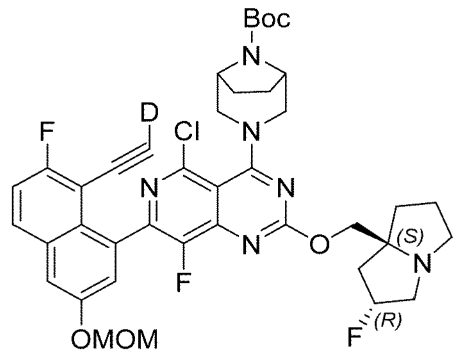

.

The present disclosure also provides a pharmaceutical composition, which comprises a therapeutically effective amount of substance A and a pharmaceutical excipient (or a pharmaceutically acceptable carrier); the substance A is the above nitrogen-containing heterocyclic compound of formula I, the stereoisomer thereof, or the pharmaceutically acceptable salt thereof.

The present disclosure also provides a use of substance A in the manufacture of a RAS inhibitor, and the substance A is the above nitrogen-containing heterocyclic compound of formula I, the stereoisomer thereof, or the pharmaceutically acceptable salt thereof. In the use, the RAS inhibitor may be used in mammalian organisms *in vivo;* it may also be used *in vitro,* mainly for experimental purposes, for example: as a standard sample or control sample to provide comparison, or making a kit according to the conventional method in the art to provide rapid detection for the effect of inhibiting RAS.

The present disclosure also provides a use of substance A in the manufacture of a medicament, and the medicament is used to treat or prevent a disease mediated by RAS; the substance A is the above nitrogen-containing heterocyclic compound of formula I, the stereoisomer thereof, or the pharmaceutically acceptable salt thereof; the substance A is in a therapeutically effective amount.

The present disclosure also provides a use of substance A in the manufacture of a medicament, and the medicament is used to treat and/or prevent a disease mediated by RAS; the substance A is the above nitrogen-containing heterocyclic compound of formula I, the stereoisomer thereof, or the pharmaceutically acceptable salt thereof; the substance A is in a therapeutically effective amount.

The present disclosure also provides a use of substance A in the manufacture of a medicament, and the medicament is used to treat or prevent cancer; the substance A is the above nitrogen-containing heterocyclic compound of formula I, the stereoisomer thereof, or the pharmaceutically acceptable salt thereof; the substance A is in a therapeutically effective amount.

The present disclosure also provides a use of substance A in the manufacture of a medicament, and the medicament is used to treat and/or prevent cancer; the substance A is the above nitrogen-containing heterocyclic compound of formula I, the stereoisomer thereof, or the pharmaceutically acceptable salt thereof; the substance A is in a therapeutically effective amount.

The present disclosure also provides a method for inhibiting RAS, which comprises administering a therapeutically effective amount of substance A to a patient; the substance A is the above nitrogen-containing heterocyclic compound of formula I, the stereoisomer thereof, or the pharmaceutically acceptable salt thereof.

The present disclosure also provides a method for treating or preventing a disease mediated by RAS, which comprises administering a therapeutically effective amount of substance A to a patient; the substance A is the above nitrogen-containing heterocyclic compound of formula I, the stereoisomer thereof, or the pharmaceutically acceptable salt thereof.

The present disclosure also provides a method for treating and/or preventing a disease mediated by RAS, which comprises administering a therapeutically effective amount of substance A to a patient; the substance A is the above nitrogen-containing heterocyclic compound of formula I, the stereoisomer thereof, or the pharmaceutically acceptable salt thereof.

The present disclosure also provides a method for treating or preventing cancer, which comprises administering a therapeutically effective amount of substance A to a patient; the substance A is the above nitrogen-containing heterocyclic compound of formula I, the stereoisomer thereof, or the pharmaceutically acceptable salt thereof.

The present disclosure also provides a method for treating and/or preventing cancer, which comprises administering a therapeutically effective amount of substance A to a patient; the substance A is the above nitrogen-containing heterocyclic compound of formula I, the stereoisomer thereof, or the pharmaceutically acceptable salt thereof. The above RAS may be KRAS or a KRAS mutation; for example, KRAS G12D, KRAS G12V.

The above disease mediated by RAS is, for example, cancer.

The above cancer may be selected from one or more than one of colon cancer, pancreatic cancer, breast cancer, prostate cancer, lung cancer, brain cancer, ovarian cancer, cervical cancer, testicular cancer, kidney cancer, head or neck cancer, bone cancer, skin cancer, rectal cancer, liver cancer, colorectal cancer, non-small cell lung cancer, small cell lung cancer, esophageal cancer, gastric cancer, thyroid cancer, bladder cancer, lymphoma, leukemia, and melanoma.

The above cancer may be gastric cancer or pancreatic cancer.

In addition to the foregoing, when used in the specification and claims of the present disclosure, the following terms have the meanings shown below unless otherwise specified.

In the present disclosure, when a carbon atom with "*" is a chiral carbon atom, it is in an R configuration, an S configuration, or a mixture thereof.

The term "more than one" refers to 2, 3, 4, or 5.

The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the neutral form of the compound into contact with a sufficient amount of pharmaceutically acceptable base in a pure solution or a suitable inert solvent. Pharmaceutically acceptable base addition salts include, but are not limited to, lithium salts, sodium salts, potassium salts, calcium salts, aluminum salts, magnesium salts, zinc salts, bismuth salts, ammonium salts, diethanolamine salts. When the compound of the present disclosure contains a relatively basic functional group, an acid addition salt can be obtained by bringing the neutral form of the compound into contact with a sufficient amount of pharmaceutically acceptable acid in a pure solution or a suitable inert solvent. The pharmaceutically acceptable acids include inorganic acids and organic acids (such as trifluoroacetic acid, hydrochloric acid). For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

As used herein, the compound of formula I of the present disclosure may contain one or more than one chiral center and exist in different optically active forms. When a compound contains a chiral center, the compound contains enantiomers. The present disclosure includes both isomers and mixtures of isomers, such as racemic mixtures. Enantiomers can be resolved by methods known in the art, such as crystallization and chiral chromatography. When the compound of formula I contains more than one chiral center, diastereomers may exist. The present disclosure includes resolved optically pure specific isomers as well as mixtures of diastereomers. Diastereomers can be resolved by methods known in the art, such as crystallization as well as preparative chromatography. The term "stereoisomer" includes conformational isomers and configurational isomers, wherein configurational isomers mainly include *cis-trans* isomers and optical isomers. The compounds described in the present disclosure may exist in the form of stereoisomers, and therefore encompass all possible stereoisomer forms, including but not limited to *cis-trans* isomers, enantiomers, diastereomers, atropisomers, etc. The compounds of the present disclosure may also exist in the form of any combination or any mixture of the aforementioned stereoisomers, such as an equivalent mixture of mesomers, racemates, atropisomers. For example, a single enantiomer, a single diastereomer, a mixture of the above, or a single atropisomer or a mixture thereof. When the compound described in the present disclosure contains an olefin double bond, unless otherwise stated, it includes *cis*-isomers and *trans*-isomers, and any combination thereof. The atropisomer of the present disclosure is an axial or planar chiral stereoisomer produced based on restricted intramolecular rotation. The compounds of the present disclosure may have two atropisomers originating from axial asymmetry, which arises due to the fact that when the substituent $R^3$ is a cyclic group such as a $C_6$-$C_{10}$ aryl group and a 5- to 14-membered heteroaryl group (in particular when there are substituents at the ortho positions at both ends of the linker bond or a group with a larger spatial structure

at the ortho positions of the linker bond) and when the linker bond between a substituted pyrido[4,3-d]pyrimidine ring is hindered by spatial steric hindrance, resulting in hindered rotation. Regarding the atropisomer of the present disclosure, wherein the compound has the structure of formula I, or the compound of formula I has isomers generated by asymmetric carbon, etc., it represents either a pair of atropisomers present in each isomeric compound. And as drugs, atropisomers having excellent activity are preferred. These stereoisomers can be separated, purified, and enriched by asymmetric synthesis methods or chiral separation methods (including but not limited to thin-layer chromatography, rotation chromatography, column chromatography, gas chromatography, high-pressure liquid chromatography, etc.), or can be obtained by chiral separation by forming bonds or salts with other chiral compounds. The term "single stereoisomer" refers to one stereoisomer of the compound of the present disclosure, the mass content of which is not less than 95% relative to all stereoisomers of the compound. The compound of formula I has optical isomers derived from asymmetric carbon, axial asymmetry, etc. If necessary, single isomers can be obtained by resolution by methods known in the art, such as crystallization or chromatography (such as chiral chromatography).

As mentioned above, the present disclosure provides the compounds shown in the various structures above, or their *cis-trans* isomers, mesomers, racemates, enantiomers, diastereomers, atropisomers, or mixtures thereof, where the "mixtures thereof" includes any form of mixing between any one of the aforementioned stereoisomers (such as *cis-trans* isomers, enantiomers, diastereomers, atropisomers) and/or mixtures (mesomers, racemates), such as a mixture of *cis-trans* isomers, a mixture of enantiomers and diastereomers, a mixture of diastereomers, a mixture of atropisomers, or a mixing of *cis-trans* isomers and racemates, a mixing of enantiomer and diastereomer mixtures, a mixing of atropisomers and diastereomers mixtures.

The compound of formula I, the stereoisomer thereof, and the pharmaceutically acceptable salt thereof are intended to encompass any isotopically labeled (or "radiolabeled") variant of the compound of formula I, the stereoisomer thereof, or the pharmaceutically acceptable salt thereof. Such variants may be obtained by substitution of one or more than one atom in the compound of formula I, the stereoisomer thereof, or the pharmaceutically acceptable salt thereof, by atoms having an atomic mass or mass number different from that normally found in nature. The radionuclide used will depend on the specific application of the radiolabeled variant. For example, for *in vitro* receptor labeling and competition assays, $^{3}H$ or $^{14}C$ is often useful. For radiographic applications, $^{11}C$ or $^{18}F$ is often useful.

Specific isotopic variants of the compounds of the present disclosure, in particular those in which one or more than one radioactive isotope has been incorporated, can be advantageous, for example, to investigate the mechanism of action or the distribution of the active ingredient *in vivo;* due to the relative ease of preparation and detectability, compounds labeled with $^{3}H$ or $^{14}C$ isotopes are particularly suitable for this purpose. In addition, the incorporation of an isotope such as deuterium can yield particular therapeutic benefits due to the better metabolic stability of the compound, for example, by extending the half-life *in vivo* or by reducing the effective dose required; thus such modifications of the compounds of the present disclosure may also constitute preferred embodiments of the present disclosure in some cases. Isotopic variants of the compounds of the present disclosure can be prepared by methods known to those skilled in the art, for example, by the methods described below and in the operating examples, by using corresponding isotopically modified specific reagents and/or starting compounds.

The term "pharmaceutical composition" of the present disclosure refers to a formulation containing the compound of the present disclosure and a medium generally accepted in the art for delivering a biologically active compound to a mammal (e.g., a human). The medium includes a pharmaceutically acceptable carrier. The purpose of the pharmaceutical composition is to promote the administration to an organism, facilitate the absorption of the active ingredient and thus exert its biological activity.

In the present disclosure, "pharmaceutically acceptable" refers to a substance (such as a pharmaceutical excipient) that does not affect the biological activity or properties of the compounds of the present disclosure and is relatively non-toxic, that is, the substance can be administered to an individual without causing an adverse biological reaction or interacting with any component contained in the composition in an adverse manner.

The term "pharmaceutical excipients" or "pharmaceutically acceptable carrier" refers to the excipients and additives used in the manufacture of drugs and the formulation of prescriptions, which are all substances contained in pharmaceutical preparations except active ingredients. Available in the Pharmacopoeia of the People’s Republic of China (2015 Edition) Part IV, or, Handbook of Pharmaceutical Excipients (Raymond C Rowe, 2009 Sixth Edition). The excipient is mainly used to provide a safe, stable, and functional pharmaceutical composition, and can also provide a method for the subject to dissolve the active ingredient at a desired rate after administration, or to facilitate effective absorption of the active ingredient after the subject receives administration of the composition. The pharmaceutical excipient can be an inert filler or provide a certain function, such as stabilizing the overall pH value of the composition or preventing degradation of the active ingredient in the composition. The pharmaceutical excipients may include one or more than one of the following excipients: binders, suspending agents, emulsifying agents, diluents, fillers, granulating agents, adhesive agents, disintegrating agents, lubricants, anti-adhesive agents, glidants, wetting agents, gelling agents, absorption retardants, dissolution inhibitors, enhancers, adsorbents, buffers, chelating agents, preservatives, coloring agents, flavoring agents, and sweeteners.

The pharmaceutical composition of the present disclosure can be prepared according to the disclosure using any method known to those skilled in the art. For example, conventional mixing, dissolving, granulating, emulsifying, levigating, encapsulating, embedding, or lyophilizing processes.

When used as a drug, the nitrogen-containing heterocyclic compound of formula I, the stereoisomer thereof, or the pharmaceutically acceptable salt thereof can be administered in any form of a pharmaceutical composition. These compositions may be prepared according to methods well known in the pharmaceutical art and may be administered by a variety of routes, depending on the desired local or systemic treatment and the area to be treated. Administration may be topical (including epidermal and transdermal, ocular and mucosal, including intranasal, vaginal, and rectal delivery), pulmonary (for example, by inhalation or insufflation of powder or aerosol, including by nebulizer; intratracheal or intranasal), oral (solid and liquid formulations), or parenteral administration. Examples of solid oral formulations include, but are not limited to, powders, capsules, caplets, softgels, and tablets. Examples of liquid formulations for oral or mucosal administration include, but are not limited to, suspensions, emulsions, elixirs, and solutions. Examples of formulations for topical use include, but are not limited to, emulsions, gels, ointments, creams, patches, pastes, foams, lotions, drops, or serum formulations. Examples of formulations for parenteral administration include, but are not limited to, solutions for injection, dry formulations that can be dissolved or suspended in a pharmaceutically acceptable carrier, suspensions for injection, and emulsions for injection. Pharmaceutical compositions and preparations for external administration may include transdermal patches, salves, emulsions, ointments, gels, drops, suppositories, sprays, liquids, and powders. Examples of other suitable formulations of the pharmaceutical composition include, but are not limited to, eye drops and other ophthalmic formulations; aerosols: such as nasal sprays or inhalants. Oral administration may include dosage forms formulated for once daily or twice daily (BID) administration. Parenteral administration includes intravenous, intraarterial, subcutaneous, intraperitoneal, or intramuscular injection or infusion; or intracranial, such as intrathecal or intraventricular administration. Parenteral administration may be in the form of a single injection dose, or may be through a continuous infusion pump. Conventional pharmaceutical carriers, water, powdered or oily bases, thickening agents, etc. may be necessary or desirable. Pharmaceutical compositions included in the present disclosure may also be in controlled or delayed release dosage forms (e.g., liposomes or microspheres).

The term "treatment" refers to therapeutic therapy or palliative measures. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder. "Treatment" can also refer to prolonging the survival compared with the expected survival without treatment.

The term "prevention" refers to the reduction of the risk of acquiring or developing diseases or disorders.

The term "therapeutically effective amount" refers to the amount of a compound that is sufficient to effectively treat the diseases or disorders described herein when administered to a patient. The "therapeutically effective amount" will vary according to the compound, the disease and its severity, and the age of the patient to be treated, but it can be adjusted by those skilled in the art as needed.

The term "patient" refers to any animal, preferably a mammal, and most preferably a human, to whom the compound or composition will be or has been administered according to examples of the present disclosure. The term "mammal" includes any mammal. Examples of the mammal include, but are not limited to, cattle, horses, sheep, pigs, cats, dogs, mice, rats, rabbits, guinea pigs, monkeys, humans, etc., with humans being the most preferred.

Unless otherwise specified, the following definitions used herein shall apply. For the purposes of the present disclosure, chemical elements are consistent with the CAS edition of the Periodic Table of the Elements, and "Handbook of Chemistry and Physics", 75th edition, 1994. In addition, general principles of organic chemistry can be found in the description of "Organic Chemistry", Thomas Sorrell, University Science Books, Sausalito:1999, and "March's Advanced Organic Chemistry" by Michael B. Smith and Jerry March, John Wiley & Sons, New York: 2007, the entire contents of which are incorporated herein by reference in their entireties.

In the present description, groups and substituents thereof can be selected by those skilled in the art to provide stable structural moieties and compounds. When a substituent is described by a conventional chemical formula written from left to right, the substituent also includes the chemically equivalent substituent obtained when the structural formula is written from right to left.

Certain chemical groups defined herein are preceded by a simplified symbol to represent the total number of carbon atoms present in the group. For example, $C_1$-$C_6$ alkyl refers to an alkyl group having a total of 1, 2, 3, 4, 5, or 6 carbon atoms as defined below. The total number of carbon atoms in the simplified symbol does not include the carbon atom that may exist in the substituents of the group.

The numerical range defined in the substituent herein such as 0 to 4, 1-4, 1 to 3, etc. indicates the integer within the range, for example, 1-6 is 1, 2, 3, 4, 5, 6.

In addition to the foregoing, when used in the description and claims of the present disclosure, the following

terms have the meanings shown below unless otherwise specified.

The term "include" or "comprise" is an open-ended expression, that is, it includes the contents specified in the present disclosure, but does not exclude other aspects.

The term "substituted" means one or more than one hydrogen atom on a specific atom is substituted by the substituent, as long as the valence of the specific atom is normal and the substituted compound is stable.

Generally speaking, the term "substituted" means that one or more than one hydrogen atom in a given structure is replaced by a specified substituent. Further, when the group is substituted by more than one substituent, the substituents are independent of each other, that is, the more than one substituent may be different from each other or may be the same. Unless otherwise indicated, a substituent group may be substituted at each substitutable position of the substituted group. When more than one position in a given structural formula can be substituted by one or more than one substituent selected from a specific group, the substituents may be identically or differently substituted at each position.

In various parts of this specification, substituents of the compounds disclosed herein are disclosed according to group type or range. In particular, the present disclosure includes each independent sub-combination of each member of these group types and ranges. The term "$C_x$-$C_y$ alkyl" refers to a linear or branched saturated hydrocarbon containing x to y carbon atoms. For example, the term "$C_1$-$C_6$ alkyl" or "$C_{1-6}$ alkyl" specifically refers to independently disclosed methyl, ethyl, $C_3$ alkyl, $C_4$ alkyl, $C_5$ alkyl, and $C_6$ alkyl; the term "$C_{1-4}$ alkyl" specifically refers to independently disclosed methyl, ethyl, $C_3$ alkyl (i.e., propyl, including *n*-propyl and isopropyl), and $C_4$ alkyl (i.e., butyl, including *n*-butyl, isobutyl, *sec*-butyl, and *tert*-butyl).

As used herein, the terms "moiety", "structural moiety", "chemical moiety", "group", and "chemical group" refer to a specific fragment or functional group in a molecule. Chemical moieties are generally considered as chemical entities embedded in or attached to molecules.

When a listed substituent does not indicate through which atom it is attached to the general chemical structure (including but not specifically mentioned compounds), such substituent may be bonded through any of its atoms. A combination of the substituent and/or the variant thereof is allowed only when the combination results in a stable compound.

When an arbitrary variable (e.g., $R^{1a}$) appears many times in the definition of a compound, the definition of each occurrence of the variable has nothing to do with the definitions of other occurrences, and their meanings are independent of each other and have no influence on each other. Therefore, if a group is substituted by 1, 2, or 3 $R^{1a}$ groups, that is, the group may be substituted by up to 3 $R^{1a}$ groups, and the definition of $R^{1a}$ groups of this position and the definition of $R^{1a}$ groups of the remaining positions are independent of each other. Additionally, a combination of substituents and/or variables is allowed only when the combination results in a stable compound.

When a listed group does not explicitly indicate that it has a substituent, such group only refers to an unsubstituted group. For example, when "$C_1$-$C_4$ alkyl" is not preceded by the limitation "substituted or unsubstituted", it refers only to "$C_1$-$C_4$ alkyl" itself or "unsubstituted $C_1$-$C_4$ alkyl".

In various parts of the present disclosure, linking substituents are described. When the structure clearly requires a linking group, the Markush variables listed for that group should be understood as linking groups. For example, if the structure requires a linking group and the definition of the Markush group for that variable lists "alkyl", it should be understood that the "alkyl" represents a linked alkylene group.

In some specific structures, when an alkyl group is expressly represented as a linking group, then the alkyl group represents a linked alkylene group, for example, $C_1$-$C_6$ alkyl in the group "halo-Ci-Cs alkyl" should be understood as $C_1$-$C_6$ alkylene.

The term "halogen" refers to fluorine, chlorine, bromine, or iodine, especially For Cl.

In the present disclosure, the term "alkyl", as a group or as a moiety of another group (for example, as used in groups such as haloalkyl, deuterated alkyl), refers to a branched and linear saturated aliphatic hydrocarbon group with a specified number of carbon atoms, consisting only of carbon atoms and hydrogen atoms, having, for example, 1 to 12 (preferably 1 to 8, more preferably 1 to 6, even more preferably 1 to 4) carbon atoms, and being connected to the rest of the molecule by a single bond, wherein propyl is $C_3$ alkyl (including isomers, such as *n*-propyl or isopropyl); butyl is $C_4$ alkyl (including isomers, such as *n*-butyl, *sec*-butyl, isobutyl, or *tert*-butyl); pentyl is $C_5$ alkyl (including isomers, such as *n*-pentyl, 1-methyl-butyl, 1-ethyl-propyl, 2-methyl-1-butyl, 3-methyl-1-butyl, isopentyl, *tert*-pentyl, or neopentyl); hexyl is $C_6$ alkyl (including isomers, such as *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl). Examples include, but are not limited to, methyl, ethyl, *n*-propyl, isopropyl, *n*-butyl, isobutyl, *sec*-butyl, *tert*-butyl, *n*-pentyl, 2-methylbutyl, 2,2-dimethylpropyl, *n*-hexyl, *n*-heptyl, 2-methylhexyl, 3-methylhexyl, *n*-octyl, nonyl, decyl, and other similar alkyl groups.

In the present disclosure, the term "alkylene", as a group or a moiety of another group, means a saturated divalent hydrocarbon group obtained by removing two hydrogen atoms from a saturated linear or branched hydrocarbon group; that is, one hydrogen in alkyl is substituted, and the alkyl is defined as above. Examples of alkylene groups include methylene ($-CH_2-$), ethylene {including $-CH_2CH_2-$ or $-CH(CH_3)-$}, isopropylene {including $-CH(CH_3)CH_2-$ or - $C(CH_3)_2-$},

etc.

In the present disclosure, the term "alkoxy", as a group or a moiety of another group, refers to -O-alkyl, and the alkyl is defined as above.

In the present disclosure, the term "alkylthio", as a group or a moiety of another group, refers to -S-alkyl, and the alkyl is defined as above.

In the present disclosure, the term "alkenyl", as a group or a moiety of another group, refers to a linear or branched hydrocarbon chain group having at least one double bond, consisting only of carbon atoms and hydrogen atoms, having, for example, 2 to 12 (preferably 2 to 8, more preferably 2 to 6, most preferably 2 to 4) carbon atoms, and being connected to the rest of the molecule by a single bond. Examples include but are but not limited to vinyl, *n*-propenyl, isopropenyl, *n*-butenyl, isobutenyl, *sec*-butenyl, *tert*-butenyl, *n*-pentenyl, 2-methylbutenyl, 2,2-dimethylpropenyl, *n*-hexenyl, heptenyl, 2-methylhexenyl, 3-methylhexenyl, octenyl, nonenyl, decenyl, etc.

In the present disclosure, the term "alkynyl", as a group or a moiety of another group, refers to a linear or branched hydrocarbon chain group having at least one triple bond, consisting only of carbon atoms and hydrogen atoms, having, for example, 2 to 12 (preferably 2 to 8, more preferably 2 to 6, most preferably 2 to 4) carbon atoms, and being connected to the rest of the molecule by a single bond. Examples include but are but not limited to ethynyl, *n*-propynyl, isopropynyl, *n*-butynyl, isobutynyl, *sec*-butynyl, *tert*-butynyl, *n*-pentynyl, 2-methylbutynyl, 2,2-dimethylpropynyl, *n*-hexynyl, heptynyl, 2-methylhexynyl, 3-methylhexynyl, octynyl, nonynyl, decynyl, etc.

In the present disclosure, the term "cycloalkyl", as a group or a moiety of another group, refers to a saturated monocyclic or polycyclic (for example, bicyclic, tricyclic, or polycyclic bridged ring, fused ring (condensed ring), or spiro ring system) carbocyclic substituent, and it can be connected to the rest of the molecule by a single bond via any suitable carbon atom; e.g., 3- to 15-membered cycloalkyl with 3 to 15 carbon atoms, preferably 3- to 10-membered cycloalkyl with 3 to 10 carbon atoms, more preferably 3- to 7-membered cycloalkyl with 3 to 7 carbon atoms, and most preferably 3- to 6-membered cycloalkyl with 3 to 6 carbon atoms. In a certain embodiment, a typical monocyclic cycloalkyl is, for example, cyclopropyl, cyclobutyl, cyclopentyl, cyclohexyl, or cycloheptyl.

In the present disclosure, the term "cycloalkenyl", as a group or a moiety of another group, refers to a monocyclic or polycyclic (for example, bicyclic, tricyclic, or polycyclic bridged ring, fused ring (condensed ring), or spiro ring system) cyclic hydrocarbon group having at least one double bond (such as carbon-carbon double bond), and it can be connected to the rest of the molecule by a single bond via any suitable carbon atom; having 3 to 15 carbon atoms, preferably having 3 to 10 carbon atoms, and more preferably having 3 to 6 carbon atoms. The "cycloalkenyl" is not aromatic. In some examples, "cycloalkenyl" is a monocyclic, unsaturated carbocycloalkenyl group having 5 to 6 ring atoms ("5- to 6-membered cycloalkenyl"). The term includes, but is not limited to, cyclopentenyl (for example,

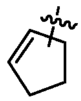

), cyclopentadienyl (for example,

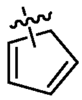

), cyclohexenyl (for example,

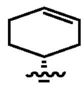

), or cyclohexadienyl, as well as stereoisomers thereof.

In the present disclosure, the term "heterocycloalkyl", as a group or a moiety of another group, refers to a stable 3- to 20-membered (preferably 3- to 12-membered, more preferably 4- to 10-membered, most preferably 3- to 7-membered) saturated monocyclic or polycyclic (for example, bicyclic, tricyclic, or polycyclic bridged ring, fused ring (condensed ring), or spiro ring system) heterocyclic hydrocarbon group consisting of 2 to 14 (preferably 2 to 6) carbon atoms and 1 to 6 heteroatoms or heteroatom groups selected from N, O, S, S(=O), and $S(=O)_2$, preferably 4- to 10-membered heterocycloalkyl containing 1, 2, or 3 ring heteroatoms independently selected from N, O, and S. The ring system of a heterocycloalkyl bicyclic ring may include one or more than one heteroatom in one or both rings; and be saturated. In

some embodiments, "heterocycloalkyl" is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl. Exemplary 3-membered heterocycloalkyl groups include, but are not limited to, aziridinyl, oxiranyl, and thiiranyl, or stereoisomers thereof; exemplary 4-membered heterocycloalkyl groups include, but are not limited to, azetidinyl, oxetanyl, thietanyl, or isomers and stereoisomers thereof; exemplary 5-membered heterocycloalkyl groups include, but are not limited to, tetrahydrofuranyl, tetrahydrothienyl, pyrrolidinyl

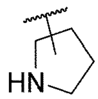

(for example,

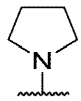

or

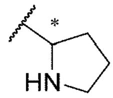

,

for another example,

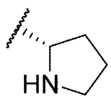

), thiazolidinyl, isothiazolidinyl, oxazolidinyl, isoxazolidinyl, imidazolidinyl, pyrazolidinyl, dioxolyl, oxathiofuryl, dithio furyl, or isomers and stereoisomers thereof. Exemplary 6-membered heterocycloalkyl groups include, but are not limited to, piperidinyl

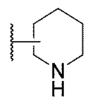

(for example,

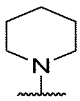

or

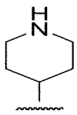

), tetrahydropyranyl, thiocyclopentanyl, morpholinyl, thiomorpholinyl, dithianyl, dioxanyl, piperazinyl

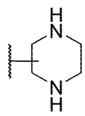

(for example,

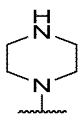

), triazinanyl,

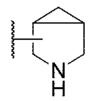

(for example,

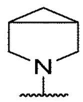

), or isomers and stereoisomers thereof. Exemplary 7-membered heterocycloalkyl groups include, but are not limited to,

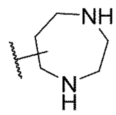

(for example,

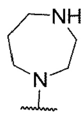

),

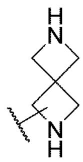

(for example,

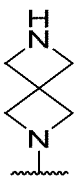

),

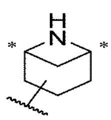

(for example,

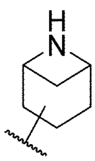

, for another example,

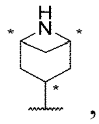

for yet another example,

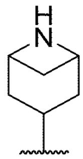

), or

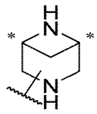

(for example,

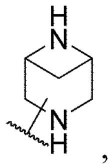

for another example,

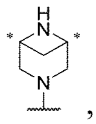

for yet another example,

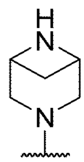

), or isomers and stereoisomers thereof. Exemplary 8-membered heterocycloalkyl groups include, but are not limited to,

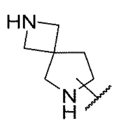

(for example,

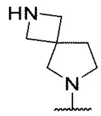

),

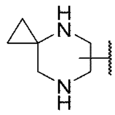

(for example,

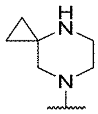

),

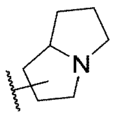

(for example,

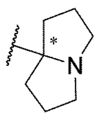

,

for yet another example,

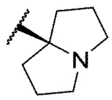

),

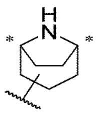

(for example,

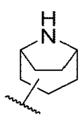

,

for another example,

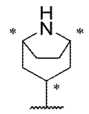

or

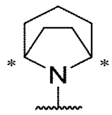,

for yet another example,

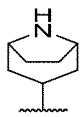, 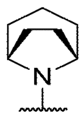, or 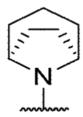

), or

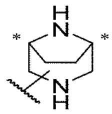

(for example,

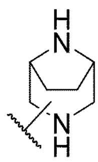,

for another example,

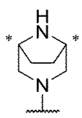,

for yet another example,

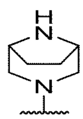

), or isomers and stereoisomers thereof. Exemplary 9-membered heterocycloalkyl groups include, but are not limited to,

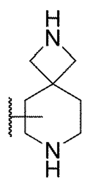

(for example,

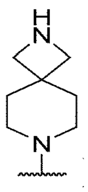

) or

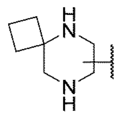

(for example,

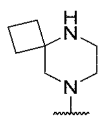

), or isomers and stereoisomers thereof. Exemplary 10-membered heterocycloalkyl groups include, but are not limited to,

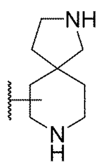

(for example,

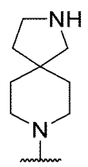

) or

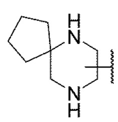

(for example,

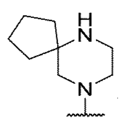

), or isomers and stereoisomers thereof. ("*" indicates that when a carbon atom with "*" is a chiral carbon atom, it is in an R configuration, an S configuration, or a mixture thereof)

In the present disclosure, the term "heterocycloalkenyl", as a group or a moiety of another group, refers to a cyclic alkenyl group linked by a heteroatom or a heteroatom group. In a certain embodiment, the "heterocycloalkenyl" is a stable 3- to 20-membered (preferably 3- to 12-membered, more preferably 4- to 10-membered, most preferably 3- to 7-membered) monocyclic or polycyclic (for example, bicyclic, tricyclic, or polycyclic bridged ring, fused ring (condensed ring), or spiro ring system) non-aromatic ring system containing an unsaturated double bond and consisting of 2 to 14 (preferably 2 to 6) carbon atoms and 1 to 6 heteroatoms or heteroatom groups selected from N, O, S, S(=O), and $S(=O)_2$. In some embodiments, "heterocycloalkenyl" is a group of a stable 4- to 10-membered heterocyclic system containing at least one unsaturated double bond and consisting of 3 to 9 carbon atoms and 1, 2, 3, or 4 heteroatoms or heteroatom groups selected from N, O, S, S(=O), and $S(=O)_2$. Unless otherwise specified in the specification, a heterocycloalkenyl group may be a monocyclic ("monocyclic heterocycloalkenyl"), bicyclic, tricyclic, or polycyclic ring system, which may include fused (fused-linked or condensed), bridged, or spiro ring systems (for example, a bicyclic system ("bicyclic heterocycloalkenyl")). The ring system of a heterocycloalkenyl bicyclic ring may include one or more than one heteroatom in one or both rings; and may contain an unsaturated double bond. The heterocycloalkenyl may be connected to the rest of the molecule via a carbon atom and through a single bond; in the case of a heterocycloalkenyl group including one or more than one nitrogen atom, the point of connection may be either a carbon atom or a nitrogen atom; or, may be connected to the rest of the molecule through a fused connection; as long as the chemical valency permits. In some embodiments, "heterocycloalkenyl" is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl. Examples of heterocycloalkeny include, but are not limited to, pyranyl, 2,3-dihydropyrrolyl, 2,3-dihydrofuryl, 1,2,3,4-tetrahydropyridyl, 1,2,3,6-tetrahydropyridyl, 3,4-dihydro-2H-pyran,

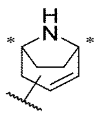 or 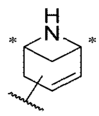 ,

for example,

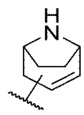

or

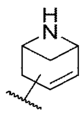 ,

for another example,

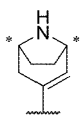 or 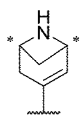 ,

for yet another example,

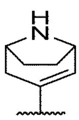 or 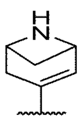 ,

"*" means that when a carbon atom with "*" is a chiral carbon atom, it is in an R configuration, an S configuration, or a mixture thereof.

In the present disclosure, the term "aryl", as a group or a moiety of another group, refers to a group of a conjugated hydrocarbon ring system with 6 to 18 carbon atoms (preferably with 6 to 10 carbon atoms) satisfying the

rule of 4n + 2. For the purposes of the present disclosure, an aryl group may be a monocyclic, bicyclic, tricyclic, or polycyclic ring system and may also be fused with a cycloalkyl, cycloalkenyl, heterocycloalkyl, or heterocycloalkeny ring as defined above, provided that the aryl group is connected to the rest of the molecule through a single bond via an atom on the aromatic ring. In a certain embodiment, the term "aryl" refers to an aromatic group consisting of carbon atoms, each ring being aromatic. Examples of aryl include, but are not limited to, phenyl, naphthyl (for example,

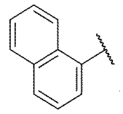

), anthracenyl, phenanthrenyl, or fluorenyl.

In the present disclosure, the term "heteroaryl", as a group or a moiety of another group, refers to a 5- to 16-membered conjugated cyclic group having 1 to 15 carbon atoms (preferably 1 to 10 carbon atoms) and 1 to 6 heteroatoms selected from nitrogen, oxygen, and sulfur in the ring. Unless otherwise specified in this specification, the heteroaryl group may be a monocyclic, bicyclic, tricyclic, or polycyclic ring system and may also be fused with a cycloalkyl, cycloalkenyl, heterocycloalkyl, or heterocycloalkeny ring as defined above, provided that the heteroaryl group is connected to the rest of the molecule through a single bond via an atom on the aromatic ring. For the purposes of the present disclosure, the heteroaryl group is preferably a stable 5- to 14-membered aromatic group containing 1 to 5 heteroatoms selected from nitrogen, oxygen, and sulfur, further preferably a stable 5- to 12-membered aromatic group containing 1 to 5 heteroatoms selected from nitrogen, oxygen, and sulfur, more preferably a stable 5- to 10-membered aromatic group containing 1 to 4 heteroatoms selected from nitrogen, oxygen, and sulfur, or a 5- to 6-membered aromatic group containing 1 to 3 heteroatoms selected from nitrogen, oxygen, and sulfur. In a certain embodiment, the term "heteroaryl" refers to an aromatic group containing a heteroatom, each ring being aromatic; preferably an aromatic 5- to 6-membered monocyclic or 9- to 10-membered bicyclic ring containing 1, 2, or 3 heteroatoms independently selected from nitrogen, oxygen, and sulfur. Examples of heteroaryl include, but are not limited to, thienyl, imidazolyl, pyrazolyl, thiazolyl, oxazolyl, diazolyl, oxadiazolyl, isoxazolyl, pyridyl (for example,

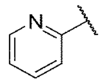

), pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoazolyl, thiadiazolyl, isoindolyl, indazolyl, isoindazolyl, purinyl, quinolyl, isoquinolinyl, diazanaphthyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzisothiazolyl, benzothienyl, oxatriazoly, cinnolinyl, quinazolinyl, indolizinyl, *o*-phenanthrolinyl, isoxazolyl, phenoxazinyl, phenothiazinyl, benzoxazolyl, or benzisoxazolyl.

It should be understood that singular forms such as "a" used in the present disclosure include plural referents unless otherwise specified.

The term "one or more than one" or "one or two or more" means 1, 2, 3, 4, 5, 6, 7, 8, 9, or more.

Unless otherwise specified, the present disclosure employs conventional methods of mass spectrometry and elemental analysis, and each step and condition can refer to the conventional operating steps and conditions in the art.

Unless otherwise indicated, the present disclosure employs standard nomenclature and standard laboratory steps and techniques of analytical chemistry, organic synthetic chemistry, and optics. In some cases, standard techniques are used for chemical synthesis, chemical analysis, and performance testing of light-emitting devices.

In addition, it should be noted that, unless otherwise clearly stated, the description "...independently" used in the present disclosure should be understood in a broad sense, meaning that each described individual is independent of each other and can be the same or different specific groups independently. In more detail, the description "...independently" can mean either that the specific options expressed by the same symbols in different groups do not affect each other; or that the specific options expressed by the same symbols in the same group do not affect each other.

Those skilled in the art can understand that, according to the convention used in the art, the

“ 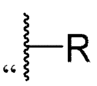 ” and " 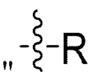 "

used in the structural formula of the group described in the present disclosure means that the corresponding group R is connected to other moieties and groups in the compound through this site.

Those skilled in the art can understand that, according to the convention used in the art, the " used in the structural formula of the group described in the present disclosure represents a single bond or a double bond.

Unless otherwise specified, all technical and scientific terms used herein have the standard meaning of the field to which the claimed subject matter belongs. Where more than one definition of a term exists, the definition herein shall prevail.

On the basis of not violating the common sense in the field, the preferred conditions above can be arbitrarily combined to obtain the preferred examples of the present disclosure.

The reagents and raw materials used in the present disclosure are commercially available.

The positive and progressive effect of the present disclosure is that the nitrogen-containing heterocyclic compound has the activity of inhibiting the proliferation of Ba/F3 KRAS-G12D cells, AGS cells, and Ba/F3 KRAS-G12V cells expressing KRAS G12D and/or KRAS G12V mutant proteins, and also shows good *in vivo* tumor inhibitory activity; and is expected to treat and/or prevent multiple diseases mediated by KRAS G12D and/or KRAS G12V.

DETAILED DESCRIPTION OF THE PREFERRED EMBODIMENT

The present disclosure is further described below by the way of examples, but the present disclosure is not thereby limited to the scope of the described examples. The reaction steps without specifying specific conditions in the following examples can be carried out in accordance with conventional methods and conditions in the art, or in accordance with the commodity instructions.

In the present disclosure, room temperature refers to the ambient temperature, which is 10°C to 35°C. Overnight refers to 8 to 15 hours. Reflux refers to the reflux temperature of solvent under atmospheric pressure.

The English abbreviations for the present disclosure are as follows:

PMB: *p*-methoxybenzyl; Boc: *tert*-butoxycarbonyl; TBS (TBDMS): *tert*butyldimethylsilyl; TIPS: triisopropylsilyl; MOM: methoxymethyl ($CH_3OCH_2$-); Cbz: benzyloxycarbonyl; Bn: benzyl; Fmoc: fluorenylmethoxycarbonyl; TBSCl: *tert*butyldimethylsilyl chloride; TIPSCl: triisopropylsilyl chloride; TMSCl: chlorotrimethylsilane; MEOH: methanol; DMSO: dimethyl sulfoxide; TFA: trifluoroacetic acid; ACN: acetonitrile; $CDCl_3$: deuterated chloroform; IPA: isopropanol; DEA: diethylamine.

**Intermediate 1: 7-(6-(bis(4-methoxybenzyl)amino)-4-methyl-3-(trifluoromethyl)pyridin-2-yl)-8-fluoropyrido [4,3-d] pyrimidine-2,4-diol**

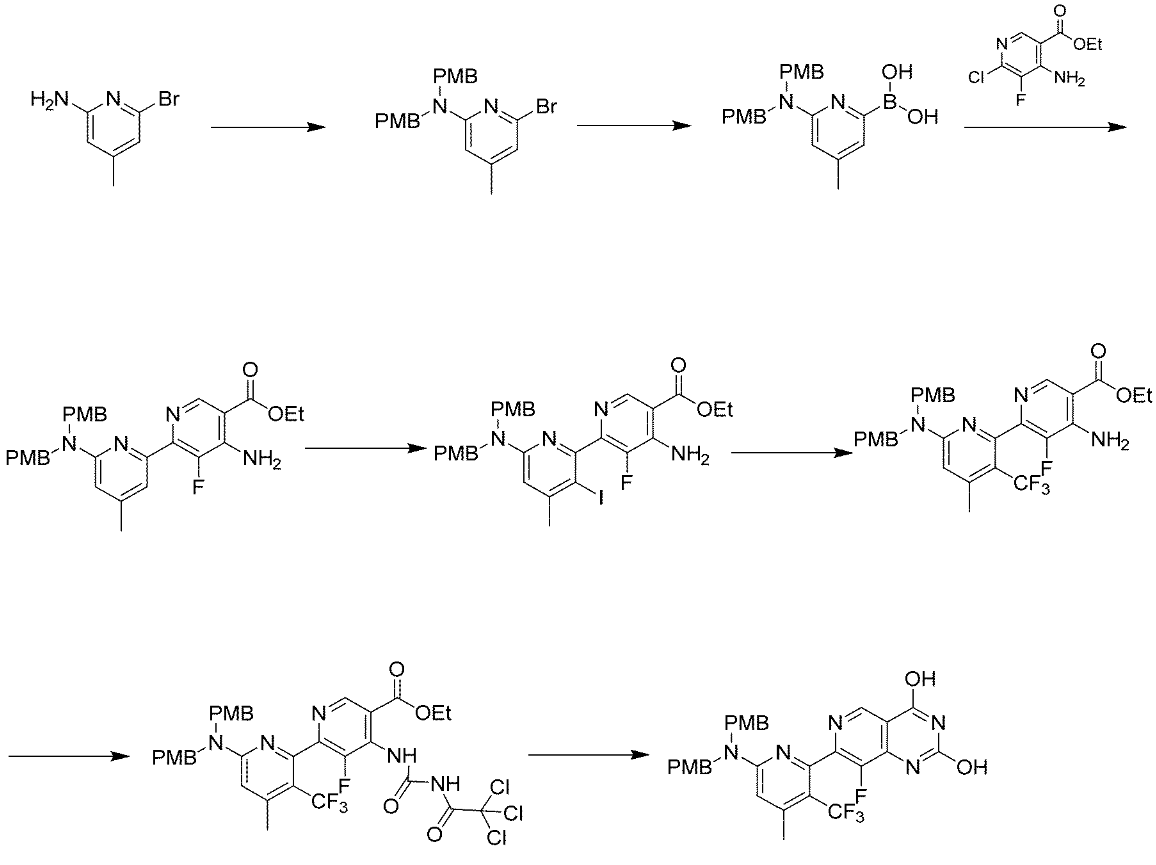

(Intermediate 1)

**Step 1: 6-bromo-N,N-bis(4-methoxybenzyl)-4-methylpyridin-2-amine**

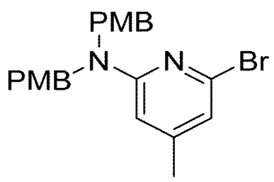

6-Bromo-4-methylpyridin-2-amine (20 g, 106.9 mmol, 1.0 eq) was completely dissolved in N,N-dimethylformamide (330 mL), and the mixture was cooled to 0°C under nitrogen atmosphere, and added with sodium hydride (12.8 g, 320.7 mmol, 2.0 eq). After the addition, the reaction mixture was naturally warmed to room temperature, stirred at room temperature for 1 hour, added with *p*-methoxybenzyl chloride (37.7 g, 240.5 mmol, 2.25 eq), and stirred at room temperature for 2 hours until the raw material was completely reacted. The reaction mixture was cooled in an ice water bath, added with ice water (650 mL) to precipitate a solid, and filtered under reduced pressure. The filter cake was washed with ice water (65 mL) and dried under vacuum under reduced pressure to obtain 6-bromo-N,N-bis(4-methoxybenzyl)-4-methylpyridin-2-amine as a yellow solid (49 g, yield: 100%), which was used directly for the next reaction step.
MS m/z: 427/429 $[M+H]^+$

**Step 2: (6-(bis(4-methoxybenzyl)amino)-4-methylpyridin-2-yl)boronic acid**

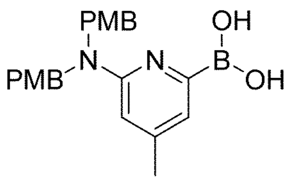

6-Bromo-N,N-bis(4-methoxybenzyl)-4-methylpyridin-2-amine (20 g, 46.84 mmol, 1.0 eq) was completely dissolved in 1,4-dioxane (100 mL), then [1,1'-bis(diphenylphosphino)ferrocene]dichloropalladium(II) (3.42 g, 4.68 mmol, 0.1 eq), potassium acetate (9.2 g, 93.68 mmol, 2.0 eq), and bis(pinacolato)diboron (23.8 g, 93.68 mmol, 2.0 eq) were added thereto, and the reaction mixture was stirred and reacted at 80°C for 3 hours under nitrogen atmosphere. The reaction mixture was cooled to room temperature, filtered, and the filtrate was concentrated under reduced pressure to obtain a crude product of (6-(bis(4-methoxybenzyl)amino)-4-methylpyridin-2-yl)boronic acid as a black oil (42 g), which was used directly in the next step.
MS m/z: 393 $[M+H]^+$

**Step 3: ethyl 4-amino-6'-(bis(4-methoxybenzyl)amino)-3-fluoro-4'-methyl-[2,2'-bipyridine]-5-carboxylate**

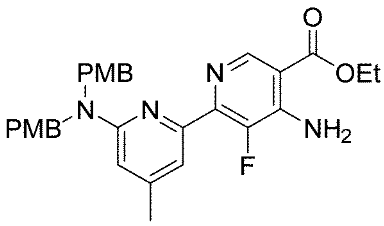

(6-(Bis(4-methoxybenzyl)amino)-4-methylpyridin-2-yl)boronic acid (122 g, 118.7 mmol, 1.0 eq) was completely dissolved in 1,4-dioxane (375 mL), then [1,1'-bis(diphenylphosphino)ferrocene]dichloropalladium(II) dichloromethane complex (6.68 g, 11.8 mmol, 0.1 eq), ethyl 4-amino-6-chloro-5-fluoronicotinate (20 g, 91.3 mmol, 0.77 eq), and potassium phosphate (58.06 g, 273.9 mmol, 2.3 eq) were added thereto, and then water (95 mL) was added thereto. The reaction mixture was stirred and reacted at 45°C for 2 hours under nitrogen atmosphere. The reaction mixture was cooled to room temperature, diluted with saturated brine (500 mL), and extracted with ethyl acetate (500 mL). The organic phase was dried over anhydrous sodium sulfate, concentrated under reduced pressure, and the residue was subjected to silica gel column chromatography (petroleum ether/ethyl acetate = 100:15, volume ratio) to obtain a crude product of ethyl 4-amino-6'-(bis(4-methoxybenzyl)amino)-3-fluoro-4'-methyl-[2,2'-bipyridine]-5-carboxylate as a yellow solid (44 g).
MS m/z: 531.2 $[M+H]^+$

**Step 4: ethyl 4-amino-6'-(bis(4-methoxybenzyl)amino)-3-fluoro-3'-iodo-4'-methyl-[2,2'-bipyridine]-5-carboxylate**

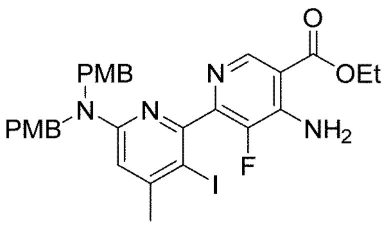

Ethyl 4-amino-6'-(bis(4-methoxybenzyl)amino)-3-fluoro-4'-methyl-[2,2'-bipyridine]-5-carboxylate (44 g, 83.0 mmol, 1.0 eq) and silver acetate (30.5 g, 182.6 mmol, 2.2 eq) were dissolved in anhydrous N,N-dimethylformamide, then iodine (63.2 g, 249.0 mmol, 3.0 eq) was added thereto under nitrogen atmosphere, and the reaction mixture was stirred and reacted at room temperature for 2 hours. The reaction mixture was diluted with saturated sodium sulfite aqueous solution, extracted with ethyl acetate, dried over anhydrous sodium sulfate, concentrated under reduced pressure, and the residue was subjected to silica gel column chromatography (petroleum ether/ethyl acetate = 5:1, volume ratio) to obtain ethyl 4-amino-6'-(bis(4-methoxybenzyl)amino)-3-fluoro-3'-iodo-4'-methyl-[2,2'-bipyridine]-5-carboxylate as a yellow solid (23.6 g, yield: 43.3%).
MS m/z: 657.1 $[M+H]^+$

**Step 5: ethyl 4-amino-6'-(bis(4-methoxybenzyl)amino)-3-fluoro-4'-methyl-3'-(trifluoromethyl)-[2,2'-bipyridine]-5-carboxylate**

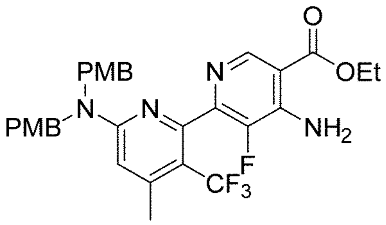

Ethyl 4-amino-6'-(bis(4-methoxybenzyl)amino)-3-fluoro-3'-iodo-4'-methyl-[2,2'-bipyridine]-5-carboxylate (23.6 g, 36.0 mmol, 1.0 eq) and cuprous iodide (13.7 g, 72.0 mmol, 2.0 eq) were dissolved in anhydrous N,N-dimethylformamide, then (1,10-phenanthroline)(trifluoromethyl)copper(I) (16.6 g, 53.0 mmol, 1.5 eq) was added thereto under nitrogen atmosphere, and the reaction mixture was stirred and reacted at room temperature for 2 hours under nitrogen atmosphere. The reaction mixture was diluted with saturated sodium chloride solution, extracted with ethyl acetate, dried over anhydrous sodium sulfate, concentrated under reduced pressure, and the residue was subjected to silica gel column chromatography (petroleum ether/ethyl acetate = 5:1, volume ratio) to obtain ethyl 4-amino-6'-(bis(4-methoxybenzyl)amino)-3-fluoro-4'-methyl-3'-(trifluoromethyl)-[2,2'-bipyridine]-5-carboxylate as a yellow solid (22 g, yield: 100%).
MS m/z: 599 $[M+H]^+$

**Step 6: ethyl 6'-(bis(4-methoxybenzyl)amino)-3-fluoro-4'-methyl-4-(3-(2,2,2-trichloroacetyl)ureido)-3'-(trifluoromethyl)-[2,2'-bipyridine]-5-carboxylate**

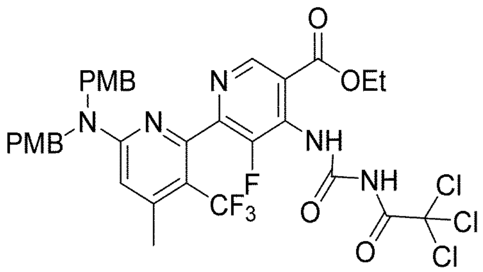

Ethyl 4-amino-6'-(bis(4-methoxybenzyl)amino)-3-fluoro-4'-methyl-3'-(trifluoromethyl)-[2,2'-bipyridine]-5-carboxylate (3.54 g, 5.92 mmol, 1.0 eq) was dissolved in tetrahydrofuran (59 mL), then the mixture was cooled to 15°C under nitrogen atmosphere, and trichloroacetyl isocyanate (1.45 g, 7.7 mmol, 1.3 eq) was added thereto. The reaction mixture was stirred and reacted at 15°C for 0.5 hours. The reaction mixture was subjected to rotary evaporation until dryness to remove the solvent to obtain a crude product. Methyl *tert*-butyl ether (20 mL) and petroleum ether (10 mL) were added thereto, and the resulting mixture was slurried for 0.5 hours, filtered, and subjected to rotary evaporation until dryness to obtain ethyl 6'-(bis(4-methoxybenzyl)amino)-3-fluoro-4'-methyl-4-(3-(2,2,2-trichloroacetyl)ureido)-3'-(trifluoromethyl)-[2,2'-bipyridine]-5-carboxylate as a white solid (4.7 g, yield: 100%), which was used directly in the next step.
MS m/z: 786/788 $[M+H]^+$

**Step 7: 7-(6-(bis(4-methoxybenzyl)amino)-4-methyl-3-(trifluoromethyl)pyridin-2-yl)-8-fluoropyrido [4,3-d] pyrimidine-2,4-diol**

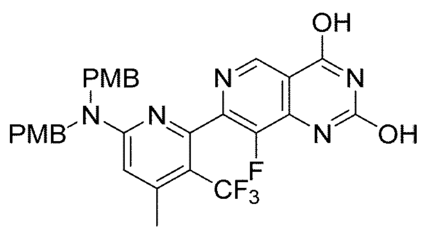

Ethyl 6'-(bis(4-methoxybenzyl)amino)-3-fluoro-4'-methyl-4-(3-(2,2,2-trichloroacetyl)ureido)-3'-(trifluoromethyl)-[2,2'-bipyridine]-5-carboxylate (4.7 g, 5.92 mmol, 1.0 eq) was placed in a 100 mL three-necked flask, cooled to 15°C under nitrogen atmosphere, and ammonia methanol solution (7 M, 37 mL) was added thereto, and the reaction mixture was reacted and stirred at 15°C for 0.5 hours. The reaction mixture was subjected to rotary evaporation until dryness to remove the solvent to obtain a crude product. Methyl *tert*-butyl ether (20 mL) and petroleum ether (10 mL) were added thereto, and the resulting mixture was slurried for 0.5 hours, filtered, and subjected to rotary evaporation until dryness to obtain 7-(6-(bis(4-methoxybenzyl)amino)-4-methyl-3-(trifluoromethyl)pyridin-2-yl)-8-fluoropyrido[4,3-d]pyrimidine-2,4-diol as a white solid (3.8 g, yield: 100%), which was used directly in the next step.

MS m/z: 596 $[M+H]^+$

$^1$H NMR (400 MHz, DMSO-$d_6$) δ 11.51 (br s, 2H), 8.81 (s, 1H), 7.17 (d, *J* = 8.0 Hz, 1H), 6.88 (d, *J* = 8.0 Hz, 1H), 6.80 (s, 1H), 4.68 (s, 4H), 3.72 (s, 6H), 2.37 (s, 3H).

**Intermediate 2: N,N-bis(4-methoxybenzyl)-4-methyl-6-(2,4,5-trichloro-8-fluoropyrido[4,3-dipyrimidin-7-yl)-5-(trifluoromethyl)pyridin-2-amine**

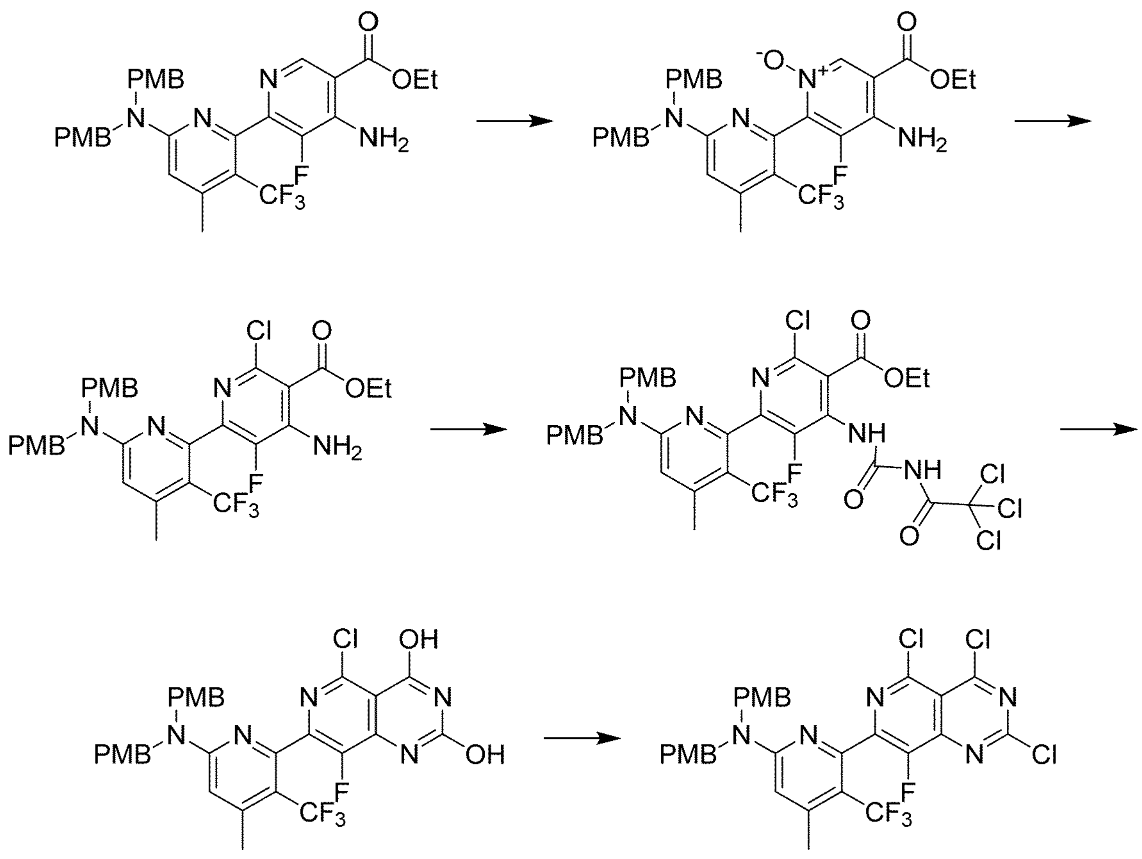

Intermediate 2

**Step 1: 4-amino-6'-(bis(4-methoxybenzyl)amino)-5-(ethoxycarbonyl)-3-fluoro-4'-methyl-3'-(trifluoromethyl)-[2,2'-bipyridine] 1-oxide**

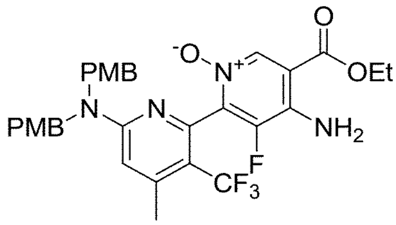

Ethyl 4-amino-6'-(bis(4-methoxybenzyl)amino)-3-fluoro-4'-methyl-3'-(trifluoromethyl)-[2,2'-bipyridine]-5-carboxylate (22 g, 36.8 mmol, 1.0 eq) was dissolved in 1,2-dichloroethane (400 mL), and *m*-chloroperoxybenzoic acid (25.4 g, 147.2 mmol, 4.0 eq) was added thereto. The reaction mixture was stirred and reacted at room temperature for 1 hour. The reaction mixture was diluted with water, extracted with dichloromethane, dried over anhydrous sodium sulfate, concentrated under reduced pressure, and the residue was subjected to silica gel column chromatography (petroleum ether/ethyl acetate = 4:1, volume ratio) to obtain 4-amino-6'-(bis(4-methoxybenzyl)amino)-5-(ethoxycarbonyl)-3-fluoro-4'-methyl-3'-(trifluoromethyl)-[2,2'-bipyridine] 1-oxide as a yellow solid (13.6 g, yield: 61.5%).
MS m/z: 615.2 $[M+H]^+$

**Step 2: ethyl 4-amino-6'-(bis(4-methoxybenzyl)amino)-6-chloro-3-fluoro-4'-methyl-3'-(trifluoromethyl)-[2,2'-bipyridine]-5-carboxylate**

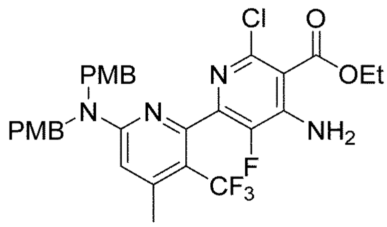

4-Amino-6'-(bis(4-methoxybenzyl)amino)-5-(ethoxycarbonyl)-3-fluoro-4'-methyl-3'-(trifluoromethyl)-[2,2'-bipyridine] 1-oxide (13.6 g, 22.1 mmol, 1.0 eq) was dissolved in phosphorus oxychloride (20 mL), and the reaction mixture was stirred at 110°C for 1 hour. The reaction mixture was subjected to rotary evaporation until dryness to remove the solvent, and the residue was subjected to silica gel column chromatography (petroleum ether/ethyl acetate = 1:1, volume ratio) to obtain ethyl 4-amino-6'-(bis(4-methoxybenzyl)amino)-6-chloro-3-fluoro-4'-methyl-3'-(trifluoromethyl)-[2,2'-bipyridine]-5-carboxylate as a yellow solid (5.7 g, yield: 40.7%).

MS m/z: 633.3/635.3 $[M+H]^+$
$^1H$ NMR (400 MHz, DMSO-$d_6$) $\delta$ 7.19-7.16 (m, 4H), 7.02 (s, 1H), 6.89-6.85 (m, 4H), 6.76 (s, 1H), 4.68 (s, 4H), 4.39-4.33 (m, 2H), 3.72 (s, 6H), 2.34 (s, 3H), 1.34-1.29 (m, 3H).

**Step 3: ethyl 6'-(bis(4-methoxybenzyl)amino)-6-chloro-3-fluoro-4'-methyl-4-(3-(2,2,2-trichloroacetyl)ureido)-3'-(trifluoromethyl)-[2,2'-bipyridine]-5-carboxylate**

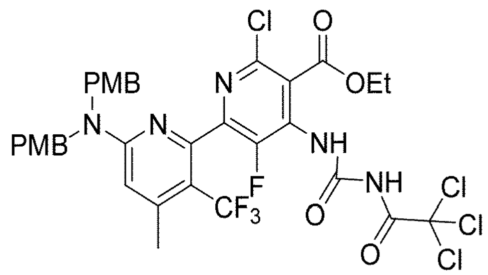

Ethyl 4-amino-6'-(bis(4-methoxybenzyl)amino)-6-chloro-3-fluoro-4'-methyl-3'-(trifluoromethyl)-[2,2'-bipyridine]-5-carboxylate (5.7 g, 9.28 mmol, 1.0 eq) was dissolved in tetrahydrofuran (93 mL), then the mixture was cooled to 15°C under nitrogen atmosphere, and trichloroacetyl isocyanate (2.27 g, 12.07 mmol, 1.3 eq) was added thereto. The reaction mixture was stirred and reacted at 15°C for 0.5 hours. The reaction mixture was subjected to rotary evaporation until dryness to remove the solvent to obtain a crude product. Methyl *tert*-butyl ether (20 mL) and petroleum ether (10 mL) were added thereto, and the resulting mixture was slurried for 0.5 hours, filtered, and subjected to rotary evaporation until dryness to obtain ethyl 6'-(bis(4-methoxybenzyl)amino)-6-chloro-3-fluoro-4'-methyl-4-(3-(2,2,2-trichloroacetyl)ureido)-3'-(trifluoromethyl)-[2,2'-bipyridine]-5-carboxylate as a white solid (7.1 g, yield: 93.3%), which was used directly in the next step.
MS m/z: 820.11822.1 $[M+H]^+$

**Step 4: 7-(6-(bis(4-methoxybenzyl)amino)-4-methyl-3-(trifluoromethyl)pyridin-2-yl)-5-chloro-8-fluoropyrido [4,3-d] pyrimidine-2,4-diol**

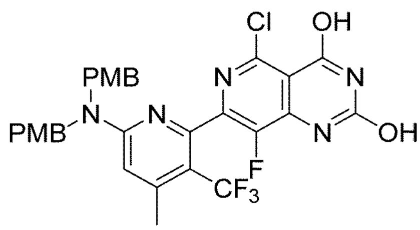

Ethyl 6'-(bis(4-methoxybenzyl)amino)-6-chloro-3-fluoro-4'-methyl-4-(3-(2,2,2-trichloroacetyl)ureido)-3'-(trifluoromethyl)-[2,2'-bipyridine]-5-carboxylate (7.1 g, 8.66 mmol, 1.0 eq) was placed in a 100 mL three-necked flask, cooled to 15°C under nitrogen atmosphere, and ammonia methanol solution (7 M, 56 mL) was added thereto, and the reaction mixture was reacted and stirred at 15°C for 0.5 hours. The reaction mixture was subjected to rotary evaporation until dryness to remove the solvent to obtain a crude product. Methyl *tert*-butyl ether (20 mL) and petroleum ether (10 mL) were added thereto, and the resulting mixture was slurried for 0.5 hours, filtered, and subjected to rotary evaporation until dryness to obtain 7-(6-(bis(4-methoxybenzyl)amino)-4-methyl-3-(trifluoromethyl)pyridin-2-yl)-5-chloro-8-fluoropyrido[4,3-d]pyrimidine-2,4-diol as a white solid (6.1 g, yield: 100%), which was used directly in the next step.
MS m/z: 630.3/632.3 $[M+H]^+$

**Step 5: N,N-bis(4-methoxybenzyl)-4-methyl-6-(2,4,5-trichloro-8-fluoropyrido[4,3-d]pyrimidin-7-yl)-5-(trifluoromethyl)pyridin-2-amine**

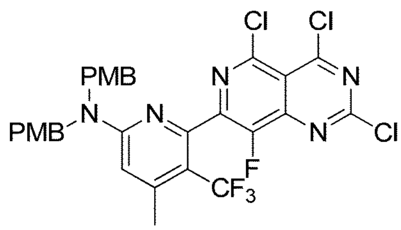

7-(6-(Bis(4-methoxybenzyl)amino)-4-methyl-3-(trifluoromethyl)pyridin-2-yl)-5-chloro-8-fluoropyrido[4,3-d]pyrimidine-2,4-diol (2.5 g, 3.97 mmol, 1 eq) was dissolved in phosphorus oxychloride (30 mL), and N,N-diisopropylethylamine (2.56 g, 19.84 mmol, 5 eq) was added thereto. The reaction mixture was reacted at 110°C for 3 hours. The reaction mixture was concentrated, and the residue was purified by column chromatography (tetrahydrofuran/petroleum ether: 0 to 20%) to obtain N,N-bis(4-methoxybenzyl)-4-methyl-6-(2,4,5-trichloro-8-fluoropyrido[4,3-d]pyrimidin-7-yl)-5-(trifluoromethyl)pyridin-2-amine (750 mg, yield: 28.34%) as a yellow solid.
MS m/z: 666.11668.1 $[M+H]^+$

**Intermediate 3: 5,7-dichloro-8-fluoropyrido[4,3-d]pyrimidine-2,4-diol**

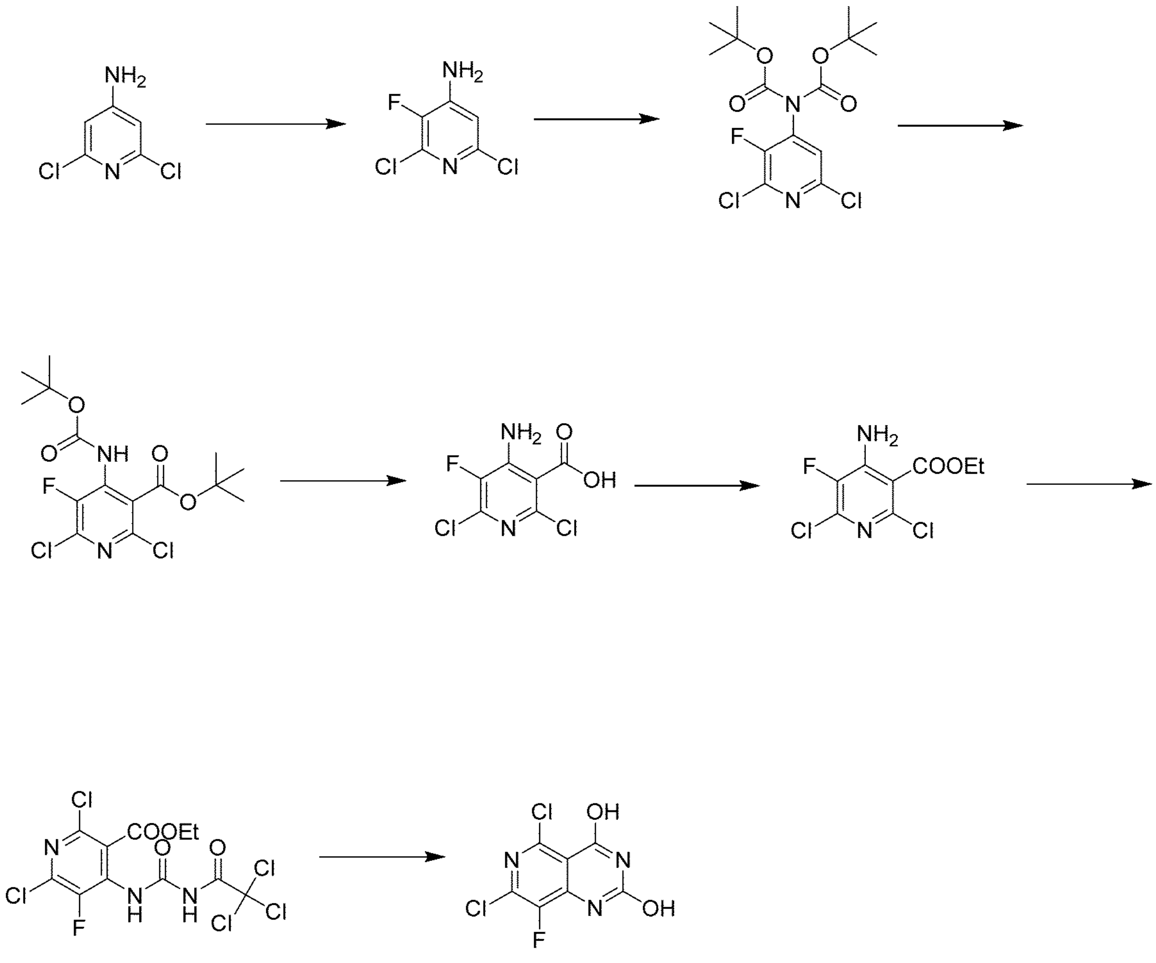

Intermediate 3

**Step 1: 2,6-dichloro-3-fluoropyridin-4-amine**

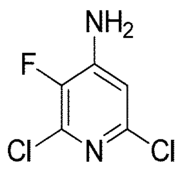

2,6-Dichloropyridin-4-amine (6.52 g, 39.99 mmol, 1 *eq*) was dissolved in N,N-dimethylformamide (30 mL) and

acetonitrile (30 mL), then 1-chloromethyl-4-fluoro-1,4-diazoniabicyclo[2.2.2]octane bis(tetrafluoroborate) (17 g, 47.99 mmol, 1.2 *eq*) was added thereto, and the reaction mixture was reacted at 80°C for half an hour. LCMS monitored that the reaction was completed. The reaction mixture was diluted with water and extracted with ethyl acetate. The organic phase was washed with saturated brine, dried over anhydrous sodium sulfate, filtered, and the filtrate was concentrated. The residue was purified by column chromatography (ethyl acetate/petroleum ether: 0 to 10%) to obtain 2,6-dichloro-3-fluoropyridin-4-amine (4.2 g, yield: 58.03%) as a white solid.
MS m/z: 181.11183.1 $[M+H]^+$

**Step 2: *tert*-butyl N-[(*tert*-butoxy)carbonyl]-N-(2,6-dichloro-3-fluoropyridin-4-yl)carbamate**

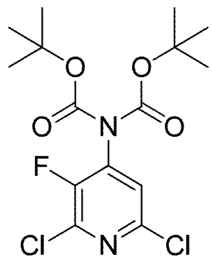

2,6-Dichloro-3-fluoropyridin-4-amine (4.2 g, 23.21 mmol, 1 *eq*) was dissolved in dichloromethane (40 mL), then BOC anhydride (10.29 g, 47.15 mmol, 2.03 *eq*) and 4-dimethylaminopyridine (457.28 mg, 3.74 mmol, 0.16 *eq)* were added thereto, and the reaction mixture was reacted at room temperature for 16 hours. LCMS monitored that the reaction was completed. The reaction mixture was concentrated, and the residue was purified by column chromatography (ethyl acetate/petroleum ether: 0 to 20%) to obtain *tert*-butyl N-[(*tert*-butoxy)carbonyl]-N-(2,6-dichloro-3-fluoropyridin-4-yl)carbamate (7.0 g, yield: 79.13%) as a white solid.

MS m/z: 381.11383.1 $[M+H]^+$
$^1H$ NMR (400 MHz, DMSO-$d_6$) $\delta$ 8.06-8.01 (m, 1H), 1.42 (s, 18H).

**Step 3: *tert*-butyl 4-((*tert*-butoxycarbonyl)amino)-2,6-dichloro-5-fluoronicotinate**

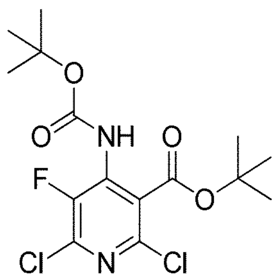

*tert-Butyl* N-[(*tert*-butoxy)carbonyl]-N-(2,6-dichloro-3-fluoropyridin-4-yl)carbamate (5.84 g, 15.32 mmol, 1 eq) was dissolved in tetrahydrofuran (30 mL), then lithium diisopropylamide (2 M, 15.32 mL, 2 *eq)* was added dropwise thereto at -78°C, and the reaction mixture was warmed to room temperature for 2 hours. The reaction mixture was quenched with water and extracted with ethyl acetate. The organic phase was washed with saturated brine, dried over anhydrous sodium sulfate, filtered, and the filtrate was concentrated. The residue was purified by column chromatography (ethyl acetate/petroleum ether: 0 to 20%) to obtain *tert*-butyl 4-((*tert*-butoxycarbonyl)amino)-2,6-dichloro-5-fluoronicotinate (5.0 g, yield: 85.62%) as a brown solid.

MS m/z: 381.11383.1 $[M+H]^+$
$^1H$ NMR (400 MHz, DMSO-$d_6$) $\delta$ 10.06 (s, 1H), 1.54 (s, 9H), 1.45 (s, 9H).

**Step 4: 2,6-dichloro-3-fluoropyridin-4-amine**

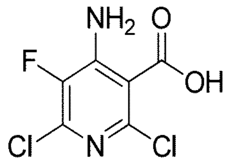

*tert*-Butyl 4-((*tert*-butoxycarbonyl)amino)-2,6-dichloro-5-fluoronicotinate (13 g, 34.10 mmol, 1 eq) was dissolved in dichloromethane (100 mL), then trifluoroacetic acid (30 mL) was added thereto, and the reaction mixture was reacted at room temperature for 16 hours. LCMS monitored that the reaction was completed. The reaction mixture was concentrated to obtain 2,6-dichloro-3-fluoropyridin-4-amine (7.67 g, crude product) as a brown solid, which was used directly in the next step.
MS m/z: 225.1 $[M+H]^+$

**Step 5: ethyl-4-amino-2,6-dichloro-5-fluoronicotinate**

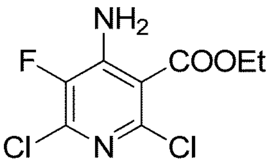

2,6-Dichloro-3-fluoropyridin-4-amine (7.67 g, 34.09 mmol, 1 eq) was dissolved in ethanol (100 mL), then concentrated sulfuric acid (102.35 g, 1.02 mol, 30 *eq)* was added thereto, and the reaction mixture was reacted at 80°C for 40 hours. The reaction mixture was cooled to room temperature, and added with saturated sodium bicarbonate aqueous solution to adjust the pH of the reaction mixture to 7. The reaction mixture was extracted with ethyl acetate. The organic phase was washed with saturated brine, dried over anhydrous sodium sulfate, filtered, and the filtrate was concentrated. The residue was purified by column chromatography (ethyl acetate/petroleum ether: 20 to 40%) to obtain ethyl-4-amino-2,6-dichloro-5-fluoronicotinate (8.0 g, yield: 92.76%) as a brown solid.
MS m/z: 253.1/255.1 $[M+H]^+$

**Step 6: ethyl-2,6-dichloro-5-fluoro-4-(3-(2,2,2-trichloroacetyl)ureido)nicotinate**

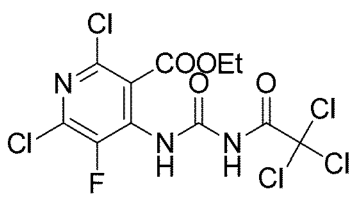

Ethyl 4-amino-2,6-dichloro-5-fluoronicotinate (1.13 g, 4.47 mmol, 1 *eq)* was dissolved in tetrahydrofuran (10 mL), then trichloroacetyl isocyanate (993.57 mg, 5.27 mmol, 1.18 *eq)* was added thereto, and the reaction mixture was reacted at room temperature for 1 hour. LCMS monitored that the reaction was completed. The reaction mixture was concentrated, and the residue was purified by column chromatography (ethyl acetate/petroleum ether: 20 to 50%) to obtain ethyl 2,6-dichloro-5-fluoro-4-(3-(2,2,2-trichloroacetyl)ureido)nicotinate (1.5 g, yield: 76.14%) as a white solid.
MS m/z: 441.9/443.9 $[M+H]^+$

**Step 7: 5,7-dichloro-8-fluoropyrido[4,3-d]pyrimidine-2,4-diol**

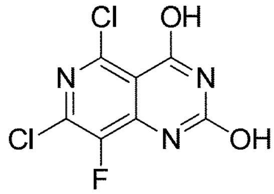

Ethyl 2,6-dichloro-5-fluoro-4-(3-(2,2,2-trichloroacetyl)ureido)nicotinate (3.0 g, 6.80 mmol, 1 *eq*) was dissolved in methanol (40 mL), then ammonia methanol solution (7 M, 2.77 mL, 2.85 *eq*) was added thereto, and the reaction mixture was reacted at room temperature for 2 hours. LCMS monitored that the reaction was completed. The reaction mixture was concentrated, and the residue was slurried with methyl *tert*-butyl ether to obtain 5,7-dichloro-8-fluoropyrido[4,3-d]pyrimidine-2,4-diol (1.8 g, yield: 100%) as a white solid.
MS m/z: 250.1/252.1 $[M+H]^+$

**Example 1: compound 1**

**(3S,5S)-5-Amino-1-(7-(6-amino-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate)**

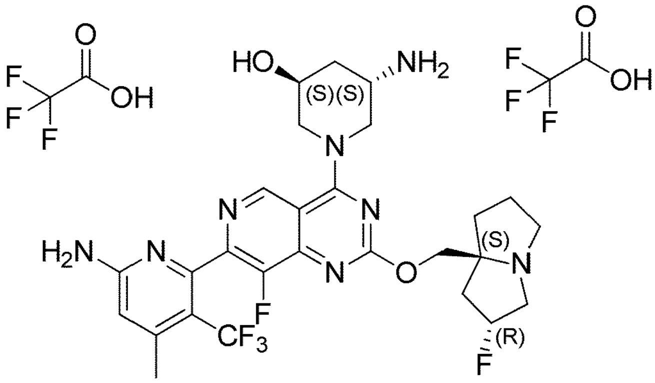

**Step 1: 6-(2,4-dichloro-8-fluoropyrido[4,3-d]pyrimidin-7-yl)-N,N-bis(4-methoxybenzyl)-4-methyl-5-(trifluoromethyl)pyridin-2-amine**

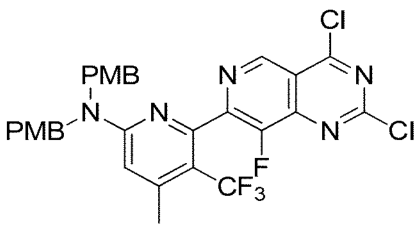

7-(6-(Bis(4-methoxybenzyl)amino)-4-methyl-3-(trifluoromethyl)pyridin-2-yl)-8-fluoropyrido[4,3-d]pyrimidine-2,4(1H,3H)-dione (370 mg, 0.621 mmol, 1 *eq*) was dissolved in phosphorus oxychloride (4 mL), then N,N-diisopropylethylamine (401.47 mg, 3.11 mmol, 5 *eq*) was added thereto, and the reaction mixture was reacted at 100°C for 3 hours. LCMS monitored that the reaction was completed. The reaction mixture was concentrated, and the residue was purified

by column chromatography (tetrahydrofuran/petroleum ether: 0 to 50%) to obtain 6-(2,4-dichloro-8-fluoropyrido[4,3-d]pyrimidin-7-yl)-N,N-bis(4-methoxybenzyl)-4-methyl-5-(trifluoromethyl)pyridin-2-amine (220 mg, yield: 55.99%) as a yellow solid.
MS m/z: 632.2/634.1 $[M+H]^+$

**Step 2: *tert*-butyl ((3S,SS)-1-(7-(6-(bis(4-methoxybenzyl)amino)-4-methyl-3-(trifluoromethyl)pyridin-2-yl)-2-chloro-8-fluoropyrido[4,3-dipyrimidin-4-yl)-5-hydroxypiperidin-3-yl)carbamate**

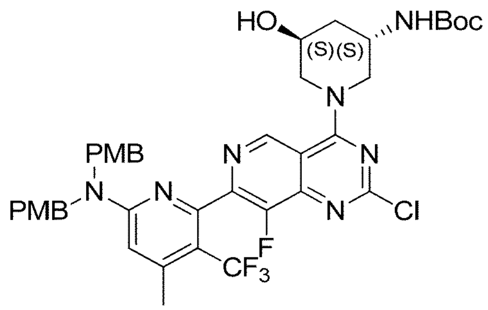

7-(6-(Bis(4-methoxybenzyl)amino)-4-methyl-3-(trifluoromethyl)pyridin-2-yl)-8-fluoropyrido[4,3-d]pyrimidine-2,4(1H,3H)-dione (200 mg, 0.316 mmol, 1 *eq*) was dissolved in dichloromethane (4 mL), then N,N-diisopropylethylamine (401.47 mg, 3.11 mmol, 5 *eq*) and tert-butyl ((3S,5S)-5-hydroxypiperidin-3-yl)carbamate (64.98 mg, 0.300 mmol, 0.95 eq) were added thereto at -30°C, and the reaction mixture was reacted at -30°C for 1 hour. LCMS monitored that the reaction was completed. The reaction mixture was extracted with dichloromethane, then the organic phase was washed with saturated brine, dried over anhydrous sodium sulfate, filtered and concentrated, and the residue was purified by column chromatography [tetrahydrofuran/petroleum ether: 0 to 50%] to obtain 7-(6-(bis(4-methoxybenzyl)amino)-4-methyl-3-(trifluoromethyl)pyridin-2-yl)-8-fluoropyrido[4,3-d]pyrimidine-2,4(1H,3H)-dione (120 mg, yield: 46.72%) as a yellow solid.
MS m/z: 812.3 $[M+H]^+$

**Step 3: *tert*-butyl ((3S,SS)-1-(7-(6-(bis(4-methoxybenzyl)amino)-4-methyl-3-(trifluoromethyl)pyridin-2-yl)-2-chloro-8-fluoropyrido[4,3-d]pyrimidin-4-yl)-5-((tertbutyldimethylsilyl)oxo)piperidin-3-yl)carbamate**

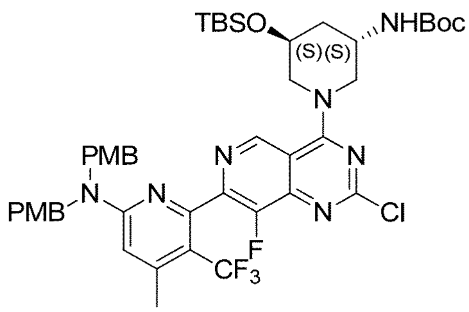

7-(6-(Bis(4-methoxybenzyl)amino)-4-methyl-3-(trifluoromethyl)pyridin-2-yl)-8-fluoropyrido[4,3-d]pyrimidine-2,4(1H,3H)-dione (160 mg, 0.197 mmol, 1 *eq*) was dissolved in tetrahydrofuran (3 mL), then imidazole (33.53 mg, 0.493 mmol, 2.5 *eq*) and *tert*-butyl dimethylchlorosilane (44.53 mg, 0.295 mmol, 1.5 *eq*) were added thereto, and the reaction mixture was reacted at 50°C overnight. LCMS monitored that the reaction was completed. The reaction mixture was extracted with ethyl acetate, then the organic phase was washed with saturated brine, dried over anhydrous sodium sulfate, filtered and concentrated, and the residue was purified by a TLC plate (tetrahydrofuran/petroleum ether: 1:1) to obtain *tert*-butyl ((3S,5S)-1-(7-(6-(bis(4-methoxybenzyl)amino)-4-methyl-3-(trifluoromethyl)pyridin-2-yl)-2-chloro-8-fluoropyrido[4,3-d]pyrimidin-4-yl)-5-((*tert*-butyldimethylsilyl)oxo)piperidin-3-yl)carbamate (100 mg, yield: 46.72%) as a pale yellow solid.
MS m/z: 926.4 $[M+H]^+$

**Step 4: *tert*-butyl ((3S,SS)-1-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)-5-((*tert*-butyldimethylsilyl)oxo)piperidin-3-yl)carbamate**

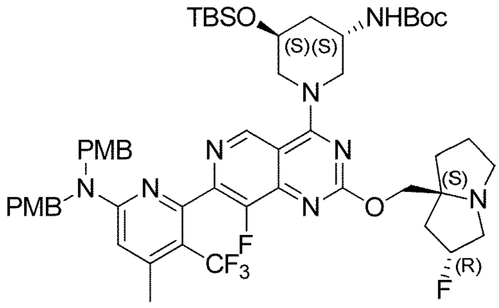

((2R,7aS)-2-Fluorohexahydro-1H-pyrrolizin-7a-yl)methanol (20.62 mg, 0.130 mmol, 1.5 *eq)* was dissolved in dichloromethane (2 mL), then sodium *tert*-butoxide (20.75 mg, 0.216 mmol, 2.5 *eq)* was added thereto, and *tert*-butyl-((3S,SS)-1-(7-(6-(bis(4-methoxybenzyl)amino)-4-methyl-3-(trifluoromethyl)pyridin-2-yl)-2-chloro-8-fluoropyrido[4,3-d]pyrimidin-4-yl)-5-((*tert*-butyldimethylsilyl)oxo)piperidin-3-yl)carbamate (80 mg, 0.0864 mmol, 1 *eq)* was added thereto under nitrogen atmosphere. The reaction mixture was reacted at room temperature for 1 hour. LCMS monitored that the reaction was completed. The reaction mixture was quenched with saturated ammonium chloride solution and extracted with ethyl acetate. The organic phase was washed with saturated brine, dried over anhydrous sodium sulfate, filtered, and the filtrate was concentrated to obtain *tert*-butyl ((3S,5S)-1-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)-5-((*tert*-butyldimethylsilyl)oxo)piperidin-3-yl)carbamate (70 mg, crude product) as a colorless oil.
MS m/z: 1049.3 $[M+H]^+$

**Step 5: (3S,SS)-5-amino-1-(7-(6-amino-4-methyl-3-(trifluoromethyl)pyridin-2-yl)-8-fluoro-2-(((2R,7aS)-2-fluorohexahydro-1H-pyrrolizin-7a-yl)methoxy)pyrido [4,3-d]pyrimidin-4-yl)piperidin-3-ol bis(2,2,2-trifluoroacetate)**

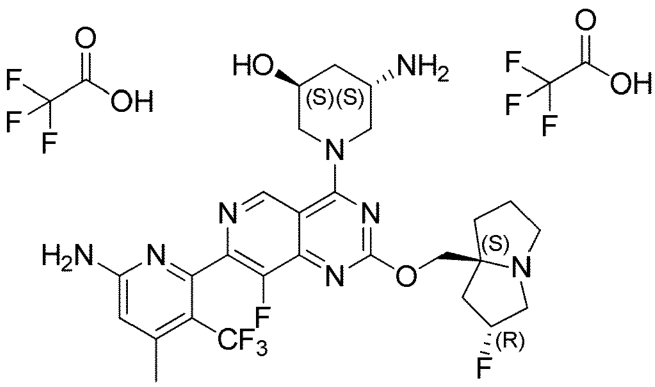

*tert*-Butyl ((3S,5S)-1-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)-5-((*tert*-butyldimethylsilyl)oxo)piperidin-3-yl)carbamate (70 mg, 0.0667 mmol, 1 *eq*) was dissolved in trifluoroacetic acid (1 mL), and reacted at 50°C for 1 hour. The reaction mixture was concentrated. The residue was dissolved in N,N-dimethylformamide (2 mL), and tetrabutylammonium fluoride solution (1 M, 0.080 mL, 1.2 *eq*) was added thereto. The reaction mixture was reacted at room temperature for 1 hour. The residue was purified by preparative HPLC to obtain (3S,5S)-5-amino-1-(7-(6-amino-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate) (9.3 mg, yield: 22.45%) as a white solid.
MS m/z: 595.3 $[M+H]^+$

HPLC separation conditions:

column: YMC-Actus Triart C18 150 * 30 mm * 5 μm; mobile phase: [water (0.1%TFA) - ACN]; B%: 20% to 40%, 10.5 min).

$^{19}$F NMR (376 MHz, DMSO-$d_6$) δ -52.66 (s, 3F), -73.79 (s, 6F), -143.07 (s, 1F), - 172.95 (s, 1F).

$^{1}$H NMR (400 MHz, DMSO-$d_6$) δ 10.90 (br s, 1H), 9.24 (br s, 1H), 8.20 (br s, 3H), 6.80 (br s, 2H), 6.52 (s, 1H), 5.59 (br d, *J* = 52.8 Hz, 1H), 5.33 (br s, 1H), 4.71-4.46 (m, 3H), 4.24-4.06 (m, 2H), 3.95-3.88 (m, 1H), 3.85 (m, 3H), 3.33 (m, 1H), 2.40-2.33 (m, 4H), 2.31-2.00 (m, 6H), 1.88 (br t, *J* = 11.0 Hz, 1H).

**Example 2: compound 2**

**6-(4-((S)-3-Aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine tetrakis(2,2,2-trifluoroacetate)**

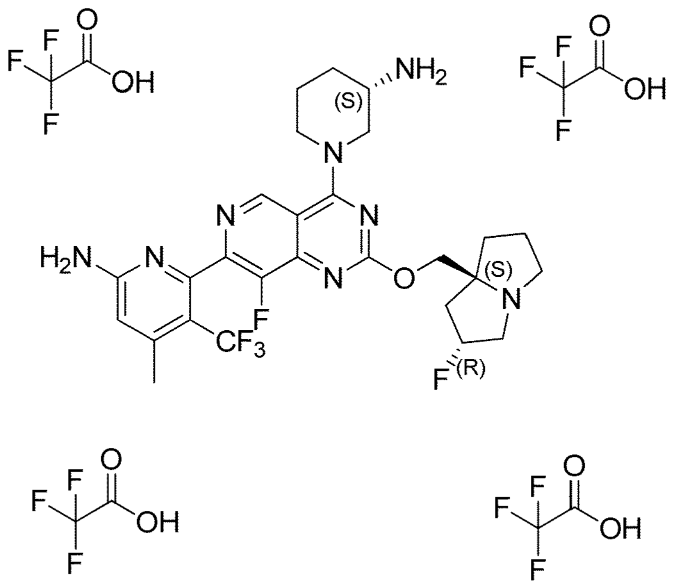

**Step 1: *tert*-butyl (S)-(1-(2,7-dichloro-8-fluoropyrido[4,3-d]pyrimidin-4-yl)piperidin-3-yl)carbamate**

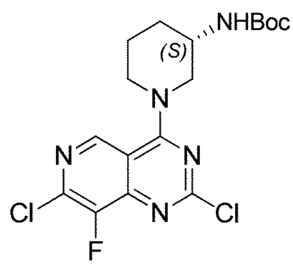

2,4,7-Trichloro-8-fluoropyrido[4,3-d]pyrimidine (900 mg, 3.56 mmol, 1 eq) (prepared with reference to the method of intermediates 4 and 7 in WO2021041671A1) was dissolved in dichloromethane (10 mL), then N,N-diisopropylethylamine (1.38 g, 10.69 mmol, 3 eq) was added thereto, and the system was replaced with nitrogen and cooled to -30°C. A solution of (S)-3-(BOC-amino)piperidine (713.97 mg, 3.56 mmol, 1 eq) in dichloromethane (10 mL) was added dropwise thereto. The reaction mixture was reacted at -30°C for 0.5 hours, and LCMS monitored that the reaction was completed. The reaction mixture was quenched with water, extracted with dichloromethane, and the organic phase was dried over anhydrous sodium sulfate, filtered, and the filtrate was concentrated to obtain *tert*-butyl (S)-(1-(2,7-dichloro-8-fluoropyrido[4,3-d]pyrimidin-4-yl)piperidin-3-yl)carbamate (1.81 g, crude product) as a yellow solid.
MS m/z: 416.1 [M+H]+

**Step 2: *tert*-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

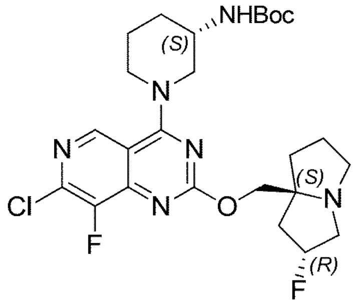

*tert*-Butyl (S)-(1-(2,7-dichloro-8-fluoropyrido[4,3-d]pyrimidin-4-yl)piperidin-3-yl)carbamate (690 mg, 1.66 mmol, 1 eq) and ((2R,7aS)-2-fluorohexahydro-1H-pyrrolizin-7a-yl)methanol (263.88 mg, 1.66 mmol, 1 eq) (prepared with reference to the method of intermediate 16 in WO2021041671A1) were dissolved in tetrahydrofuran (7 mL), and the mixture was cooled to 0°C, added with sodium *tert*-butoxide (318.58 mg, 3.32 mmol, 2 eq), and reacted at 0°C for 1 hour. LCMS monitored that the reaction was completed, and the reaction mixture was quenched by adding water and extracted with ethyl acetate. The organic phase was washed with saturated brine, dried over anhydrous sodium sulfate, filtered, and the filtrate was concentrated to obtain tert-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (850 mg, crude product) as a yellow solid, which was used directly in the next step.
MS m/z: 539.2 $[M+H]^+$

**Step 3: *tert*-butyl ((S)-1-(7-(6-(bis(4-methoxybenzyl)amino)-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

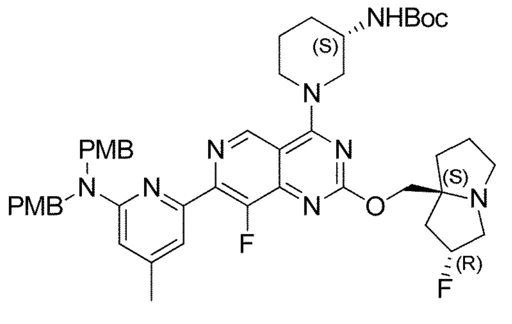

*tert*-Butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (300 mg, 0.557 mmol, 1 eq), cesium carbonate (725.36 mg, 2.23 mmol, 4 eq), and 6-(bis(4-methoxybenzyl)amino)-4-methylpyridine-2-boronic acid (436.63 mg, 1.11 mmol, 2 eq) were dissolved in a mixture of dioxane (4 mL) and water (1 mL), and 1,1'-bis(di-*tert*-butylphosphino)ferrocene palladium dichloride (72.55 mg, 0.1113 mmol, 0.2 eq) was added thereto. The system was replaced with nitrogen and reacted at 80°C for 50 minutes. LCMS monitored that the reaction was completed. The reaction mixture was concentrated, then the residue was dissolved in ethyl acetate, washed with water, washed with saturated brine, dried over anhydrous sodium sulfate, filtered, and concentrated. The residue was purified by column chromatography (methanol/dichloromethane: 0 to 5%) to obtain *tert*-butyl ((S)-1-(7-(6-(bis(4-methoxybenzyl)amino)-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (700 mg, yield: 49.26%) as a white solid.
MS m/z: 851.4 $[M+H]^+$

**Step 4: *tert*-butyl ((S)-1-(7-(6-(bis(4-methoxybenzyl)amino)-3-iodo-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

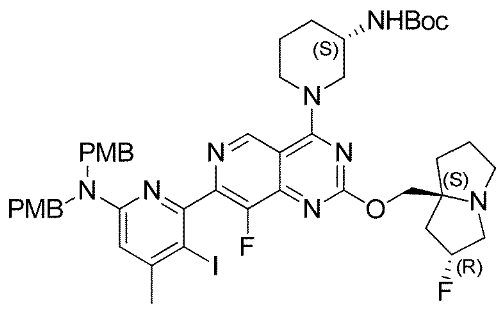

*tert*-Butyl ((S)-1-(7-(6-(bis(4-methoxybenzyl)amino)-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (700 mg, 0.8226 mmol, 1 eq) was dissolved in N,N-dimethylformamide (10 mL), then silver acetate (343.24 mg, 2.06 mmol, 2.5 eq) and iodine (626.32 mg, 2.47 mmol, 3 eq) were added thereto, and the reaction mixture was reacted at room temperature for 1 hour. LCMS monitored that the reaction was completed. The reaction mixture was quenched with saturated sodium sulfite aqueous solution, filtered, and the filtrate was extracted with ethyl acetate. The organic phase was washed with saturated brine, dried over anhydrous sodium sulfate, filtered, and the filtrate was concentrated to obtain *tert*-butyl ((S)-1-(7-(6-(bis(4-methoxybenzyl)amino)-3-iodo-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (570 mg, crude product) as a dark green oil, which was used directly in the next step.
MS m/z: 977.3 $[M+H]^+$

**Step 5: *tert*-butyl ((S)-1-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

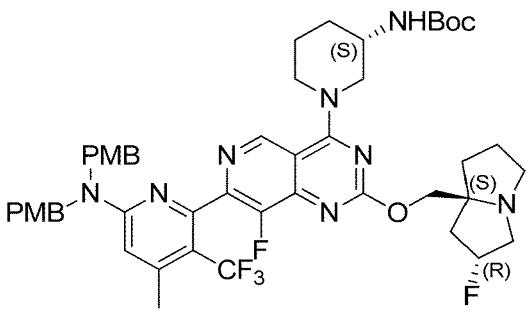

*tert*-Butyl ((S)-1-(7-(6-(bis(4-methoxybenzyl)amino)-3-iodo-4-methylpyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (570 mg, 0.5835 mmol, 1 eq) and cuprous iodide (555.62 mg, 2.92 mmol, 5 eq) were dissolved in N,N-dimethylformamide (10 mL), and trifluoromethyl(1,10-phenanthroline)copper (912.44 mg, 2.92 mmol, 5 eq) was added thereto. After the system was replaced with nitrogen, the reaction mixture was reacted at 50°C for 1 hour. LCMS monitored that the reaction was completed. The reaction mixture was quenched by adding water at room temperature, and extracted with ethyl acetate. The organic phase was washed with saturated brine, dried over anhydrous sodium sulfate, filtered, and the filtrate was concentrated to obtain *tert*-butyl ((S)-1-(7-(6-(bis(4-methoxybenzyl)amino)-4-methyl-3-(triffuoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (200 mg, crude product) as a green oil, which was used directly in the next step.
MS m/z: 919.4 $[M+H]^+$

**Step 6: 6-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine tetrakis(2,2,2-trifluoroacetate)**

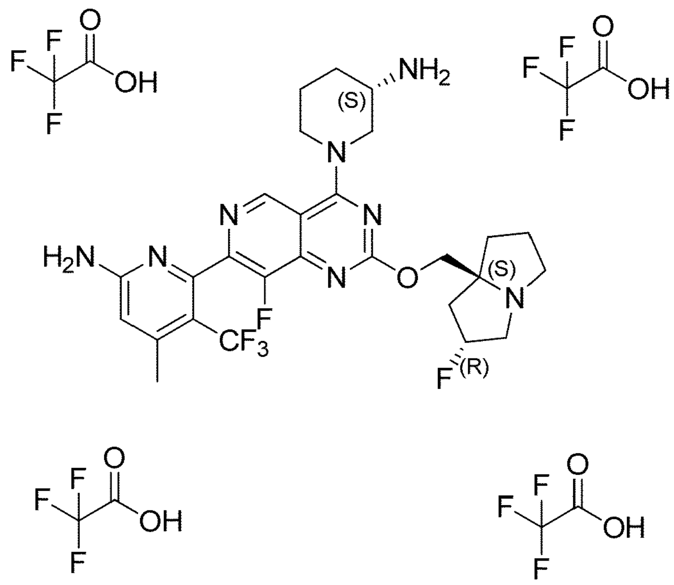

*tert*-Butyl ((S)-1-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (250 mg, 0.272 mmol, 1 eq) was dissolved in trifluoroacetic acid (5 mL), and reacted at 50°C for 1 hour. LCMS monitored that the reaction was completed. The reaction mixture was concentrated, and the residue was purified by HPLC to obtain 6-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine tetrakis(2,2,2-trifluoroacetate) (84.24 mg, yield: 44.71%) as a white solid.

HPLC separation conditions:

column: Boston Green ODS 150 * 30 mm * 5 $\mu$m;
mobile phase: [Water (0.1%TFA) - ACN]; B%: 0% to 35%, 9 min).
MS m/z: 579.2 $[M+H]^+$

$^{19}$F NMR (376 MHz, DMSO-$d_6$) $\delta$ -52.71 (s, 3F), -74.27 (s, 12F), -142.96 (s, 1F), - 172.97 (s, 1F).

$^1$H NMR (400 MHz, DMSO-$d_6$) $\delta$ 11.13 (br s, 1H), 9.15 (s, 1H), 8.27 (br s, 3H), 7.35-6.65 (br s, 1H), 6.58 (s, 1H), 5.64 (br d, *J* = 52.4 Hz, 1H), 4.73-4.61 (m, 2H), 4.47 (m, 1H), 4.30 (m, 2H), 3.88-3.75 (m, 4H), 3.68 (m, 1H), 3.57 (m, 2H), 3.43-3.30 (m, 1H), 2.43 (m, 4H), 2.32-2.09 (m, 4H), 2.02 (m, 1H), 1.80 (m, 2H).

**Example 3: compound 3**

**4-(4-((S)-3-Aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol**

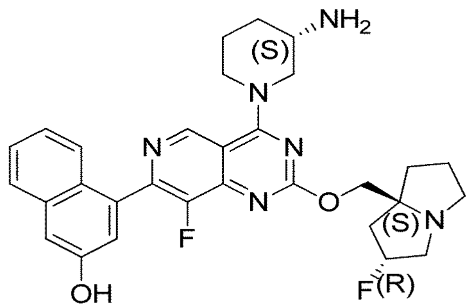

**Step 1: *tert*-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

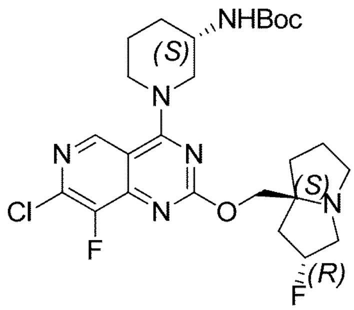

*tert*-Butyl (S)-(1-(2,7-dichloro-8-fluoropyrido[4,3-d]pyrimidin-4-yl)piperidin-3-yl)carbamate (500 mg, 1.20 mmol, 1 *eq*) was dissolved in tetrahydrofuran (5 mL), then ((2R,7aS)-2-fluorohexahydro-1H-pyrrolizin-7a-yl)methanol (191.22 mg, 1.20 mmol, 1 *eq*) was added thereto, and sodium *tert*-butoxide (230.86 mg, 2.40 mmol, 2 *eq*) was added thereto at 0°C. The reaction mixture was reacted at 0°C for 0.5 hours. LCMS monitored that the reaction was completed. The reaction mixture was diluted with water and extracted with ethyl acetate. The organic phase was washed with saturated brine, dried over anhydrous sodium sulfate, filtered, and the filtrate was concentrated to obtain *tert*-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (630 mg, crude product) as a yellow solid, and the crude product was used directly in the next reaction step. MS m/z: 539.1 $[M+H]^+$

**Step 2: *tert*-butyl ((S)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-yl)carbamate**

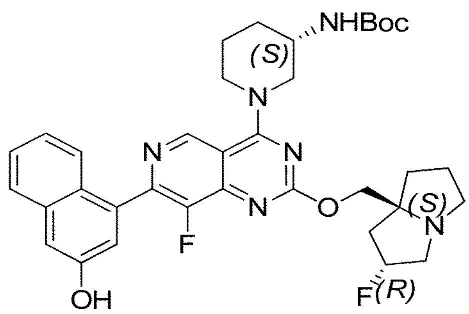

*tert*-Butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (570 mg, 1.06 mmol, 1 *eq*) was dissolved in dioxane (5 mL) and water (2 mL), then (3-hydroxynaphthalen-1-yl)boronic acid (258.43 mg, 1.37 mmol, 1.3 *eq*), cesium carbonate (1.03 g, 3.17 mmol, 3 *eq*), and tetrakis(triphenylphosphine)palladium (122.20 mg, 0.106 mmol, 0.1 *eq*) were added thereto. The reaction mixture was reacted at 100°C for 3 hours under nitrogen atmosphere. LCMS monitored that the reaction was completed.

The reaction mixture was diluted with water (20 mL) and extracted with ethyl acetate. The organic phase was washed with saturated brine, dried over anhydrous sodium sulfate, filtered, and the filtrate was concentrated. The residue was purified by column chromatography (tetrahydrofuran/petroleum ether: 60 to 100%) to obtain *tert*-butyl ((S)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-yl)carbamate (350 mg, yield: 51.18%) as a brown solid.
MS m/z: 647.3 $[M+H]^+$

**Step 3: 4-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol**

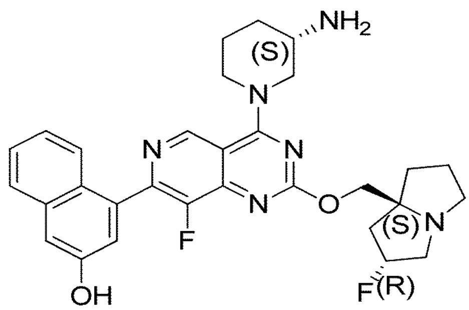

*tert*-Butyl ((S)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-yl)carbamate (300 mg, 0.464 mmol, 1 *eq*) was dissolved in dichloromethane (3 mL), then trifluoroacetic acid (1 mL) was added thereto, and the reaction mixture was reacted at room temperature for 3 hours. LCMS monitored that the reaction was completed. The reaction mixture was concentrated, and the residue was purified by HPLC to obtain 4-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol (45 mg, yield: 17.75%) as a white solid.

HPLC separation conditions:

column: YMC Triart C18 250 * 50 mm * 7 μm;
mobile phase: [Water (ammonia hydroxide v/v) - ACN]; B%: 39% to 79%, 9 min.
MS m/z: 547.3 $[M+H]^+$

$^{19}F$ NMR (376 MHz, DMSO-$d_6$) δ -139.77 (s, 1F), -172.11 (s, 1F).

$^1H$ NMR (400 MHz, DMSO-$d_6$) δ 9.19 (s, 1H), 7.81 (d, *J* = 8.3 Hz, 1H), 7.66-7.53 (m, 2H), 7.50-7.37 (m, 1H), 7.32-7.19 (m, 3H), 5.29 (br d, *J* = 54.0 Hz, 1H), 4.44-4.22 (m, 2H), 4.15 (d, *J* = 10.4 Hz, 1H), 4.05 (d, *J* = 10.4 Hz, 1H), 3.24-2.99 (m, 6H), 2.92 (br s, 1H), 2.89-2.75 (m, 2H), 2.19-1.91 (m, 5H), 1.91-1.74 (m, 4H), 1.75-1.67 m, 1H), 1.54-1.27 (m, 1H).

**Example 4: compound 4**

**(3S,SS)-5-Amino-1-(8-fluoro-2-(((2R,7aS)-2-fluorohexahydro-1H-pyrrolizin-7a-yl)methoxy)-7-(3-hydroxynaphthalen-1-yl)pyrido [4,3-d]pyrimidin-4-yl)piperidin-3-ol**

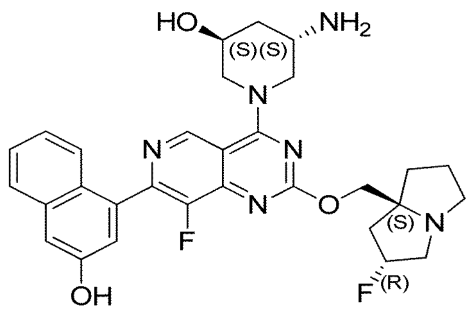

**Step 1: *tert*-butyl ((3S,5S)-1-(2,7-dichloro-8-fluoropyrido[4,3-d]pyrimidin-4-yl)-5-hydroxypiperidin-3-yl)carbamate**

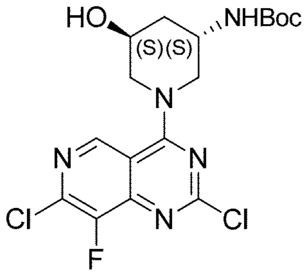

2,4,7-Trichloro-8-fluoropyrido[4,3-d]pyrimidine (648.50 mg, 2.57 mmol, 1 eq) was dissolved in dichloromethane (12 mL), then N,N-diisopropylethylamine (995.95mg, 7.71 mmol, 3 eq) was added thereto, and the system was replaced with nitrogen and cooled to -30°C. A solution of *tert*-butyl ((3S,SS)-5-hydroxypiperidin-3-yl)carbamate (0.5 g, 2.31 mmol, 0.9 eq) in dichloromethane (6 mL) was added dropwise thereto. The reaction mixture was reacted at -30°C for 0.5 hours, and LCMS monitored that the reaction was completed. The reaction mixture was quenched with water (30 mL), extracted with dichloromethane, and the organic phase was dried over anhydrous sodium sulfate, filtered, and the filtrate was concentrated to obtain *tert*-butyl ((3S,5S)-1-(2,7-dichloro-8-fluoropyrido[4,3-d]pyrimidin-4-yl)-5-hydroxypiperidin-3-yl)carbamate (1.1 g, crude product) as a yellow solid.

MS m/z: 432.0 $[M+H]^+$
$^1$H NMR (400 MHz, $CDCl_3$) $\delta$ 9.20 (br s, 1H), 4.74-4.52 (m, 2H), 4.45 (br d, *J* = 12.8 Hz, 1H), 4.34 (m, 1H), 4.14 (m, 1H), 3.62 (br d, *J* = 12.5 Hz, 1H), 3.50 (m, 1H), 2.21 (m, 1H), 1.88 (m, 1H), 1.44 (s, 9H).

**Step 2: *tert*-butyl ((3S,5S)-1-(7-chloro-8-fluoro-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate**

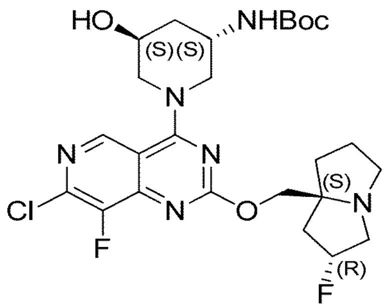

*tert*-Butyl ((3S,5S)-1-(2,7-dichloro-8-fluoropyrido[4,3-d]pyrimidin-4-yl)-5-hydroxypiperidin-3-yl)carbamate (1.1 g, 2.54 mmol, 1 *eq*) and ((2R,7aS)-2-fluorohexahydro-1H-pyrrolizin-7a-yl)methanol (810.23 mg, 5.09 mmol, 2 *eq*) were dissolved in dioxane (22 mL), and cesium carbonate (2.49 g, 7.63 mmol, 3 *eq*) was added thereto. The reaction mixture was reacted at 80°C overnight. LCMS monitored that the reaction was completed. The reaction mixture was added with water (20 mL) and extracted with ethyl acetate. The organic phase was washed with saturated brine, dried over anhydrous sodium sulfate, filtered, and the filtrate was concentrated to obtain *tert*-butyl ((3S,5S)-1-(7-chloro-8-fluoro-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate (1.4 g, crude product) as a yellow solid.

MS m/z: 555.3/557.3 $[M+H]^+$
$^1$H NMR (400 MHz, $CDCl_3$) $\delta$ 7.20 (s, 1H), 5.32-5.13 (m, 1H), 4.61 (br s, 1H), 4.37-4.26 (m, 2H), 4.22-4.01 (m, 4H), 3.59 (br d, *J* = 12.8 Hz, 1H), 3.43 (br dd, *J* = 8.8, 12.7 Hz, 1H), 3.32-3.09 (m, 4H), 2.96-2.88 (m, 1H), 2.25-2.05 (m, 5H), 1.92-1.85 (m, 3H), 1.31 (s, 9H).

**Step 3: *tert-butyl* ((3S,5S)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)-5-hydroxypiperidin-3-yl)carbamate ES16193-302**

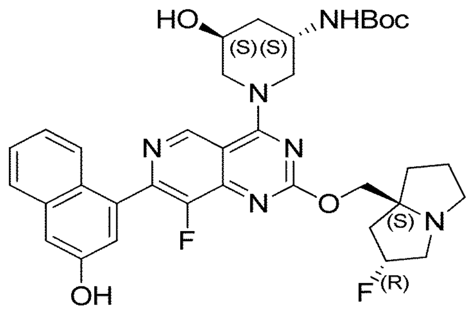

(((2R,7aS)-2-Fluorohexahydro-1H-pyrrolizin-7a-yl)methoxy)pyrido[4,3-d]pyrimidin-4-yl)-5-hydroxypiperidin-3-yl)carbamate (0.15 g, 0.270 mmol, 1 *eq*) and 3-hydroxynaphthalene-1-boronic acid (66.05 mg, 0.351 mmol, 1.3 *eq*) were dissolved in a mixture of water (0.5 mL) and dioxane (1.5 mL), then tetrakis(triphenylphosphine)palladium (31.23 mg, 0.027 mmol, 0.1 *eq*) and cesium carbonate (264.17 mg, 0.81 mmol, 3 *eq*) were added thereto. After the system was replaced with nitrogen, the reaction mixture was reacted at 100°C for 3 hours. LCMS monitored that the reaction was completed. The reaction mixture was added with ethyl acetate and water to separate the phases. The organic phase was washed with saturated brine, dried over anhydrous sodium sulfate, filtered, and the filtrate was concentrated. The residue was purified by column chromatography (methanol/dichloromethane: 0 to 7%) to obtain *tert*-butyl ((3S,SS)-1-(8-ffuoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)-5-hydroxypiperidin-3-yl)carbamate (0.1 g, yield: 55.86%) as a yellow solid.
MS m/z: 663.5 $[M+H]^+$

**Step 4: (3S,5S)-5-amino-1-(8-fluoro-2-(((2R,7aS)-2-fluorohexahydro-1H-pyrrolizin-7a-yl)methoxy)-7-(3-hydroxynaphthalen-1-yl)pyrido[4,3-dipyrimidin-4-yl)piperidin-3-ol**

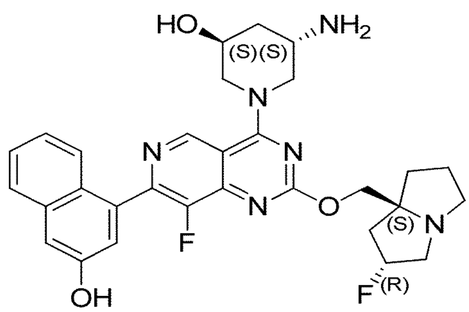

*tert*-Butyl ((3S,SS)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)-5-hydroxypiperidin-3-yl)carbamate (0.05 g, 0.0754 mmol, 1 *eq*) was dissolved in dichloromethane (2.5 mL), then trifluoroacetic acid (770.0 mg, 6.75 mmol, 0.5 mL, 89.51 *eq*) was added thereto, and the reaction mixture was reacted at room temperature for 1 hour. LCMS monitored that the reaction was completed. The reaction mixture was concentrated, and the residue was purified by HPLC to obtain (3S,5S)-5-amino-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-ol (25 mg, yield: 48.30%) as a white solid.

HPLC separation conditions:

column: Phenomenex C18 80 * 40 mm * 3 μm;
mobile phase: [Water (ammonia hydroxide v/v) - ACN]; B%: 29% to 69%, 9 min
MS m/z: 563.4 $[M+H]^+$

$^{19}F$ NMR (376 MHz, DMSO-$d_6$) δ -139.81 (s, 1F), -172.14 (s, 1F).

$^1H$ NMR (400 MHz, DMSO-$d_6$) δ 9.42 (s, 1H), 7.85-7.78 (m, 1H), 7.57-7.42 (m, 2H), 7.33-7.28 (m, 1H), 7.28-7.21 (m, 2H), 5.55 (br s, 1H), 5.30 (br d, *J* = 53.6 Hz, 1H), 4.26-4.12 (m, 2H), 3.15-2.99 (m, 6H), 2.95 (br d, *J* = 13.0 Hz, 1H), 2.84 (br d, *J* = 10.9 Hz, 2H), 2.31-2.20 (m, 2H), 2.17 (m, 1H), 2.11-1.99 (m, 2H), 1.92 -1.75 (m, 4H), 1.69-1.57 (m, 1H).

**Example 5: compound 5**

**4-(4-((R)-3-Aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol**

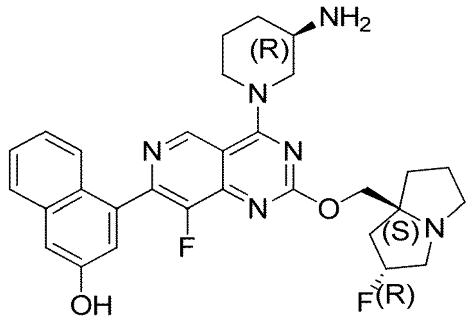

**Step 1: *tert-butyl* (R)-(1-(2,7-dichloro-8-fluoropyrido[4,3-d]pyrimidin-4-yl)piperidin-3-yl)carbamate**

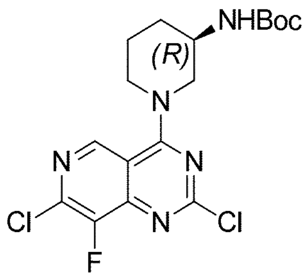

2,4,7-Trichloro-8-fluoropyrido[4,3-d]pyrimidine (1.0 g, 3.96 mmol, 1 *eq*) was dissolved in dichloromethane (10 mL), then N,N-diisopropylethylamine (1.54 mg, 11.88 mmol, *3 eq*) was added thereto, and R-3-(*tert*-butylammonium carbamate)piperazine (793.3 mg, 3.96 mmol, 1 *eq*) was added thereto at -30°C. The reaction mixture was reacted for 30 minutes. LCMS detected that the reaction was completed, and the reaction was quenched by adding water and extracted with dichloromethane. The organic phase was washed with saturated brine, dried over anhydrous sodium sulfate, filtered, and the filtrate was concentrated to obtain *tert*-butyl (R)-(1-(2,7-dichloro-8-fluoropyrido[4,3-d]pyrimidin-4-yl)piperidin-3-yl)carbamate (1.0 g, crude product) as a white solid.
MS m/z: 416.0/418.0 $[M+H]^+$

**Step 2: *tert*-butyl ((R)-1-(7-chloro-8-fluoro-2-(((2R,7aS)-2-fluorohexahydro-1H-pyrrolizin-7a-yl)methoxy)pyrido[4,3-dipyrimidin-4-yl)piperidin-3-yl)carbamate**

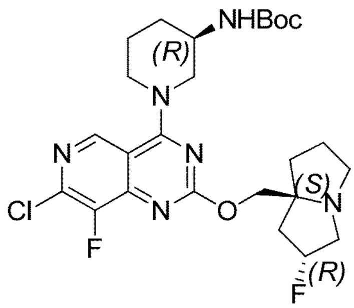

*tert*-Butyl (R)-(1-(2,7-dichloro-8-fluoropyrido[4,3-d]pyrimidin-4-yl)piperidin-3-yl)carbamate (500 mg, 1.58 mmol, 1 *eq*) was dissolved in tetrahydrofuran (6 mL), then ((2R,7aS)-2-fluorohexahydro-1H-pyrrolizin-7a-yl)methanol (254.29 mg, 1.60 mmol, 1.01 *eq*) was added thereto, and sodium *tert*-butoxide (303.96 mg, 3.16 mmol, 2 *eq*) was added thereto at 0°C under nitrogen atmosphere. The reaction mixture was reacted at 0°C for 1 hour. LCMS detected that the reaction was completed, and the reaction was quenched by adding water and extracted with ethyl acetate. The organic phase was washed with saturated brine, dried over anhydrous sodium sulfate, filtered, and the filtrate was concentrated to obtain *tert*-butyl ((R)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (500.0 mg, crude product) as a yellow solid.
MS m/z: 539.11541.1 [M+H]$^+$

**Step 3: *tert*-butyl ((R)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-yl)carbamate**

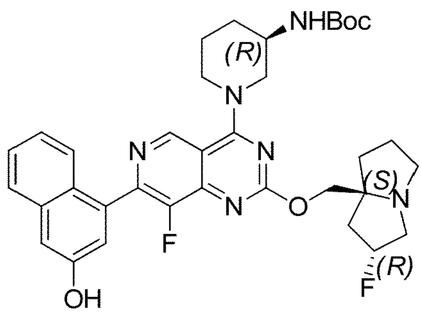

*tert*-Butyl ((R)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (430.8 mg, 0.80 mmol, 1 *eq*) was dissolved in dioxane (6 mL) and pure water (2 mL), then (3-hydroxynaphthalen-1-yl)boronic acid (195.3 mg, 1.04 mmol, 1.3 *eq*), cesium carbonate (780 mg, 2.40 mmol, 3 *eq*), and tetrakis(triphenylphosphine)palladium (92.35 mg, 0.80 mmol, 0.1 *eq*) were sequentially added thereto. The reaction mixture was reacted at 100°C for 3 hours under nitrogen atmosphere. LCMS detected that the reaction was completed. The reaction mixture was filtered, then the filtrate was concentrated, extracted with water and dichloromethane, and the organic phase was concentrated and purified by column chromatography (tetrahydrofuran/petroleum ether: 0 to 60%) to obtain *tert*-butyl ((R)-1-(8-fluoro-2-(((2R,7aS)-2-fluorohexahydro- 1H-pyrrolizin-7a-yl)methoxy)-7-(3-hydroxynaphthalen-1 -yl)pyrido[4,3-d]pyrimidin-4-yl)piperidin-3-yl)carbamate (330 mg, yield: 63.8%) as a yellow solid.
MS m/z: 647.3 [M+H]$^+$

**Step 4: 4-(4-((R)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol**

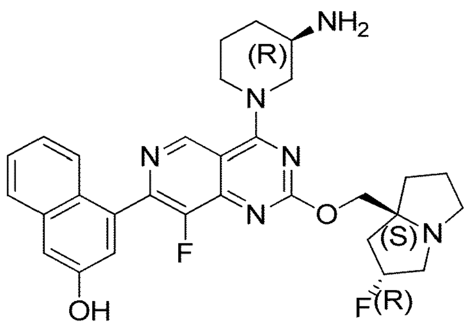

*tert*-Butyl ((R)-1-(8-fluoro-2-(((2R,7aS)-2-fluorohexahydro-1H-pyrrolizin-7a-yl)methoxy)-7-(3-hydroxynaphthalen-1-yl)pyrido[4,3-d]pyrimidin-4-yl)piperidin-3-yl)carbamate (320 mg, 0.495 mmol, 1 *eq*) was dissolved in dichloromethane (5 mL), then trifluoroacetic acid (2 mL) was added thereto, and the reaction mixture was reacted at room temperature for 2 hours. LCMS detected that the reaction was completed. The reaction mixture was concentrated and separated by HPLC to obtain 4-(4-((R)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)naphthalen-2-ol (35.0 mg, yield: 12.94%) as a yellow solid.

HPLC preparation conditions:

column: YMC Triart C18 250 * 50 mm * 7 $\mu$m;
mobile phase: [water (0.05% ammonia hydroxide v/v) - ACN]; B%: 40% to 80%, 9 min.
MS m/z: 547.3 $[M+H]^+$

$^{19}$F NMR (376 MHz, DMSO-$d_6$) $\delta$ -139.66 (br s, 1F), -172.12 (br s, 1F).

$^1$H NMR (400 MHz, DMSO-$d_6$) $\delta$ 9.19 (br s, 1H), 8.28 (br s, 1H), 7.81 (br d, *J* = 8.3 Hz, 1H), 7.54 (br d, *J* = 8.0 Hz, 1H), 7.45 (br t, *J* = 7.4 Hz, 1H), 7.31-7.13 (m, 3H), 5.38-5.29 (br d, *J* = 54.0 Hz, 1H), 4.52-4.26 (m, 2H), 4.19-3.99 (m, 4H), 3.15-3.04 (m, 3H), 2.88-2.80 (m, 1H), 2.26-1.51 (m, 10H).

**Example 6: compound 6-P1 and compound 6-P2**

**4-(4-((R)-5-Amino-3,3-difluoropiperidin-1-yl)-8-fluoro-2-(((2R,7aS)-2-fluorotetrahydro-1H-pyrrolizin-7a(SH)-yl)methoxy)pyrido [4,3-d]pyrimidin-7-yl)-5-ethynyl-6-fluoronaphthalen-2-ol and 4-(4-((S)-5-amino-3,3-difluoropiperidin-1-yl)-8-fluoro-2-(((2R,7aS)-2-fluorotetrahydro-1H-pyrrolizin-7a(5H)-yl)methoxy)pyrido[4,3-d]pyrimidin-7-yl)-5-ethynyl-6-fluoronaphthalen-2-ol**

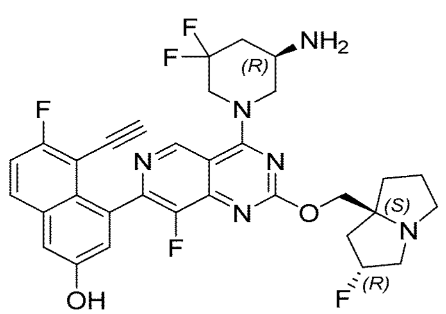

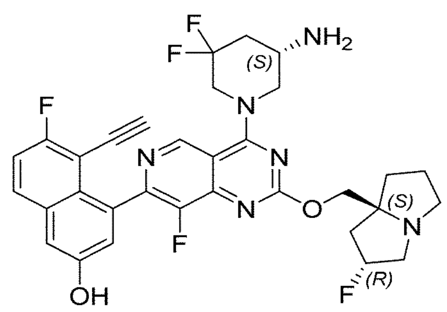

**Step 1: *tert*-butyl (1-(2,7-dichloro-8-fluoropyrido[4,3-dipyrimidin-4-yl)-5,5-difluoropiperidin-3-yl)carbamate**

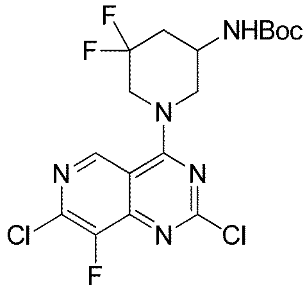

2,4,7-Trichloro-8-fluoropyrido[4,3-d]pyrimidine (500 mg, 1.98 mmol, 1 *eq*) was dissolved in dichloromethane (2 mL), then N,N-diisopropylethylamine (767.88 mg, 5.94 mmol, 1.03 mL, 3 *eq*) was added thereto, and a solution of *tert*-butyl (S)-(5,5-difluoropiperidin-3-yl)carbamate (467.79 mg, 1.98 mmol, 1 *eq*) in dichloromethane (2 mL) was added dropwise thereto at -30°C. The reaction mixture was reacted at -30°C for 0.5 hours. LCMS monitored that the reaction was completed. The reaction mixture was diluted with water and extracted with ethyl acetate. The organic phase was washed with saturated brine, dried over anhydrous sodium sulfate, filtered, and the filtrate was concentrated to obtain *tert*-butyl (1-(2,7-dichloro-8-fluoropyrido[4,3-d]pyrimidin-4-yl)-5,5-difluoropiperidin-3-yl)carbamate (610 mg, crude product) as a yellow solid, which was used directly in the next reaction step.
MS m/z: 452.1/454.1 $[M+H]^+$

**Step 2: *tert*-butyl (1-(7-chloro-8-fluoro-2-(((2R,7aS)-2-fluorohexahydro-1H-pyrrolizin-7a-yl)methoxy)pyrido[4,3-d]pyrimidin-4-yl)-5,5-difluoropiperidin-3-yl)carbamate**

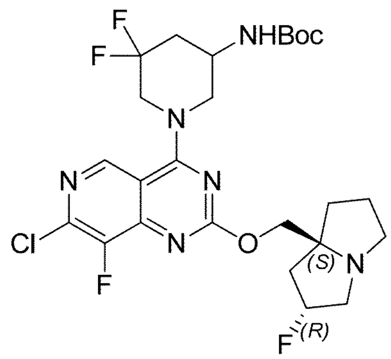

*tert*-Butyl (1-(2,7-dichloro-8-fluoropyrido[4,3-d]pyrimidin-4-yl)-5,5-difluoropiperidin-3-yl)carbamate (500 mg, 1.11 mmol, 1 *eq*) was dissolved in tetrahydrofuran (12 mL), then ((2R,7aS)-2-fluorohexahydro-1H-pyrrolizin-7a-yl)methanol (176.01 mg, 1.11 mmol, 1 *eq*) was added thereto, and sodium *tert*-butoxide (212.50 mg, 2.21 mmol, 2 *eq*) was added thereto at 0°C. The reaction mixture was reacted at 0°C for 1 hour. LCMS monitored that the reaction was completed. The reaction mixture was diluted with water and extracted with ethyl acetate. The organic phase was washed with saturated brine, dried over anhydrous sodium sulfate, filtered, and the filtrate was concentrated to obtain *tert*-butyl (1-(7-chloro-8-fluoro-2-(((2R,7aS)-2-fluorohexahydro-1H-pyrrolizin-7a-yl)methoxy)pyrido[4,3-d]pyrimidin-4-yl)-5,5-difluoropiperidin-3-yl)carbamate (563 mg, crude product) as a yellow solid, which was used directly in the next reaction step.
MS m/z: 575.11577.1 $[M+H]^+$

**Step 3: *tert-butyl* (5,5-difluoro-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate**

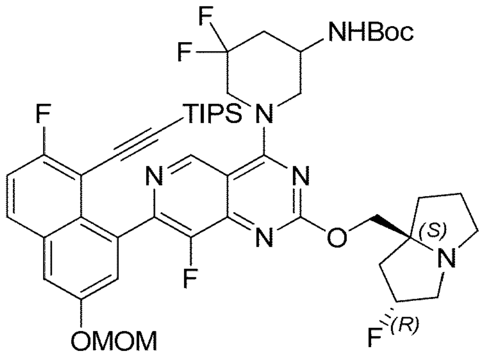

*tert*-Butyl (1-(7-chloro-8-fluoro-2-(((2R,7aS)-2-fluorohexahydro-1H-pyrrolizin-7a-yl)methoxy)pyrido[4,3-d]pyrimidin-4-yl)-5,5-difluoropiperidin-3-yl)carbamate (550 mg, 0.957 mmol, 1 *eq*) was dissolved in dioxane (6 mL) and water (2 mL), then ((2-fluoro-6-(methoxymethoxy)-8-(4,4,5,5-tetramethyl-1,3,2-dioxaborolan-2-yl)naphthalen-1-yl)ethynyl)triisopropylsilane (735.38 mg, 1.43 mmol, 1.5 *eq*) (prepared with reference to the method of intermediate 15 in WO2021041671A1), cesium carbonate (934.96 mg, 2.87 mmol, 3 *eq*), and [(bis(1-adamantyl)-N-butylphosphine)-2-(2-aminobiphenyl)palladium(II) chloride (63.96 mg, 0.0957 mmol, 0.1 *eq*) were added thereto. The reaction mixture was reacted at 100°C for 3 hours under nitrogen atmosphere. LCMS monitored that the reaction was completed. The reaction mixture was concentrated, and the residue was purified by column chromatography (tetrahydrofuran/petroleum ether: 30% to 60%) to obtain *tert*-butyl (5,5-difluoro-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (600 mg, yield: 67.81%) as a brown solid.
MS m/z: 925.3 $[M+H]^+$

**Step 4: *tert*-butyl (1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido [4,3-d]pyrimidin-4-yl)-5,5-difluoropiperidin-3-yl)carbamate**

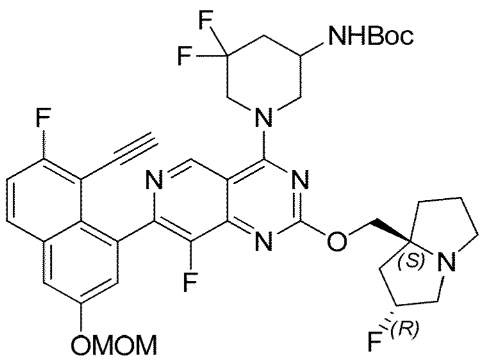

*tert*-Butyl (5,5-difluoro-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (400 mg, 0.432 mmol, 1 *eq*) was dissolved in N,N-dimethylformamide (5 mL), then cesium fluoride (328.40 mg, 2.16 mmol, 5 *eq*) was added thereto, and the reaction mixture was reacted at room temperature for 1 hour. LCMS monitored that the reaction was completed. The reaction mixture was diluted with water and extracted with ethyl acetate. The organic phase was washed with saturated brine, dried over anhydrous sodium sulfate, filtered, and the filtrate was concentrated to obtain *tert*-butyl (1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-5,5-difluoropiperidin-3-yl)carbamate (500 mg, crude product) as a brown solid, which was used directly in the next reaction step.
MS m/z: 769.3 $[M+H]^+$

**Step 5: compound 6-P1 and compound 6-P2**

**4-(4-((R)-5-Amino-3,3-difluoropiperidin-1-yl)-8-fluoro-2-(((2R,7aS)-2-fluorotetrahydro-1H-pyrrolizin-7a(5H)-yl)methoxy)pyrido [4,3-d]pyrimidin-7-yl)-5-ethynyl-6-fluoronaphthalen-2-ol and 4-(4-((S)-5-amino-3,3-difluoropiperidin-1-yl)-8-fluoro-2-(((2R,7aS)-2-fluorotetrahydro-1H-pyrrolizin-7a(SH)-yl)methoxy)pyrido[4,3-d]pyrimidin-7-yl)-5-ethynyl-6-fluoronaphthalen-2-ol**

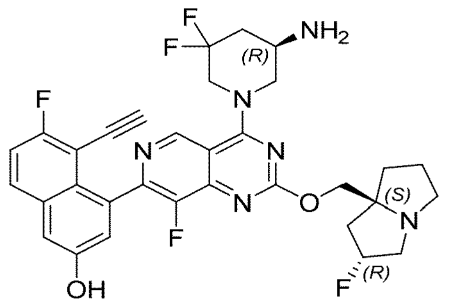

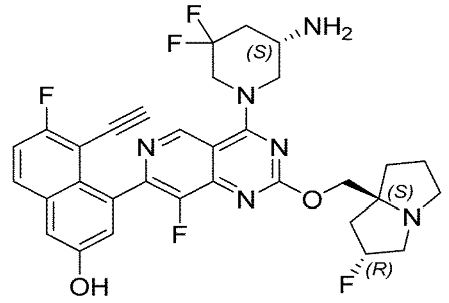

*tert*-Butyl (1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-5,5-difluoropiperidin-3-yl)carbamate (500 mg, 0.650 mmol, 1 *eq*) was dissolved in acetonitrile (5 mL), then hydrochloric acid/dioxane (4 M, 5 mL, 20 mmol) was added thereto, and the reaction mixture was reacted at room temperature for 1 hour. LCMS detected that the reaction was completed. The reaction mixture was concentrated, and the residue was purified by HPLC to obtain a crude product of 4-(4-(5-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol. The crude product was subjected to chiral separation by SFC to obtain compound 6-P1 (5.0 mg, yield: 1.2%) and compound 6-P2 (6.0 mg, yield: 1.44%), both of which were white solids.

Compound 6-P1:

SFC separation retention time: 5.639 min.
MS m/z: 625.3 $[M+H]^+$

$^{19}F$ NMR (377 MHz, $CDCl_3$) $\delta$ -98.48 (s, 1F), -109.21(s, 1F), -137.54 (s, 1F), -173.72 (s, 1F).

$^1H$ NMR (400 MHz, $CDCl_3$) $\delta$ 9.18-8.99 (m, 1H), 7.69 (br dd, *J* = 5.26, 9.05 Hz, 1H), 7.27-7.08 (m, 3H), 5.46-5.23 (m, 1H), 4.50-4.17 (m, 4H), 3.86-3.67 (m, 1H), 3.53-3.12 (m, 5H), 3.04 (br s, 1H), 2.84 (br d, *J* = 18.10 Hz, 1H), 2.53-2.13 (m, 4H) 2.09-1.92 (m, 6H).

Compound 6-P2:

SFC separation retention time: 8.575 min.
MS m/z: 625.3 $[M+H]^+$

$^{19}F$ NMR (377 MHz, DMSO-$d_6$) $\delta$ -97.08 (m, 1F), -110.65 (s, 1F), -139.93 (m, 1F), - 172.17 (s, 1F).

$^1H$ NMR (400 MHz, DMSO-$d_6$) $\delta$ 10.21 (br s, 1H), 9.23-9.05 (m, 1H), 7.99 (dd, *J* = 6.0, 9.0 Hz, 1H), 7.53-7.38 (m, 2H), 7.22 (dd, *J* = 2.3, 11.7 Hz, 1H), 5.35-5.22 (br d, *J* = 53.6 Hz, 1H), 4.72-4.43 (m, 1H), 4.37 (br t, *J* = 9.7 Hz, 1H), 4.21-4.03 (m, 2H), 4.00-3.65 (m, 1H), 3.30-3.19 (m, 3H), 3.16-2.99 (m, 4H), 2.91-2.77 (m, 1H), 2.20-2.11 (m, 1H), 2.09-1.92 (m, 3H), 1.91-1.68 (m, 3H).

HPLC separation conditions:

column: Phenomenex C18 80 * 40 mm * 3 μm;
mobile phase: [Water (ammonia hydroxide v/v) - ACN]; B%: 29% to 69%, 9 min.

SFC separation conditions:

column: Phenomenex-Cellulose-2 (250 mm * 30 mm, 10 μm);
mobile phase: [0.1% $NH_3H_2O$ MEOH]; B%: 40% to 40%.

**Example 7: compound 7**

**4-(4-((S)-3-Aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

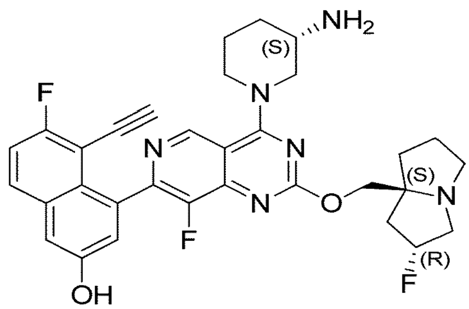

**Step 1: *tert*-butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate**

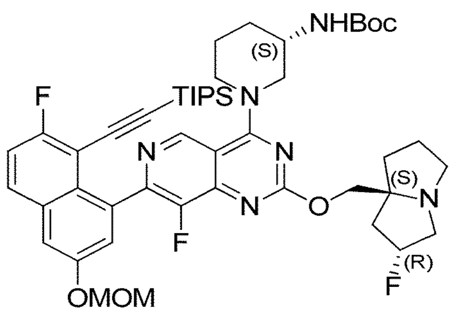

*tert*-Butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (480 mg, 0.89 mmol, 1 *eq*) and ((2-fluoro-6-(methoxymethoxy)-8-(4,4,5,5-tetramethyl-1,3,2-dioxaborolan-2-yl)naphthalen-1-yl)ethynyl)triisopropylsilane (684 mg, 1.34 mmol, 1.5 *eq*) were dissolved in 1,4-dioxane (15 mL), then [1,1'-bis(diphenylphosphino)feffocene]dichloropalladium(II) (65 mg, 0.089 mmol, 0.1 *eq*), cesium carbonate (580 mg, 1.78 mmol, 2 *eq*), and water (5 mL) were added thereto. After the system was replaced with nitrogen, the reaction mixture was reacted at 100°C for 1 hour. LCMS monitored that the reaction was completed. The reaction mixture was added with water and ethyl acetate, and extracted to separate the phases. The organic phase was washed with saturated brine, dried over anhydrous sodium sulfate, filtered, and the filtrate was concentrated. The residue was purified by column chromatography (tetrahydrofuran/petroleum ether: 0 to 50%) to obtain *tert*-butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido [4,3 -d]pyrimidin-4-yl)piperidin-3 - yl)carbamate (459 mg, yield: 58.0%) as a yellow solid. MS m/z: 889.4 $[M+H]^+$

**Step 2: *tert*-butyl ((S)-1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

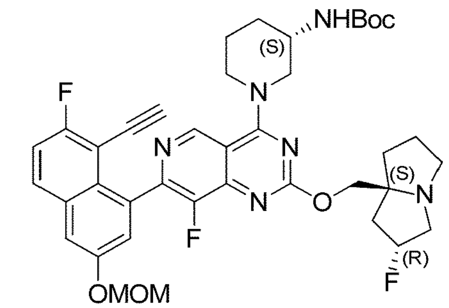

*tert*-Butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (459 mg, 0.516 mmol, 1 *eq*) was dissolved in N,N-dimethylformamide (5 mL), then cesium fluoride (78 mg, 0.516 mmol, 1 *eq*) was added thereto, and the reaction mixture was reacted at room temperature for 30 minutes. LCMS monitored that the reaction was completed. The reaction mixture was extracted with saturated brine and ethyl acetate to separate the phases. The organic phase was washed with saturated brine, dried over anhydrous sodium sulfate, filtered, and the filtrate was concentrated to obtain *tert*-butyl ((S)-1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (544 mg, crude product) as a brown oil.
MS m/z: 733.3 $[M+H]^+$

**Step 3: 4-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

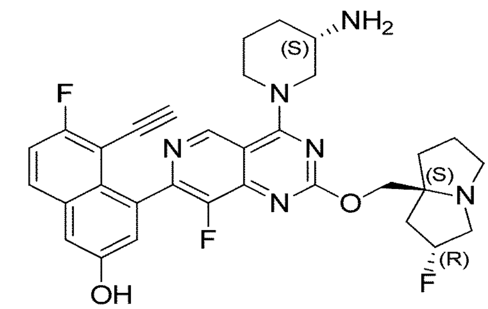

*tert*-Butyl ((S)-1-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (545 mg, 0.743 mmol, 1 *eq*) was dissolved in acetonitrile (12 mL), then hydrochloric acid/1,4-dioxane (12 mL, 4 M, 48 mmol) was added thereto, and the reaction mixture was reacted at room temperature for 30 minutes. LCMS monitored that the reaction was completed. The reaction mixture was filtered and concentrated, and the residue was purified by HPLC to obtain 4-(4-((S)-3-aminopiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (82 mg, yield: 18.7%) as a brown oil.
HPLC separation conditions:

column: Phenomenex C18 80 * 40 mm * 3 μm;
mobile phase: [Water (ammonia hydroxide v/v) - ACN]; B%: 35% to 75%, 9 min.
MS m/z: 589.2 $[M+H]^+$

$^{19}F$ NMR (376 MHz, $CDCl_3$) δ -110.75 (s, 1F), -140.50 (s, 1F), -172.17 (s, 1F).
$^1H$ NMR (400 MHz, DMSO-$d_6$) δ 9.03 (d, *J* = 13.69 Hz, 1H), 7.97 (dd, *J* = 9.23, 5.93 Hz, 1H), 7.42 (t, *J* = 9.20 Hz, 2H), 7.19 (d, *J* = 2.20 Hz, 1H), 5.28 (br d, *J* = 55.2 Hz, 1H), 4.37-4.14 (m, 2H), 4.11-4.05 (m, 1H), 4.05-3.95 (m, 2H), 3.41-3.35 (m, 2H), 3.25-3.00 (m, 5H), 3.00-2.95 (m, 1H), 2.84-2.75 (m, 1H), 2.20-1.60 (m, 10H), 1.45-1.35 (m, 1H).

**Example 8: compound 8**

**(3S,5S)-5-Amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol**

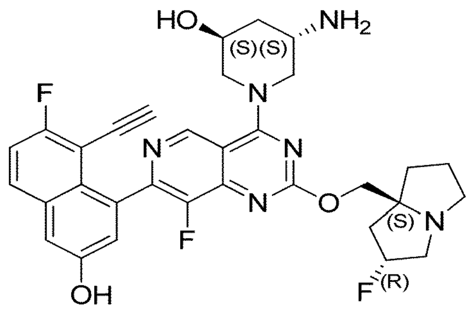

**Step 1: *tert*-butyl ((3S,5S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate**

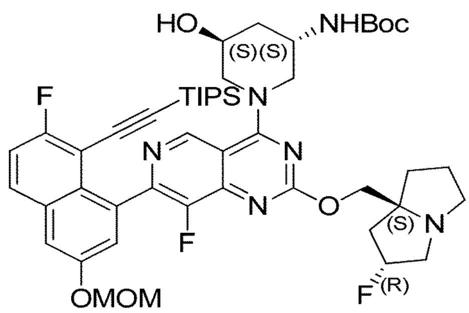

*tert*-Butyl ((3S,5S)-1-(7-chloro-8-fluoro-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate (180 mg, 0.324 mmol, 1 eq), 7-fluoro-3-(methoxymethoxy)-8-((triisopropylsilyl)ethynyl)naphthalene-1-borate (249.33 mg, 0.486 mmol, 1.5 eq), and cesium carbonate (211.34 mg, 0.649 mmol, 2 eq) were dissolved in a mixture of dioxane (4.5 mL) and water (1.5 mL), and [1,1'-bis(diphenylphosphino)ferrocene]dichloropalladium(II) (23.73 mg, 0.032 mmol, 0.1 eq) was added thereto. After the system was replaced with nitrogen, the reaction mixture was reacted at 110°C for 0.5 hours. LCMS monitored that the reaction was completed. The reaction mixture was concentrated, and the residue was purified by column chromatography (ethyl acetate/petroleum ether: 0 to 35%) to obtain tert-butyl ((3S,5S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate (200 mg, yield: 68.13%) as a yellow solid.

MS m/z: 905.4 $[M+H]^+$

$^1$H NMR (400 MHz, $CDCl_3$) δ 9.20 (d, *J* = 2.8 Hz, 1H), 7.78 (dd, *J* = 5.6, 9.0 Hz, 1H), 7.50 (d, *J* = 2.5 Hz, 1H), 7.35-7.31 (m, 1H), 7.31-7.27 (m, 1H), 5.35 (br s, 1H), 5.32-5.27 (m, 2H), 5.25-5.13 (m, 1H), 4.33-4.23 (m, 2H), 4.21-4.07 (m, 2H), 3.51 (s, 3H), 3.36-3.23 (m, 2H), 3.18 (br s, 1H), 3.02-2.91 (m, 1H), 2.36-2.14 (m, 3H), 1.94-1.83 (m, 7H), 1.48 (br s, 9H), 0.91-0.84 (m, 18H), 0.69 (br t, *J* = 5.2 Hz, 1H), 0.62-0.51 (m, 3H).

**Step 2: (3S,5S)-5-amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol**

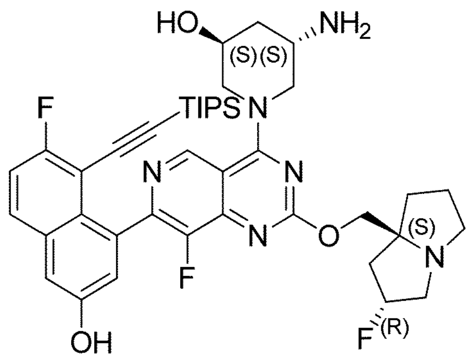

*tert*-Butyl ((3S,5S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-5-hydroxypiperidin-3-yl)carbamate (200 mg, 0.221 mmol, 1 eq) was dissolved in acetonitrile (5 mL), then a solution of hydrochloric acid in dioxane (4 M, 5 mL) was added thereto, and the reaction mixture was reacted at room temperature for 1.5 hours. LCMS monitored that the reaction was completed. The reaction mixture was concentrated to obtain (3S,5S)-5-amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol (160 mg, crude product) as a yellow oil, which was used directly in the next step.
MS m/z: 761.3 $[M+H]^+$

**Step 3: (3S,SS)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol**

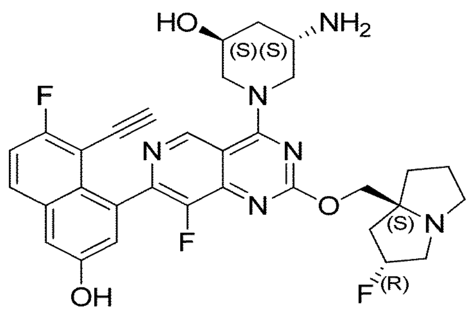

(3S,5S)-5-Amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol (190 mg, 0.250 mmol, 1 eq) was dissolved in N,N-dimethylformamide (10 mL), then cesium fluoride (379.28 mg, 2.50 mmol, 10 eq) was added thereto, and the reaction mixture was reacted at room temperature overnight. LCMS monitored that the reaction was completed. The reaction mixture was purified by HPLC to obtain (3S,5S)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol (50 mg , yield: 33.12%) as a pale yellow solid.
HPLC separation conditions:

column: Phenomenex C18 80 * 40 mm * 3 $\mu$m;
mobile phase: [Water (0.05% ammonia hydroxide v/v) - ACN]; B%: 27% to 67%, 9 min.
MS m/z: 605.2 $[M+H]^+$

$^{19}F$ NMR (376 MHz, DMSO-$d_6$) $\delta$ -110.72 (s, 1F), -140.87 (s, 1F), -172.08 (s, 1F).
$^1H$ NMR (400 MHz, DMSO-$d_6$) $\delta$ 9.35-9.06 (m, 1H), 8.01-7.93 (m, 1H), 7.46 (t, *J* = 9.0 Hz, 1H), 7.39 (t, *J* = 2.1 Hz, 1H), 7.21 (dd, *J* = 2.4, 13.9 Hz, 1H), 5.26 (br d, *J* = 54.0 Hz, 1H), 5.15-4.89 (m, 1H), 4.45-4.23 (m, 1H), 4.18-3.90 (m, 5H), 3.70 (br dd, *J* = 11.3, 19.9 Hz, 1H), 3.13-2.98 (m, 4H), 2.89-2.76 (m, 1H), 2.18-2.10 (m, 1H), 2.08-1.99 (m, 2H), 1.95-1.76 (m, 4H), 1.59 (br dd, *J* = 3.8, 9.2 Hz, 1H).

**Example 9: compound 9**

**4-(4-((1R,5S)-1-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

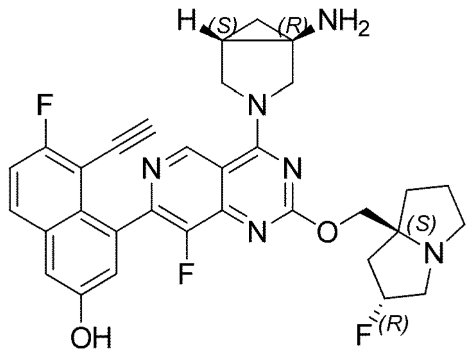

**Step 1: *tert*-butyl ((1R,5S)-3-(2,7-dichloro-8-fluoropyrido[4,3-dipyrimidin-4-yl)-3-azabicyclo[3.1.0]hexan-1-yl)carbamate**

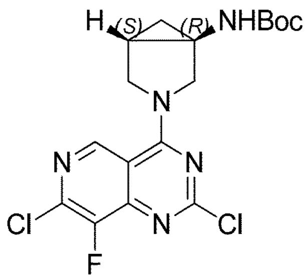

2,4,7-Trichloro-8-fluoropyrido[4,3-d]pyrimidine (400 mg, 1.58 mmol, 1 eq) and N,N-diisopropylethylamine (614.32 mg, 4.75 mmol, 0.827 mL, 3 eq) were dissolved in dichloromethane (4 mL). The system was replaced with nitrogen, and a solution of *tert*-butyl (1R,5S)-3-azabicyclo[3.1.0]hexan-1-ylcarbamate (251.30 mg, 1.27 mmol, 0.8 eq) in dichloromethane was slowly added thereto at -30°C. The reaction mixture was reacted at 0°C for 1 hour. LCMS monitored that the reaction was completed. The reaction mixture was added with saturated citric acid monohydrate aqueous solution at room temperature, and extracted with dichloromethane. The organic phase was dried over anhydrous sodium sulfate, filtered, and the filtrate was concentrated to obtain a crude product of *tert*-butyl ((1R,5S)-3-(2,7-dichloro-8-fluoropyrido[4,3-d]pyrimidin-4-yl)-3-azabicyclo[3.1.0]hexan-1-yl)carbamate (595 mg, crude product) as a yellow solid, which was used directly in the next reaction step.

MS m/z: 414.1/416.1 $[M+H]^+$

$^1H$ NMR (400 MHz, $CDCl_3$) δ 9.01 (s, 1H), 5.15 (br s, 1H), 4.61-3.91 (m, 4H), 2.02 (br s, 1H), 1.50-1.43 (m, 9H), 1.31-1.26 (m, 1H), 0.72 (t, *J* = 5.4 Hz, 1H).

**Step 2: *tert*-butyl ((1R,5S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate**

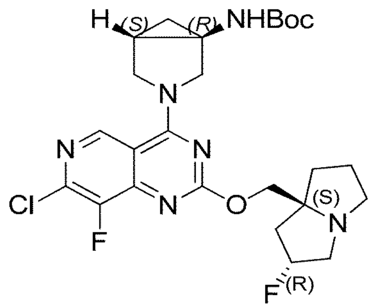

*tert*-Butyl ((1R,5S)-3-(2,7-dichloro-8-fluoropyrido[4,3-d]pyrimidin-4-yl)-3-azabicyclo[3.1.0]hexan-1-yl)carbamate (525 mg, 1.27 mmol, 1 eq) was dissolved in dioxane (10 mL), then cesium carbonate (1.24 g, 3.80 mmol, 3 eq) and ((2R,7aS)-2-fluorohexahydro-1H-pyrrolizin-7a-yl)methanol (403.52 mg, 2.53 mmol, 2 eq) were added thereto. The reaction mixture was reacted at 80°C overnight. LCMS monitored that the reaction was completed. The reaction mixture was quenched by adding water at room temperature and extracted with ethyl acetate. The organic phase was washed with saturated brine, dried over anhydrous sodium sulfate, filtered, and the filtrate was concentrated. The residue was purified by column chromatography [tetrahydrofuran (containing 0.5% ammonia hydroxide)/petroleum ether: 0 to 40%] to obtain *tert*-butyl ((1R,5S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (380 mg, yield: 55.84%) as a pale yellow oil.
MS m/z: 537.11539.1 $[M+H]^+$

**Step 3: *tert*-butyl ((1R,5S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate**

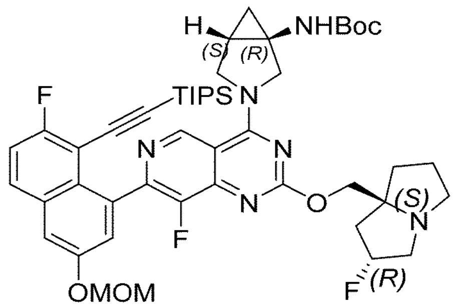

*tert*-Butyl ((1R,SS)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (350 mg, 0.651 mmol, 1 eq), ((2-fluoro-6-(methoxymethoxy)-8-(4,4,5,5-tetramethyl-1,3,2-dioxaborolan-2-yl)naphthalen-1-yl)ethynyl)triisopropylsilane (668.11 mg, 1.30 mmol, 2 eq), cesium carbonate (424.72 mg, 1.30 mmol, 2 eq), and methanesulfonato(diadamantyl-*n*-butylphosphino)-2'-amino-1,1'-biphenyl-2-yl)palladium(II) (47.47 mg, 0.065 mmol, 0.1 eq) were dissolved in a solvent mixture of dioxane (10 mL) and water (5 mL). The system was replaced with nitrogen, and the reaction mixture was reacted at 80°C for 1.5 hours. LCMS monitored that the reaction was completed. The reaction mixture was cooled to room temperature and extracted with ethyl acetate. The organic phase was washed with saturated brine, dried over anhydrous sodium sulfate, filtered, and the filtrate was concentrated. The residue was purified by column chromatography [tetrahydrofuran (containing 0.5% ammonia hydroxide)/petroleum ether: 0 to 50%] to obtain *tert*-butyl ((1R,5S)-3-(8-ffuoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (500 mg, yield: 86.48%) as a yellow oily liquid.
MS m/z: 887.4 $[M+H]^+$

**Step 4: *tert*-butyl ((1R,SS)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate**

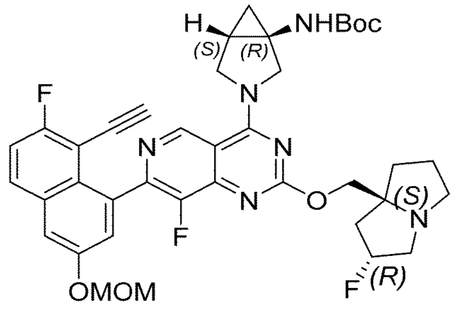

*tert*-Butyl ((1R,SS)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (400 mg, 0.451 mmol, 1 eq) was dissolved in N,N-dimethylformamide (5 mL), then cesium fluoride (684.93 mg, 4.51 mmol, 10 eq) was added thereto, and the reaction mixture was reacted at room temperature for 2 hours. LCMS monitored that the reaction was completed, and the reaction mixture was diluted with water and extracted with ethyl acetate. The organic phase was washed with saturated brine, dried over anhydrous sodium sulfate, filtered, and the filtrate was concentrated to obtain *tert*-butyl ((1R,5S)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (3 g, crude product) as a yellow oily liquid.
MS m/z: 731.2 $[M+H]^+$

**Step 5: 4-(4-((1R,5S)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

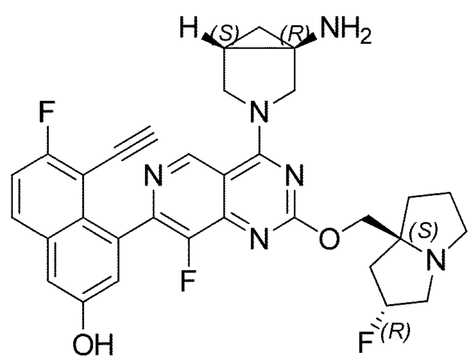

*tert*-Butyl ((1R,5S)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (300 mg, 0.411 mmol, 1 eq) was dissolved in a hydrochloric acid/methanol solution (4 M, 10 mL), and the reaction mixture was reacted at room temperature for 1 hour. LCMS monitored that the reaction was completed. The reaction mixture was concentrated, and the residue was purified by HPLC to obtain 4-(4-((1R,5S)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (50 mg, yield: 20.76%) as a white solid.
MS m/z: 587.1 $[M+H]^+$

HPLC purification conditions:

column: Phenomenex Gemini-NX 150 * 30 mm * 5 μm;
mobile phase: [water (0.05% ammonia hydroxide v/v) - ACN]; B%: 34% to 74%, 9 min).

$^{19}F$ NMR (376 MHz, $CDCl_3$) δ -109.80 (s, 1F), -136.70 (s, 1F), -172.72 (s, 1F).

$^{1}H$ NMR (400 MHz, $CDCl_3$) δ 9.08-8.83 (m, 1H), 7.54 (br s, 1H), 7.23-7.02 (m, 3H), 5.27 (br d, *J* = 53.2 Hz, 1H), 4.50-4.36 (m, 1H), 4.25-4.15 (m, 2H), 4.16-3.63 (m, 4H), 3.31 (br s, 2H), 3.25-3.10 (m, 2H), 3.05-2.90 (m, 1H), 2.77 (br s, 1H), 2.32-2.04 (m, 4H), 2.02-1.85 (m, 4H), 1.07 (br s, 1H), 0.37 (br s, 1H).

**Example 10: compound 10**

**4-(4-((1S,5R)-1-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol bis(2,2,2-trifluoroacetate)**

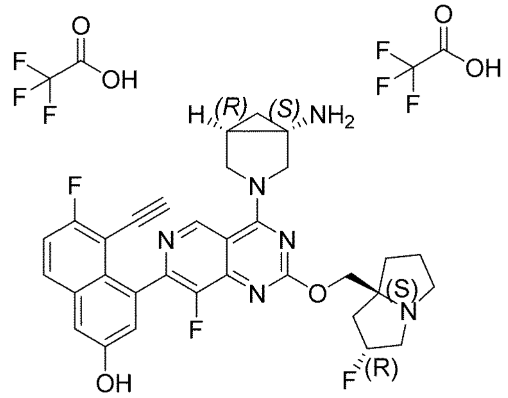

**Step 1: *tert*-butyl ((1S,5R)-3-(2,7-dichloro-8-fluoropyrido[4,3-d]pyrimidin-4-yl)-3-azabicyclo[3.1.0]hexan-1-yl)carbamate**

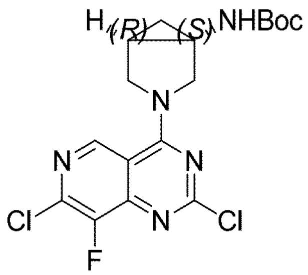

2,4,7-Trichloro-8-fluoropyrido[4,3-d]pyrimidine (1.0 g, 3.96 mmol, 1 eq) was dissolved in dichloromethane (20 mL), then N,N-diisopropylethylamine (1.54 g, 11.88 mmol, 2.07 mL, 3 eq) was added thereto, and the system was replaced with nitrogen and cooled to - 30°C. A solution of *tert*-butyl N-[(1S,5R)-3-azabicyclo[3.1.0]hexan-1-yl]carbamate (706.8 mg, 3.56 mmol, 0.9 eq) in dichloromethane (10 mL) was added dropwise thereto. The reaction mixture was reacted at -30°C for 0.5 hours, and LCMS monitored that the reaction was completed. The reaction mixture was quenched with water, extracted with dichloromethane, and the organic phase was dried over anhydrous sodium sulfate, filtered, and the filtrate was concentrated to obtain *tert*-butyl ((1S,5R)-3-(2,7-dichloro-8-fluoropyrido[4,3-d]pyrimidin-4-yl)-3-azabicyclo[3.1.0]hexan-1-yl)carbamate (2.1 g, crude product) as a yellow solid.
MS m/z: 414.0/416.0 $[M+H]^+$

**Step 2: *tert*-butyl ((1S,SR)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate**

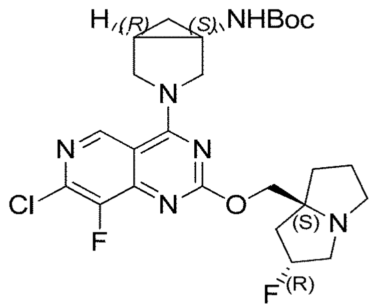

*tert*-Butyl ((1S,SR)-3-(2,7-dichloro-8-fluoropyrido[4,3-d]pyrimidin-4-yl)-3-azabicyclo[3.1.0]hexan-1-yl)carbamate (1.1 g, 2.66 mmol, 1 eq) and ((2R,7aS)-2-fluorohexahydro-1H-pyrrolizin-7a-yl)methanol (845.46 mg, 5.31 mmol, 2 eq) were dissolved in dioxane (30 mL), and cesium carbonate (2.60 g, 7.97 mmol, 3 eq) was added thereto. The reaction mixture was reacted at 80°C overnight. LCMS monitored that the reaction was completed. The reaction mixture was added with water and extracted with ethyl acetate. The organic phase was washed with saturated brine, dried over anhydrous sodium sulfate, filtered, and the filtrate was concentrated to obtain *tert*-butyl ((1S,5R)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (1.5 g, crude product) as a yellow solid.
MS m/z: 537.11539.1 $[M+H]^+$

**Step 3: *tert*-butyl ((1S,SR)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate**

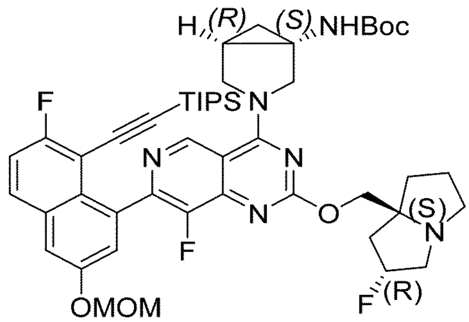

*tert*-Butyl ((1S,SR)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (500 mg, 0.931 mmol, 1 eq), 7-fluoro-3-(methoxymethoxy)-8-((triisopropylsilyl)ethynyl)naphthalene-1-borate (715.83 mg, 1.40 mmol, 1.5 eq), and cesium carbonate (606.74 mg, 1.86 mmol, 2 eq) were dissolved in a mixture of dioxane (20 mL) and water (20 mL), and [1,1'-bis(diphenylphosphino)feffocene]dichloropalladium(II) (68.13 mg, 93.11 mmol, 0.1 eq) was added thereto. After the system was replaced with nitrogen, the reaction mixture was reacted at 100°C for 0.5 hours. LCMS monitored that the reaction was completed. The reaction mixture was concentrated, and the residue was purified by column chromatography (ethyl acetate/petroleum ether: 0 to 35%) to obtain *tert*-butyl ((1S,5R)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (300 mg, yield: 36.32%) as a yellow solid.

MS m/z: 887.3 $[M+H]^+$
$^1$H NMR (400 MHz, $CDCl_3$) δ 9.12 (d, *J* = 4.8 Hz, 1H), 7.70 (dd, *J* = 5.6, 9.0 Hz, 1H), 7.42 (d, *J* = 2.4 Hz, 1H), 7.27-7.18 (m, 3H), 5.39-5.00 (m, 5H), 4.54-4.34 (m, 1H), 4.25-4.14 (m, 2H), 4.10-3.96 (m, 2H), 3.43 (s, 3H), 3.22-3.04 (m, 3H), 2.96-2.82 (m, 1H), 2.26-2.03 (m, 4H), 1.99-1.77 (m, 7H), 1.40 (br s, 9H), 1.27-1.13 (m, 7H), 0.80 (ddd, *J* = 3.1, 4.7, 7.5 Hz, 20H).

**Step 4: 4-(4-((1S,5R)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol**

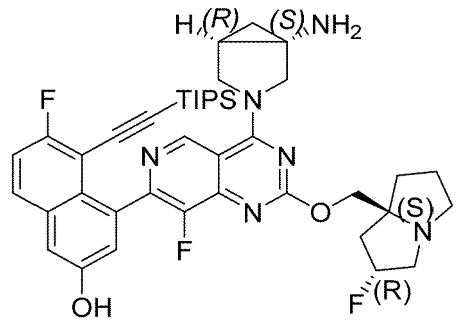

*tert*-Butyl ((1S,5R)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-1-yl)carbamate (200 mg, 0.225 mmol, 1 eq) was dissolved in acetonitrile (10 mL), then a solution of hydrochloric acid in dioxane (4 M, 10 mL, 40 mmol) was added thereto, and the reaction mixture was reacted at room temperature for 1 hour. LCMS monitored that the reaction was completed. The reaction mixture was concentrated to obtain 4-(4-((1S,5R)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (160 mg, crude product) as a yellow solid, which was used directly in the next step.
MS m/z: 743.4 $[M+H]^+$

**Step 5: 4-(4-((1S,SR)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol bis(2,2,2-trifluoroacetate)**

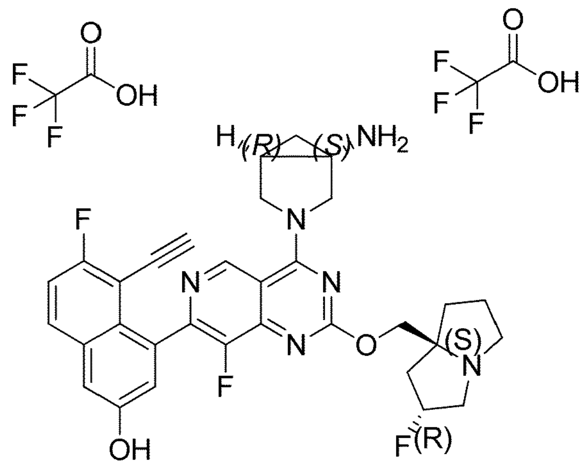

4-(4-((1S,5R)-1-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (160 mg, 0.215 mmol, 1 eq) was dissolved in N,N-dimethylformamide (10 mL), then cesium fluoride (981.41 mg, 6.46 mmol, 30 eq) was added thereto, and the reaction mixture was reacted at room temperature overnight. LCMS monitored that the reaction was completed. The reaction mixture was purified by HPLC to obtain 4-(4-((1S,SR)-1-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol bis(2,2,2-trifluoroacetate) (40 mg, yield: 31.66%) as a pale yellow solid.

HPLC separation conditions:

column: Boston Green ODS 150 * 30 mm * 5 μm;

mobile phase: [Water (TFA) - ACN]; B%: 15% to 35%, 10 min.
MS m/z: 587.2 $[M+H]^+$

$^{19}F$ NMR (376 MHz, DMSO-$d_6$) δ -73.98 (s, 6F), -110.67 (s, 1F), -139.43 (s, 1F), - 173.01 (s, 1F).

$^1H$ NMR (400 MHz, DMSO-$d_6$) δ 11.18 (br s, 1H), 9.22 (s, 1H), 9.01 (br s, 2H), 7.98 (dd, *J* = 6.0, 9.1 Hz, 1H), 7.47 (t, *J* = 9.0 Hz, 1H), 7.41 (s, 1H), 7.19 (br s, 1H), 5.57 (m, *J* = 53.6 Hz, 1H), 4.71-4.53 (m, 3H), 4.37-4.14 (m, 1H), 4.10-3.95 (m, 5H), 3.84-3.71 (m, 3H), 3.38-3.23 (m, 1H), 2.31 (br d, *J* = 12.4 Hz, 1H), 2.26-2.12 (m, 3H), 2.06 (br s, 1H), 1.47-1.38 (m, 1H), 0.87 (br t, *J* = 5.3 Hz, 1H).

**Example 11: compound 11**

**4-(4-((1R,SS,6R)-6-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol dihydrochloride**

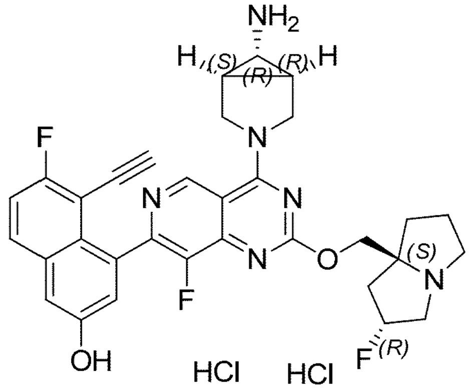

**Step 1: *tert*-butyl ((1R,5S,6s)-3-(2,7-dichloro-8-fluoropyrido[4,3-d]pyrimidin-4-yl)-3-azabicyclo [3.1.0] hexan-6-yl)carbamate**

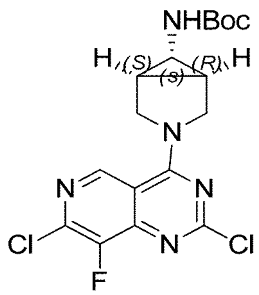

2,4,7-Trichloro-8-fluoropyrido[4,3-d]pyrimidine (1 g, 3.96 mmol, 1 eq) was dissolved in dichloromethane (30 mL), then N,N-diisopropylethylamine (1.02 g, 7.92 mmol, 2 eq) was added thereto, and *tert*-butyl (1R,5S,6s)-3-azabicyclo[3.1.0]hexan-6-ylcarbamate (746.05 mg, 3.76 mmol, 0.95 eq) was added thereto at -30°C. The reaction mixture was reacted for 30 minutes. LCMS monitored that the reaction was completed. The reaction mixture was extracted with ethyl acetate, washed with saturated brine, dried over anhydrous sodium sulfate, filtered, and concentrated, and the residue was purified by column chromatography (tetrahydrofuran/petroleum ether: 0 to 20%) to obtain *tert*-butyl ((1R,5S,6s)-3-(2,7-dichloro-8-fluoropyrido[4,3-d]pyrimidin-4-yl)-3-azabicyclo[3.1.0]hexan-6-yl)carbamate (1.4 g, yield: 85.32%) as a white solid.
MS m/z: 414.2/416.2 $[M+H]^+$

**Step 2: *tert*-butyl ((1R,5S,6R)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo [3.1.0] hexan-6-yl)carbamate**

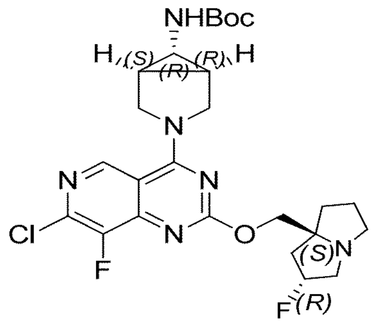

((2R,7aS)-2-Fluorohexahydro-1H-pyrrolizin-7a-yl)methanol (269.01 mg, 1.69 mmol, 1 eq) was dissolved in tetrahydrofuran (15 mL), then sodium *tert*-butoxide (324.77 mg, 3.38 mmol, 2 *eq*) was added thereto, and *tert*-butyl ((1R,5S,6s)-3-(2,7-dichloro-8-fluoropyrido[4,3-d]pyrimidin-4-yl)-3-azabicyclo[3.1.0]hexan-6-yl)carbamate (700 mg, 1.69 mmol, 1 *eq*) was added thereto under nitrogen atmosphere. The reaction mixture was reacted at room temperature for 30 minutes. LCMS monitored that the reaction was completed. The reaction mixture was quenched with saturated ammonium chloride solution and extracted with ethyl acetate. The organic phase was washed with saturated brine, dried over anhydrous sodium sulfate, filtered, and the filtrate was concentrated. The residue was purified by column chromatography (ethyl acetate/petroleum ether: 0 to 30%) to obtain *tert*-butyl ((1R,SS,6R)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (787.2 mg, yield: 86.75%) as a colorless oil.
MS m/z: 537.2/539.2 $[M+H]^+$

**Step 3: *tert*-butyl ((1R,5S,6R)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido [4,3-d] pyrimidin-4-yl)-3-azabicyclo [3.1.0] hexan-6-yl)carbamate**

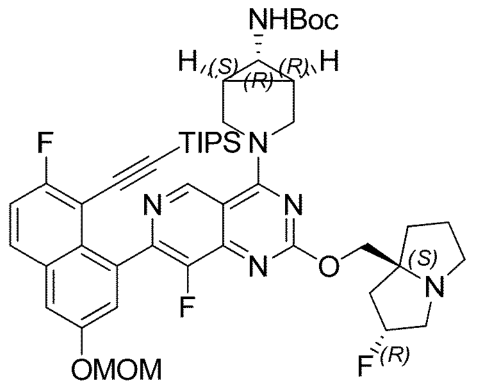

*tert*-Butyl ((1R,5S,6R)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (200 mg, 0.372 mmol, 1 *eq*) was dissolved in dioxane (5 mL) and water (1.5 mL), and methanesulfonato(diadamantyl-n-butylphosphino)-2'-amino-1,1'-biphenyl-2-yl)palladium(II) (27.12 mg, 0.0372 mmol, 0.1 *eq*) and cesium carbonate (242.70 mg, 0.745 mmol, 2 *eq*) were added thereto. After the system was replaced with nitrogen, the reaction mixture was reacted at 80°C for 2 hours. LCMS monitored that the reaction was completed, and the reaction mixture was extracted with ethyl acetate and water. The organic phase was washed with saturated brine, dried over anhydrous sodium sulfate, filtered, and the filtrate was concentrated. The residue was purified by column chromatography (ethyl acetate/petroleum ether: 0 to 70%) to obtain *tert*-butyl ((1R,SS,6R)-3-(8-fluoro-7-(7-fluoro-3-(methoxymethoxy)-8-((triisopropylsilyl)ethynyl)naphthalen-1-yl)-2-(((2R,7aS)-2-fluorohexahydro-

1H-pyrrolizin-7a-yl)methoxy)pyrido[4,3-d]pyrimidin-4-yl)-3-azabicyclo[3.1.0]hexan-6-yl)carbamate (240 mg, yield: 72.64%) as a white solid.
MS m/z: 887.5 $[M+H]^+$

**Step 4: *tert*-butyl ((1R,5S,6R)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate**

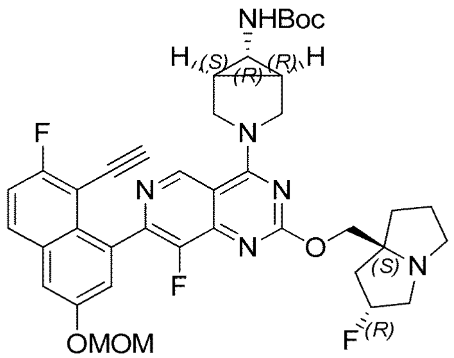

*tert*-Butyl ((1R,SS,6R)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (30.00 mg, 0.033 mmol, 1 *eq*) was dissolved in N,N-dimethylformamide (1 mL), then cesium fluoride (51.37 mg, 0.338 mmol, 10 *eq*) was added thereto, and the reaction mixture was reacted at room temperature for 4 hours. LCMS monitored that the reaction was completed. The reaction mixture was extracted with ethyl acetate, then the organic phase was washed with saturated brine, dried over anhydrous sodium sulfate, filtered, and the filtrate was concentrated to obtain *tert*-butyl ((1R,5S,6R)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (24.71 mg, crude product), which was used directly in the next step.
MS m/z: 731.3 $[M+H]^+$

**Step 5: 4-(4-((1R,SS,6R)-6-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol dihydrochloride**

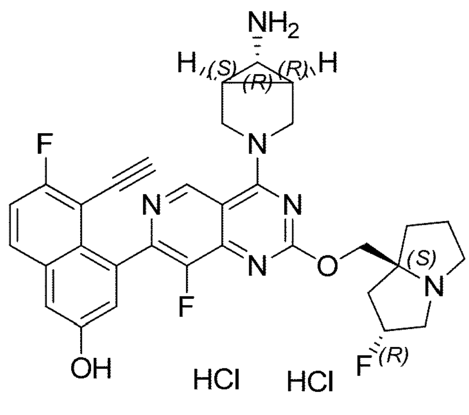

*tert*-Butyl ((1R,5S,6R)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (24.71 mg, 0.0338 mmol, 1 *eq*) was dissolved in acetonitrile (1 mL), then hydrochloric acid/dioxane (4 M, 1 mL, 4 mmol) was added thereto, and the reaction mixture was reacted at room temperature for 1 hour. LCMS monitored that the reaction was completed. The reaction mixture was concentrated, and the residue was purified by HPLC to obtain the

product (9.3 mg, yield: 42.98%) as a white solid.
MS m/z: 587.2 $[M+H]^+$

HPLC separation conditions:

column: Boston Green ODS 150 * 30 mm * 5 μm;
mobile phase: [Water (HCl) - ACN]; B%: 1% to 41%, 9 min.

$^{19}F$ NMR (376 MHz, DMSO-$d_6$) δ -110.61 (s, 1F), -139.41 (s, 1F), -172.73 (s, 1F).

$^1H$ NMR (400 MHz, DMSO-$d_6$) δ 11.39 (br s, 1H), 10.76-9.94 (m, 1H), 9.25 (s, 1H), 8.65 (br s, 3H), 7.99 (br dd, *J* = 6.3, 8.9 Hz, 1H), 7.53-7.38 (m, 2H), 7.31-7.17 (m, 1H), 5.57 (br d, *J* = 52.0 Hz, 1H), 4.71-4.55 (m, 2H), 4.43-4.08 (m, 4H), 4.00-3.81 (m, 3H), 3.78 (br d, *J* = 14.5 Hz, 2H), 3.30 (br s, 1H), 2.33 (br s, 3H), 2.31-1.92 (m, 5H).

**Example 12: compound 12**

**4-(4-((1R,5S,6S)-6-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol bis(2,2,2-trifluoroacetate)**

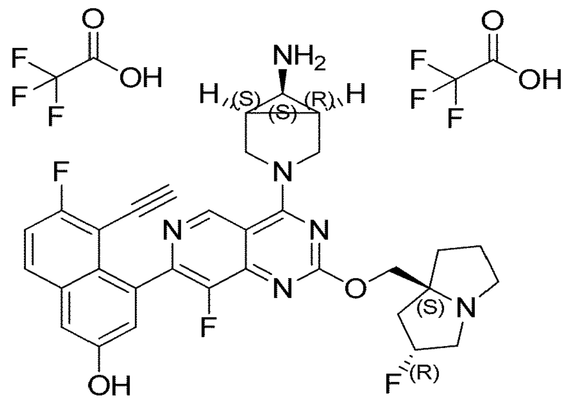

**Step 1: *tert*-butyl (1R,SS,6r)-6-(((benzyloxy)carbonyl)amino)-3-azabicyclo[3.1.0]hexane-3-carboxylate**

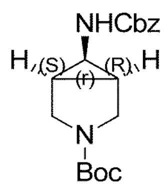

Compound *tert*-butyl (1R,5S,6r)-6-amino-3-azabicyclo[3.1.0]hexane-3-carboxylate (5 g, 25.22 mmol, 1 eq) was dissolved in tetrahydrofuran (150 mL), then potassium carbonate (3.83 g, 27.74 mmol, 1.1 eq) and benzyl chloroformate (4.73 g, 27.74 mmol, 3.94 mL, 1.1 eq) were added thereto, and the reaction mixture was reacted overnight at room temperature. LCMS detected that the reaction was completed. The reaction mixture was extracted with dichloromethane and water, then the organic phase was washed with saturated brine, dried over anhydrous sodium sulfate, filtered, and the filtrate was concentrated to obtain *tert*-butyl (1R,5S,6r)-6-(((benzyloxy)carbonyl)amino)-3-azabicyclo[3.1.0]hexane-3-carboxylate (6.54 g, yield: 78.02%) as a light yellow oil, and the crude product was used directly in the next step.
MS m/z: 233.0 $[M+H-Boc]^+$

**Step 2: benzyl (1R,SS,6r)-3-azabicyclo[3.1.0]hexan-6-ylcarbamate**

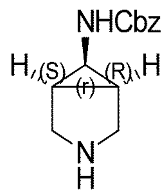

*tert*-Butyl (1R,SS,6r)-6-(((benzyloxy)carbonyl)amino)-3-azabicyclo[3.1.0]hexane-3-carboxylate (6.17 g, 18.56 mmol, 1 eq) was dissolved in anhydrous dichloromethane (75 mL), then trifluoroacetic acid (23.10 g, 202.60 mmol, 10.91 eq) was added thereto, and the reaction mixture was reacted at room temperature for 1 hour. LCMS monitored that the reaction was completed, and the reaction mixture was concentrated to obtain benzyl (1R,5S,6r)-3-azabicyclo[3.1.0]hexan-6-ylcarbamate (4 g, crude product) as a brown oily compound.
MS m/z: 233.0 [M+H]$^+$

**Step 3: benzyl ((1R,5S,6r)-3-(2,7-dichloro-8-fluoropyrido[4,3-d]pyrimidin-4-yl)-3-azabicyclo[3.1.0]hexan-6-yl)carbamate**

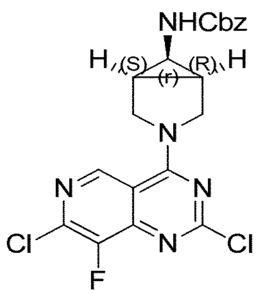

Benzyl (1R,5S,6r)-3-azabicyclo[3.1.0]hexan-6-ylcarbamate (2 g, 4.74 mmol, purity of approximately 55%, 1 eq) was dissolved in dichloromethane (25 mL), then N,N-diisopropylethylamine (6.12 g, 47.36 mmol, 10 eq) was added thereto, and then a solution of 2,4,7-trichloro-8-fluoropyrido[4,3-d]pyrimidine (1.20 g, 4.74 mmol, 1 eq) in dichloromethane (25 mL) was added thereto. The reaction mixture was reacted at -30°C for 0.5 hours, and LCMS monitored that the reaction was completed. The reaction mixture was extracted with dichloromethane and water, and the organic phase was dried over anhydrous sodium sulfate, filtered, and the filtrate was concentrated to obtain benzyl ((1R,5S,6r)-3-(2,7-dichloro-8-fluoropyrido[4,3-d]pyrimidin-4-yl)-3-azabicyclo[3.1.0]hexan-6-yl)carbamate (1.46 g, yield: 68.94%) as a pale yellow solid.
MS m/z: 448.3/450.3 [M+H]$^+$

**Step 4: benzyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate**

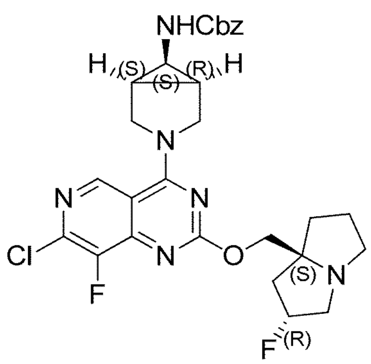

Benzyl ((1R,5S,6r)-3-(2,7-dichloro-8-fluoropyrido[4,3-d]pyrimidin-4-yl)-3-azabicyclo[3.1.0]hexan-6-yl)carbamate (1.4 g, 3.12 mmol, 1 *eq*) and ((2R,7aS)-2-fluorohexahydro-1H-pyrrolizin-7a-yl)methanol (745.79 mg, 4.68 mmol, 1.5 *eq*) were dissolved in tetrahydrofuran (50 mL), and sodium tert-butoxide (900.38 mg, 9.37 mmol, 3 *eq*) was added thereto. The reaction mixture was reacted at 0°C for 0.5 hours, and LCMS monitored that the reaction was completed. The reaction mixture was extracted with ethyl acetate and water, then the organic phase was washed with saturated brine, dried over anhydrous sodium sulfate, filtered, and the filtrate was concentrated to obtain benzyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (2.03 g, crude product) as a yellow oil.
MS m/z: 571.2/573.2 $[M+H]^+$

**Step 5: (1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-amine**

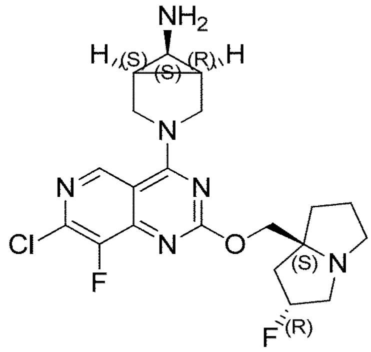

Benzyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (0.3 g, 0.525 mmol, 1 eq) was dissolved in dichloromethane (10 mL), then boron trichloride (1 M, 5.25 mL, 5.25 mmol, 10 eq) was added thereto at 0°C, and the reaction mixture was reacted at room temperature for 3.5 hours. LCMS monitored that the reaction was completed. The reaction mixture was quenched with water and extracted with dichloromethane. The organic phase was washed with saturated brine, dried over anhydrous sodium sulfate, filtered, and the filtrate was concentrated to obtain (1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-amine (0.2 g, crude product), which was used directly in the next reaction step.
MS m/z: 437.11439.1 $[M+H]^+$

**Step 6: *tert*-butyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate**

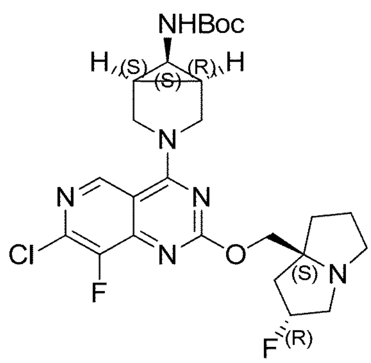

(1R,5S,6S)-3-(7-Chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-amine (0.3 g, 0.687 mmol, 1 eq) was dissolved in tetrahydrofuran (2 mL)

and saturated sodium bicarbonate aqueous solution (2 mL), and di-*tert*-butyl dicarbonate (149.87 mg, 0.687 mmol, 1 eq) was added thereto at 0°C. The reaction mixture was reacted at room temperature for 1 hour. LCMS monitored that the reaction was completed. The reaction mixture was extracted with ethyl acetate and water, then the organic phase was washed with saturated brine, dried over anhydrous sodium sulfate, filtered, and the filtrate was concentrated. The residue was purified by column chromatography (ethyl acetate/petroleum ether: 10% to 80%) to obtain *tert*-butyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (0.058 g, yield: 15.73%) as a yellow solid.
MS m/z: 537.3/539.3 $[M+H]^+$

**Step 7: *tert*-butyl ((1R,5S,6S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d] pyrimidin-4-yl)-3-azabicyclo[3.1.0]hexan-6-yl)carbamate**

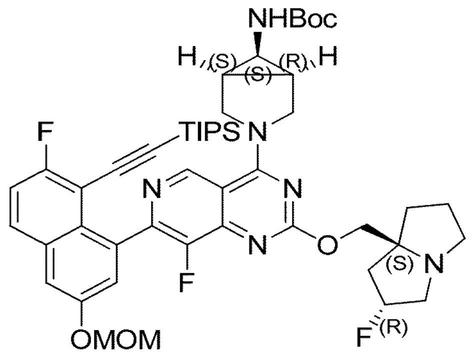

*tert*-Butyl ((1R,5S,6S)-3-(7-chloro-8-fluoro-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (0.05 g, 0.0931 mmol, 1*eq*) and ((2-fluoro-6-(methoxymethoxy)-8-(4,4,5,5-tetramethyl-1,3,2-dioxaborolan-2-yl)naphthalen-1-yl)ethynyl)triisopropylsilane (71.58 mg, 0.140 mmol, 1.5 *eq*) were dissolved in dioxane (3 mL) and water (1 mL). Cesium carbonate (60.67 mg, 0.186 mmol, 2 eq) and chloro[(*n*-butylbis(1-adamantyl)phosphine)-2-(2-aminobiphenyl)]palladium(II) (6.78 mg, 0.00931 mmol, 0.1 *eq*) were added thereto. After the system was replaced with nitrogen three times, the reaction mixture was reacted at 80°C for 2 hours. LCMS monitored that the reaction was completed. The reaction mixture was extracted with ethyl acetate, then the organic phase was washed with saturated brine, dried over anhydrous sodium sulfate, filtered, and the filtrate was concentrated. The residue was purified by TLC [tetrahydrofuran/petroleum ether = 3:2] to obtain *tert*-butyl ((1R,SS,6S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (0.026 g, yield: 31.48%) as a white solid.
MS m/z: 887.4 $[M+H]^+$

**Step 8: 4-(4-((1R,5S,6S)-6-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol**

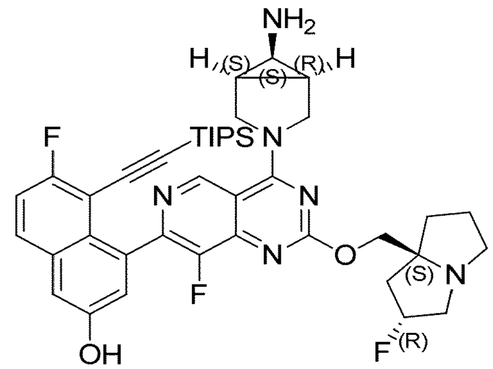

*tert*-Butyl ((1R,5S,6S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3-azabicyclo[3.1.0]hexan-6-yl)carbamate (0.026 g, 0.0293 mmol, 1 *eq*) was dissolved in acetonitrile (5 mL), then hydrochloric acid/dioxane (4 M, 0.37 mL, 50 *eq*) was added thereto, and the reaction mixture was reacted at room temperature for 1 hour. LCMS monitored that the reaction was completed. The reaction mixture was concentrated to obtain a crude product of compound 4-(4-((1R,5S,6S)-6-amino-3-azabicyclo[3.1.0]hexan-3-yl)-8-fluoro-2-(((2R,7aS)-2-fluorahexahydro-1H-pyrrolizin-7a-yl)methoxy)pyrido[4,3-d]pyrimidin-7-yl)-6-fluoro-5-((triisopropylsilyl)ethynyl)naphthalen-2-ol (25 mg, crude product) as a pale yellow solid.
MS m/z: 743.5 $[M+H]^+$

**Step 9: 4-(4-((1R,5S,6S)-6-amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol bis(2,2,2-trifluoroacetate)**

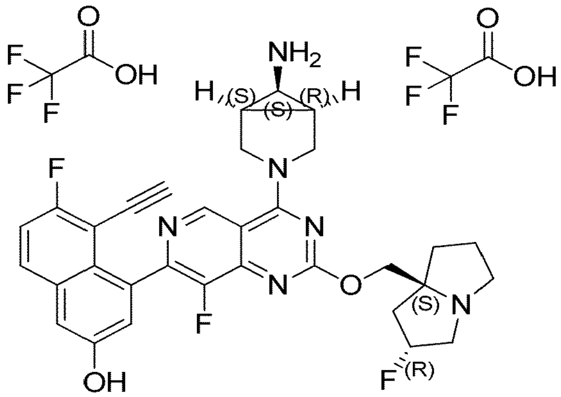

4-(4-((1R,SS,6S)-6-Amino-3-azabicyclo[3.1.0]hexan-3-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (25 mg, 0.0321 mmol, 1 *eq*) was dissolved in N,N-dimethylformamide (1 mL), then cesium fluoride (243.62 mg, 1.60 mmol, 50 *eq*) was added thereto, and the reaction mixture was reacted at room temperature overnight. LCMS monitored that the reaction was completed, and the reaction mixture was purified by preparative HPLC to obtain the product (3 mg, yield: 11.5%) as a pale yellow solid.

HPLC separation conditions:

column: Boston Green ODS 150 * 30 mm * 5 mm;
mobile phase: [water (0.1% TFA) - ACN], B%: 6% to 46%, 9 min].
MS m/z: 587.4 $[M+H]^+$

$^{19}F$ NMR (376 MHz, DMSO-$d_6$) δ -73.79 (s, 6F), -110.60 (s, 1 F), -139.73 (s, 1 F), - 173.06 (s, 1 F).

$^1H$ NMR (400 MHz, DMSO-$d_6$) δ 10.83 (s, 1H), 10.26 (br s, 1H), 9.26 (s, 1H), 8.37 (br s, 3H), 8.00 (dd, *J* = 5.94, 9.07 Hz, 1H), 7.48 (t, *J* = 8.80 Hz, 1H), 7.42 (s, 1H), 7.30-6.94 (m, 2H), 5.57 (br d, *J* = 53.6 Hz, 1H), 4.66-4.52 (m, 2H),

4.35 (br d, *J* = 12.13 Hz, 1H), 4.30-4.10 (m, 3H), 3.95-3.70 (m, 4H), 3.36-3.26 (m, 3H), 2.32-2.01 (m, 6H).

**Example 13: compound 13**

**(3S,SR)-5-Amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol**

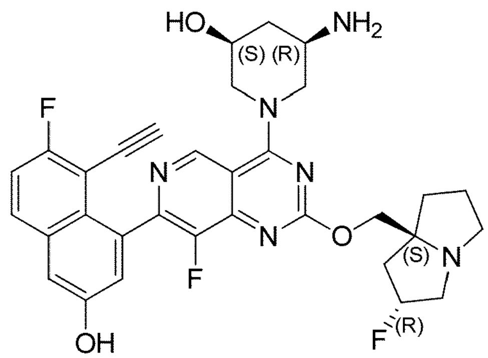

**Step 1: *tert*-butyl ((3R,5S)-1-(2,7-dichloro-8-fluoropyrido[4,3-d]pyrimidin-4-yl)-5-hydroxypiperidin-3-yl)carbamate**

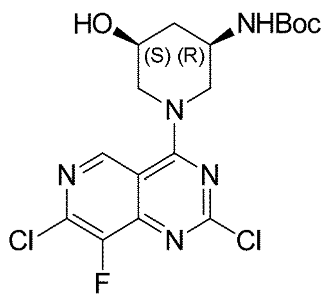

2,4,7-Trichloro-8-fluoropyrido[4,3-d]pyrimidine (1.0 g, 3.96 mmol, 1 *eq*) was dissolved in dichloromethane (10 mL), then N,N-diisopropylethylamine (1.54 g, 11.88 mmol, *3 eq*) was added thereto, and *tert*-butyl ((3R,5S)-5-hydroxypiperidin-3-yl)carbamate (865.24 mg, 4.00 mmol, 1.01 *eq*) was added thereto at -30°C. The reaction mixture was reacted for 30 minutes. LCMS detected that the reaction was completed, and the reaction was quenched by adding water and extracted with dichloromethane. The organic phase was washed with saturated brine, dried over anhydrous sodium sulfate, filtered, and the filtrate was concentrated to obtain *tert*-butyl ((3R,5S)-1-(2,7-dichloro-8-fluoropyrido[4,3-d]pyrimidin-4-yl)-5-hydroxypiperidin-3-yl)carbamate (1.3 g, crude product) as a yellow solid.
MS m/z: 432.0/434.0 $[M+H]^+$

**Step 2: *tert*-butyl ((3R,5S)-5-((*tert*-butyldimethylsilyl)oxo)-1-(2,7-dichloro-8-fluoropyrido[4,3-d]pyrimidin-4-yl)piperidin-3-yl)carbamate**

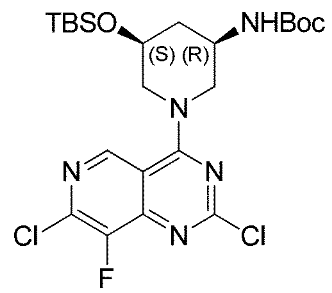

*tert*-Butyl ((3R,SS)-1-(2,7-dichloro-8-fluoropyrido[4,3-d]pyrimidin-4-yl)-5-hydroxypiperidin-3-yl)carbamate (700 mg, 1.61 mmol, 1 *eq*) was dissolved in dichloromethane (10 mL), then imidazole (220.62 mg, 3.25 mmol, 2 *eq*) and *tert*-butyldimethylsilyl chloride (366.30 mg, 2.43 mmol, 1.5 eq) were sequentially added thereto. The reaction mixture was reacted at room temperature overnight. LCMS detected that most of the raw materials were completely reacted, and the reaction was quenched by adding water and extracted with dichloromethane. The organic phase was dried over anhydrous sodium sulfate, filtered, and the filtrate was concentrated. The residue was purified by column chromatography (ethyl acetate/petroleum ether: 0 to 30%) to obtain *tert*-butyl ((3R,5S)-5-((*tert*-butyldimethylsilyl)oxo)-1-(2,7-dichloro-8-fluoropyrido[4,3-d]pyrimidin-4-yl)piperidin-3-yl)carbamate (600 mg, yield: 67.70%).
MS m/z: 546.0/548.0 $[M+H]^+$

**Step 3: *tert-butyl* ((3R,5S)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido [4,3-d]pyrimidin-4-yl)piperidin-3-yl)carbamate**

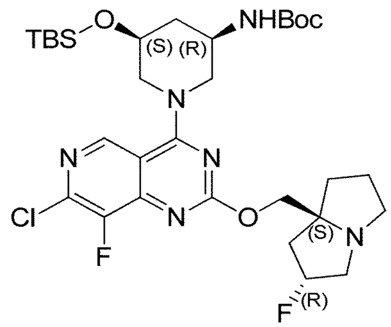

*tert*-Butyl ((3R,5S)-5-((*tert*-butyldimethylsilyl)oxo)-1-(2,7-dichloro-8-fluoropyrido[4,3-d]pyrimidin-4-yl)piperidin-3-yl)carbamate (320 mg, 0.492 mmol, purity of 84%, 1 *eq*) was dissolved in anhydrous dichloromethane (10 mL), then 4A molecular sieve (120 mg, 2.46 mmol, 5 *eq*) and ((2R,7aS)-2-fluorohexahydro-1H-pyrrolizin-7a-yl)methanol (79.08 mg, 0.497 mmol, 1.01 *eq*) were sequentially added thereto. Sodium *tert*-butoxide (94.53 mg, 0.984 mmol, 2 *eq*) was added thereto at 0°C under nitrogen atmosphere, and the reaction mixture was reacted at 0°C for 1 hour. LCMS detected that the reaction was completed. The reaction mixture was filtered, then the filtrate was concentrated, and the residue was purified by column chromatography (tetrahydrofuran/petroleum ether: 0 to 30%) to obtain *tert*-butyl ((3R,SS)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (320 mg, yield: 97.22%) as a yellow solid.

MS m/z: 669.3/671.3 $[M+H]^+$
$^1H$ NMR (400 MHz, $CDCl_3$) $\delta$ 8.99 (s, 1 H), 5.82 (br d, *J* = 8.19 Hz, 1 H), 5.32 (br d, *J* = 52.8 Hz, 1H), 4.37-4.03 (m, 6 H), 3.64 (br d, *J* = 5.50 Hz, 2 H), 3.49-3.16 (m, 3 H), 3.02 (br s, 1 H), 2.38-2.14 (m, 3 H), 2.13-1.93 (m, 5 H), 1.43 (s, 9 H), 0.90 (s, 9 H), 0.15 (s, 6 H).

**Step 4: *tert*-butyl ((3S,5R)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate**

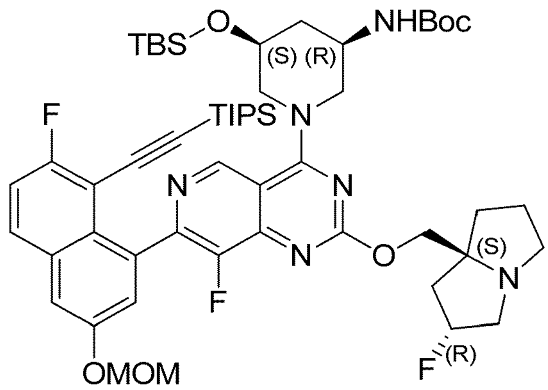

*tert*-Butyl ((3R,5S)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (450 mg, 0.673 mmol, 1 *eq*) was dissolved in dioxane (4 mL) and pure water (4 mL), then ((2-fluoro-6-(methoxymethoxy)-8-(4,4,5,5-tetramethyl-1,3,2-dioxaborolan-2-yl)naphthalen-1-yl)ethynyl)triisopropylsilane (516.92 mg, 1.01 mmol, 1.5 *eq*), cesium carbonate (657.21 mg, 2.02 mmol, 3 *eq*), and dichlorobis(triphenylphosphine)palladium(II) (88.56 mg, 0.121 mmol, 0.18 *eq*) were sequentially added thereto. The reaction mixture was reacted at 100°C for 3 hours under nitrogen atmosphere. LCMS detected that the reaction was completed. The reaction mixture was filtered, then the filtrate was concentrated, extracted with water and ethyl acetate, and the organic phase was dried over anhydrous sodium sulfate, filtered. The filtrate was concentrated, and the residue was separated by column chromatography (tetrahydrofuran/petroleum ether: 0 to 60%) to obtain *tert*-butyl ((3S,5R)-5-((*tert*-butyldimethylsilyl)oxo)- 1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (330 mg, yield: 48.15%) as a yellow solid.
MS m/z: 1019.4 $[M+H]^+$

**Step 5: 4-(4-((3R,5S)-3-amino-5-((*tert*-butyldimethylsilyl)oxo)piperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

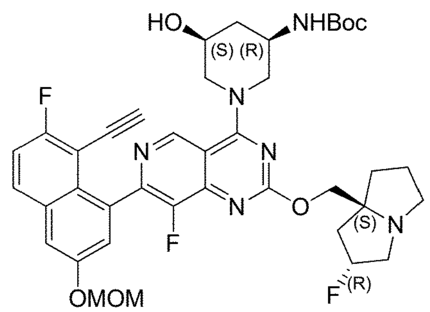

*tert*-Butyl ((3S,5R)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (300 mg, 0.294 mmol, 1 *eq*) was dissolved in N,N-dimethylformamide (10 mL), then cesium fluoride (223.52 mg, 1.47 mmol, 5 *eq*) was added thereto, and the reaction mixture was reacted at room temperature for 1 hour. LCMS detected that the reaction was completed. The reaction mixture was extracted with water and ethyl acetate, then the organic phase was dried over anhydrous sodium sulfate, filtered, and the filtrate was concentrated to obtain 4-(4-((3R,5 S)-3-amino-5-((*tert*-butyldimethylsilyl)oxo)piperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (300 mg, crude product) as a yellow oil.
MS m/z: 749.3 $[M+H]^+$

**Step 6: (3S,SR)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol**

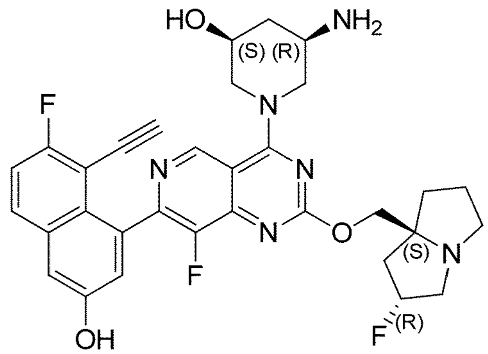

4-(4-((3R,5S)-3-Amino-5-((*tert*-butyldimethylsilyl)oxo)piperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (300 mg, 0.240 mmol, 1 eq) was dissolved in acetonitrile (3 mL), then a solution of hydrochloric acid in dioxane (4 M, 3.46 mL) was added thereto, and the reaction mixture was reacted at room temperature for 2 hours. LCMS detected that the reaction was completed. The reaction mixture was concentrated, and the residue was purified by preparative HPLC to obtain (3S,5R)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol (40.0 mg, total yield over two steps: 22.0%) as a yellow solid.

HPLC preparation conditions:

column: Boston Prime C18 150 * 30 mm * 5 μm;
mobile phase: [Water (0.05% ammonia hydroxide v/v) - ACN]; B%: 26% to 56%, 9 min.
MS m/z: 605.1 $[M+H]^+$

$^{19}F$ NMR (377 MHz, $CDCl_3$) δ -109.41 (br s, 1F), -134.61 (br s, 1F), -172.88 (br s, 1F).

$^{1}H$ NMR (400 MHz, $CDCl_3$) δ 10.05 (s, 1H), 7.84 (dd, *J* = 5.7, 9.2 Hz, 1 H), 7.60 (s, 1H), 7.40 (d, *J* = 2.2 Hz, 1H), 7.35-7.29 (m, 1H), 5.27 (br d, *J* = 53.2 Hz, 1H), 5.11-4.94 (br d, *J* = 13.1 Hz, 2H), 4.46 (br s, 1H), 4.33-4.14 (m, 2H), 3.49 (br s, 1H), 3.39 (br d, *J* = 13.8 Hz, 1H), 3.33-3.13 (m, 5H), 3.07-2.89 (m, 2H), 2.32-2.08 (m, 3H), 2.04-1.61 (m, 6H).

**Example 14: compound 14**

**(3R,5R)-5-Amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate)**

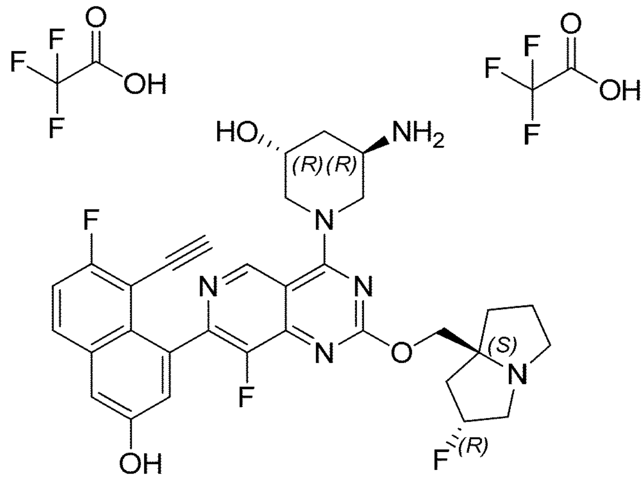

**Step 1: *tert*-butyl ((3R,SR)-1-(2,7-dichloro-8-fluoropyrido[4,3-d]pyrimidin-4-yl)-5-hydroxypiperidin-3-yl)carbamate**

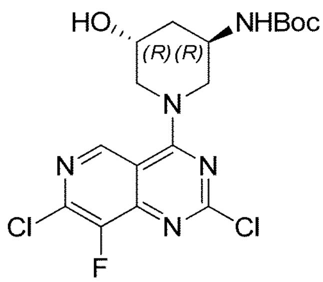

2,4,7-Trichloro-8-fluoropyrido[4,3-d]pyrimidine (648.50 mg, 2.57 mmol, 1 eq) was dissolved in dichloromethane (12 mL), then N,N-diisopropylethylamine (995.95 mg, 7.71 mmol, 1.34 mL, 3 eq) was added thereto, and the system was replaced with nitrogen and then cooled to -30°C. A solution of *tert*-butyl ((3R,5R)-5-hydroxypiperidin-3-yl)carbamate (0.5 g, 2.31 mmol, 0.9 eq) in dichloromethane (6 mL) was added dropwise thereto. The reaction mixture was reacted at -30°C for half an hour, and LCMS monitored that the reaction was completed. The reaction mixture was quenched with water and extracted with dichloromethane. The organic phase was dried over anhydrous sodium sulfate, filtered, and the filtrate was concentrated to obtain tert-butyl ((3R,5R)-1-(2,7-dichloro-8-fluoropyrido[4,3-d]pyrimidin-4-yl)-5-hydroxypiperidin-3-yl)carbamate (1 g, crude product) as a yellow solid.

MS m/z: 432.0/434.0 $[M+H]^+$

$^1H$ NMR (400 MHz, $CDCl_3$) $\delta$ 9.11 (br s, 1H), 4.57 (br s, 1H), 4.47 (br d, $J$ = 12.0 Hz, 1H), 4.37 (br d, $J$ = 12.3 Hz, 1H), 4.25 (br s, 1H), 4.06 (br s, 1H), 3.53 (br d, $J$ = 13.6 Hz, 1H), 3.42 (br s, 1H), 2.12 (br d, $J$ = 13.1 Hz, 1H), 1.79 (br s, 1H), 1.35 (s, 9H).

**Step 2: *tert*-butyl ((3R,5R)-5-((*tert*-butyldimethylsilyl)oxo)-1-(2,7-dichloro-8-fluoropyrido [4,3-d] pyrimidin-4-yl)piperidin-3-yl)carbamate**

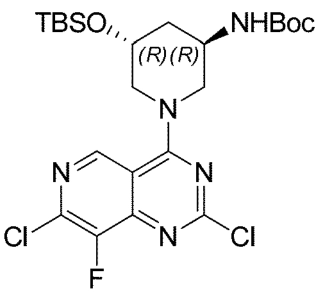

*tert*-Butyl ((3R,5R)-1 -(2,7-dichloro-8-fluoropyrido[4,3-d]pyrimidin-4-yl)-5-hydroxypiperidin-3-yl)carbamate (0.5 g, 1.16 mmol, 1 eq) was dissolved in dichloromethane (10 mL), then imidazole (322.85 mg, 4.74 mmol, 4.1 eq) and *tert*-butyldimethylsilyl chloride (697.34 mg, 4.63 mmol, 4 eq) were added thereto, and the reaction mixture was reacted at room temperature for 72 hours. LCMS monitored that the reaction was completed, and the reaction mixture was quenched with water and extracted with dichloromethane. The organic phase was dried over anhydrous sodium sulfate, filtered, and the filtrate was concentrated. The residue was purified by column chromatography (tetrahydrofuran/petroleum ether: 0 to 10%) to obtain *tert*-butyl ((3R,5R)-5-((*tert*-butyldimethylsilyl)oxo)-1-(2,7-dichloro-8-fluoropyrido[4,3-d]pyrimidin-4-yl)piperidin-3-yl)carbamate (3.7 g, yield: 39.55%) as a yellow solid.

MS m/z: 546.3/548.3 $[M+H]^+$
$^1$H NMR (400 MHz, $CDCl_3$) $\delta$ 9.06 (br s, 1H), 4.56 (br s, 1H), 4.38 (br s, 1H), 4.20 (br dd, *J* = 4.6, 13.1 Hz, 1H), 4.13-3.98 (m, 2H), 3.63-3.45 (m, 2H), 1.98 (br d, *J* = 13.0 Hz, 1H), 1.59 (br s, 1H), 1.50-1.25 (d, *J* = 6.0 Hz, 9H), 0.69 (br s, 9H), -0.06 (s, 6H).

**Step 3: *tert*-butyl ((3R,5R)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

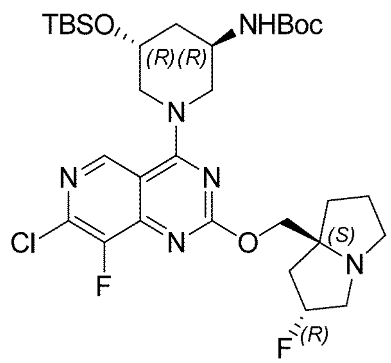

*tert*-Butyl ((3R,5R)-5-((*tert*-butyldimethylsilyl)oxo)-1-(2,7-dichloro-8-fluoropyrido[4,3-d]pyrimidin-4-yl)piperidin-3-yl)carbamate (0.5 g, 0.914 mmol, 1 eq) and ((2R,7aS)-2-fluorohexahydro-1H-pyrrolizin-7a-yl)methanol (145.65 mg, 0.914 mmol, 1 eq) were dissolved in dichloromethane (10 mL), then sodium *tert*-butoxide (175.84 mg, 1.83 mmol, 2 eq) was added thereto at 0°C, and the reaction mixture was reacted at 0°C for 1 hour. LCMS monitored that the reaction was completed, and the reaction mixture was quenched with water and extracted with dichloromethane. The organic phase was washed with saturated brine, dried over anhydrous sodium sulfate, filtered, and the filtrate was concentrated. The residue was purified by column chromatography (tetrahydrofuran/petroleum ether: 0 to 25%) to obtain *tert*-butyl ((3R,5R)-5-((*tert*-butyldimethylsilyl)oxo)-1 -(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (0.5 g, yield: 81.66%) as a yellow solid.

MS m/z: 669.4/671.4 $[M+H]^+$
$^1$H NMR (400 MHz, $CDCl_3$) $\delta$ 8.89 (s, 1H), 5.29 (br d, *J* = 53.2 Hz, 1H), 4.77 (br s, 1H), 4.39-4.27 (m, 2H), 4.26-4.20 (m, 1H), 4.19-4.07 (m, 2H), 3.99 (br d, *J* = 13.1 Hz, 1H), 3.51 (br s, 1H), 3.34-3.15 (m, 3H), 3.03-2.94 (m, 1H), 2.28 (br d, *J* = 3.8 Hz, 1H), 2.22-2.12 (m, 2H), 2.01-1.89 (m, 6H), 1.44 (br d, *J* = 6.3 Hz, 9H), 0.76 (s, 9H), 0.05 (s, 3H), -0.04 (s, 3H).

**Step 4: *tert*-butyl ((3R,5R)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate**

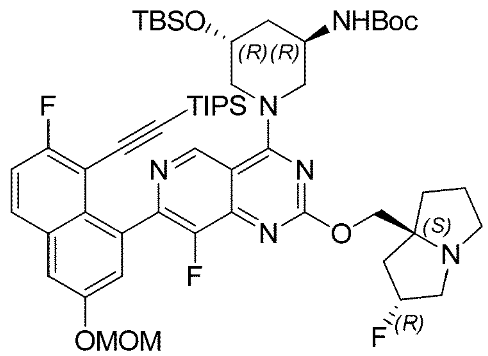

*tert*-Butyl ((3R,5R)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (0.12 g, 0.179 mmol, 1 *eq*), ((2-fluoro-6-(methoxymethoxy)-8-(4,4,5,5-tetramethyl-1, 3,2-dioxaborolan-2-yl)naphthalen-1-yl)ethynyl)triisopropylsilane (137.84 mg, 0.269 mmol, 1.5 *eq*), and cesium carbonate (175.26 mg, 0.537 mmol, 3 *eq*) were dissolved in a mixture of dioxane (8 mL) and water (4 mL), and methanesulfonato(diadamantyl-*n*-butylphosphino)-2'-amino-1,1'-biphenyl-2-yl)palladium(II) (11.99 mg, 0.0179 mmol, 0.1 *eq*) was added thereto. After the system was replaced with nitrogen, the reaction mixture was reacted at 80°C for half an hour. LCMS monitored that the reaction was completed. The reaction mixture was concentrated, and the crude product was purified by a TLC plate (petroleum ether: tetrahydrofuran = 1:2) to obtain *tert*-butyl ((3R,5R)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (0.125 g, yield: 68.39%) as a yellow solid.
MS m/z: 1019.5 $[M+H]^+$

**Step 5: (3R,5R)-5-amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol**

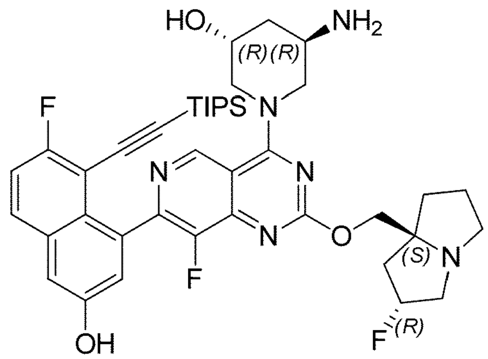

*tert*-Butyl ((3R,5R)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (0.4 g, 0.392 mmol, 1 *eq*) was dissolved in acetonitrile (20 mL), then a solution of hydrochloric acid in dioxane (4 M, 9.81 mL, 39.24 mmol) was added thereto, and the reaction mixture was reacted at room temperature for 1.5 hours. LCMS monitored that the reaction was completed. The reaction mixture was concentrated to obtain (3R,5R)-5-amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol (0.29 g, crude product) as an orange solid, which was used directly in the next step.

MS m/z: 761.6 $[M+H]^+$

**Step 6:(3R,5R)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate)**

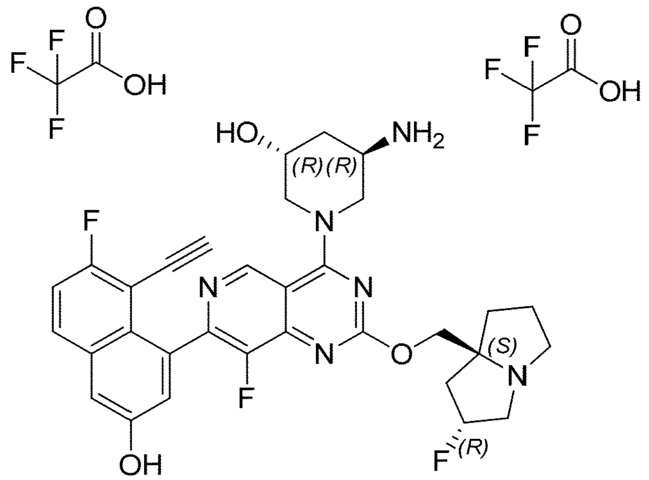

(3R,5R)-5-Amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol (0.29 g, 0.381 mmol, 1 eq) was dissolved in N,N-dimethylformamide (5 mL), then cesium fluoride (1.16 g, 7.62 mmol, 20 *eq)* was added thereto, and the reaction mixture was reacted at room temperature overnight. LCMS monitored that the reaction was completed. The reaction mixture was purified by HPLC to obtain the product (120 mg, yield: 40.2%) as a white solid.

HPLC separation conditions:

column: YMC-Actus Triart C18 150 * 30 mm * 5 μm;
mobile phase: [water (TFA) - ACN]; B%: 0% to 40%, 9 min.
MS m/z: 605.4 $[M+H]^+$

$^{19}F$ NMR (376 MHz, DMSO-$d_6$) δ -73.99 (s, 6F), -110.55 (s, 1F), -140.35 (s, 1F), - 172.86 (s, 1F).

$^1H$ NMR (400 MHz, DMSO-$d_6$) δ 11.09 (br s, 1H), 9.41-9.12 (m, 1H), 8.36 (br s, 3H), 8.09-7.87 (m, 1H), 7.55-7.36 (m, 2H), 7.23 (br d, *J* = 19.1 Hz, 1H), 5.59 (br d, *J* = 52.4 Hz, 1H), 4.79-4.46 (m, 3H), 4.37-4.05 (m, 3H), 4.00-3.70 (m, 5H), 3.31 (br s, 1H), 2.67-2.56 (m, 1H), 2.39-2.03 (m, 6H), 1.99-1.82 (m, 1H).

**Example 15: compound 15**

**4-(4-((S)-3-Aminopyrrolidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

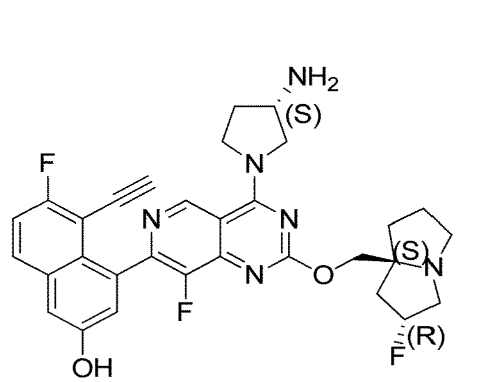

**Step 1: *tert*-butyl (S)-(1-(2,7-dichloro-8-fluoropyrido[4,3-dipyrimidin-4-yl)pyrrolidin-3-yl)carbamate**

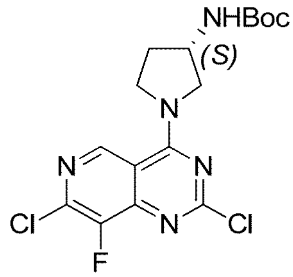

2,4,7-Trichloro-8-fluoropyrido[4,3-d]pyrimidine (1.0 g, 3.96 mmol, 1 *eq*) was dissolved in dichloromethane (20 mL), then N,N-diisopropylethylamine (1.54 g, 11.88 mmol, 2.07 mL, 3 *eq*) and (S)-3-BOC-aminopyrrolidine (663.97 mg, 3.56 mmol, 0.9 *eq*) were added thereto. The reaction mixture was reacted at -30°C for half an hour. LCMS monitored that the reaction was completed, and the reaction mixture was extracted with dichloromethane and water. The organic phase was washed with saturated brine, dried over anhydrous sodium sulfate, filtered, and the filtrate was concentrated. The residue was purified by column chromatography (ethyl acetate/petroleum ether: 0 to 30%) to obtain *tert*-butyl (S)-(1-(2,7-dichloro-8-fluoropyrido[4,3-d]pyrimidin-4-yl)pyrrolidin-3-yl)carbamate (1 g, yield: 62.76%) as a yellow solid. MS m/z: 402.0/404.0 $[M+H]^+$

**Step 2: *tert*-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)pyrrolidin-3-yl)carbamate**

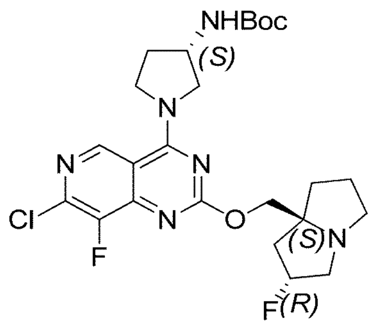

*tert*-Butyl (S)-(1-(2,7-dichloro-8-fluoropyrido[4,3-d]pyrimidin-4-yl)pyrrolidin-3-yl)carbamate (0.5 g, 1.24 mmol, 1 *eq*) was dissolved in dioxane (10 mL), then cesium carbonate (1.21 g, 3.73 mmol, 3 *eq*) and ((2R,7aS)-2-fluorohexahydro-1H-pyrrolizin-7a-yl)methanol (257.25 mg, 1.62 mmol, 1.3 *eq*) were added thereto. The reaction mixture was reacted at 80°C overnight. LCMS monitored that raw materials were not completely reacted, and the main peak was the product. The reaction mixture was extracted with ethyl acetate and water, and the organic phase was washed with saturated brine, dried over anhydrous sodium sulfate, filtered, and the filtrate was concentrated. The residue was purified by column chromatography (tetrahydrofuran/petroleum ether: 0 to 50%) to obtain *tert*-butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)pyrrolidin-3-yl)carbamate (0.3 g, yield: 45.97%) as a white solid.
MS m/z: 525.1 $[M+H]^+$

**Step 3: *tert*-butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)pyrrolidin-3-yl)carbamate**

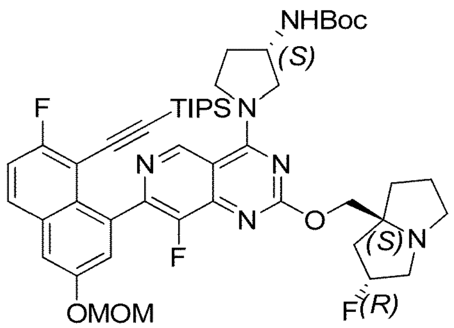

*tert*-Butyl ((S)-1-(7-chloro-8-fluoro-2-(((2R,7aS)-2-fluorohexahydro-1H-pyrrolizin-7a-yl)methoxy)pyrido[4,3-d]pyrimidin-4-yl)pyrrolidin-3-yl)carbamate (200 mg, 0.381 mmol, 1 *eq*), ((2-fluoro-6-(methoxymethoxy)-8-(4,4,5,5-tetramethyl-1,3,2-dioxaborolan-2-yl)naphthalen-1-yl)ethynyl)triisopropylsilane (195.26 mg, 0.381 mmol, 1 *eq*), cesium carbonate (372.37 mg, 1.14 mmol, 3 *eq*), and [1,1'-bis(diphenylphosphino)ferrocene]dichloropalladium(II) (27.88 mg, 0.0381 mmol, 0.1 *eq*) were dissolved in dioxane (2 mL) and water (2 mL). After the system was replaced with nitrogen, the reaction mixture was reacted at 100°C for 1 hour. LCMS monitored that the reaction was completed, and the reaction mixture was extracted with ethyl acetate and water. The organic phase was washed with saturated brine, dried over anhydrous sodium sulfate, filtered, and the filtrate was concentrated. The residue was purified by column chromatography (tetrahydrofuran/petroleum ether: 0 to 50%) to obtain *tert*-butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido [4,3 -d]pyrimidin-4-yl)pyrrolidin-3 - yl)carbamate (140 mg, yield: 41.99%) as a yellow foamy solid.
MS m/z: 875.5 $[M+H]^+$

**Step 4: 4-(4-((S)-3-aminopyrrolidin-1-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol**

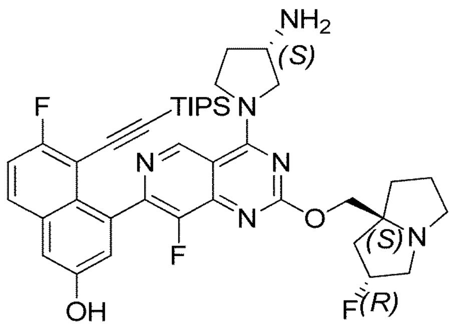

*tert*-Butyl ((S)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)pyrrolidin-3-yl)carbamate (0.14 g, 0.160 mmol, 1 *eq*) was dissolved in acetonitrile (3 mL), then hydrochloric acid/dioxane (4 M, 1 mL, 4 mmol) was added thereto, and the reaction mixture was reacted at room temperature for 1 hour. LCMS monitored that the reaction was completed. The reaction mixture was concentrated to obtain a crude product (116 mg) as a brown foamy solid, which was used directly in the next step.
MS m/z: 731.4 $[M+H]^+$

**Step 5: 4-(4-((S)-3-aminopyrrolidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

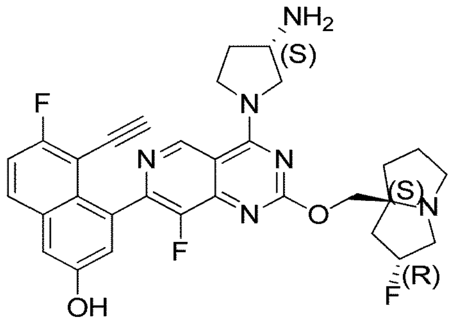

4-(4-((S)-3-Aminopyrrolidin-1-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (116.94 mg, 0.160 mmol, 1 *eq*) was dissolved in N,N-dimethylformamide (2 mL), then cesium fluoride (1.22 g, 8.00 mmol, 0.294 mL, 50 *eq*) was added thereto, and the reaction mixture was reacted at room temperature overnight. LCMS monitored that the reaction was completed. The reaction mixture was purified by HPLC to obtain 4-(4-((S)-3-aminopyrrolidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (30 mg, yield: 32.64%) as an off-white solid.

HPLC separation conditions:

column: Phenomenex Gemini-NX 80 * 40 mm * 3 $\mu$m;
mobile phase: [water (0.05% ammonia hydroxide v/v) - ACN]; B%: 31% to 71%, 9 min.

$^{19}F$ NMR (377 MHz, DMSO-$d_6$) $\delta$ -110.838 (s, 1F), -140.119 (s, 1F), -172.145 (s, 1F).

$^1H$ NMR (400 MHz, DMSO-$d_6$) $\delta$ 10.60-9.70 (m, 1H), 9.21 (br s, 1H), 7.98 (dd, *J* = 6.0, 9.0 Hz, 1H), 7.53-7.36 (m, 2H), 7.17 (t, *J* = 2.7 Hz, 1H), 5.28 (br d, *J* = 54.8 Hz, 1H), 4.15-3.96 (m, 5 H), 3.66 (br d, *J* = 5.0 Hz, 2H), 3.16-2.99 (m, 3H), 2.88-2.79 (m, 1H), 2.17-1.95 (m, 4H), 1.93-1.72 (m, 4H).

**Example 16: compound 16**

**(3R,5S)-5-Amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate)**

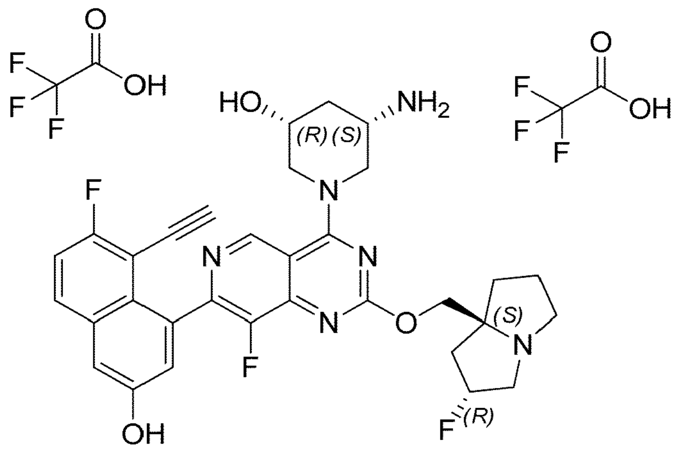

**Step 1: *tert*-butyl ((3S,5R)-1-(2,7-dichloro-8-fluoropyrido[4,3-d]pyrimidin-4-yl)-5-hydroxypiperidin-3-yl)carbamate**

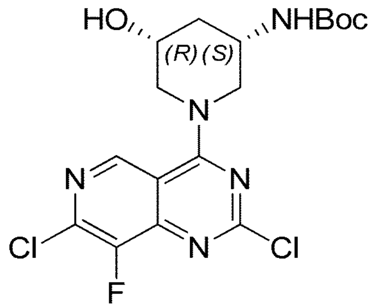

2,4,7-Trichloro-8-fluoropyrido[4,3-d]pyrimidine (160 mg, 0.6338 mmol, 1 eq) was dissolved in dichloromethane (2 mL), then N,N-diisopropylethylamine (245.72 mg, 1.90 mmol, 3 eq) was added thereto, and the system was replaced with nitrogen and then cooled to -30°C. A solution of (3R,5S)-5-(Boc-amino)piperidin-3-ol (123.36 mg, 0.5704 mmol, 0.9 eq) in dichloromethane (1 mL) was added dropwise thereto. The reaction mixture was reacted at - 30°C for 0.5 hours. LCMS monitored that the reaction was completed, and the reaction mixture was quenched with water (3 mL) and extracted with dichloromethane. The organic phase was washed with saturated brine, dried over anhydrous sodium sulfate, filtered, and the filtrate was concentrated. The residue was purified by column chromatography (methanol/dichloromethane: 0 to 2%) to obtain *tert*-butyl ((3S,5R)-1-(2,7-dichloro-8-fluoropyrido[4,3-d]pyrimidin-4-yl)-5-hydroxypiperidin-3-yl)carbamate (250 mg, yield: 91.25%) as a pale yellow solid.

MS m/z: 432.0/434.0 $[M+H]^+$
$^1$H NMR (400 MHz, DMSO-$d_6$) δ 9.17 (s, 1H), 5.41 (d, *J* = 4.3 Hz, 1H), 4.60-4.24 (m, 2H), 3.82 (m, *J* = 4.4 Hz, 1H), 3.69 (m, 1H), 3.29-3.17 (m, 2H), 3.11 (m, 1H), 2.13 (br d, *J* = 11.8 Hz, 1H), 1.54 (m, 1H), 1.40 (s, 9H).

**Step 2: *tert*-butyl ((3S,SR)-5-((*tert*-butyldimethylsilyl)oxo)-1-(2,7-dichloro-8-fluoropyrido[4,3-d]pyrimidin-4-yl)piperidin-3-yl)carbamate**

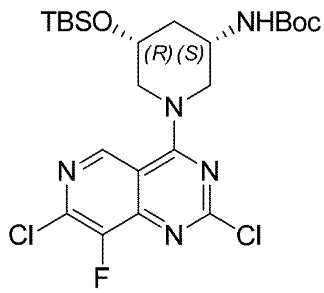

*tert*-Butyl ((3S,SR)-1-(2,7-dichloro-8-fluoropyrido[4,3-d]pyrimidin-4-yl)-5-hydroxypiperidin-3-yl)carbamate (250 mg, 0.578 mmol, 1 eq) was dissolved in dichloromethane (5 mL), then imidazole (80.71 mg, 1.19 mmol, 2.05 eq) and *tert*-butyldimethylsilyl chloride (174.33 mg, 1.16 mmol, 2 eq) were added thereto, and the reaction mixture was reacted at 50°C for 3 hours. LCMS monitored that the reaction was completed, and the reaction mixture was quenched with water (5 mL) and extracted with dichloromethane. The organic phase was dried over anhydrous sodium sulfate, filtered, and the filtrate was concentrated. The residue was purified by TLC (petroleum ether: tetrahydrofuran = 2:1) to obtain *tert*-butyl ((3S,5R)-5-((*tert*-butyldimethylsilyl)oxo)-1-(2,7-dichloro-8-fluoropyrido[4,3-d]pyrimidin-4-yl)piperidin-3-yl)carbamate (246 mg, yield: 77.83%) as colorless oil.
MS m/z: 546.3/548.3 $[M+H]^+$

**Step 3: *tert*-butyl ((3S,5R)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate**

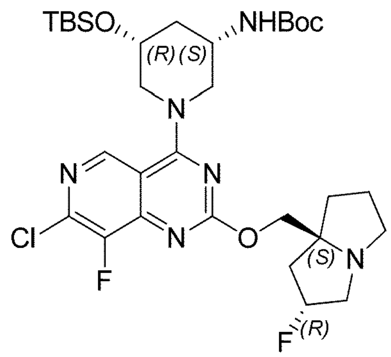

*tert*-Butyl ((3S,5R)-5-((*tert*-butyldimethylsilyl)oxo)-1-(2,7-dichloro-8-fluoropyrido[4,3-d]pyrimidin-4-yl)piperidin-3-yl)carbamate (246 mg, 0.4501 mmol, 1 eq) and ((2R,7aS)-2-fluorohexahydro-1H-pyrrolizin-7*a*-yl)methanol (107.49 mg, 0.6752 mmol, 1.5 eq) were dissolved in dichloromethane (3 mL), then sodium *tert*-butoxide (86.51 mg, 0.9002 mmol, 2 eq) was added thereto after the system was replaced with nitrogen, and the reaction mixture was reacted at room temperature for 1 hour. LCMS monitored that the reaction was completed, and the reaction mixture was quenched with water (3 mL) and extracted with dichloromethane. The organic phase was washed with saturated brine, dried over anhydrous sodium sulfate, filtered, and the filtrate was concentrated. The residue was purified by column chromatography (tetrahydrofuran/petroleum ether: 0 to 40%) to obtain *tert*-butyl ((3S,5R)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (208 mg, yield: 69.05%) as a colorless oil.
MS m/z: 669.4/671.4 $[M+H]^+$

**Step 4: *tert*-butyl ((3R,SS)-5-((*tert*-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate**

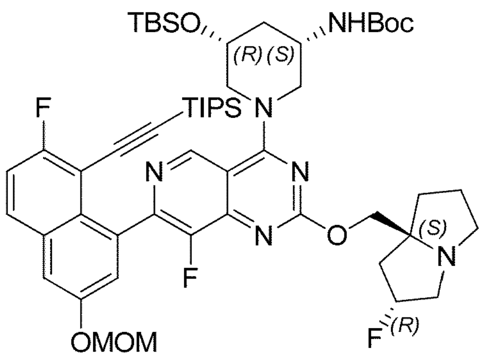

*tert*-Butyl ((3S,5R)-5-((*tert*-butyldimethylsilyl)oxo)-1-(7-chloro-8-fluoro-2-(((2R,7aS)-2-fluorohexahydro-1H-pyrrolizin-7a-yl)methoxy)pyrido[4,3-d]pyrimidin-4-yl)piperidin-3-yl)carbamate (160 mg, 0.239 mmol, 1 eq), 7-fluoro-3-(methoxymethoxy)-8-((triisopropylsilyl)ethynyl)naphthalene-1-borate (183.79 mg, 0.359 mmol, 1.5 eq), and cesium carbonate (155.78 mg, 0.478 mmol, 2 eq) were dissolved in a mixture of dioxane (4 mL) and water (3 mL), and [1,1'-bis(diphenylphosphino)feffocene]dichloropalladium(II) (17.49 mg, 0.024 mmol, 0.1 eq) was added thereto. After the system was replaced with nitrogen, the reaction mixture was reacted at 80°C for 1.5 hours. LCMS monitored that the reaction was completed. The reaction mixture was concentrated, and the crude product was purified by TLC (petroleum ether: tetrahydrofuran = 1:2) to obtain tert-butyl ((3R,5S)-5-((tertbutyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (66 mg, yield: 27.08%) as a yellow solid.
MS m/z: 1019.5$[M+H]^+$

**Step 5: (3R,5S)-5-amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol**

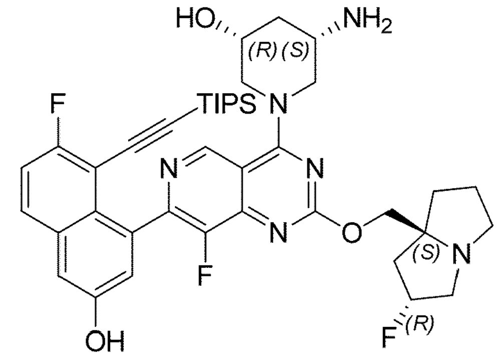

tert-Butyl ((3R,5S)-5-((tert-butyldimethylsilyl)oxo)-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-yl)carbamate (66 mg, 0.065 mmol, 1eq) was dissolved in acetonitrile (1 mL), then a solution of hydrochloric acid in dioxane (4 M, 1 mL, 4.0 mmol) was added thereto, and the reaction mixture was reacted at 40°C for 0.5 hours. LCMS monitored that the reaction was completed. The reaction mixture was concentrated to obtain (3R,5S)-5-amino-1-(8-fluoro-7-(7-fluoro-3-hydroxy-8-((triisopropylsilyl)ethynyl)naphthalen-1-yl)-2-(((2R, 7aS)-2-fluorohexahydro-1 H-pyrrolizin-7a-yl)methoxy)pyrido[4,3-d]pyrimidin-4-yl)piperidin-3-ol (78.2 mg, crude product) as a red solid, which was used directly in the next step.
MS m/z: 761.4 $[M+H]^+$

**Step 6: (3R,SS)-5-amino-1-(7-(8-ethynyl-7-fluoro-3-hydroxynaphthalen-1-yl)-8-fluoro-2-(((2R,7aS)-2-fluorohexahydro-1H-pyrrolizin-7a-yl)methoxy)pyrido[4,3-d]pyrimidin-4-yl)piperidin-3-ol bis(2,2,2-trifluoroacetate)**

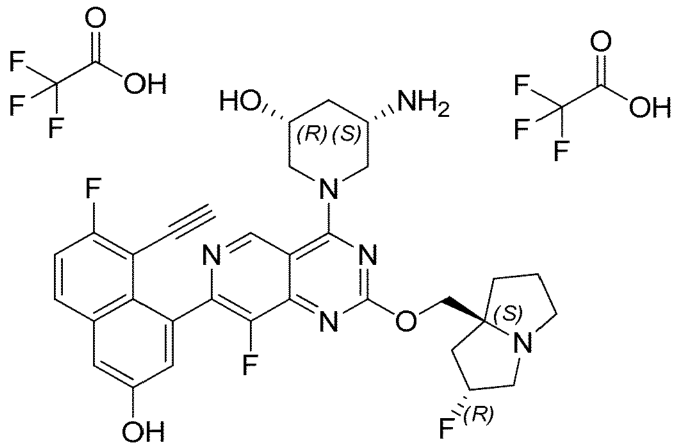

(3R,5S)-5-Amino-1-(8-fluoro-7-(7-fluoro-3-hydroxy-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-3-ol (78 mg, 0.0978 mmol, 1 eq) was dissolved in N,N-dimethylformamide (2 mL), then cesium fluoride (323.28 mg, 4.89 mmol, 50 eq) was added thereto, and the reaction mixture was reacted at room temperature overnight. LCMS monitored that the reaction was completed. The reaction mixture was directly purified by HPLC to obtain the product (15 mg, yield: 21.34%) as a red solid.

HPLC separation conditions:

column: Boston Green ODS 150 * 30 mm * 5 μm;
mobile phase: [Water (0.1%TFA) - ACN]; B%: 5% to 45%, 9 min.

MS m/z: 605.2 [M+H]+

$^{19}$F NMR (377 MHz, DMSO-$d_6$) δ -73.76 (s, 6F), -110.58 (s, 1F), -139.88 (s, 1F), - 172.98 (s, 1F).

$^{1}$H NMR (400 MHz, DMSO-$d_6$) δ 10.95 (br s, 1 H), 10.27 (br s, 1H), 9.32-9.08 (m, 1H), 8.13 (d, *J* = 9.5 Hz, 2H), 8.00 (dd, *J* = 6.0, 9.2 Hz, 1H), 7.48 (t, *J* = 9.0 Hz, 1H), 7.42 (d, *J* = 2.4 Hz, 1H), 7.23-7.19 (m, 1H), 5.59 (br d, *J* = 52.0 Hz, 1H), 4.69-4.55 (m, 2H), 4.54-4.25 (m, 2H), 4.05-3.72 (m, 5H), 3.58 (m, 2H), 3.32 (m, 3H), 2.38-2.27 (m, 3H), 2.24-2.13 (m, 3H), 2.07 (m, 1H), 1.80-1.64 (m, 1H).

**Example 17: compound 17**

**6-(4-((1R,SS)-3,8-Diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine bis(2,2,2-trifluoroacetate)**

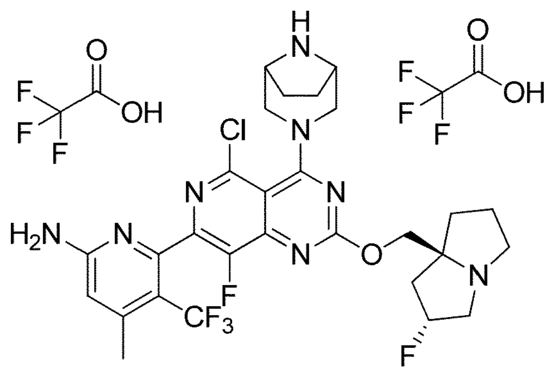

**Step 1: *tert*-butyl (1R,5S)-3-(7-(6-(bis(4-methoxybenzyl)amino)-4-methyl-3-(trifluoromethyl)pyridin-2-yl)-2,5-dichloro-8-fluoropyrido[4,3-d]pyrimidin-4-yl)-3,8-diazabicyclo[3.2.1]octane-8-carboxylate**

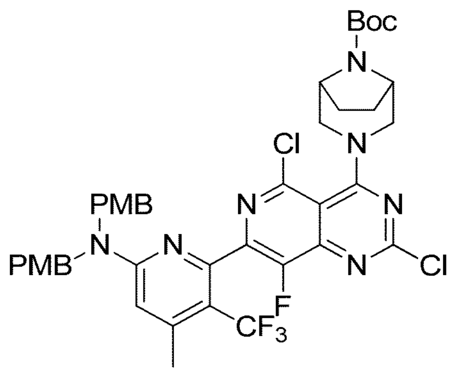

N,N-Bis(4-methoxybenzyl)-4-methyl-6-(2,4,5-trichloro-8-fluoropyrido[4,3-d]pyrimidin-7-yl)-5-(trifluoromethyl)pyridin-2-amine (500 mg, 0.750 mmol, 1 *eq)* was dissolved in dichloromethane (5 mL), then N,N-diisopropylethylamine (1.02 g, 7.92 mmol, 2 eq) was added thereto, and a solution of tert-butyl (1R,5S)-3,8-diazabicyclo[3.2.1]octane-8-carboxylate (143.25 mg, 0.675 mmol, 0.9 *eq)* in dichloromethane (5 mL) was added thereto at -30°C. The reaction mixture was reacted for 1 hour. LCMS monitored that the reaction was completed. The reaction mixture was extracted with ethyl acetate, then the organic phase was washed with saturated brine, dried over anhydrous sodium sulfate, filtered, and concentrated, and the residue was purified by column chromatography (tetrahydrofuran/petroleum ether: 0 to 20%) to obtain tert-butyl (1R,5S)-3-(7-(6-(bis(4-methoxybenzyl)amino)-4-methyl-3-(trifluoromethyl)pyridin-2-yl)-2,5-dichloro-8-fluoropyrido[4,3-d]pyrimidin-4-yl)-3,8-diazabicyclo[3.2.1]octane-8-carboxylate (580 mg, yield: 91.80%) as a yellow foamy solid.
MS m/z: 842.6/844.6 [M+H]+

**Step 2: *tert*-butyl (1R,5S)-3-(7-(6-(bis(4-methoxybenzyl)amino)-4-methyl-3-(trifluoromethyl)pyridin-2-yl)-5-chloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-**carboxylate

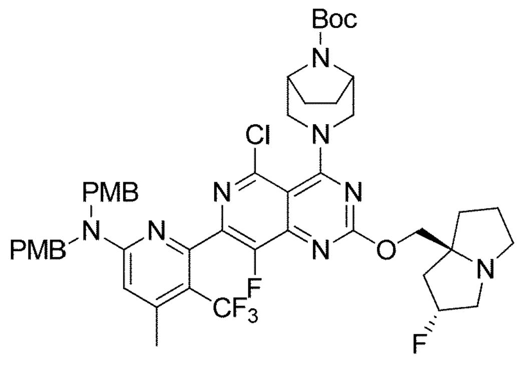

((2R,7aS)-2-Fluorohexahydro-1H-pyrrolizin-7a-yl)methanol (164.36 mg, 1.03 mmol, 1.5 *eq*) was dissolved in dichloromethane (5 mL), and sodium tert-butoxide (132.29 mg, 1.38 mmol, 2 *eq*) was added thereto, and then tert-butyl (1R,5S)-3-(7-(6-(bis(4-methoxybenzyl)amino)-4-methyl-3-(trifluoromethyl)pyridin-2-yl)-2,5-dichloro-8-fluoropyrido[4,3-d]pyrimidin-4-yl)-3,8-diazabicyclo[3.2.1]octane-8-carboxylate (580 mg, 0.688 mmol, 1 *eq)* was added thereto. The reaction mixture was reacted at room temperature for 1 hour. LCMS monitored that the reaction was completed. The reaction mixture was quenched with saturated ammonium chloride solution, extracted with ethyl acetate, and the organic phase was washed with saturated brine, dried over anhydrous sodium sulfate, filtered. The filtrate was concentrated, and the residue was purified by column chromatography (tetrahydrofuran/petroleum ether: 0 to 30%) to obtain tert-butyl (1R,5S)-3-(7-(6-(bis(4-methoxybenzyl)amino)-4-methyl-3-(trifluoromethyl)pyridin-2-yl)-5-chloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (400 mg, yield: 60.20%) as a white solid.
MS m/z: 965.4/967.4 $[M+H]^+$

**Step 3: 6-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine bis(2,2,2-trifluoroacetate)**

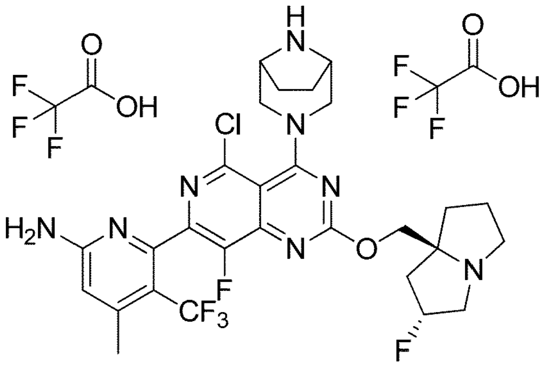

*tert*-Butyl (1R,5S)-3-(7-(6-(bis(4-methoxybenzyl)amino)-4-methyl-3-(trifluoromethyl)pyridin-2-yl)-5-chloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (100 mg, 0.104 mmol, 1 *eq)* was dissolved in trifluoroacetic acid (3 mL), and the reaction mixture was reacted at 50°C for 1 hour. The reaction mixture was concentrated, and the resulting residue was purified by HPLC to obtain 6-(4-((1R,5S)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine bis(2,2,2-trifluoroacetate) (27 mg, yield: 30.56%) as a white solid.

HPLC separation conditions:

column: YMC-Actus Triart C18 150 * 30 mm * 5 μm;
mobile phase: [Water (TFA) - ACN]; B 22% to 42%, 10.5 min.
MS m/z: 625.2/627.2 $[M+H]^+$

$^{19}F$ NMR (377 MHz, DMSO-$d_6$) δ -52.87 (s, 3F), -74.07 (s, 6F), -144.25 (s, 1F), - 172.96 (s, 1F).

$^1H$ NMR (400 MHz, DMSO-$d_6$) δ 10.98 (br s, 1H), 9.57-9.10 (m, 2H), 6.84 (br s, 2H), 6.54 (s, 1H), 5.59 (br d, *J*= 52.4 Hz, 1H), 4.61 (s, 2H), 4.32-4.04 (m, 2H), 3.99-3.82 (m, 4H), 3.30 (br s, 2H), 2.54 (br s, 1H), 2.49-2.46 (m, 1H), 2.40-2.31 (m, 4H), 2.26-1.96 (m, 4H), 1.85 (br s, 3H).

**Example 18: compound 18**

**6-(4-((1R,SS)-3,8-Diazabicyclo[3.2.1]octan-3-yl)-5,8-difluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine bis(2,2,2-trifluoroacetate)**

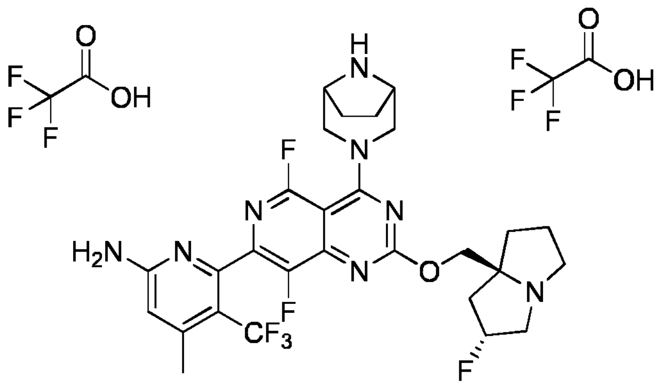

**Step 1: *tert*-butyl (1R,5S)-3-(7-(6-(bis(4-methoxybenzyl)amino)-4-methyl-3-(trifluoromethyl)pyridin-2-yl)-5,8-difluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-**carboxylate

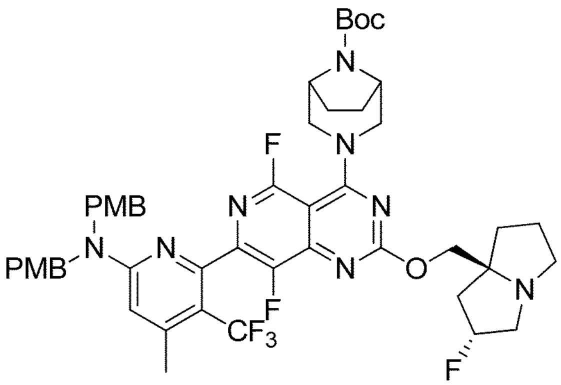

*tert-Butyl* (1R,5S)-3-(7-(6-(bis(4-methoxybenzyl)amino)-4-methyl-3-(trifluoromethyl)pyridin-2-yl)-5-chloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (140 mg, 0.145 mmol, 1 eq) was dissolved in dimethyl sulfoxide (6 mL), and cesium fluoride (1.32 g, 8.70 mmol, 60 eq) was added thereto. The reaction mixture was reacted at 80°C for 2 hours. LCMS monitored that the reaction was completed. The reaction mixture was extracted with ethyl acetate, and the organic phase was washed with saturated brine, dried over anhydrous sodium sulfate, and filtered. The filtrate was concentrated, and the

residue was purified by TLC (ethyl acetate: petroleum ether = 1:2) to obtain tert-butyl (1R,5S)-3-(7-(6-(bis(4-methoxybenzyl)amino)-4-methyl-3-(trifluoromethyl)pyridin-2-yl)-5,8-difluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (120 mg, yield: 87.2%) as a white solid.
MS m/z: 949.4 [M+H]$^+$

**Step 2: 6-(4-((1R,SS)-3,8-diazabicyclo[3.2.1]octan-3-yl)-5,8-difluoro-2-(((2R,7aS)-2-fluorohexahydro-1H-pyrrolizin-7a-yl)methoxy)pyrido[4,3-d]pyrimidin-7-yl)-4-methyl-5-(trifluoromethyl)pyridin-2-amine bis(2,2,2-trifluoroacetate)**

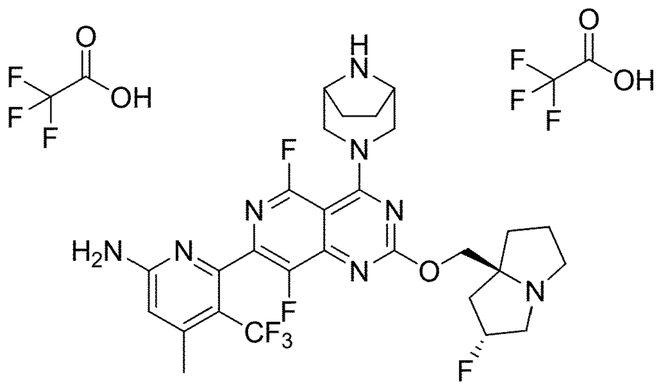

*tert*-Butyl (1R,5S)-3-(7-(6-(bis(4-methoxybenzyl)amino)-4-methyl-3-(triffuoromethyl)pyridin-2-yl)-5,8-difluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (60 mg, 0.0632 mmol, 1 eq) was dissolved in trifluoroacetic acid (3 mL), and the reaction mixture was reacted at 50°C for 2 hours. The reaction mixture was concentrated, and the resulting residue was purified by HPLC to obtain the product (8.7 mg, yield: 22.6%) as a white solid.
HPLC separation conditions:

column: Boston Green ODS 150 * 30 mm * 5 μm;
mobile phase: [Water (TFA) - ACN]; B 0% to 34%, 9 min.
MS m/z: 609.2 [M+H]$^+$

$^{19}$F NMR (377 MHz, DMSO-$d_6$) δ -52.94 (s, 3F), -74.07 (s, 6F), -144.79 (s, 1F), - 144.87 (s, 1F), -172.97 (s, 1F).
$^1$H NMR (400 MHz, DMSO-$d_6$) δ 11.12 (br s, 1H), 9.52-9.18 (m, 2H), 7.03-6.69 (m, 2H), 6.53 (s, 1H), 5.59 (br d, *J*= 52.4 Hz, 1H), 4.62 (s, 2H), 4.34 (br s, 2H), 4.18 (br s, 2H), 3.96-3.68 (m, 3H), 3.36-3.26 (m, 1H), 2.71-2.55 (m, 1H), 2.40-2.29 (m, 5H), 2.24-2.02 (m, 3H), 1.93-1.82 (m, 4H).

**Example 19: compound 19**

**7-(6-Amino-4-methyl-3-(trifluoromethyl)pyridin-2-yl)-4-((1R,SS)-3,8-diazabicyclo[3.2.1]octan-3-yl)-8-fluoro-2-(((2R,7aS)-2-fluorohexahydro-1H-pyrrolizin-7a-yl)methoxy)pyrido[4,3-d]pyrimidin-5-amine**

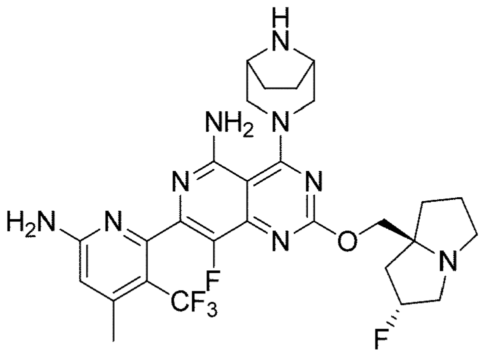

**Step 1: *tert*-butyl (1R,5S)-3-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)-5-((4-methoxybenzyl)amino)pyrido[4,3-d]pyrimidin-4-yl)-3,8-diazabicyclo [3.2.1] octane-8-carboxylate**

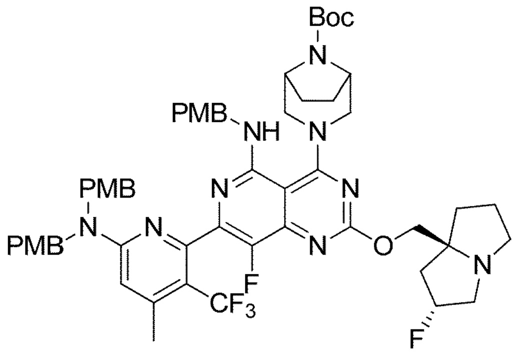

*tert-Butyl* (1R,5S)-3-(7-(6-(bis(4-methoxybenzyl)amino)-4-methyl-3-(trifluoromethyl)pyridin-2-yl)-5,8-difluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (50 mg, 0.0527 mmol, 1 *eq)* was dissolved in dimethyl sulfoxide (2 mL), and *p*-methoxybenzylamine (108.41 mg, 0.790 mmol, 15 *eq)* was added thereto. The reaction mixture was reacted under microwave irradiation at 110°C for 4 hours. LCMS monitored that the reaction was completed. The reaction mixture was extracted with ethyl acetate and water, and the organic phase was washed with saturated brine, dried over anhydrous sodium sulfate, filtered. The filtrate was concentrated, and the residue was purified by HPLC to obtain *tert*-butyl (1R,5S)-3-(7-(6-(bis(4-methoxybenzyl)amino)-4-methyl-3-(trifluoromethyl)pyridin-2-yl)-8-fluoro-2-(((2R,7aS)-2-fluorohexahydro-1H-pyrrolizin-7a-yl)methoxy)-5-((4-methoxybenzyl)amino)pyrido[4,3-d]pyrimidin-4-yl)-3,8-diazabicyclo[3.2.1]octane-8-carboxylate (4 mg, yield: 7.12%) as a white solid.
MS m/z: 1066.6 $[M+H]^+$

HPLC separation conditions:

column: Phenomenex C18 80 * 30 mm * 5 μm;
mobile phase: [Water (FA) - ACN]; B%: 45% to 65%, 12 min.

**Step 2: 7-(6-amino-4-methyl-3-(trifluoromethyl)pyridin-2-yl)-4-((1R,SS)-3,8-diazabicyclo[3.2.1]octan-3-yl)-8-fluoro-2-(((2R,7aS)-2-fluorohexahydro-1H-pyrrolizin-7a-yl)methoxy)pyrido[4,3-d]pyrimidin-5-amine**

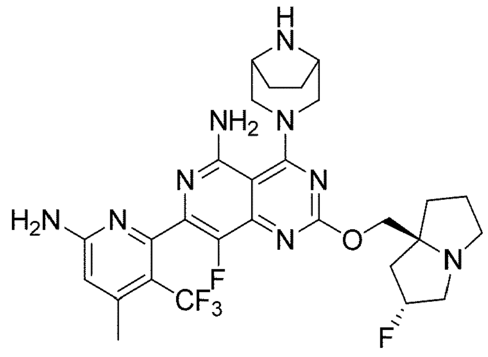

*tert-Butyl* (1R,5S)-3-(7-(6-(bis(4-methoxybenzyl)amino)-4-methyl-3-(trifluoromethyl)pyridin-2-yl)-8-fluoro-2-(((2R,7aS)-2-fluorohexahydro-1 H-pyrrolizin-7a-yl)methoxy)-5-((4-methoxybenzyl)amino)pyrido[4,3-d]pyrimidin-4-yl)-3,8-diazabicyclo[3.2.1]octane-8-carboxylate (3 mg, 0.00281 mmol, 1 *eq)* was dissolved in trifluoroacetic acid (3 mL), and the reaction mixture was reacted at 50°C for 8 hours. The reaction mixture was concentrated, and the residue was purified by HPLC to obtain 7-(6-amino-4-methyl-3-(trifluoromethyl)pyridin-2-yl)-4-((1R,5S)-3,8-diazabicyclo[3.2.1]octan-3-yl)-8-fluoro-2-(((2R,7aS)-2-fluorohexahydro-1H-pyrrolizin-7a-yl)methoxy)pyrido[4,3-d]pyrimidin-5-amine (1.06 mg, yield: 58.8%) as a white solid.

HPLC separation conditions:

column: Boston Green ODS 150 * 30 mm * 5 $\mu$m;
mobile phase: [Water (TFA) - ACN]; B 0% to 31%, 9 min.
MS m/z: 606.2 $[M+H]^+$

**Example 20: compound 20-P1 and compound 20-P2**

**4-(4-((S)-4-Amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol and 4-(4-((R)-4-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

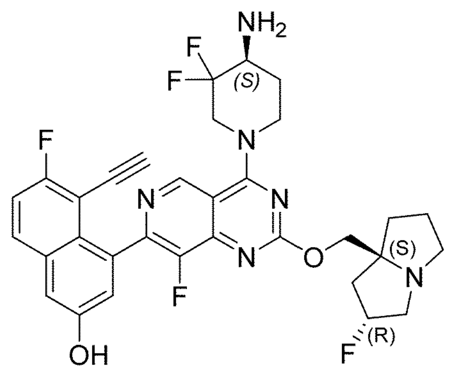

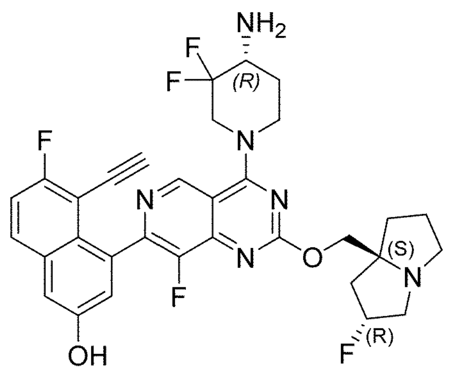

**Step 1: *tert*-butyl (1-(2,7-dichloro-8-fluoropyrido[4,3-d]pyrimidin-4-yl)-3,3-difluoropiperidin-4-yl)carbamate**

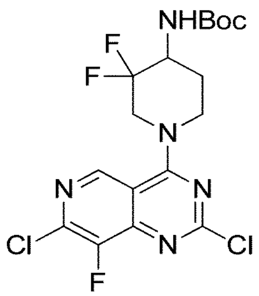

2,4,7-Trichloro-8-fluoropyrido[4,3-d]pyrimidine (500 mg, 1.98 mmol, 1 eq) was dissolved in dichloromethane (5 mL), and N,N-diisopropylethylamine (767.88 mg, 5.94 mmol, 3 eq) was added thereto. The system was replaced with nitrogen, and a solution of tert-butyl (3,3-difluoropiperidin-4-yl)carbamate (374.33 mg, 1.58 mmol, 0.8 eq) in dichloromethane (2 mL) was slowly added thereto at -30°C. The reaction mixture was reacted at -30°C for 1 hour. LCMS monitored that the reaction was completed, and the reaction mixture was added with saturated citric acid aqueous solution and extracted with dichloromethane. The organic phase was washed with saturated brine, dried over anhydrous sodium sulfate, filtered, and the filtrate was concentrated. The residue was purified by column chromatography [ethyl acetate/petroleum ether: 10 to 30%] to obtain tert-butyl (1-(2,7-dichloro-8-fluoropyrido[4,3-d]pyrimidin-4-yl)-3,3-difluoropiperidin-4-yl)carbamate (550 mg, yield: 76.76%) as a white solid.
MS m/z: 452.0/454.0 $[M+H]^+$

$^1H$ NMR (400 MHz, $CDCl_3$) δ 8.91 (s, 1H), 4.85 (br s, 2H), 4.67 (br d, *J* = 13.1 Hz, 1H), 4.27 (br s, 1H), 3.71-3.55 (m, 1H), 3.39 (br t, *J* = 12.4 Hz, 1H), 2.28 (br d, *J* = 13.1 Hz, 1H), 1.96 (dq, *J* = 3.9, 12.7 Hz, 1H), 1.48 (s, 9H).

$^{19}F$ NMR (376 MHz, $CDCl_3$) δ -110.62 (br d, *J* = 246.8 Hz, 1F), -119.23 (br d, *J* = 244.6 Hz, 1F), -131.07 (s, 1F).

**Step 2: *tert*-butyl (1-(7-chloro-8-fluoro-2-(((2R,7aS)-2-fluorohexahydro-1H-pyrrolizin-7a-yl)methoxy)pyrido[4,3-d]pyrimidin-4-yl)-3,3-difluoropiperidin-4-yl)carbamate**

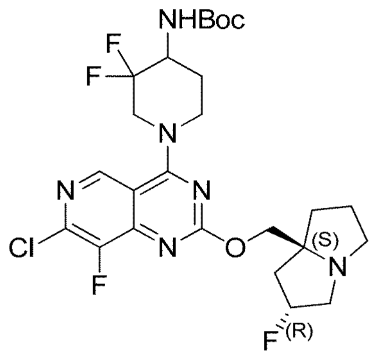

((2R,7aS)-2-Fluorohexahydro-1H-pyrrolizin-7a-yl)methanol (352.01 mg, 2.21 mmol, 1 eq) and sodium tert-butanol (424.99 mg, 4.42 mmol, 2 eq) were added to dichloromethane (10 mL), and the reaction was carried out at room temperature for 10 minutes under nitrogen atmosphere. A solution of tert-butyl (1-(2,7-dichloro-8-fluoropyrido[4,3-d]pyrunidin-4-yl)-3,3-difluoropiperidin-4-yl)carbamate (1 g, 2.21 mmol, 1 eq) in dichloromethane (10 mL) was slowly added thereto, and the reaction was carried out at room temperature for half an hour under nitrogen atmosphere. LCMS monitored that the reaction was completed. The reaction mixture was slowly poured into saturated ammonium chloride aqueous solution and extracted with dichloromethane. The organic phase was washed with saturated brine, dried over anhydrous sodium sulfate, filtered, and the filtrate was concentrated. The residue was purified by column chromatography [tetrahydrofuran (containing 0.5% ammonia hydroxide)/petroleum ether: 0 to 50%] to obtain tert-butyl (1-(7-chloro-8-fluoro-2-(((2R,7aS)-2-fluorohexahydro-1H-pyrrolizin-7a-yl)methoxy)pyrido[4,3-d]pyrimidin-4-yl)-3,3-difluoropiperidin-4-yl)carbamate (1 g, yield: 78.65%) as a yellow solid.
MS m/z: 575.1/577.1 $[M+H]^+$

$^1H$ NMR (400 MHz, $CDCl_3$) δ 8.76 (s, 1H), 5.28 (d, *J* = 52.0 Hz, 1H), 4.91 (br d, *J* = 7.8 Hz, 1H), 4.73 (br s, 1H), 4.52 (br d, *J* = 13.6 Hz, 1H), 4.33-4.15 (m, 3H), 3.58-3.44 (m, 1H), 3.35-3.18 (m, 3H), 3.16 (s, 1H), 3.02-2.92 (m, 1H), 2.28-2.20 (m, 3H), 2.19-2.07 (m, 2H), 1.97-1.89 (m, 3H), 1.47 (s, 9H).

$^{19}F$ NMR (376 MHz, $CDCl_3$) δ -110.65 (br d, *J* = 242.6 Hz, 1F), -119.20 (br d, *J* = 260.4 Hz, 1F), -133.82 (br s, 1F), -173.14 (br d, *J* = 13.7 Hz, 1F).

**Step 3: *tert*-butyl (3,3-difluoro-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-4-yl)carbamate**

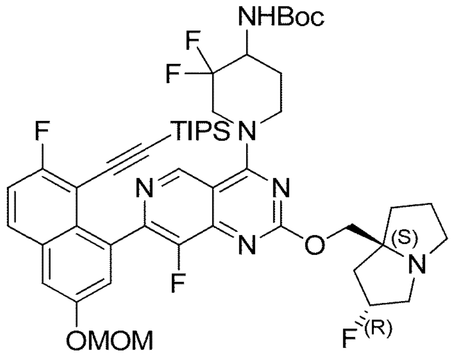

*tert*-Butyl (1-(7-chloro-8-fluoro-2-(((2R,7aS)-2-fluorohexahydro-1H-pyrrolizin-7a-yl)methoxy)pyrido[4,3-d]pyrimidin-4-yl)-3,3-difluoropiperidin-4-yl)carbamate (300 mg, 0.522 mmol, 1 eq), ((2-fluoro-6-(methoxymethoxy)-8-(4,4,5,5-tetramethyl-1,3,2-dioxaborolan-2-yl)naphthalen-1-yl)ethynyl)triisopropylsilane (534.82 mg, 1.04 mmol, 2 eq), cesium carbonate (339.99 mg, 1.04 mmol, 2 eq), and methanesulfonato(diadamantyl-n-butylphosphino)-2'-amino-1,1'-biphenyl-2-yl)palladium(II) (38.00 mg, 0.0522 mmol, 0.1 eq) were dissolved in a solvent mixture of dioxane (6 mL) and water (3 mL). The reaction mixture was reacted at 80°C for 2 hours under nitrogen atmosphere. LCMS monitored that the reaction was completed, and the phases of the reaction mixture were separated. The organic phase was washed with saturated brine, dried over anhydrous sodium sulfate, filtered, and the filtrate was concentrated. The residue was purified by column chromatography [tetrahydrofuran (containing 0.5% ammonia hydroxide)/petroleum ether: 0 to 50%] to obtain tert-butyl (3,3-difluoro-1-(8-fluoro-7-(7-fluoro-3-(methoxymethoxy)-8-((triisopropylsilyl)ethynyl)naphthalen-1 -yl)-2-(((2R,7aS)-2-fluorohexahydro-1 H-pyrrolizin-7a-yl)methoxy)pyrido[4,3-d]pyrimidin-4-yl)piperidin-4-yl)carbamate (390 mg, yield: 80.80%) as a yellow solid.
MS m/z: 925.4 $[M+H]^+$

**Step 4: 4-(4-(4-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol**

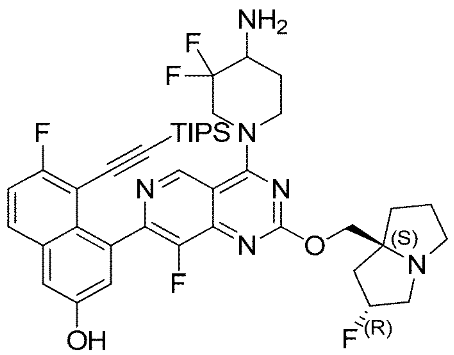

*tert*-Butyl (3,3-difluoro-1-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)piperidin-4-yl)carbamate (560 mg, 0.605 mmol, 1 eq) was dissolved in acetonitrile (5 mL), then a solution of hydrochloric acid in dioxane (4 M, 5 mL) was added thereto, and the reaction mixture was reacted at room temperature for 1 hour. LCMS monitored that the

reaction was completed. The reaction mixture was concentrated to obtain 4-(4-(4-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (500 mg, crude product) as a yellow solid, and the crude product was used directly in the next step.
MS m/z: 781.2 $[M+H]^+$

**Step 5: compound 20-P1 and compound 20-P2**

**4-(4-((S)-4-Amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol and 4-(4-((R)-4-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

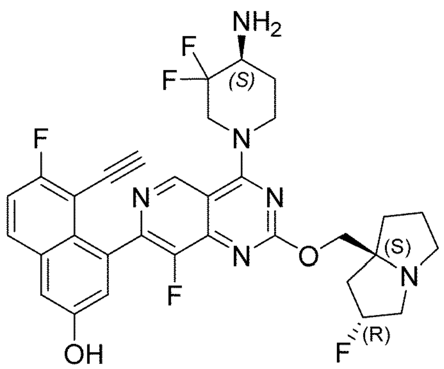
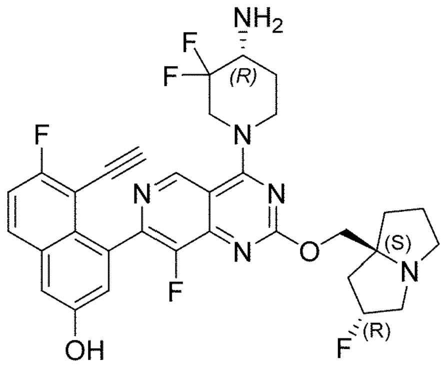

4-(4-(4-Amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (472 mg, 0.604 mmol, 1 eq) was dissolved in N,N-dimethylformamide (7 mL), then cesium fluoride (918.08 mg, 6.04 mmol, 10 eq) was added thereto, and the reaction mixture was reacted at room temperature overnight. LCMS monitored that the reaction was completed. The reaction mixture was filtered, and the filtrate was separated by HPLC to obtain 4-(4-(4-amino-3,3-difluoropiperidin-1-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (120 mg) as a brown solid. The resulting brown solid was then subjected to chiral separation by SFC to obtain compound 20-P1 (40 mg, yield: 10.6%) and compound 20-P2 (40 mg, yield: 10.6%), both of which were yellow solids.

Compound 20-P1:

SFC separation retention time: 2.528 min.
MS m/z: 625.1 $[M+H]^+$
$^1H$ NMR (400 MHz, DMSO-$d_6$) $\delta$ 10.18 (br s, 1H), 9.06 (d, *J* = 8.1 Hz, 1H), 7.98 (dd, *J* = 5.9, 9.1 Hz, 1H), 7.47 (t, *J* = 9.0 Hz, 1H), 7.40 (d, *J* = 2.3 Hz, 1H), 7.21 (dd, *J* = 2.4, 5.4 Hz, 1H), 5.29 (d, *J* = 54.0 Hz, 1H), 4.62-4.42 (m, 1H), 4.25 (br d, *J* = 12.6 Hz, 1H), 4.19-4.11 (m, 1H), 4.06 (br dd, *J* = 7.8, 10.1 Hz, 1H), 4.00-3.85 (m, 2H), 3.69 (br t, *J* = 10.6 Hz, 1H), 3.15-3.04 (m, 2H), 3.02 (s, 1H), 2.88-2.78 (m, 1H), 2.26-1.91 (m, 6H), 1.88-1.72 (m, 4H).
$^{19}F$ NMR (376 MHz, DMSO-$d_6$) $\delta$ -108.84 (br dd, *J* = 40.4, 237.9 Hz, 1F), -110.65 (br d, *J* = 18.0 Hz, 1F), -118.13 (br dd, *J* = 53.9, 235.6 Hz, 1F), -139.89 (br d, *J* = 62.8 Hz, 1F), - 172.14 (br d, *J* = 35.9 Hz, 1F).

Compound 20-P2:

SFC separation retention time: 3.807 min.
MS m/z: 625.1 $[M+H]^+$
$^1H$ NMR (400 MHz, DMSO-$d_6$) $\delta$ 10.19 (br s, 1H), 9.06 (d, *J* = 7.9 Hz, 1H), 7.98 (dd, *J* = 5.9, 8.8 Hz, 1H), 7.47 (t, *J* = 9.0 Hz, 1H), 7.40 (d, *J* = 1.9 Hz, 1H), 7.22 (br d, *J* = 2.8 Hz, 1H), 5.29 (d, *J* = 53.6 Hz, 1H), 4.65-4.43 (m, 1H), 4.26 (br d, *J* = 13.5 Hz, 1H), 4.15 (dd, *J* = 7.1, 10.3 Hz, 1H), 4.09-4.01 (m, 1H), 4.00-3.84 (m, 2H), 3.70 (br t, *J* = 10.7 Hz, 1H), 3.16-3.05 (m, 2H), 3.03 (br s, 1H), 2.89-2.79 (m, 1H), 2.17-1.71 (m, 10H).
$^{19}F$ NMR (376 MHz, DMSO-$d_6$) $\delta$ -108.81 (br dd, *J* = 42.6, 237.9 Hz, 1F), -110.66 (br d, *J* = 26.9 Hz, 1F), -118.11

(br dd, *J* = 50.5, 236.7 Hz, 1F), -139.89 (br d, *J* = 40.4 Hz, 1F), -172.16 (br d, *J* = 31.4 Hz, 1F).

HPLC separation conditions:

column: YMC-Triart Prep C18 150 * 40 mm * 7 μm;
mobile phase: [Water (ammonia hydroxide v/v) - ACN]; B%: 24% to 64%, 9 min.

SFC chiral separation conditions:

column: ChiralPakAD-3 150 × 4.6 mm I.D., 3 μm
mobile phase: A: $CO_2$ B: IPA (0.05% DEA) Isocratic: 40% B.

Example 21: compound 21

4-(4-((1R,SS)-3,8-Diazabicyclo[3.2.1]octan-3-yl)-5-chloro-8-fluoro-2-(((2R,7aS)-**2-fluorohexahydro-1H-pyrrolizin-7a-yl)methoxy)pyrido[4,3-d]pyrimidin-7-yl)-5-ethynyl-6-fluoronaphthalen-2-ol**

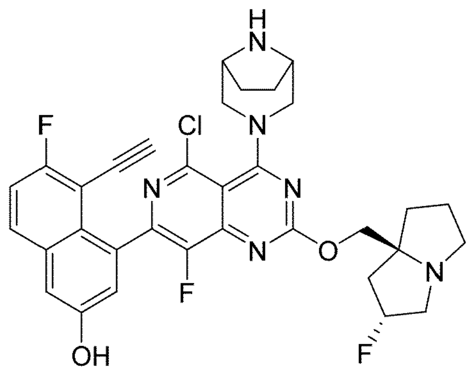

**Step 1: 2,4,5,7-tetrachloro-8-fluoropyrido[4,3-d]pyrimidine**

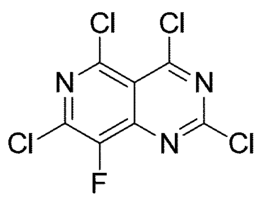

5,7-Dichloro-8-fluoropyrido[4,3-d]pyrimidin-2,4-diol (12 g, 48.00 mmol, 1 *eq)* was dissolved in phosphorus oxychloride (100 mL), and N,N-diisopropylethylamine (30.71 g, 237.59 mmol, 41.38 mL, 4.95 *eq)* was slowly added dropwise thereto. The system was replaced with nitrogen, and the reaction mixture was reacted at 100°C for 16 hours. LCMS monitored that the reaction was completed. The reaction mixture was concentrated under reduced pressure to obtain 2,4,5,7-tetrachloro-8-fluoropyrido[4,3-d]pyrimidine (30 g, crude product) as a dark brown oil, and the crude product was used directly in the next step.
MS m/z: 288.2/290.2 $[M+H]^+$

**Step 2: tert-butyl (1R,5S)-3-(2,5,7-trichloro-8-fluoropyrido[4,3-d]pyrimidin-4-yl)-3,8-diazabicyclo [3.2.1] octane-8-carboxylate**

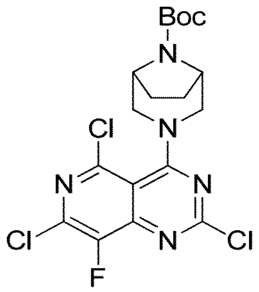

2,4,5,7-Tetrachloro-8-fluoropyrido[4,3-d]pyrimidine (13.3 g, 27.81 mmol, 1 eq) was dissolved in dichloromethane (100 mL), and N,N-diisopropylethylamine (10.78 g, 83.44 mmol, 14.53 mL, 3 eq) was added thereto. The system was replaced with nitrogen, and 8-BOC-3,8-diazabicyclo[3.2.1]octane (5.90 g, 27.81 mmol, 1 eq) was slowly added thereto at -30°C, and the reaction mixture was reacted for half an hour. LCMS monitored that the reaction was completed. The reaction mixture was slowly added to saturated ammonium chloride aqueous solution for quenching and extracted with dichloromethane. The organic phase was washed with saturated brine, dried over anhydrous sodium sulfate, filtered, and the filtrate was concentrated. The residue was purified by column chromatography (ethyl acetate/petroleum ether: 0 to 16%) to obtain *tert*-butyl (1R,5S)-3-(2,5,7-trichloro-8-fluoropyrido[4,3-d]pyrimidin-4-yl)-3,8-diazabicyclo[3.2.1]octane-8-carboxylate (8.6 g, two-step yield: 38.74%) as a white solid.
MS m/z: 462.1/464.1 $[M+H]^+$

**Step 3: *tert*-butyl (1R,SS)-3-(5,7-dichloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo [3.2.1]octane-8-carboxylate**

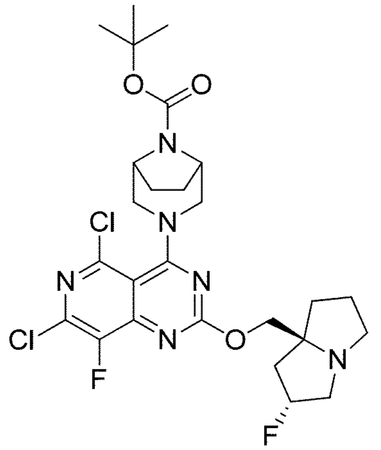

*tert*-Butyl (1R,5S)-3-(2,5,7-trichloro-8-fluoropyrido[4,3-d]pyrimidin-4-yl)-3,8-diazabicyclo[3.2.1]octane-8-carboxylate (8.6 g, 18.59 mmol, 1 eq) and ((2R,7aS)-2-fluorohexahydro-1H-pyrrolizin-7a-yl)methanol (2.96 g, 18.59 mmol, 1 eq) were dissolved in dichloromethane (100 mL), then sodium tert-butoxide (3.57 g, 37.17 mmol, 2 eq) was slowly added thereto, and the reaction mixture was reacted at room temperature for 1 hour under nitrogen atmosphere. LCMS monitored that the reaction was completed. The reaction mixture was slowly poured into saturated ammonium chloride aqueous solution and extracted with dichloromethane. The organic phase was washed with saturated brine, dried over anhydrous sodium sulfate, filtered, and the filtrate was concentrated. The residue was purified by column chromatography (ethyl acetate/petroleum ether: 30 to 60%) to obtain *tert*-butyl (1R,SS)-3-(5,7-dichloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (9 g, yield: 82.71%) as a yellow solid.
MS m/z: 585.1/587.1 $[M+H]^+$

$^1$H NMR (400 MHz, $CDCl_3$) δ 5.35 (br d, *J* = 53.2 Hz, 1H), 4.35-4.15 (m, 4H) 3.57-3.20 (m, 4H), 3.08 (br s, 1H), 2.47-1.95 (m, 5H), 1.89-1.59 (m, 8H), 1.53 (s, 9H).

$^{19}$F NMR (376 MHz, $CDCl_3$) δ -137.27 (s), -172.89 (s).

**Step 4: *tert*-butyl (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate**

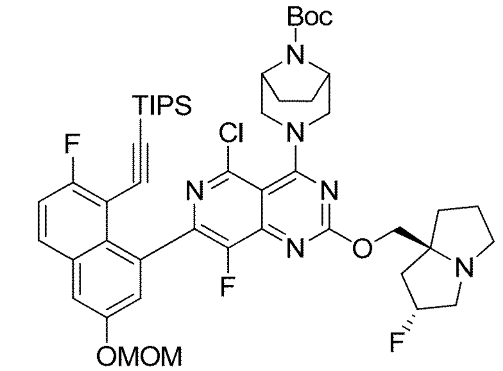

*tert-Butyl* (1-(7-chloro-8-fluoro-2-(((2R,7aS)-2-fluorohexahydro-1H-pyrrolizin-7a-yl)methoxy)pyrido[4,3-d]pyrimidin-4-yl)-3,3-difluoropiperidin-4-yl)carbamate (5.0 g, 8.54 mmol, 1 eq), ((2-fluoro-6-(methoxymethoxy)-8-(4,4,5,5-tetramethyl-1,3,2-dioxaborolan-2-yl)naphthalen-1-yl)ethynyl)triisopropylsilane (5.03 g, 9.82 mmol, 1.15 eq), potassium carbonate (3.54 g, 25.62 mmol, 3 eq), 2-dicyclohexylphosphino-2,6-diisopropoxy-1,1-biphenyl (797.02 mg, 1.71 mmol, 0.2 eq), and methanesulfonato(2-dicyclohexylphosphino-2,6-diisopropoxy-1,1-biphenyl)(2-amino-1,1-biphenyl-2-yl)palladium(II) (1.43 g, 1.71 mmol, 0.2 eq) were dissolved in a solvent mixture of toluene (50 mL), water (25 mL), and ethanol (25 mL). The system was replaced with nitrogen, and the reaction mixture was reacted at 80°C for 16 hours. LCMS monitored that the reaction was completed. The phases of the reaction mixture were separated. The organic phase was washed with saturated brine, dried over anhydrous sodium sulfate, filtered, and the filtrate was concentrated. The residue was purified by column chromatography (tetrahydrofuran/petroleum ether: 0 to 30%) to obtain *tert*-butyl (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (2.5 g, yield: 25%) as a yellow solid.
MS m/z: 935.4/937.4 $[M+H]^+$

**Step 5: 4-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol**

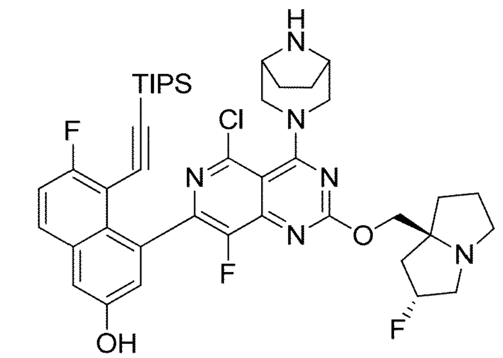

*tert-Butyl* (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (1.5 g, 1.36 mmol, 1 eq) was dissolved in acetonitrile (5 mL), then a solution of hydrochloric acid in dioxane (4 M, 5 mL, 20 mmol) was added thereto, and the reaction mixture was reacted at room temperature for 1 hour. LCMS monitored that the reaction was completed, and the reaction mixture was concentrated to obtain 4-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (1.2 g, crude product) as a yellow solid. The crude product was used directly in the next step.
MS m/z: 791.2/793.1 [M+H]$^+$

**Step 6: 4-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

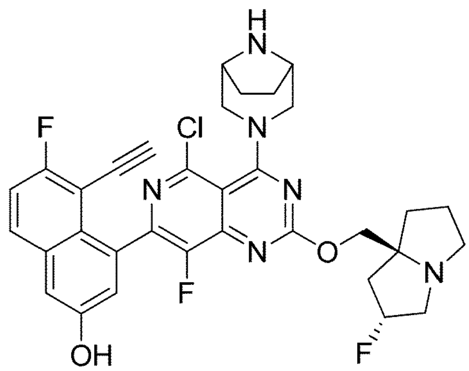

4-(4-((1R,5S)-3,8-Diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (1.2 g of crude product, 1.36 mmol) was dissolved in N,N-dimethylformamide (10 mL), then cesium fluoride (2.30 g, 15.16 mmol, 11.15 eq) was added thereto, and the reaction mixture was reacted at room temperature overnight. LCMS monitored that the reaction was completed. The reaction mixture was filtered, and the filtrate was separated by HPLC to obtain the product (350 mg, yield: 40.53%) as a yellow solid.
MS m/z: 635.2 [M+H]$^+$

$^{19}$F NMR (376 MHz, DMSO-$d_6$) $\delta$ -110.26 (s), -142.18 (s), -172.12 (s), 172.17 (s).

$^1$H NMR (400 MHz, DMSO-$d_6$) $\delta$ 10.22 (br s, 1H), 8.00 (dd, *J* = 9.23, 5.93 Hz, 1H), 7.49 (t, *J* = 9.05 Hz, 1H), 7.42 (d, *J* = 2.45 Hz, 1H), 7.26 (br s, 1H), 5.28 (br d, *J* = 54 Hz, 1H), 4.18-4.11 (m, 1H), 4.03 (dd, *J* = 10.33, 3.73 Hz, 1H), 3.87 (br s, 1H), 3.55-3.40 (m, 4H), 3.15-2.96 (m, 4H), 2.88-2.80 (m, 1H), 2.22-1.92 (m, 4H), 1.91-1.70 (m, 4H), 1.56 (br s, 3H).

$^{13}$C NMR (101 MHz, DMSO-$d_6$) $\delta$ 163.95, 163.53, 161.48, 154.63, 151.57, 151.23, 148.68, 143.80, 143.64, 140.35, 132.98, 132.72, 131.25, 131.17, 125.27, 123.69, 116.69, 116.43, 112.45, 108.66, 104.05, 103.91, 99.13, 97.40, 91.49, 75.75, 73.89, 72.35, 60.37, 60.16, 56.82, 55.24, 42.88, 42.68, 36.04, 30.88, 27.47, 27.32, 25.55.

HPLC separation conditions:

column: Boston Prime C18 150 * 30 mm * 5 $\mu$m
mobile phase: [Water ($NH_3H_2O$ + $NH_4HCO_3$) - ACN]; B%: 38% to 68%, 10 min.

**Example 22: compound 22**

4**-(4-((1R,5S)-3,8-Diazabicyclo[3.2.1]octan-3-yl)-5,8-difluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

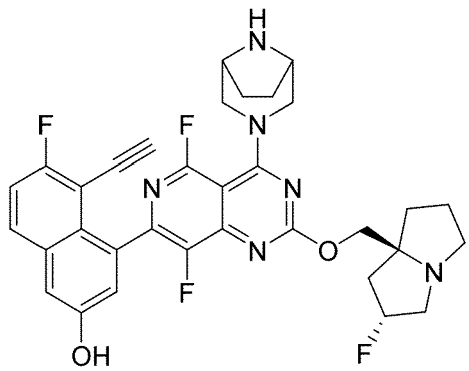

**Step 1: *tert*-butyl (1R,5S)-3-(7-(8-ethynyl-7-fluoro-3-(methoxymethoxy)naphthalen-1-yl)-5,8-difluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate**

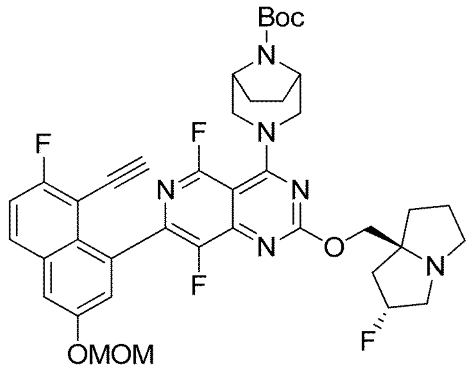

*tert-Butyl* (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (3 g, 2.73 mmol, 1 eq) was dissolved in dimethyl sulfoxide (10 mL), then cesium fluoride (14.61 g, 96.30 mmol, 35 eq) was added thereto, and the reaction mixture was reacted at 80°C for 12 hours. The reaction was monitored by LCMS. After the reaction was completed, the reaction mixture was filtered, and the filtrate was purified by HPLC to obtain the product (600 mg, yield: 28.8%) as a yellow solid.
MS m/z: 763.2 $[M+H]^+$

$^1$H NMR (400 MHz, DMSO-$d_6$) δ 8.12 (dd, *J* = 9.11, 5.93 Hz, 1H), 7.77 (d, *J* = 2.45 Hz, 1H), 7.58 (t, *J* = 8.99 Hz, 1H), 7.44 (s, 1H), 5.46-5.19 (m, 3H), 4.29 (br d, *J* = 12.10 Hz, 2H), 4.22-4.10 (m, 4H), 3.45 (s, 3H), 3.19-2.99 (m, 2H), 2.85 (br d, *J* = 5.87 Hz, 1H), 2.18-1.97 (m, 6H), 1.93-1.66 (m, 8H), 1.46 (s, 9H).

$^{19}$F NMR (376 MHz, DMSO-$d_6$) δ -53.91 (s), -53.99 (s), -108.60 (s), -142.15 (s), - 172.14 (s), -172.20 (s).

HPLC separation conditions:

column: YMC-Triart Prep C18 150 * 40 mm * 7 μm
mobile phase: [Water (ammonia hydroxide v/v) - ACN]; B%: 58% to 98%, 9 min.

**Step 2: 4-(4-((1R,SS)-3,8-diazabicyclo[3.2.1]octan-3-yl)-5,8-difluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

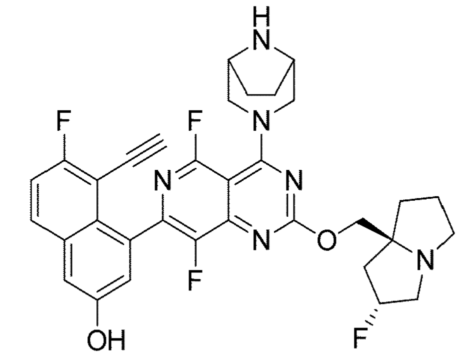

*tert-Butyl* (1R,5S)-3-(7-(8-ethynyl-7-fluoro-3-(methoxymethoxy)naphthalen-1-yl)-5,8-diffuoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (600 mg, 0.786 mmol, 1 eq) was dissolved in acetonitrile (5 mL), then a solution of hydrochloric acid in dioxane (4 M, 5 mL) was added thereto, and the reaction mixture was reacted at room temperature for 1 hour. LCMS detected that the reaction was completed. The reaction mixture was concentrated and purified by HPLC to obtain the product (280 mg, yield: 57.54%) as a light yellow solid.
MS m/z: 619.2 $[M+H]^+$

$^1$H NMR (400 MHz, DMSO-$d_6$) δ 10.23 (br s, 1H), 7.99 (dd, *J* = 9.16, 5.90 Hz, 1H), 7.49 (t, *J* = 9.03 Hz, 1H), 7.41 (d, *J* = 2.51 Hz, 1H), 7.23 (s, 1H), 5.29 (br d, *J* = 55.2 Hz, 1H), 4.18-4.08 (m, 2H), 4.07-3.93 (m, 3H), 3.58-3.40 (m, 5H), 3.09 (br d, *J* = 5.02 Hz, 2H), 3.02 (s, 1H), 2.88-2.79 (m, 1H), 2.17-1.93 (m, 3H), 1.91-1.71 (m, 3H), 1.68-1.47 (m, 4H).

$^{19}$F NMR (376 MHz, DMSO-$d_6$) δ -53.80 (s), -53.83 (s), -53.88 (s), -53.90 (s), -110.34 (s), -142.73 (s), -142.76 (s), -142.81 (s), -142.84 (s), -172.12 (s), -172.18 (s).

$^{13}$C NMR (101 MHz, DMSO-$d_6$) δ 164.46, 163.86, 163.51, 163.45, 161.39, 154.60, 152.95, 152.81, 152.47, 150.40, 150.34, 150.08, 147.90, 147.84, 141.67, 132.96, 132.66, 131.19, 131.10, 125.25, 123.46, 116.70, 116.44, 112.42, 104.07, 103.91, 99.22, 99.18, 98.49, 98.15, 97.48, 97.45, 91.68, 91.62, 75.53, 73.81, 72.31, 60.29, 60.13, 56.83, 55.56, 54.59, 54.54, 42.88, 42.68, 36.03, 27.71, 27.45, 25.55.

HPLC separation conditions:

column: Boston Prime C18 150 * 30 mm * 5 μm
mobile phase: [Water ($NH_3H_2O$ + $NH_4HCO_3$) - ACN]; B%: 36% to 66%, 10 min.

**Example 23: compound 23**

**4-(4-((1R,SS)-8-Azabicyclo[3.2.1]oct-3-en-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

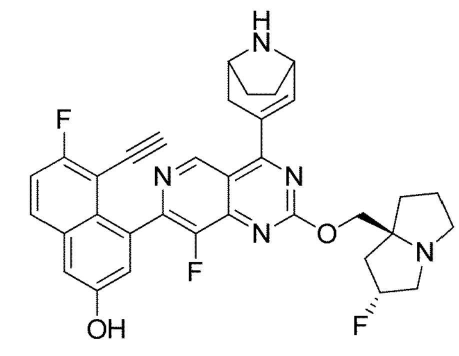

**Step 1: 4-amino-6-chloro-5-fluoronicotinic acid**

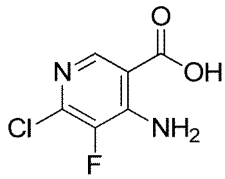

Ethyl 4-amino-6-chloro-5-fluoronicotinate (10 g, 45.74 mmol, 1 *eq)* was dissolved in tetrahydrofuran (90 mL). Sodium hydroxide (7.32 g, 182.97 mmol, 4 *eq)* was dissolved in water (45 mL) and then slowly added dropwise to a solution of ethyl 4-amino-6-chloro-5-fluoronicotinate in tetrahydrofuran, and the reaction mixture was reacted for 16 hours at room temperature. LCMS monitored that the reaction was completed, and the reaction mixture was concentrated to remove tetrahydrofuran. The pH of the concentrate was adjusted to 2 with 2M dilute hydrochloric acid aqueous solution, and the resulting mixture was filtered. The filter cake was washed twice with water and dried under reduced pressure to obtain 4-amino-6-chloro-5-fluoronicotinic acid (8.4 g, yield: 96.37%) as a white solid, which was used directly in the next step.

MS m/z: 191.0/193.0 $[M+H]^+$
$^1$H NMR (400 MHz, DMSO-$d_6$) δ 8.36 (s, 1H), 7.61 (br s, 2H).

**Step 2: 7-chloro-8-fluoro-2-thio-2,3-dihydropyrido[4,3-d]pyrimidin-4(1H)-one**

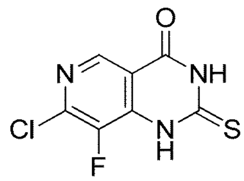

4-Amino-6-chloro-5-fluoronicotinic acid (9.1 g, 47.75 mmol, 1 *eq)* was dissolved in phosphorus oxychloride (90 mL), and the reaction mixture was reacted at 90°C for 2 hours, then cooled to room temperature. The reaction mixture was distilled under reduced pressure to remove phosphorus oxychloride, and the residue was dissolved in tetrahydrofuran (60 mL). A solution of ammonium thiocyanate (7.27 g, 95.51 mmol, 7.27 mL, 2 *eq)* in tetrahydrofuran (120 mL) was added dropwise thereto, and the reaction mixture was reacted at room temperature for 16 hours. LCMS monitored that the reaction was completed, and the reaction mixture was quenched by adding water and extracted with ethyl acetate. The organic phase was washed with saturated brine, dried over anhydrous sodium sulfate, filtered, and the filtrate was concentrated. The residue was slurried with ethyl acetate to obtain 7-chloro-8-fluoro-2-thio-2,3-dihydropyrido[4,3-d]pyrimidin-4(1H)-one (7.5 g, yield: 67.80%) as a yellow solid.

MS m/z: 231.9/233.9 $[M+H]^+$
$^1$H NMR (400 MHz, DMSO-$d_6$) δ 13.31 (br s, 1H), 12.89 (s, 1H), 8.64 (s, 1H).

**Step 3: 7-chloro-8-fluoro-2-(methylthio)pyrido[4,3-d]pyrimidin-4-ol**

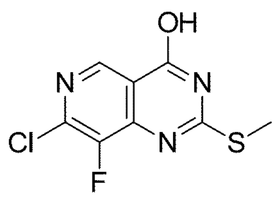

7-Chloro-8-fluoro-2-thio-2,3-dihydropyrido[4,3-d]pyrimidin-4(1H)-one (8 g, 34.54 mmol, 1 eq) was dissolved in N,N-dimethylformamide (80 mL), then sodium methoxide solution (5.4 M, 6.40 mL, 1 *eq)* was added dropwise thereto at room temperature, and the reaction mixture was stirred for 10 minutes. Iodomethane (4.90 g, 34.54 mmol, 2.15 mL,

1 *eq)* was dissolved in N,N-dimethylformamide (10 mL), then added dropwise to the reaction mixture, and the reaction was carried out at room temperature for 2 hours. LCMS monitored that the reaction was completed. The reaction mixture was quenched by adding ice water and filtered, and the filter cake was washed twice with ice water and dried under reduced pressure to obtain 7-chloro-8-fluoro-2-(methylthio)pyrido[4,3-d]pyrimidin-4-ol (7.4 g, yield: 87.22%) as a yellow solid, which was used directly in the next step.

MS m/z: 246.1/248.1 $[M+H]^+$
$^1$H NMR (400 MHz, DMSO-$d_6$) δ 13.24 (br s, 1H), 8.79 (s, 1H), 2.61 (s, 3H).

**Step 4: 4,7-dichloro-8-fluoro-2-(methylthio)pyrido[4,3-d]pyrimidine**

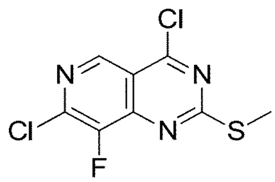

7-Chloro-8-fluoro-2-(methylthio)pyrido[4,3-d]pyrimidin-4-ol (5 g, 20.35 mmol, 1 eq) was dissolved in phosphorus oxychloride (50 mL), then N,N-diisopropylethylamine (5.26 g, 40.71 mmol, 7.09 mL, 2 *eq)* was slowly added dropwise thereto, and the reaction mixture was reacted at 90°C for 2 hours. LCMS monitored that the reaction was completed. The reaction mixture was concentrated, and the residue was dissolved in ethyl acetate, quenched by pouring into ice water, and extracted with ethyl acetate. The organic phase was washed with saturated brine, dried over anhydrous sodium sulfate, filtered, and the filtrate was concentrated to obtain 4,7-dichloro-8-fluoro-2-(methylthio)pyrido[4,3-d]pyrimidine (5.5 g, crude product) as a brown solid, which was used directly in the next step.

MS m/z: 264.0/266.0 $[M+H]^+$
$^1$H NMR (400 MHz, DMSO-$d_6$) δ 8.78 (s, 1H), 2.60 (s, 3H).

**Step 5: *tert-butyl* (1R,5S)-3-(7-chloro-8-fluoro-2-(methylthio)pyrido[4,3-d]pyrimidin-4-yl)-8-azabicyclo[3.2.1]oct-3-ene-8-carboxylate**

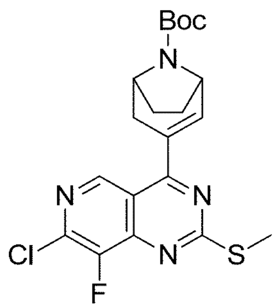

4,7-Dichloro-8-fluoro-2-(methylthio)pyrido[4,3-d]pyrimidine (1.0 g, 3.79 mmol, 1.0 *eq)* was dissolved in dioxane (10 mL) and water (5 mL), then tert-butyl 3-(4,4,5,5-tetramethyl-1,3,2-dioxaborolan-2-yl)-8-azabicyclo[3.2.1]oct-3-ene-8-carboxylate (1.14 g, 3.41 mmol, 0.9 eq), [1,1'-bis(diphenylphosphino)ferrocene]dichloropalladium(II) (277.05 mg, 0.379 mmol, 0.1 *eq),* and potassium carbonate (1.05 g, 7.57 mmol, 2 *eq)* were added thereto, and the reaction mixture was reacted at 60°C for 30 minutes under nitrogen atmosphere. LCMS monitored that the reaction was completed, and the reaction mixture was quenched by adding water and extracted with ethyl acetate. The organic phase was washed with saturated brine, dried over anhydrous sodium sulfate, filtered, and the filtrate was concentrated. The residue was purified by column chromatography (tetrahydrofuran/petroleum ether: 0 to 10%) to obtain *tert*-butyl (1R,5S)-3-(7-chloro-8-fluoro-2-(methylthio)pyrido[4,3-d]pyrimidin-4-yl)-8-azabicyclo[3.2.1]oct-3-ene-8-carboxylate (0.92 g, yield: 55.9%) as a pale yellow solid.

MS m/z: 437.1/439.1 [M+H]+

$^1$H NMR (400 MHz, DMSO-$d_6$) δ 8.98 (s, 1H), 6.83 (br d, *J* = 5.1 Hz, 1H), 4.53 (br t, *J* = 5.5 Hz, 1H), 4.37 (br s, 1H), 3.08 (br d, *J* = 16.4 Hz, 1H), 2.64 (s, 3H), 2.45 (br d, *J* = 17.6 Hz, 1H), 2.31-2.14 (m, 1H), 2.14-1.94 (m, 2H), 1.80 (br s, 1H), 1.41 (s, 9H).

**Step 6: tert-butyl (1R,5S)-3-(8-fluoro-7-(7-fluoro-3-(methoxymethoxy)-8-((triisopropylsilyl)ethynyl)naphthalen-1-yl)-2-(methylthio)pyrido[4,3-d]pyrimidin-4-yl)-8-azabicyclo [3.2.1] oct-3-ene-8-carboxylate**

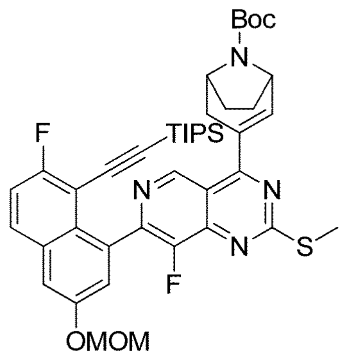

*tert-Butyl* (1R,5S)-3-(7-chloro-8-fluoro-2-(methylthio)pyrido[4,3-d]pyrimidin-4-yl)-8-azabicyclo[3.2.1]oct-3-ene-8-carboxylate (900 mg, 2.06 mmol, 1 *eq)* was dissolved in dioxane (8 mL) and water (4 mL), then ((2-fluoro-6-(methoxymethoxy)-8-(4,4,5,5-tetramethyl-1,3,2-dioxaborolan-2-yl)naphthalen-1-yl)ethynyl)triisopropylsilane (1.58 g, 3.09 mmol, 1.5 *eq),* methanesulfonato(diadamantyl-*n*-butylphosphino)-2'-amino-1,1'-biphenyl-2-yl)palladium(II) (150.01 mg, 0.206 mmol, 0.1 *eq),* and cesium carbonate (1.34 g, 4.12 mmol, 2 *eq)* were added thereto, and the reaction mixture was reacted at 100°C for 2 hours under nitrogen atmosphere. LCMS monitored that the reaction was completed, and the reaction mixture was quenched by adding water and extracted with ethyl acetate. The organic phase was washed with saturated brine, dried over anhydrous sodium sulfate, filtered, and the filtrate was concentrated. The residue was purified by column chromatography (tetrahydrofuran/petroleum ether: 0 to 15%) to obtain tert-butyl (1R,5S)-3-(8-fluoro-7-(7-fluoro-3-(methoxymethoxy)-8-((triisopropylsilyl)ethynyl)naphthalen-1-yl)-2-(methylthio)pyrido[4,3-d]pyrimidin-4-yl)-8-azabicyclo[3.2.1]oct-3-ene-8-carboxylate (1.5 g, yield: 90.5%) as a pale yellow solid.

MS m/z: 787.4 [M+H]+

$^1$H NMR (400 MHz, DMSO-$d_6$) δ 9.30-9.16 (m, 1H), 8.13 (dd, *J* = 5.9, 9.2 Hz, 1H), 7.78 (d, *J* = 2.3 Hz, 1H), 7.58 (t, *J* = 8.9 Hz, 1H), 7.41-7.35 (m, 1H), 6.97-6.85 (m, 1H), 5.41-5.32 (m, 2H), 4.64-4.37 (m, 2H), 3.94 (s, 1H), 3.43 (s, 3H), 2.62 (d, *J* = 2.5 Hz, 3H), 2.31-2.13 (m, 2H), 2.12-1.93 (m, 2H), 1.85-1.69 (m, 1H), 1.43 (s, 9H), 0.76 (ddd, *J* = 5.1, 7.4, 9.5 Hz, 18H), 0.47-0.27 (m, 3H).

**Step 7: *tert-butyl* (1R,5S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]oct-3-ene-8-carboxylate**

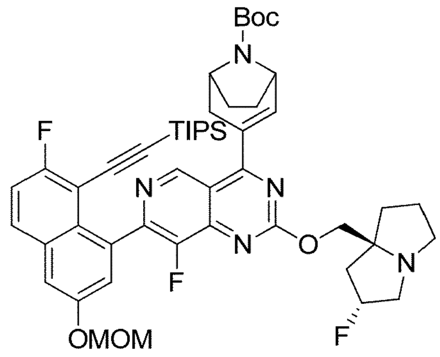

Sodium hydride (76.23 mg, 1.91 mmol, 60% content, 3 *eq)* was dissolved in tetrahydrofuran (10 mL), then the system was replaced with nitrogen, and ((2R,7aS)-2-fluorohexahydro-1H-pyrrolizin-7a-yl)methanol (121.37 mg, 0.7624 mmol, 1.2 *eq)* was added thereto at 0°C. After the reaction was carried out at 0°C for 0.5 hours, tert-butyl (1R,5S)-3-(8-fluoro-7-(7-fluoro-3-(methoxymethoxy)-8-((triisopropylsilyl)ethynyl)naphthalen-1-yl)-2-(methylthio)pyrido[4,3-d]pyrimidin-4-yl)-8-azabicyclo[3.2.1]oct-3-ene-8-carboxylate (500 mg, 0.6353 mmol, 1 *eq)* was added thereto, and the reaction was carried out at 50°C for 2 hours. LCMS monitored that the reaction was completed. The reaction mixture was quenched by adding saturated ammonium chloride at 0°C, extracted with ethyl acetate, washed with saturated brine, dried over anhydrous sodium sulfate, filtered, and concentrated. The residue was purified by column chromatography (methanol/dichloromethane: 0 to 2%) to obtain *tert*-butyl (1R,5S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]oct-3-ene-8-carboxylate (413 mg, yield: 72.38%) as a yellow solid.
MS m/z: 898.5 $[M+H]^+$

**Step 8: *tert-butyl* (1R,5S)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido [4,3-d] pyrimidin-4-yl)-8-azabicyclo [3.2.1]oct-3-ene-8-carboxylate**

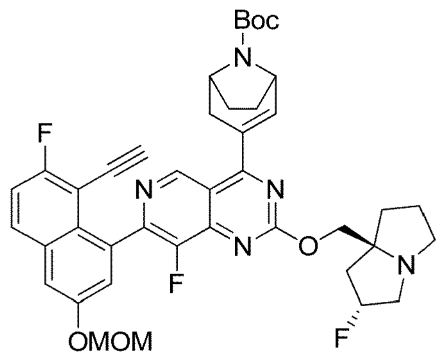

*tert-Butyl* (1R,5S)-3-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]oct-3-ene-8-carboxylate (130 mg, 0.145 mmol, 1 *eq)* was dissolved in N,N-dimethylformamide (2 mL), then cesium fluoride (109.93 mg, 0.724 mmol, 5 *eq)* was added thereto, and the reaction mixture was reacted at room temperature for 1 hour. LCMS monitored that the reaction was completed. The reaction mixture was poured into water, extracted with ethyl acetate, washed with saturated brine, dried over anhydrous sodium sulfate, filtered, and concentrated to obtain tert-butyl (1R,5S)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-8-azabicyclo[3.2.1]oct-3-ene-8-carboxylate (107 mg, crude product) as an orange solid, which was used directly in the next step.
MS m/z: 742.3 $[M+H]^+$

**Step 9: 4-(4-((1R,SS)-8-azabicyclo[3.2.1]oct-3-en-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

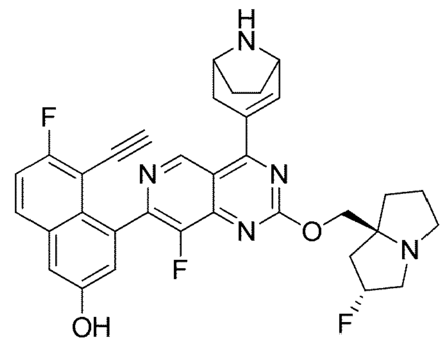

*tert*-Butyl (1R,5S)-3-(7-(8-ethynyl-7-fluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-8-azabicyclo[3.2.1]oct-3-ene-8-carboxylate (107 mg, 0.1442 mmol, 1 eq) was dissolved in a mixture of dichloromethane (2 mL) and trifluoroacetic acid (0.4 mL), and the reaction mixture was reacted at room temperature for 1 hour. LCMS monitored that the reaction was completed.

The reaction mixture was concentrated, and the crude product was prepared by HPLC to obtain 4-(4-((1R,5S)-8-azabicyclo[3.2.1]oct-3-en-3-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (30 mg, yield: 33.51%) as an orange solid.
MS m/z: 598.3 $[M+H]^+$

$^{19}$F NMR (376 MHz, DMSO-$d_6$) $\delta$ -110.63 (s), -110.69 (s), -140.53 (s), -140.57 (s), - 140.70 (s), 140.74 (s), -172.07(s), -172.20 (s).

$^1$H NMR (400 MHz, DMSO-$d_6$) $\delta$ 10.26 (s, 1H), 9.27 (d, *J* = 4.3 Hz, 1H), 8.00 (dd, *J* = 5.9, 9.2 Hz, 1H), 7.48 (t, *J* = 9.0 Hz, 1H), 7.42 (d, *J* = 2.4 Hz, 1H), 7.22 (dd, *J* = 2.3, 7.8 Hz, 1H), 6.77 (br t, *J* = 6.5 Hz, 1H), 5.30 (br d, *J* = 54.0 Hz, 1H), 4.25-4.10 (m, 2H), 3.93-3.76 (m, 3H), 3.20-2.93 (m, 5H), 2.90-2.79 (m, 2H), 2.13-1.96 (m, 5H), 1.93-1.62 (m, 5H).

HPLC separation conditions:

column: Phenomenex C18 75 * 30 mm * 3 μm
mobile phase: [Water (ammonia hydroxide v/v) - ACN]; B%: 26% to 66%, 9 min

**Example 24: compound 24**

**4-4-(4-((1R,2S,3R,5S)-3-Amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

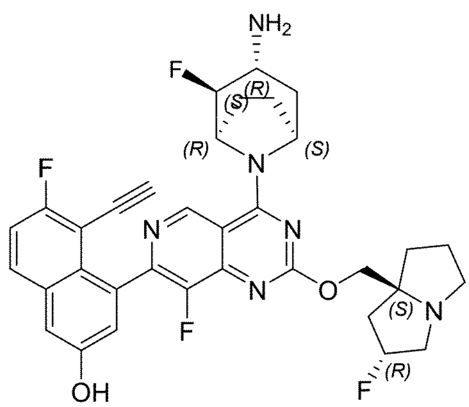

**Step 1: *tert*-butyl ((1R,2S,3R,5S)-8-(2,7-dichloro-8-fluoropyrido[4,3-d]pyrimidin-4-yl)-2-fluoro-8-azabicyclo[3.2.1]octan-3-yl)carbamate**

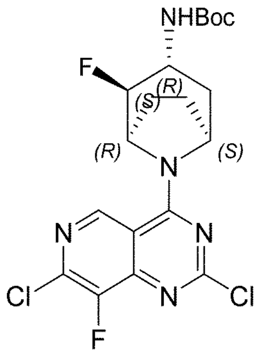

2,4,7-Trichloro-8-fluoropyrido[4,3-d]pyrimidine (500 mg, 1.98 mmol, 1 *eq)* was dissolved in dichloromethane (5 mL), then N,N-diisopropylethylamine (767.90 mg, 5.94 mmol, *3 eq)* was added thereto, and the system was replaced with nitrogen and then cooled to -40°C. A solution of *tert*-butyl ((1R,2R,3R,5S)-2-fluoro-8-azabicyclo[3.2.1]octan-3-yl)carbamate (435.46 mg, 1.78 mmol, 0.9 *eq)* in dichloromethane (2 mL) was added thereto, and the reaction mixture was reacted at -40°C for 30 minutes under nitrogen atmosphere. LCMS detected that the reaction was completed. The reaction mixture was added to saturated citric acid monohydrate aqueous solution at 0°C, and extracted with dichloromethane. The organic phase was washed with saturated brine, dried over anhydrous sodium sulfate, filtered, and the filtrate was concentrated. The residue was purified by column chromatography (ethyl acetate/petroleum ether: 10 to 30%) to obtain tert-butyl ((1R,2S,3R,5S)-8-(2,7-dichloro-8-fluoropyrido[4,3-d]pyrimidin-4-yl)-2-fluoro-8-azabicyclo[3.2.1]octan-3-yl)carbamate (800 mg, yield: 87.75%) as a yellow solid.
MS m/z: 460.0/462.0 $[M+H]^+$

**Step 2: *tert-butyl* ((1R,2S,3R,SS)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate**

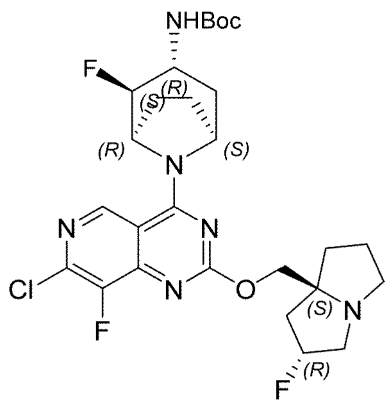

((2R,7aS)-2-Fluorohexahydro-1H-pyrrolizin-7a-yl)methanol (415.03 mg, 2.61 mmol, 1.5 *eq)* was dissolved in dichloromethane (8 mL), and sodium tert-butoxide (501.08 mg, 5.21 mmol, 3 *eq)* was added thereto. The system was replaced with nitrogen, and the reaction mixture was stirred for 10 minutes under nitrogen atmosphere. The reaction mixture was cooled to 0°C, and a solution of *tert*-butyl ((1R,2S,3R,5S)-8-(2,7-dichloro-8-fluoropyrido[4,3-d]pyrimidin-4-yl)-2-fluoro-8-azabicyclo[3.2.1]octan-3-yl)carbamate (800 mg, 1.74 mmol, 1 eq) in dichloromethane (8 mL) was slowly added dropwise thereto under nitrogen atmosphere. The reaction mixture was reacted at room temperature for 1 hour. LCMS detected that the reaction was completed. The reaction mixture was slowly added to saturated ammonium chloride aqueous solution for quenching at room temperature, and extracted with dichloromethane. The organic phase was washed with saturated brine, dried over anhydrous sodium sulfate, filtered, and the filtrate was concentrated. The residue

was purified by column chromatography [tetrahydrofuran (0.5% ammonia hydroxide)/petroleum ether: 15 to 50%] to obtain *tert-butyl* ((1R,2S,3R,5S)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate (630 mg, yield: 62.17%) as a yellow solid.
MS m/z: 583.1/585.1 $[M+H]^+$

**Step 3: *tert-butyl* ((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate**

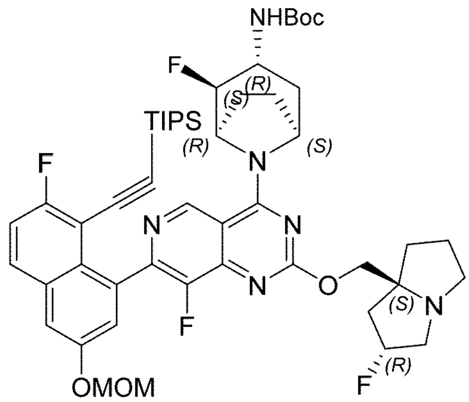

*tert*-Butyl ((1R,2S,3R,5S)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate (630 mg, 1.08 mmol, 1 *eq)* was dissolved in a solvent mixture of dioxane (9 mL) and water (4.5 mL), then ((2-fluoro-6-(methoxymethoxy)-8-(4,4,5,5-tetramethyl-1,3,2-dioxaborolan-2-yl)naphthalen-1-yl)ethynyl)triisopropylsilane (664.57 mg, 1.30 mmol, 1.2 *eq),* cesium carbonate (704.12 mg, 2.16 mmol, 2 *eq),* and methanesulfonato(diadamantyl-n-butylphosphino)-2'-amino-1,1'-biphenyl-2-yl)palladium(II) (157.38 mg, 0.216 mmol, 0.2 *eq)* were added thereto, and the system was replaced with nitrogen. The reaction mixture was reacted at 80°C for 2 hours under nitrogen atmosphere. LCMS monitored that the reaction was completed. The reaction mixture was extracted with ethyl acetate, and the organic phase was washed with saturated brine, dried over anhydrous sodium sulfate, filtered, and the filtrate was concentrated. The residue was purified by column chromatography [tetrahydrofuran (0.5% ammonia hydroxide)/petroleum ether: 15 to 60%] to obtain tert-butyl ((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate (823 mg, yield: 81.62%) as a yellow solid.
MS m/z: 933.5 $[M+H]^+$

**Step 4: 4-(4-((1R,2S,3R,SS)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-dipyrimidin-7-yl)-6-fluoro-5-((triisopropylsilyl)ethynyl)naphthalen-2-ol**

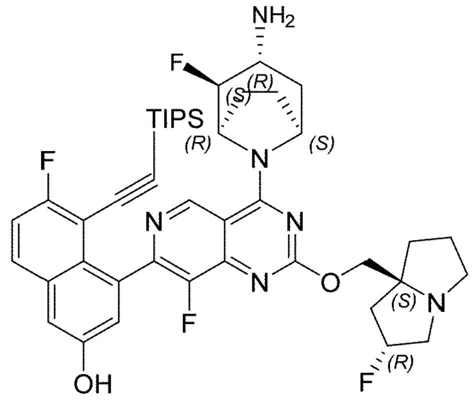

*tert*-Butyl ((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate (820 mg, 0.879 mmol, 1 *eq)* was dissolved in acetonitrile (10 mL), then a solution of hydrochloric acid in dioxane (4 M, 5 mL) was added thereto. The reaction mixture was reacted at room temperature for 1 hour. LCMS monitored that the reaction was completed. The reaction mixture was concentrated to obtain 4-(4-((1R,2S,3R,5S)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (1.1 g, crude product) as a yellow solid, and the crude product was used directly in the next step.
MS m/z: 789.3 $[M+H]^+$

**Step 5: 4-4-(4-((1R,2S,3R,SS)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

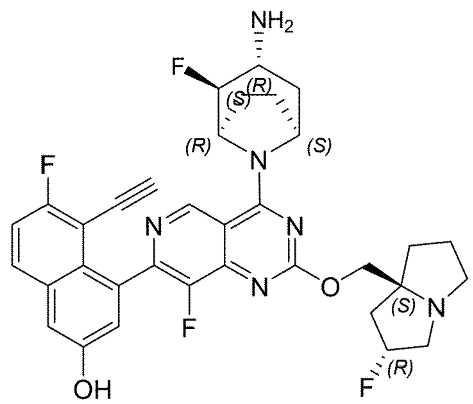

4-(4-((1R,2S,3R,5S)-3-Amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (693 mg, 0.878 mmol, 1 *eq)* was dissolved in N,N-dimethylformamide (5 mL), then cesium fluoride (1.33 g, 8.78 mmol, 10 *eq)* was added thereto, and the reaction mixture was reacted at room temperature overnight. LCMS monitored that the reaction was completed. The reaction mixture was filtered, and the filtrate was separated by HPLC to obtain the product (150 mg, yield: 26.99%) as a yellow solid.
MS m/z: 633.2 $[M+H]^+$

$^{19}F$ NMR (376 MHz, $CDCl_3$) δ -110.03 (s), -137.54 (s), -169.41 (s), -172.72 (s).

$^1H$ NMR (400 MHz, $CDCl_3$) δ 9.25-8.97 (m, 1H), 7.62-7.50 (m, 1H), 7.21-7.02 (m, 3H), 5.37-4.90 (m, 3H), 4.56-4.28 (m, 1H), 4.27-4.15 (m, 2H), 3.47-3.10 (m, 4H), 3.05-2.92 (m, 1H), 2.87-2.77 (m, 1H), 2.74-2.62 (m, 1H), 2.60-2.44 (m, 1H), 2.38-2.13 (m, 4H), 2.13-1.82 (m, 7H), 1.53-1.37 (m, 1H).

HPLC separation conditions

column: Phenomenex C18 80 * 30 mm * 5 μm;
mobile phase: [Water (ammonia hydroxide v/v) - ACN]; B%: 40% to 60%, 10 min.

**Example 25: compound 25**

**4-(4-((1S,2R,3S,SR)-3-Amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

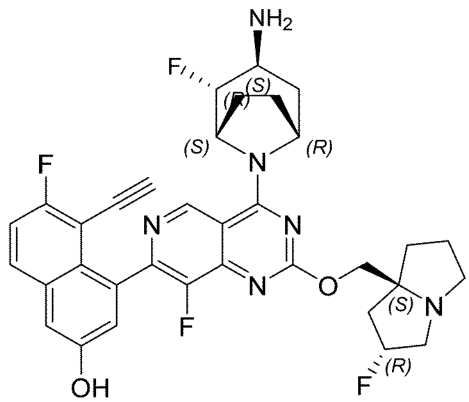

**Step 1: tert-butyl ((1S,2R,3S,SR)-8-(2,7-dichloro-8-fluoropyrido[4,3-d]pyrimidin-4-yl)-2-fluoro-8-azabicyclo[3.2.1]octan-3-yl)carbamate**

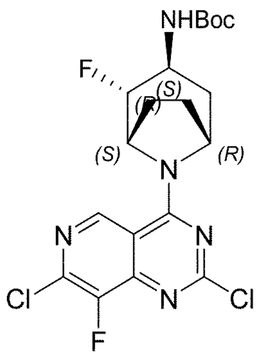

2,4,7-Trichloro-8-fluoropyrido[4,3-d]pyrimidine (500 mg, 1.98 mmol, 1 *eq)* was dissolved in dichloromethane (5 mL), then N,N-diisopropylethylamine (767.90 mg, 5.94 mmol, *3 eq)* was added thereto, and the system was replaced with nitrogen and then cooled to -40°C. A solution of tert-butyl ((1S,2S,3S,5R)-2-fluoro-8-azabicyclo[3.2.1]octan-3-yl)carbamate (435.46 mg, 1.78 mmol, 0.9 eq) in dichloromethane (2 mL) was added thereto, and the reaction mixture was reacted at -40°C for 30 minutes under nitrogen atmosphere. LCMS detected that the reaction was completed. The reaction mixture was added to saturated citric acid monohydrate aqueous solution at 0°C, and extracted with dichloromethane. The organic phase was washed with saturated brine, dried over anhydrous sodium sulfate, filtered, and the filtrate was concentrated. The residue was purified by column chromatography [ethyl acetate/petroleum ether: 10 to 30%] to obtain tert-butyl ((1S,2R,3S,5R)-8-(2,7-dichloro-8-fluoropyrido[4,3-d]pyrimidin-4-yl)-2-fluoro-8-azabicyclo[3.2.1]octan-3-yl)carbamate (728 mg, yield: 79.88%) as a yellow solid.
MS m/z: 460.0/462.0 $[M+H]^+$

**Step 2: *tert*-butyl ((1S,2R,3S,SR)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate**

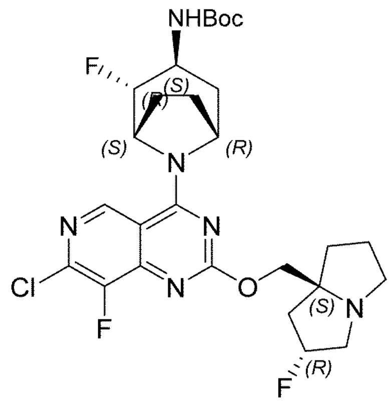

((2R,7aS)-2-Fluorohexahydro-1H-pyrrolizin-7a-yl)methanol (373.53 mg, 2.35 mmol, 1.5 *eq)* was dissolved in dichloromethane (8 mL), and sodium tert-butoxide (450.97 mg, 4.69 mmol, 3 *eq)* was added thereto. The system was replaced with nitrogen, and the reaction mixture was stirred for 10 minutes. The reaction mixture was cooled to 0°C, and a solution of tert-butyl ((1 S,2R,3S,SR)-8-(2,7-dichloro-8-fluoropyrido[4,3-d]pyrimidin-4-yl)-2-fluoro-8-azabicyclo[3.2.1]octan-3-yl)carbamate (720 mg, 1.56 mmol, 1 *eq)* in dichloromethane (8 mL) was slowly added dropwise thereto under nitrogen atmosphere. The reaction mixture was reacted at room temperature for 1 hour under nitrogen atmosphere. LCMS detected that the reaction was completed. The reaction mixture was slowly added to saturated ammonium chloride aqueous solution for quenching at room temperature, and extracted with dichloromethane. The organic phase was washed with saturated brine, dried over anhydrous sodium sulfate, filtered, and the filtrate was concentrated. The residue was purified by column chromatography [tetrahydrofuran (0.5% ammonia hydroxide)/petroleum ether: 15 to 50%] to obtain *tert*-butyl ((1S,2R,3S,5R)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate (710 mg, yield: 77.85%) as a yellow solid.
MS m/z: 583.1/585.1 $[M+H]^+$

**Step** 3: ***tert*-butyl ((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate**

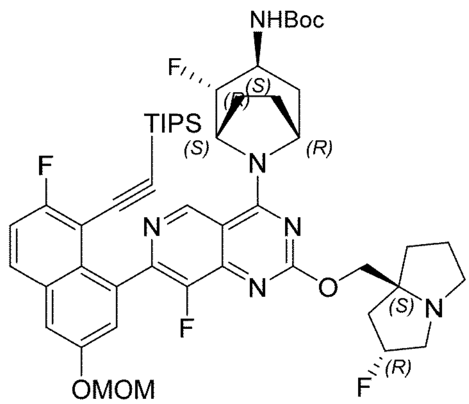

tert-Butyl ((1S,2R,3S,SR)-8-(7-chloro-8-fluoro-2-(((2R,7aS)-2-fluorohexahydro-1H-pyrrolizin-7a-yl)methoxy)pyrido[4,3-d]pyrimidin-4-yl)-2-fluoro-8-azabicyclo[3.2.1]octan-3-yl)carbamate (700 mg, 1.20 mmol, 1 *eq)* was dissolved in a solvent mixture of dioxane (10 mL) and water (5 mL), then ((2-fluoro-6-(methoxymethoxy)-8-(4,4,5,5-tetramethyl-1,3,2-dioxaborolan-2-yl)naphthalen-1-yl)ethynyl)triisopropylsilane (738.42 mg, 1.44 mmol, 1.2 *eq),* cesium carbonate (782.35 mg, 2.40 mmol, 2 *eq),* and methanesulfonato(diadamantyl-n-butylphosphino)-2'-amino-1,1'-biphenyl-2-yl)palladium(II) (174.87 mg, 0.240 mmol, 0.2 *eq)* were added thereto, and the system was replaced with nitrogen. The reaction mixture was reacted at 80°C for 2 hours under nitrogen atmosphere. LCMS monitored that the reaction was completed. The reaction mixture was extracted with ethyl acetate, and the organic phase was washed with saturated brine, dried over anhydrous sodium sulfate, filtered, and the filtrate was concentrated. The residue was purified by column chromatography [tetrahydrofuran (0.5% ammonia hydroxide)/petroleum ether: 15 to 60%] to obtain tert-butyl

((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate (850 mg, yield: 75.87%) as a yellow solid.
MS m/z: 933.5 [M+H]$^+$

**Step 4: 4-(4-((1S,2R,3S,SR)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-dipyrimidin-7-yl)-6-fluoro-5-((triisopropylsilyl)ethynyl)naphthalen-2-ol**

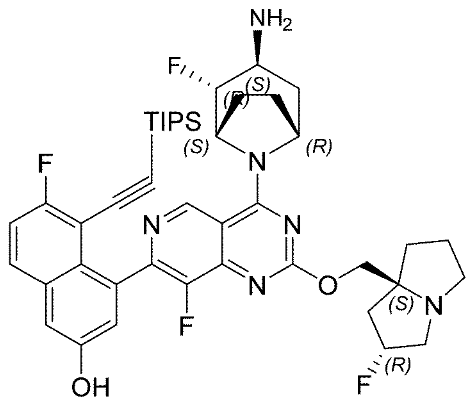

*tert*-Butyl ((1R,2S,3R,5S)-2-fluoro-8-(8-fluoro-7-(7-fluoro-3-(methoxymethoxy)-8-((triisopropylsilyl)ethynyl)naphthalen-1-yl)-2-(((2R,7aS)-2-fluorohexahydro-1H-pyrrolizin-7a-yl)methoxy)pyrido[4,3-d]pyrimidin-4-yl)-8-azabicyclo[3.2.1]octan-3-yl)carbamate (850 mg, 0.911 mmol, 1 *eq)* was dissolved in acetonitrile (10 mL), then a solution of hydrochloric acid in dioxane (4 M, 5 mL) was added thereto. The reaction mixture was reacted at room temperature for 1 hour. LCMS monitored that the reaction was completed. The reaction mixture was concentrated to obtain 4-(4-((1S,2R,3S,5R)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (1.1 g, crude product) as a yellow solid, and the crude product was used directly in the next step.
MS m/z: 789.3 [M+H]$^+$

**Step 5: 4-(4-((1S,2R,3S,5R)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol**

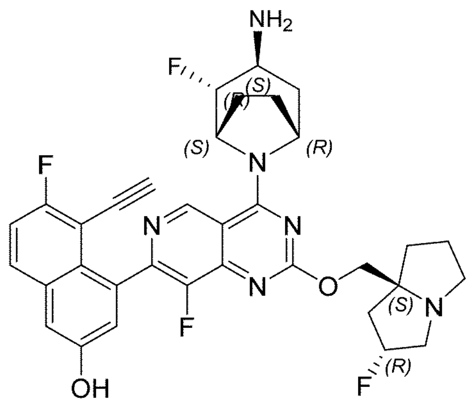

4-(4-((1S,2R,3S,5R)-3-Amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-6-fluoro-5-((triisopropylsilyl)ethynyl)naphthalen-2-ol (700 mg, 0.887 mmol, 1 *eq)* was dissolved in N,N-dimethylformamide (5 mL), then cesium fluoride (1.35 g, 8.87 mmol, 10 *eq)* was added thereto, and the reaction mixture was reacted at room temperature overnight. LCMS monitored that the

reaction was completed. The reaction mixture was filtered, and the filtrate was separated by HPLC to obtain 4-(4-((1S,2R,3S,SR)-3-amino-2-fluoro-8-azabicyclo[3.2.1]octan-8-yl)-8-fluoro-2-(((2R,7aS)-2-fluorohexahydro-1H-pyrrolizin-7a-yl)methoxy)pyrido[4,3-d]pyrimidin-7-yl)-5-ethynyl-6-fluoronaphthalen-2-ol (50 mg, yield: 8.91%) as a yellow solid.

MS m/z: 633.2 $[M+H]^+$

$^{19}F$ NMR (376 MHz, $CDCl_3$) $\delta$ -110.10 (s), -137.68 (s), -169.31 (s), -172.49 (s).

$^1H$ NMR (400 MHz, $CDCl_3$) $\delta$ 9.24-8.91 (m, 1H), 7.63-7.47 (m, 1H), 7.25-7.01 (m, 3H), 5.36-4.87 (m, 3H), 4.51-4.28 (m, 1H), 4.26-4.11 (m, 2H), 3.38-3.10 (m, 4H), 3.01-2.90 (m, 1H), 2.86-2.73 (m, 1H), 2.73-2.59 (m, 1H), 2.59-2.42 (m, 1H), 2.27-2.06 (m, 4H), 2.04-1.76 (m, 7H), 1.47-1.39 (m, 1H).

HPLC separation conditions:

column: YMC-Triart Prep C18 150 * 40 mm * 7 μm;
mobile phase: [Water (ammonia hydroxide v/v) - ACN]; B%: 25% to 65%, 9 min.

**Example 26: compound 26**

**Compound 26: 4-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-5,6-difluoronaphthalen-2-ol**

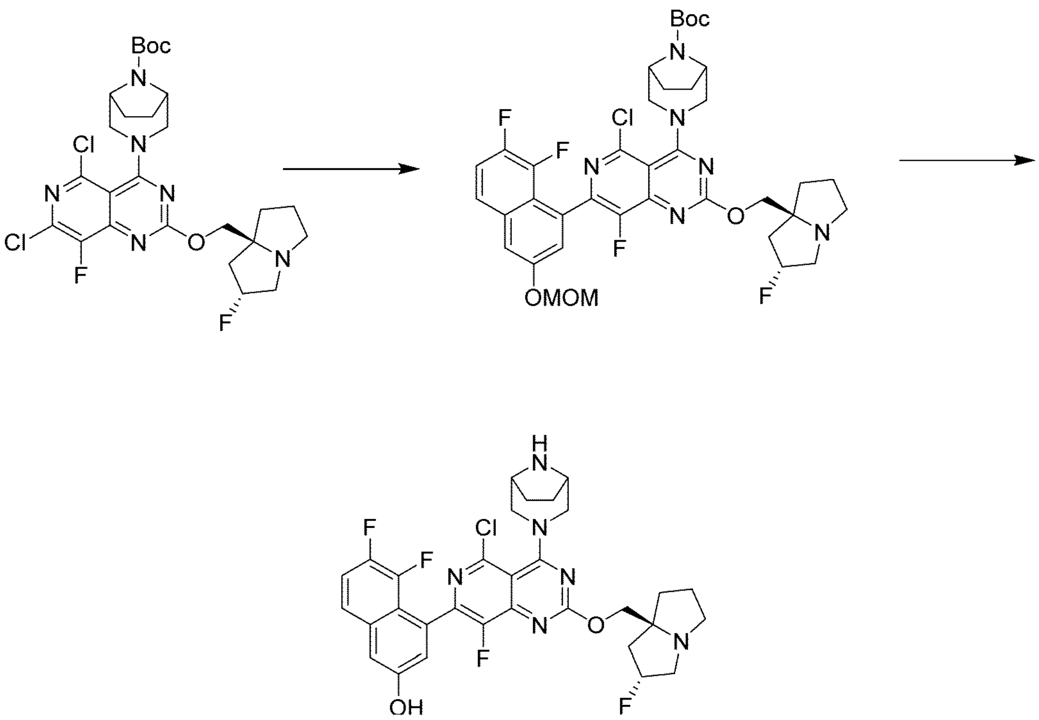

Compound 26

**Step 1: *tert*-butyl (1R,5S)-3-(5-chloro-7-(7,8-difluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate**

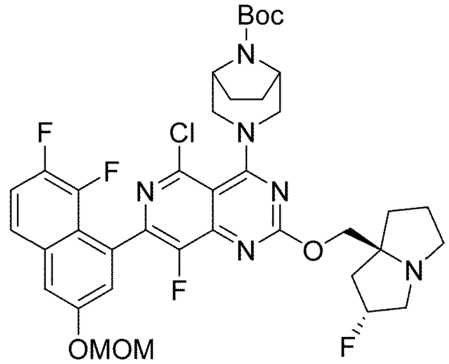

*tert*-Butyl (1R,5S)-3-(5,7-dichloro-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (1060 mg, 1.81 mmol, 1 *eq)* was dissolved in dioxane (10 mL) and water (5 mL), then 2-(7,8-difluoro-3-(methoxymethoxy)naphthalen-1-yl)-4,4,5,5-tetramethyl-1,3,2-dioxaborolane (697.37 mg, 1.99 mmol, 1.1 *eq),* cesium carbonate (1.18 g, 3.62 mmol, 2 *eq),* and [1,1'-bis(diphenylphosphino)ferrocene]dichloropalladium(II) (132.48 mg, 0.181 mmol, 0.1 *eq)* were added thereto. The reaction mixture was reacted at 80°C for 1 hour under nitrogen atmosphere. LCMS monitored that the reaction was completed. The reaction mixture was concentrated, and the residue was extracted with ethyl acetate. The organic phase was washed with saturated brine, dried over anhydrous sodium sulfate, filtered, and the filtrate was concentrated. The crude product was purified by column chromatography (tetrahydrofuran/petroleum ether: 10 to 30%) to obtain *tert*-butyl (1R,5S)-3-(5-chloro-7-(7,8-difluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (310 mg, yield: 22.1%) as a brown solid.
MS m/z: 773.3 $[M+H]^+$

**Step 2: 4-(4-((1R,SS)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-5,6-difluoronaphthalen-2-ol (compound 26)**

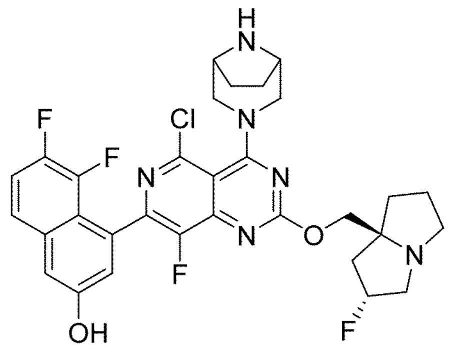

Compound 26

*tert*-Butyl (1R,5S)-3-(5-chloro-7-(7,8-difluoro-3-(methoxymethoxy)naphthalen-1-yl)-8-fluoro-2-(((2R,7aS)-2-fluorohexahydro-1H-pyrrolizin-7a-yl)methoxy)pyrido[4,3-d]pyrimidin-4-yl)-3,8-diazabicyclo[3.2.1]octane-8-carboxylate (600 mg, 0.776 mmol, 1 *eq)* was dissolved in dichloromethane (10 mL), then trifluoroacetic acid (3 mL) was added thereto, and the reaction mixture was reacted at room temperature for 1 hour. LCMS detected that the reaction was completed. The reaction mixture was concentrated, and the residue was purified by HPLC to obtain 4-(4-((1R,5S)-3,8-diazabicyclo[3.2.1]octan-3-yl)-5-chloro-8-fluoro-2-(((2R,7aS)-2-fluorohexahydro-1H-pyrrolizin-7a-yl)methoxy)pyrido[4,3-d]pyrimidin-7-yl)-5,6-difluoronaphthalen-2-ol (103 mg, yield: 21.10%) as a white solid.
MS m/z: 629.2 $[M+H]^+$

$^{19}F$ NMR (377 MHz, DMSO-$d_6$) δ -143.16, -144.42, -144.47, -172.09.

$^1H$ NMR (400 MHz, DMSO-$d_6$) δ 10.31 (s, 1H), 7.80-7.71 (m, 1H), 7.65-7.53 (m, 1H), 7.41 (s, 1H), 7.33 (br s, 1H), 5.28 (br d, *J* = 53.2 Hz, 1H), 4.17-4.03 (m, 2H), 3.55-3.40 (m, 7H), 3.15-3.01 (m, 3H), 2.86-2.75 (m, 1H), 2.18-1.94 (m, 3H), 1.91-1.67 (m, 4H), 1.75-1.42 (m, 3H).

HPLC separation conditions:

column: Waters Xbridge BEH C18 100 * 30 mm * 10 μm;
mobile phase: Water (ammonia hydroxide v/v) - ACN]; B%: 24% to 64%, 10 min, 214 nm.

**Example 27: compound 27**

**4-(4-((1R,5S)-3,8-Diazabicyclo[3.2.1]octan-3-yl)-5-chloro-8-fluoro-2-(((2R,7aS)-2-fluorotetrahydro-1H-pyrrolizin-7a(5*H*)-yl)methoxy-d2)pyrido[4,3-d]pyrimidin-7-yl)-5-ethynyl-6-fluoronaphthalen-2-ol**

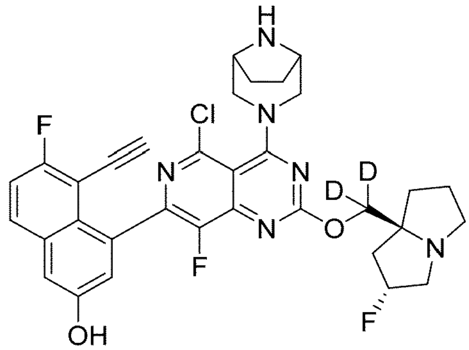

**Step 1: ((2R,7aS)-2-fluorotetrahydro-1*H*-pyrrolizin-7a(5*H*)-yl)methan-d2-ol**

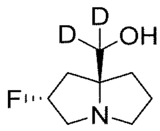

(2R,7aS)-2-Fluorotetrahydro-1*H*-pyrrolizin-7a(5H)-carboxylic acid hydrochloride (9.8 g, 46.7 mmol, 1 *eq)* was dissolved in tetrahydrofuran (120 mL), and lithium aluminum deuteride (5.7 g, 135.7 mmol, 3 eq) was slowly added thereto in batches. The reaction mixture was reacted at room temperature for 16 hours. TLC monitored that the reaction was completed. The reaction mixture was quenched in an ice-water bath and extracted with ethyl acetate. The organic phase was washed with saturated brine (100 mL), dried over anhydrous sodium sulfate, filtered, and the filtrate was concentrated to obtain ((2R,7aS)-2-fluorotetrahydro-1H-pyrrolizin-7a(5H)-yl)methan-d2-ol (3.6 g, crude product) as a yellow oil. The crude product was used directly in the next step.
MS m/z: 162 $[M+H]^+$

**Step 2: *tert-butyl* (1R,5S)-3-(5,7-dichloro-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate**

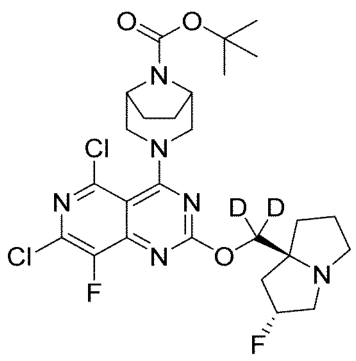

*tert*-Butyl (1R,5S)-3-(2,5,7-trichloro-8-fluoropyrido[4,3-d]pyrimidin-4-yl)-3,8-diazabicyclo[3.2.1]octane-8-carboxylate (6.3 g, 13.6 mmol, 1 eq) and ((2R,7aS)-2-fluorotetrahydro-1*H*-pyrrolizin-7a(5*H*)-yl)methan-d2-ol (2.2 g, 13.6 mmol, 1 eq) were dissolved in tetrahydrofuran (60 mL), then lithium tert-butoxide (2.1 g, 27.2 mmol, 2 eq) was slowly added thereto, and the reaction mixture was reacted at room temperature for 4 hours. LCMS monitored that the reaction was completed. The reaction mixture was slowly poured into saturated ammonium chloride aqueous solution and extracted with dichloromethane. The organic phase was washed with saturated brine, dried over anhydrous sodium sulfate, filtered, and the filtrate was concentrated. The residue was purified by column chromatography (ethyl acetate/petroleum ether: 40 to 60%) to obtain tert-butyl (1R,5S)-3-(5,7-dichloro-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (6 g, yield: 75%) as a yellow solid.
MS m/z: 587 $[M+H]^+$

**Step 3: *tert*-butyl (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate**

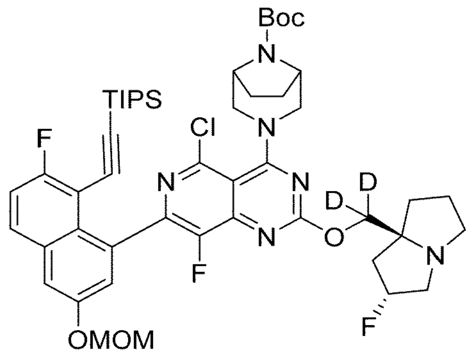

*tert*-Butyl (1R,5S)-3-(5,7-dichloro-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (1.1 g, 1.87 mmol, 1 eq), (7-fluoro-3-(methoxymethoxy)-8-(triisopropylsilyl)ethynyl)naphthalen-1-yl)boronic acid (0.97 g, 2.24 mmol, 1.2 eq), potassium carbonate (0.77 g, 5.62 mmol, 3 eq), 2-dicyclohexylphosphino-2,6-diisopropoxy-1,1-biphenyl (175 mg, 0.374 mmol, 0.2 eq), and methanesulfonato(2-dicyclohexylphosphino-2,6-diisopropoxy-1,1-biphenyl)(2-amino-1,1-biphenyl-2-yl)palladium(II) (313 mg, 0.374 mmol, 0.2 eq) were dissolved in a solvent mixture of toluene (20 mL), water (10 mL), and ethanol (10 mL). The system was replaced with nitrogen, and the reaction mixture was reacted at 80°C for 16 hours. The phases of the reaction mixture were separated. The organic phase was washed with saturated brine, dried over anhydrous sodium sulfate, filtered, and the filtrate was concentrated. The residue was purified by column chromatography (tetrahydrofuran/petroleum ether: 0 to 50%) to obtain tert-butyl (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (0.5 g, yield: 30%) as a yellow solid.
MS m/z: 937 $[M+H]^+$

**Step 4: *tert*-butyl (1R,5S)-3-(5-chloro-7-(8-ethynyl-7-fluoro-3-(methoxymethoxy)naphthalen-1-yl)-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate**

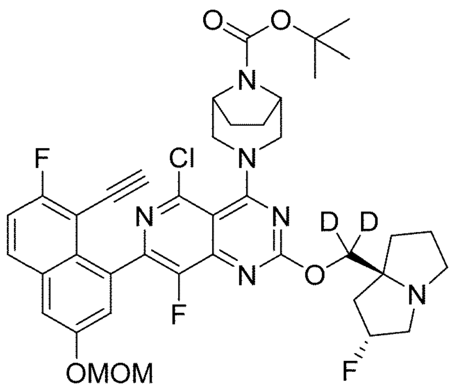

*tert*-Butyl (1R,5S)-3-(5-chloro-8-fluoro-7-(7-fluoro-3-(methoxymethoxy)-8-((triisopropylsilyl)ethynyl)naphthalen-1-yl)-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (240 mg, 0.26 mmol, 1 eq) was dissolved in N,N-dimethylformamide (20 mL), then cesium fluoride (389 mg, 2.6 mmol, 10 eq) was added thereto, and the reaction mixture was reacted at room temperature for 1 hour. The reaction mixture was filtered, then slowly poured into saturated ammonium chloride aqueous solution, and extracted with ethyl acetate. The organic phase was washed with saturated brine, dried over anhydrous sodium sulfate, and concentrated to obtain *tert*-butyl (1R,5S)-3-(5-chloro-7-(8-ethynyl-7-fluoro-3-(methoxymethoxy)naphthalen-1-yl)-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (200 mg, crude product) as a yellow oil.
MS m/z: 781 $[M+H]^+$

**Step 5: 4-(4-((1R,SS)-3,8-diazabicyclo[3.2.1]octan-3-yl)-5-chloro-8-fluoro-2-(((2R,7aS)-2-fluorotetrahydro-1*H*-pyrrolizin-7a(5*H*)-yl)methoxy-d2)pyrido[4,3-d]pyrimidin-7-yl)-5-ethynyl-6-fluoronaphthalen-2-ol**

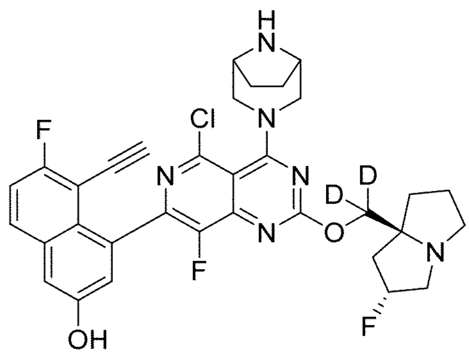

*tert*-Butyl (1R,5S)-3-(5-chloro-7-(8-ethynyl-7-fluoro-3-(methoxymethoxy)naphthalen-1-yl)-8-fluoro-2-(((2R,7aS)-2-fluorotetrahydro-1*H*-pyrrolizin-7a(5*H*)-yl)methoxy-d2)pyrido[4,3-d]pyrimidin-4-yl)-3,8-diazabicyclo[3.2.1]octane-8-carboxylate (200 mg, 0.26 mmol, 1 eq) was dissolved in acetonitrile (5 mL), then a solution of hydrochloric acid in dioxane (4 M, 3 mL) was added thereto, and the reaction mixture was reacted at room temperature for 1 hour. LCMS detected that the reaction was completed. The reaction mixture was concentrated and purified by HPLC to obtain 4-(4-((1R,5S)-3,8-diazabicyclo[3.2.1]octan-3-yl)-5-chloro-8-fluoro-2-(((2R,7aS)-2-fluorotetrahydro-1*H*-pyrrolizin-7a(5*H*)-yl)methoxy-d2)pyrido[4,3-d]pyrimidin-7-yl)-5-ethynyl-6-fluoronaphthalen-2-ol (11 mg) as a white solid.
MS m/z: 637 $[M+H]^+$

$^1$H NMR (400 MHz, DMSO-d6) δ 8.20 (s, 1H), 8.00 (dd, J = 9.2, 5.9 Hz, 1H), 7.55 - 7.21 (m, 2H), 5.28 (d, J =

54.8 Hz, 1H), 3.50 (s, 6H), 2.83 (q, J = 8.3 Hz, 9H), 2.31 - 1.21 (m, 6H).

$^{19}F$ NMR (376 MHz, DMSO-d6) δ -110.25, -142.14, -172.12.

HPLC separation conditions:

column: Phenomenex C18 80 * 40 mm * 3 μm; flow rate: 25 mL/min
mobile phase A: Water (0.1% formic acid v/v) mobile phase B: ACN; gradient: 25% to 65% (v/v), 11 min; detection wavelength: 254/214 nm.

**Example 28: compound 28**

**4-(4-((1R,SS)-3,8-Diazabicyclo[3.2.1]octan-3-yl)-5-chloro-8-fluoro-2-(((2R,7aS)-2-fluorotetrahydro-1H-pyrrolizin-7a(5H)-yl)methoxy)pyrido[4,3-d]pyrimidin-7-yl)-5-(ethynyl-d)-6-fluoronaphthalen-2-ol hydrochloride**

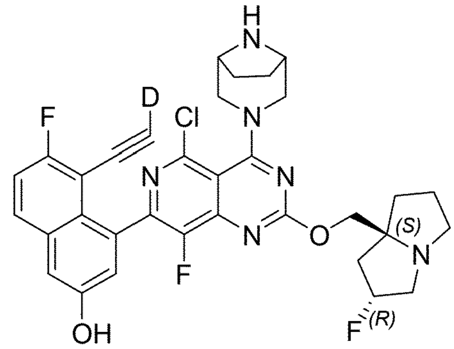

hydrochloride

**Step 1: *tert*-butyl (1R,5S)-3-(5-chloro-7-(8-(ethynyl-d)-7-fluoro-3-(methoxymethoxy)naphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate**

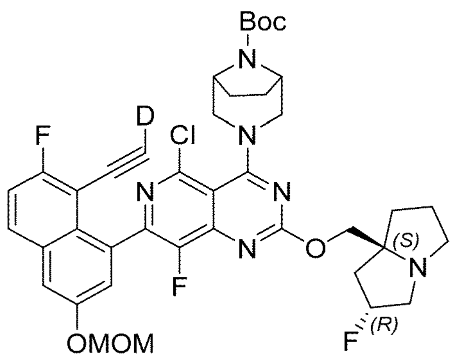

*tert*-Butyl (1R,5S)-3-(5-chloro-7-(8-ethynyl-7-fluoro-3-(methoxymethoxy)naphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate (1.5 g, 1.92 mmol, 1 *eq)* was dissolved in anhydrous tetrahydrofuran (50 mL), and the mixture was cooled to -30°C under nitrogen atmosphere. Lithium diisopropylamide (2.9 mL, 2 mol/L) was slowly added thereto, and the reaction was carried out at -30°C for 1.5 hours. Deuterated methanol (3 mL)was slowly added thereto, and the reaction was carried out at - 30°C for 1 hour. LCMS monitored that the reaction was completed. Deuterium oxide (5 mL) was slowly added to the reaction mixture, and the phases were separated. The organic phase was extracted with ethyl acetate, dried over anhydrous sodium sulfate, filtered, and subjected to rotary evaporation until dryness to obtain a crude product, which was purified by column chromatography (dichloromethane/methanol: 0 to 5%) to obtain tert-butyl (1R,5S)-3-(5-chloro-7-(8-(ethynyl-d)-7-fluoro-3-(methoxymethoxy)naphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carboxylate (1.46 g, yield: 97.3%) as a yellow solid.
MS m/z: 780 $[M+H]^+$

**Step 2: 4-(4-((1R,5S)-3,8-diazabicyclo[3.2.1]octan-3-yl)-5-chloro-8-fluoro-2-(((2R,7aS)-2-fluorotetrahydro-1H-pyrrolizin-7a(5H)-yl)methoxy)pyrido[4,3-d]pyrimidin-7-yl)-5-(ethynyl-d)-6-fluoronaphthalen-2-ol hydrochloride**

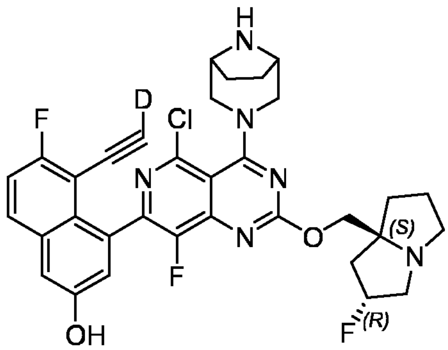

hydrochloride

*tert*-Butyl (1R,5S)-3-(5-chloro-7-(8-(ethynyl-d)-7-fluoro-3-(methoxymethoxy)naphthalen-1-yl)-8-fluoro-2-(((2R,7aS)-2-fluorotetrahydro-1H-pyrrolizin-7 a(5H)-yl)methoxy)pyrido [4,3 -d]pyrimidin-4-yl)-3,8-diazabicyclo[3.2.1] octane-8-carboxylate (900 mg, 1.155 mmol, 1 *eq)* was dissolved in acetonitrile (70 mL), and the mixture was cooled to 0°C under nitrogen atmosphere. Hydrochloric acid/dioxane (4 M, 3 mL, 12 mmol) was slowly added thereto, and the reaction mixture was reacted at 0°C for 3 hours. LCMS detected that the reaction was completed. The reaction mixture was concentrated to obtain 4-(4-((1R,SS)-3,8-diazabicyclo[3.2.1]octan-3-yl)-5-chloro-8-fluoro-2-(((2R,7aS)-2-fluorotetrahydro-1H-pyrrolizin-7a(5H)-yl)methoxy)pyrido[4,3-d]pyrimidin-7-yl)-5-(ethynyl-d)-6-fluoronaphthalen-2-ol hydrochloride (818 mg, yield: 100%) as a white solid.
MS m/z: 636 $[M+H]^+$

$^{19}F$ NMR (376 MHz, DMSO-$d_6$) $\delta$ -110.35, -138.99, -172.67.

$^1H$ NMR (400 MHz, DMSO-$d_6$) $\delta$ 11.80 (s, 1H), 10.11 (s, 1H), 9.87 (s, 1H), 8.02-7.98 (m, 1H), 7.52-7.43 (m, 2H), 7.30-7.28 (m, 1H), 4.51 (br, 6H), 4.29-4.07 (m, 3H), 3.86-3.73 (m, 3H), 3.27-3.24 (m, 1H), 2.62-2.40 (m, 1H), 2.37-2.31 (m, 2H), 2.21-2.17 (m, 2H), 2.15-2.05 (m, 2H), 1.91-1.86 (m, 1H), 1.77-1.74 (m, 1H), 1.42-1.34 (m, 1H).

**Test Example 1:** proliferation inhibitory activity on Ba/F3 KRAS-G12D, Ba/F3 KRAS-G12V stably transfected cell lines and AGS tumor cell lines containing KRAS G12D mutations

To determine the proliferation inhibitory activity of the compounds *in vitro* on mouse pro-B cells Ba/F3 (Ba/F3 KRAS-G12D and Ba/F3 KRAS-G12V cells stably expressing KRAS G12D/V mutant proteins) and gastric cancer AGS cells expressing KRAS G12D mutant protein.

Cell sources: Ba/F3 KRAS-G12D and Ba/F3 KRAS-G12V were purchased from Kyinno Biotech (Beijing) Co., Ltd, Cat. No.: KC-1259 and KC-1261, respectively; AGS was purchased from Shanghai Bioleaf Biotech Co., Ltd.

Cells in the logarithmic growth phase were seeded in a 96-well plate (8000, 8000, 4000 cells/well, 90 μL/well for Ba/F3 KRAS-G12D, Ba/F3 KRAS-G12V, and AGS cells respectively), incubated at 37°C for 1 day with 5% $CO_2$, and then a gradient dilution of the test compound was added thereto (compounds 1 to 28 and comparative compounds). The details were as follows: the compound stock solution (10 mM) previously dissolved in DMSO was diluted to 10 gradient concentrations at a fold ratio (4 fold) and diluted to 10 fold of the target concentration in another 96-well plate with culture medium, and then 10 μL/well of the compound solution was added to the 96-well plate seeded with cells to reach the target concentration (10000, 2500, 625, 156, 39, 10, 2.5, 0.6, 0.15, 0.04 nM). Each concentration was provided with 3 duplicate wells and a blank control. After continued incubation at 37°C and 5% $CO_2$ for 72 hours, 50 μL of CellTiter-Glo® **2.0** reagent (luciferase ATP bioluminescence detection reagent, purchased from Promega, Cat. No.: G9243) was added to each well, shaken for 2 minutes, and incubated at room temperature for 8 minutes, and then fluorescence luminescence intensity was detected (light collection time was 100 ms). The inhibitory rate of compounds with various concentrations on cell proliferation was calculated. Cell proliferation inhibition rate (%) = [(luminous intensity control group containing cell culture medium for 72 hours - luminous intensity compound group for 72 hours) / (luminous intensity

control group containing cell culture medium for 72 hours - luminous intensity control group without cell culture medium for 72 hours)] × 100%. GraphPad Prism 8.3 software was used to analyze the data, and nonlinear S-curve regression was used to fit the data to obtain a dose-effect curve, from which the $IC_{50}$ value was calculated. The results are shown in Table 1.

Table 1

| Compound | **Ba/F3 KRAS-G12D $IC_{50}$ (μM)** | **AGS $IC_{50}$ (μM)** | **Ba/F3 KRAS-G12V $IC_{50}$ (μM)** |
|---|---|---|---|
| Compound 1 | - | - | **C** |
| Compound 2 | - | - | **C** |
| Compound 3 | - | - | **C** |
| Compound 4 | - | - | **C** |
| Compound 5 | - | - | **C** |
| Compound 6-P1 | **B** | **C** | **C** |
| Compound 6-P2 | **A** | **B** | **C** |
| Compound 7 | **C** | **-** | **C** |
| Compound 8 | **-** | **-** | **C** |
| Compound 9 | **C** | **-** | **C** |
| Compound 10 | **B** | **B** | **C** |
| Compound 11 | **-** | **-** | **C** |
| Compound 12 | **-** | **-** | **C** |
| Compound 13 | **-** | **-** | **C** |
| Compound 14 | **-** | **-** | **C** |
| Compound 15 | **C** | **-** | **C** |
| Compound 16 | **-** | **-** | **C** |
| Compound 17 | **B** | **C** | **C** |
| Compound 18 | **B** | **C** | **C** |
| Compound 19 | **-** | **-** | **C** |
| Compound 20-P1 | **A** | **B** | **C** |
| Compound 20-P2 | **B** | **B** | **C** |
| Compound 21 | **0.001** | **0.009** | **C** |
| Compound 22 | **0.002** | **0.016** | **C** |
| Compound 23 | **-** | **2.5** | **C** |
| Compound 24 | **1.9** | **2.5** | **C** |
| Compound 25 | **1.0** | **2.5** | **C** |
| Compound 26 | **0.0017** | **0.028** | **C** |
| Compound 27 | **0.001** | **0.006** | **-** |
| Compound 28 | **0.001** | **0.006** | **-** |
| Comparative compound | **0.003** | **0.041** | **C** |

**A** <0.1 μM; 0.1 μM < **B** ≤ 5 μM; 5 μM < **C** ≤ 10 μM.

The structure of the comparative compound is as follows, which was prepared with reference to the method of Example 252 in patent application WO2021041671A1,

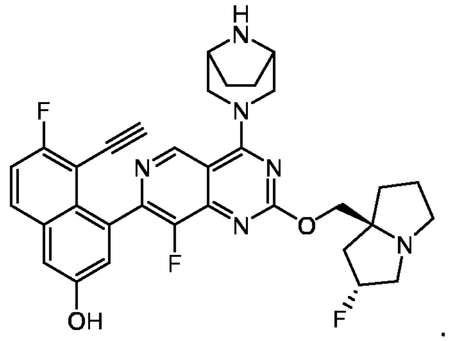

The test results show that the compounds of the present disclosure (compounds 1 to 28) have good proliferation inhibitory activity on Ba/F3 KRAS-G12D, Ba/F3 KRAS-G12V, and AGS cells containing KRAS G12D/V mutations.

**Test Example 2: *in vivo* efficacy of test compounds in HPAC model**

This experiment is used to evaluate the *in vivo* efficacy of compounds in the HPAC model.

Source of experimental animals: Beijing Vital River Laboratory Animal Technology Co., Ltd., certificate number: 20170011007990.

Animal modeling and random grouping: HPAC cells were cultured and expanded to 30 T175 $cm^2$ culture flasks. Cells were collected, resuspended in serum-free culture medium DMEM for counting, and added with matrix glue at a ratio of 1:1, and inoculated into the right anterior subcutaneous region of BALB/c Nude mice at $5 \times 10^6$ cells/0.1 mL. When the average tumor volume reached approximately 281 $mm^3$, mice were randomly divided into various experimental groups according to tumor size, with 5 mice per group. The day of grouping was defined as day 0, that is, D0.

Experimental scheme: BALB/c nude mice were subcutaneously inoculated with HPAC cells to establish a cell line xenograft tumor model. The test was divided into a 30 mg/kg group of compound 21, a 30 mg/kg group of comparative compound, and a vehicle group, with 5 mice in each group. The mice were administered intraperitoneally in an administration volume of 10 μL/g. The vehicle group was given an equal amount of vehicle (sulfobutyl-β-cyclodextrin sodium salt dissolved in 50 mM citric acid buffer, the mass-to-volume concentration of sulfobutyl-β-cyclodextrin sodium salt was 10%, and the pH value of the vehicle was 5.0), administered twice a day for 10 days. During the entire experiment, the body weight and tumor size of the mice were measured on days 4, 7, and 10 to observe whether toxic reactions occurred. Efficacy was evaluated based on relative tumor proliferation rate. Other compounds of the present disclosure (compounds 1 to 20 and 22 to 28) were tested with reference to compound 21.

Formula for calculating tumor volume: $V = 1/2 \times a \times b \times b$, wherein a and b represent the length and width of the tumor, respectively.

Formula for calculating weight change: weight change (%) = (weight/D0 weight-1) × 100%.

Formula for calculating the relative tumor proliferation rate T/C%:

$$\text{If } V_{10} > V_0,$$

$$T/C\% = [(V_{10} - V_0) / (V_{con10} - V_{con0})] \times 100\%;$$

$$\text{if } V_{10} < V_0,$$

$$T/C\% = [(V_{10} - V_0) / V_0] \times 100\%;$$

wherein $V_{10}$: tumor volume of mice on day 10; $V_0$: tumor volume of mice on day 0; $V_{con10}$: tumor volume of mice in the vehicle group on day 10; $V_{con0}$: tumor volume of mice in the vehicle group on day 0.

The experimental results are shown in the table below:

| Compound | T/C% | Weight change (%) |
|---|---|---|
| Compound 21 | -64.96 | -7.27 |
| Comparative compound | -60.08 | -9.44 |

The structure of the comparative compound is as follows, which was prepared with reference to the method of Example 252 in patent application WO2021041671A1,

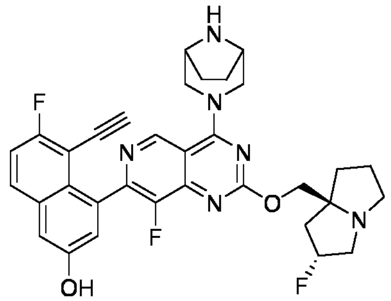

.

The conclusion indicates that the compounds of the present disclosure (compounds 1 to 28) show good anti-tumor effects in the BALB/c nude mice animal model, and have little effect on the body weight of nude mice.

Although specific embodiments of the present disclosure have been described above, those skilled in the art should understand that these are merely examples, and various changes or modifications can be made to these embodiments without departing from the principles and essence of the present disclosure. Therefore, the scope of protection of the present disclosure is defined by the appended claims.

## Claims

1. A nitrogen-containing heterocyclic compound of formula I, a stereoisomer thereof, or a pharmaceutically acceptable salt thereof,

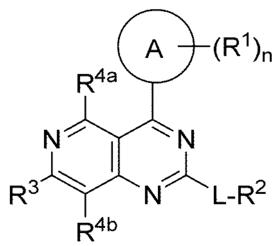

I

wherein ring A is 3- to 7-membered cycloalkyl, 4- to 10-membered heterocycloalkyl, or 4- to 10-membered heterocycloalkenyl; the number of heteroatoms of the 4- to 10-membered heterocycloalkyl and 4- to 10-membered heterocycloalkenyl is 1 or 2, wherein the heteroatom is selected from one or two of N, O, and S;

n is 0, 1, 2, 3, 4, 5, 6, 7, 8, 9, or 10;

each $R^1$ is independently deuterium, halogen, -CN, -OH, $-N(R^5)_2$, $C_1$-$C_6$ alkyl, $C_1$-$C_6$ alkyl substituted by one or more than one $R^{1a}$, $C_1$-$C_6$ alkyl-O-, $C_1$-$C_6$ alkyl-O- substituted by one or more than one $R^{1b}$, $C_2$-$C_6$ alkenyl, $C_2$-$C_6$ alkynyl, $C_2$-$C_6$ alkenyl substituted by one or more than one $R^{1c}$, $C_2$-$C_6$ alkynyl substituted by one or more than one $R^{1d}$, -C(=O)H, $-CO_2R^5$, - $C(=O)N(R^5)_2$, 5- to 6-membered heteroaryl, or two $R^1$ on the same ring atom form an oxo group; the heteroatom of the 5- to 6-membered heteroaryl is selected from one or more than one of N, O, and S, and the number of heteroatoms is 1, 2, or 3; when there is more than one substituent, the substituents are the same or different;

$R^{1a}$, $R^{1b}$, $R^{1c}$, and $R^{1d}$ are each independently deuterium, -CN, halogen, or -OH;

L is $-(CR^{6a}R^{6b})_{n1}$-, $-O-(CR^{6a}R^{6b})_{n2}$-, $-S-(CR^{6a}R^{6b})_{n3}$-, or $-N(R^5)(CR^{6a}R^{6b})_{n4}$-;

$R^2$ is H, -COOH, -N($R^5$)$_2$, $C_1$-$C_6$ alkyl, $C_1$-$C_6$ alkyl-O-, 3- to 7-membered cycloalkyl, 4-to 10-membered heterocycloalkyl, $C_6$-$C_{10}$ aryl, 5- to 10-membered heteroaryl, - NHC(=NH)$NH_2$, -C(O)N($R^5$)$_2$, -($CH_2OR^5$)($CH_2$)$_{n5}$$OR^5$, -$NR^5$C(=O)-$C_6$-$C_{10}$ aryl, -C(=O)O-Ci-$C_6$ alkyl, 3- to 7-membered cycloalkyl substituted by one or more than one $R^{2a}$, 4- to 10-membered heterocycloalkyl substituted by one or more than one $R^{2b}$, $C_6$-$C_{10}$ aryl-C(=O)$NR^5$-substituted by one or more than one $R^{2c}$, $C_6$-$C_{10}$ aryl substituted by one or more than one $R^{2d}$, or 5- to 10-membered heteroaryl substituted by one or more than one $R^{2e}$; the heteroatom of the 4- to 10-membered heterocycloalkyl is selected from one or more than one of N, O, and S, and the number of heteroatoms is 1, 2, or 3; the heteroatom of the 5- to 10-membered heteroaryl is selected from one or more than one of N, O, and S, and the number of heteroatoms is 1, 2, or 3; when there is more than one substituent, the substituents are the same or different;
$R^{2a}$, $R^{2b}$, and $R^{2c}$ are each independently halogen, -OH, deuterium, -CN, -C(=O)H, $C_1$-$C_4$ alkyl, $C_2$-$C_4$ alkenyl, $C_2$-$C_4$ alkynyl, $C_1$-$C_4$ alkyl substituted by one or more than one $R^{2\text{-}a}$, Ci-$C_4$ alkyl-O-, phenyl-Q-, FOzS-phenylene-Q-, phenyl-C(=O)NH-, pyrazolyl substituted by one or more than one $C_1$-$C_4$ alkyl, -N($R^5$)$_2$, ($C_1$-$C_4$ alkyl)-O-$C_1$-$C_4$ alkyl, ($C_1$-$C_4$ alkyl)-C(=O)-, =O, ($C_1$-$C_4$ alkyl substituted by one or more than one halogen)-C(=O)-, -$SO_2F$, ($C_1$-$C_4$ alkyl)-$SO_2$-, ($C_1$-$C_4$ alkyl)-O-($C_1$-$C_4$ alkyl)-O-, -$CH_2$OC(=O)N($R^5$)$_2$, ($C_1$-$C_4$ alkyl)-O-C(=O)-$NHCH_2$-, -$CH_2$NHC(=O)N($R^5$)$_2$, ($C_1$-$C_4$ alkyl)-C(=O)$NHCH_2$-, (pyrazolyl)-$CH_2$-, ($C_1$-$C_4$ alkyl)-$SO_2$-$NHCH_2$-, (4- to 10-membered heterocycloalkyl)-C(=O)-$OCH_2$-, ($R^5$)$_2$N-C(=O)-O-, ($C_1$-$C_4$ alkyl)-O-($C_1$-$C_4$ alkyl)-NH-C(=O)-O-, phenyl-($C_1$-$C_4$ alkyl)-NH-C(=O)-O-, (4- to 10-membered heterocycloalkyl)-C(=O)-O-, or (4- to 10-membered heterocycloalkyl)-$CH_2$-; phenyl in the phenyl-C(=O)NH- and phenyl-($C_1$-$C_4$ alkyl)-NH-C(=O)-O- is optionally substituted by -C(=O)H, halogen, CN, or OH; 4- to 10-membered heterocycloalkyl in the (4-to 10-membered heterocycloalkyl)-C(=O)-$OCH_2$-, (4- to 10-membered heterocycloalkyl)-C(=O)-O-, and (4- to 10-membered heterocycloalkyl)-$CH_2$- is optionally substituted by =O; the heteroatom of the 4- to 10-membered heterocycloalkyl in the (4- to 10-membered heterocycloalkyl)-C(=O)-$OCH_2$-, (4- to 10-membered heterocycloalkyl)-C(=O)-O-, and (4- to 10-membered heterocycloalkyl)-$CH_2$- is selected from one or more than one of N, O, and S, and the number of heteroatoms is 1, 2, or 3; when there is more than one substituent, the substituents are the same or different;
Q is independently a linker bond or -O-;
each $R^{2\text{-}a}$ is independently deuterium, -CN, halogen, or -OH;
$R^{2d}$ and $R^{2e}$ are each independently halogen, -OH, -C(=O)H, $C_1$-$C_4$ alkyl, $C_2$-$C_4$ alkenyl, $C_2$-$C_4$ alkynyl, $C_1$-$C_4$ alkyl-O-, $C_1$-$C_4$ alkyl substituted by one or more than one halogen or OH, or -N($R^5$)$_2$; when there is more than one substituent, the substituents are the same or different;
$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;
$R^{3a}$ and $R^{3b}$ are each independently deuterium, halogen, -OH, -CN, $C_1$-$C_6$ alkyl, $C_1$-$C_6$ alkyl-O-, $C_1$-$C_6$ alkyl-S-, $C_1$-$C_6$ alkyl substituted by one or more than one $R^{3\text{-}a}$, $C_1$-$C_6$ alkyl-O-substituted by one or more than one $R^{3\text{-}b}$, $C_1$-$C_6$ alkyl-S- substituted by one or more than one $R^{3\text{-}c}$, $C_2$-$C_6$ alkenyl, $C_2$-$C_6$ alkynyl, $C_2$-$C_6$ alkenyl substituted by one or more than one $R^{3\text{-}d}$, $C_2$-$C_6$ alkynyl substituted by one or more than one $R^{3\text{-}e}$, -N($R^5$)$_2$, -($CH_2$)-C(=O)N($R^5$)$_2$, 3- to 6-membered cycloalkyl, 3- to 6-membered cycloalkyl substituted by one or more than one $R^{3\text{-}f}$, or triazolyl; when there is more than one substituent, the substituents are the same or different;
$R^{3\text{-}a}$, $R^{3\text{-}b}$, $R^{3\text{-}c}$, $R^{3\text{-}d}$, $R^{3\text{-}e}$, and $R^{3\text{-}f}$ are each independently deuterium, halogen, -CN, -OH, $C_1$-$C_4$ alkyl, $C_1$-$C_4$ alkyl-O-, or 3- to 6-membered cycloalkyl;
$R^{4a}$ and $R^{4b}$ are each independently H, deuterium, -N($R^5$)$_2$, halogen, -OH, $C_1$-$C_6$ alkyl, - CN, halo-$C_1$-$C_6$ alkyl, $C_1$-$C_6$ alkyl-O-, deuterated $C_1$-$C_6$ alkyl, $C_2$-$C_6$ alkenyl, or $C_2$-$C_6$ alkynyl; when there is more than one substituent, the substituents are the same or different;
n1, n2, n3, n4, or n5 are each independently 0, 1, 2, or 3;
each $R^5$ is independently H or $C_1$-$C_6$ alkyl;
$R^{6a}$ and $R^{6b}$ are each independently H, deuterium, halogen, -CN, -OH, $C_1$-$C_4$ alkyl, or deuterated $C_1$-$C_4$ alkyl; and, when ring A comprises two heteroatoms, $R^{4a}$ is not H.

**2.** The nitrogen-containing heterocyclic compound of formula I, the stereoisomer thereof, or the pharmaceutically acceptable salt thereof according to claim 1, wherein the nitrogen-containing heterocyclic compound of formula I, the stereoisomer thereof, or the pharmaceutically acceptable salt thereof satisfies one or more than one of the following conditions:

a) when ring A is 4- to 10-membered heterocycloalkyl, the 4- to 10-membered heterocycloalkyl is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

b) when ring A is 4- to 10-membered heterocycloalkyl, the number of heteroatoms of the 4- to 10-membered heterocycloalkyl is 1 or 2, wherein the heteroatom is N;

c) when ring A is 4- to 10-membered heterocycloalkyl, the 4- to 10-membered heterocycloalkyl is connected to the pyrimidine ring shown in formula I through an N atom;

d) when ring A is 4- to 10-membered heterocycloalkenyl, the 4- to 10-membered heterocycloalkenyl is 5- to 7-membered monocyclic heterocycloalkenyl, 6- to 8-membered fused heterocycloalkenyl, 6- to 8-membered bridged heterocycloalkenyl, or 7- to 10-membered spiro heterocycloalkenyl;

e) when ring A is 4- to 10-membered heterocycloalkenyl, the number of heteroatoms of the 4- to 10-membered heterocycloalkenyl is 1 or 2, wherein the heteroatom is N;

f) when ring A is 4- to 10-membered heterocycloalkenyl, the 4- to 10-membered heterocycloalkenyl is connected to the pyrimidine ring shown in formula I through a C atom or an N atom;

g) when $R^1$ is halogen, the halogen is fluorine, chlorine, bromine, or iodine;

h) when $R^2$ is 4- to 10-membered heterocycloalkyl or 4- to 10-membered heterocycloalkyl substituted by one or more than one $R^{2b}$, 4- to 10-membered heterocycloalkyl in the 4- to 10-membered heterocycloalkyl and 4- to 10-membered heterocycloalkyl substituted by one or more than one $R^{2b}$ is 5- to 7-membered monocyclic heterocycloalkyl, 6- to 8-membered fused heterocycloalkyl, 6- to 8-membered bridged heterocycloalkyl, or 7- to 10-membered spiro heterocycloalkyl;

i) when $R^{2a}$, $R^{2b}$, $R^{2c}$, $R^{2d}$, and $R^{2e}$ are each independently halogen, the halogen is fluorine, chlorine, bromine, or iodine;

j) when $R^3$ is $C_6$-$C_{10}$ aryl or $C_6$-$C_{10}$ aryl substituted by one or more than one $R^{3a}$, $C_6$-$C_{10}$ aryl in the $C_6$-$C_{10}$ aryl and $C_6$-$C_{10}$ aryl substituted by one or more than one $R^{3a}$ is phenyl or naphthyl;

k) when $R^3$ is $C_6$-$C_{10}$ aryl or $C_6$-$C_{10}$ aryl substituted by one or more than one $R^{3a}$, $C_6$-$C_{10}$ aryl in the $C_6$-$C_{10}$ aryl and $C_6$-$C_{10}$ aryl substituted by one or more than one $R^{3a}$ is not fused with other rings;

l) when $R^3$ is 5- to 14-membered heteroaryl or 5- to 14-membered heteroaryl substituted by one or more than one $R^{3b}$, 5- to 14-membered heteroaryl in the 5- to 14-membered heteroaryl and 5- to 14-membered heteroaryl substituted by one or more than one $R^{3b}$ is 5- to 10-membered heteroaryl;

m) when $R^3$ is 5- to 14-membered heteroaryl or 5- to 14-membered heteroaryl substituted by one or more than one $R^{3b}$, 5- to 14-membered heteroaryl in the 5- to 14-membered heteroaryl and 5- to 14-membered heteroaryl substituted by one or more than one $R^{3b}$ is not fused with other rings;

n) when $R^{3a}$ and $R^{3b}$ are independently halogen, the halogen is fluorine, chlorine, bromine, or iodine;

o) when $R^{3a}$ and $R^{3b}$ are independently $C_1$-$C_6$ alkyl, $C_1$-$C_6$ alkyl-O-, $C_1$-$C_6$ alkyl-S-, Ci-$C_6$ alkyl substituted by one or more than one $R^{3-a}$, $C_1$-$C_6$ alkyl-O- substituted by one or more than one $R^{3-b}$, or $C_1$-$C_6$ alkyl-S- substituted by one or more than one $R^{3-c}$, Ci-$C_6$ alkyl in the Ci-Cs alkyl, Ci-Cs alkyl-O-, $C_1$-$C_6$ alkyl-S-, $C_1$-$C_6$ alkyl substituted by one or more than one $R^{3-a}$, $C_1$-$C_6$ alkyl-O- substituted by one or more than one $R^{3-b}$, and $C_1$-$C_6$ alkyl-S- substituted by one or more than one $R^{3-c}$ is independently $C_1$-$C_4$ alkyl;

p) when $R^{3a}$ and $R^{3b}$ are independently $C_2$-$C_6$ alkynyl or $C_2$-$C_6$ alkynyl substituted by one or more than one $R^{3-e}$, $C_2$-$C_6$ alkynyl in the $C_2$-$C_6$ alkynyl and $C_2$-$C_6$ alkynyl substituted by one or more than one $R^{3-c}$ is $C_2$-$C_4$ alkynyl;

q) when $R^{3-a}$, $R^{3-b}$, $R^{3-c}$, $R^{3-d}$, $R^{3-e}$, and $R^{3-f}$ are independently halogen, the halogen is fluorine, chlorine, bromine, or iodine;

r) when $R^{3a}$ and $R^{3b}$ are independently $C_1$-$C_6$ alkyl substituted by one or more than one $R^{3-a}$, $C_1$-$C_6$ alkyl-O- substituted by one or more than one $R^{3-b}$, $C_1$-$C_6$ alkyl-S-substituted by one or more than one $R^{3-c}$, $C_2$-$C_6$ alkenyl substituted by one or more than one $R^{3-d}$, $C_2$-$C_6$ alkynyl substituted by one or more than one $R^{3-e}$, or 3- to 6-membered cycloalkyl substituted by one or more than one $R^{3-f}$, the number of the substituents is 1, 2, 3, or 4;

s) when $R^{4a}$ and $R^{4b}$ are halogen, the halogen is fluorine, chlorine, bromine, or iodine;

t) all atoms in the nitrogen-containing heterocyclic compound of formula I, the stereoisomer thereof, or the pharmaceutically acceptable salt thereof have atomic masses typically found in nature;

u) $R^5$ is independently H;

v) $R^{6a}$ and $R^{6b}$ are independently H;

w) n is 0, 1, 2, or 3; for example, n is 1, 2, or 3;

x) n1, n2, n3, n4, or n5 are each independently 1;

y) the pharmaceutically acceptable salt is a pharmaceutically acceptable base addition salt or a pharmaceutically acceptable acid addition salt; the pharmaceutically acceptable base addition salt is, for example, a lithium salt, a sodium salt, a potassium salt, a calcium salt, an aluminum salt, a magnesium salt, a zinc salt, a bismuth salt, an ammonium salt, a diethanolamine salt; the pharmaceutically acceptable acid addition salt is, for example, an inorganic acid salt or an organic acid salt (such as trifluoroacetate, hydrochloride);

z) the nitrogen-containing heterocyclic compound of formula I is not any one of the following compounds:

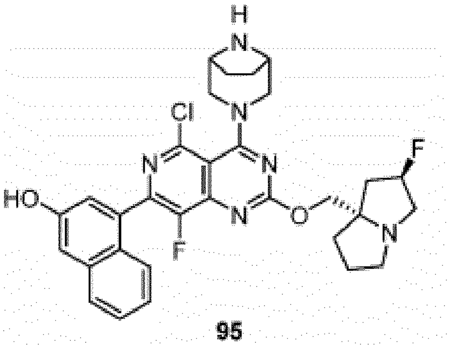

95

,

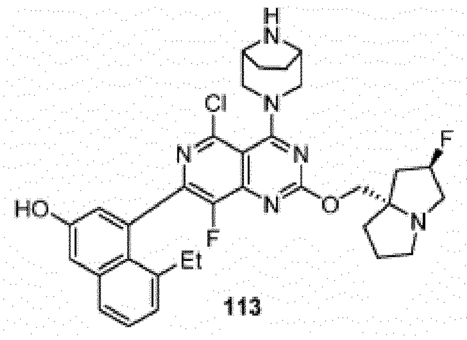

113

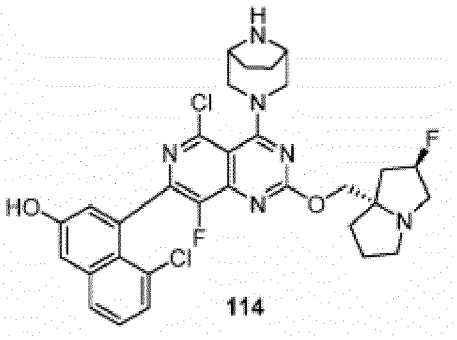

114

,

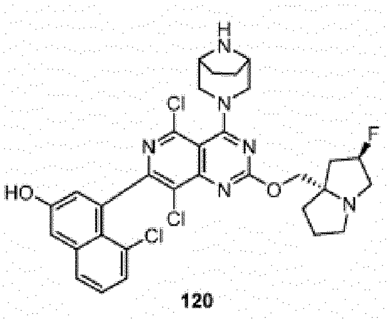

120

,

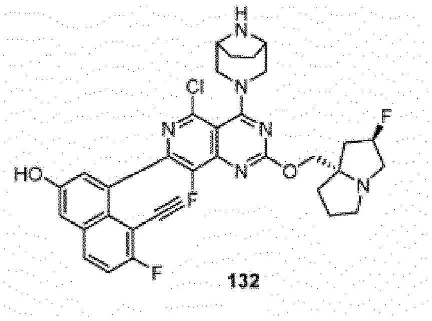

132

,

and

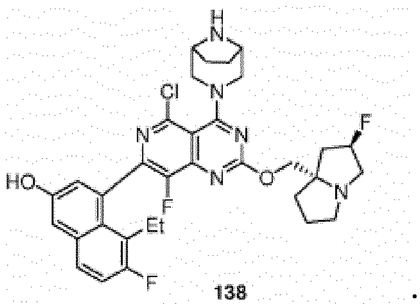

138

;

aa) the nitrogen-containing heterocyclic compound of formula I is not any one of the following compounds:

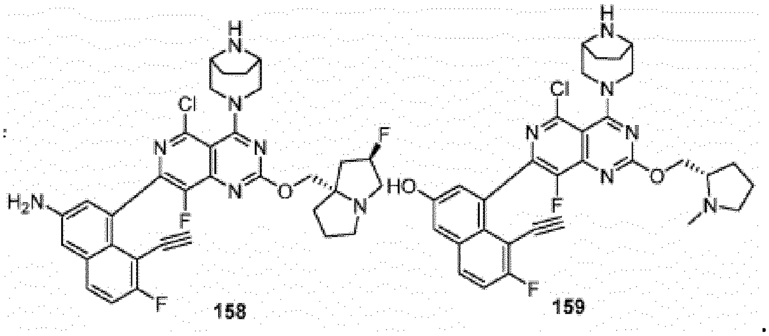

158 159.

3. The nitrogen-containing heterocyclic compound of formula **I**, the stereoisomer thereof, or the pharmaceutically acceptable salt thereof according to claim 2, wherein the nitrogen-containing heterocyclic compound of formula I, the stereoisomer thereof, or the pharmaceutically acceptable salt thereof satisfies one or more than one of the following conditions:

bb) in ring A, the 5- to 7-membered monocyclic heterocycloalkyl is

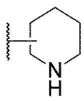

(for example,

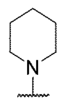

),

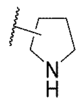

(for example,

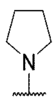 or 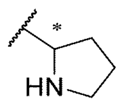,

for another example,

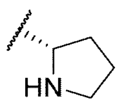

),

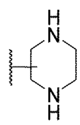

(for example,

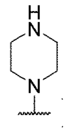

), or

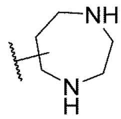

(for example,

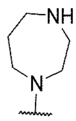

); "*" means that when a carbon atom with "*" is a chiral carbon atom, it is in an R configuration, an S configuration, or a mixture thereof;
cc) in ring A, the 6- to 8-membered fused heterocycloalkyl is

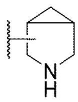

(for example,

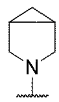

) or

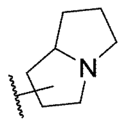

(for example,

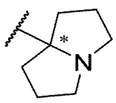,

for another example,

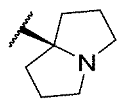

); "*" means that when a carbon atom with "*" is a chiral carbon atom, it is in an R configuration, an S configuration, or a mixture thereof;

dd) in ring A, the 6- to 8-membered bridged heterocycloalkyl is

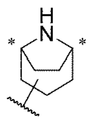

(for example,

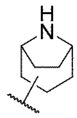

,

for another example,

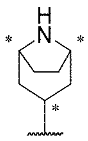

or

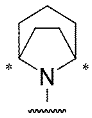

,

for yet another example,

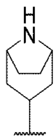

,

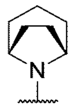

,

or

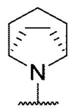

),

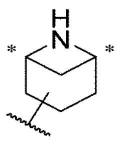

(for example,

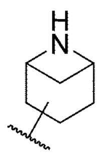

,

for another example,

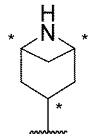,

for yet another example,

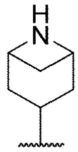

),

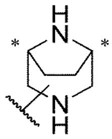

(for example,

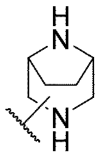,

for another example,

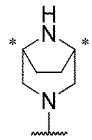,

for yet another example,

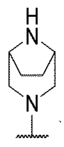

), or

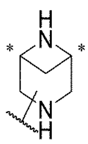

(for example,

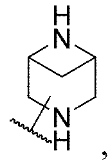,

for another example,

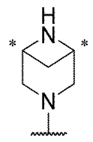,

for yet another example,

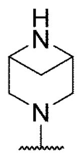

), "*" means that when a carbon atom with "*" is a chiral carbon atom, it is in an R configuration, an S configuration, or a mixture thereof;
ee) in ring A, the 7- to 10-membered spiro heterocycloalkyl is

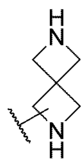

(for example,

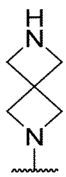

),

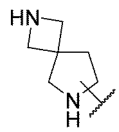

(for example,

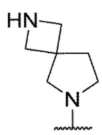

),

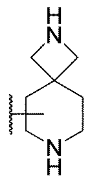

(for example,

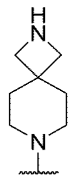

),

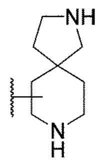

(for example,

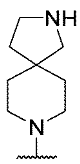

),

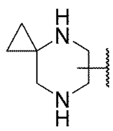

(for example,

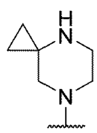

),

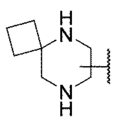

(for example,

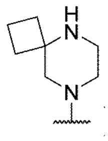

), or

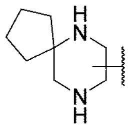

(for example,

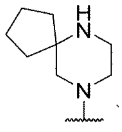

);

ff) in ring A, the 6- to 8-membered bridged heterocycloalkenyl is

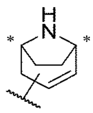 or 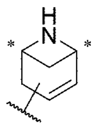,

for example,

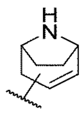 or 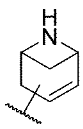,

for another example,

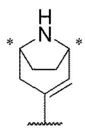 or 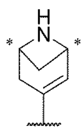,

for yet another example,

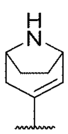 or 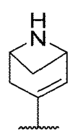;

"*" means that when a carbon atom with "*" is a chiral carbon atom, it is in an R configuration, an S configuration,

or a mixture thereof;
gg) when ring A is 4- to 10-membered heterocycloalkenyl, the number of heteroatoms of the 4- to 10-membered heterocycloalkenyl is 1, wherein the heteroatom is N;
hh) when ring A is 4- to 10-membered heterocycloalkenyl, the 4- to 10-membered heterocycloalkenyl is connected to the pyrimidine ring shown in formula I through a C atom;
ii) when $R^1$ is halogen, the halogen is fluorine;
jj) in $R^2$, the 5- to 7-membered monocyclic heterocycloalkyl is

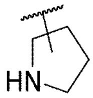,

for example,

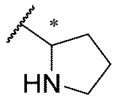,

"*" means that when a carbon atom with "*" is a chiral carbon atom, it is in an R configuration, an S configuration, or a mixture thereof; for another example,

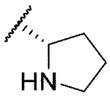, 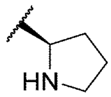,

or a mixture thereof;
kk) in $R^2$, the 6- to 8-membered fused heterocycloalkyl is

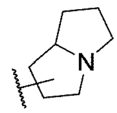,

for example,

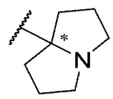,

"*" means that when a carbon atom with "*" is a chiral carbon atom, it is in an R configuration, an S configuration, or a mixture thereof; for another example,

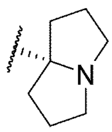, 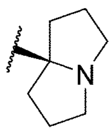,

or a mixture thereof;
ll) when $R^{2a}$, $R^{2b}$, $R^{2c}$, $R^{2d}$, and $R^{2e}$ are each independently halogen, the halogen is fluorine;
mm) when $R^3$ is $C_6$-$C_{10}$ aryl or $C_6$-$C_{10}$ aryl substituted by one or more than one $R^{3a}$, $C_6$-$C_{10}$ aryl in the $C_6$-$C_{10}$ aryl and $C_6$-$C_{10}$ aryl substituted by one or more than one $R^{3a}$ is

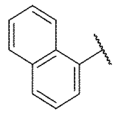

;

nn) when $R^3$ is 5- to 14-membered heteroaryl or 5- to 14-membered heteroaryl substituted by one or more than one $R^{3b}$, 5- to 14-membered heteroaryl in the 5- to 14-membered heteroaryl and 5- to 14-membered heteroaryl substituted by one or more than one $R^{3b}$ is pyridyl, pyrimidinyl, quinolinyl, quinazolinyl, benzothienyl, benzothiazolyl, or indazolyl, and for example,

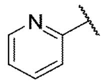

;

oo) when $R^{3a}$ and $R^{3b}$ are independently halogen, the halogen is fluorine or chlorine;

pp) when $R^{3a}$ and $R^{3b}$ are independently $C_1$-$C_6$ alkyl, $C_1$-$C_6$ alkyl-O-, $C_1$-$C_6$ alkyl-S-, Ci-$C_6$ alkyl substituted by one or more than one $R^{3-a}$, $C_1$-$C_6$ alkyl-O- substituted by one or more than one $R^{3-b}$, or $C_1$-$C_6$ alkyl-S- substituted by one or more than one $R^{3-c}$, Ci-$C_6$ alkyl in the $C_1$-$C_6$ alkyl, $C_1$-$C_6$ alkyl-O-, $C_1$-$C_6$ alkyl-S-, $C_1$-$C_6$ alkyl substituted by one or more than one $R^{3-a}$, $C_1$-$C_6$ alkyl-O- substituted by one or more than one $R^{3-b}$, and $C_1$-$C_6$ alkyl-S-substituted by one or more than one $R^{3-c}$ is independently methyl or ethyl;

qq) when $R^{3a}$ and $R^{3b}$ are independently $C_2$-$C_6$ alkynyl or $C_2$-$C_6$ alkynyl substituted by one or more than one $R^{3-e}$, $C_2$-$C_6$ alkynyl in the $C_2$-$C_6$ alkynyl and $C_2$-$C_6$ alkynyl substituted by one or more than one $R^{3-e}$ is ethynyl;

rr) when $R^{3-a}$, $R^{3-b}$, $R^{3-c}$, $R^{3-d}$, $R^{3-e}$, and $R^{3-f}$ are independently halogen, the halogen is fluorine or chlorine;

ss) when $R^{3a}$ and $R^{3b}$ are independently $C_1$-$C_6$ alkyl substituted by one or more than one $R^{3-a}$, $C_1$-$C_6$ alkyl-O-substituted by one or more than one $R^{3-b}$, $C_1$-$C_6$ alkyl-S-substituted by one or more than one $R^{3-c}$, $C_2$-$C_6$ alkenyl substituted by one or more than one $R^{3-d}$, $C_2$-$C_6$ alkynyl substituted by one or more than one $R^{3-e}$, or 3- to 6-membered cycloalkyl substituted by one or more than one $R^{3-f}$, the number of substituents is 3;

tt) when $R^{4a}$ and $R^{4b}$ are halogen, the halogen is fluorine or chlorine;

uu) the nitrogen-containing heterocyclic compound of formula I is not any one of the following compounds:

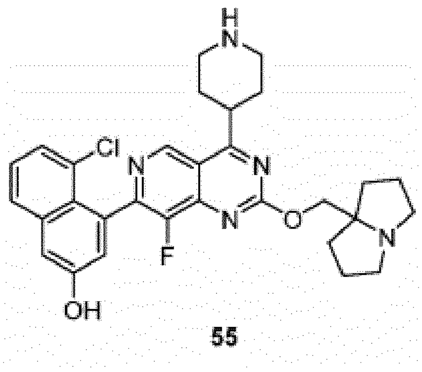

55

,

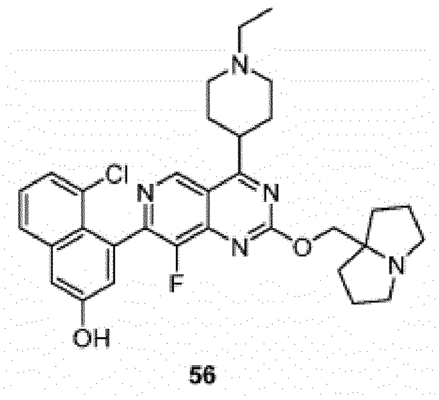

56

,

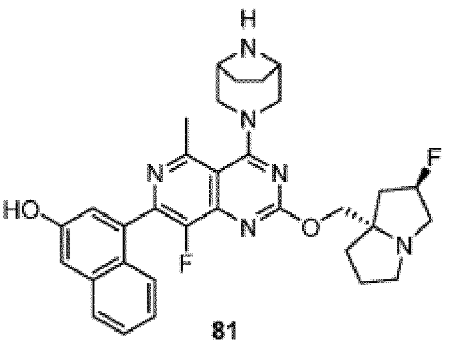

81

,

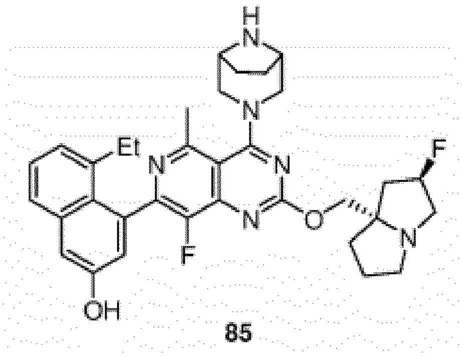

85

,

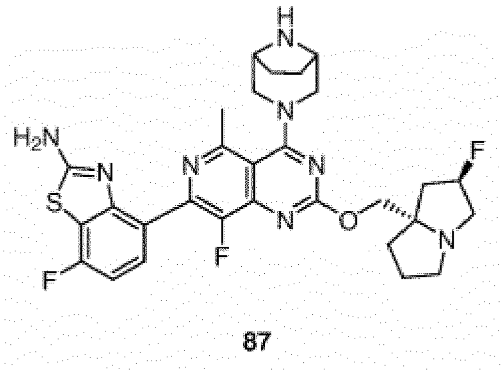

87

,

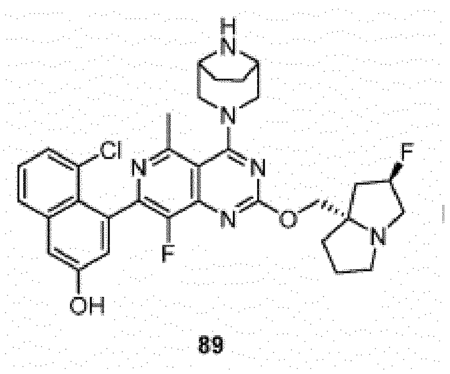

89

,

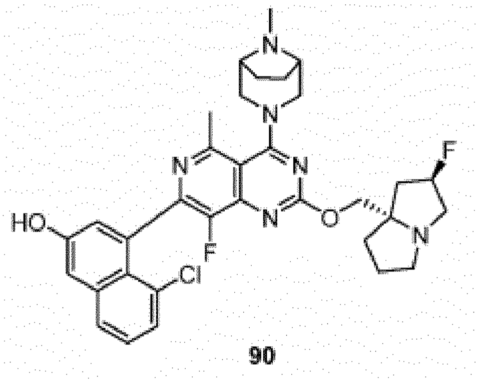

90

,

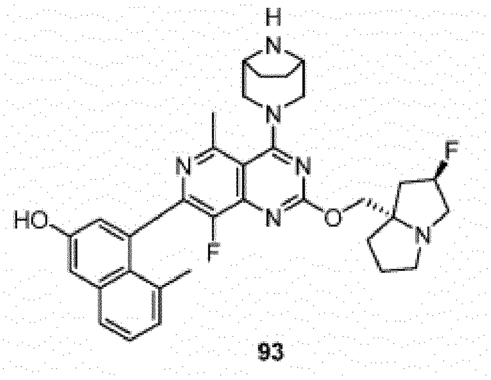

93

,

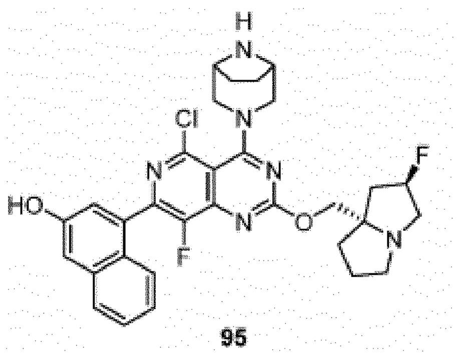

95

,

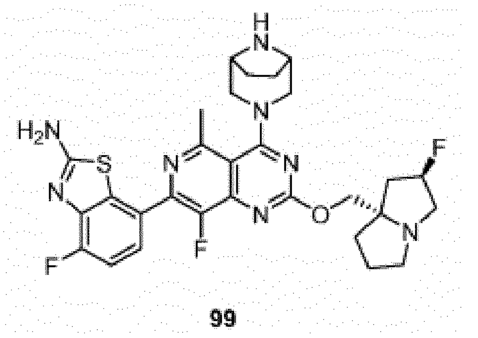

99

,

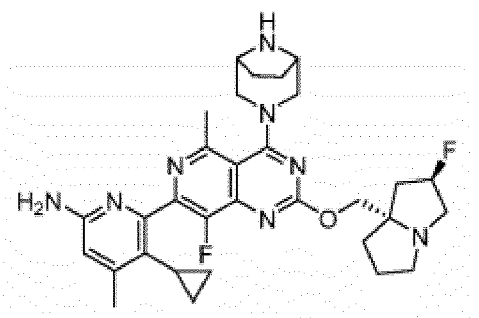

102

,

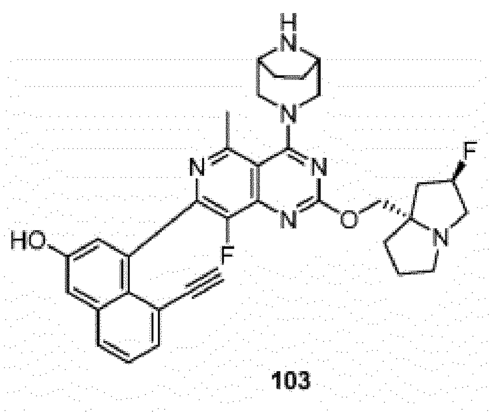

103

,

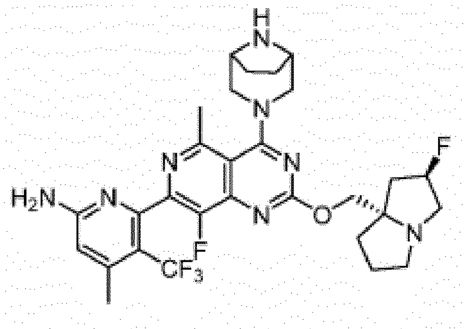

105

,

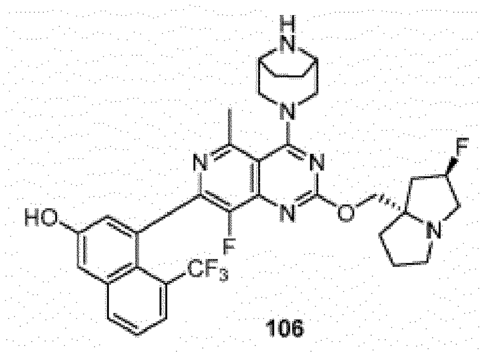

106

,

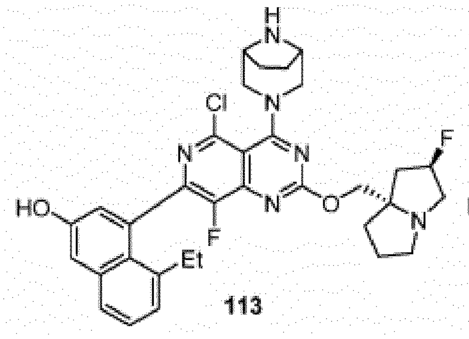

113

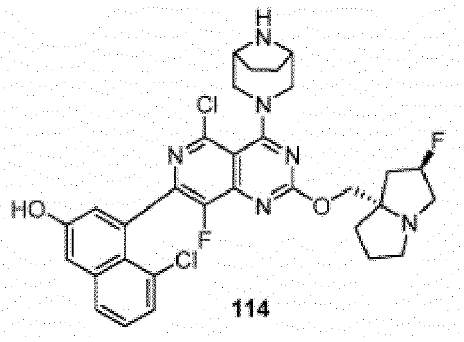

114

,

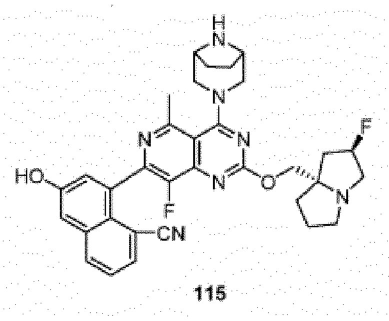

115

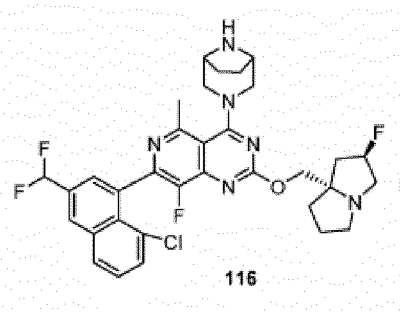

116

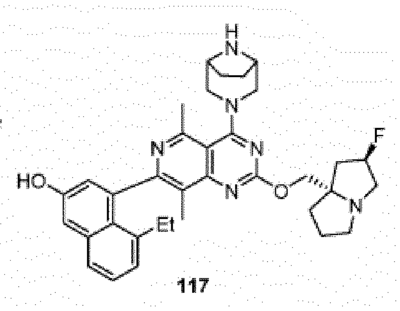

117

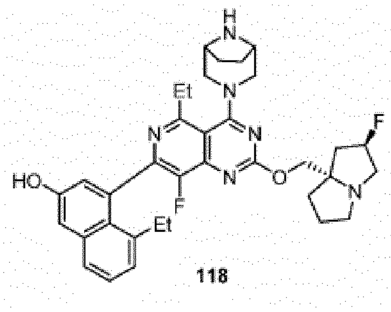

118

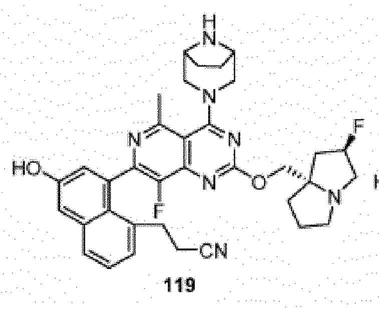

119

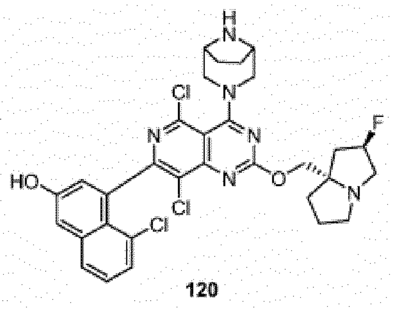

120

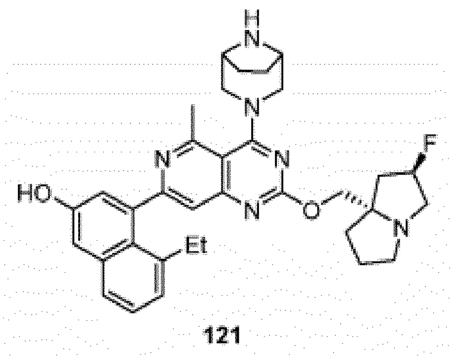

121

,

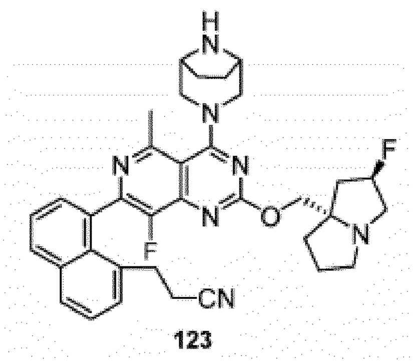

123

,

,

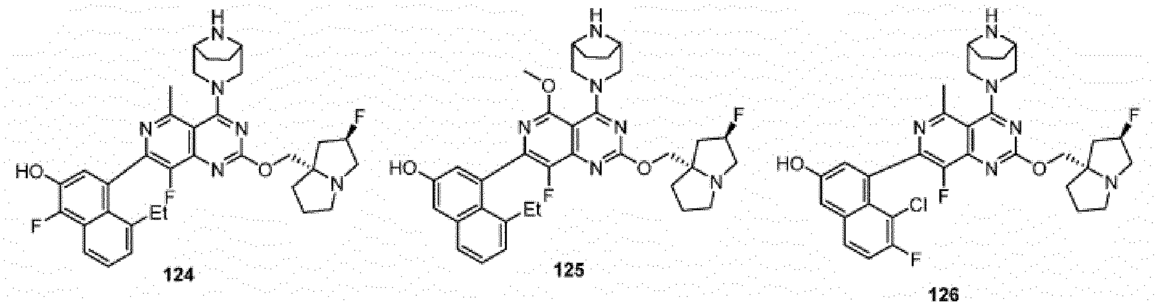

124 125 126

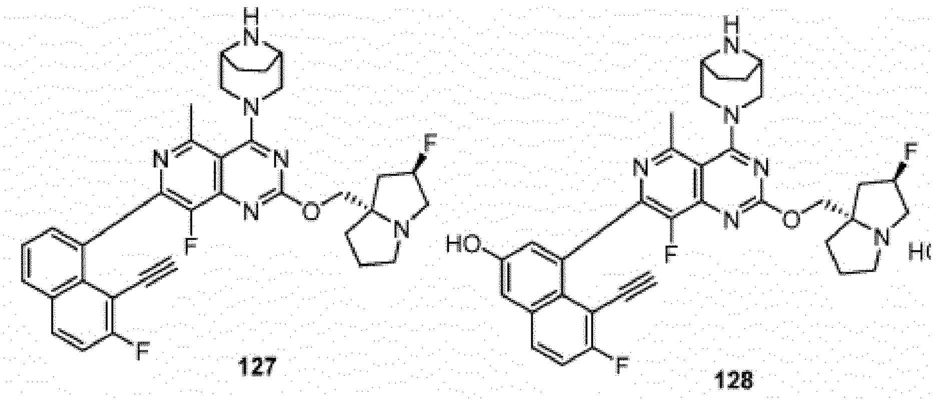

127

,

128

,

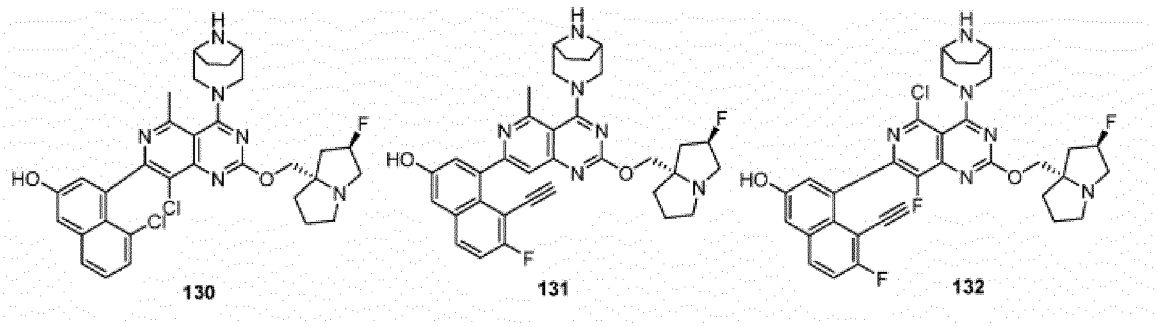

130 131 132

,

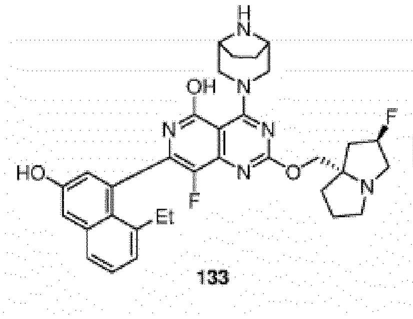
133
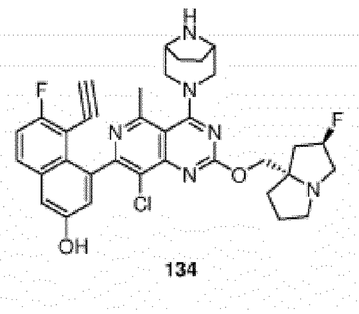
134
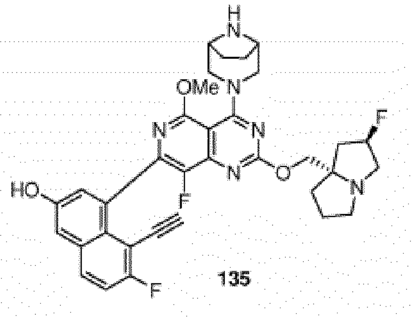
135

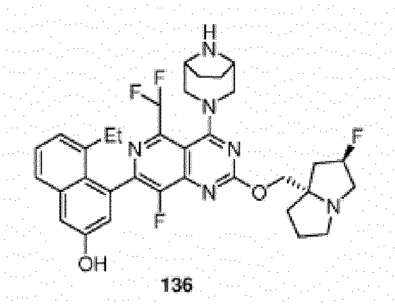
136
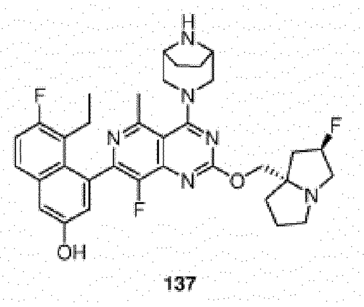
137
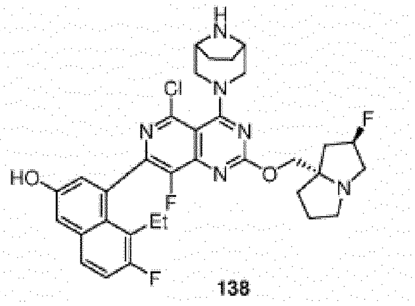
138

, 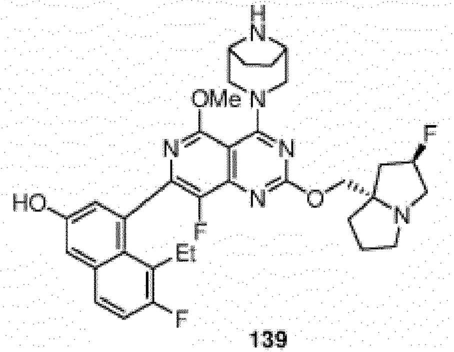
139
, and 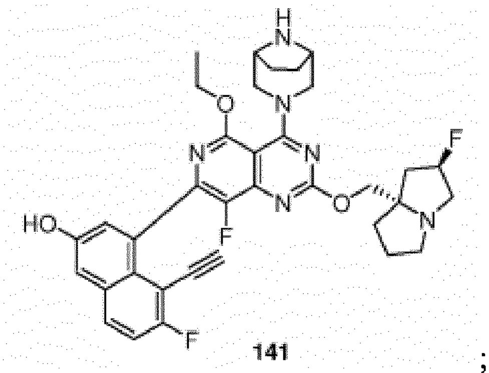
141
;

vv) the nitrogen-containing heterocyclic compound of formula **I** is not any one of the following compounds:

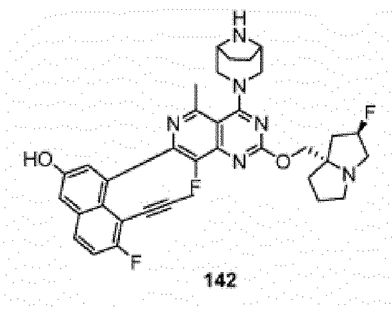
142
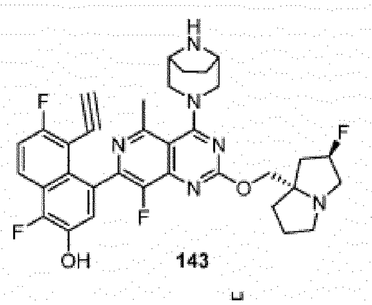
143
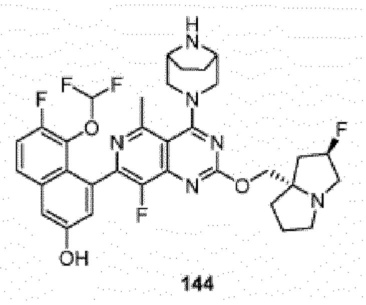
144

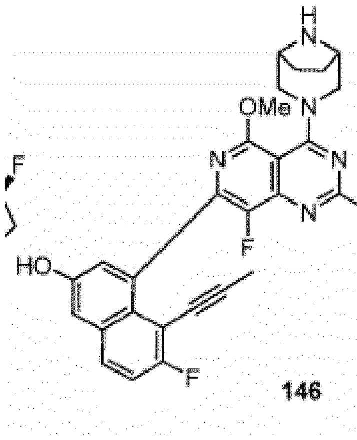
146
,

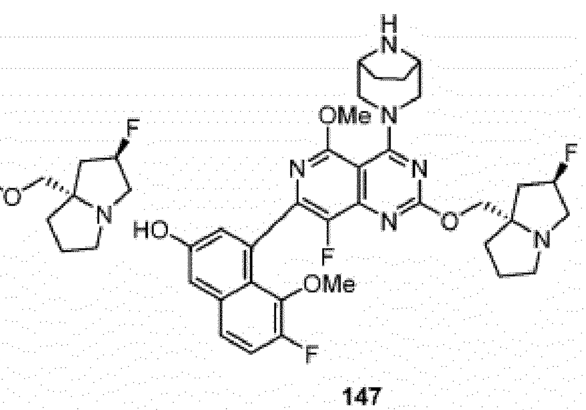
147
,

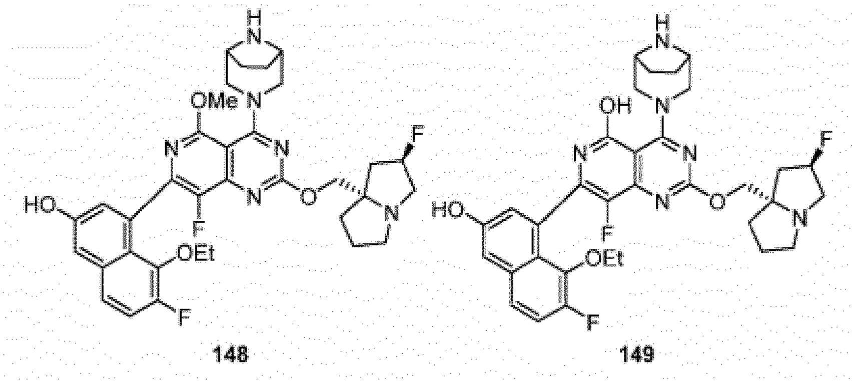
148

149

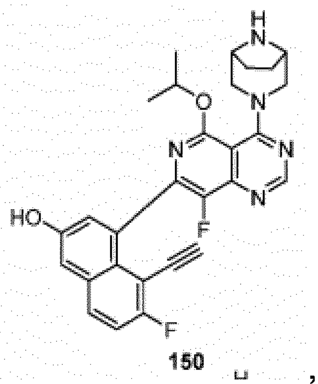
150
,

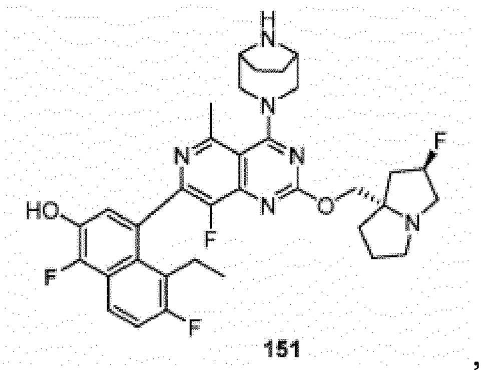
151
,

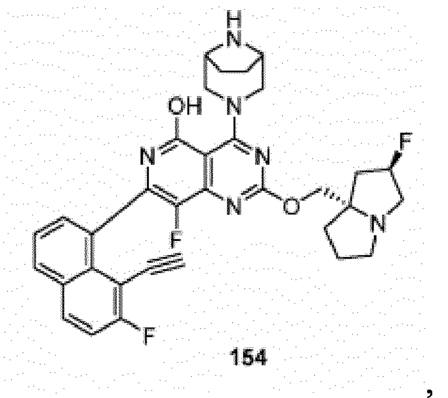
154
,

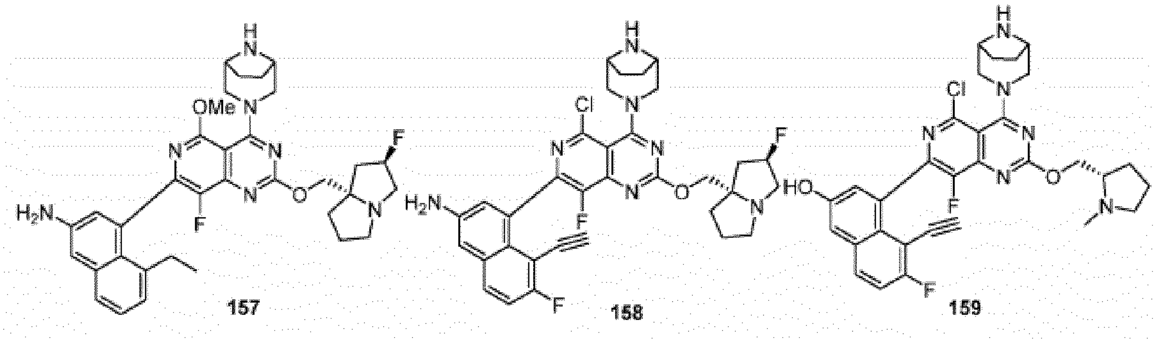
157

158

159

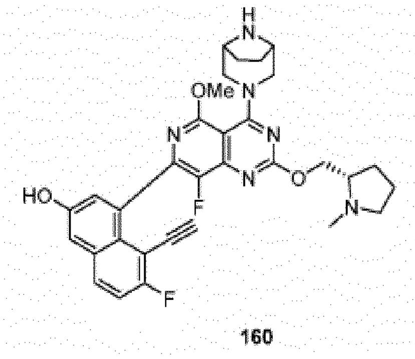

160

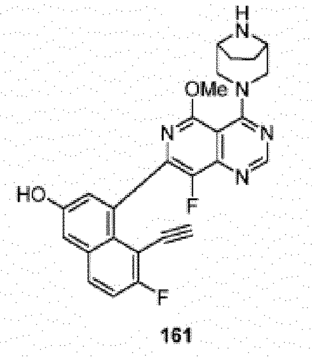

161

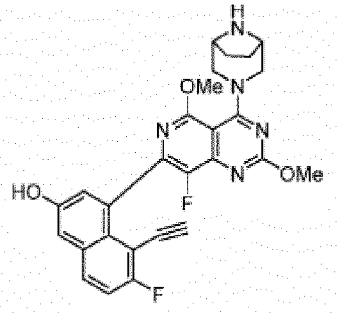

162

,

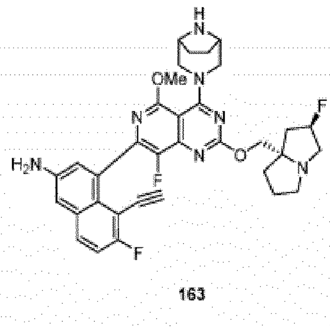

163

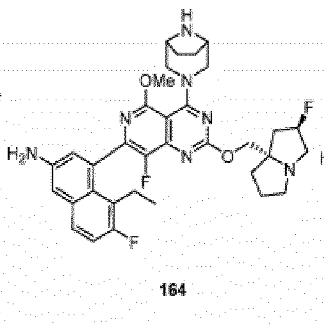

164

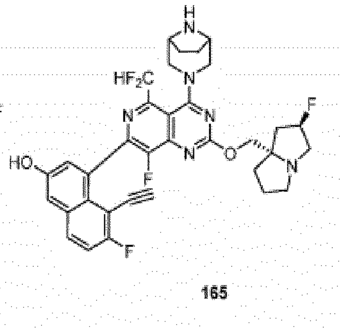

165

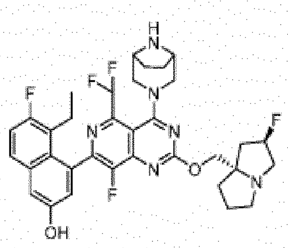

166

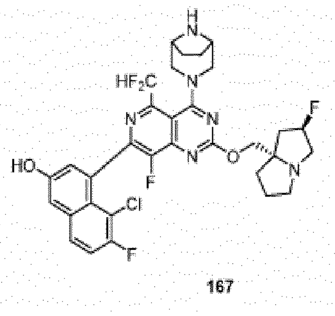

167

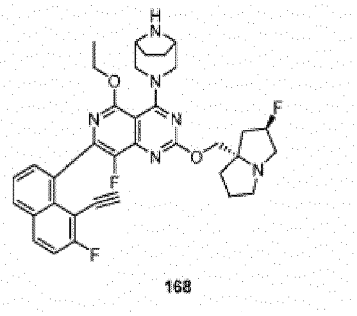

168

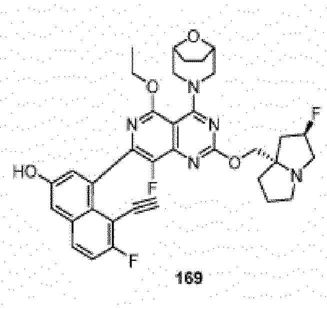

169

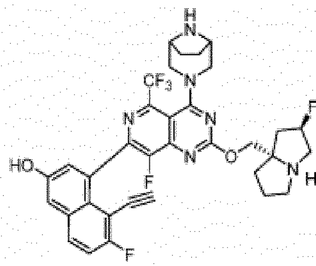

170

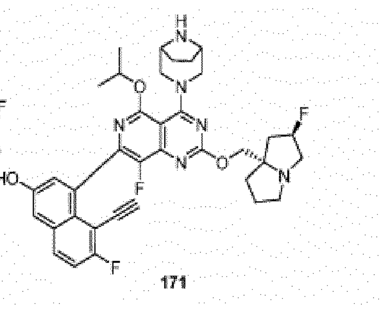

171

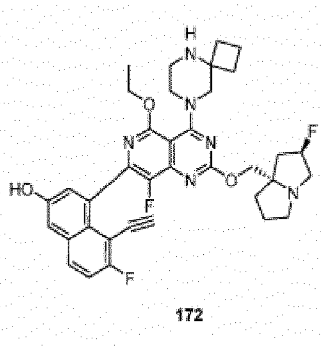

172

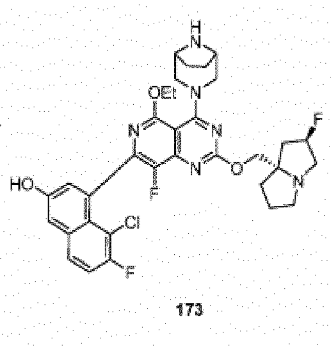

173

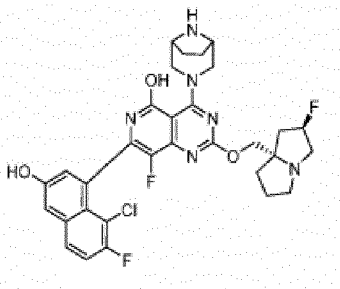

174

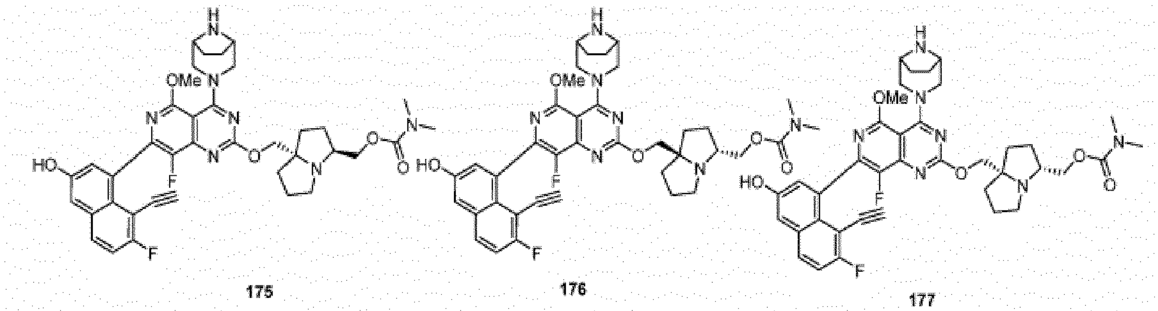

175 176 177

,

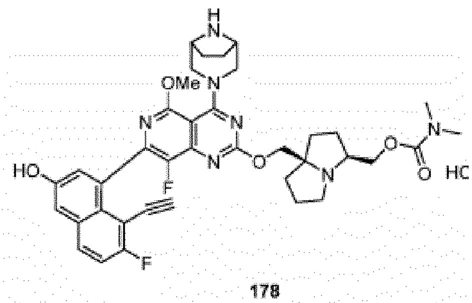

178

,

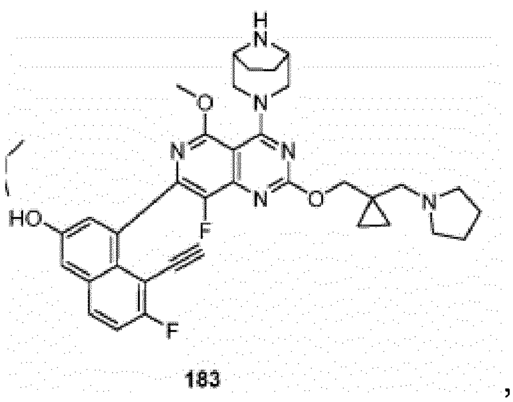

183

,

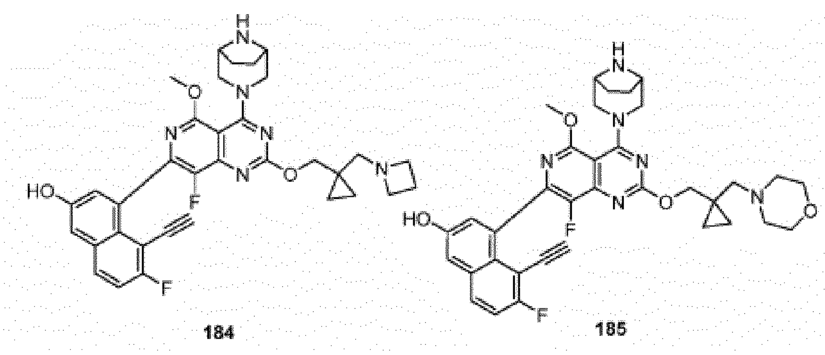

184 185

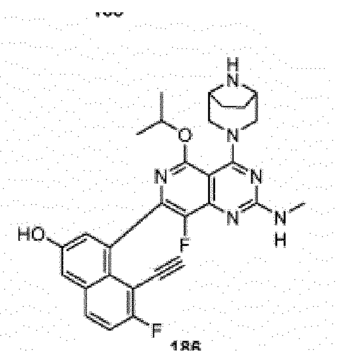

186

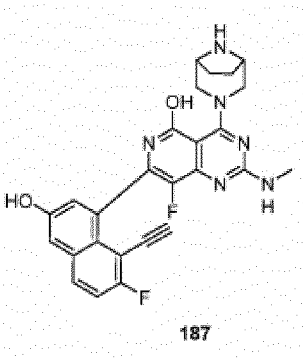

**187**

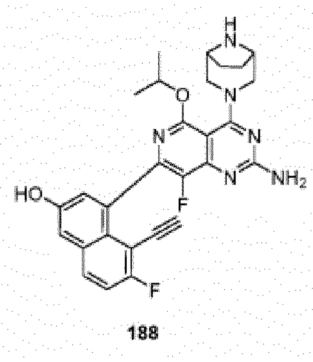

**188**

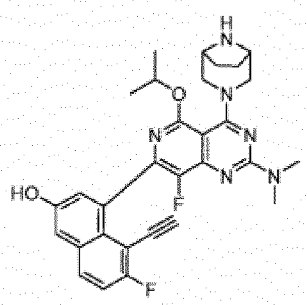

**189**

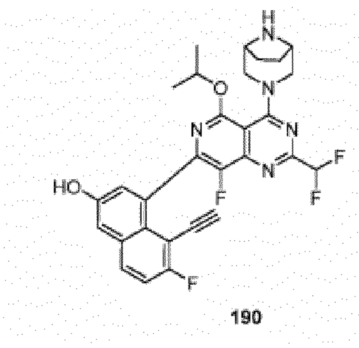

**190**

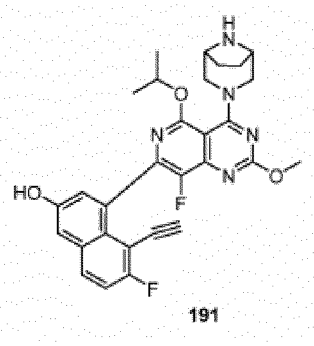

**191**

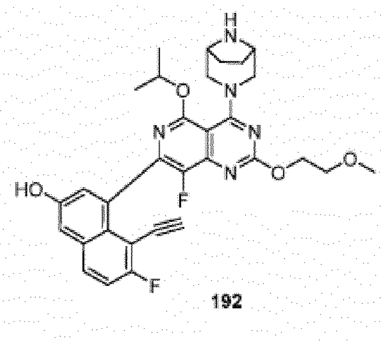

**192**

,

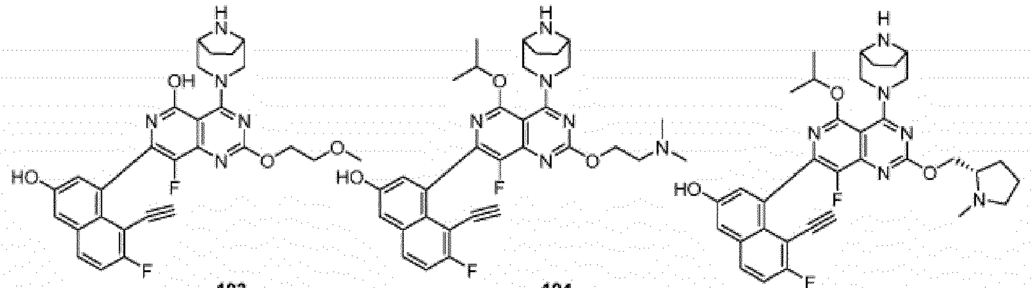

193 194 195

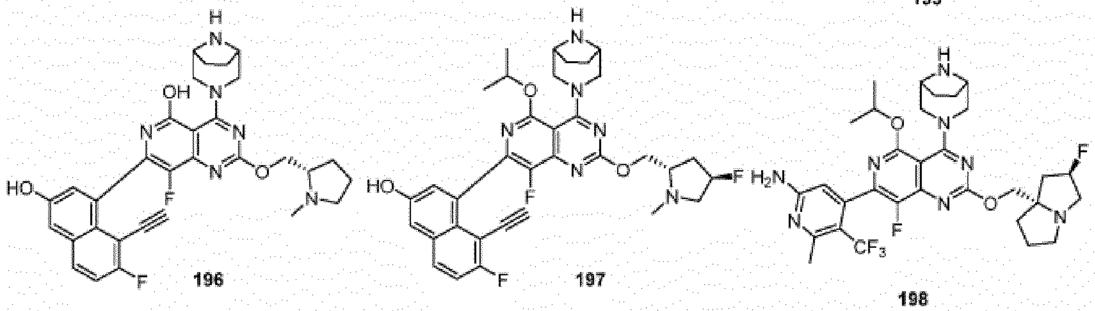

196 197 198

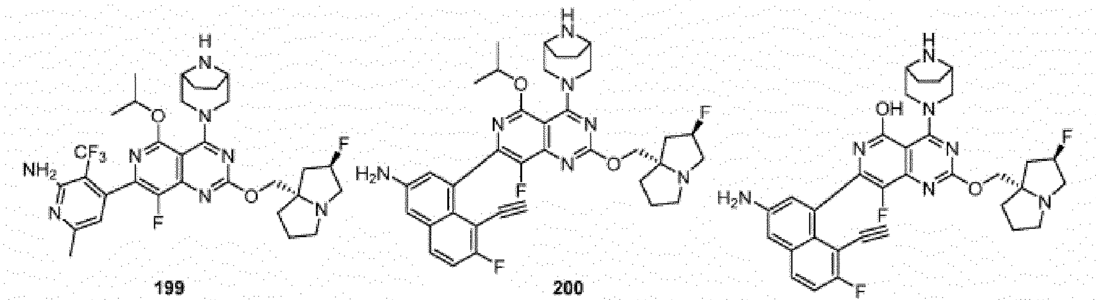

199 200 201

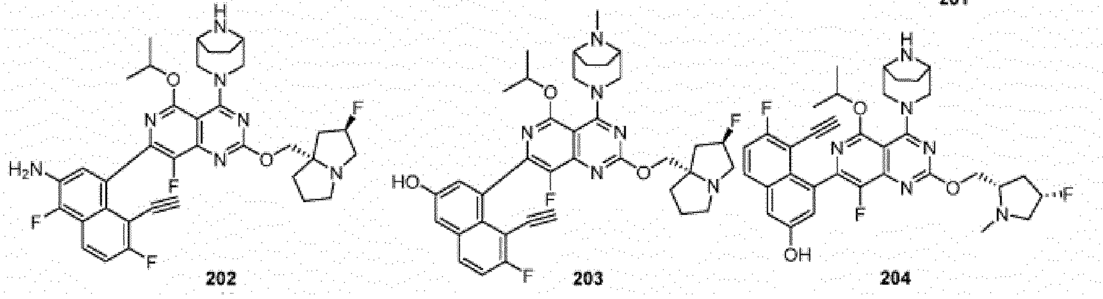

202 203 204 ,

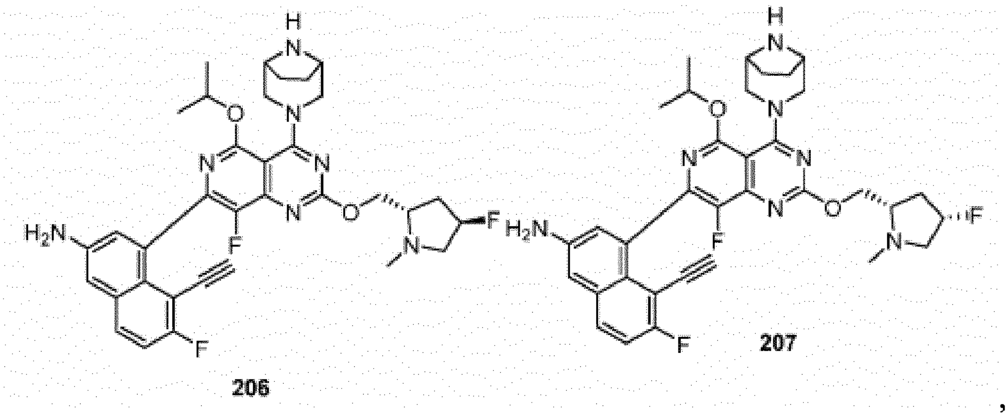

206 207 ,

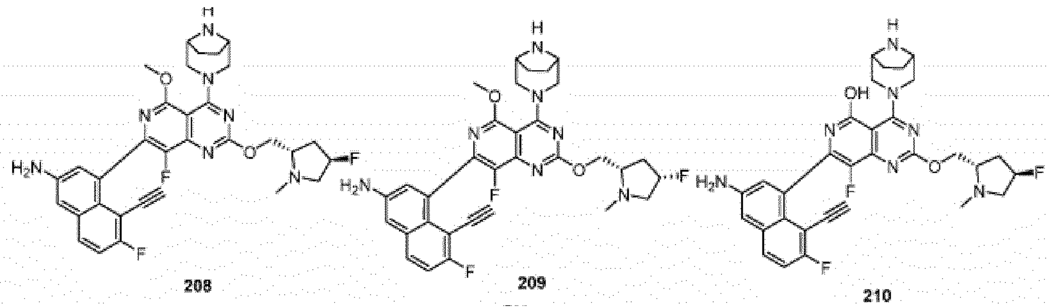

208 209 210

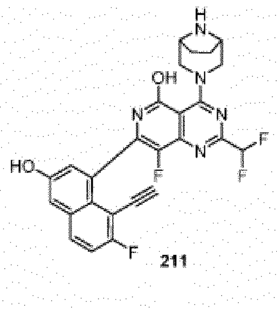

211

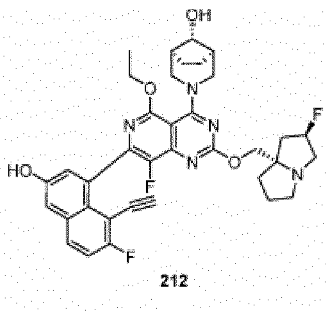

212

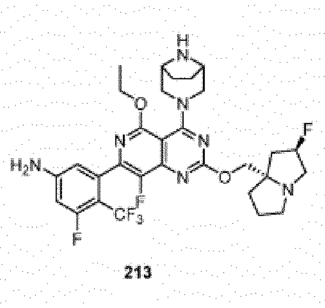

213

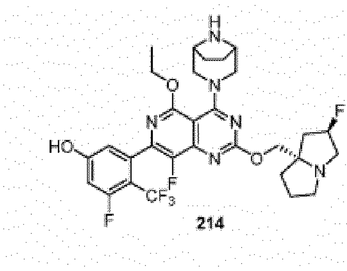

214

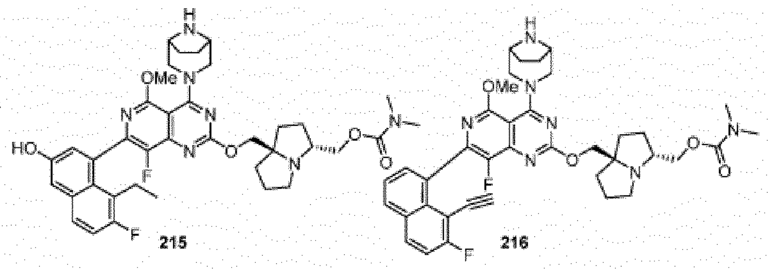

215 216

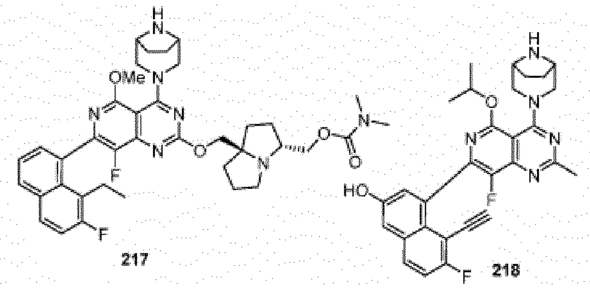

217 218

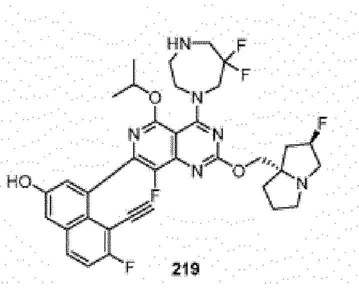

219

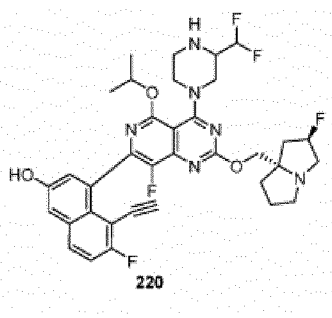

220

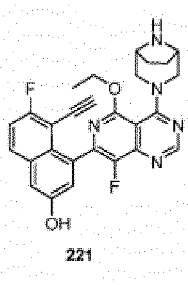

221

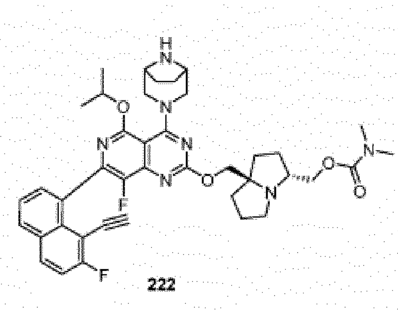

222

,

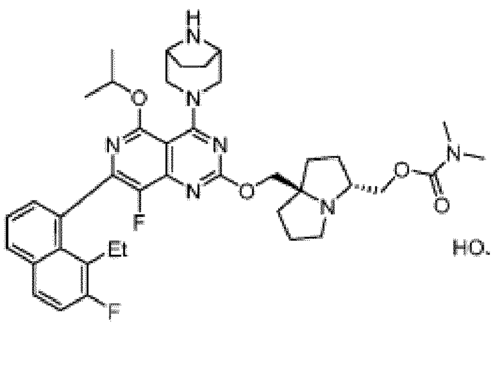

223

,

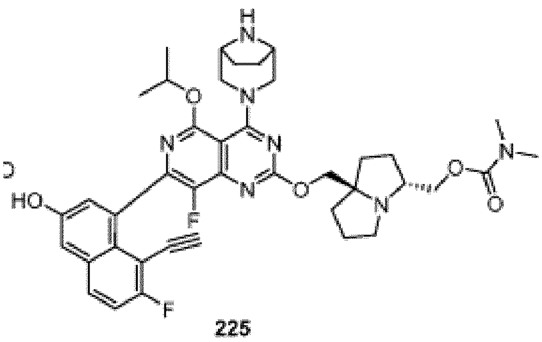

225

,

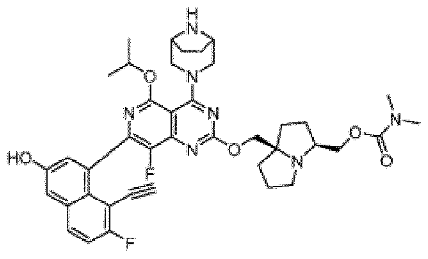

226

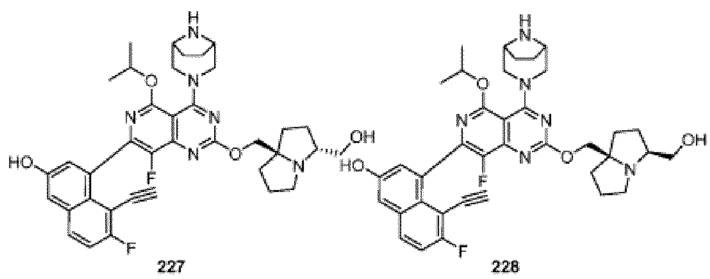

227

228

,

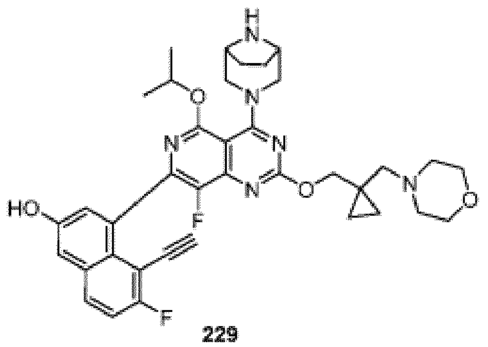

229

,

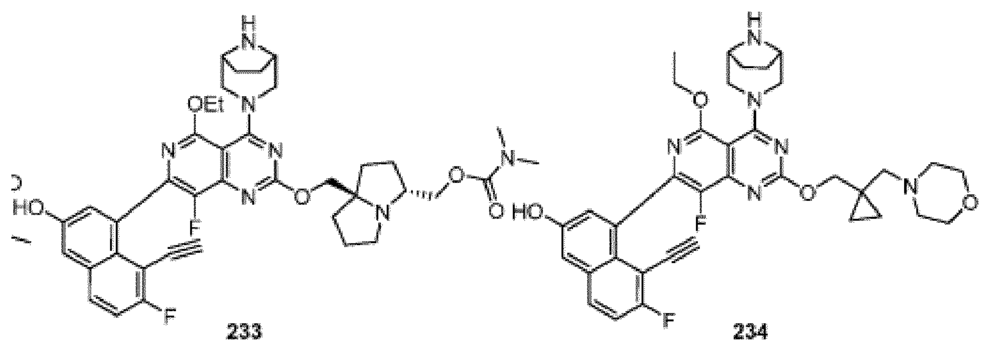

233

234

,

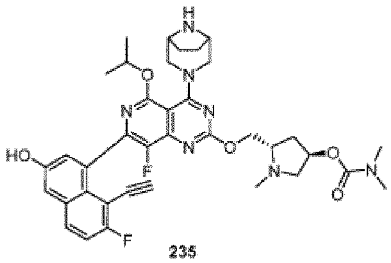

235

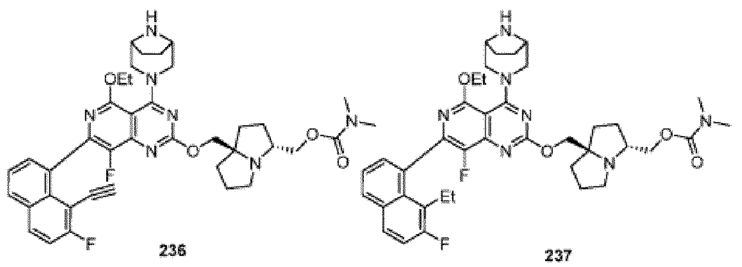

236 237

,

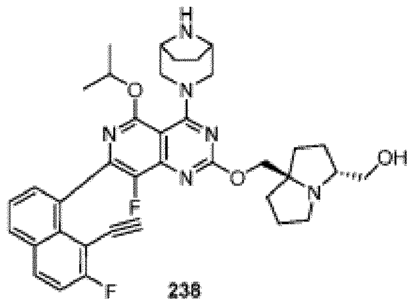

238

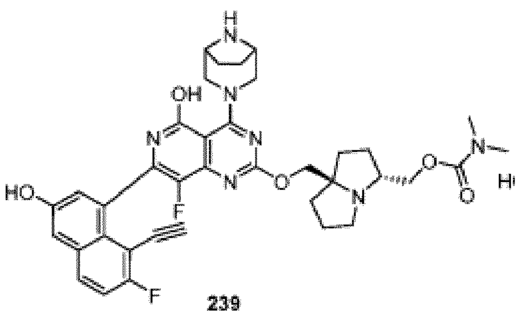

239

,

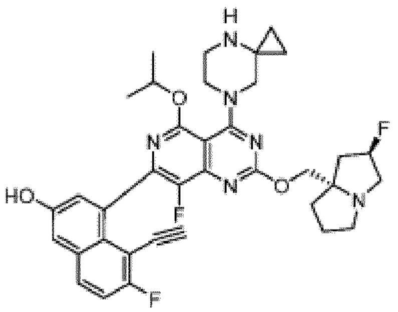

241

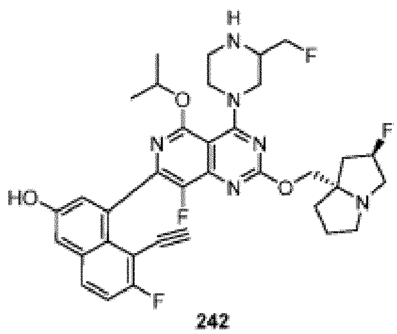

242

,

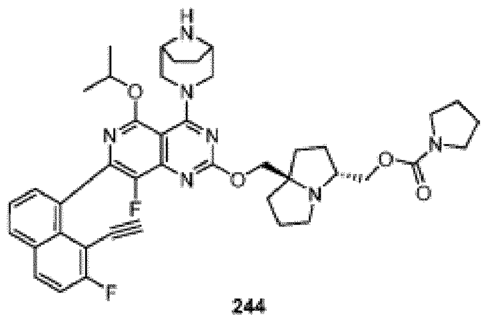

244

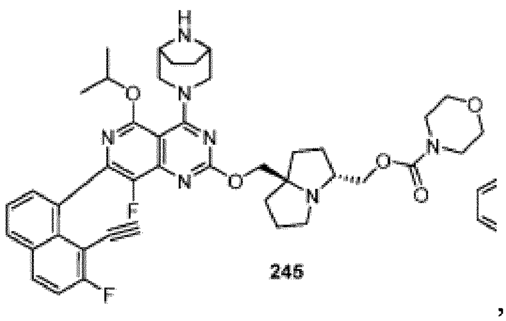

245

,

and

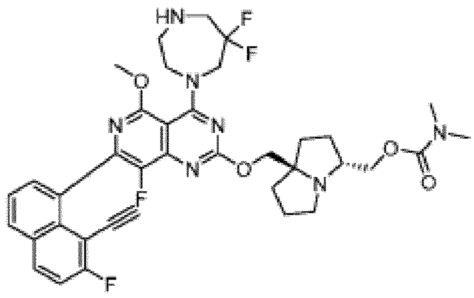

247 ;

ww) the nitrogen-containing heterocyclic compound of formula **I** is not any one of the following compounds:

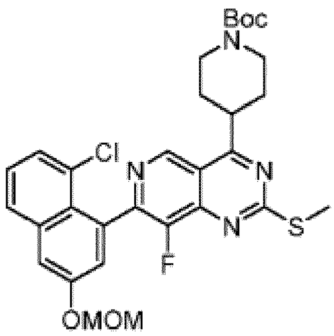

56-6 ,

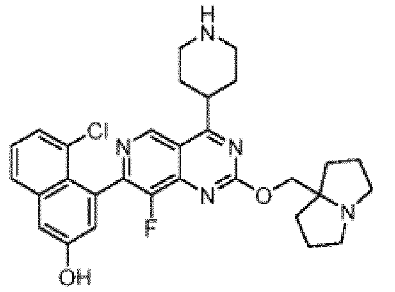

56-9 ,

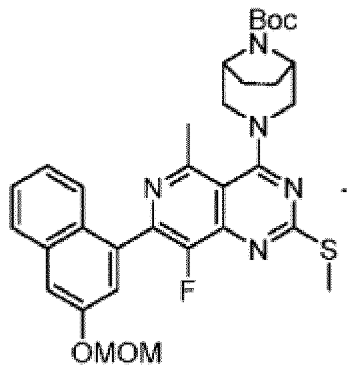

81-9 ,

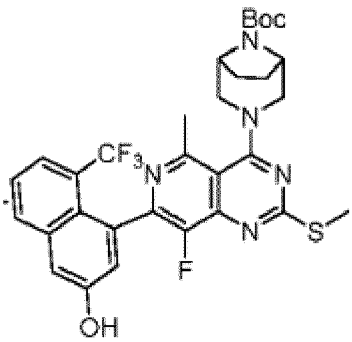

106-4 ,

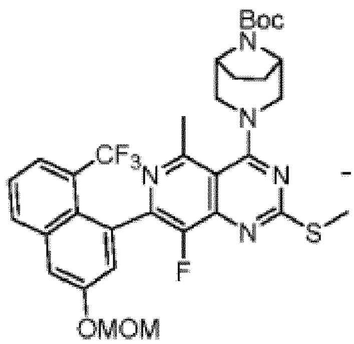

106-5 ,

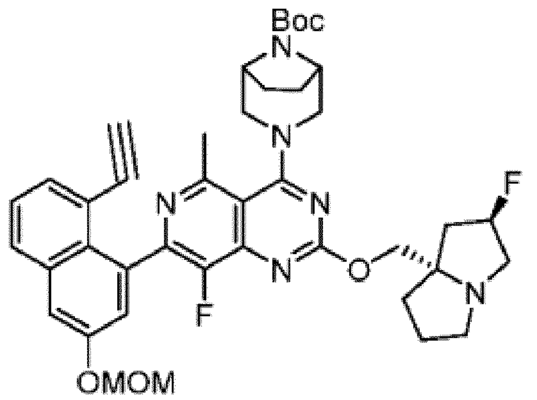

103-8

,

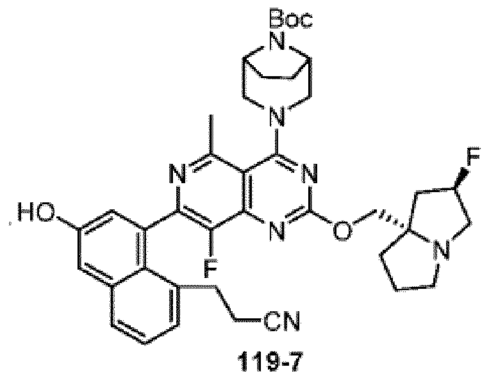

119-7

,

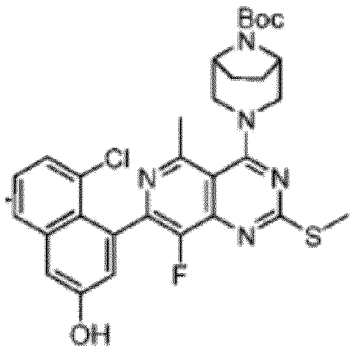

116-1

,

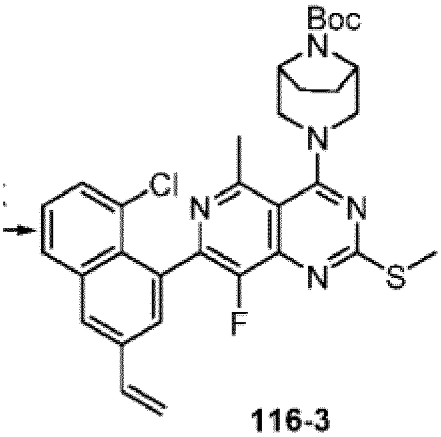

116-3

,

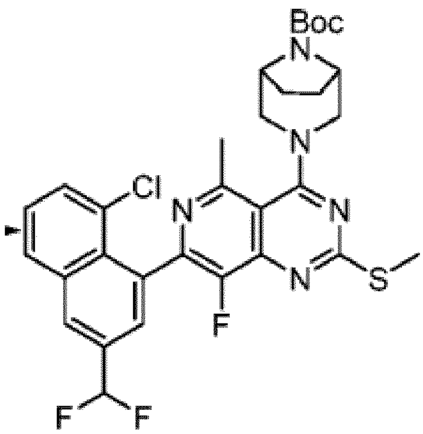

116-5

,

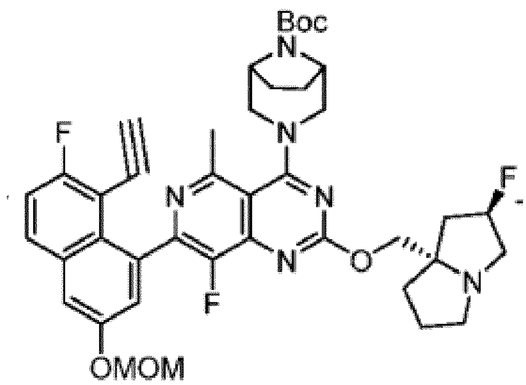

128-15

,

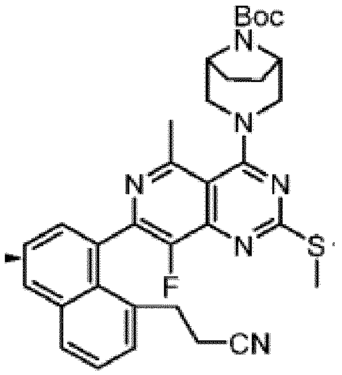

123-5 ,

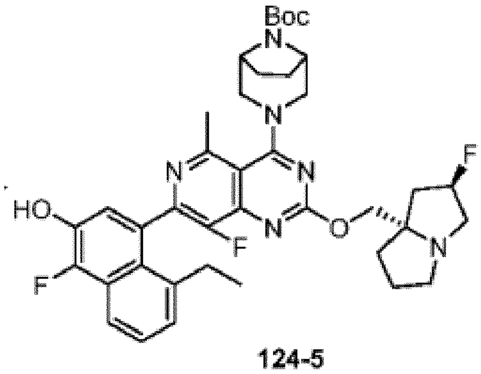

124-5 ,

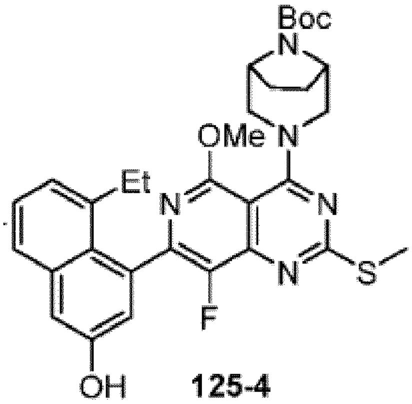

125-4 ,

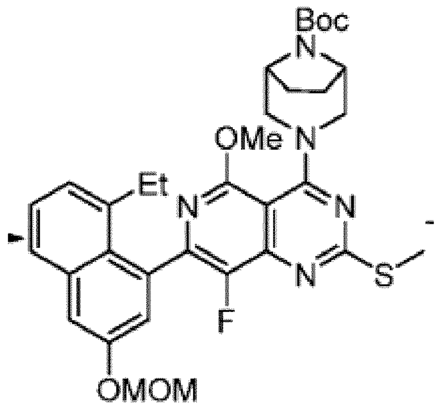

125-5 ,

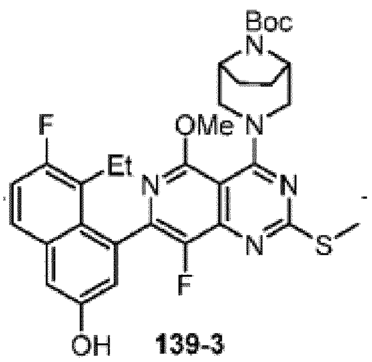

139-3 ,

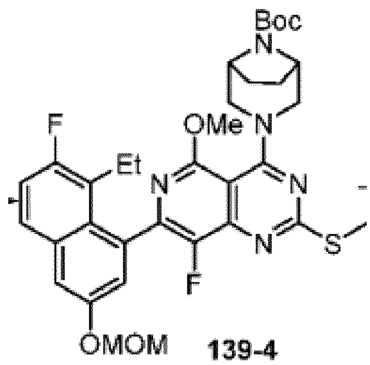

139-4 ,

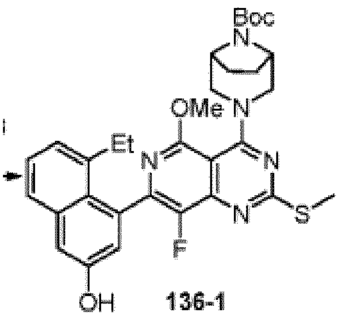

136-1 ,

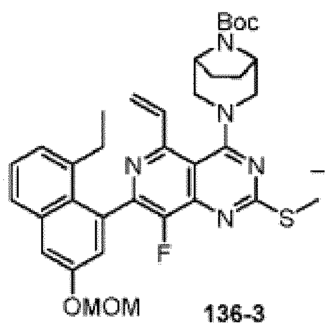

136-3 ,

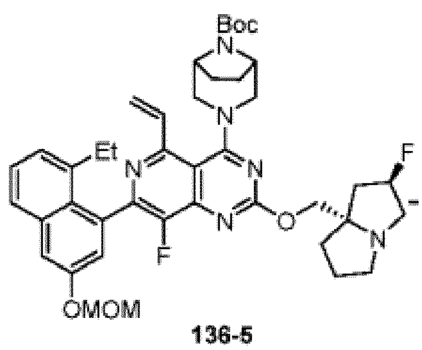

136-5 ,

and

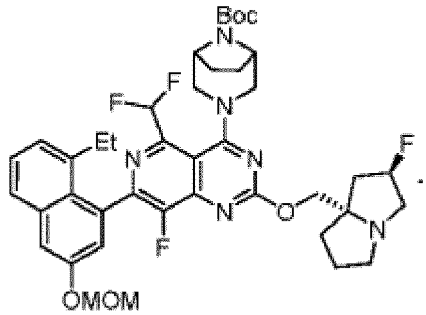

136-7 ;

xx) the nitrogen-containing heterocyclic compound of formula **I** is not any one of the following compounds:

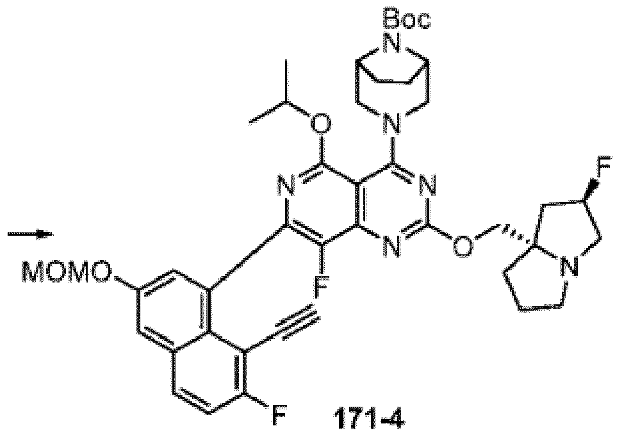

171-4 ,

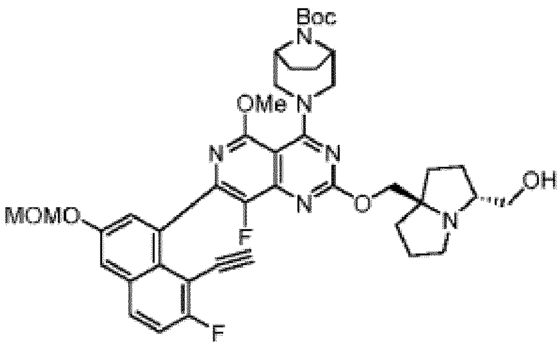

176-8 ,

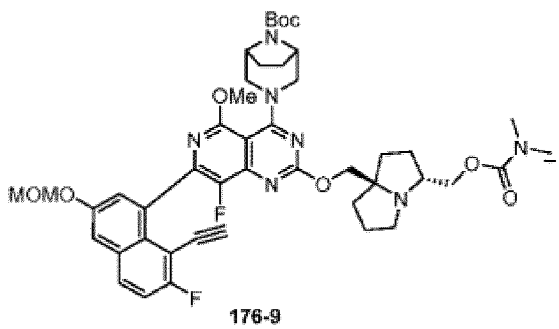

176-9 ,

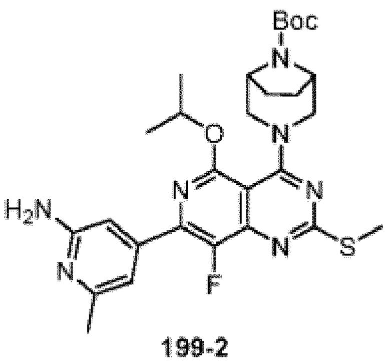

199-2 ,

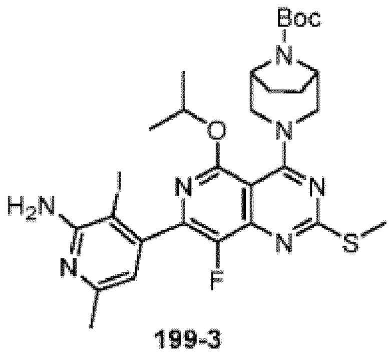

199-3 ,

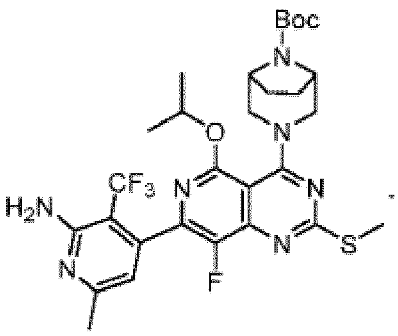

199-4 ,

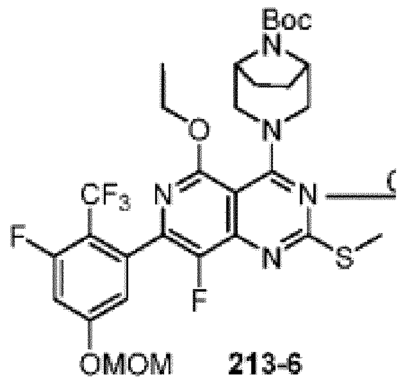

213-6 ,

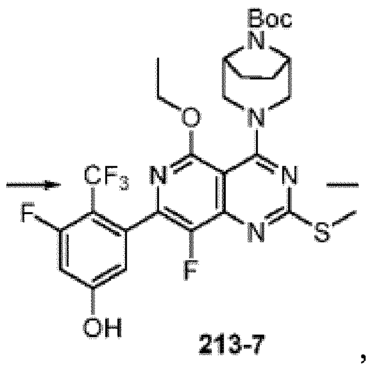

213-7 ,

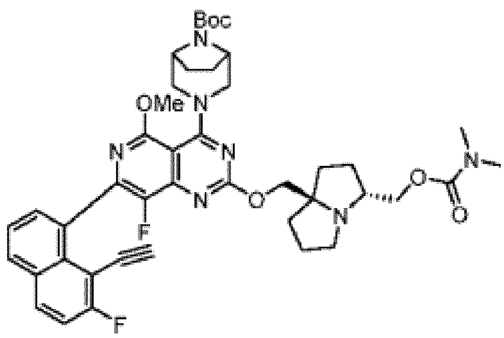

217-3 ,

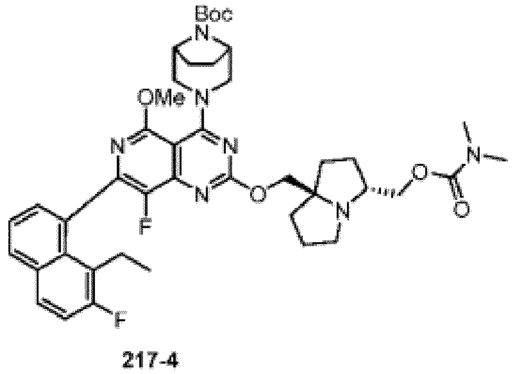

217-4 ,

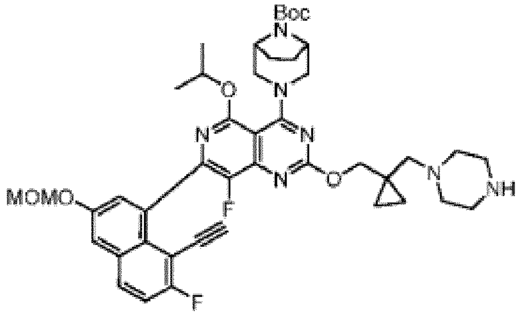

231-3 ,

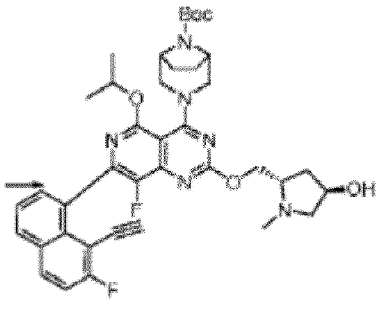

246-7 ,

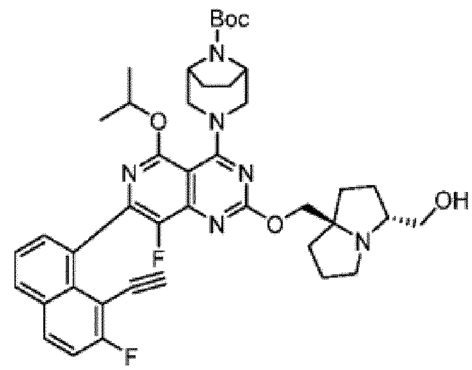

244-2 ,

and

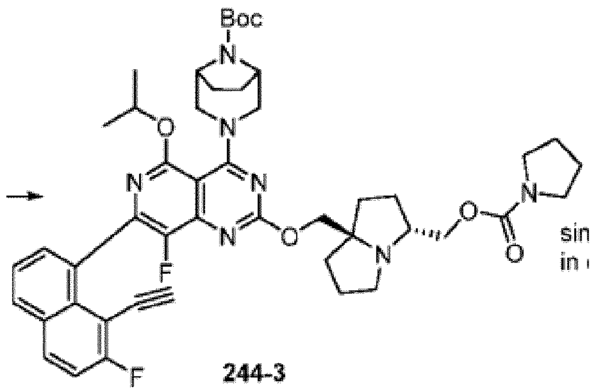

244-3 .

4. The nitrogen-containing heterocyclic compound of formula **I**, the stereoisomer thereof, or the pharmaceutically acceptable salt thereof according to claim 1, wherein the nitrogen-containing heterocyclic compound of formula **I**, the stereoisomer thereof, or the pharmaceutically acceptable salt thereof satisfies one or more than one of the following conditions:

yy) ring A is

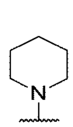 , 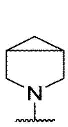 , 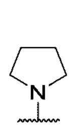 , 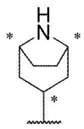 , 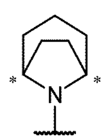 , 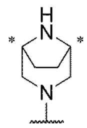 , 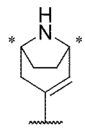 , 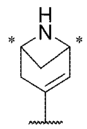 , 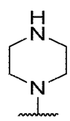 ,

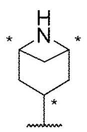 , 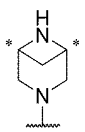 , 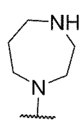 , 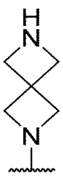 , 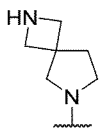 , 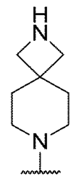 , 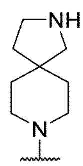 , 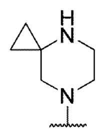 , 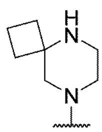 ,

or

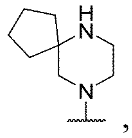 ,

for example,

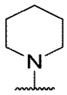 , 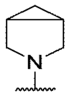 , 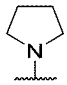 , 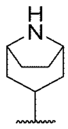 , 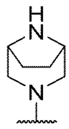 , 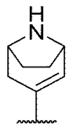 , 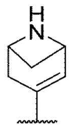 , 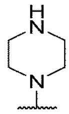 ,

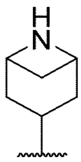 , 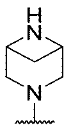 , 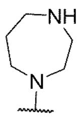 , 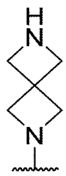 , 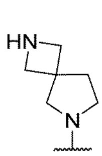 , 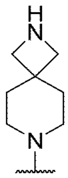 , 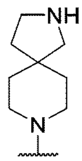 , 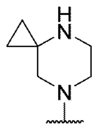 , 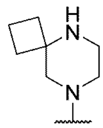 , 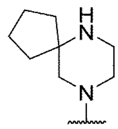 ,

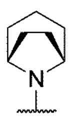 , or 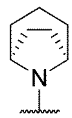 ;

zz) $R^1$ is independently -OH, F, or $-NH_2$;
aaa)L is $-O-(CH_2)-$ or $-O-(CD_2)-$;
bbb) $R^2$ is

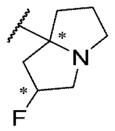 or 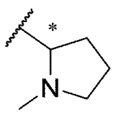 ,

for example,

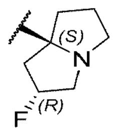 or 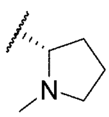 ;

ccc)$R^{3a}$ and $R^{3b}$ are each independently -OH, fluorine, $-CH_3$, $-CF_3$, ethyl, ethynyl,

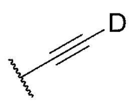 ,

or $-NH_2$;
ddd) $R^{4a}$ and $R^{4b}$ are independently H, F, Cl, or $-NH_2$;
eee)$R^3$ is

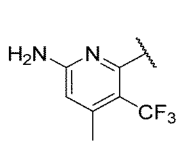, 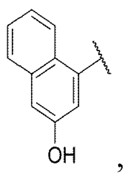, 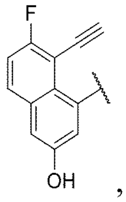, 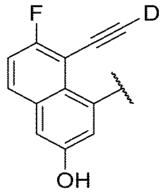, 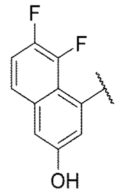, or 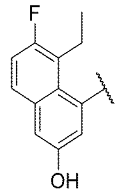;

fff) the nitrogen-containing heterocyclic compound of formula I is not any one of the following compounds:

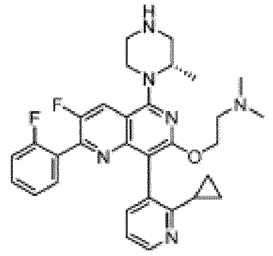

1

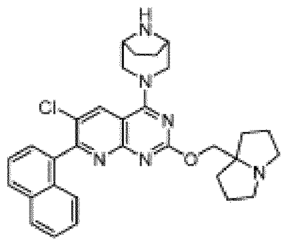

2

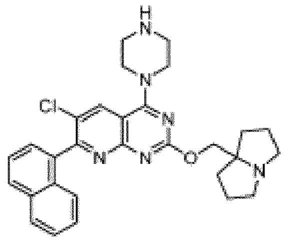

3

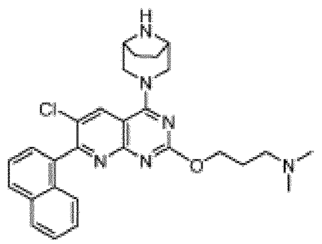

4

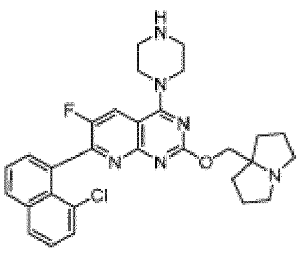

5

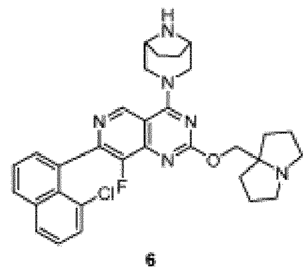

6

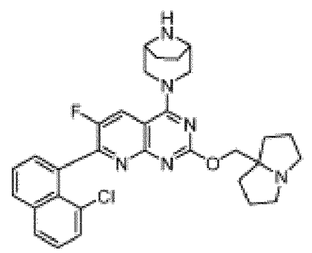

7

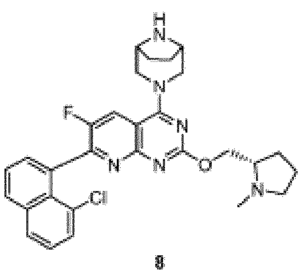

8

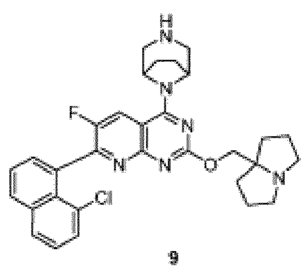

9

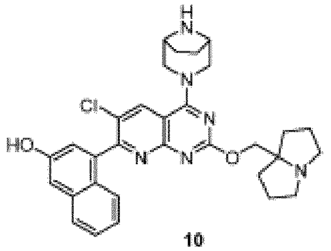

10

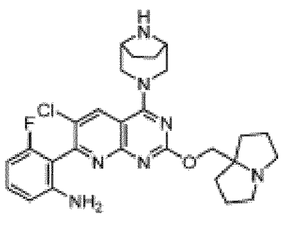

11

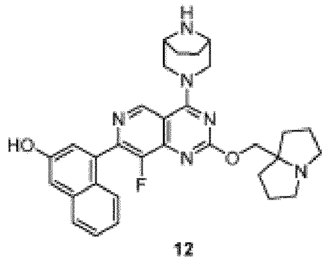

12

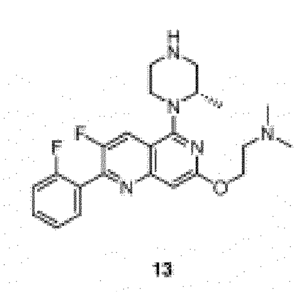

13

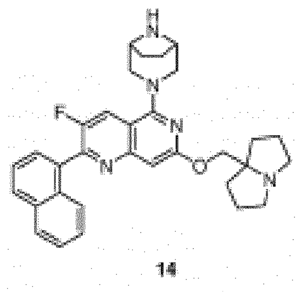

14

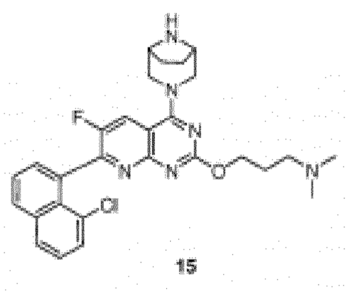

15

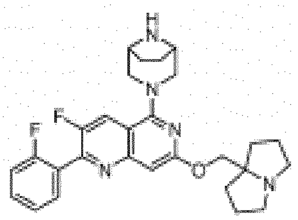

16

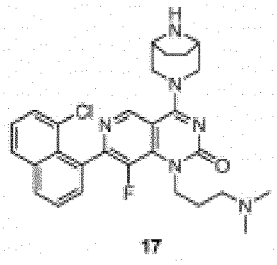

17

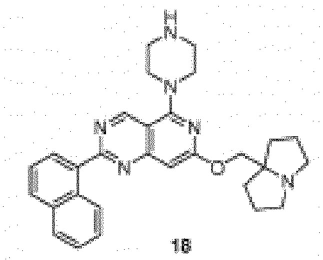

18

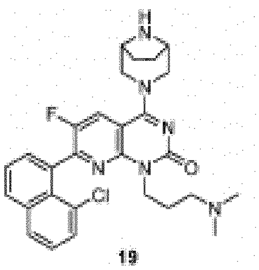

19

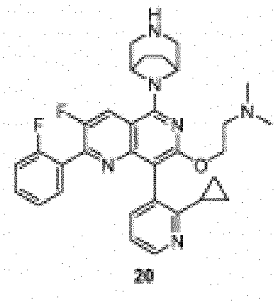

20

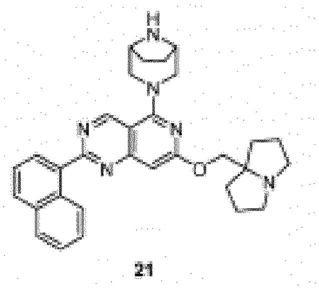

21

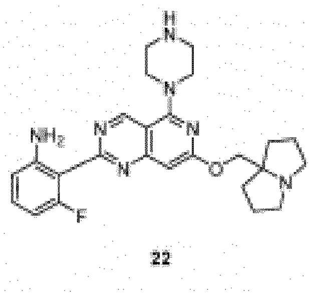

22

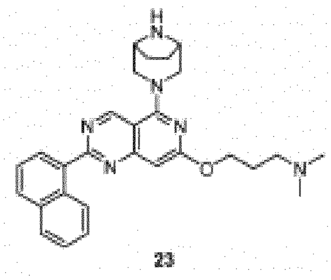

23

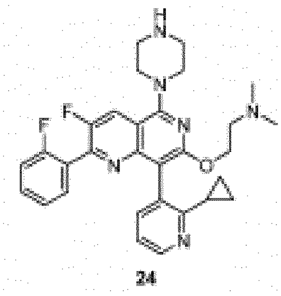

24

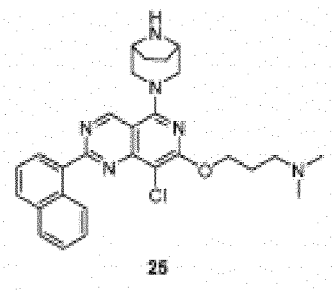

25

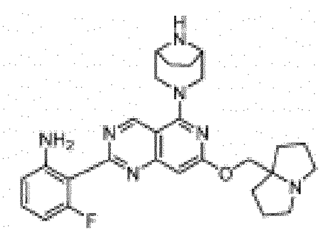

26

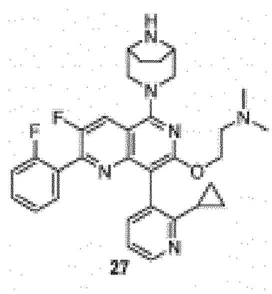

27

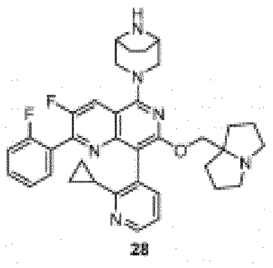
28

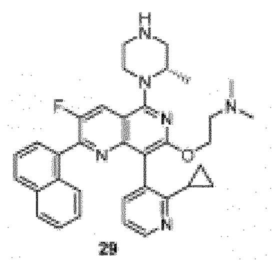
29

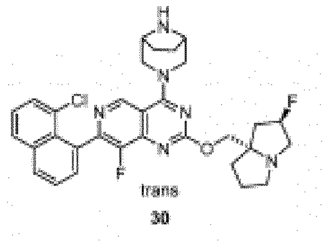
trans
30

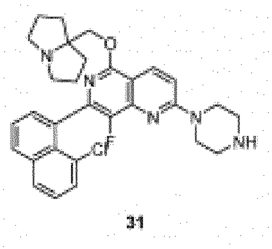
31

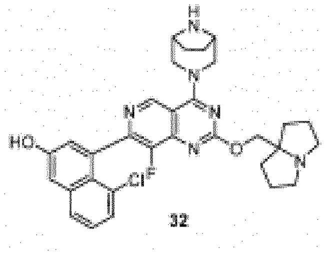
32

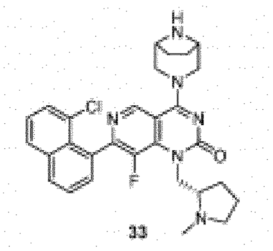
33

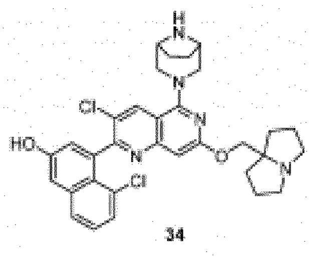
34

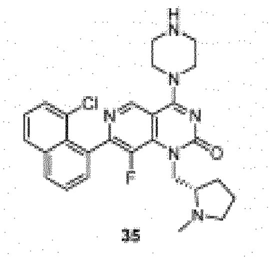
35

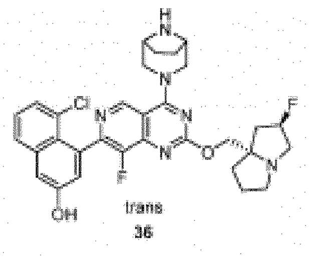
trans
36

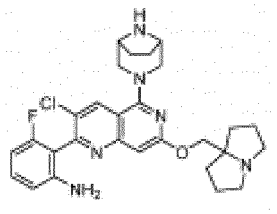
37

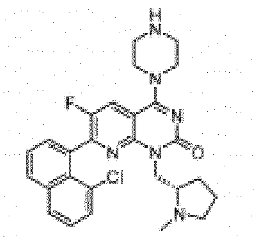
38

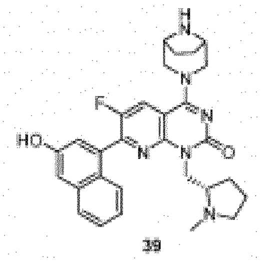
39

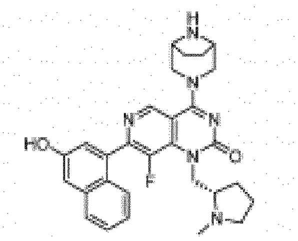
40

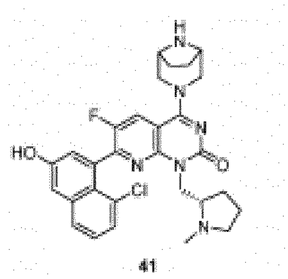
41

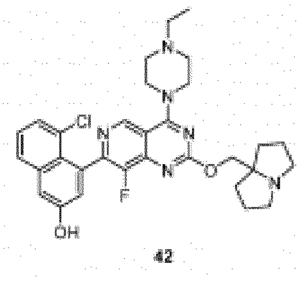
42

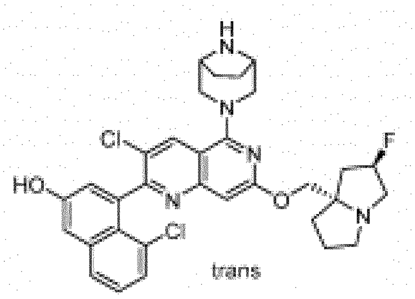

trans

43

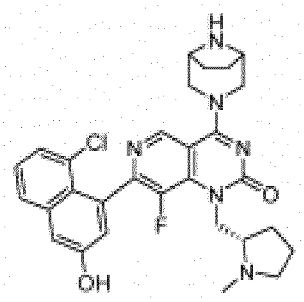

44

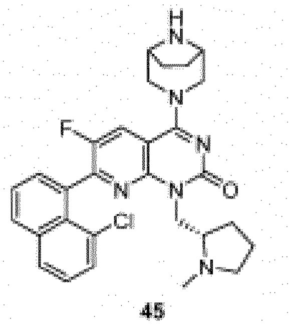

45

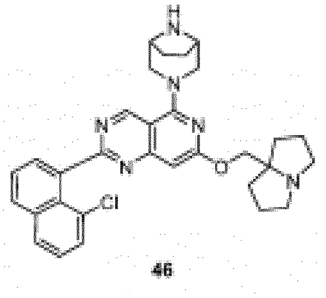

46

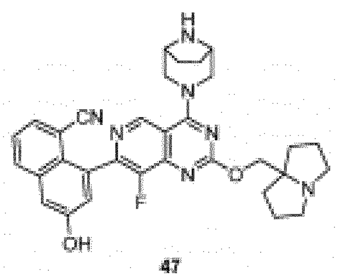

47

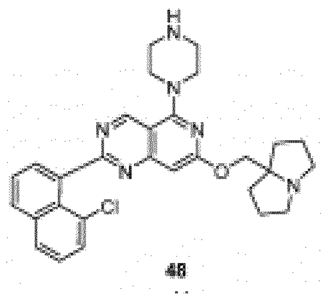

48

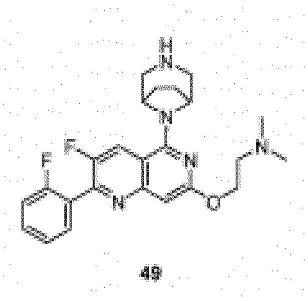

49

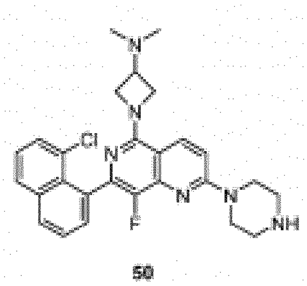

50

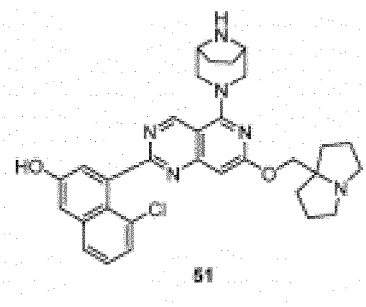

51

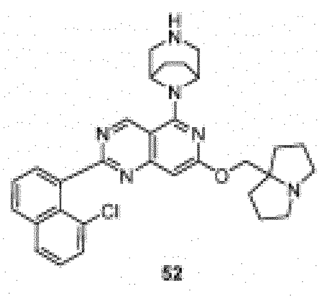

52

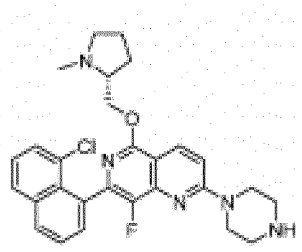

53

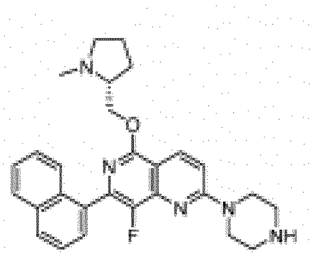

54

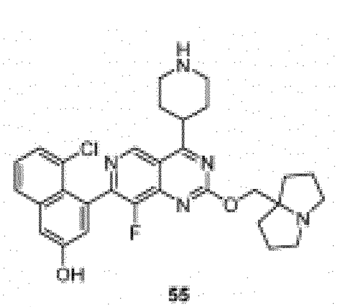

55

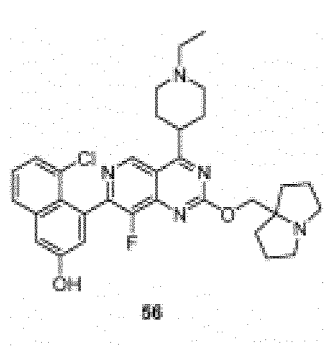

56

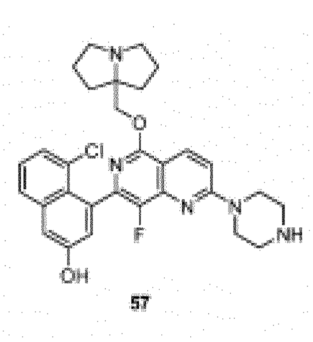

57

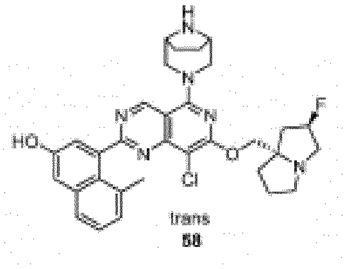

trans
58

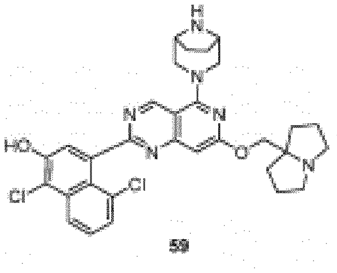

59

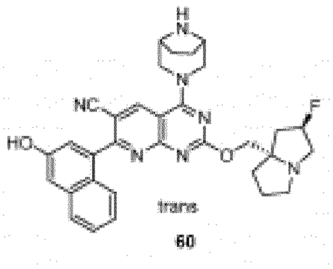

trans
60

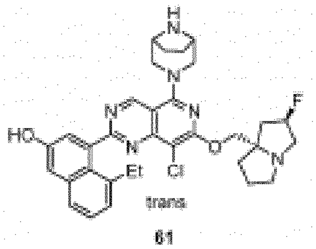

trans
61

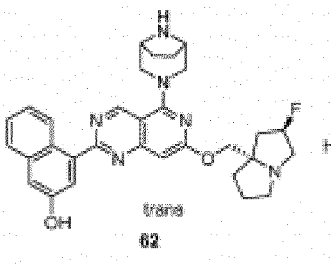

trans
62

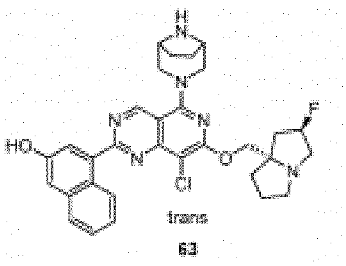

trans
63

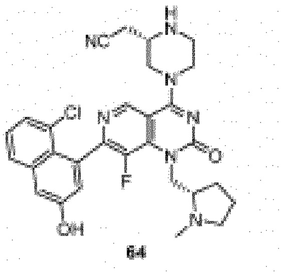

64

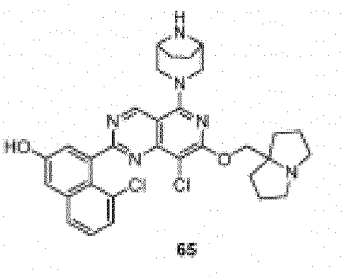

65

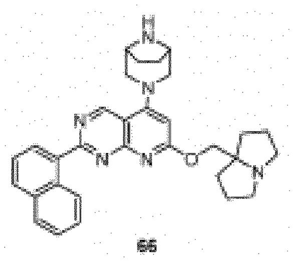

66

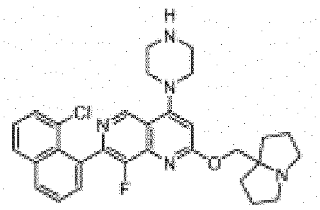

67

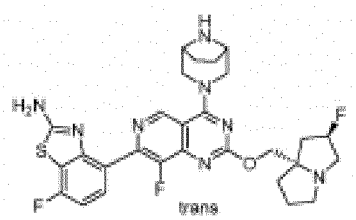

trans
68

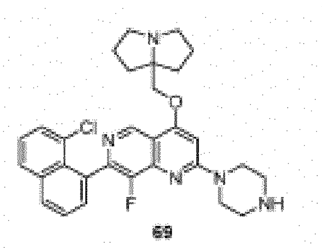

69

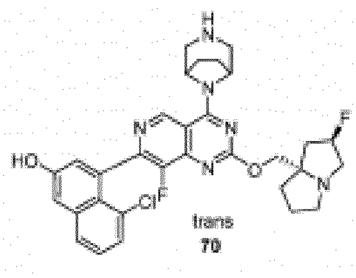

trans
70

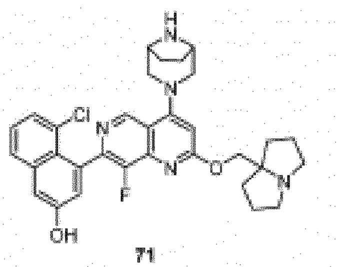

71

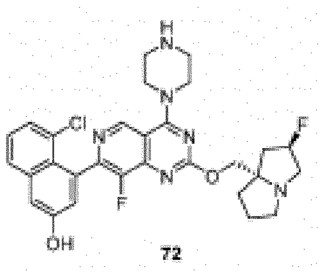

72

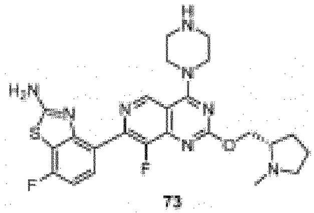
73

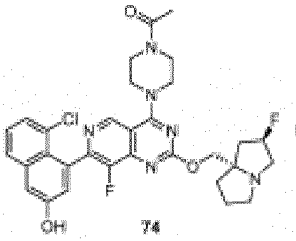
74

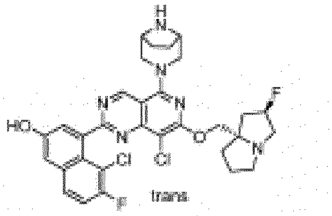
75

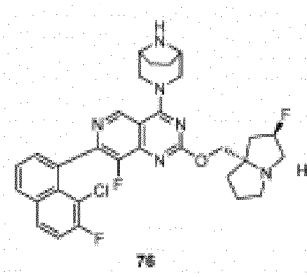
76

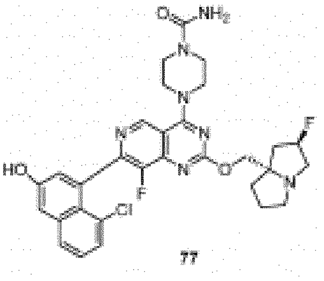
77

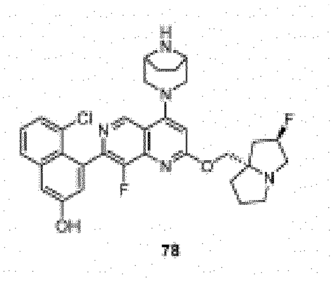
78

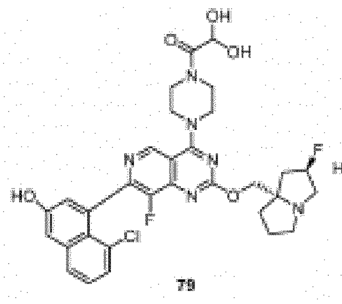
79

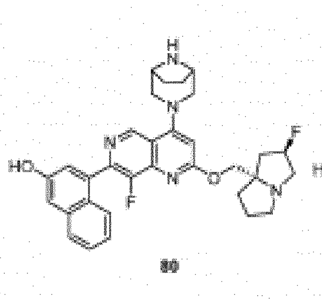
80

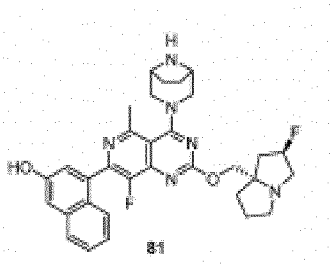
81

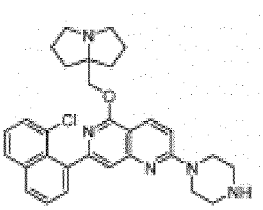
82

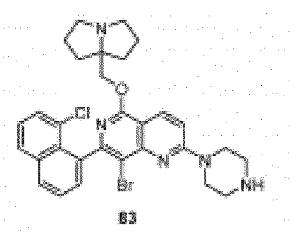
83

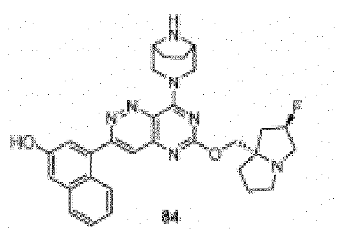
84

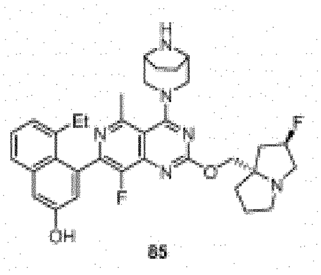
85

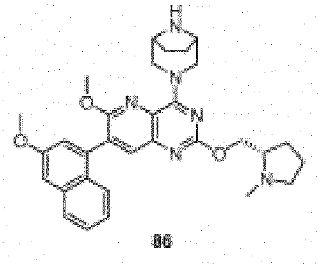
86

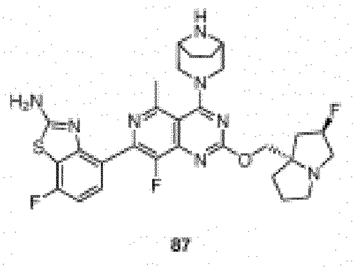
87

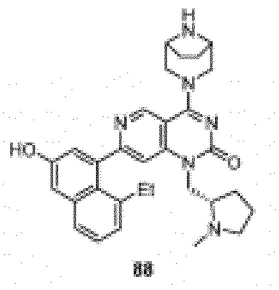
88

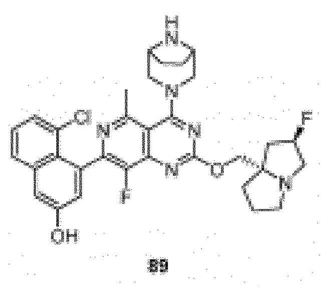
89

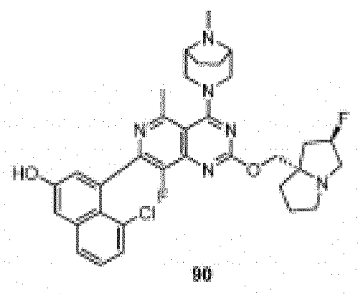
90

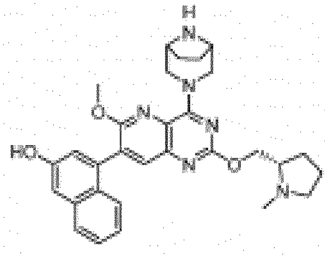
91

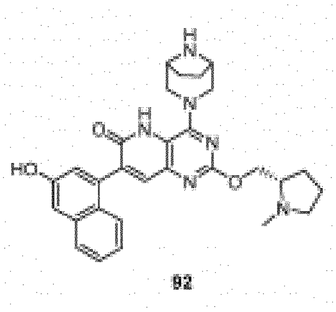
92

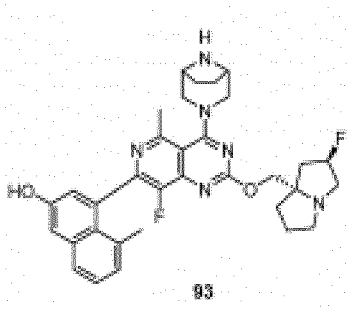
93

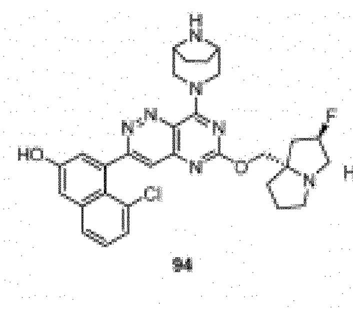
94

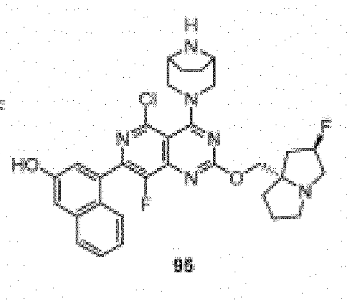
95

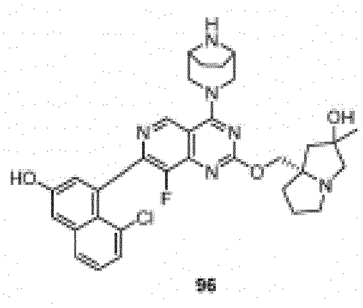
96

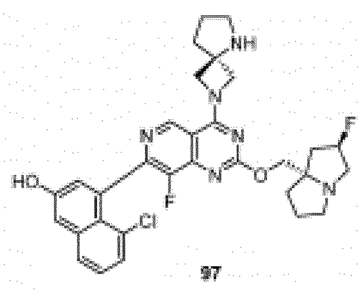
97

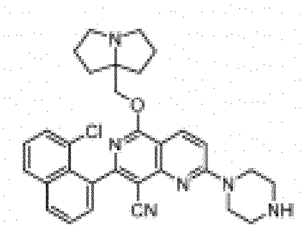
98

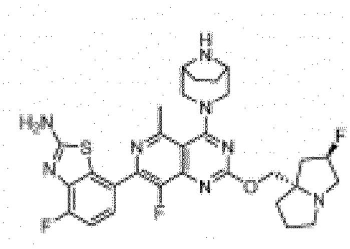
99

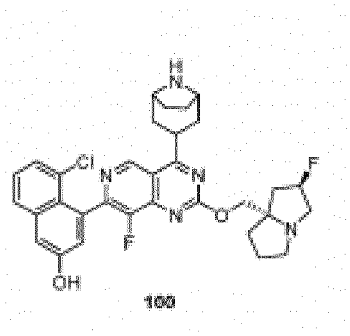
100

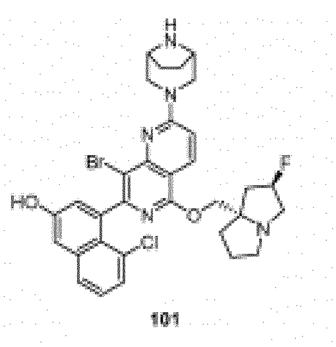
101

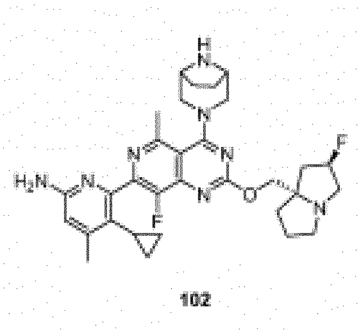
102

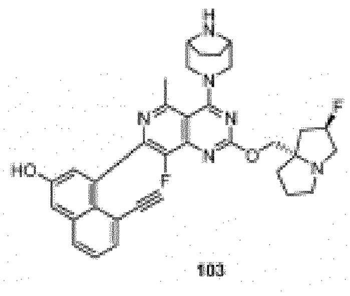
103

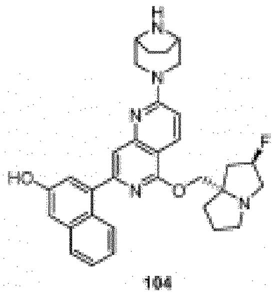
104

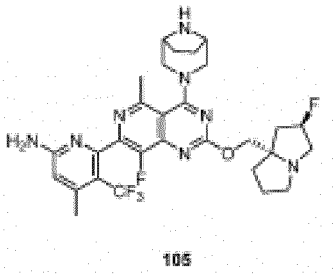
105

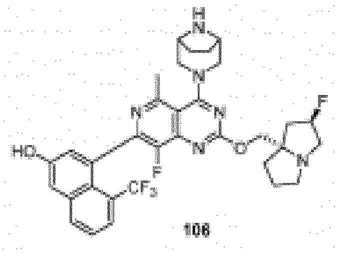
106

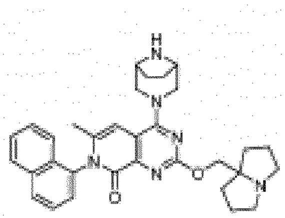
107

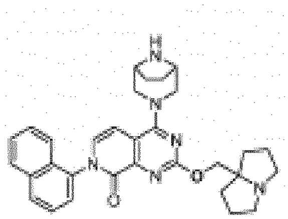
108

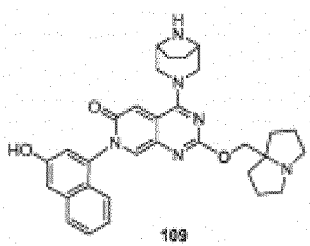
109

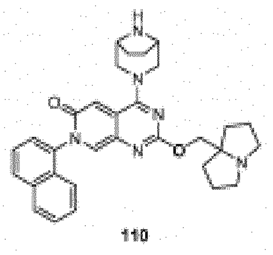
110

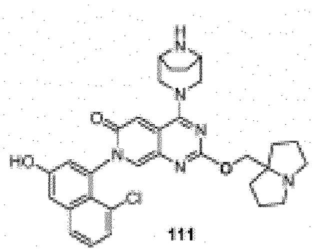
111

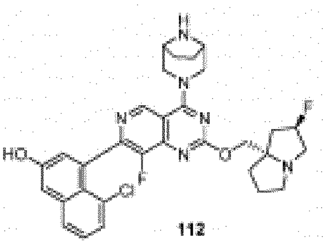
112

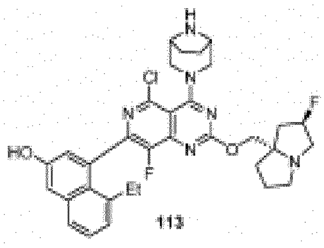
113

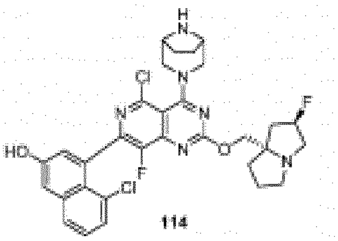
114

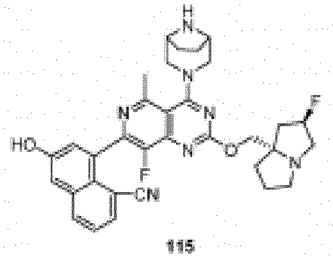
115

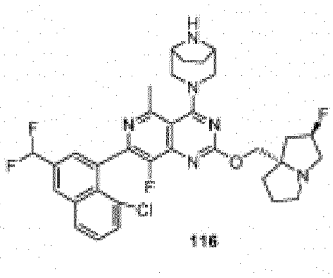
116

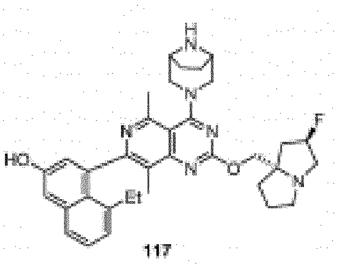
117

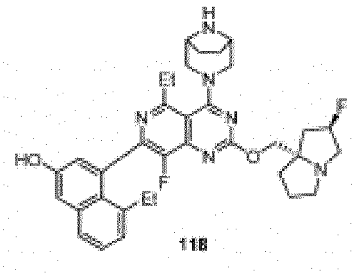

118

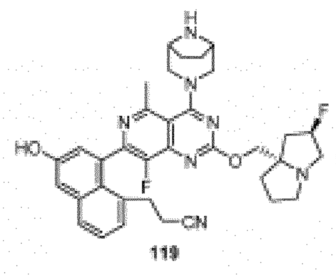

119

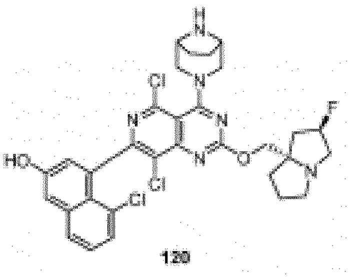

120

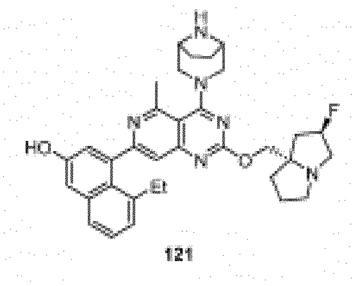

121

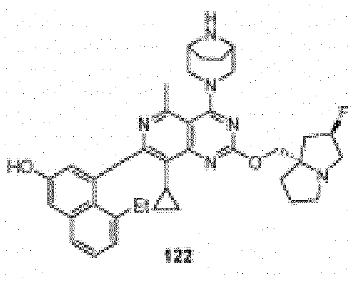

122

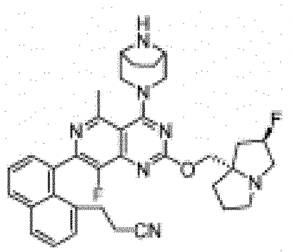

123

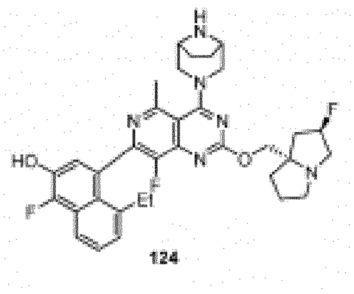

124

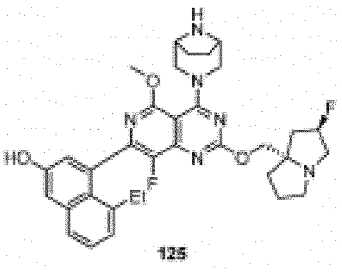

125

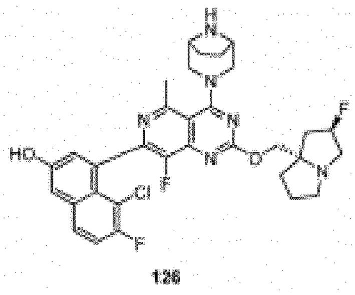

126

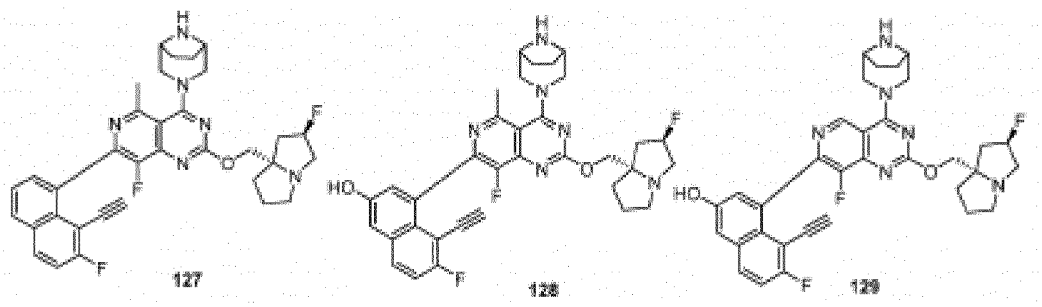

127

128

129

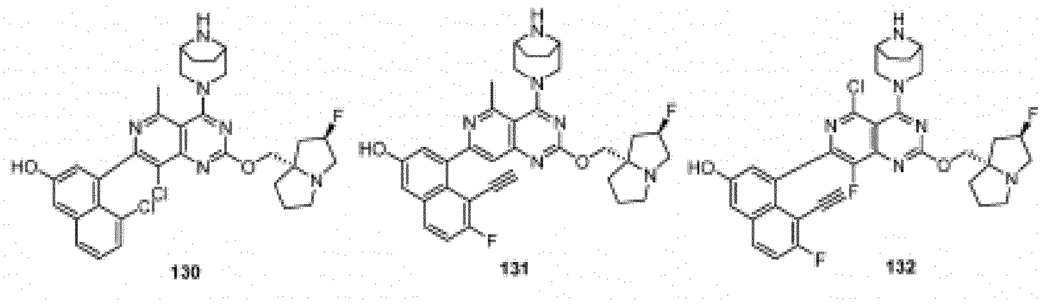

130

131

132

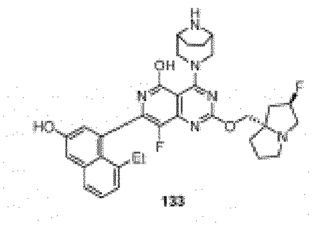
133
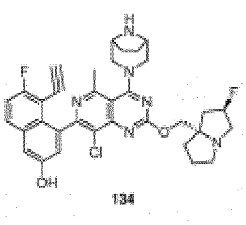
134
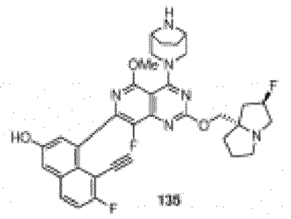
135

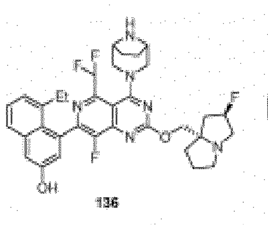
136
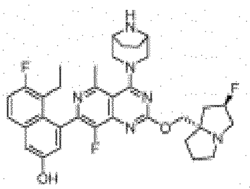
137
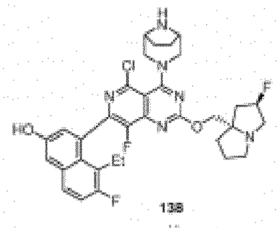
138

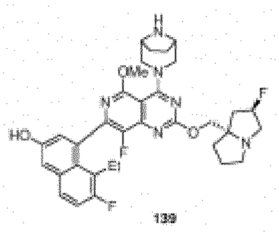
139
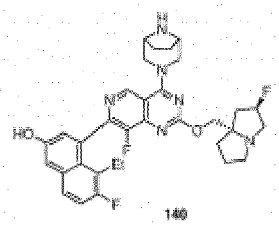
140
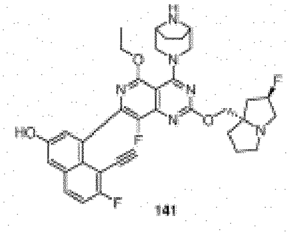
141
;

ggg) the nitrogen-containing heterocyclic compound of formula **I** is not any one of the following compounds:

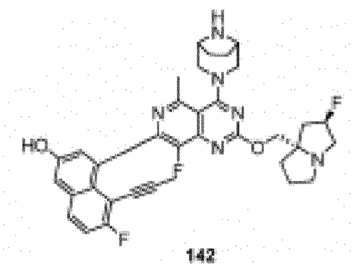
142
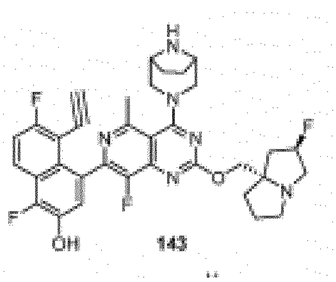
143
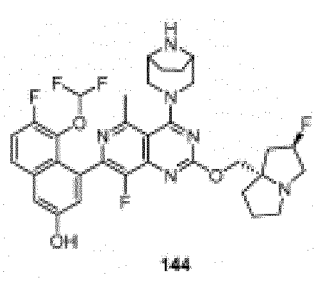
144

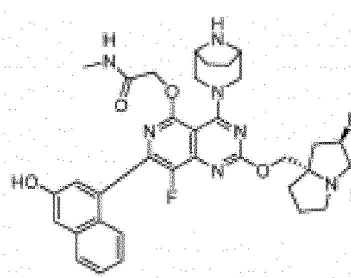
145
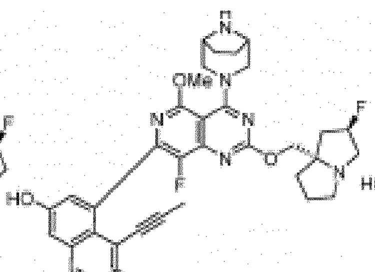
146
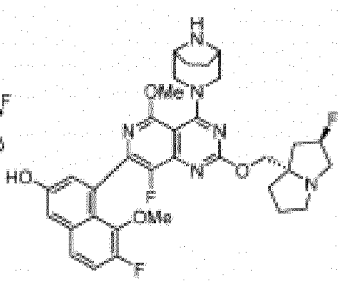
147

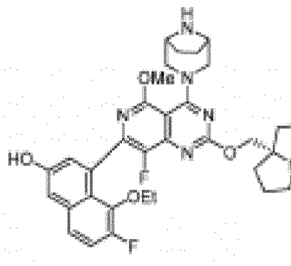
148

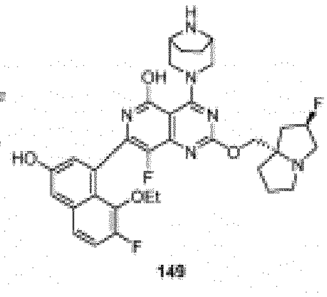
149

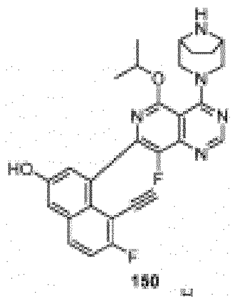
150

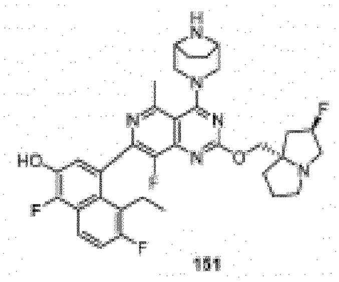
151

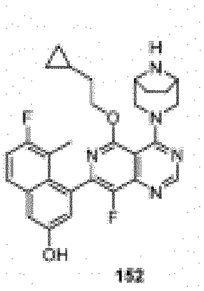
152

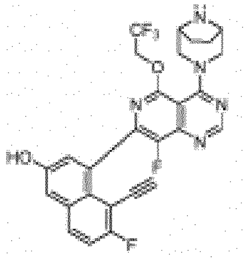
153

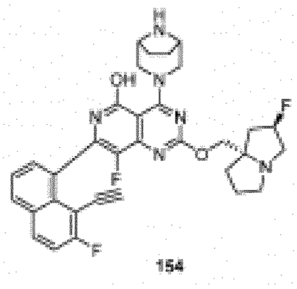
154

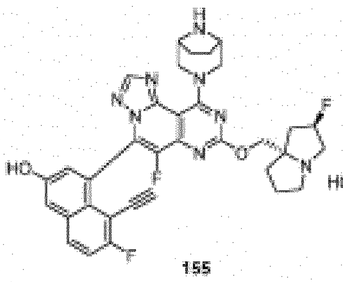
155

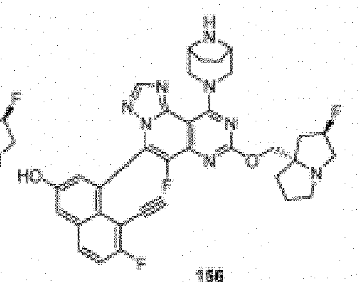
156

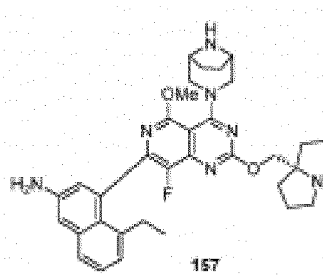
157

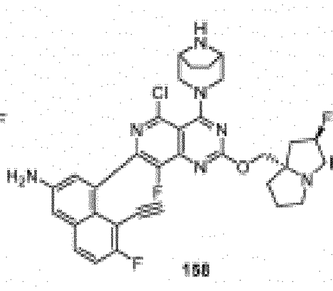
158

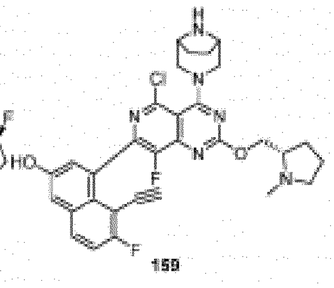
159

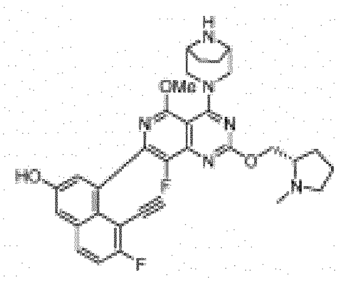
160

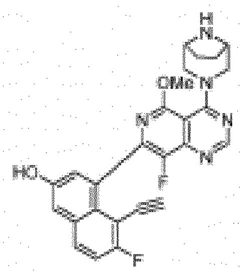
161

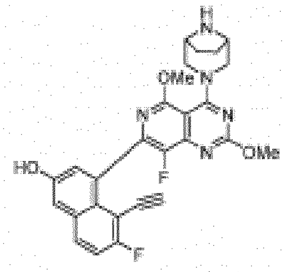
162

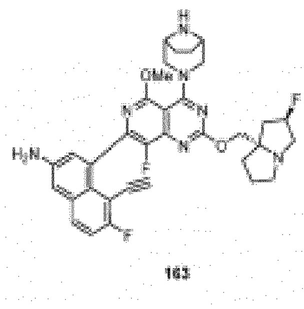

163

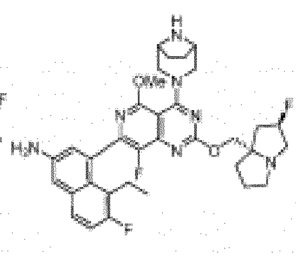

164

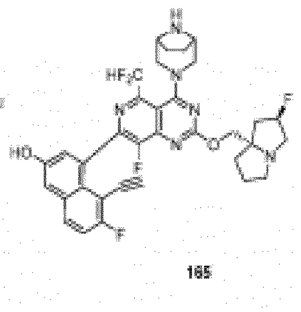

165

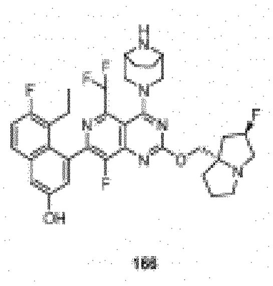

166

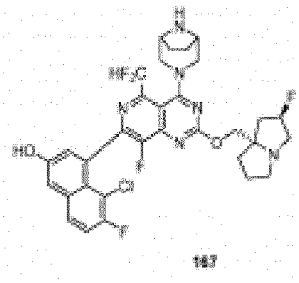

167

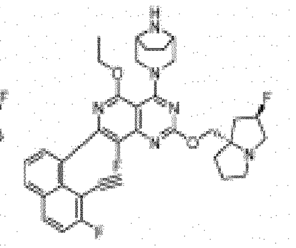

168

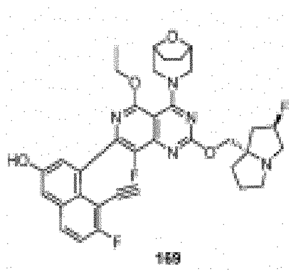

169

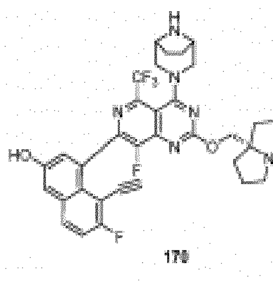

170

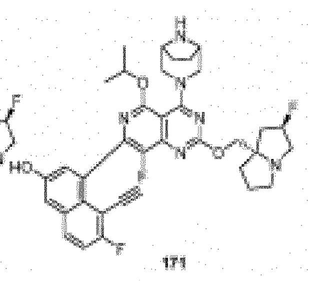

171

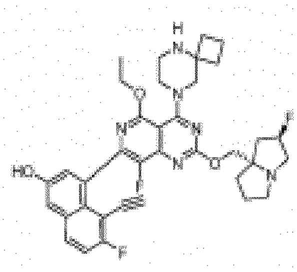

172

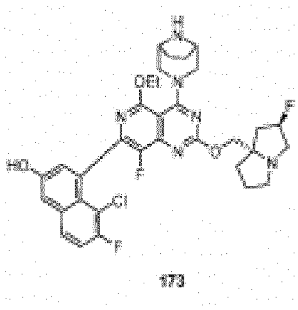

173

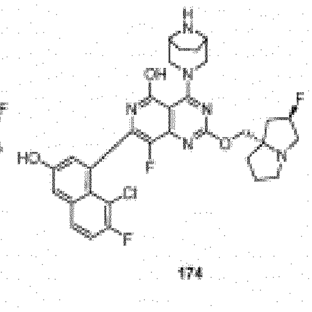

174

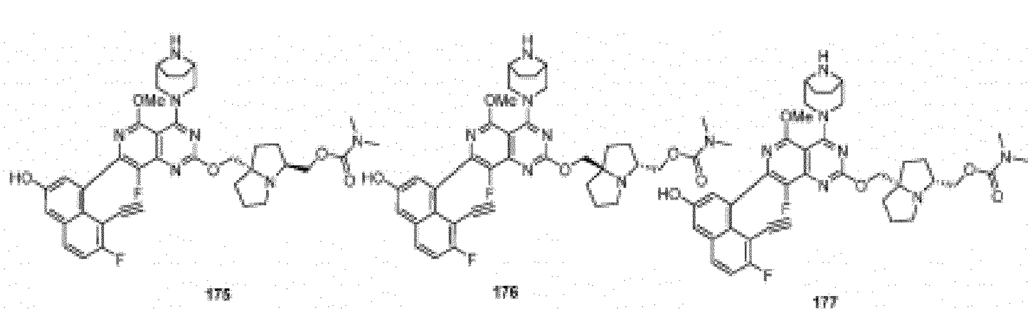

175

176

177

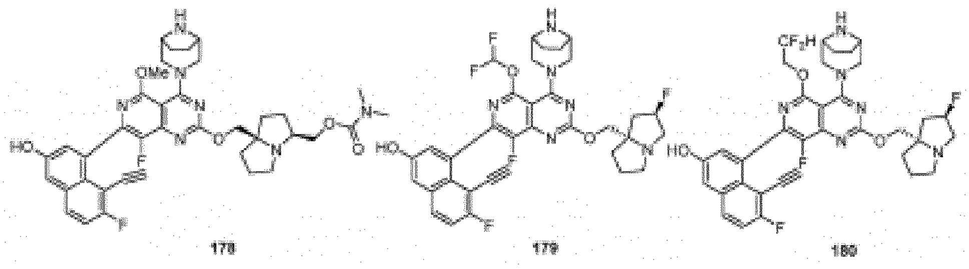

178 179 180

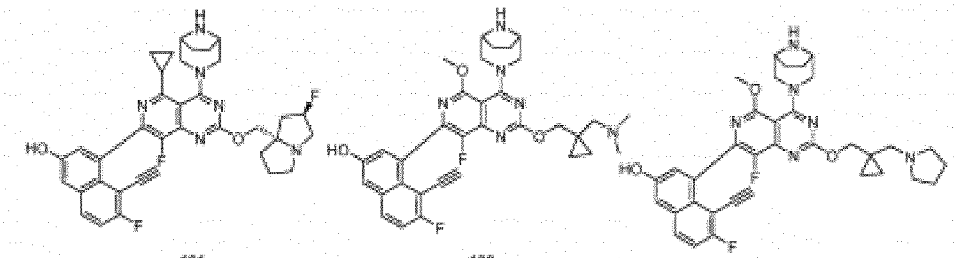

181 182 183

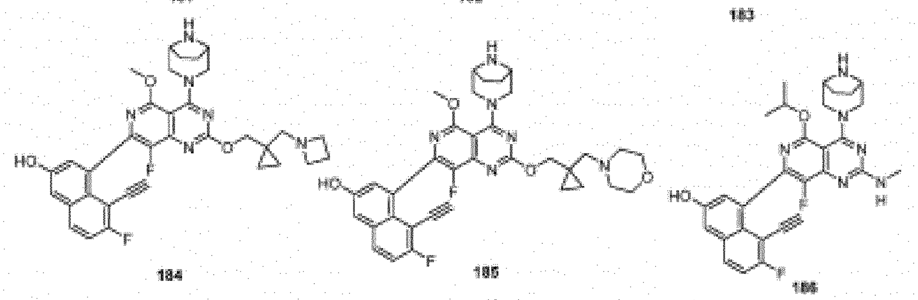

184 185 186

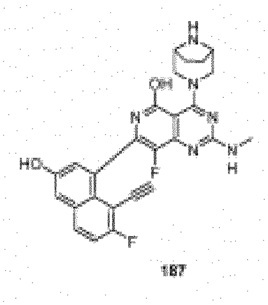

187

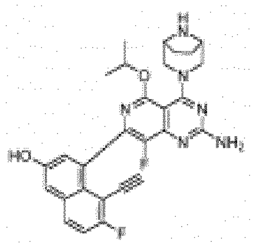

188

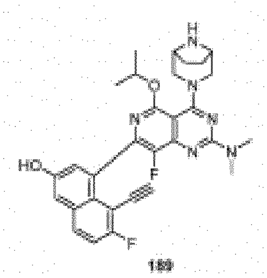

189

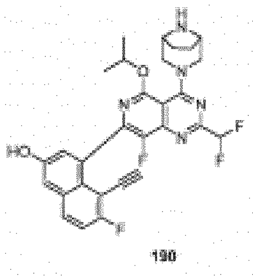

190

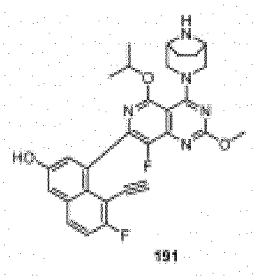

191

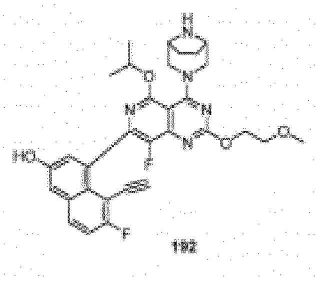

192

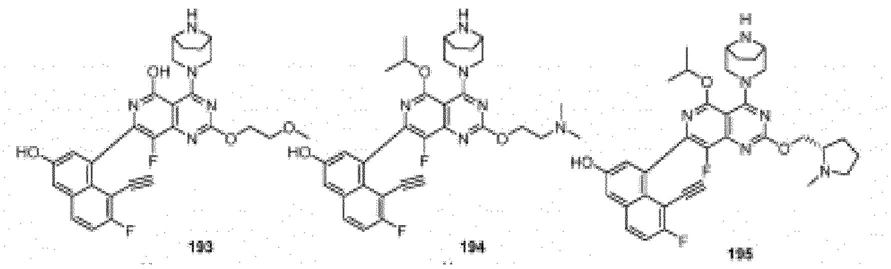

193 194 195

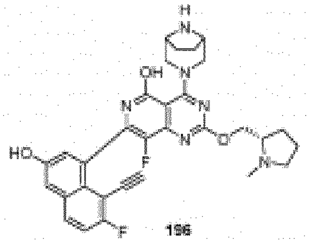
196

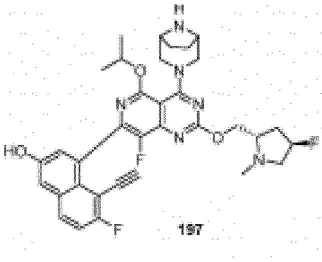
197

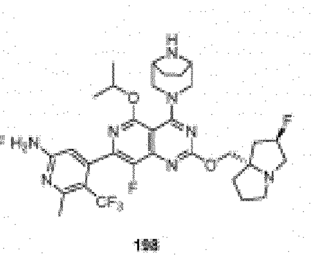
198

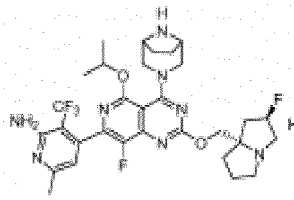
199

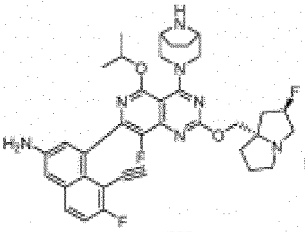
200

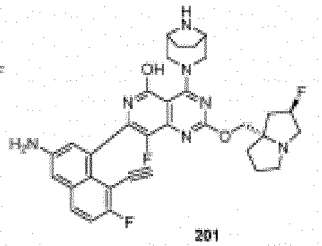
201

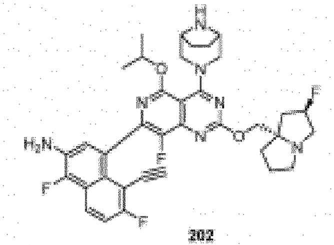
202

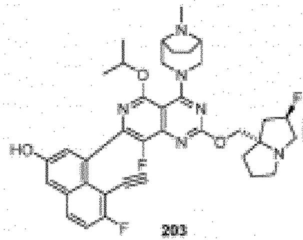
203

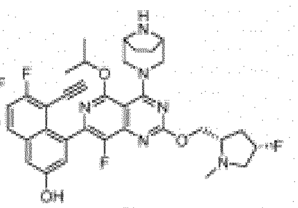
204

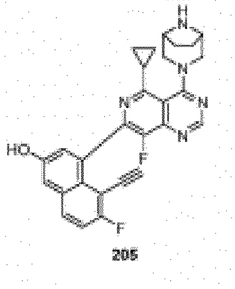
205

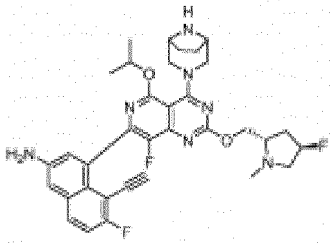
206

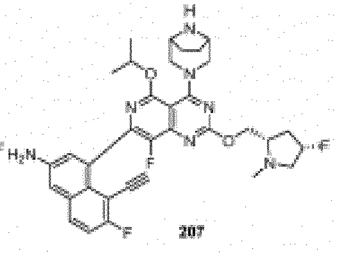
207

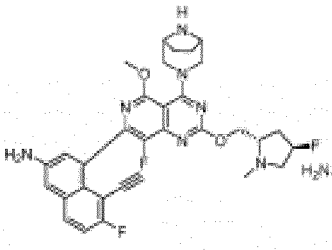
208

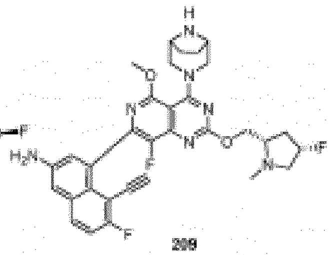
209

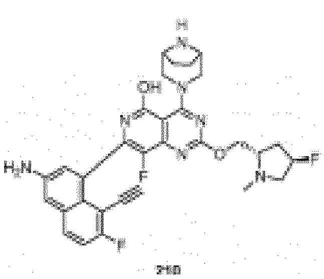
210

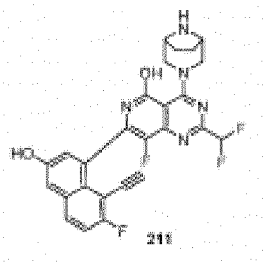
211

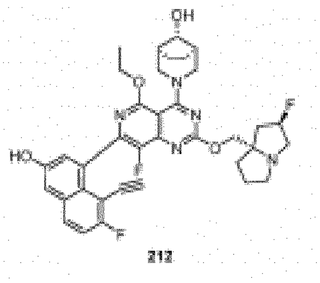
212

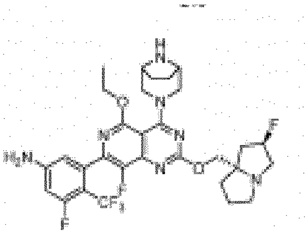
213

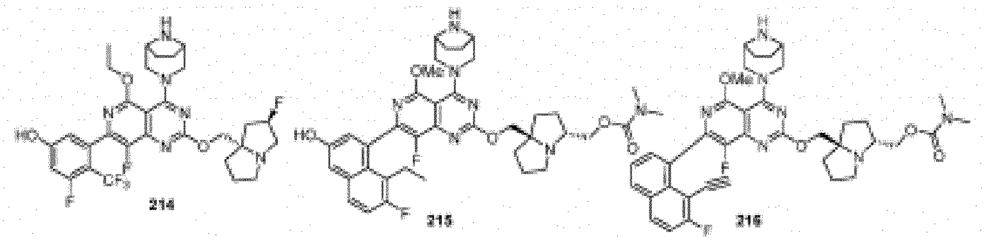

214 215 216

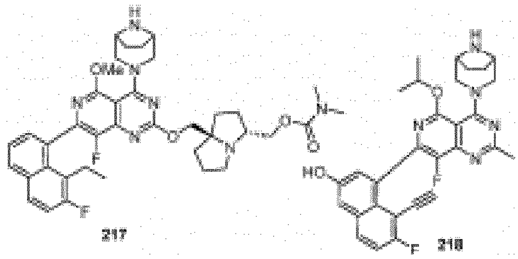

217 218

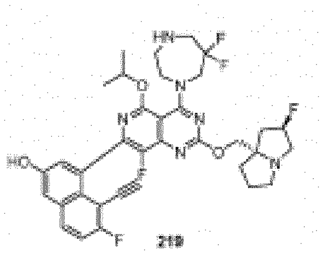

219

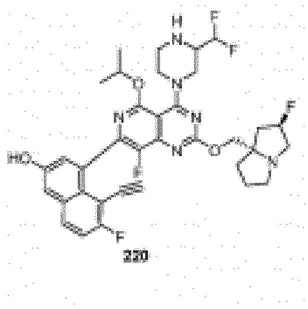

220

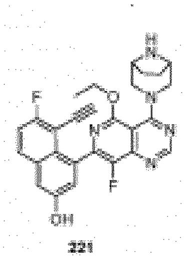

221

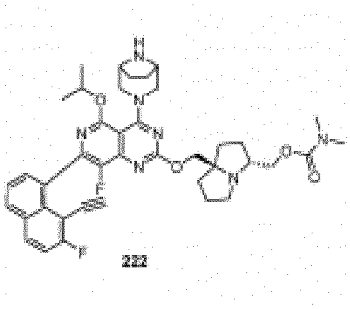

222

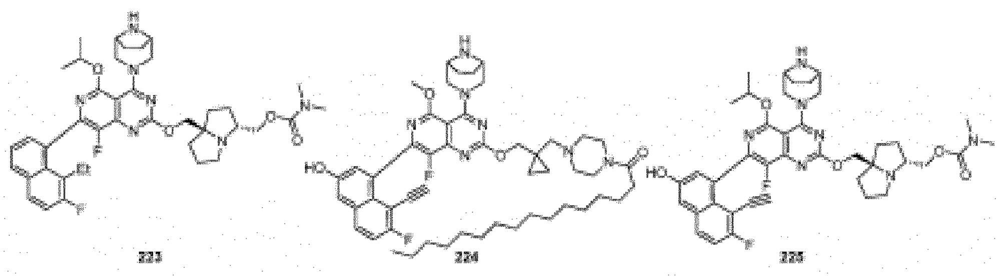

223 224 225

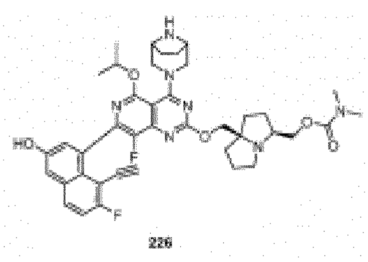

226

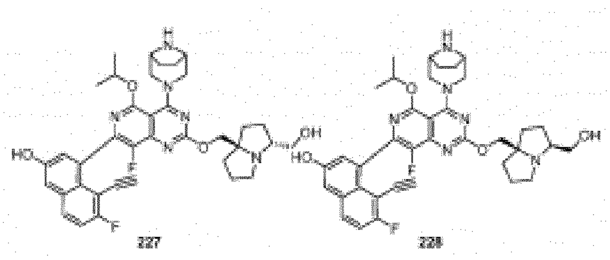

227 228

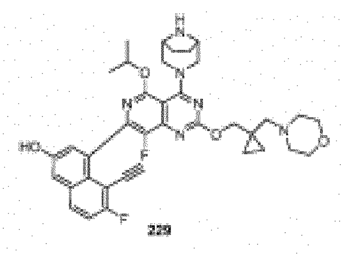

229

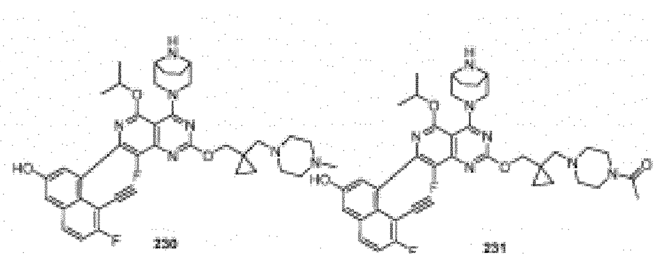

230 231

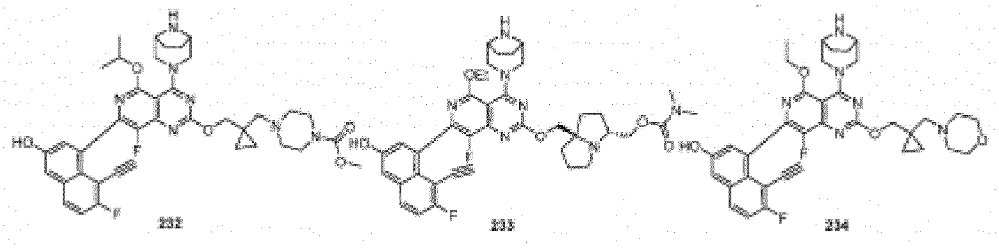

232 233 234

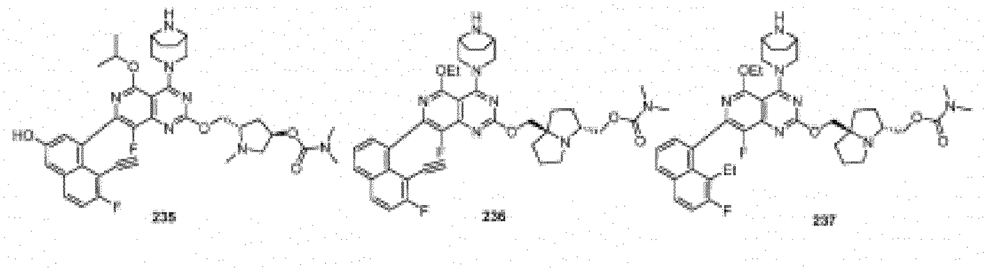

235 236 237

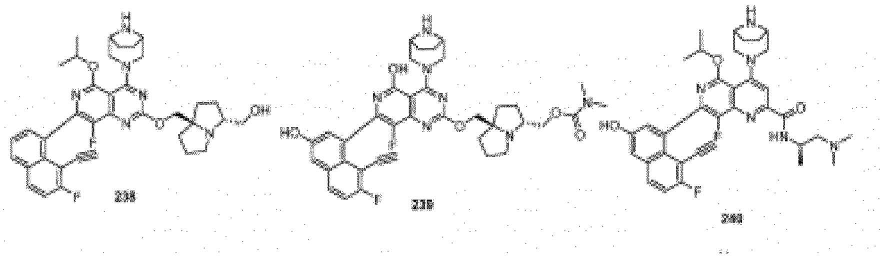

238 239 240

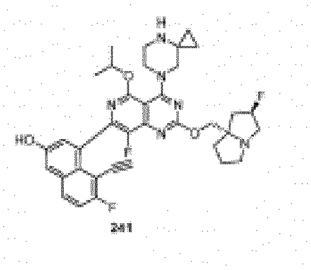

241

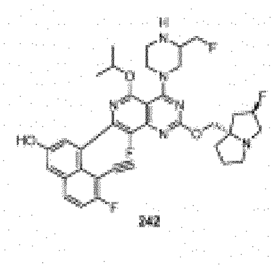

242

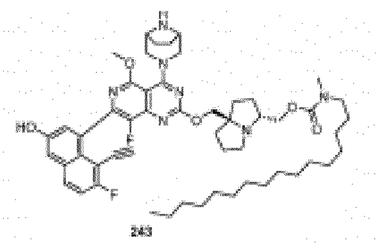

243

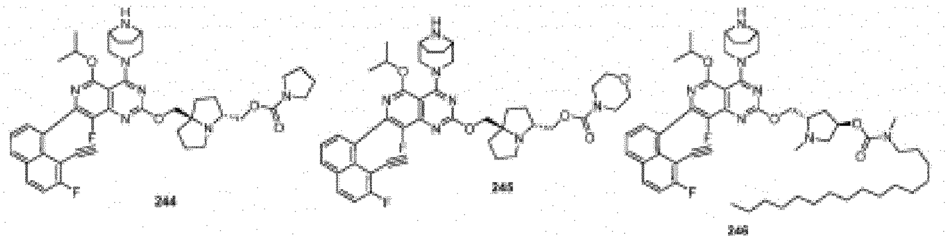

244 245 246

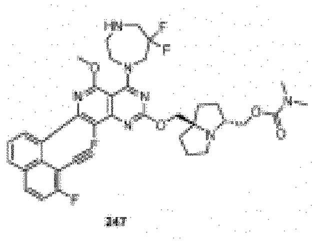

247

;

hhh) the nitrogen-containing heterocyclic compound of formula **I** is not any one of the following compounds:

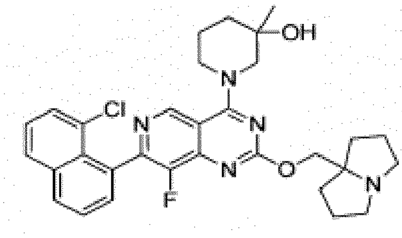,

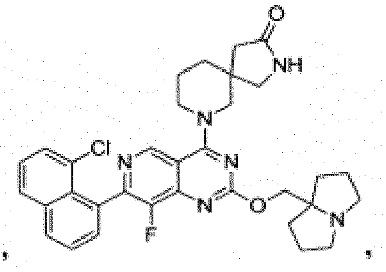,

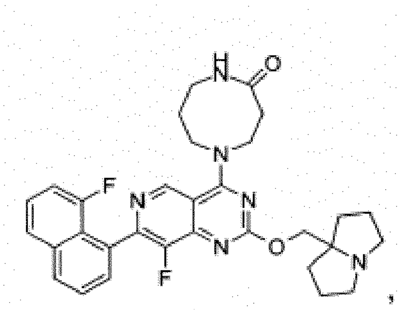,

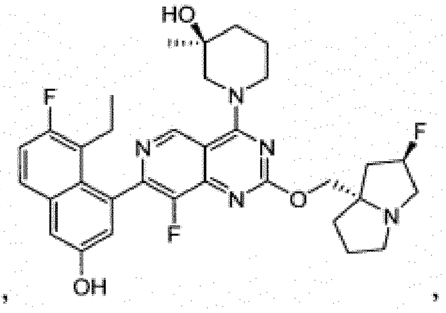,

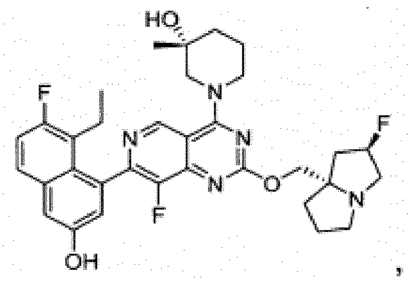,

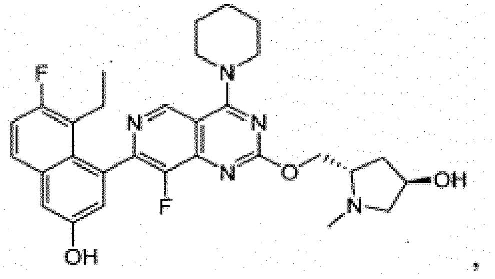,

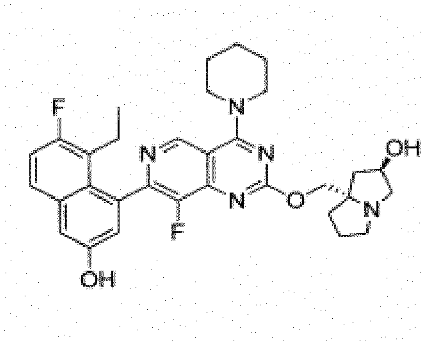,

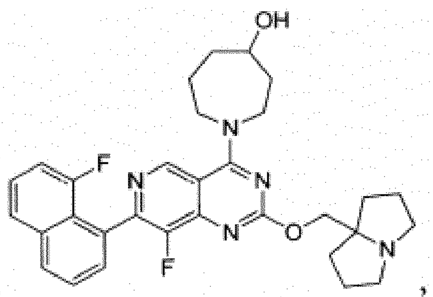,

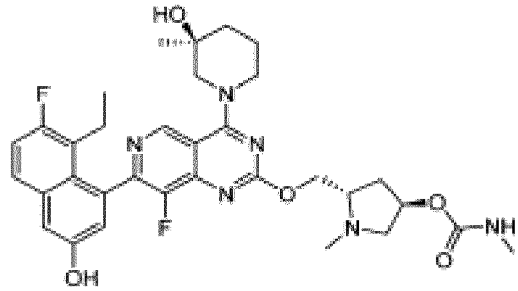,
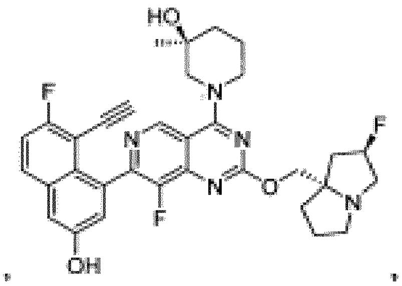,
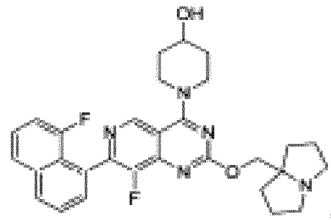,
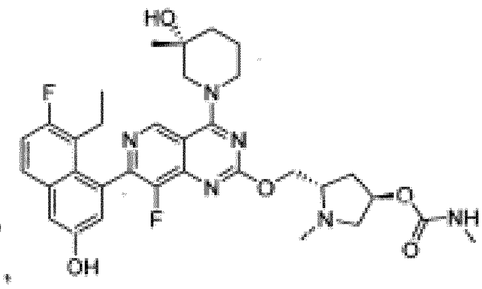,
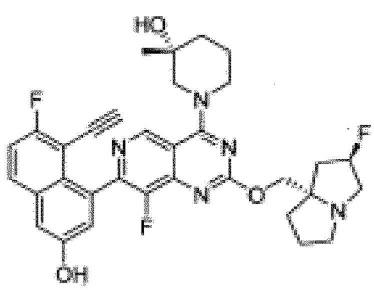,
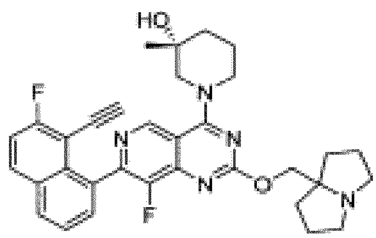,
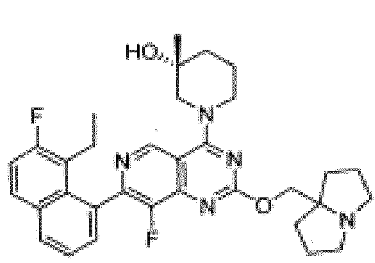,
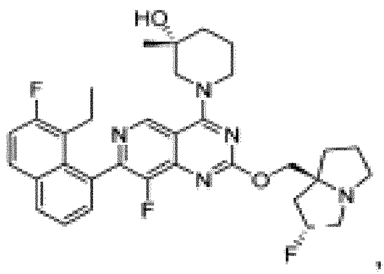,
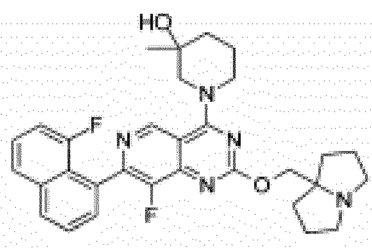,
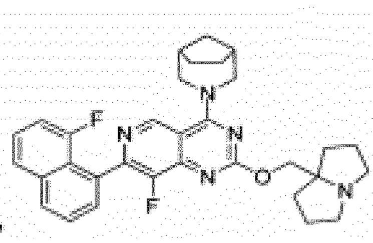,
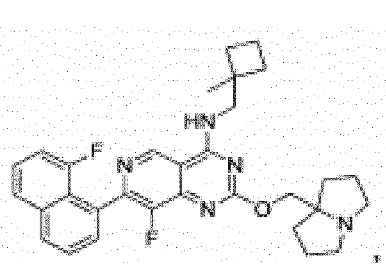,
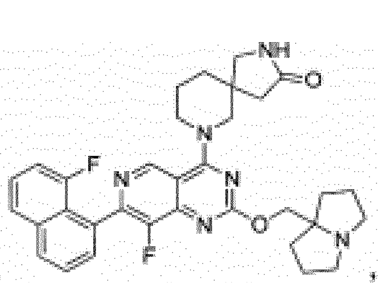,

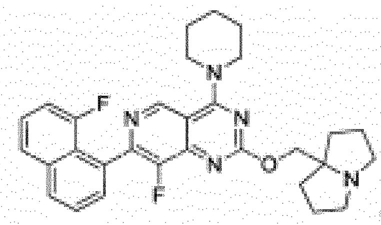
,
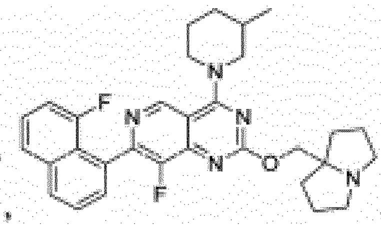
,

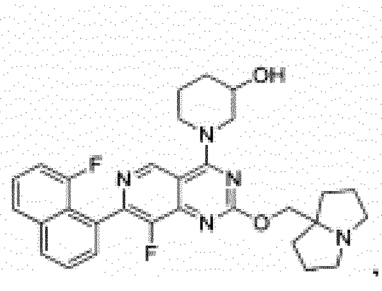
,
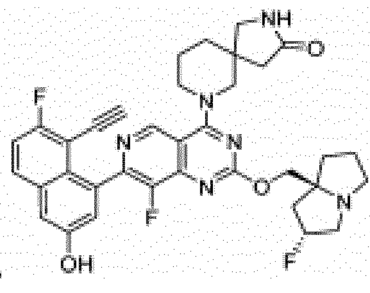
,

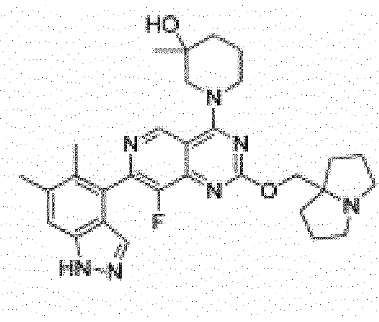
,
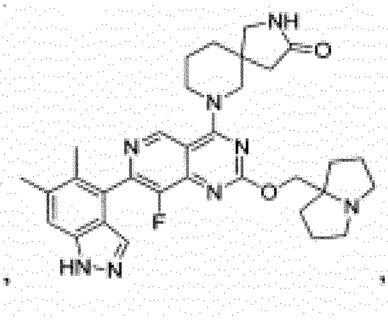
,

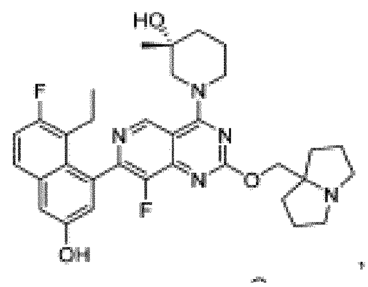
,
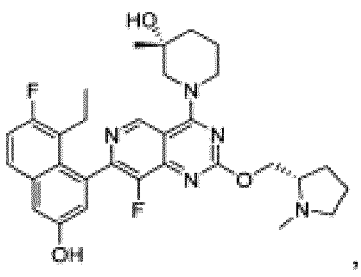
,

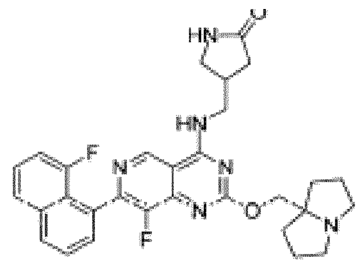
,
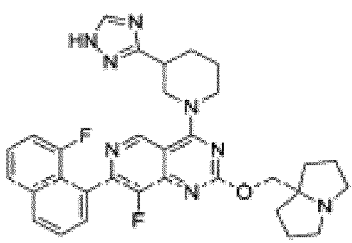
,

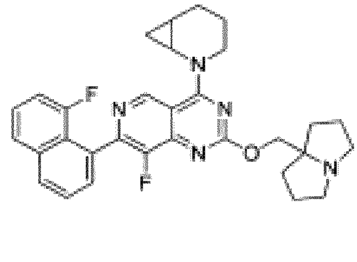
,
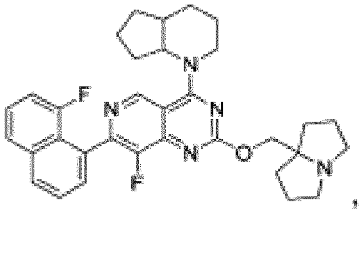
,

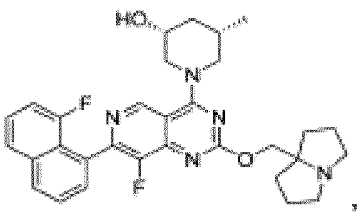,
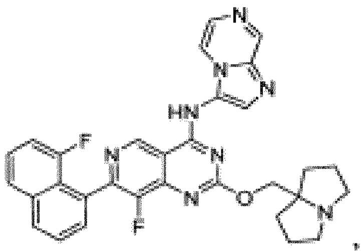,

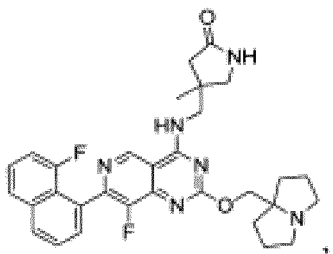,
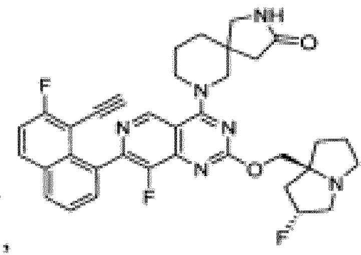,

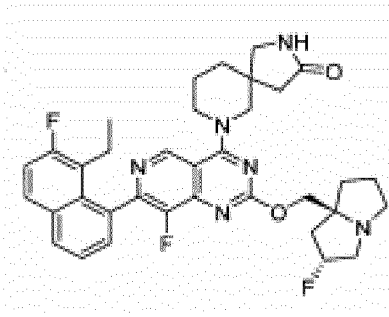,
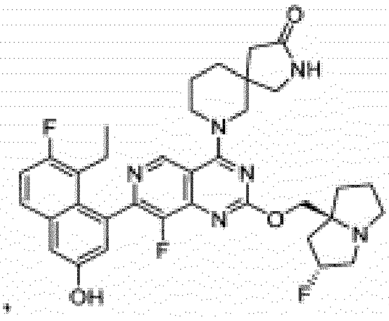,

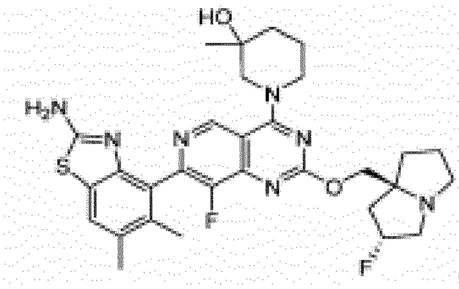,
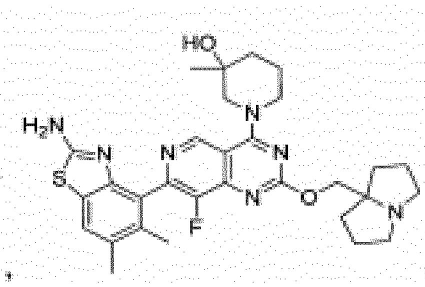,

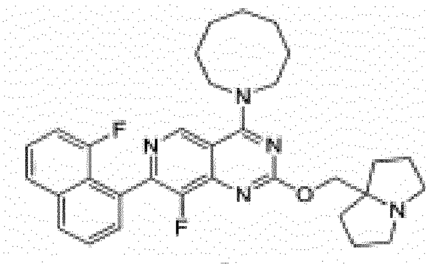,
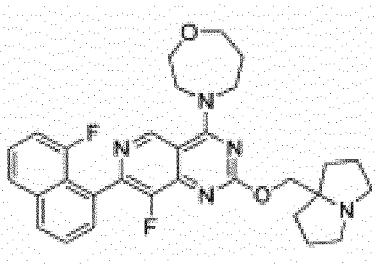,

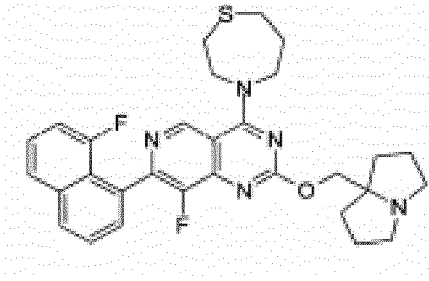,
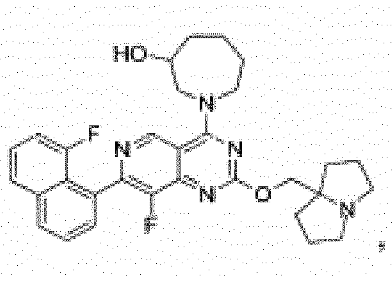,

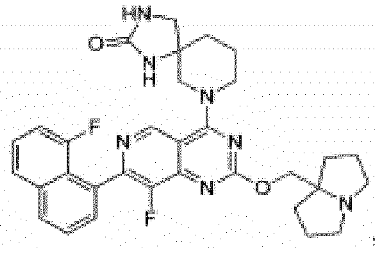,
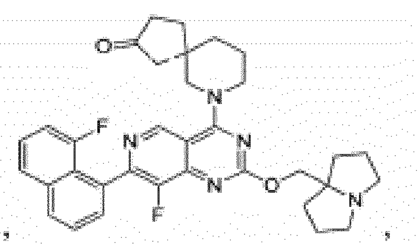,

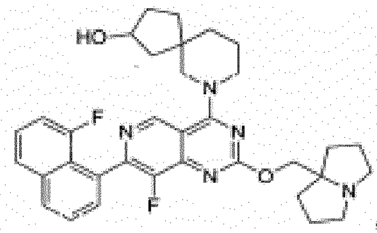,
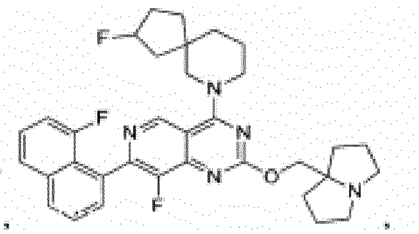,

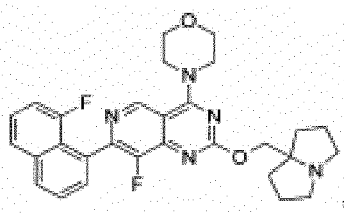,
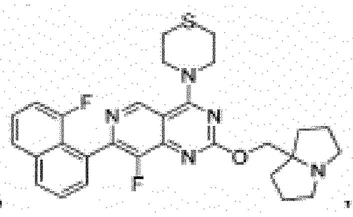,

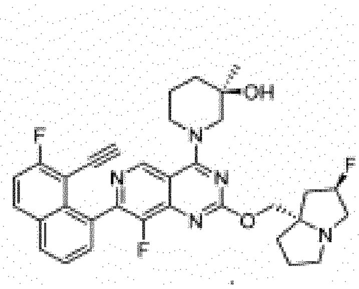,
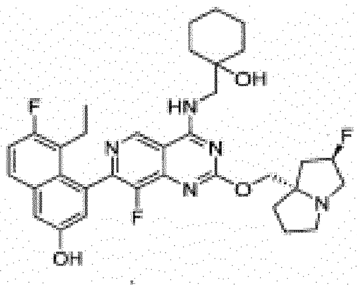,

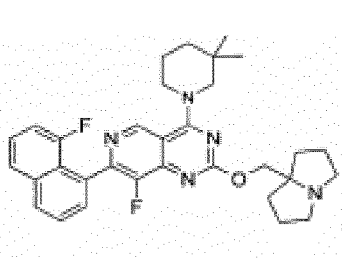,
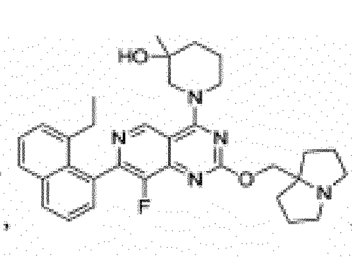,

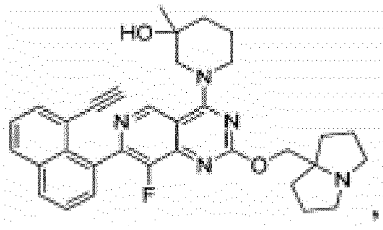,
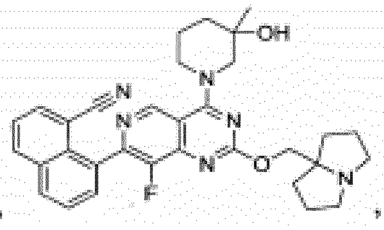,

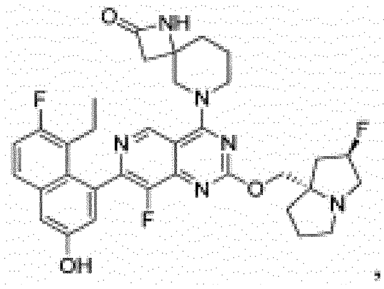,
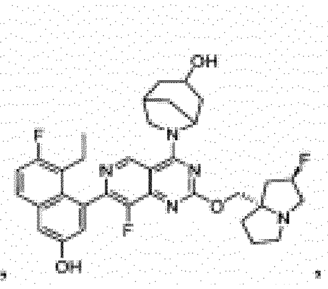,

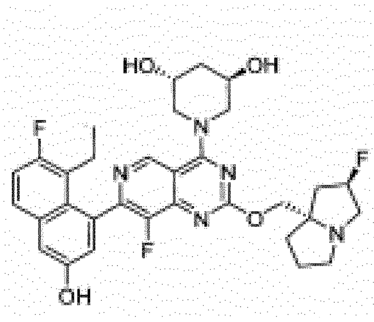,
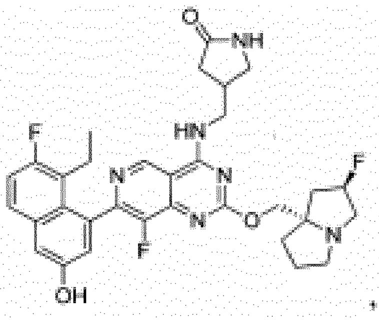,

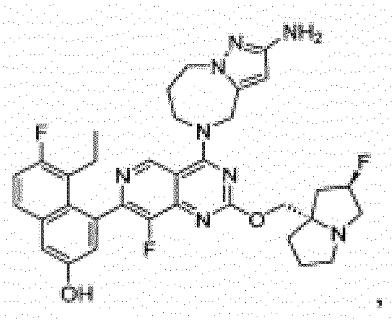,
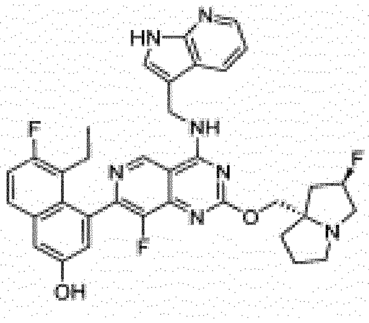,

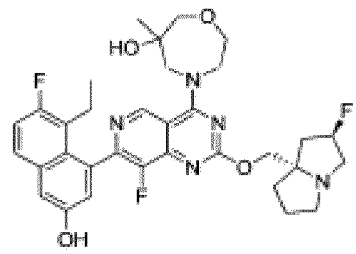,
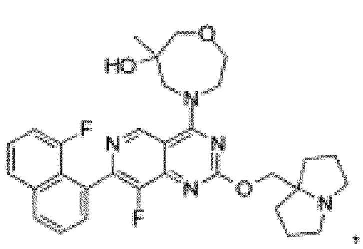,

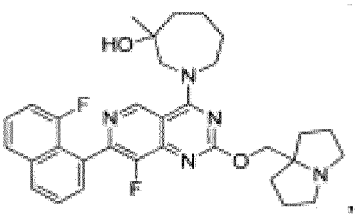, 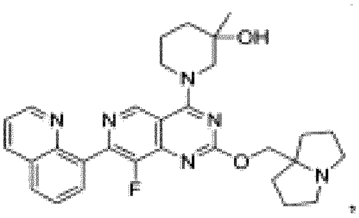,

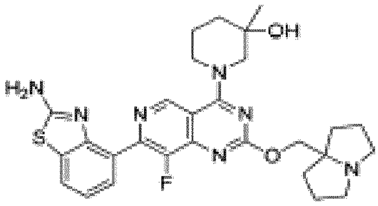, 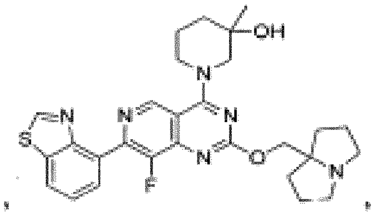,

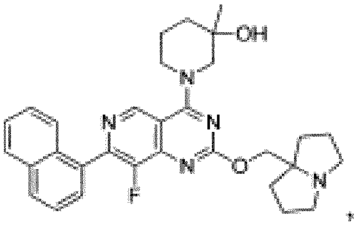, 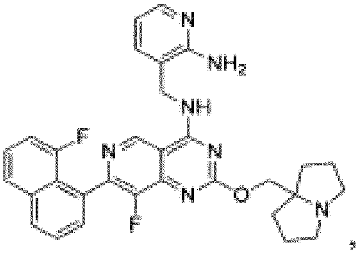,

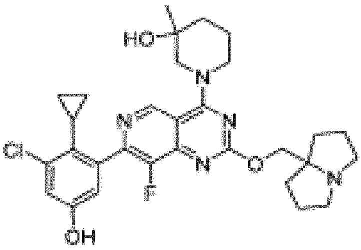, 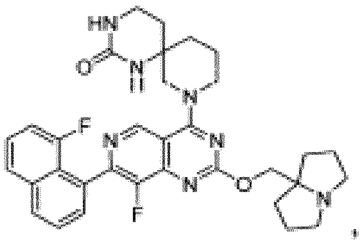,

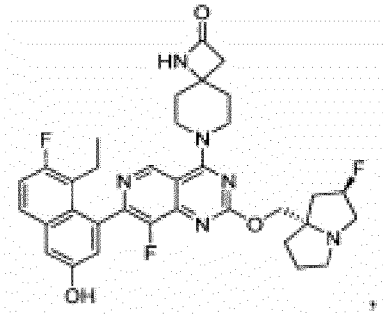, 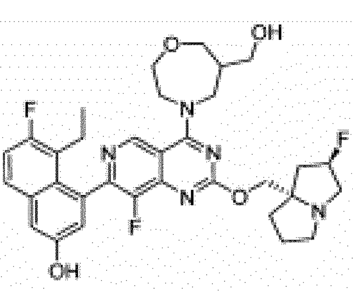,

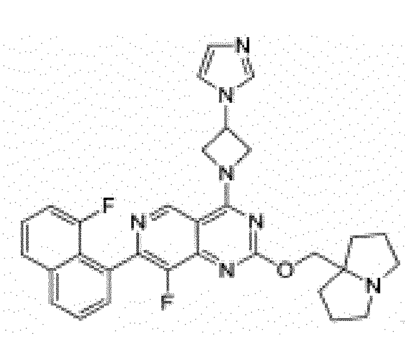, 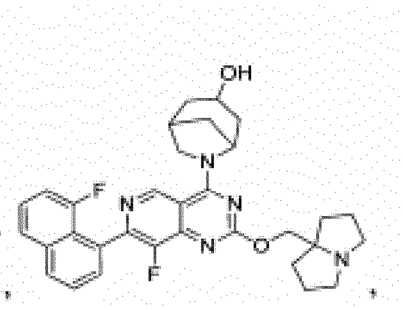,

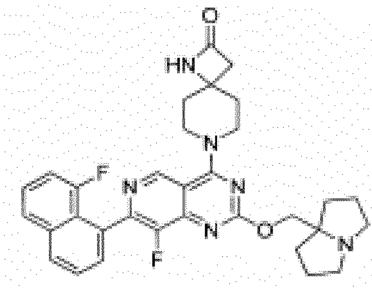, 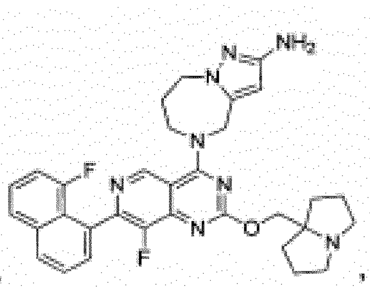,

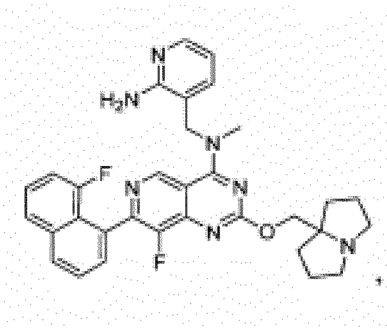, 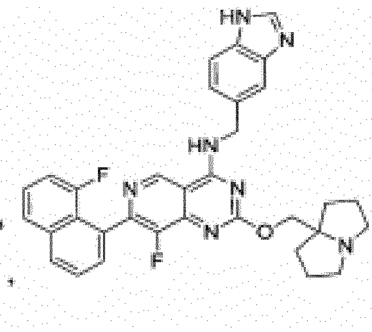,

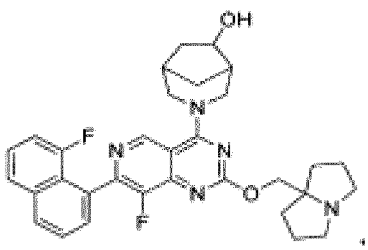, 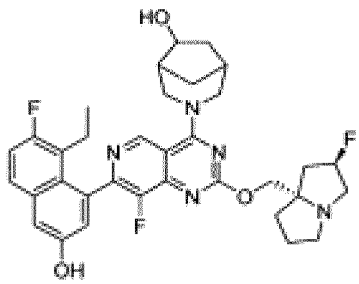,

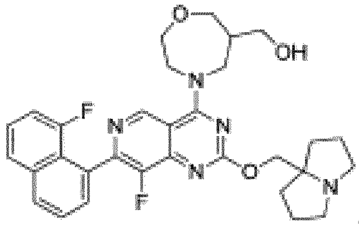, 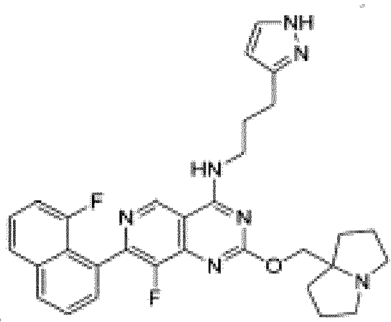,

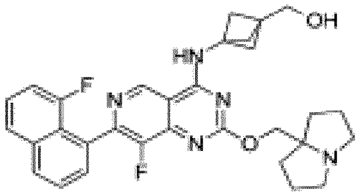, 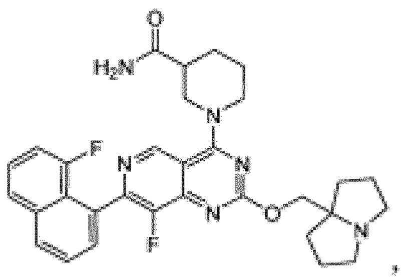,

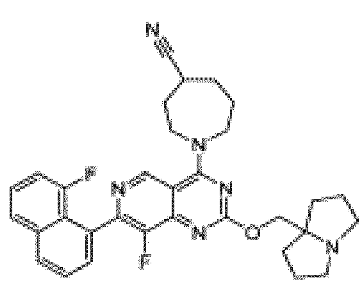,
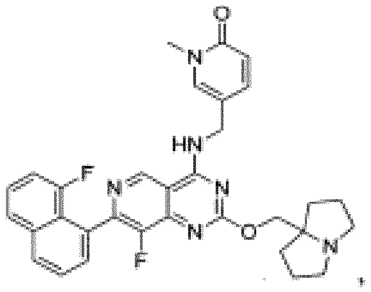,

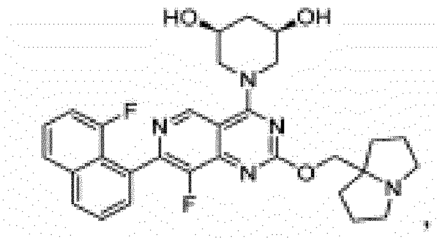,
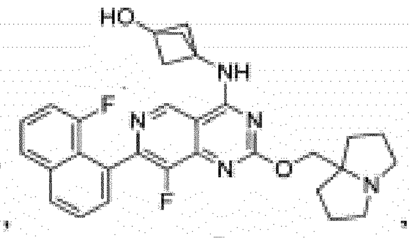,

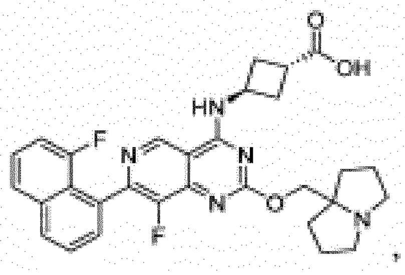,
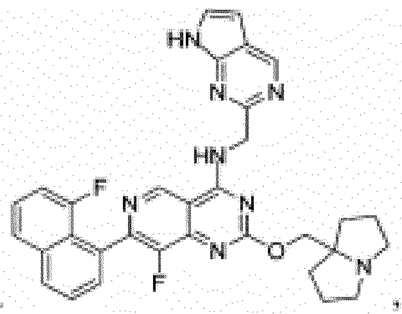,

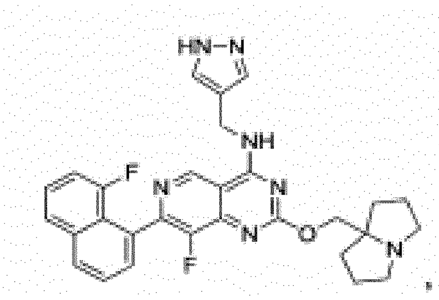,
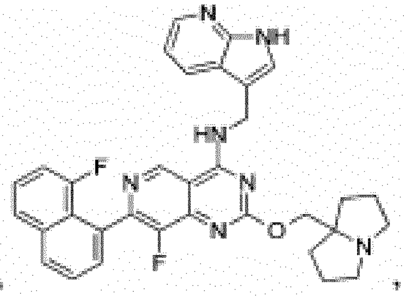,

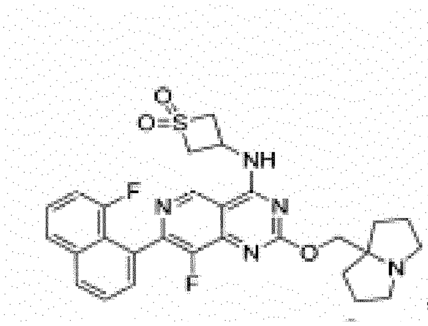,
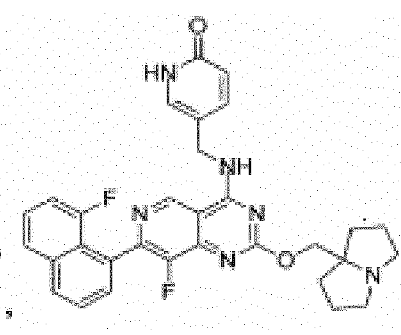,

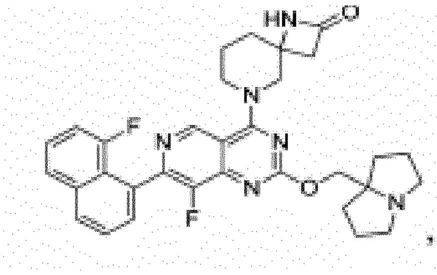, 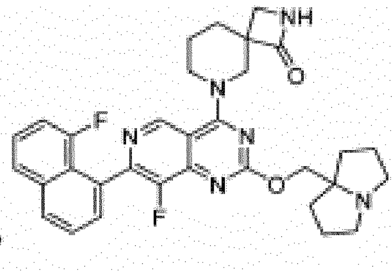,

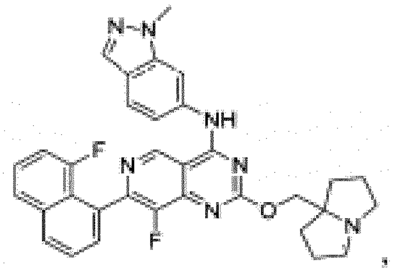, 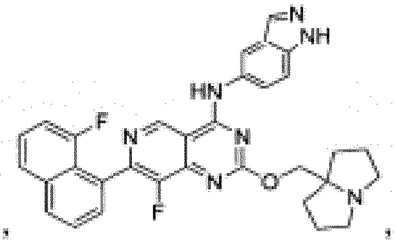,

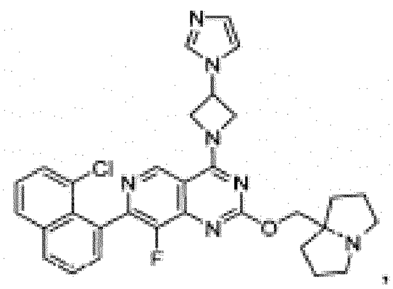, 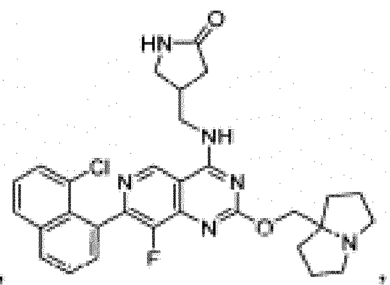,

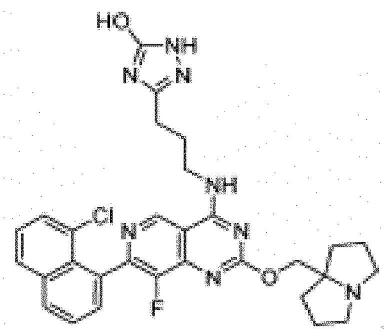, 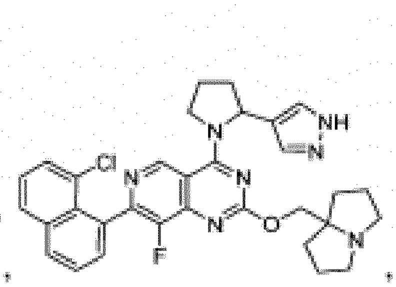,

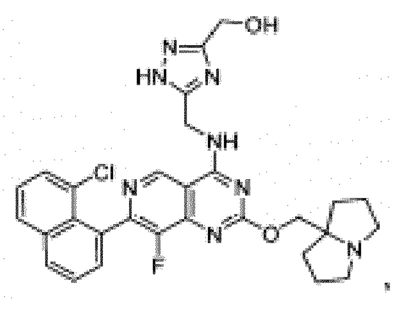, 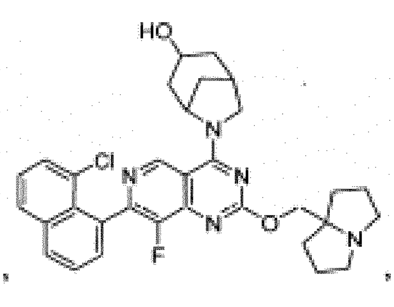,

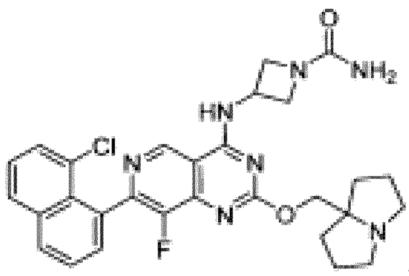,
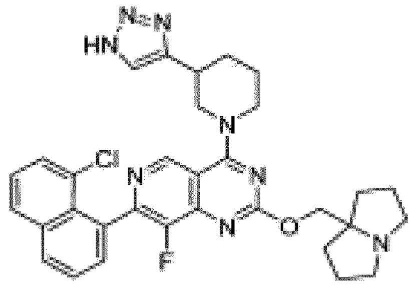,

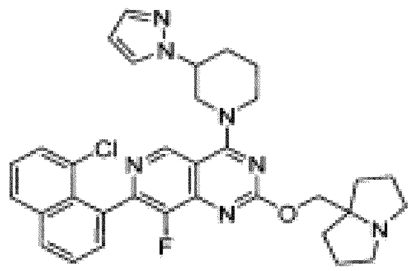,
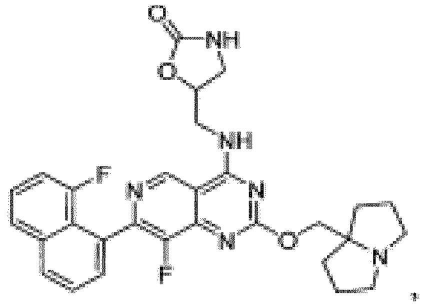,

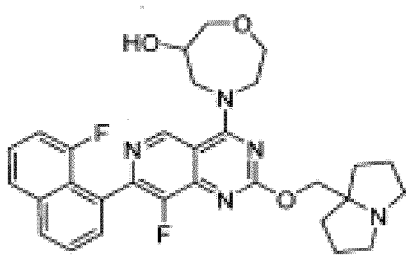,
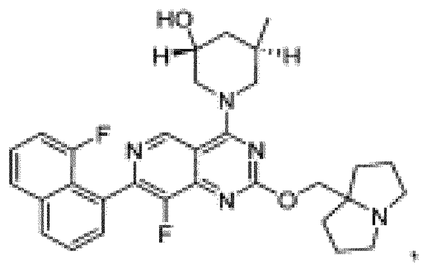,

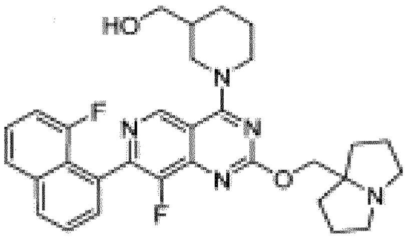,
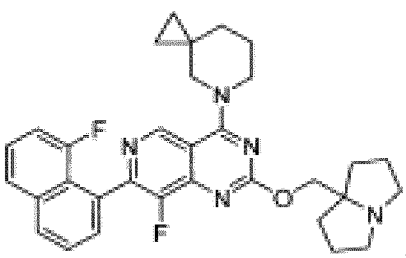,

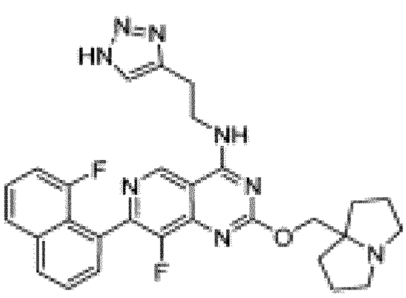,
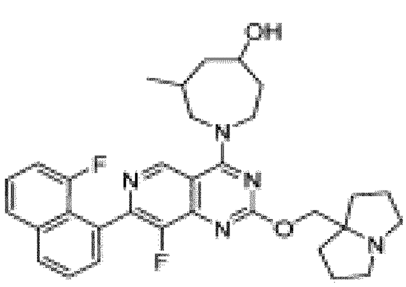,

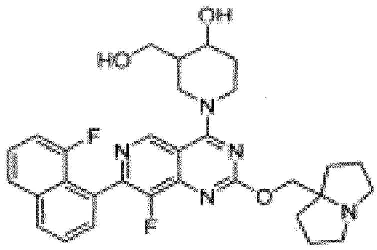,
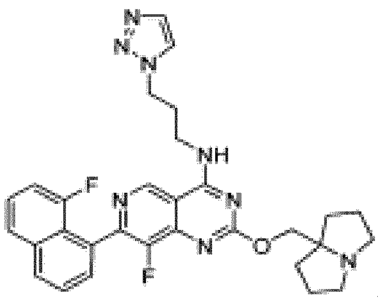,

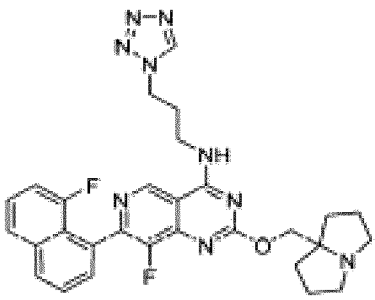,
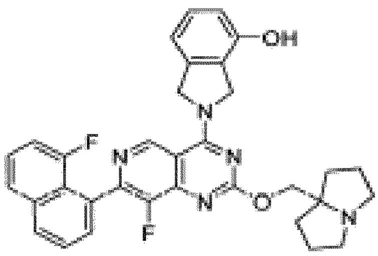,

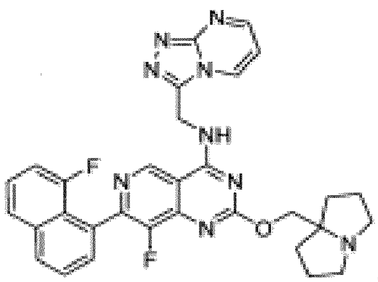,
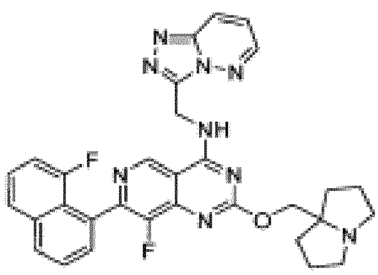,

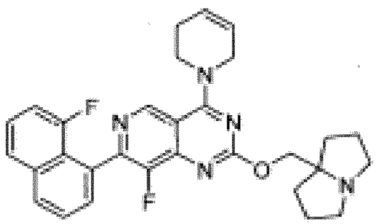,
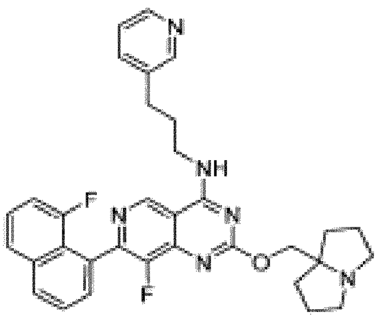,

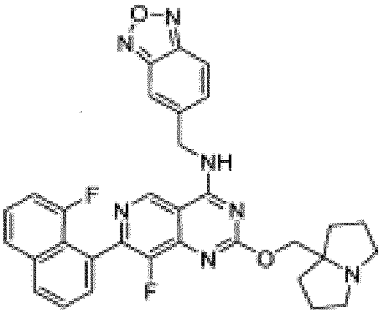,
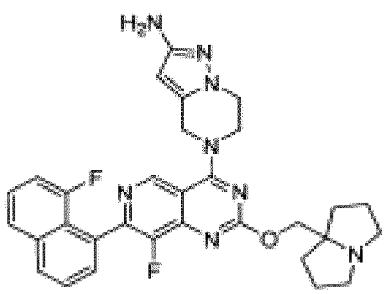,

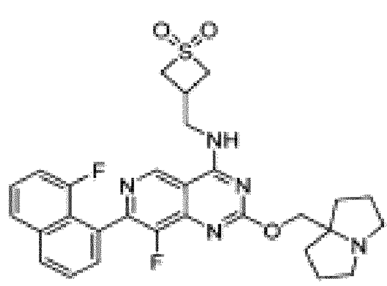, 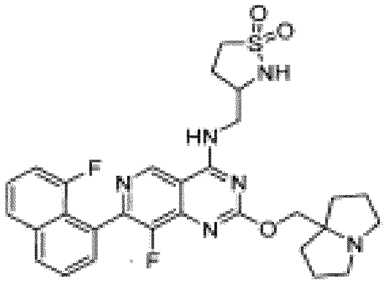,

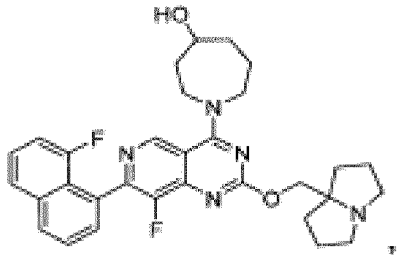, 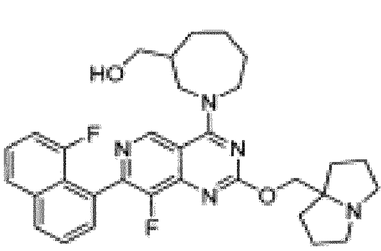,

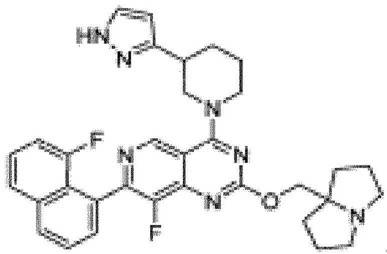, 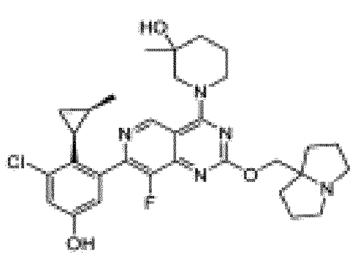,

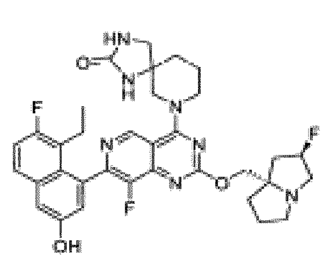, 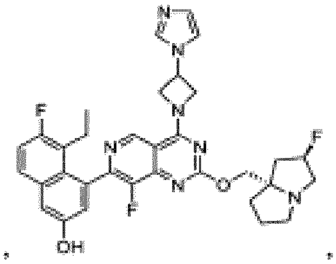, 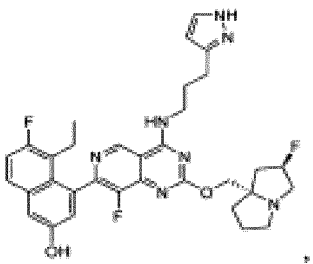,

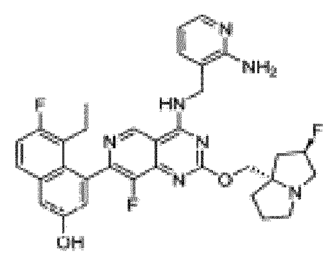, 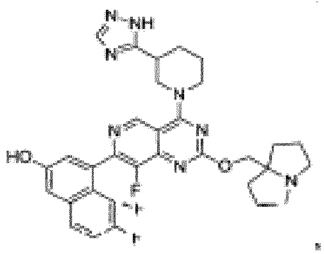, 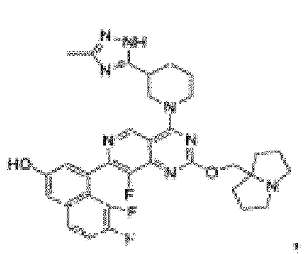,

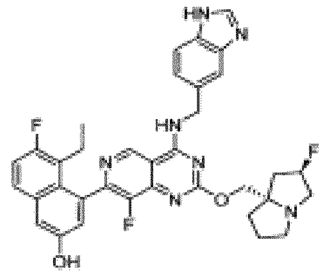 , 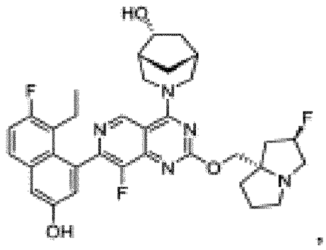 , 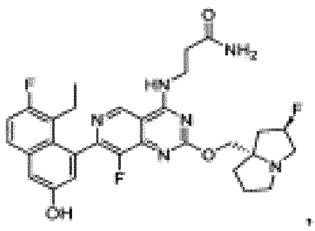 ,

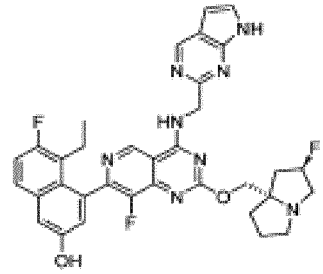 , 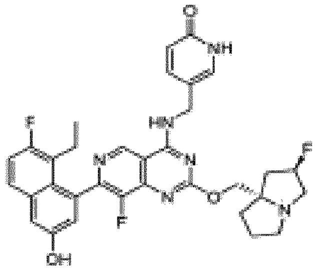 ,

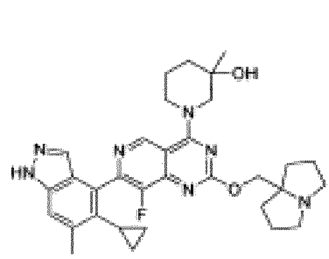 , 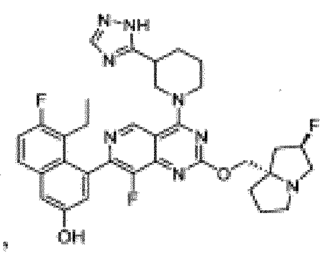 ,

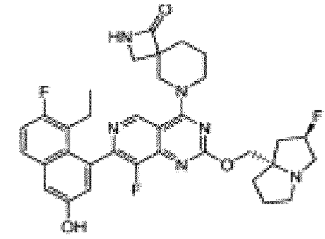 , 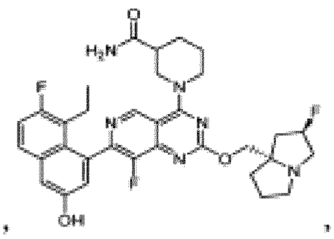 , 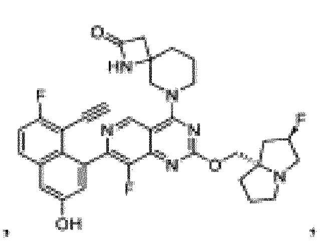 ,

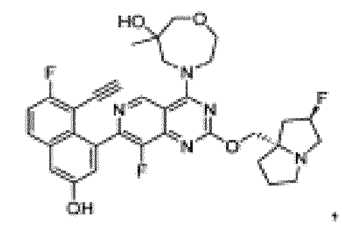 , 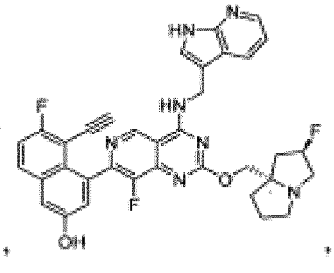 , 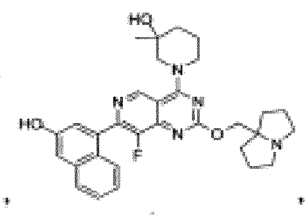 ,

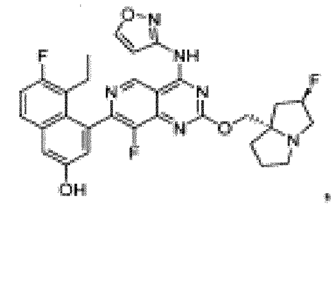 , 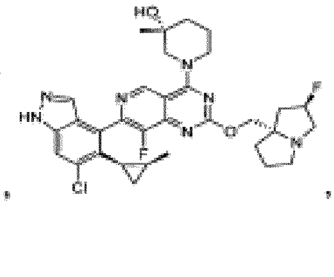 , 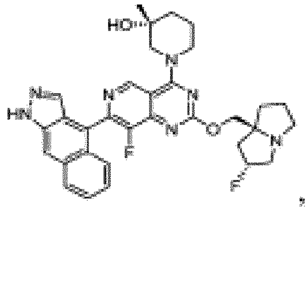 ,

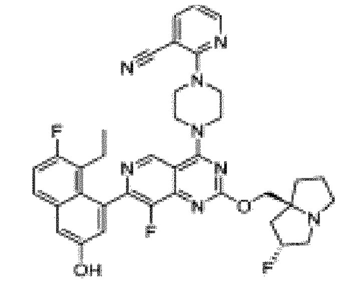, 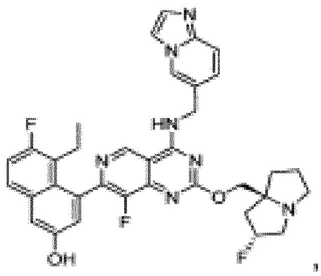, 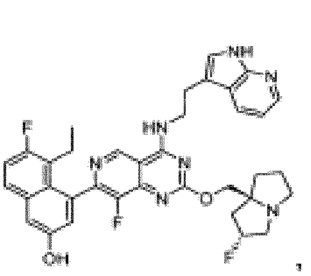,

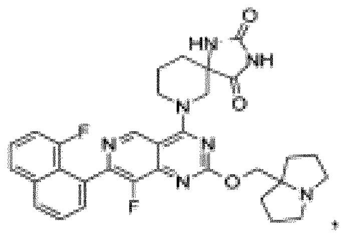, 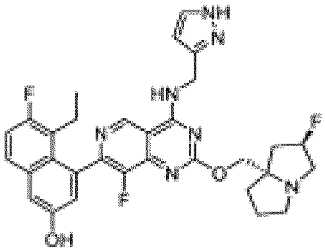, 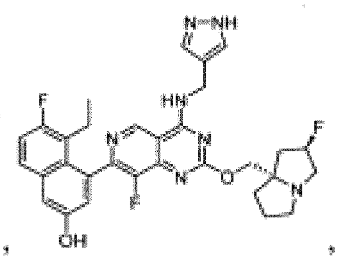,

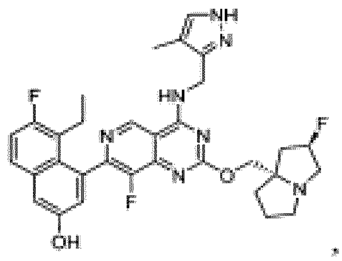, 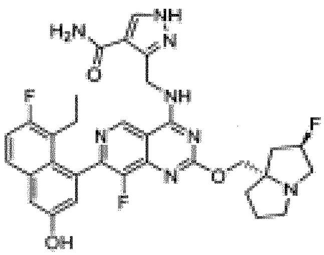, 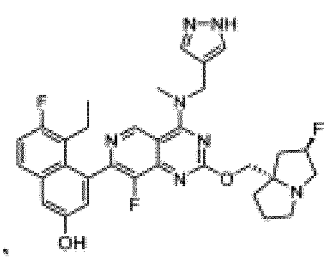,

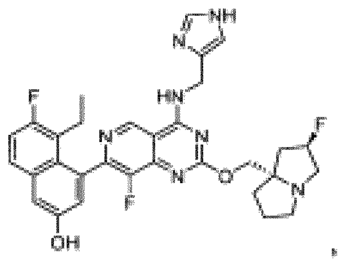, 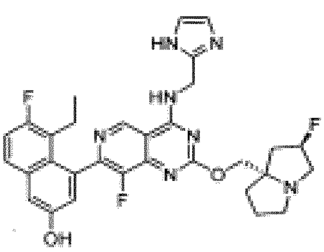, 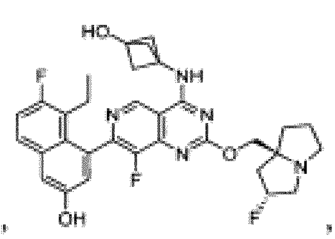,

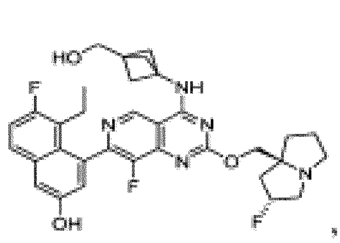, 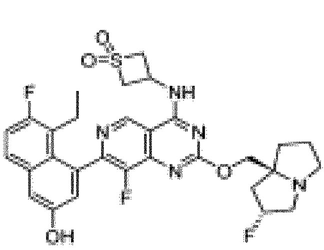, 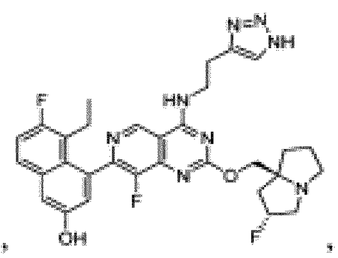,

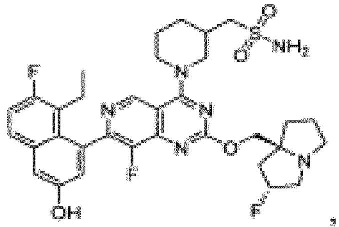 , 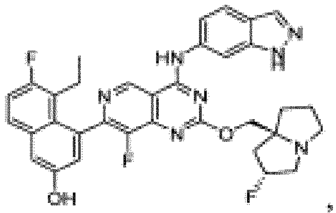 , 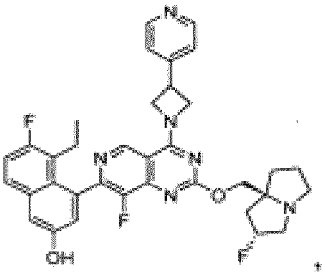 ,

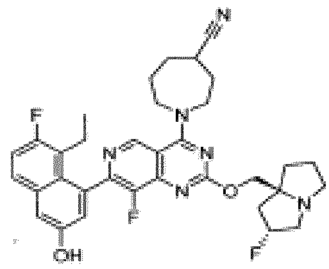 , 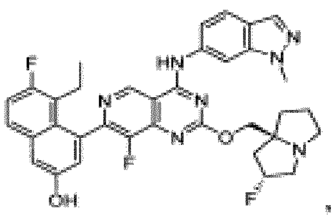 , 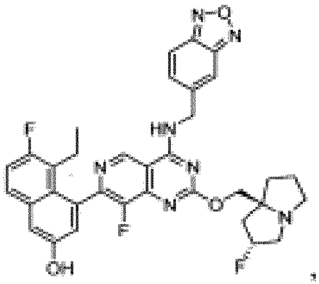 ,

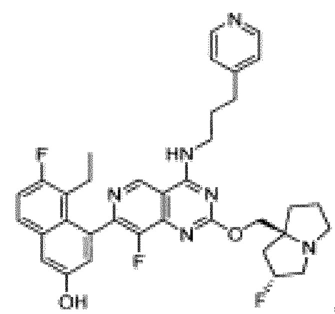 , 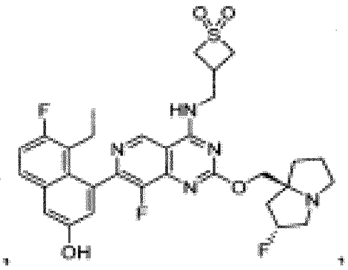 , 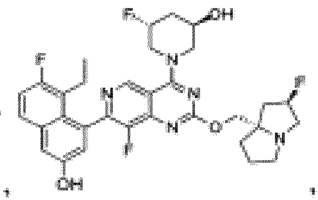 ,

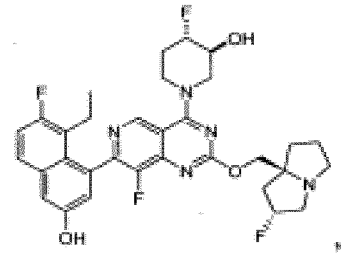 , 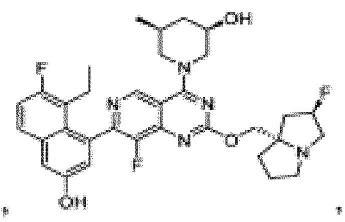 , 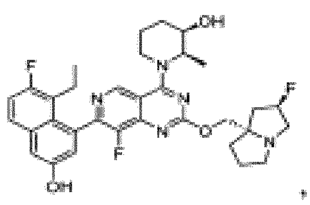 ,

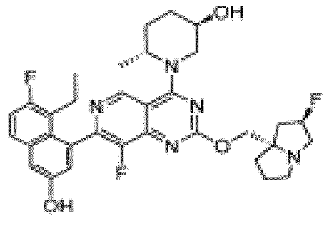 , 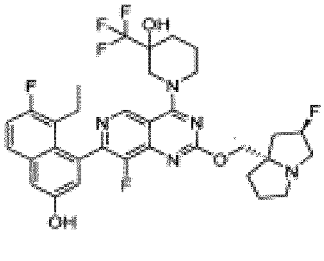 , 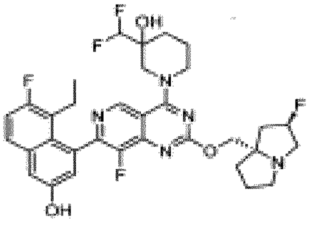 ,

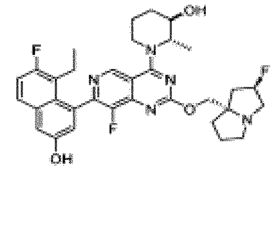 , 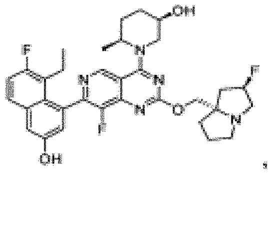 , 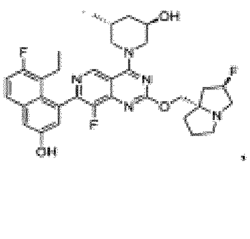 ,

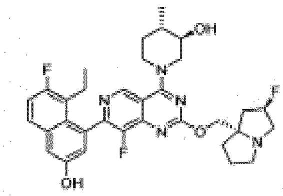 , 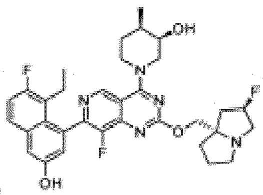 , 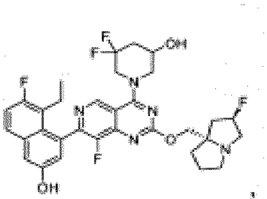 ,

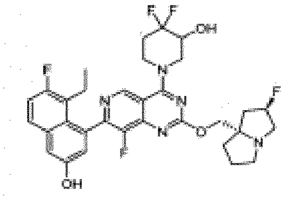 , 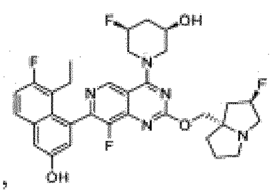 , 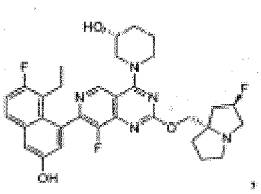 ,

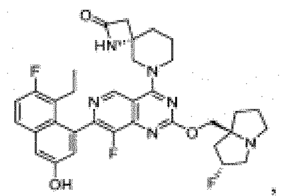 , 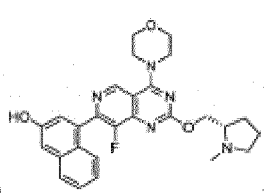 , 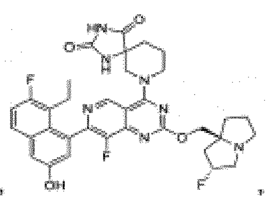 ,

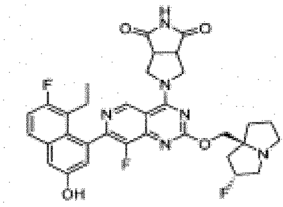 , 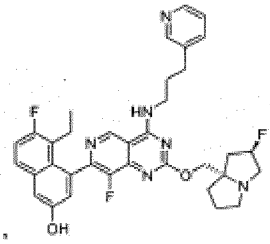 , 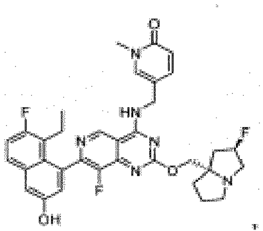 ,

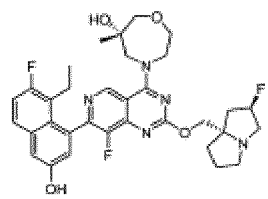 , 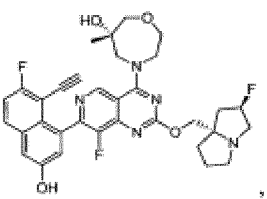 ,

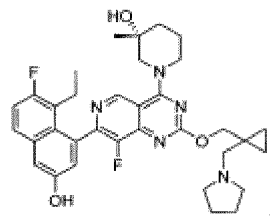 , 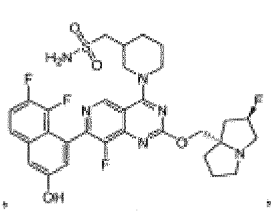 , 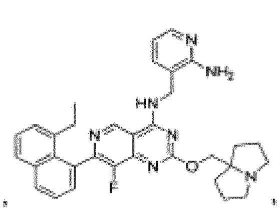 ,

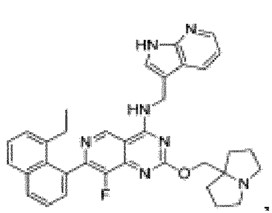 , 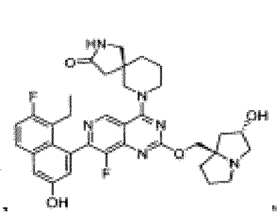 ,

iii) the nitrogen-containing heterocyclic compound of formula **I** is not any one of the following compounds:

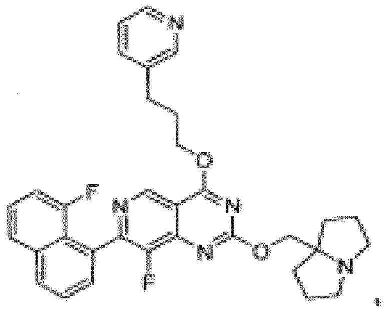,
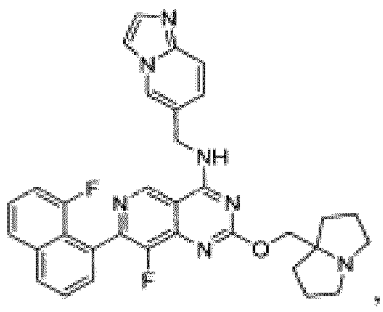,
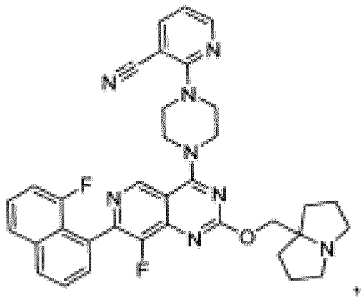,
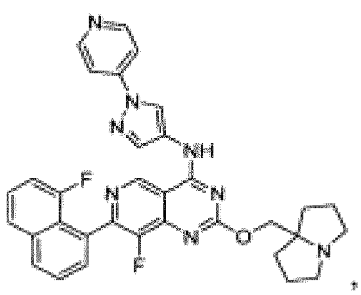,
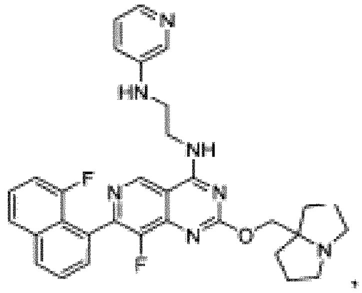,
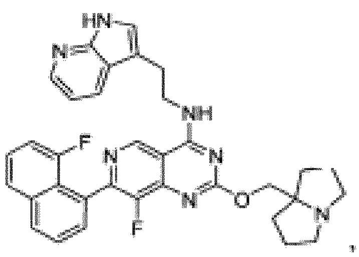,
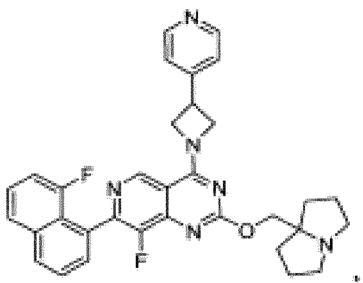,
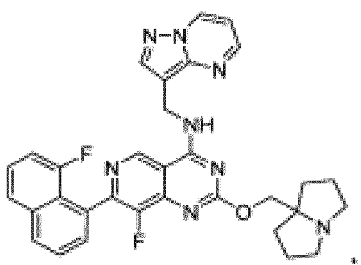,
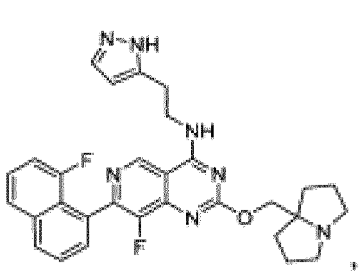,
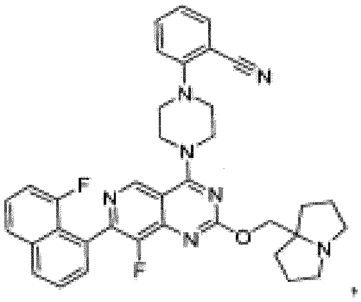,

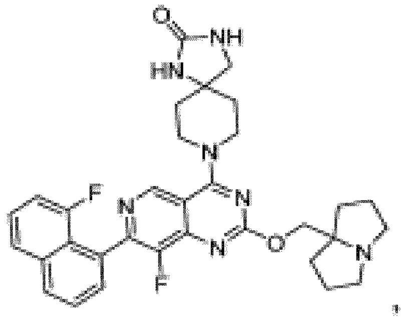,
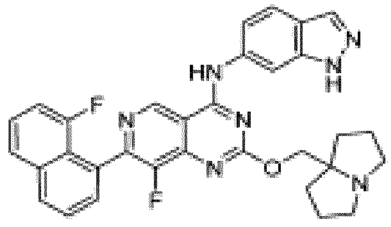,

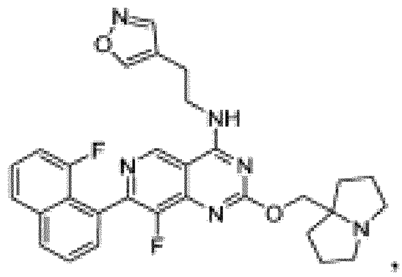,
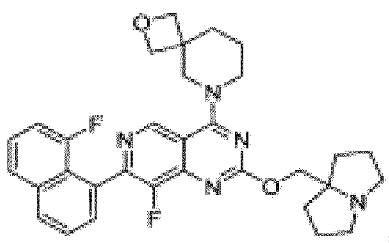,

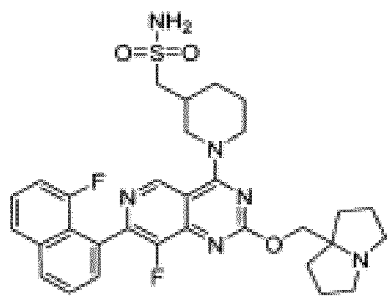,
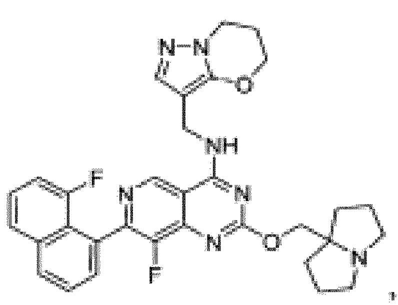,

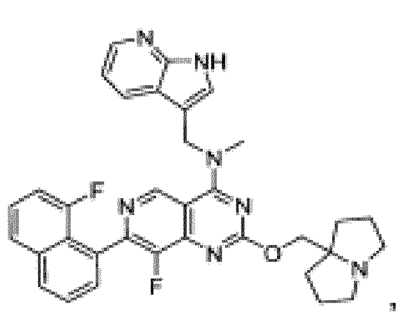,
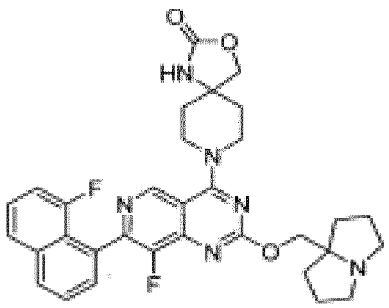,

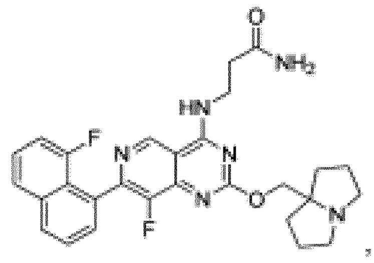,
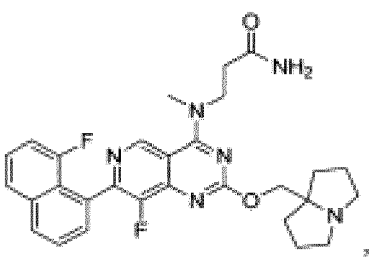,

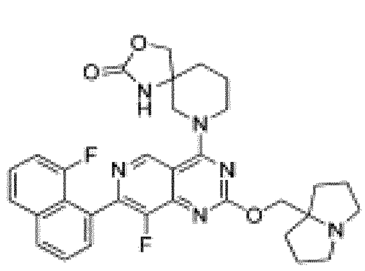,
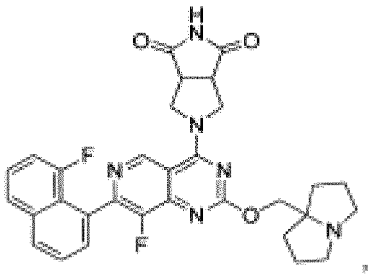,
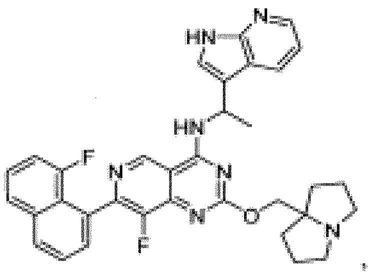,
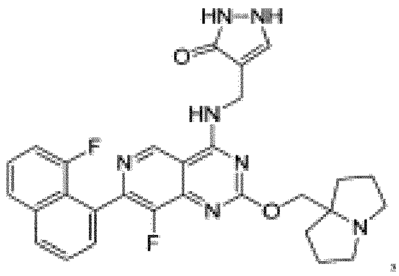,
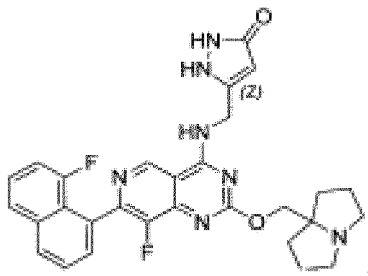,
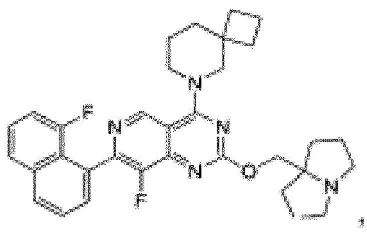,
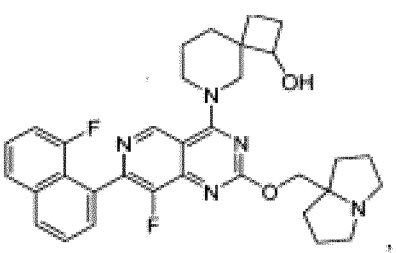,
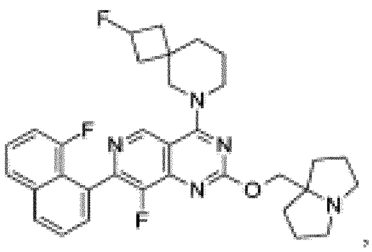,
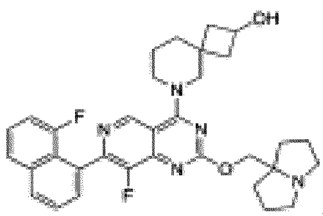,
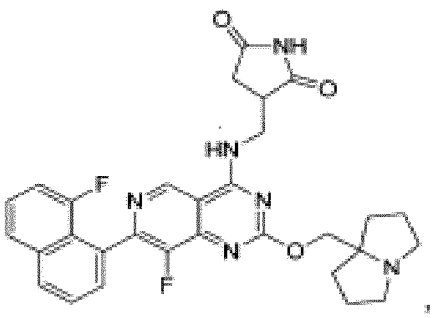,

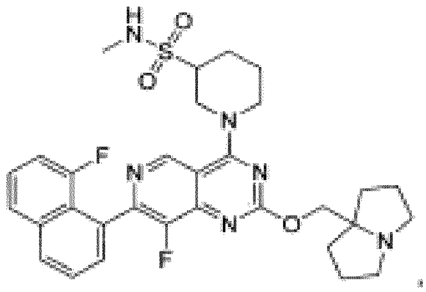,

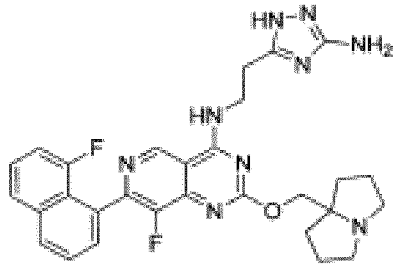,

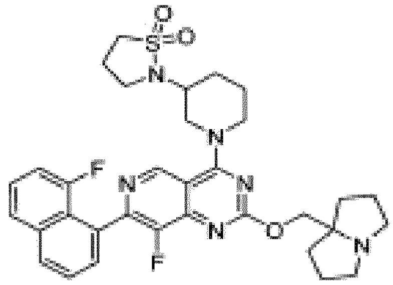,

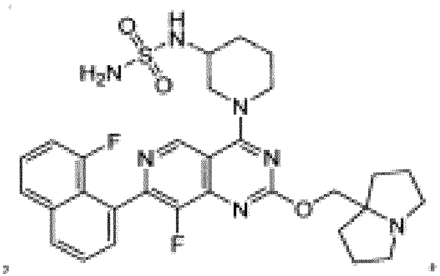,

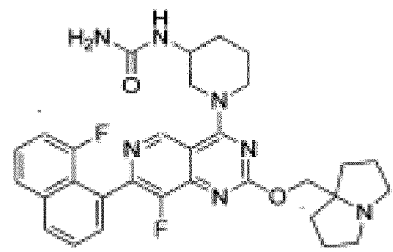,

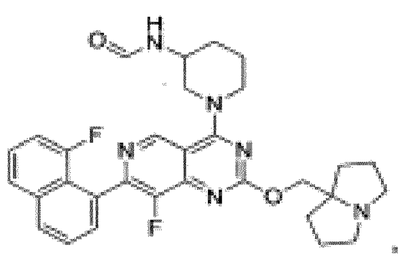,

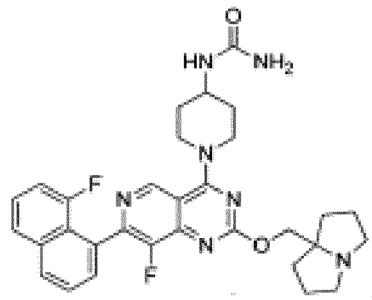,

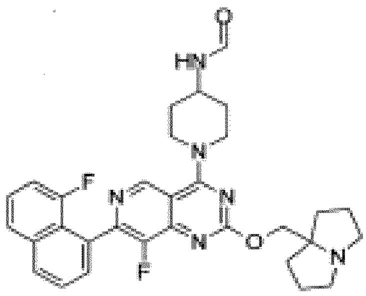,

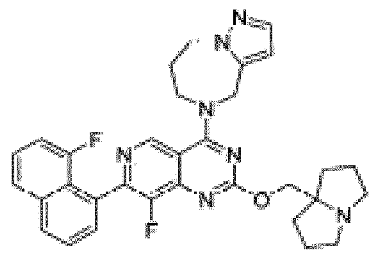,

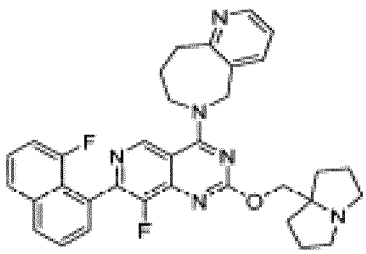,

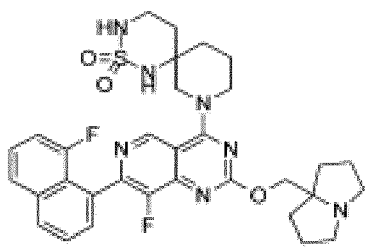 , 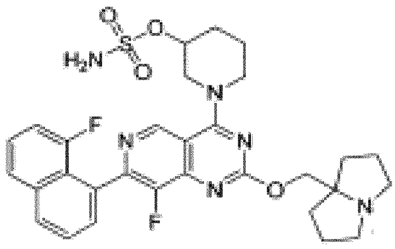 ,

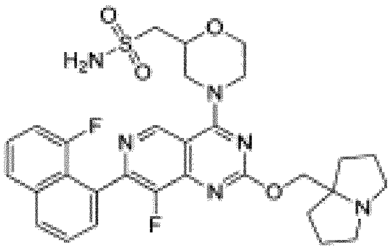 , 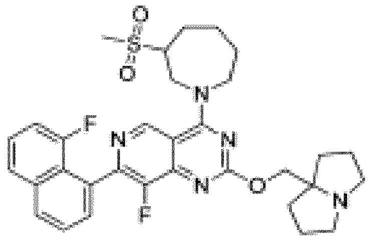 ,

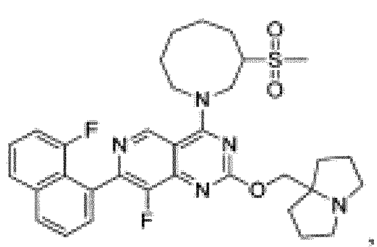 , 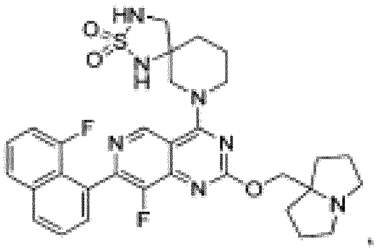 ,

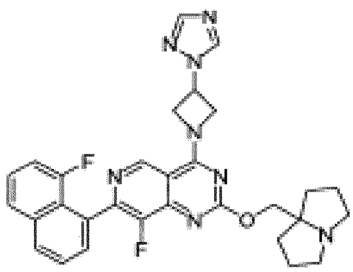 , 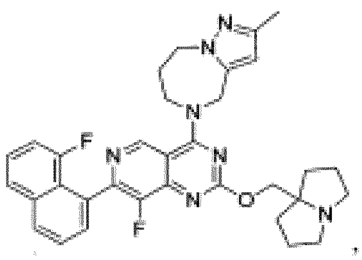 ,

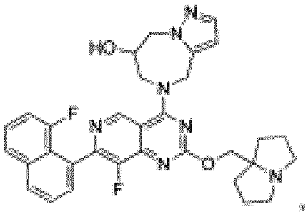 , 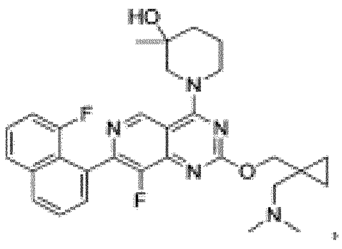 , 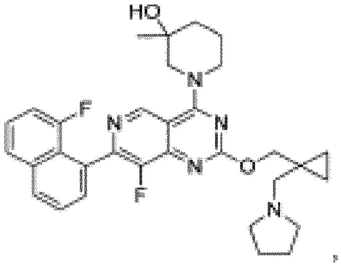 ,

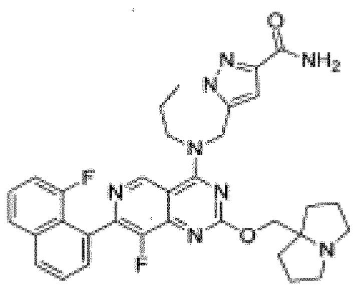
,
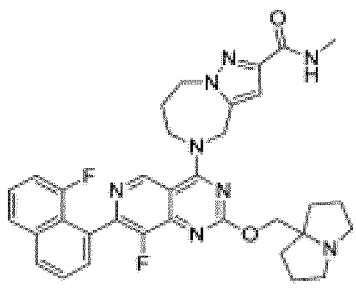
,

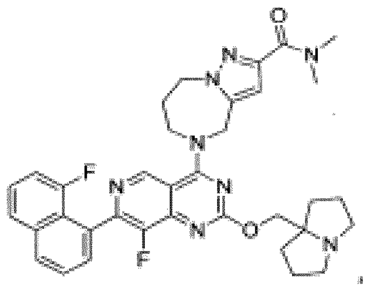
,
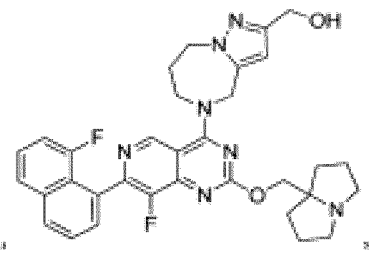
,

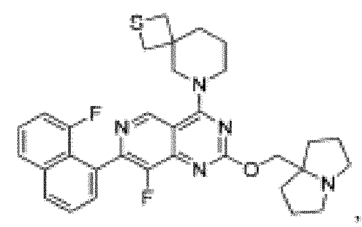
,
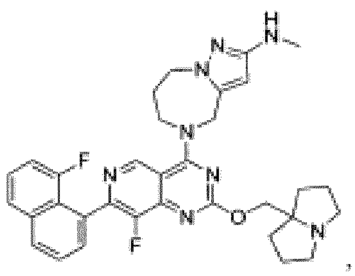
,

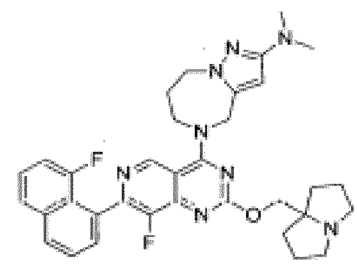
,
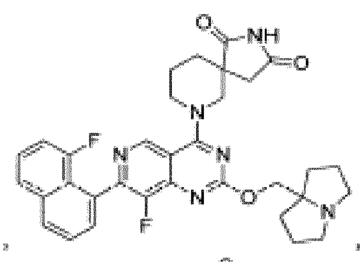
,

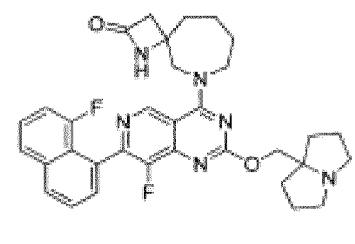
,
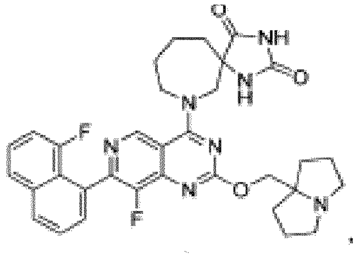
,

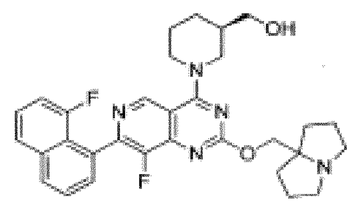,

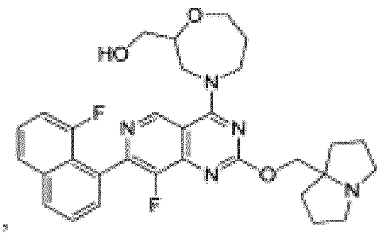,

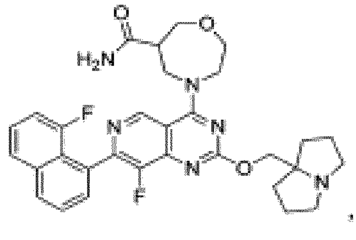,

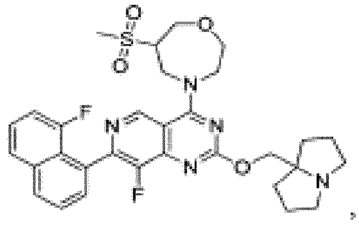,

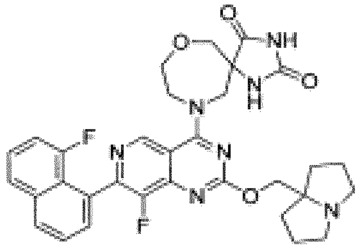,

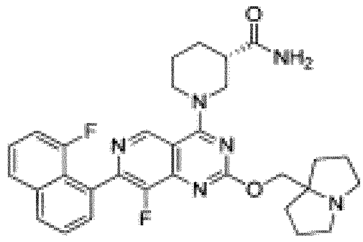,

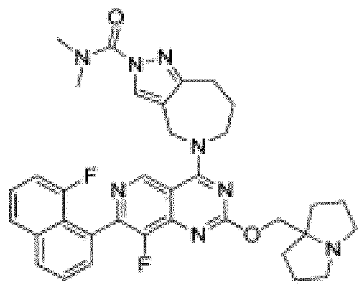,

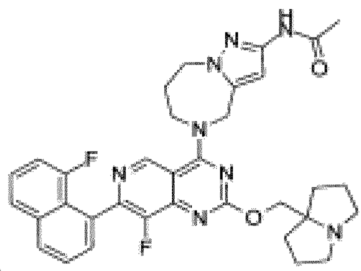,

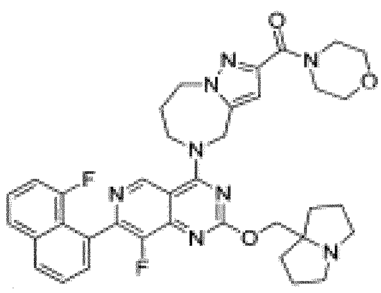,

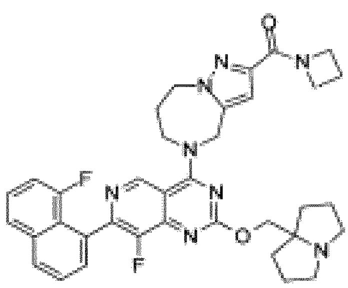,

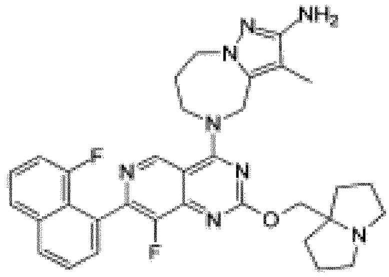,
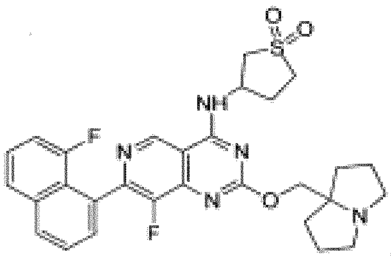,

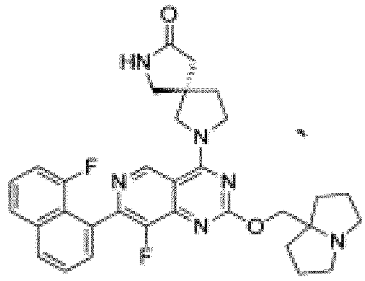,
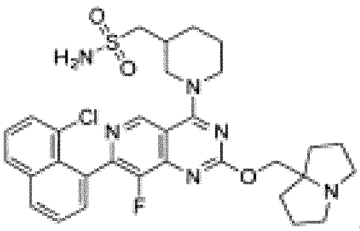,

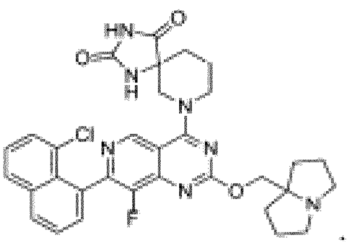,
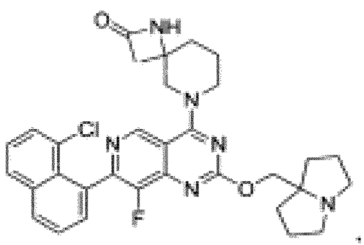,

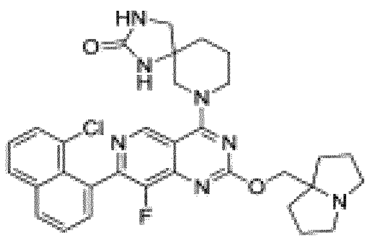,
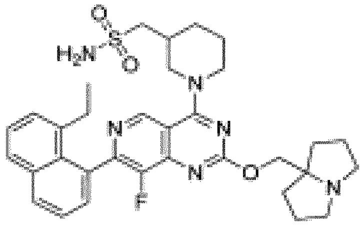,

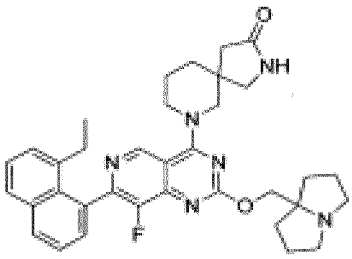,
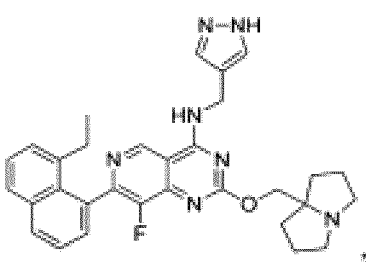,

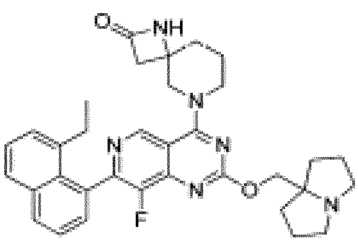,
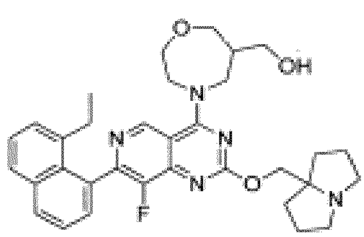,

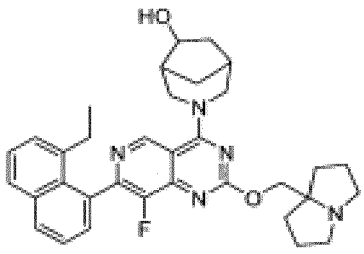,
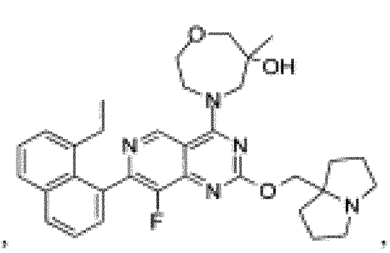,

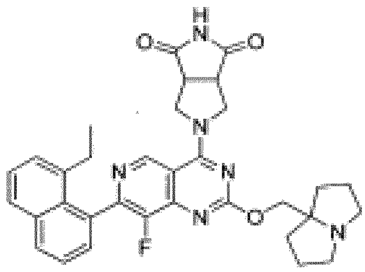,
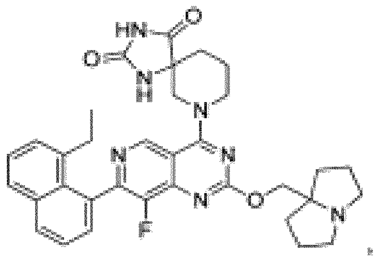,

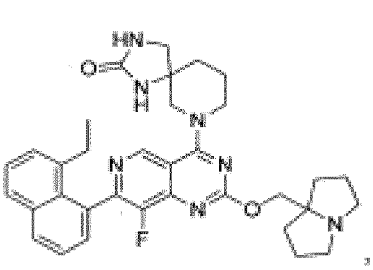,
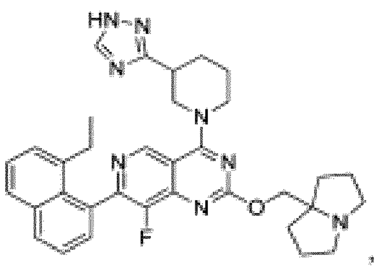,

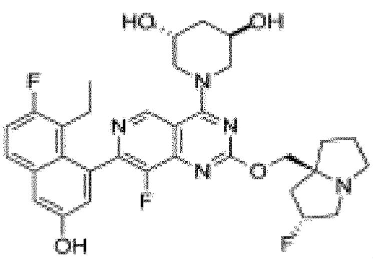,
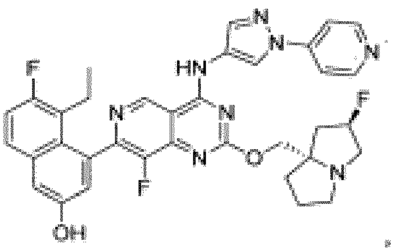,

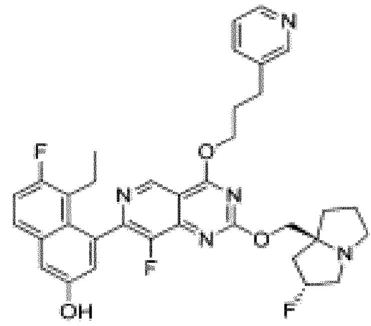,
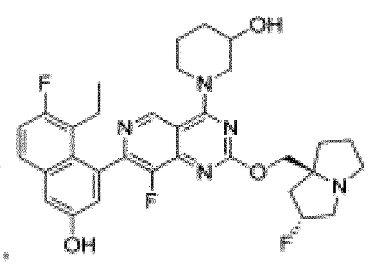,

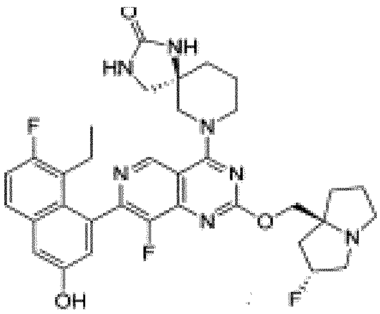
,
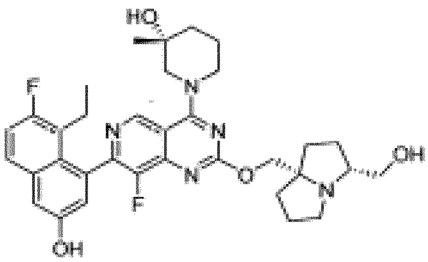
,

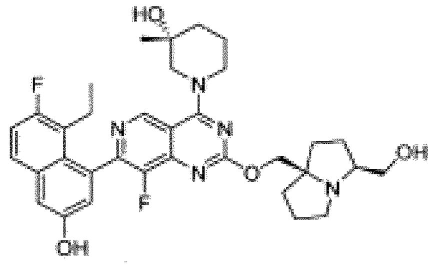
,
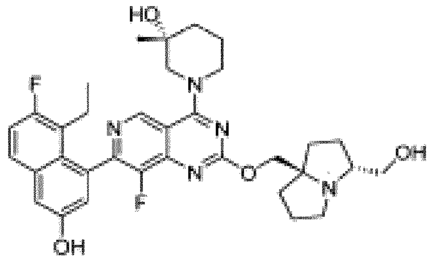
,

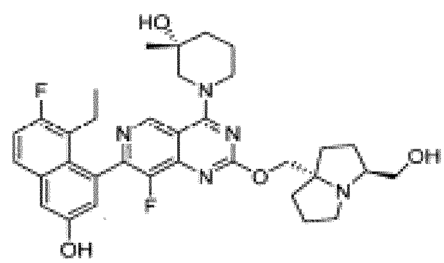
,
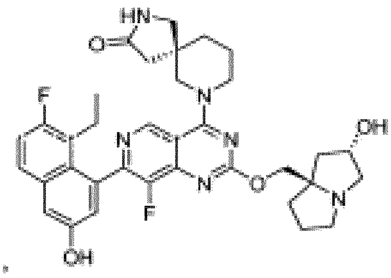
,

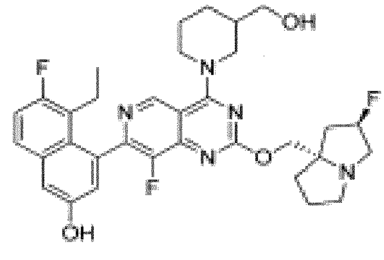
,
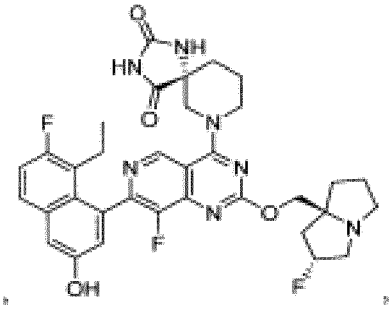
,

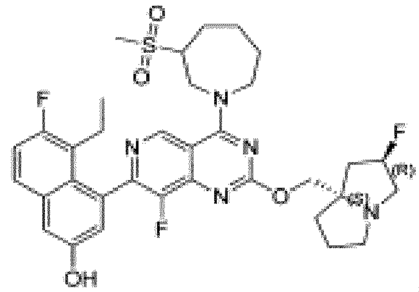
,
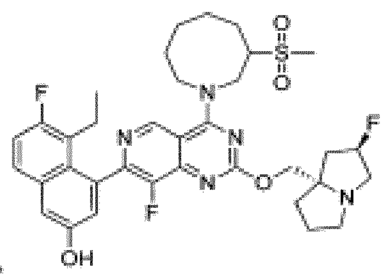
,

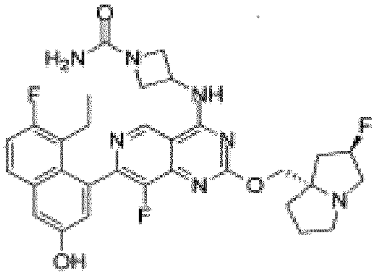
,
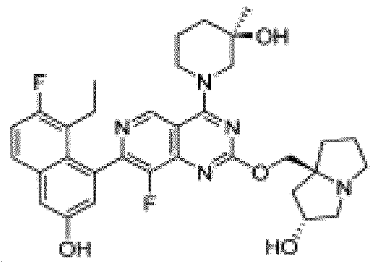
,

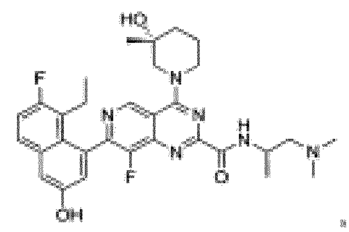
,
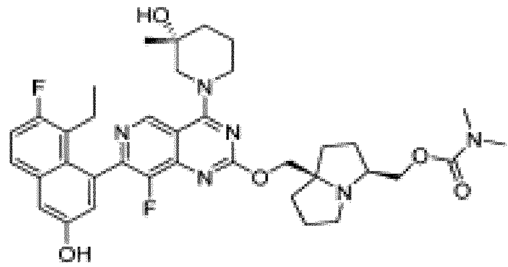
,

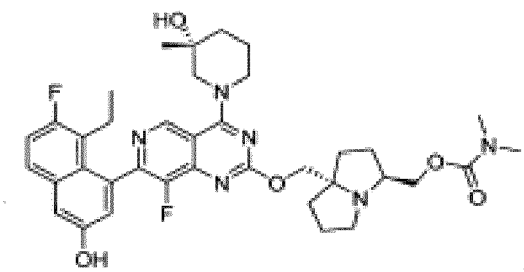
,
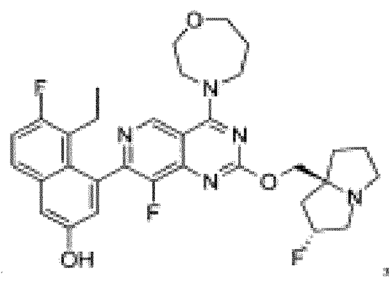
,

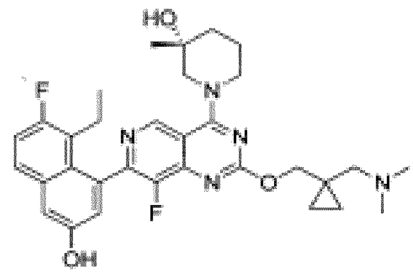
,
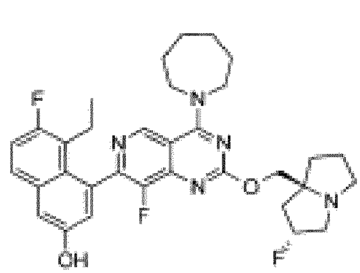
,

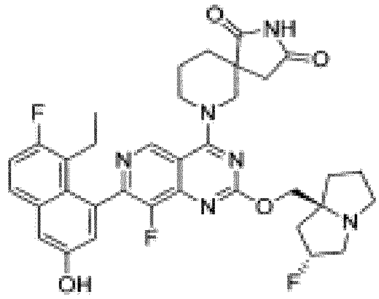
,
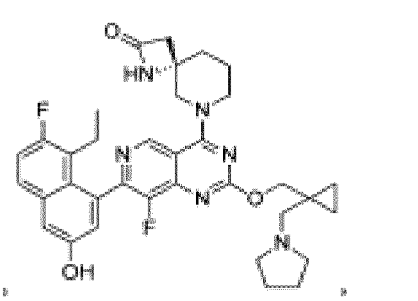
,
,

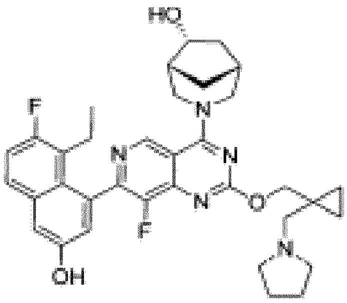
,
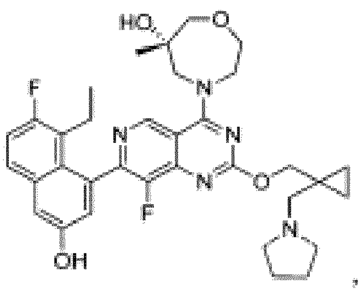
,
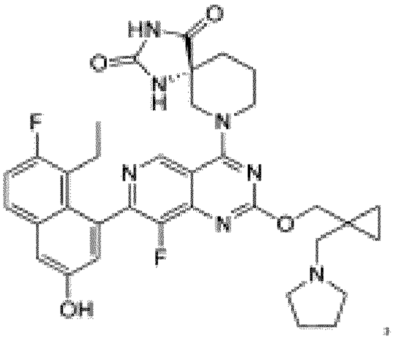
,
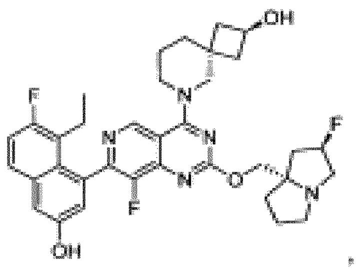
,
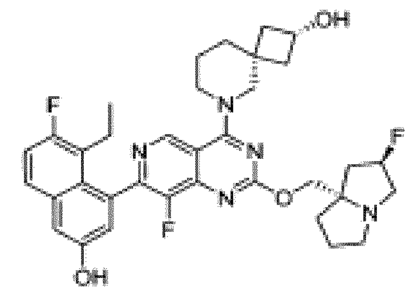
,
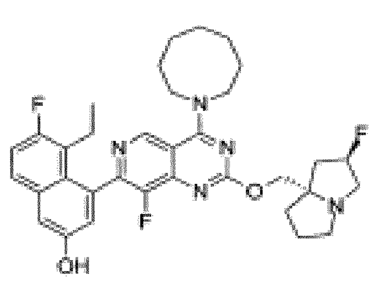
,
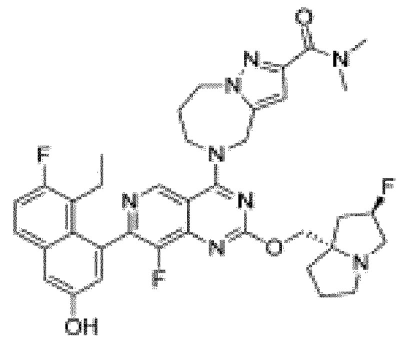
,
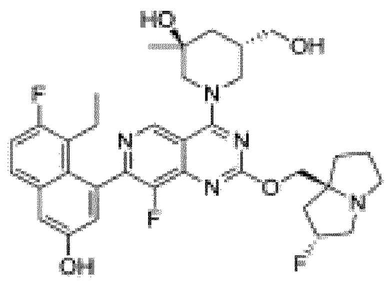
,
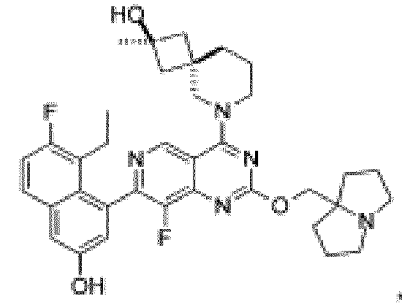
,
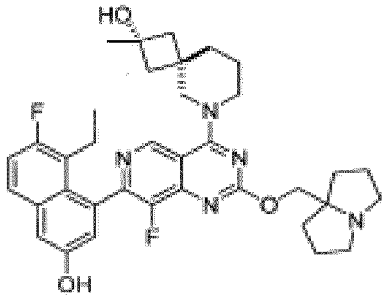
,

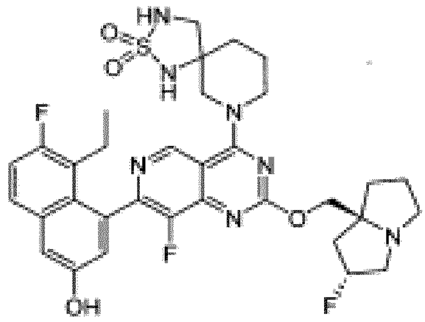
,
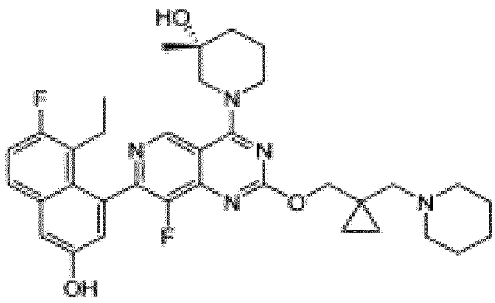
,

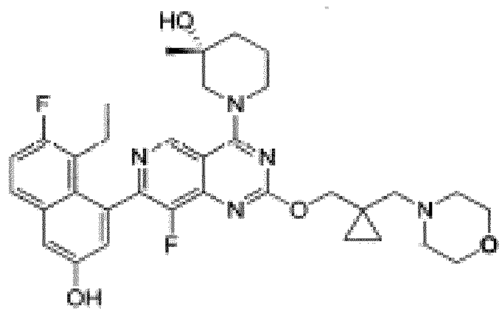
,
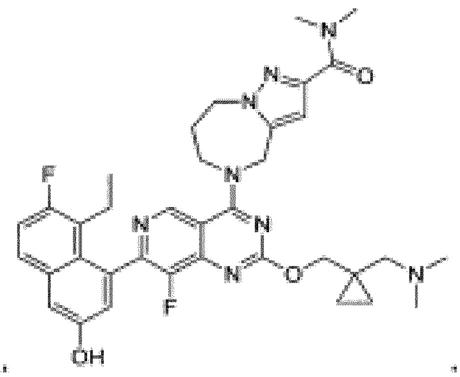
,

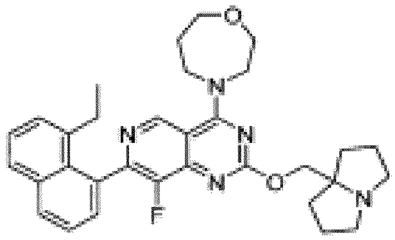
,
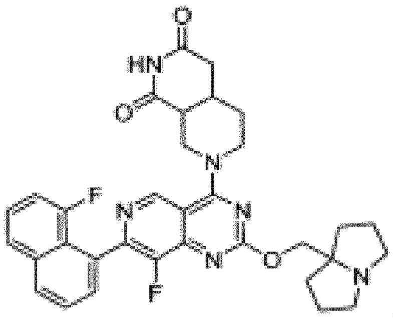
,

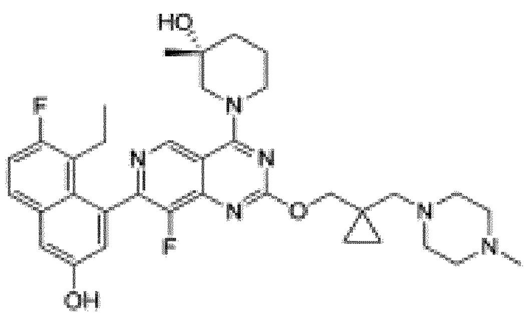
,
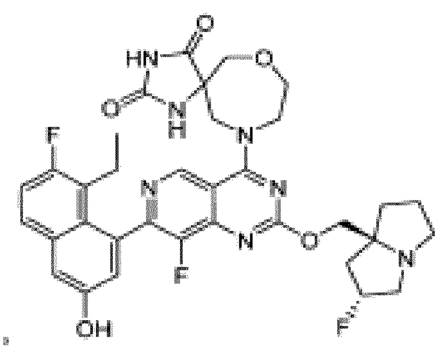
,

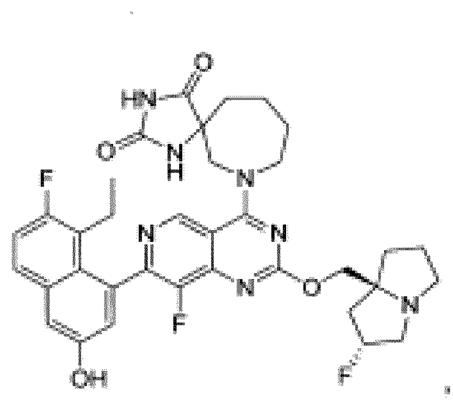,
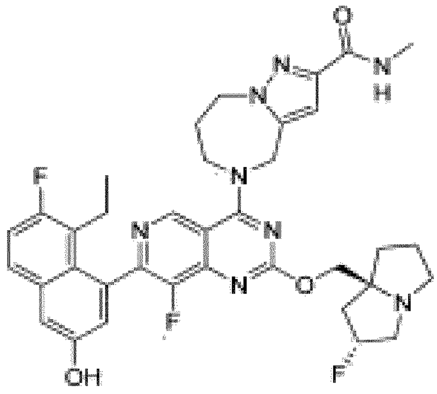,

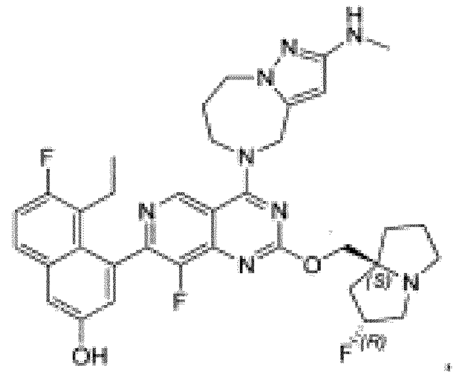,
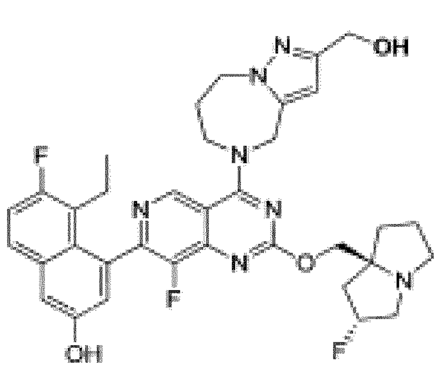,

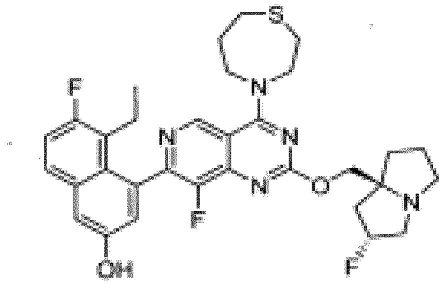,
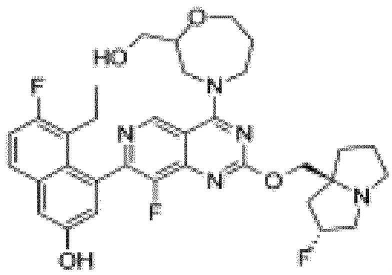,

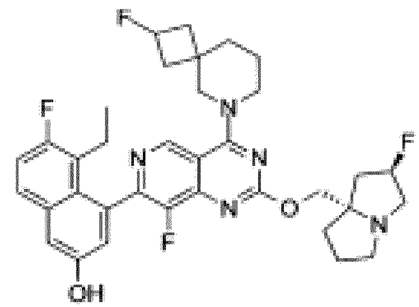,
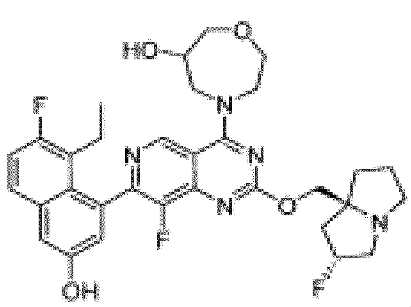,

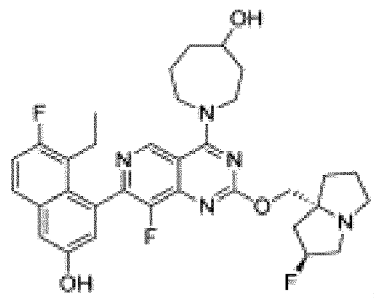, 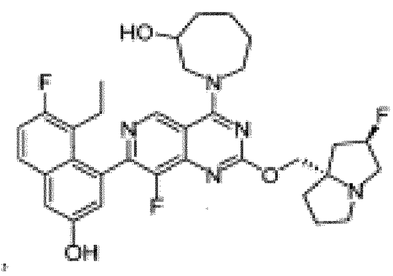,

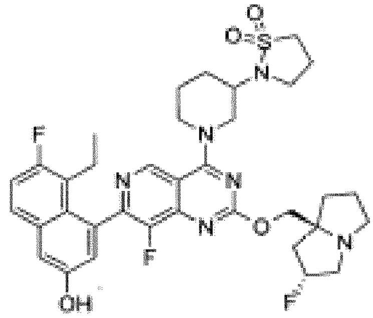, 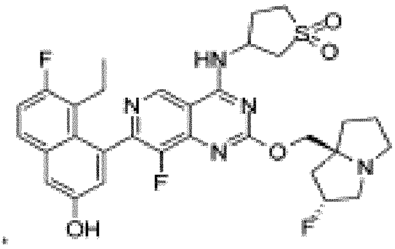,

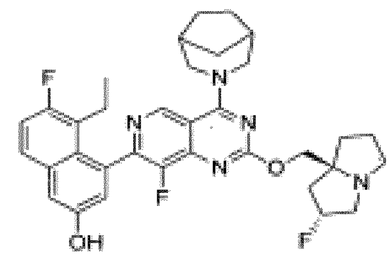, 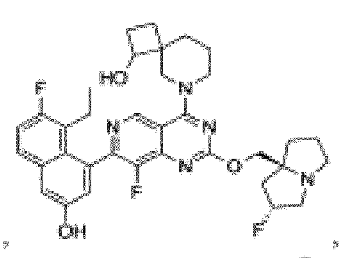,

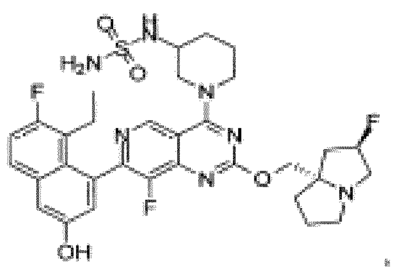, 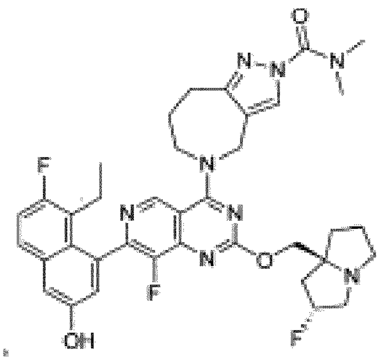,

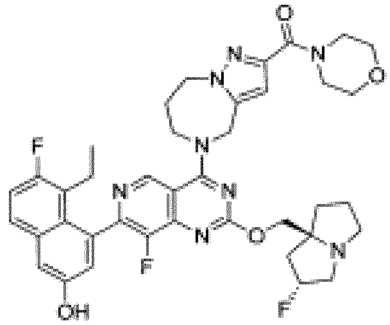, 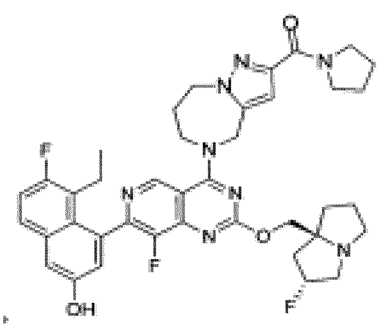,

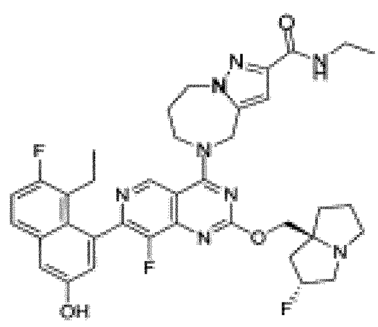,
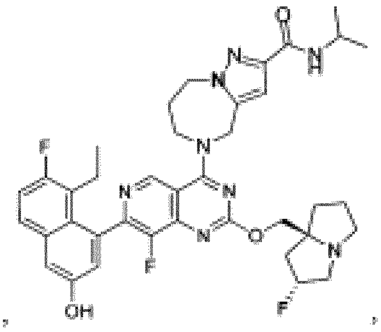,
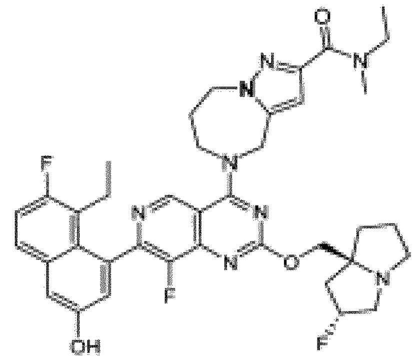,
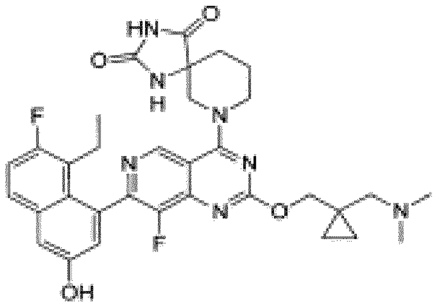,
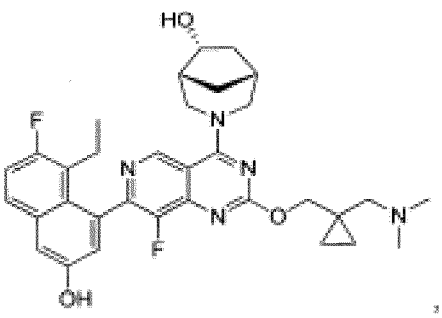,
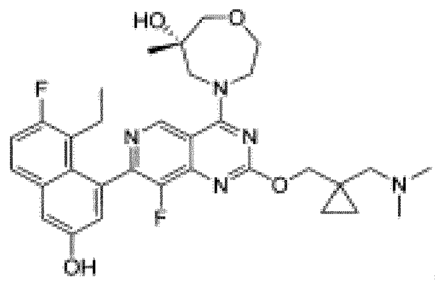,
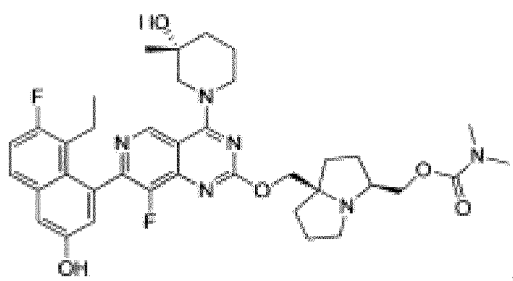,
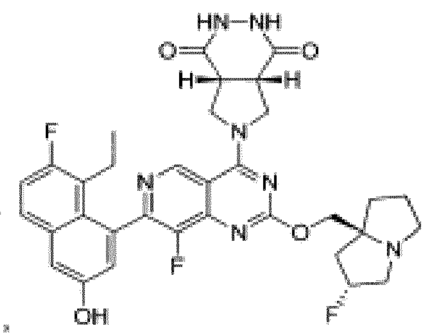,

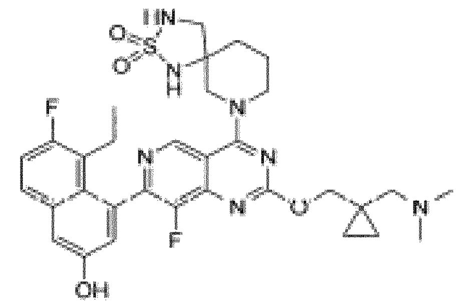
,
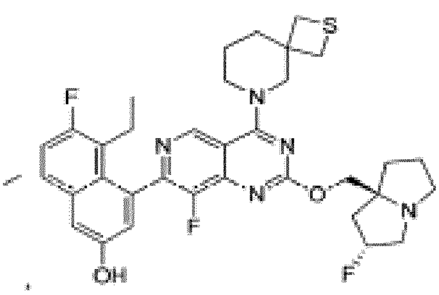
,

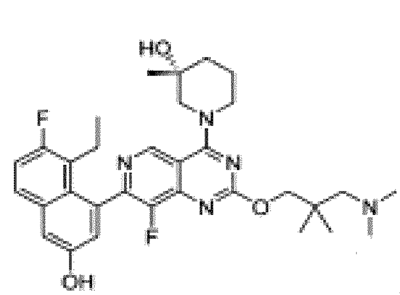
,
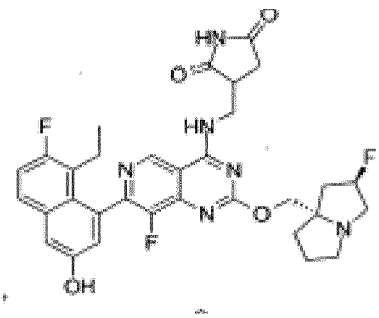
,

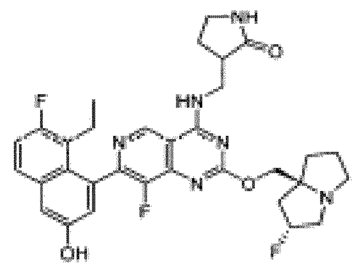
,
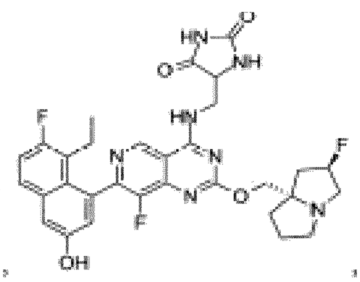
,

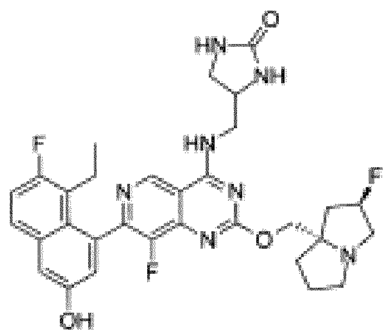
,
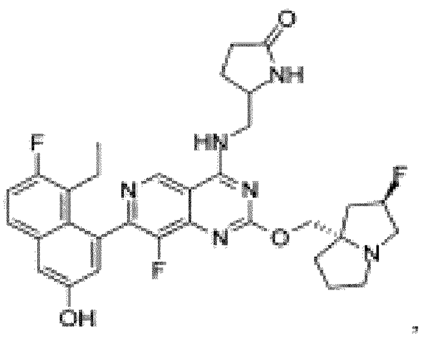
,

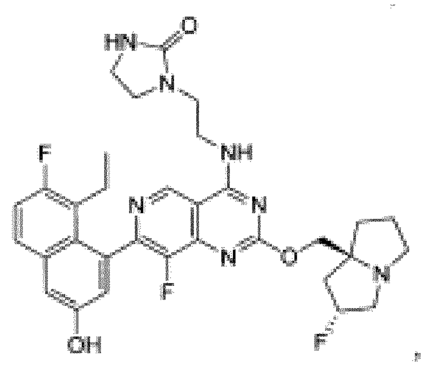
,
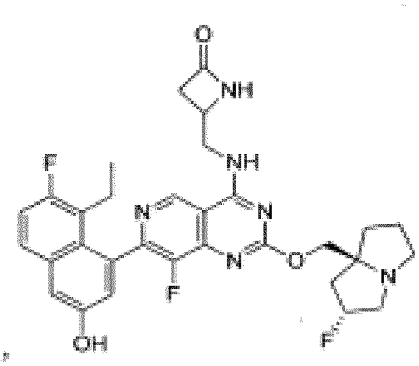
,

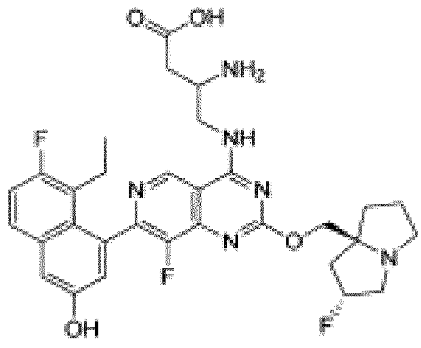,
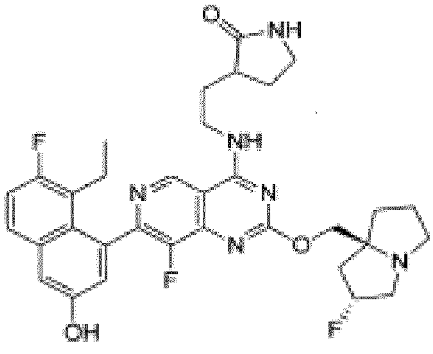,

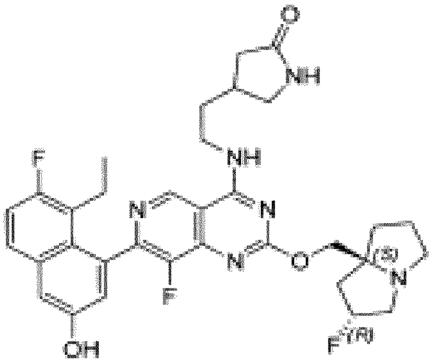,
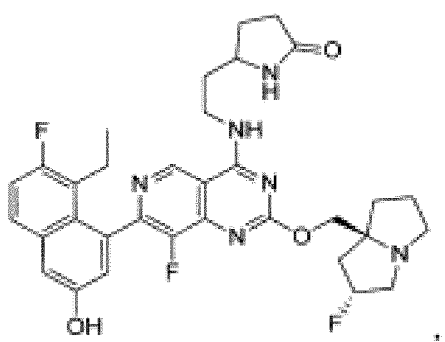,

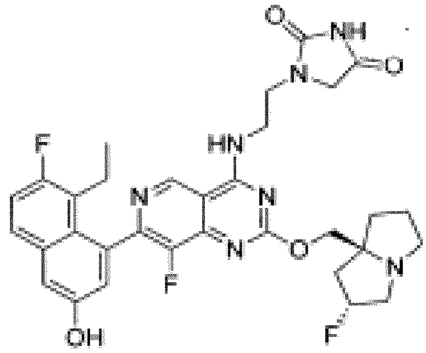,
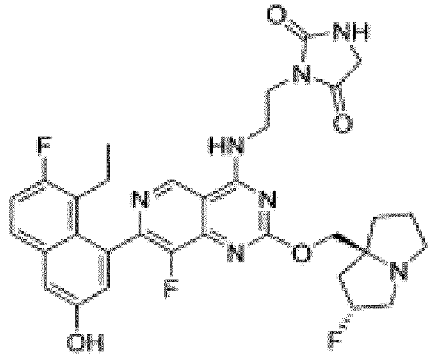,

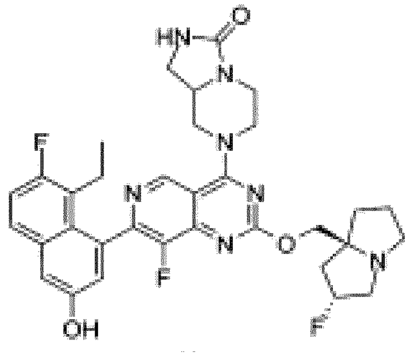,
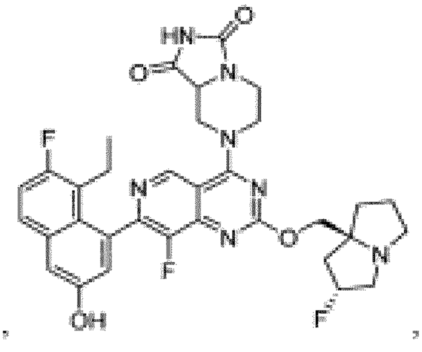,

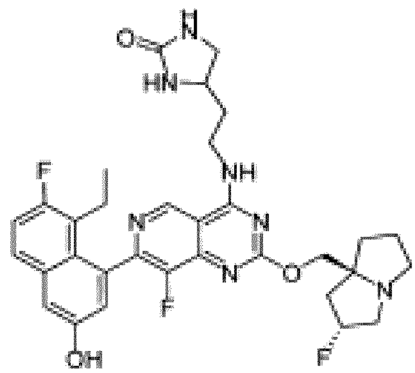
,
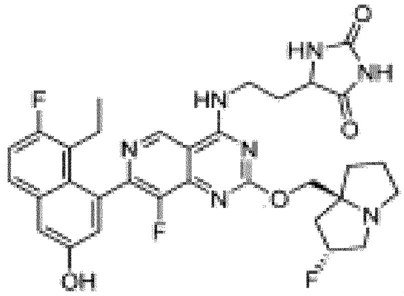
,
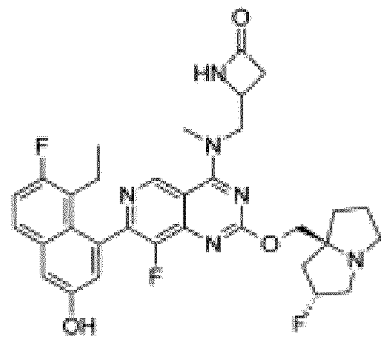
,
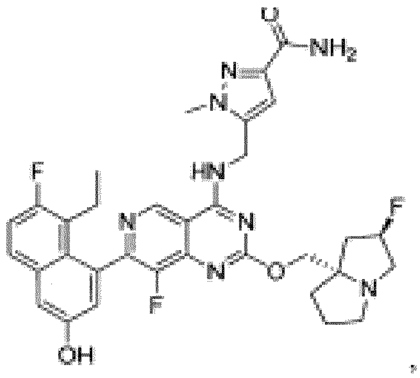
,
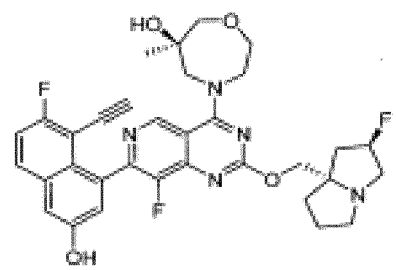
,
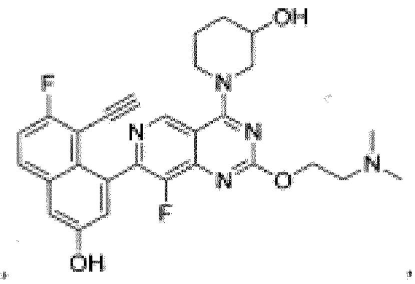
,
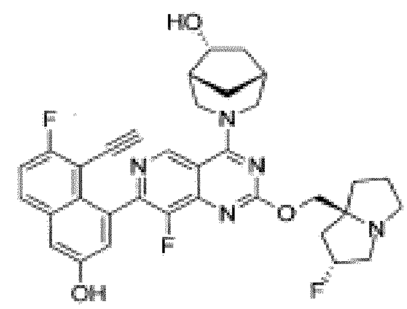
,
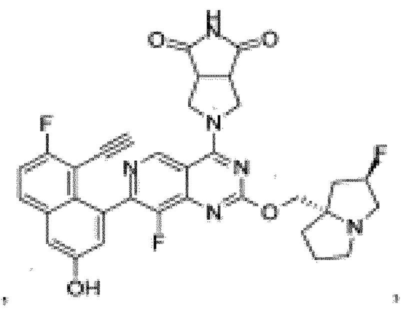
,
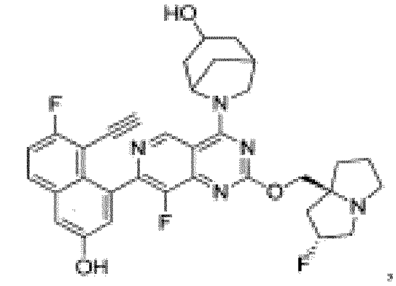
,
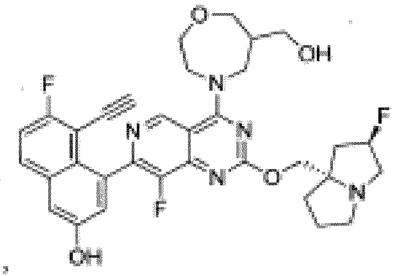
,

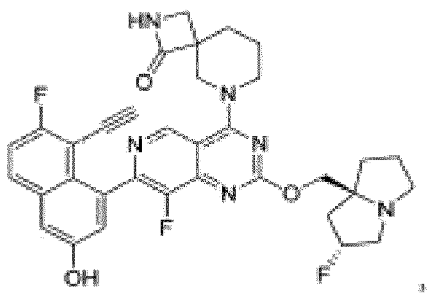
,
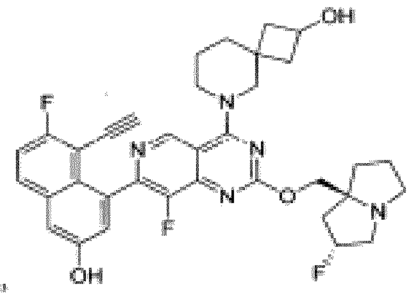
,

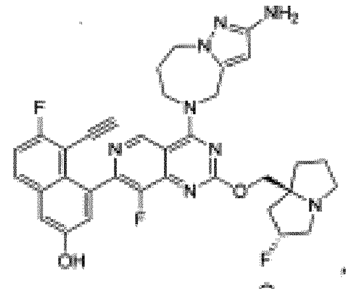
,
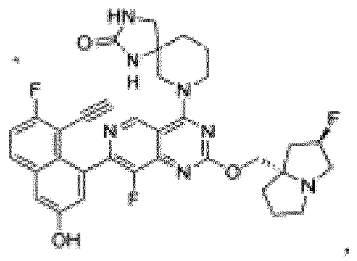
,

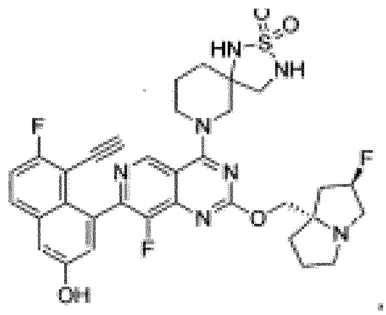
,
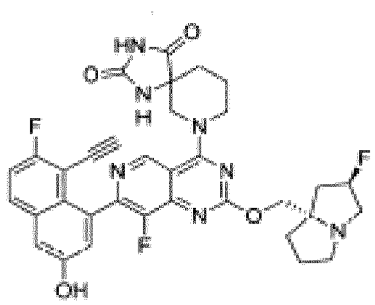
,

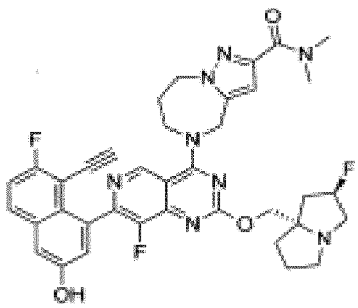
,
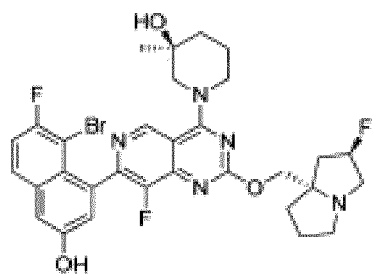
,

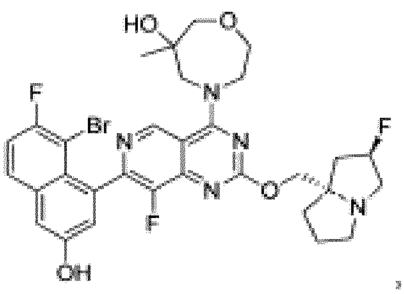
,
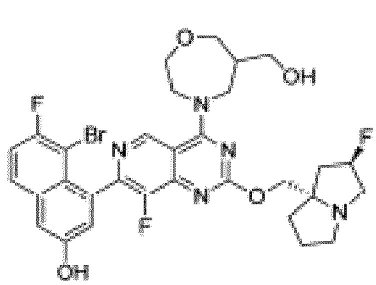
,

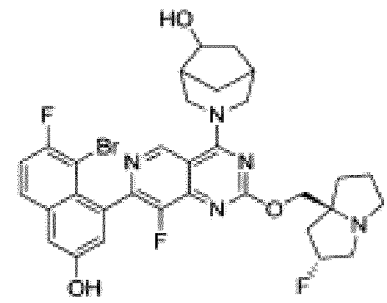,

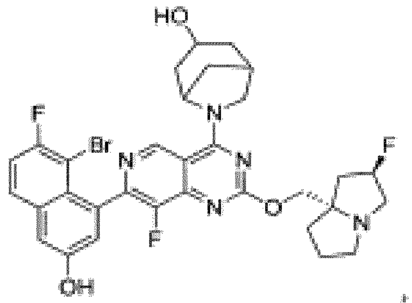,

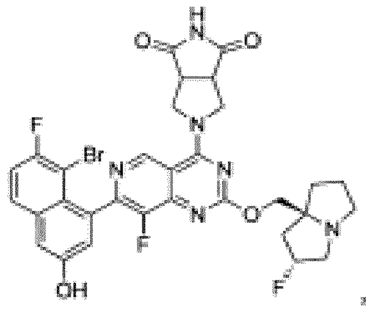,

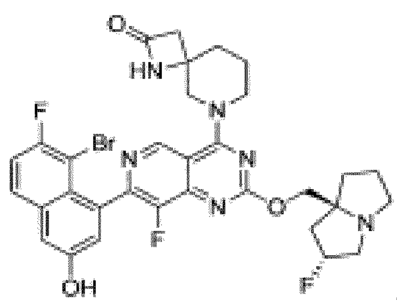,

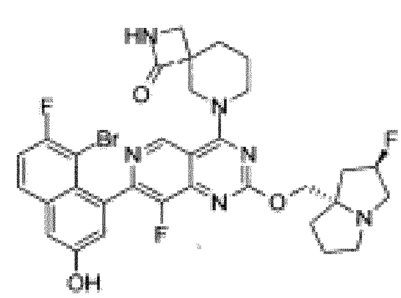,

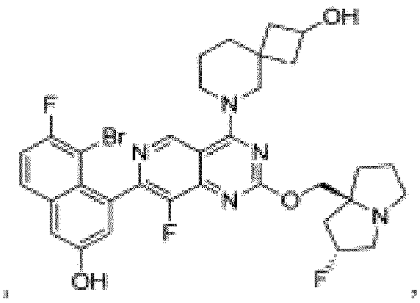,

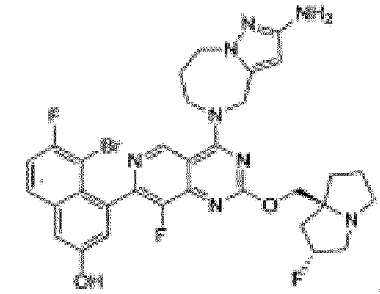,

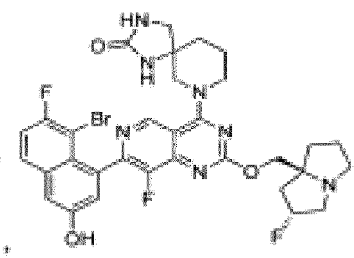,

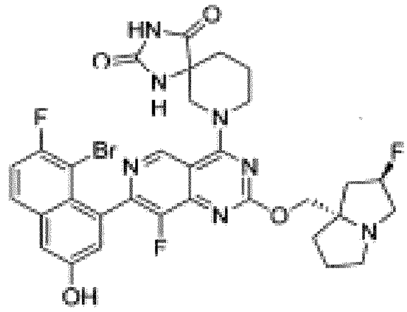,

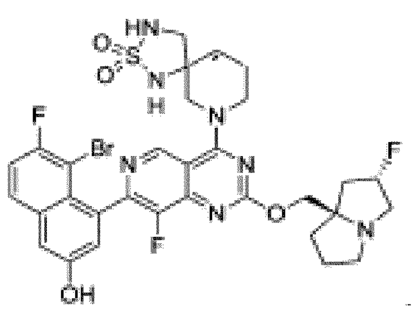,

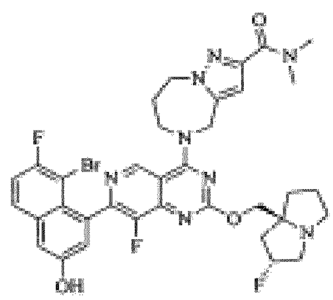, 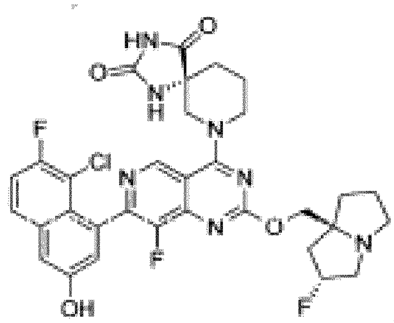,

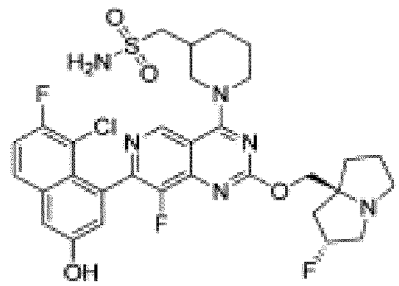, 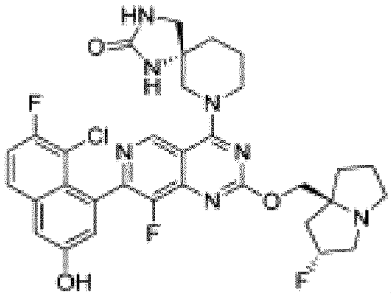,

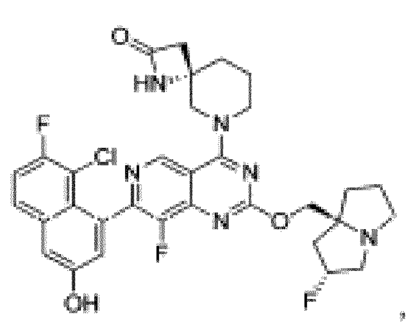, 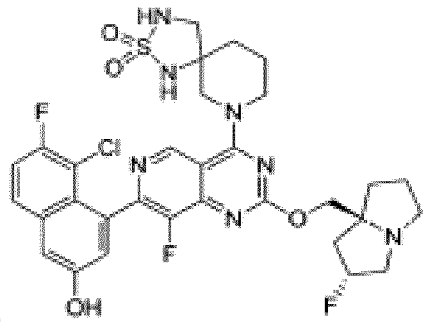,

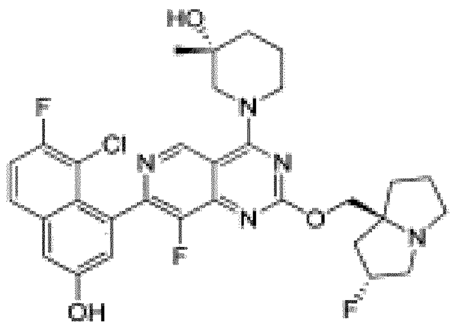, 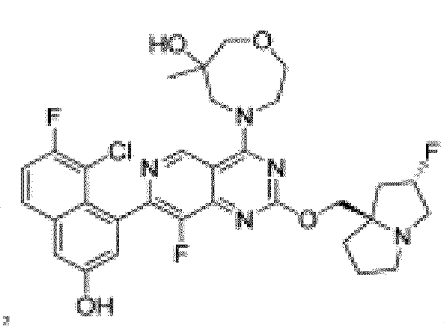,

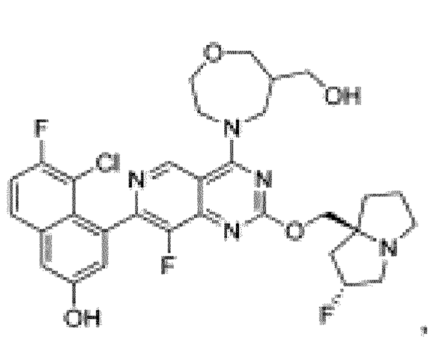, 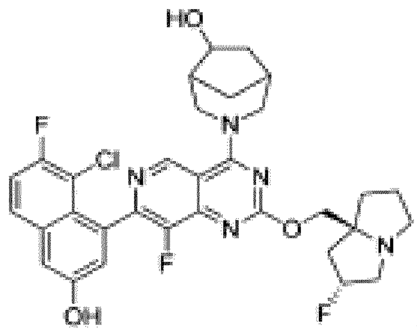,

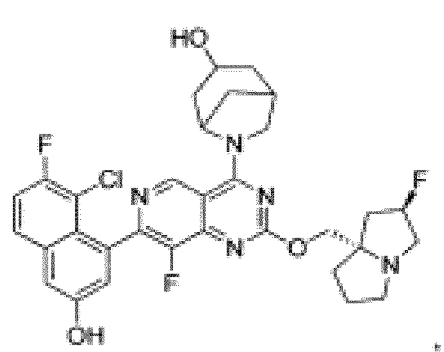,
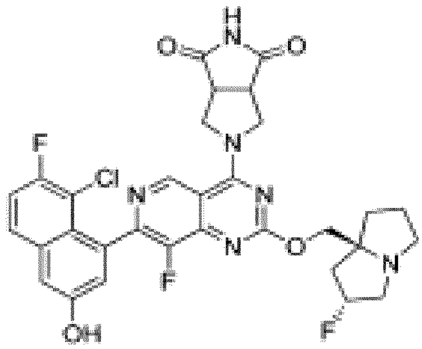,

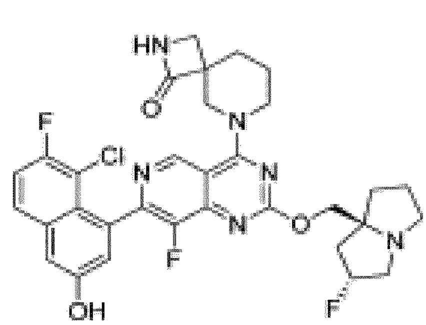,
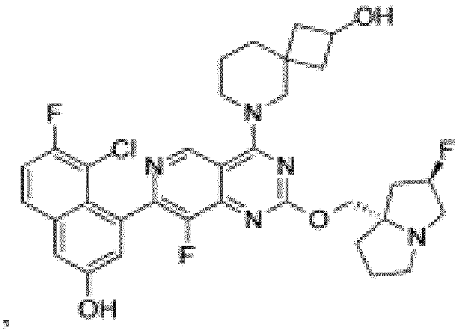,

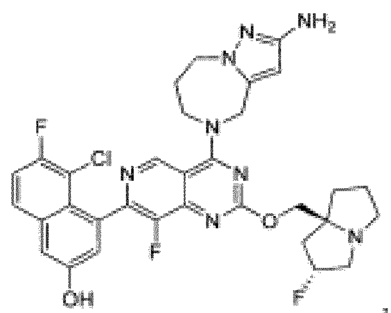,
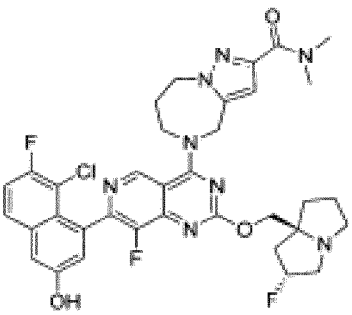,

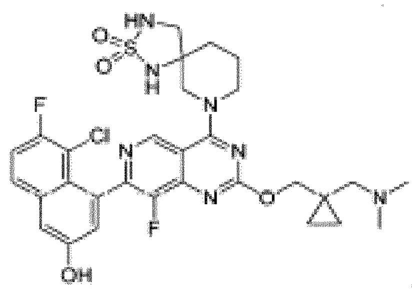,
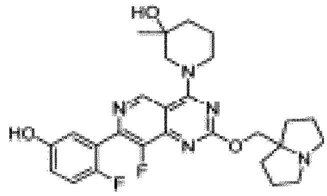,

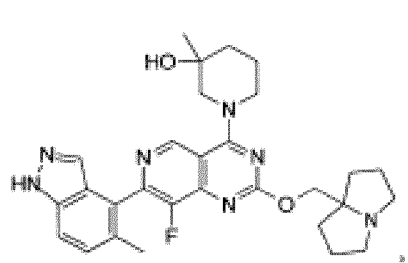,
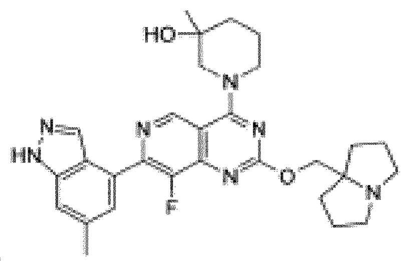,

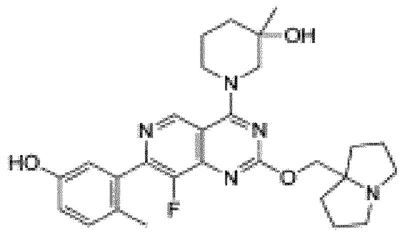,
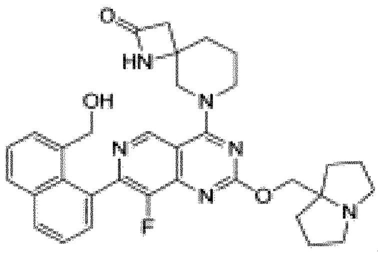,
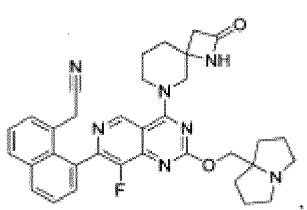,
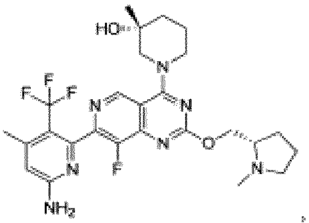,
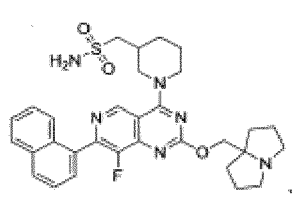,
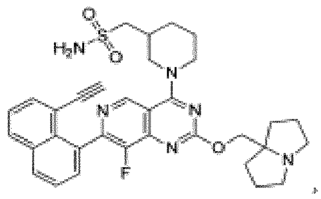,
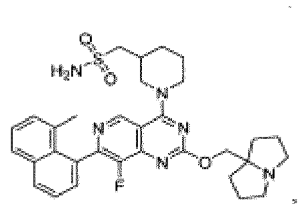,
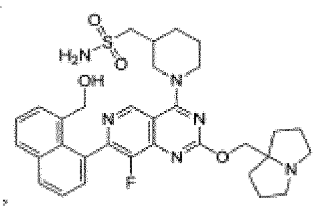,
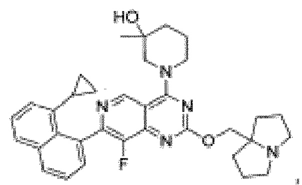,
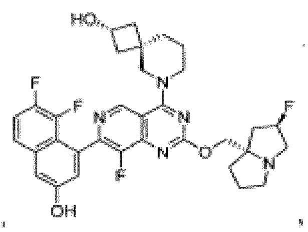,
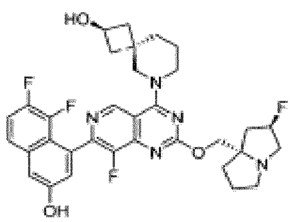,
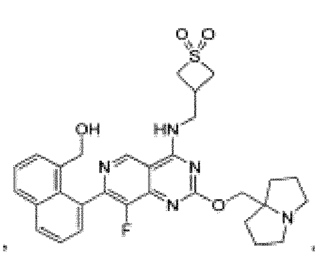,

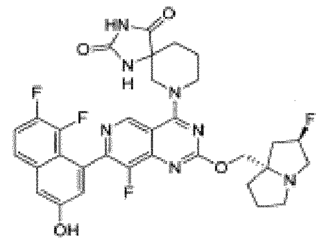,
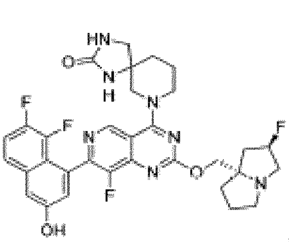,

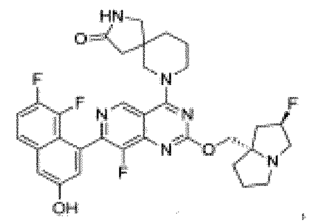,
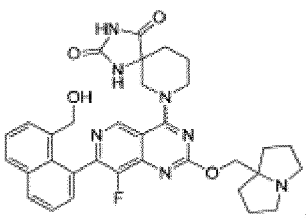,

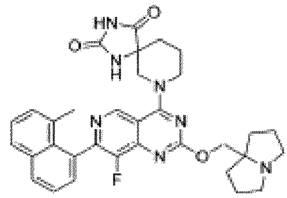,
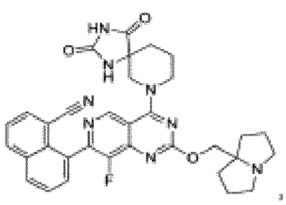,

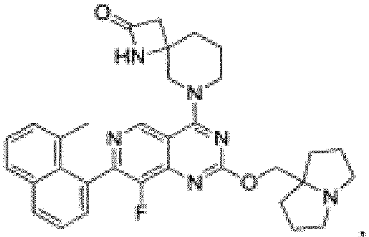,
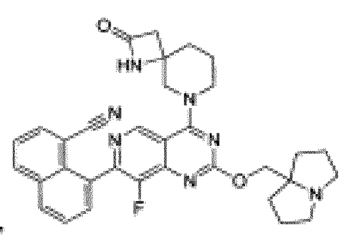,

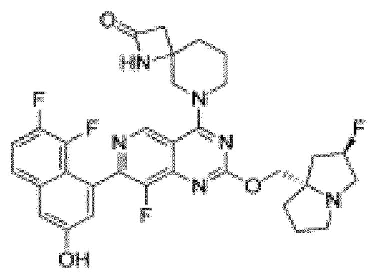,
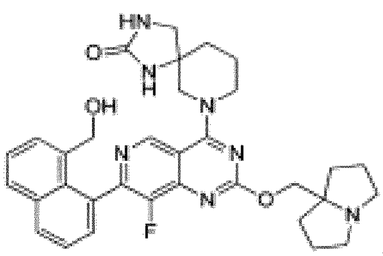,

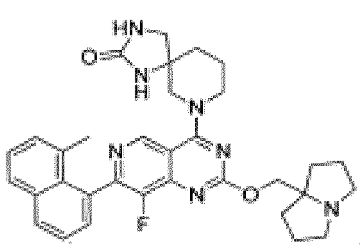,
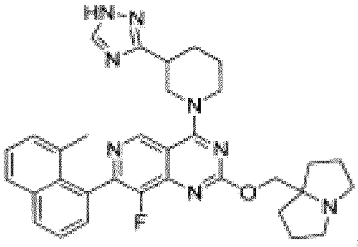,

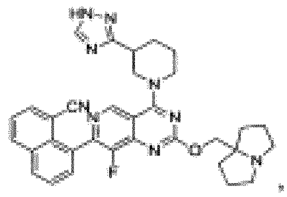, 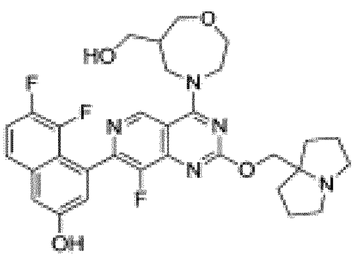,

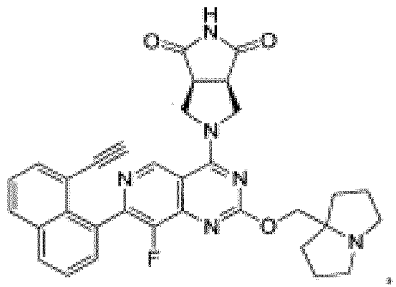, 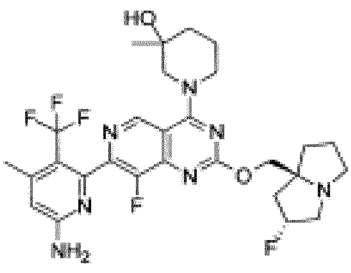,

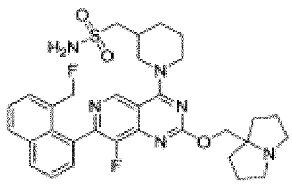, 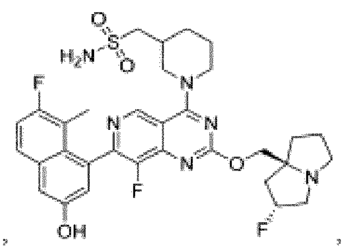,

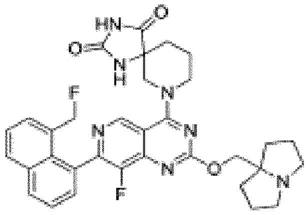, 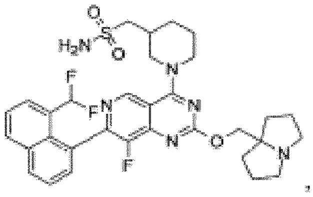,

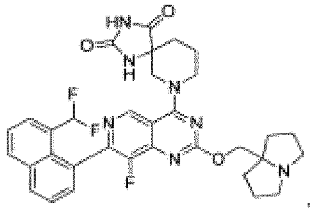, 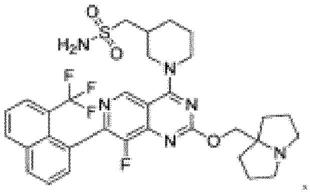,

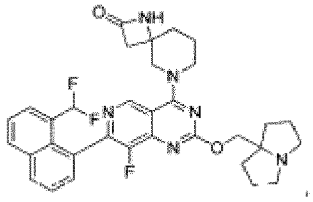, 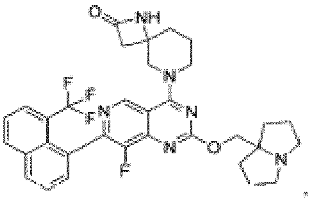,

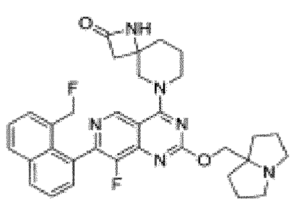,
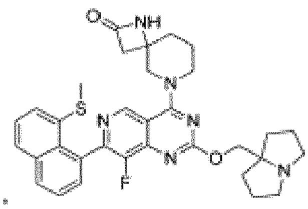,

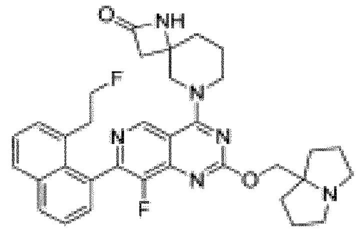,
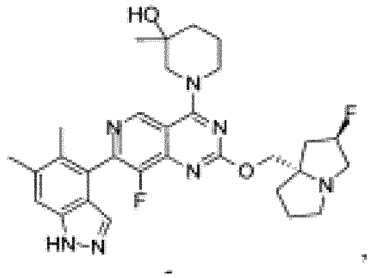,

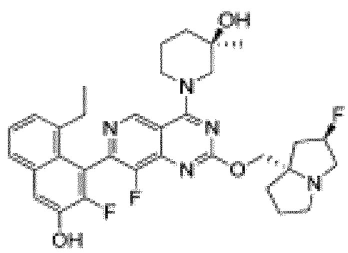,
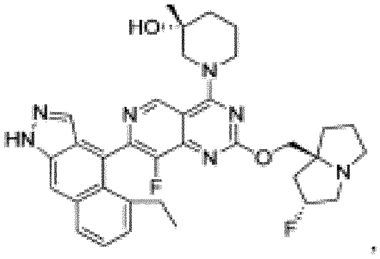,

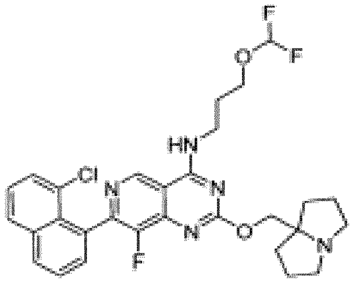,
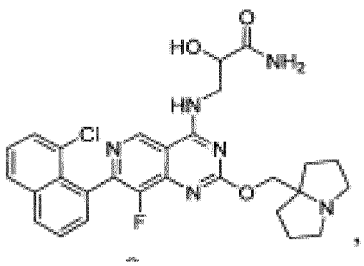,

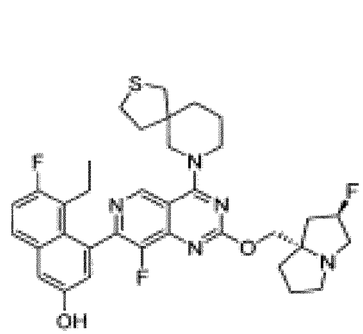,
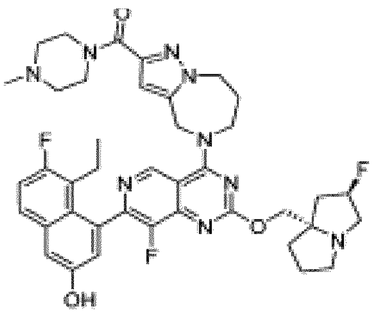,

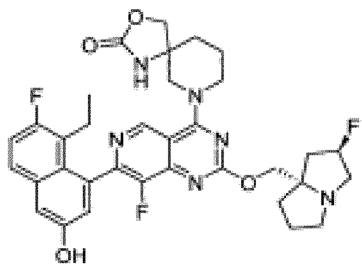,
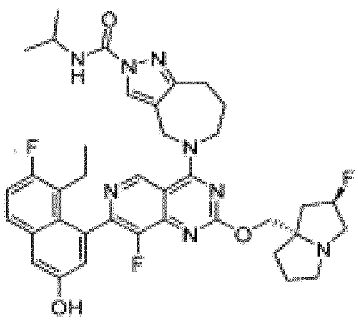,
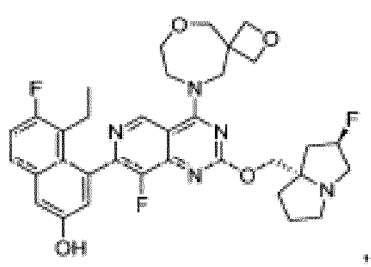,
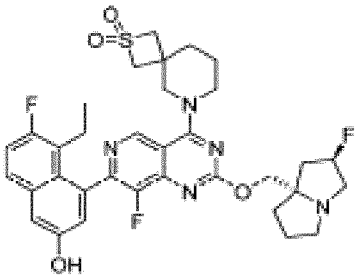,
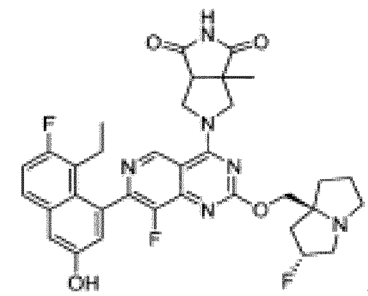,
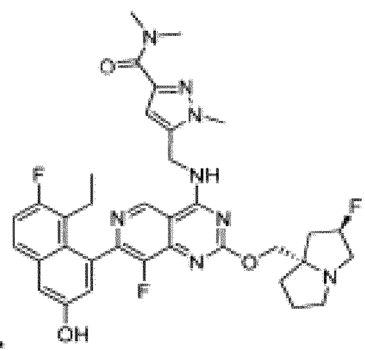,
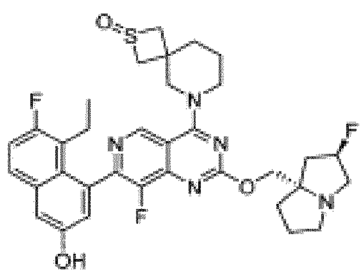,
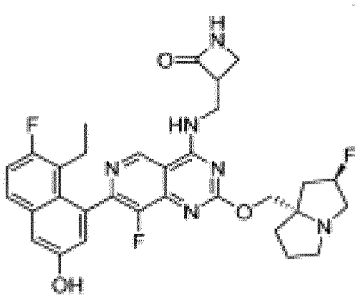,
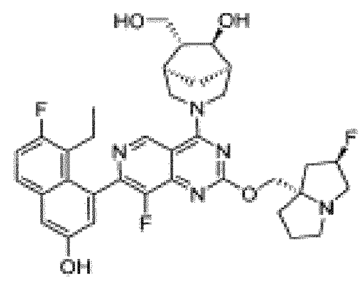,
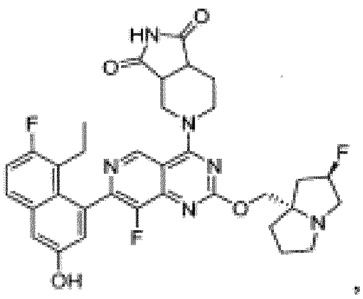,

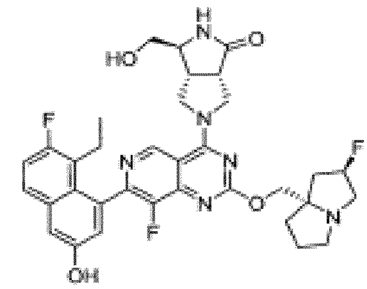,
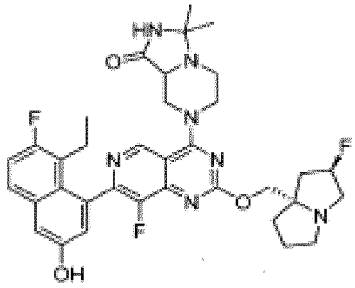,

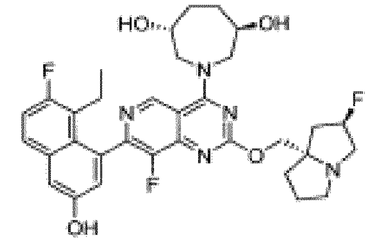,
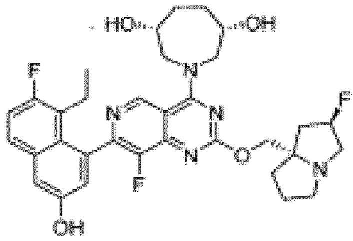,

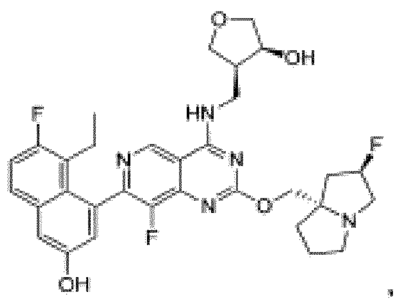,
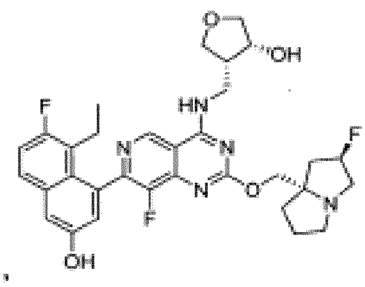,

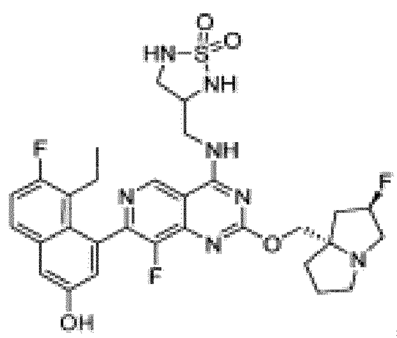,
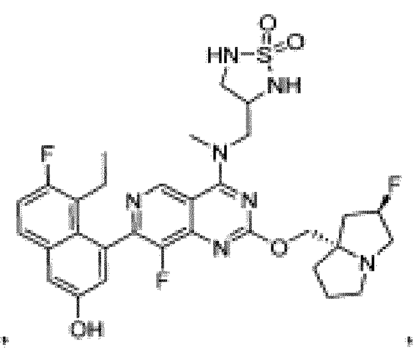,

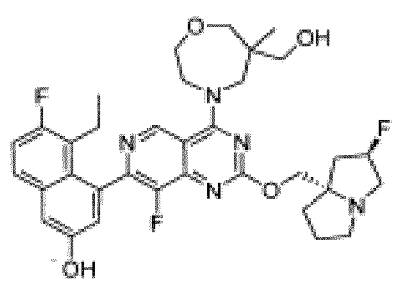,
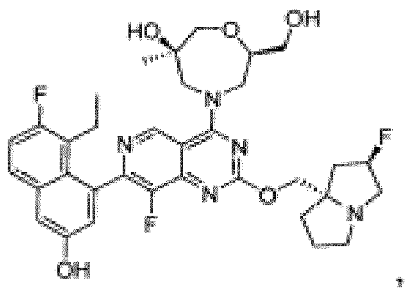,

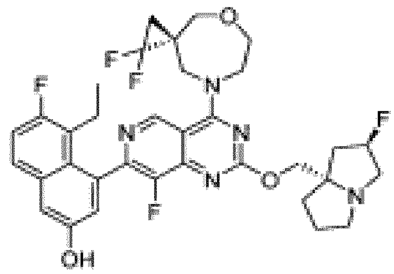
,
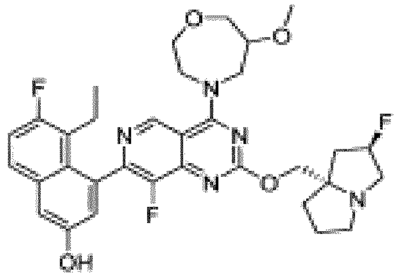
,
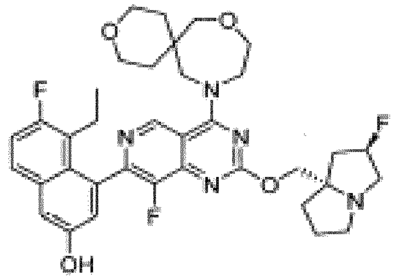
,
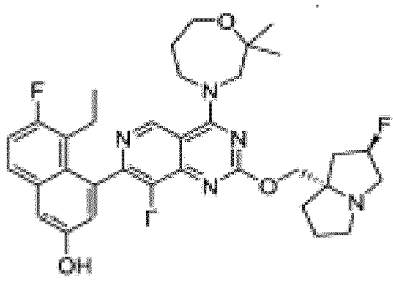
,
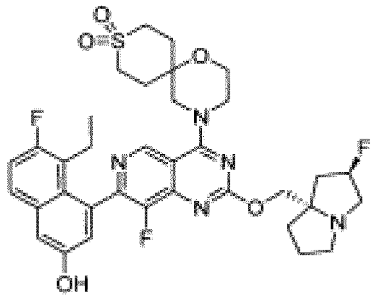
,
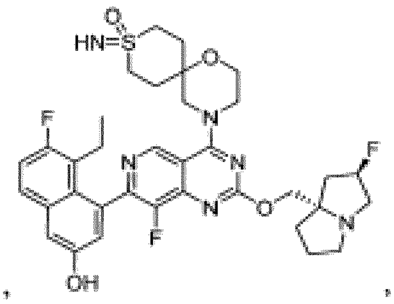
,
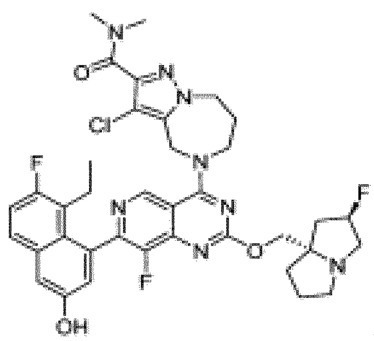
,
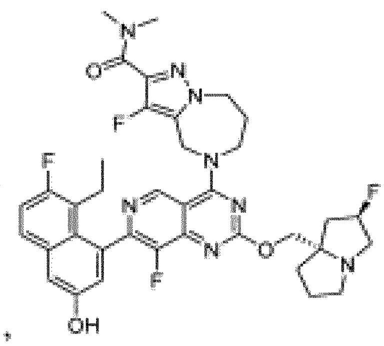
,
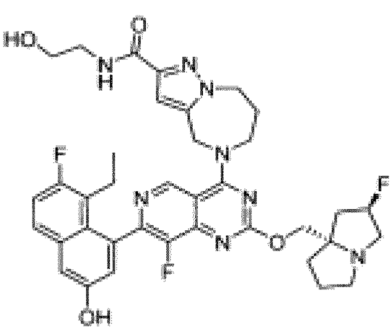
,
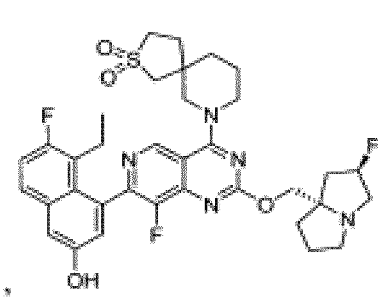

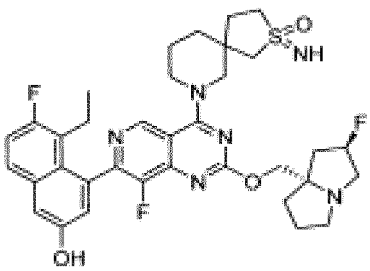,
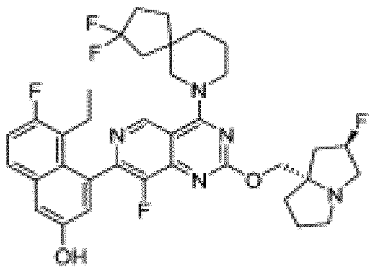,

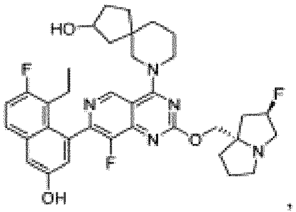,
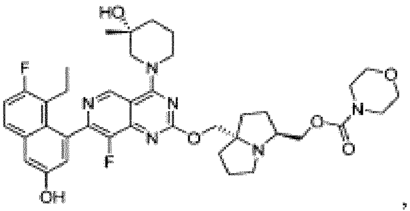,

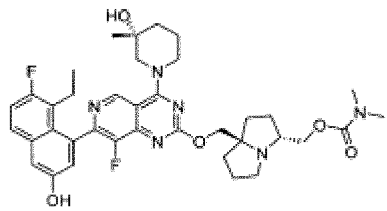,
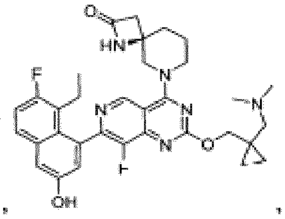,

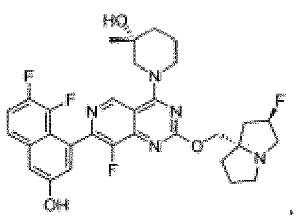,
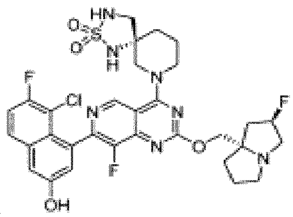,

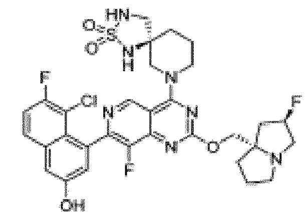,
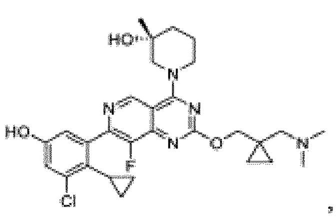,

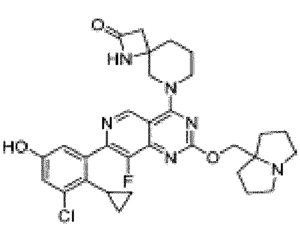,
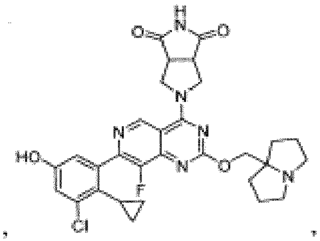,

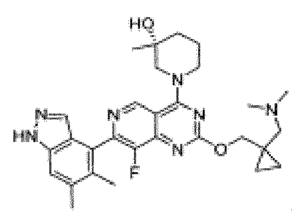, 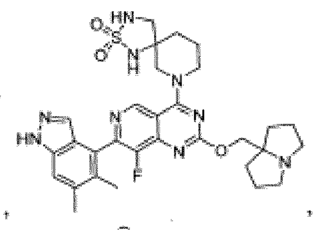,

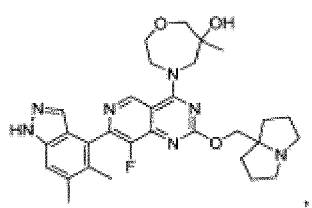, 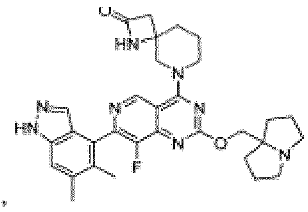,

jjj) the nitrogen-containing heterocyclic compound of formula **I** is not any one of the following compounds:

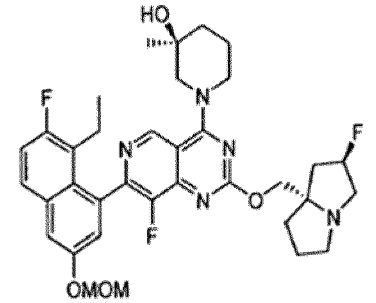, 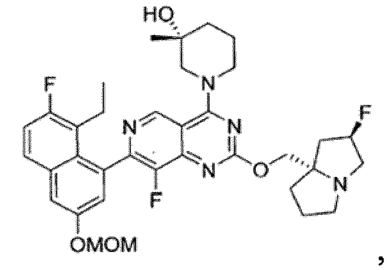, 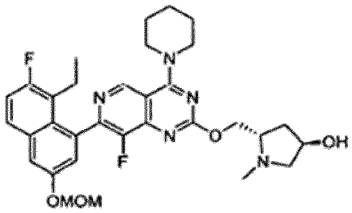,

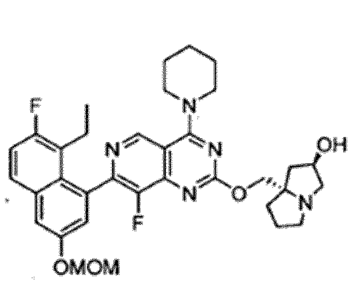, 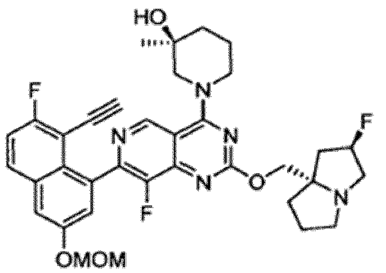, 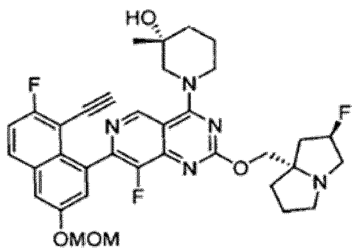,

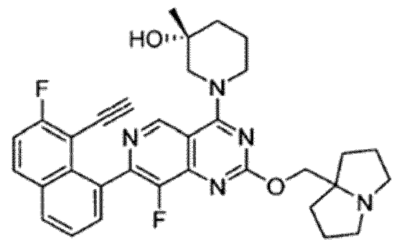 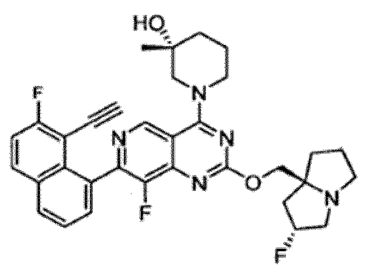 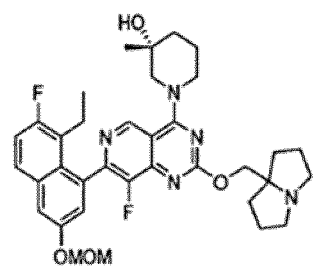

, , ,

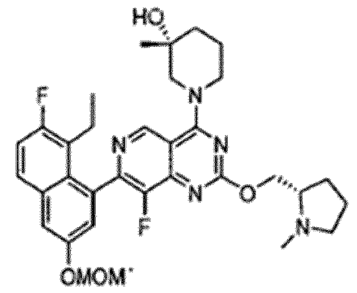
,
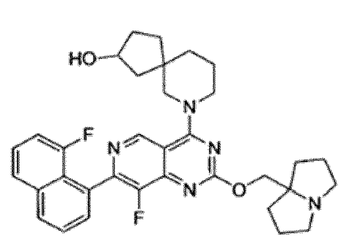
,
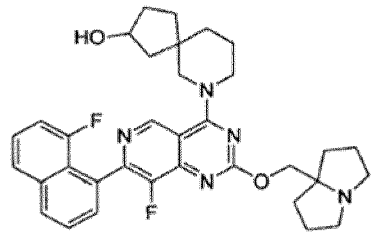
,

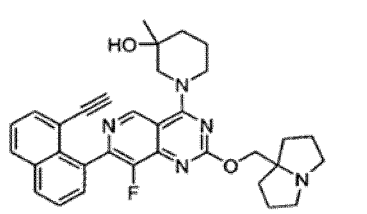
,
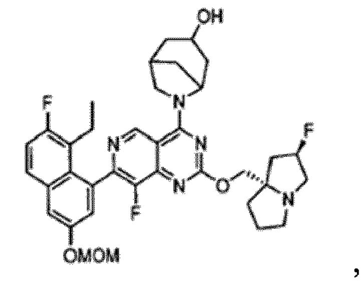
,
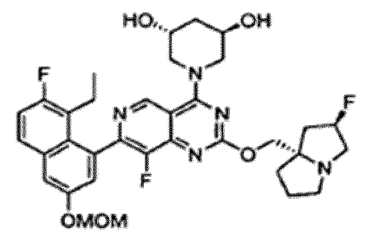
,

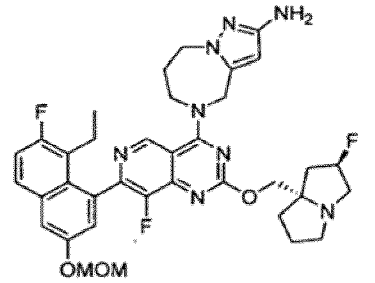
,
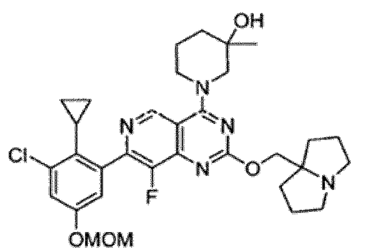
,
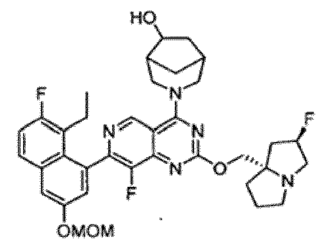
,

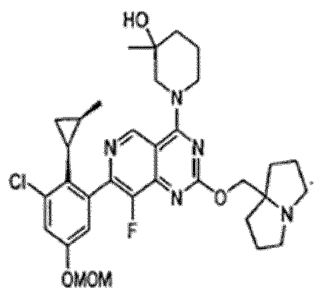
,
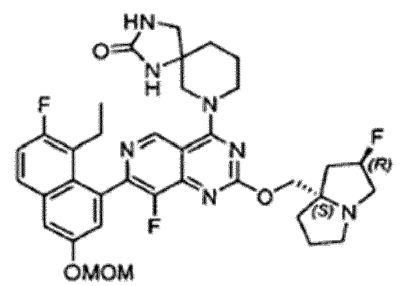
,
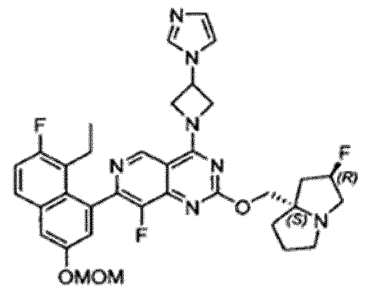
,

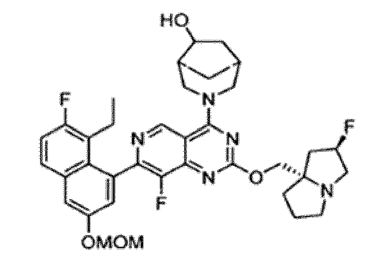
,
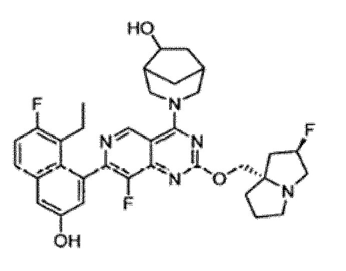
,
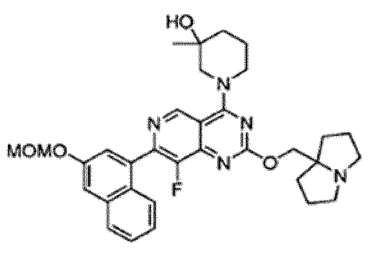
,

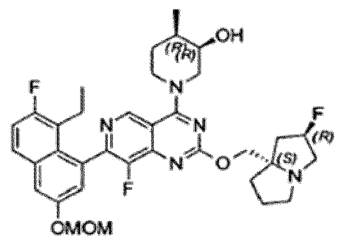,
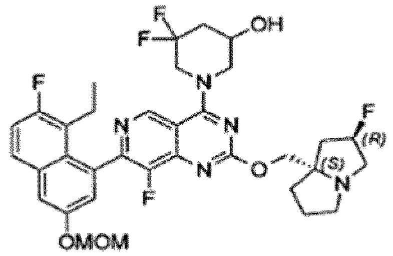,

and

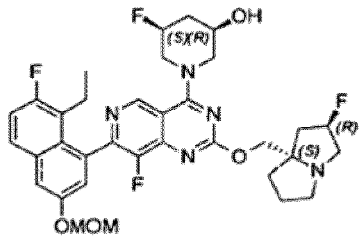;

kkk) the nitrogen-containing heterocyclic compound of formula **I** is not any one of the following compounds:

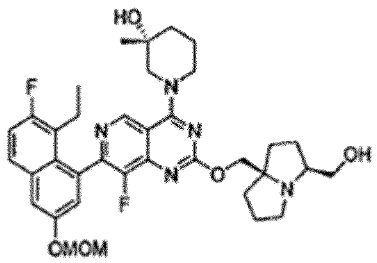,
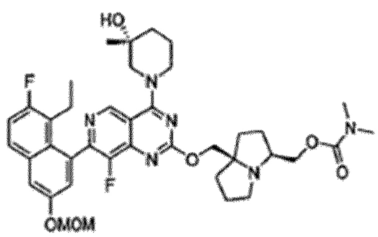,

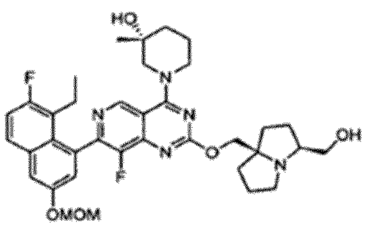,
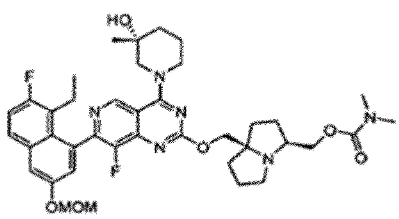,

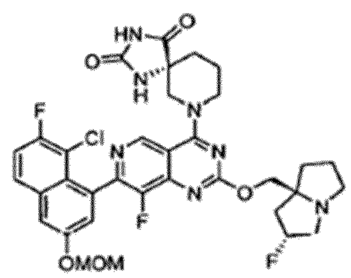,
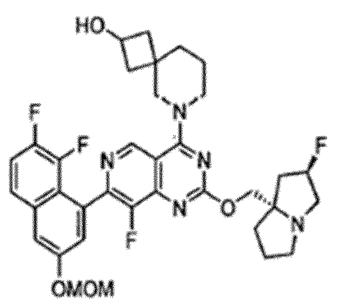, and
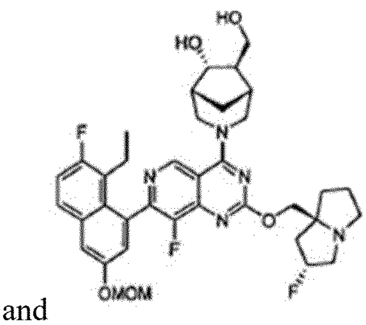.

**5.** The nitrogen-containing heterocyclic compound of formula **I**, the stereoisomer thereof, or the pharmaceutically acceptable salt thereof according to claim 1, wherein the nitrogen-containing heterocyclic compound of formula **I,** the stereoisomer thereof, or the pharmaceutically acceptable salt thereof satisfies one or more than one of the following conditions:

a)

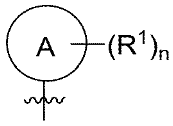

is

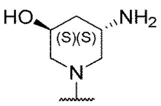, 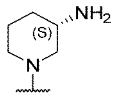, 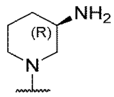, 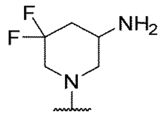, 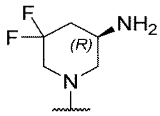,

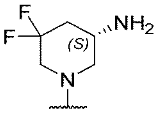, 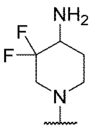, 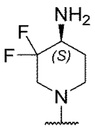, 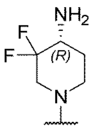, 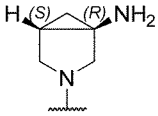, 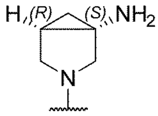, 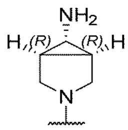,

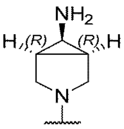, 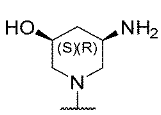, 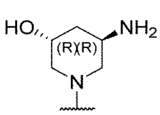, 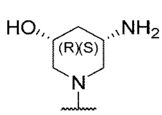, 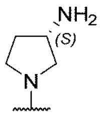, 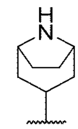, 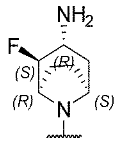,

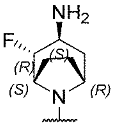, 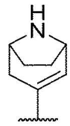, or 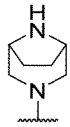;

b) $-L-R^2$ is

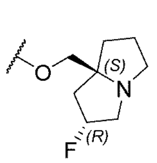 or 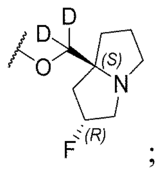;

c)

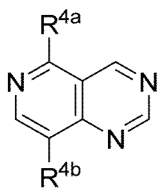

is

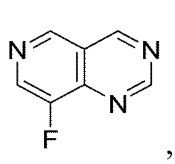, 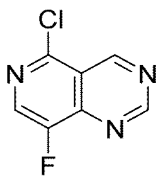, 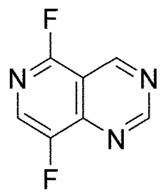, or 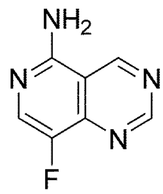;

d) $R^3$ is

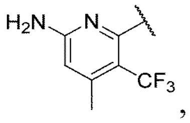, 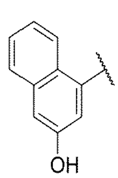, 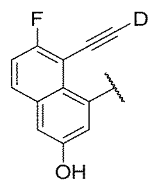, 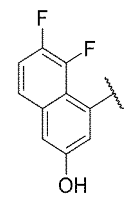, 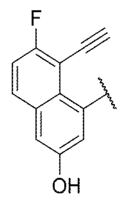, or 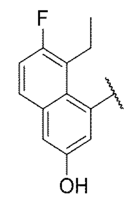.

**6.** The nitrogen-containing heterocyclic compound of formula **I,** the stereoisomer thereof, or the pharmaceutically acceptable salt thereof according to any one of claims 1 to 3, wherein the nitrogen-containing heterocyclic compound of formula **I,** the stereoisomer thereof, or the pharmaceutically acceptable salt thereof satisfies 1 or 2 of the following conditions:

(1) ring A is 3- to 7-membered cycloalkyl, 4- to 10-membered heterocycloalkyl, or 4- to 10-membered heterocycloalkenyl; the number of heteroatoms of the 4- to 10-membered heterocycloalkyl and 4- to 10-membered heterocycloalkenyl is 1, wherein the heteroatom is selected from one of N, O, and S;
(2) $R^{4a}$ is deuterium, $-N(R^5)_2$, halogen, -OH, $C_1$-$C_6$ alkyl, -CN, halo-$C_1$-$C_6$ alkyl, $C_1$-$C_6$ alkyl-O-, deuterated $C_1$-$C_6$ alkyl, $C_2$-$C_6$ alkenyl, or $C_2$-$C_6$ alkynyl.

**7.** The nitrogen-containing heterocyclic compound of formula **I,** the stereoisomer thereof, or the pharmaceutically acceptable salt thereof according to claim 6, wherein the nitrogen-containing heterocyclic compound of formula **I,** the stereoisomer thereof, or the pharmaceutically acceptable salt thereof satisfies any one of the following conditions:

a) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is selected from one of N, O, and S;
b) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is selected from one of N, O, and S; n is 1, 2, 3, 4, 5, 6, 7, 8, 9, or 10, and at least one of $R^1$ is $-N(R^5)_2$;
c) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is selected from one of N, O, and S; n is 1, 2, 3, 4, 5, 6, 7, 8, 9, or 10, and only one of $R^1$ is $-N(R^5)_2$;
d) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is N;
e) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is N; n is 1, 2, 3, 4, 5, 6, 7, 8, 9, or 10, and at least one of $R^1$ is $-N(R^5)_2$;
f) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1, wherein the heteroatom is N; n is 1, 2, 3, 4, 5, 6, 7, 8, 9, or 10, and only one of $R^1$ is $-N(R^5)_2$;
g) $R^{4a}$ is deuterium, $-N(R^5)_2$, halogen, -OH, $C_1$-$C_6$ alkyl, -CN, halo-$C_1$-$C_6$ alkyl, $C_1$-$C_6$ alkyl-O-, deuterated $C_1$-$C_6$ alkyl, $C_2$-$C_6$ alkenyl, or $C_2$-$C_6$ alkynyl;
h) $R^{4a}$ is $-N(R^5)_2$ or halogen;
i) $R^{4a}$ is halogen;
j) $R^{4a}$ is fluorine or chlorine.

**8.** The nitrogen-containing heterocyclic compound of formula **I,** the stereoisomer thereof, or the pharmaceutically

acceptable salt thereof according to any one of claims 1 to 3, wherein the nitrogen-containing heterocyclic compound of formula **I,** the stereoisomer thereof, or the pharmaceutically acceptable salt thereof satisfies one or more than one of the following conditions:

(1) each $R^1$ is independently halogen, -OH, or $-NH_2$; for example, each $R^1$ is independently halogen, -OH, or $-NH_2$, and at least one $R^1$ is $-NH_2$;
(2) ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1 or 2, and the heteroatom is N; for example, the number of heteroatoms of the 4- to 10-membered heterocycloalkyl and 4- to 10-membered heterocycloalkenyl is 1, the heteroatom is N, and the N is connected to the pyrimidine ring shown in formula I; or, the number of heteroatoms of the 4- to 10-membered heterocycloalkyl and 4- to 10-membered heterocycloalkenyl is 1, the heteroatom is N, and the atom in ring A connected to the pyrimidine ring shown in formula I is a carbon atom; or, the number of heteroatoms of the 4- to 10-membered heterocycloalkyl and 4- to 10-membered heterocycloalkenyl is 2, the heteroatoms are N, and one of the N is connected to the pyrimidine ring shown in formula I;
(3) L is $-O-(CR^{6a}R^{6b})n2-$;
(4) $R^2$ is 4- to 10-membered heterocycloalkyl substituted by one or more than one $R^{2b}$;
(5) $R^3$ is $C_6$-$C_{10}$ aryl substituted by one or more than one $R^{3a}$ or 5- to 14-membered heteroaryl substituted by one or more than one $R^{3b}$;
(6) $R^{3a}$ and $R^{3b}$ are each independently halogen, -OH, Ci-Ce alkyl, $C_1$-$C_6$ alkyl substituted by one or more than one $R^{3-a}$, $C_2$-$C_6$ alkynyl, or $-N(R^5)_2$; for example, $R^{3a}$ and $R^{3b}$ are each independently halogen, -OH, $C_1$-$C_4$ alkyl, $C_1$-$C_4$ alkyl substituted by one or more than one $R^{3-a}$, $C_2$-$C_4$ alkynyl, or $-N(R^5)_2$;
(7) $R^{4a}$ and $R^{4b}$ are independently H, $-N(R^5)_2$, or halogen;
(8) $R^5$ is independently H;
(9) $R^{6a}$ and $R^{6b}$ are each independently H;
(10) $R^{2b}$ is halogen or $C_1$-$C_4$ alkyl;
(11) $R^{3-a}$ is halogen.

**9.** The nitrogen-containing heterocyclic compound of formula **I,** the stereoisomer thereof, or the pharmaceutically acceptable salt thereof according to any one of claims 1 to 3, wherein the nitrogen-containing heterocyclic compound of formula **I,** the stereoisomer thereof, or the pharmaceutically acceptable salt thereof satisfies one or more than one of the following conditions:

k) ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;
l) n is 0;
m) L is $-O-(CR^{6a}R^{6b})n2-$; n2 is 1; $R^{6a}$ and $R^{6b}$ are each independently H or deuterium;
n) $R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one or more than one of N, O, and S, and the number of heteroatoms is 1, 2, or 3; each $R^{2b}$ is independently halogen or $C_1$-$C_4$ alkyl;
o) $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, Ci-Cs alkyl, $C_1$-$C_6$ alkyl substituted by one or more than one $R^{3-a}$, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different; each $R^{3-a}$ is independently halogen; $R^{3-e}$ is deuterium; each $R^5$ is H or $C_1$-$C_6$ alkyl;
p) $R^{4a}$ and $R^{4b}$ are each independently halogen.

**10.** The nitrogen-containing heterocyclic compound of formula I, the stereoisomer thereof, or the pharmaceutically acceptable salt thereof according to claim 9, wherein the nitrogen-containing heterocyclic compound of formula **I,** the stereoisomer thereof, or the pharmaceutically acceptable salt thereof satisfies one or more than one of the following conditions:

q) $R^2$ is 4- to 10-membered heterocycloalkyl substituted by one or more than one $R^{2b}$; in 4- to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3; each $R^{2b}$ is independently halogen;

r) $R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different; $R^{3a}$ and $R^{3b}$ are each independently deuterium, halogen, -OH, -CN, $C_2$-$C_6$ alkynyl, $C_2$-$C_6$ alkynyl substituted by one or more than one $R^{3\text{-}e}$, -N($R^5$)$_2$, or triazolyl; $R^{3\text{-}e}$ is deuterium; when there is more than one substituent, the substituents are the same or different; each $R^5$ is H.

**11.** The nitrogen-containing heterocyclic compound of formula **I,** the stereoisomer thereof, or the pharmaceutically acceptable salt thereof according to claim 1, wherein the nitrogen-containing heterocyclic compound of formula **I,** the stereoisomer thereof, or the pharmaceutically acceptable salt thereof is any one of the following schemes:

scheme 1: in the nitrogen-containing heterocyclic compound of formula I, the stereoisomer thereof, or the pharmaceutically acceptable salt thereof,

ring A is 4- to 10-membered heterocycloalkyl or 4- to 10-membered heterocycloalkenyl; the number of heteroatoms of the 4- to 10-membered heterocycloalkyl and 4- to 10-membered heterocycloalkenyl is 1 or 2, and the heteroatom is N;
n is 0, 1, 2, or 3;
$R^1$ is independently halogen, -OH, or -N($R^5$)$_2$;
L is -O-($CR^{6a}R^{6b}$)n2-;
$R^2$ is 4- to 10-membered heterocycloalkyl or 4- to 10-membered heterocycloalkyl substituted by one or more than one $R^{2b}$;
$R^{2b}$ is independently halogen;
$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$;
$R^{3a}$ and $R^{3b}$ are independently halogen, -OH, $C_1$-$C_6$ alkyl, $C_1$-$C_6$ alkyl substituted by one or more than one $R^{3a}$, $C_2$-$C_6$ alkynyl, or -N($R^5$)$_2$;
$R^{3\text{-}a}$ is independently halogen;
$R^{4a}$ and $R^{4b}$ are independently H, -N($R^5$)$_2$, or halogen;
$R^5$ is independently hydrogen;
$R^{6a}$ and $R^{6b}$ are H;
n2 is 1;
and, when ring A comprises two heteroatoms, $R^{4a}$ is not H;

scheme 2: the nitrogen-containing heterocyclic compound of formula I is shown in formula 1-1,

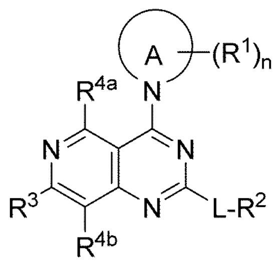

**I-1**

,

wherein ring A is 4- to 10-membered heterocycloalkyl; the number of heteroatoms of the 4- to 10-membered heterocycloalkyl is 1;
n is 1, 2, or 3;
$R^1$ is independently halogen, -OH, or -N($R^5$)$_2$;

L is $-O-(CR^{6a}R^{6b})_n2-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is

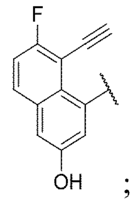

;

$R^{4a}$ and $R^{4b}$ are independently H, $-N(R^5)_2$, or halogen;
$R^5$ is independently hydrogen;
$R^{6a}$ and $R^{6b}$ are H;
n2 is 1;

scheme 3: the nitrogen-containing heterocyclic compound of formula I is shown in formula 1-1;

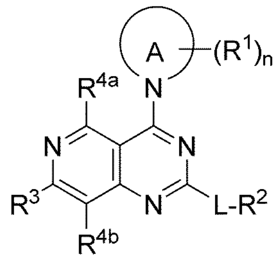

**I-1**

wherein ring A is 4- to 10-membered heterocycloalkyl; the number of heteroatoms of the 4- to 10-membered heterocycloalkyl is 2, and the heteroatoms are N; and one of the N is connected to the pyrimidine ring shown in formula I;
n is 0;
L is $-O-(CR^{6a}R^{6b})n2-$;
$R^2$ is 4- to 10-membered heterocycloalkyl or 4- to 10-membered heterocycloalkyl substituted by one or more than one $R^{2b}$;
$R^{2b}$ is independently halogen;
$R^3$ is

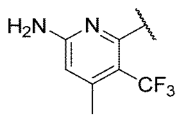

or

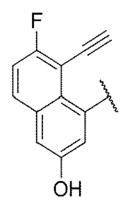

;

$R^{4a}$ and $R^{4b}$ are independently $-N(R^5)_2$ or halogen;
$R^5$ is independently hydrogen;
$R^{6a}$ and $R^{6b}$ are H;
n2 is 1;

scheme 4:
the nitrogen-containing heterocyclic compound of formula I is shown in formula I-2',

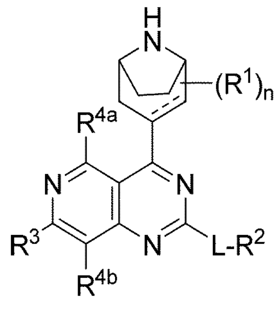

I-2'

,

wherein - - - represents a single bond or a double bond;

scheme 5:

the nitrogen-containing heterocyclic compound of formula I is shown in formula 1-3,

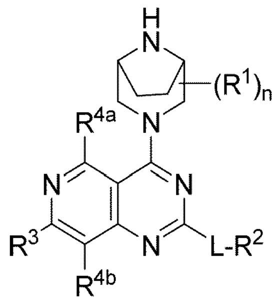

I-3

,

wherein $R^{4a}$ is halogen or $-N(R^5)_2$, and $R^5$ is independently hydrogen;

scheme 6:

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})n2-$;

$R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen or $C_1$-$C_4$ alkyl;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, $C_1$-$C_6$ alkyl, $C_1$-$C_6$ alkyl substituted by one or more than one $R^{3-a}$, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different;

each $R^{3-a}$ is independently halogen; $R^{3-e}$ is deuterium;

$R^{4a}$ and $R^{4b}$ are each independently halogen;

n2 is 1;

each $R^5$ is H or $C_1$-$C_6$ alkyl;

$R^{6a}$ and $R^{6b}$ are each independently H or deuterium;

the nitrogen-containing heterocyclic compound of formula I is not any one of the following compounds:

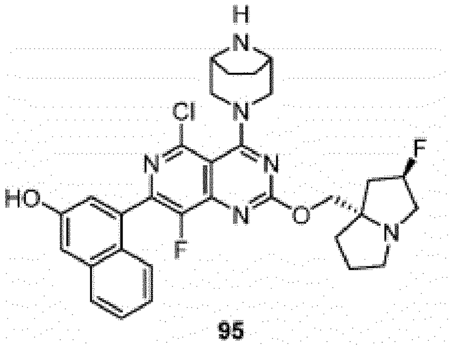

95

,

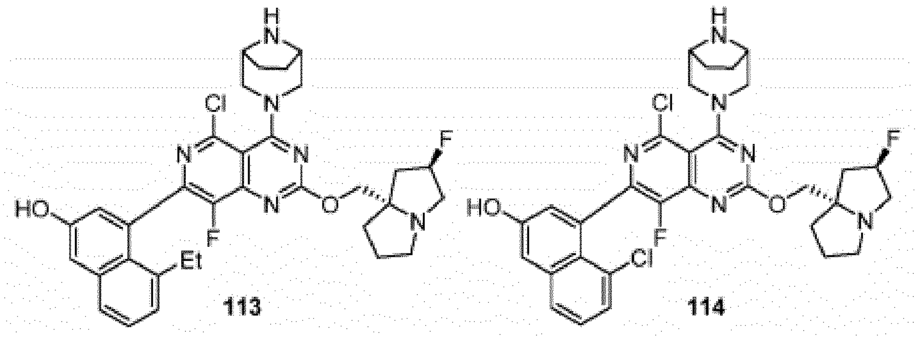

113 114

,

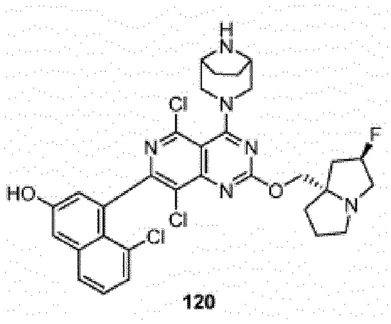

120

,

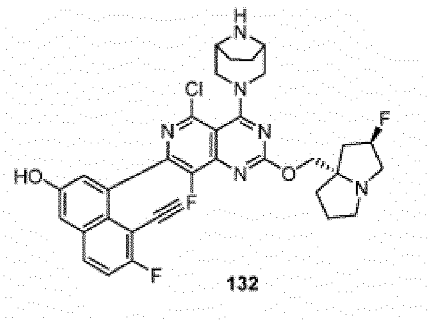

132

,

and

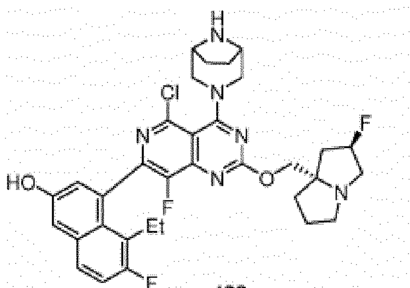

138

;

scheme 7:

wherein ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;
n is 0;

L is -O-$(CR^{6a}R^{6b})n2$-;

$R^2$ is 4- to 10-membered heterocycloalkyl or 4- to 10-membered heterocycloalkyl substituted by one or more than one $R^{2b}$; in 4- to 10-membered heterocycloalkyl of the 4- to 10-membered heterocycloalkyl and 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen or $C_1$-$C_4$ alkyl;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, $C_1$-$C_6$ alkyl, $C_1$-$C_6$ alkyl substituted by one or more than one $R^{3-a}$, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different;

each $R^{3-a}$ is independently halogen; $R^{3-e}$ is deuterium;

$R^{4a}$ and $R^{4b}$ are each independently halogen;

n2 is 1;

each $R^5$ is H or Ci-Ce alkyl;

$R^{6a}$ and $R^{6b}$ are each independently H or deuterium;

the nitrogen-containing heterocyclic compound of formula I is not any one of the following compounds:

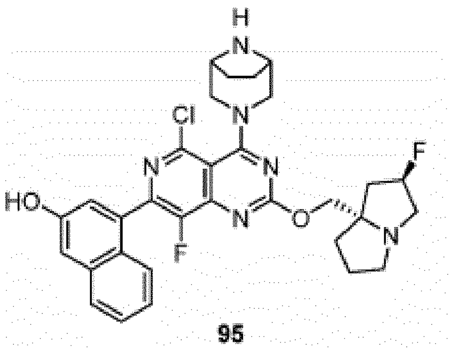

95

,

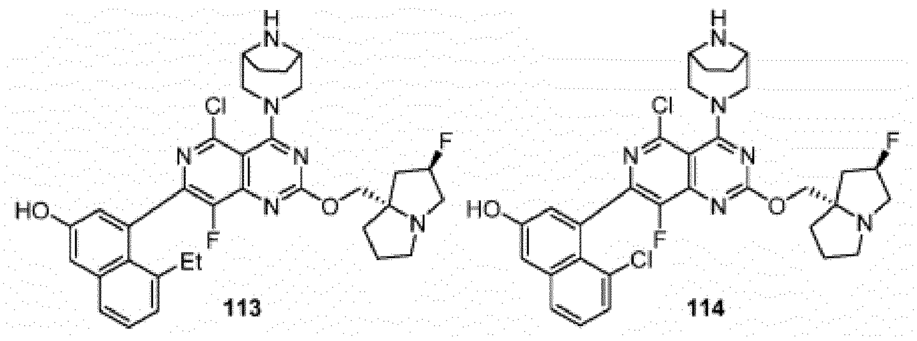

113 114

,

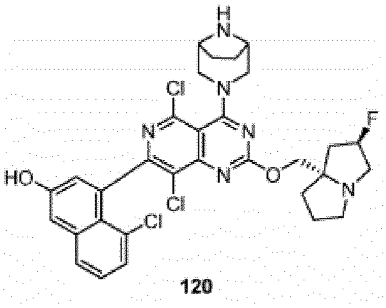

120

,

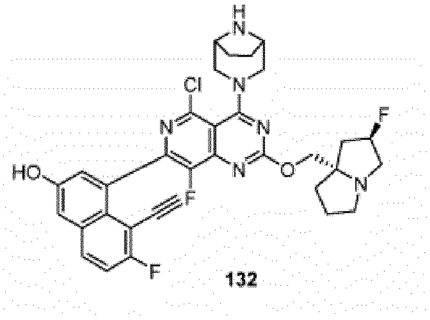

132

,

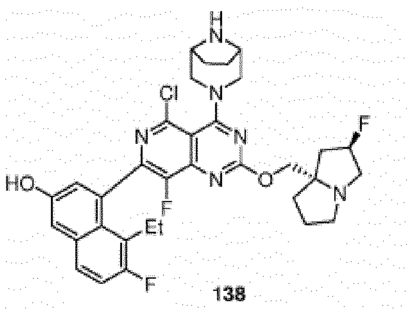

138

,

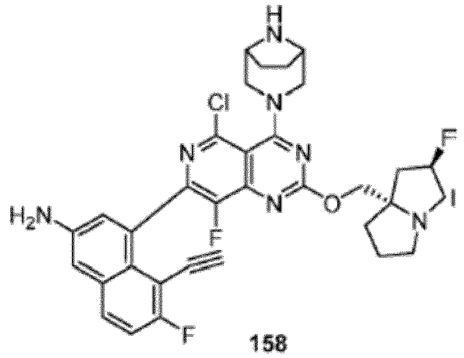

158

,

and

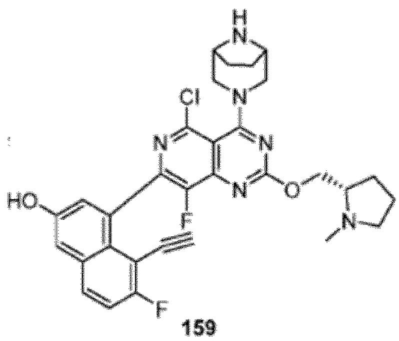

159

;

scheme 8:

ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is $-O-(CR^{6a}R^{6b})n2-$;

$R^2$ is 4- to 10-membered heterocycloalkyl substituted by one or more than one $R^{2b}$; in 4-to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, -CN, $C_2$-$C_6$ alkynyl, $C_2$-$C_6$ alkynyl substituted by one or more than one $R^{3-e}$, $-N(R^5)_2$, or triazolyl; when there is more than one substituent, the substituents are the same or different; $R^{3-e}$ is deuterium;

$R^{4a}$ and $R^{4b}$ are each independently halogen;

n2 is 1;

each $R^5$ is H;

$R^{6a}$ and $R^{6b}$ are each independently H or deuterium;

the nitrogen-containing heterocyclic compound of formula I is not any one of the following compounds:

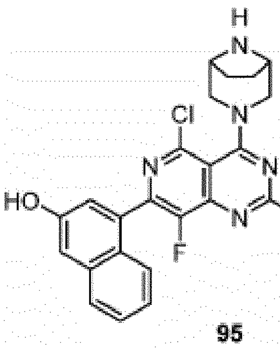

95

,

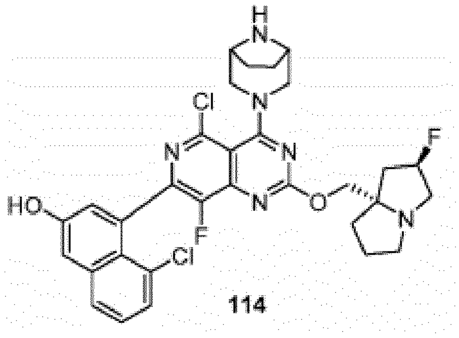

114

,

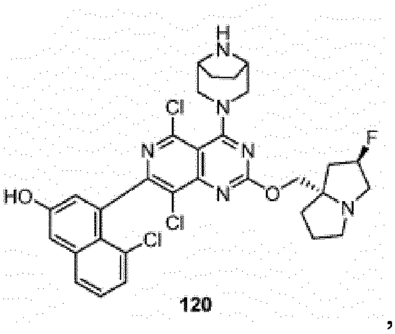

120

, and

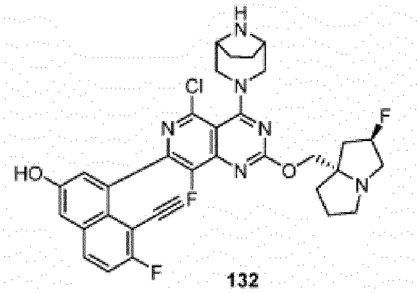

132

;

scheme 9:

ring A is 4- to 10-membered heterocycloalkyl; the 4- to 10-membered heterocycloalkyl is 6- to 8-membered bridged heterocycloalkyl; the number of heteroatoms of the 4- to 10-membered heterocycloalkyl is 2, wherein the heteroatoms are selected from one or two of N, O, and S;

n is 0;

L is -O-($CR^{6a}R^{6b}$)n2-;

$R^2$ is 4- to 10-membered heterocycloalkyl substituted by one or more than one $R^{2b}$; in 4-to 10-membered heterocycloalkyl of the 4- to 10-membered heterocycloalkyl substituted by one or more than one $R^{2b}$, the heteroatom is selected from one or more than one of N, O, and S, and the number of heteroatoms is 1, 2, or 3;

each $R^{2b}$ is independently halogen;

$R^3$ is $C_6$-$C_{10}$ aryl, $C_6$-$C_{10}$ aryl substituted by one or more than one $R^{3a}$, 5- to 14-membered heteroaryl, or 5- to 14-membered heteroaryl substituted by one or more than one $R^{3b}$; 5- to 14-membered heteroaryl in the 5- to 14-membered heteroaryl and 5- to 14-membered heteroaryl substituted by one or more than one $R^{3b}$ comprises 1, 2, or 3 heteroatoms independently selected from nitrogen, oxygen, and sulfur; when there is more than one substituent, the substituents are the same or different;

$R^{3a}$ and $R^{3b}$ are each independently deuterium, halogen, -OH, -CN, $C_2$-$C_6$ alkynyl, $C_2$-$C_6$ alkynyl substituted by one or more than one $R^{3-e}$, -N($R^5$)$_2$, or triazolyl; when there is more than one substituent, the substituents are the same or different; $R^{3-e}$ is deuterium;

$R^{4a}$ and $R^{4b}$ are each independently halogen;

n2 is 1;

each $R^5$ is H;

$R^{6a}$ and $R^{6b}$ are each independently H or deuterium;

the nitrogen-containing heterocyclic compound of formula I is not any one of the following compounds:

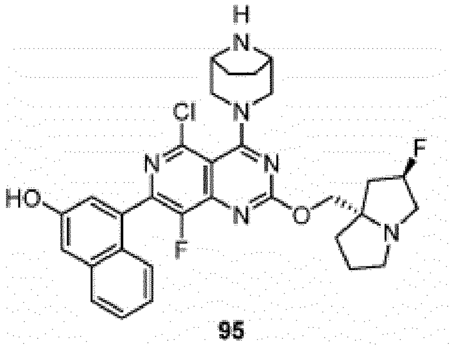

95

,

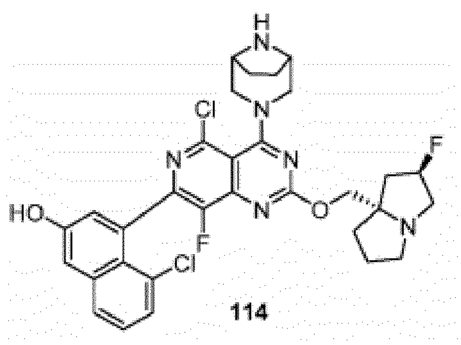

114

,

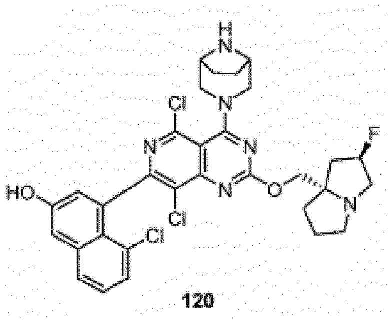

120

,

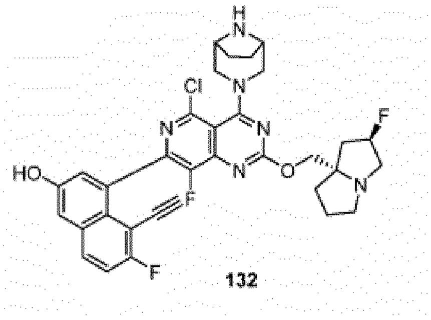

132

,

and

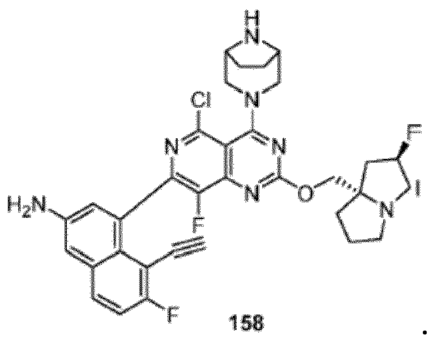

158

.

12. The nitrogen-containing heterocyclic compound of formula I, the stereoisomer thereof, or the pharmaceutically acceptable salt thereof according to any one of claims 1 to 5, wherein the nitrogen-containing heterocyclic compound of formula I is selected from the following compounds:

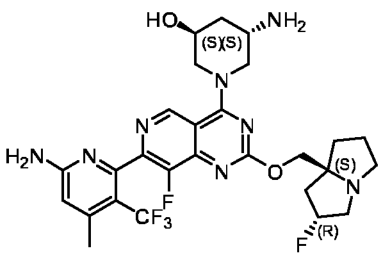

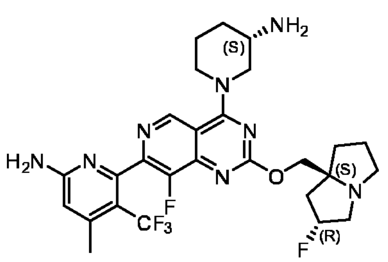

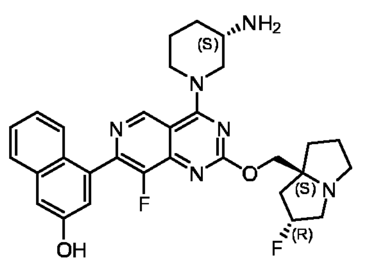

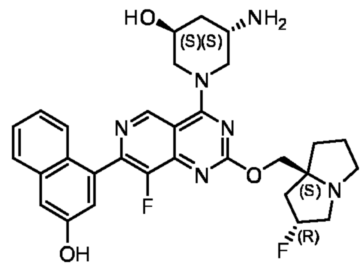
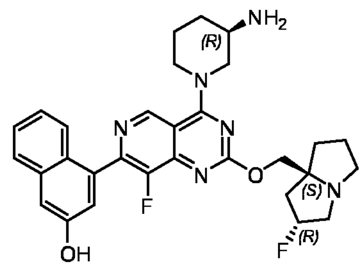
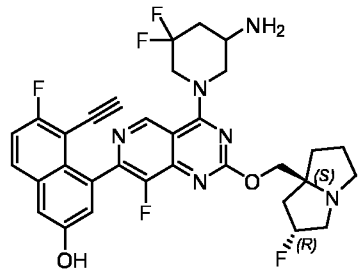
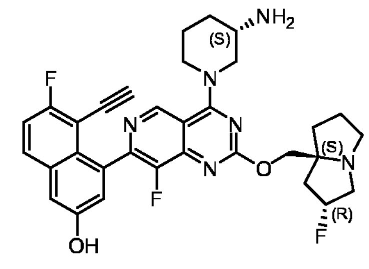
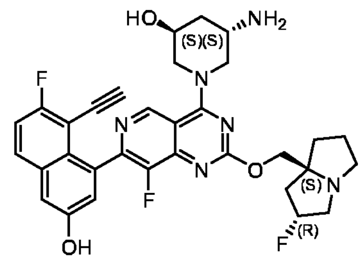
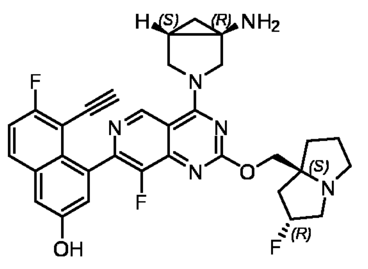
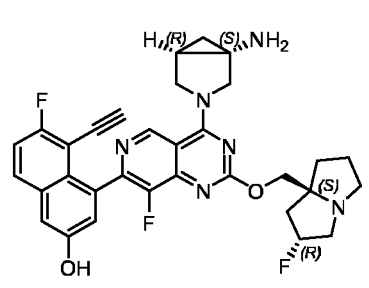
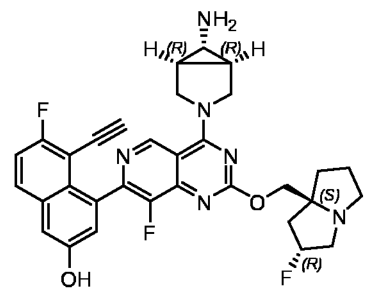
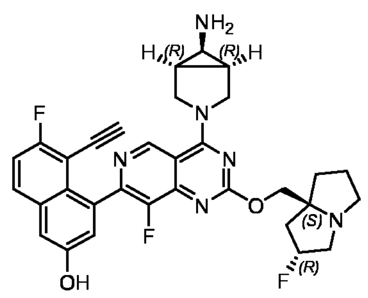
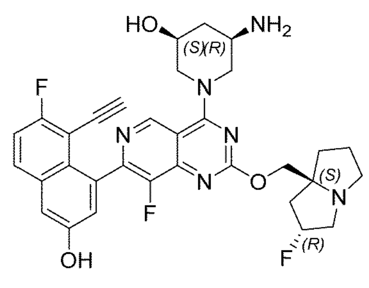
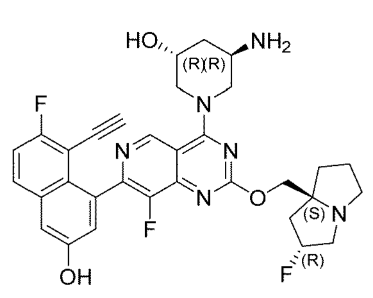
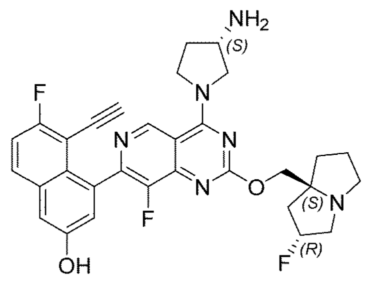
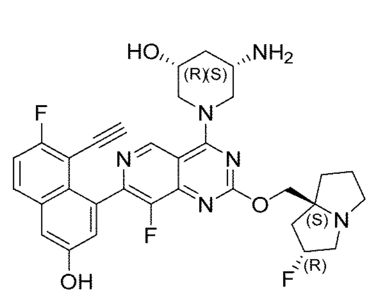
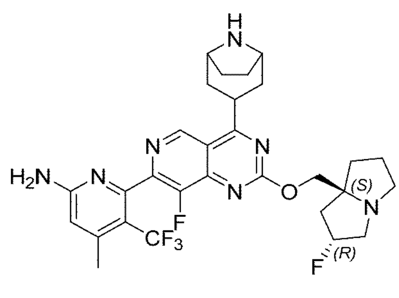
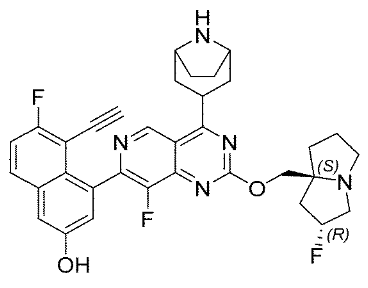

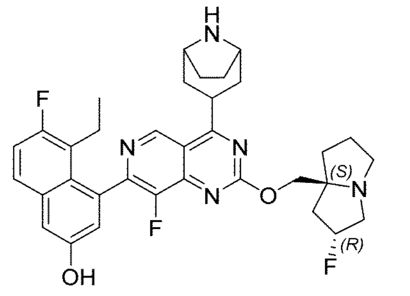
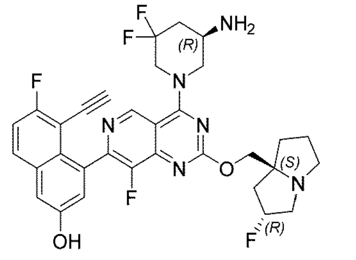
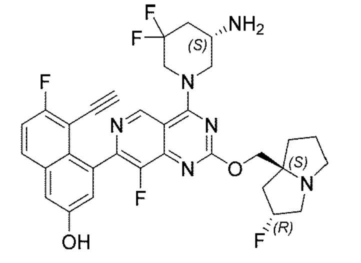

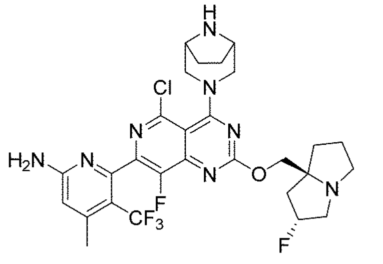
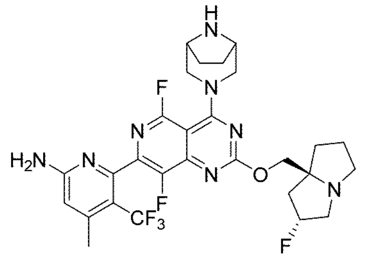
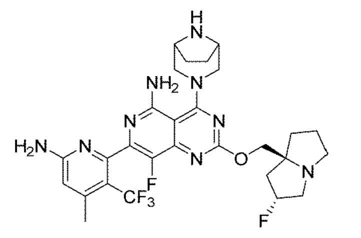

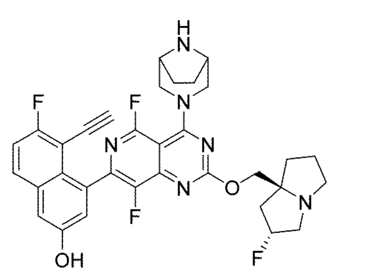
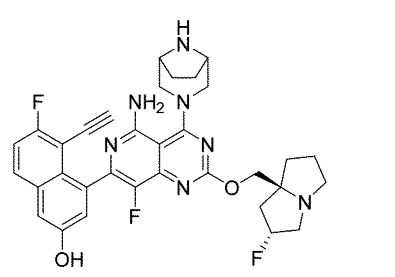
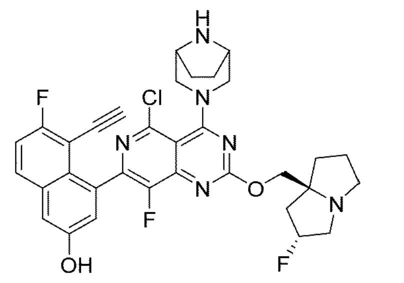

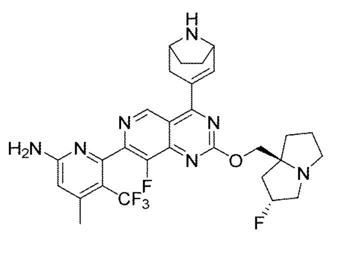
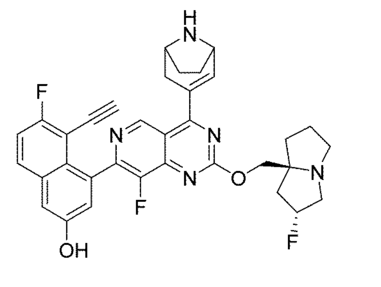
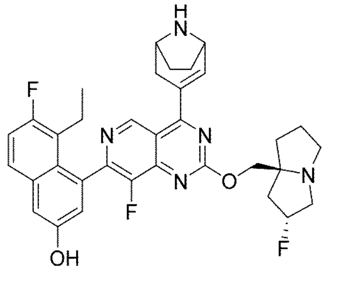

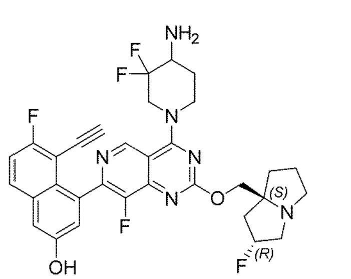
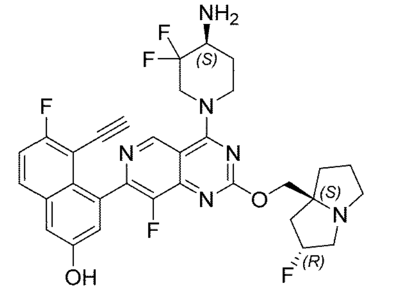
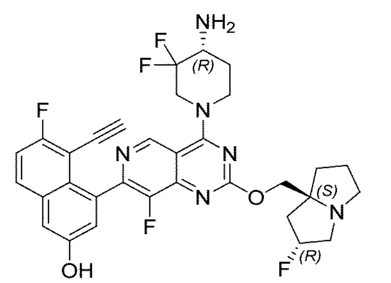

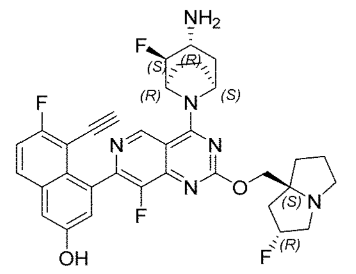 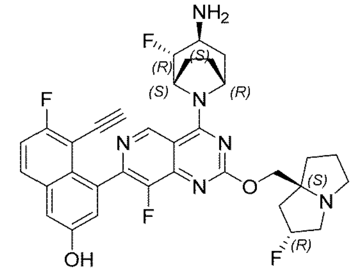, 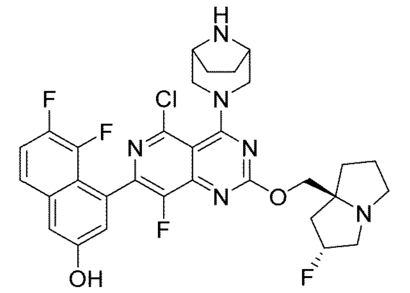,

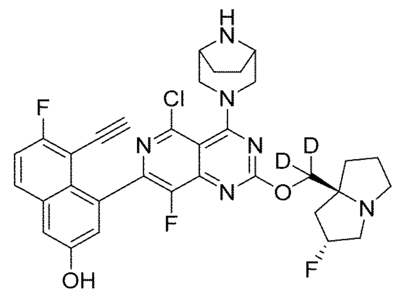, and 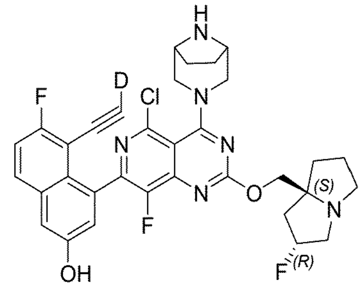;

and/or, the pharmaceutically acceptable salt of the nitrogen-containing heterocyclic compound of formula I has any one of the following structures:

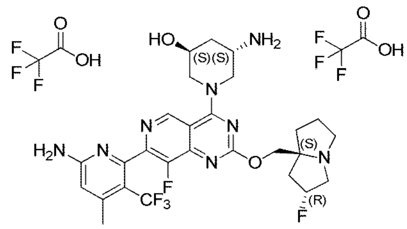, 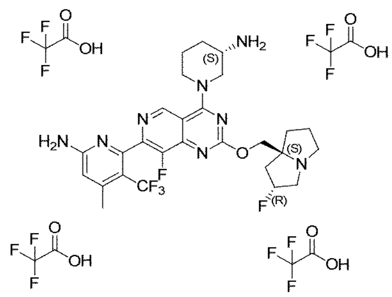, 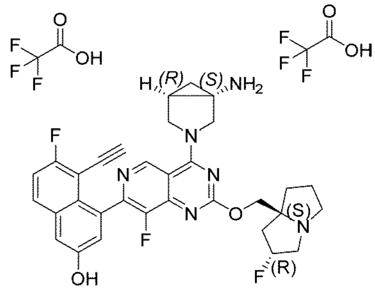,

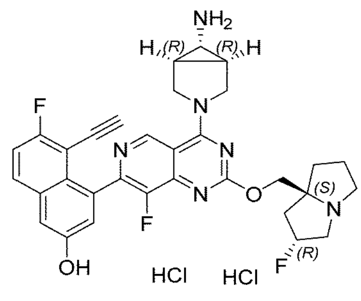, 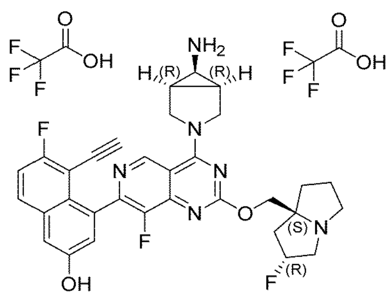, 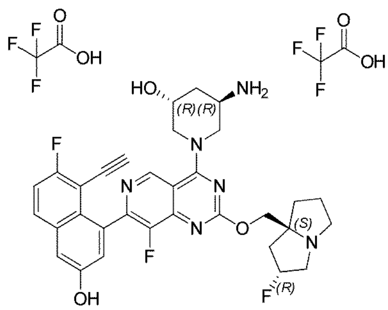,

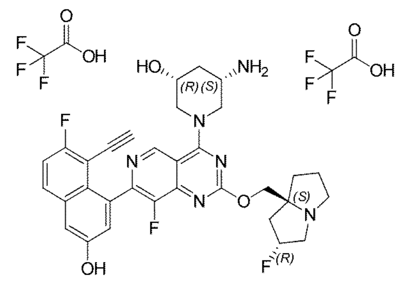, 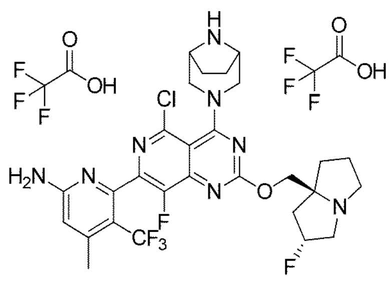,

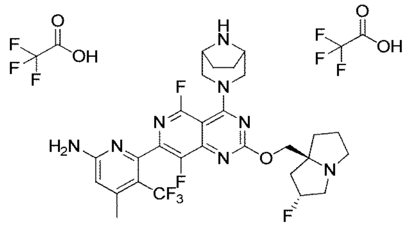, and 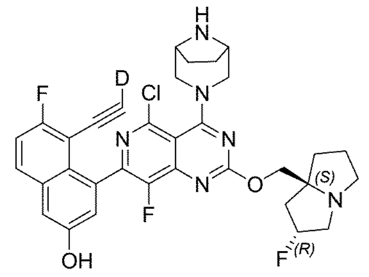

hydrochloride.

**13.** The pharmaceutically acceptable salt of the nitrogen-containing heterocyclic compound of formula I according to any one of claims 1 to 5, wherein the nitrogen-containing heterocyclic compound of formula I in the pharmaceutically acceptable salt of the nitrogen-containing heterocyclic compound of formula I is selected from the following compounds:

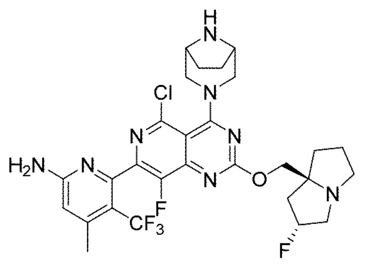, 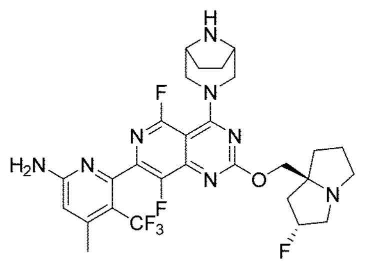, 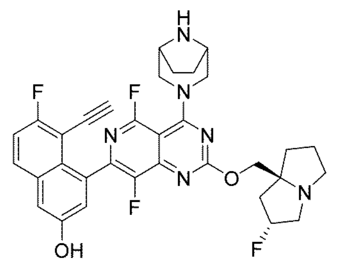,

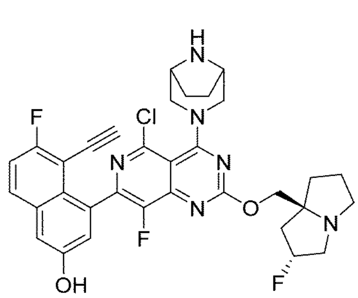, 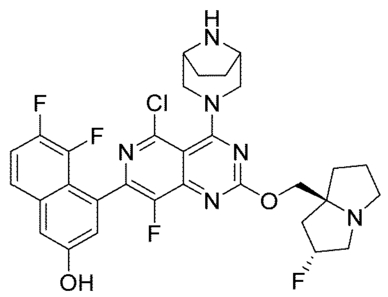, 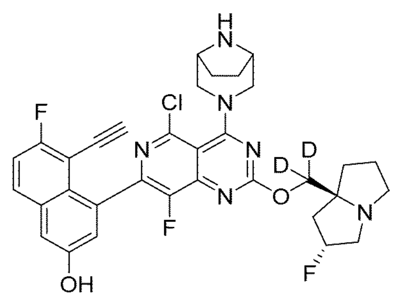,

and

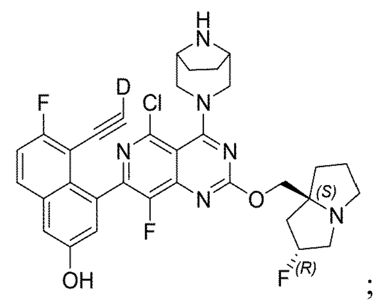;

the pharmaceutically acceptable salt in the pharmaceutically acceptable salt of the nitrogen-containing heterocyclic compound of formula I is a pharmaceutically acceptable base addition salt or a pharmaceutically acceptable acid addition salt; the pharmaceutically acceptable base addition salt is, for example, a lithium salt, a sodium salt, a potassium salt, a calcium salt, an aluminum salt, a magnesium salt, a zinc salt, a bismuth salt, an ammonium salt, a diethanolamine salt; the pharmaceutically acceptable acid addition salt is, for example, an inorganic acid salt or an organic acid salt (such as trifluoroacetate, hydrochloride).

**14.** A method for preparing the nitrogen-containing heterocyclic compound of formula I, the stereoisomer thereof, or the pharmaceutically acceptable salt thereof according to any one of claims 1 to 13, wherein when

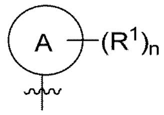

is

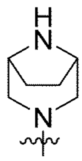

,

$R^3$ is

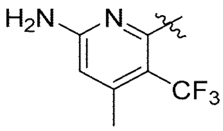

,

and $R^{4a}$ is halogen or amino, the corresponding nitrogen-containing heterocyclic compound of formula I, the stereoisomer thereof, or the pharmaceutically acceptable salt thereof is prepared by the following method,

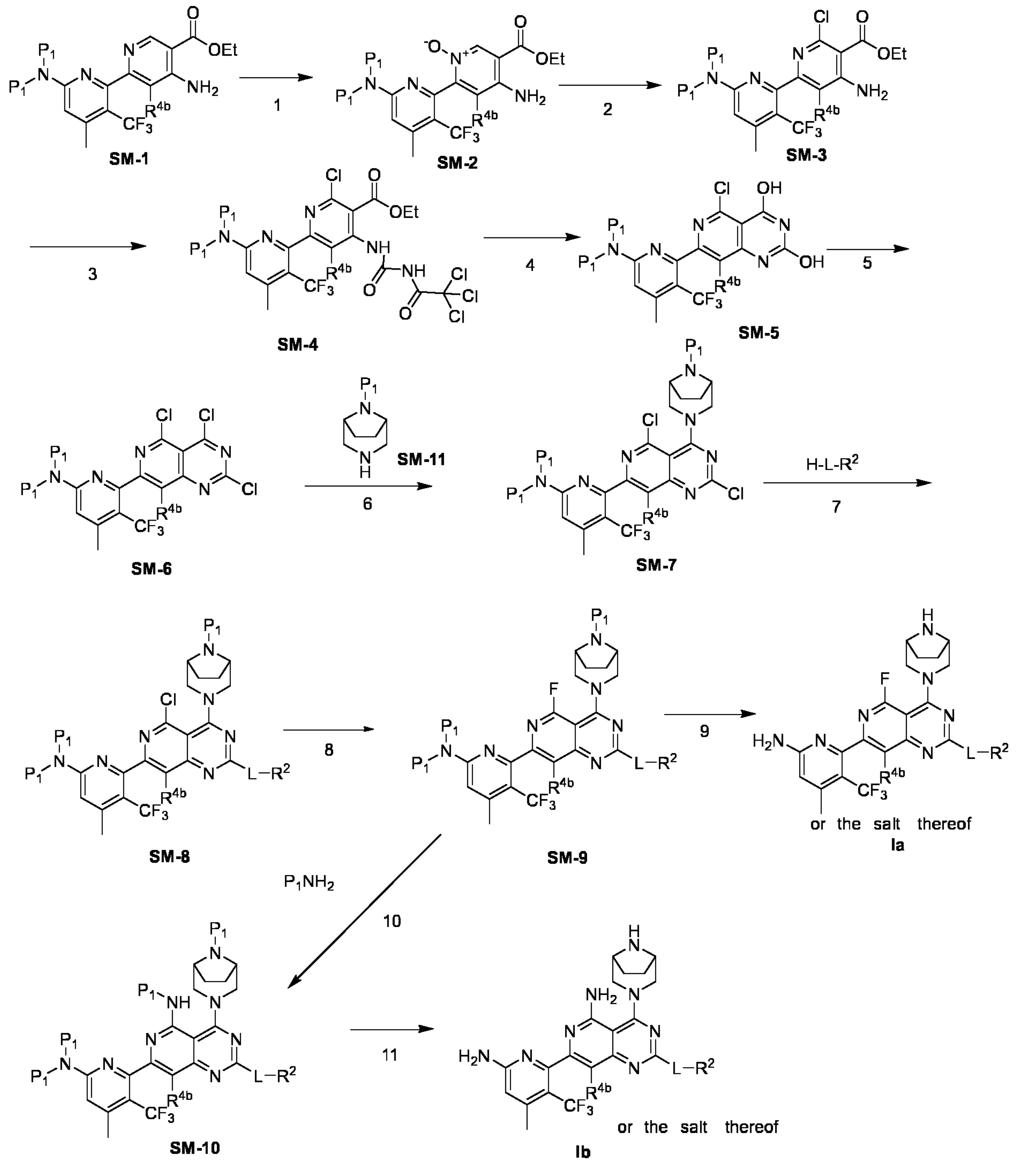

SM-1, SM-2, SM-3, SM-4, SM-5, SM-6, SM-7, SM-8, SM-9, Ia, SM-10, Ib

wherein Pi is an amino protecting group, such as Boc, PMB, Bn, Cbz, or Fmoc; each Pi is the same or different; $R^{4b}$, L, and $R^2$ are as defined in any one of claims 1 to 13;
the method comprises the following steps:

step 1: carrying out an oxidation reaction with a compound of formula SM-1 in a solvent in the presence of an oxidant to obtain a compound of formula SM-2;
step 2: carrying out a chlorination reaction with a compound of formula SM-2 in the presence of a chlorinating reagent to obtain a compound of formula SM-3;
step 3: carrying out a substitution reaction with the compound of formula SM-3 and trichloroacetyl isocyanate in a solvent to obtain a compound of formula SM-4;
step 4: carrying out an amination ring-closing reaction with the compound of formula SM-4 in a solvent to obtain a compound of formula SM-5;

step 5: carrying out a chlorination reaction with a compound of formula SM-5 in the presence of a chlorinating reagent to obtain a compound of formula SM-6;
step 6: carrying out a substitution reaction with the compound of formula SM-6 and a compound of formula SM-11 in a solvent under an alkaline condition to obtain a compound of formula SM-7;
step 7: carrying out a substitution reaction with the compound of formula SM-7 and a compound of formula H-L-$R^2$ in a solvent in the presence of a base to obtain a compound of formula SM-8;
step 8: carrying out a substitution reaction with the compound of formula SM-8 in a solvent in the presence of a fluorinating reagent to obtain a compound of formula SM-9;
step 9: carrying out a deamination protecting group reaction with the compound of formula SM-9 under an acidic condition to obtain a compound of formula Ia or a pharmaceutically acceptable salt thereof;
step 10: carrying out a substitution reaction with the compound of formula SM-9 and a compound of formula $P_1$-$NH_2$ in a solvent to obtain a compound of formula SM-10;
step 11: carrying out a deamination protecting group reaction with the compound of formula SM-10 under an acidic condition to obtain a compound of formula Ib or a pharmaceutically acceptable salt thereof;

alternatively, when

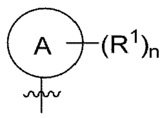

is

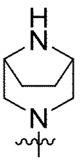

,

$R^3$ is

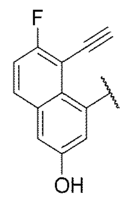

,

and $R^{4a}$ is halogen, the corresponding nitrogen-containing heterocyclic compound of formula I, the stereoisomer thereof, or the pharmaceutically acceptable salt thereof is prepared by the following method,

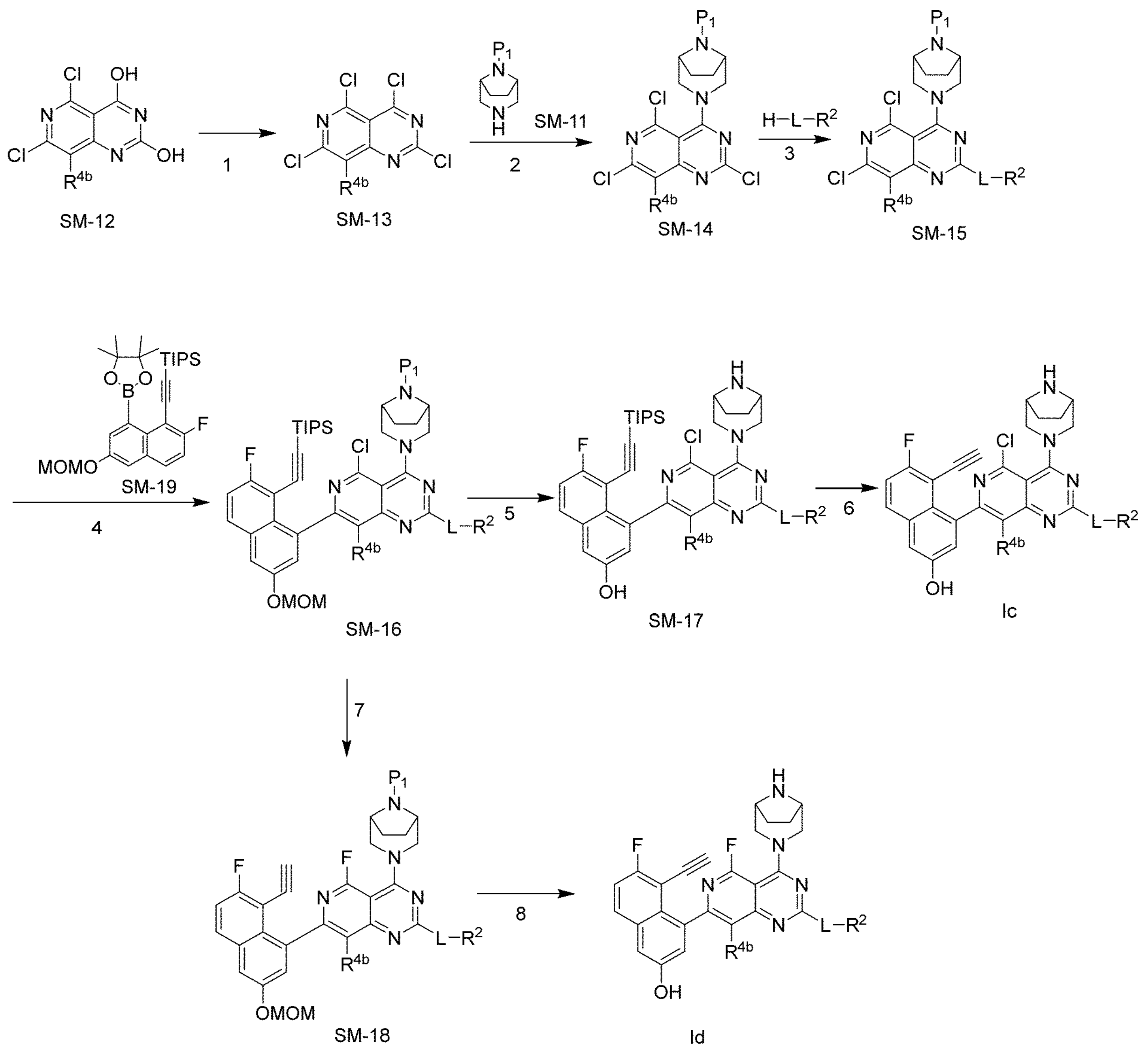


wherein Pt is an amino protecting group, such as Boc, PMB, Bn, Cbz, or Fmoc;
$R^{4b}$, L, and $R^2$ are as defined in any one of claims 1 to 13;
the method comprises the following steps:

step 1: carrying out a chlorination reaction with a compound of formula SM-12 in the presence of a chlorinating reagent to obtain a compound of formula SM-13;
step 2: carrying out a substitution reaction with the compound of formula SM-13 and a compound of formula SM-11 in a solvent under an alkaline condition to obtain a compound of formula SM-14;
step 3: carrying out a substitution reaction with the compound of formula SM-14 and a compound of formula H-L-$R^2$ in a solvent in the presence of a base to obtain a compound of formula SM-15;
step 4: carrying out a Suzuki coupling reaction with the compound of formula SM-15 and a compound of formula SM-19 in the presence of a catalyst to obtain a compound of formula SM-16;
step 5: carrying out a deamination protecting group and dehydroxyl protecting group reaction with the compound of formula SM-16 under an acidic condition to obtain a compound of formula SM-17;
step 6: carrying out a dealkynyl protecting group reaction with the compound of formula SM-17 in the presence of tetrabutylammonium fluoride, tetramethylammonium fluoride, or CsF to obtain a compound of formula Ic or a pharmaceutically acceptable salt thereof;
step 7: carrying out a dealkynyl protecting group reaction and a substitution reaction with the compound of formula SM-16 in the presence of tetrabutylammonium fluoride, tetramethylammonium fluoride, or CsF to obtain a compound of formula SM-18;
step 8: carrying out a deamination protecting group and dehydroxyl protecting group reaction with the

compound of formula SM-18 under an acidic condition to obtain a compound of formula Id or a pharmaceutically acceptable salt thereof.

**15.** A compound as shown below,

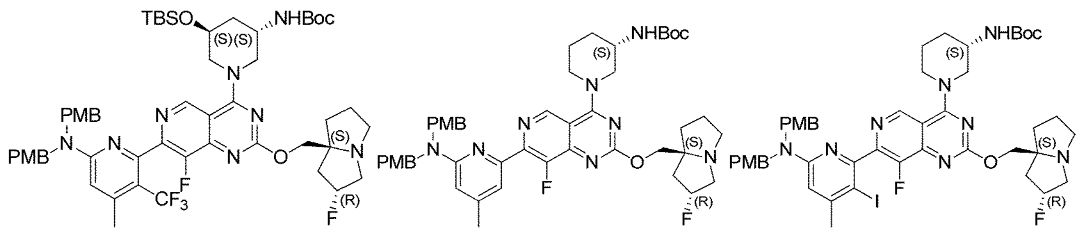

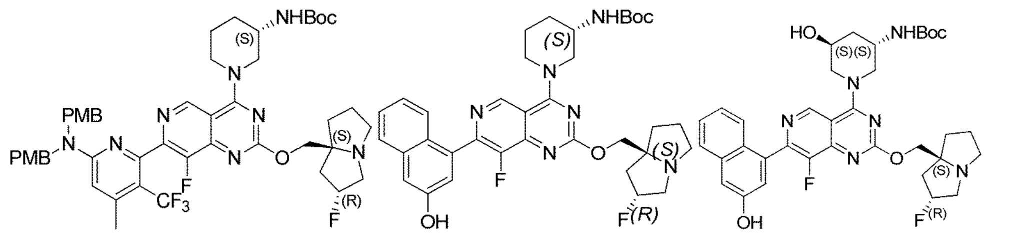

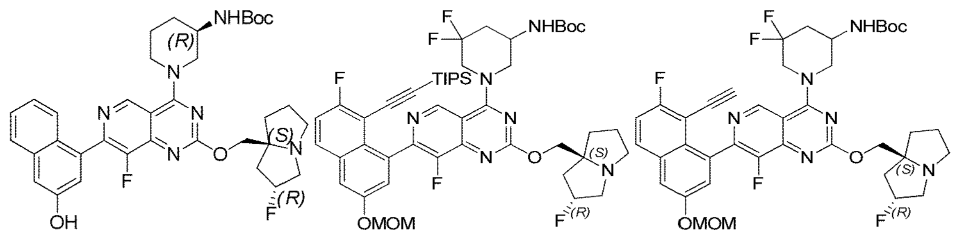

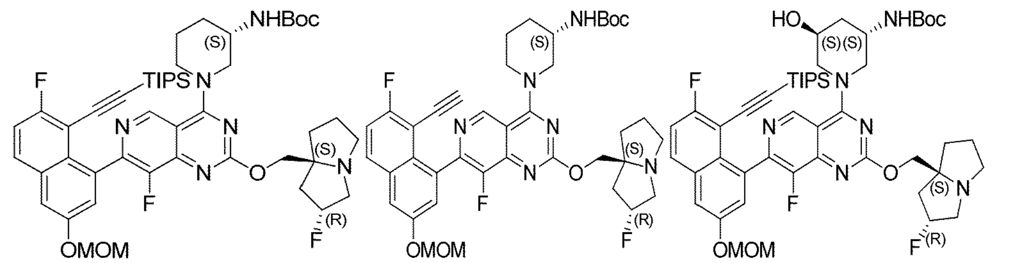

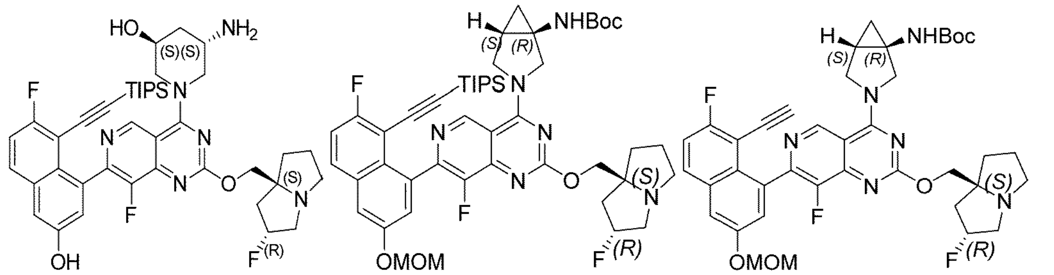

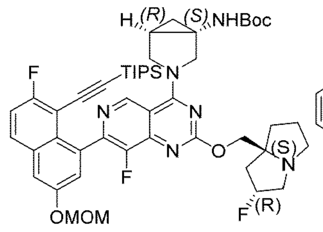
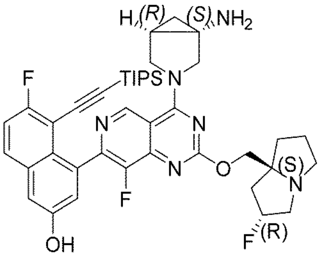
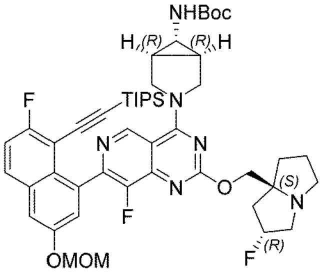

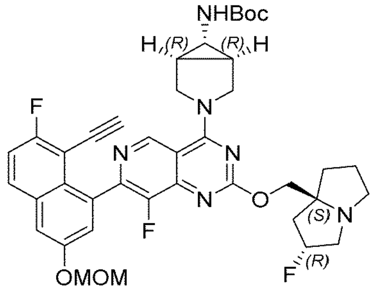
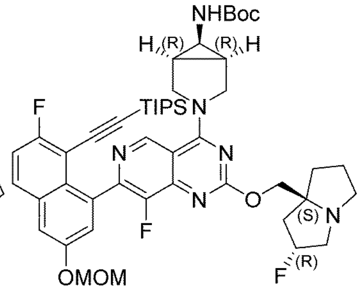
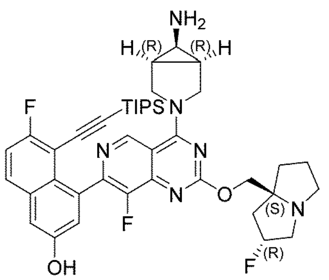

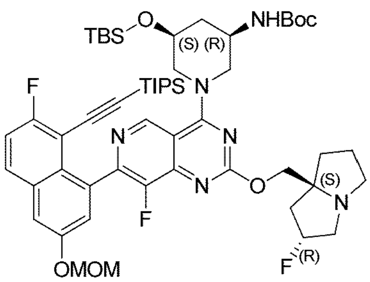
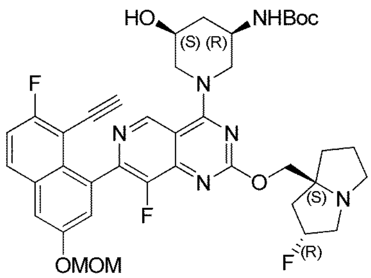
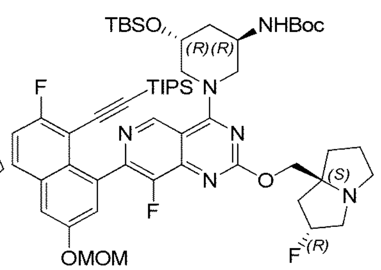

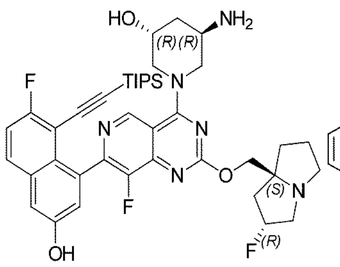
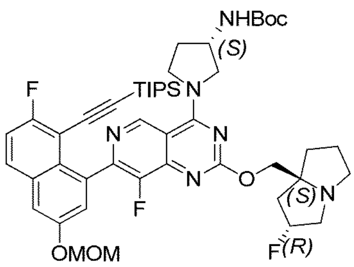
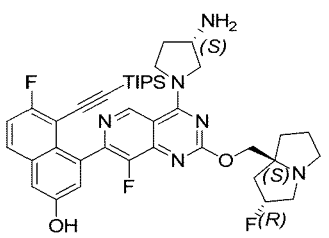

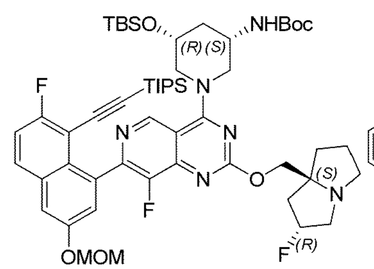
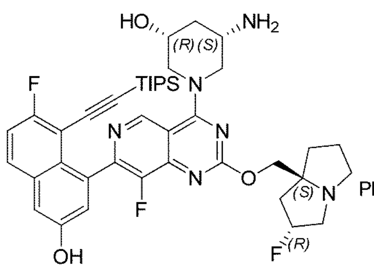
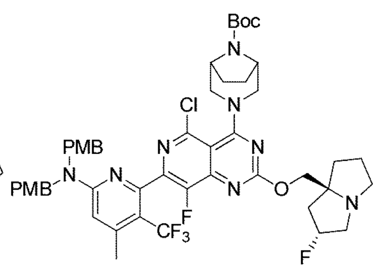

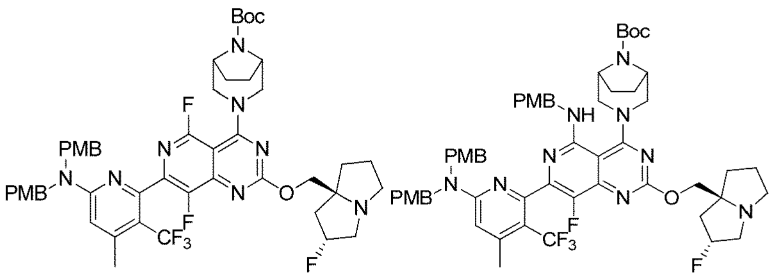

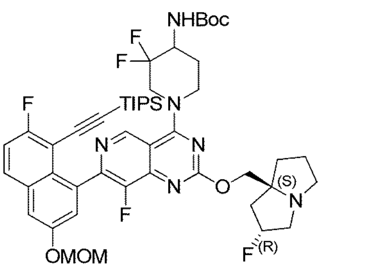

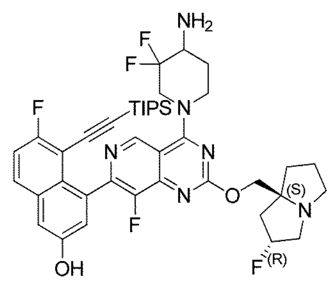

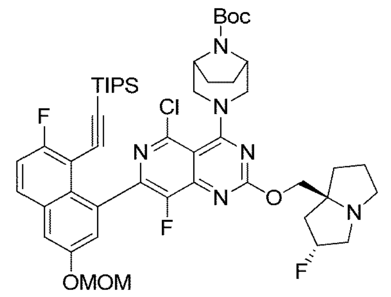

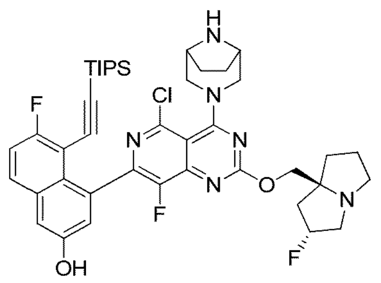

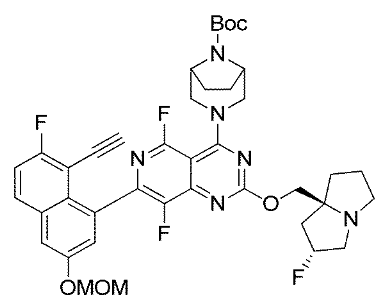

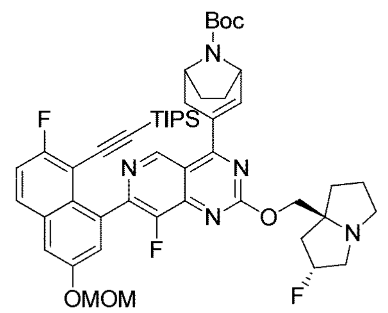

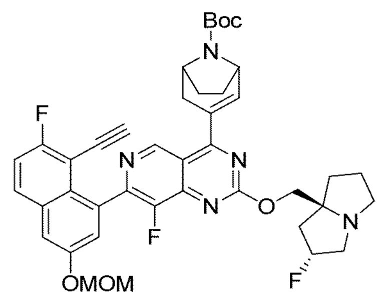

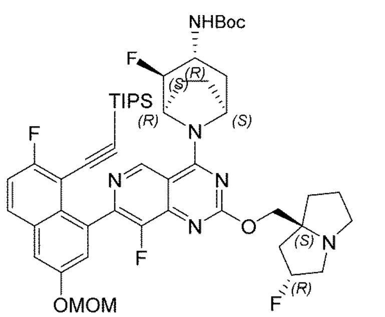

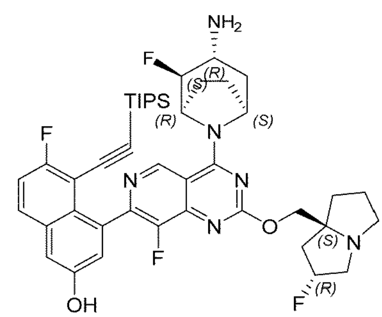

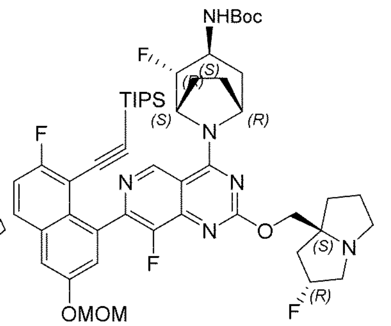

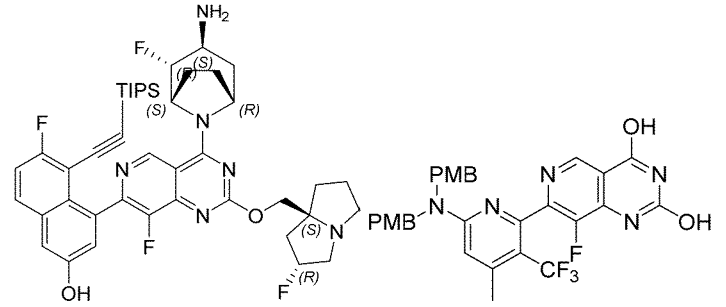

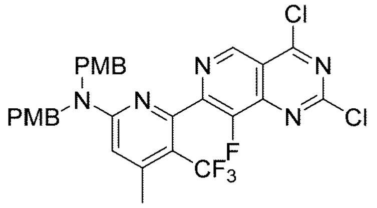

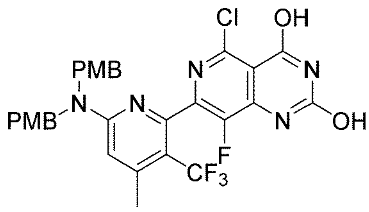

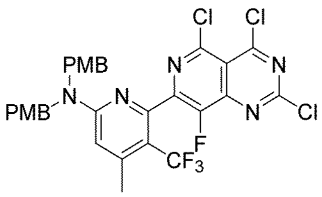

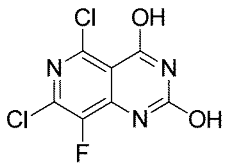

,

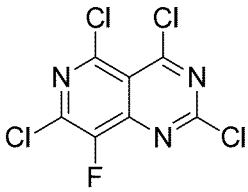

,

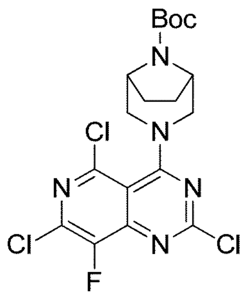

,

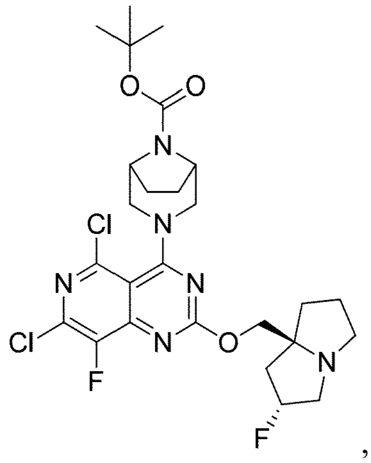

,

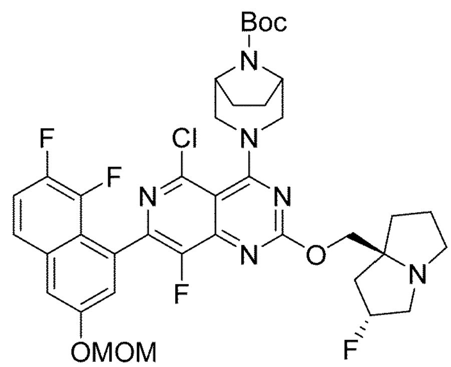

,

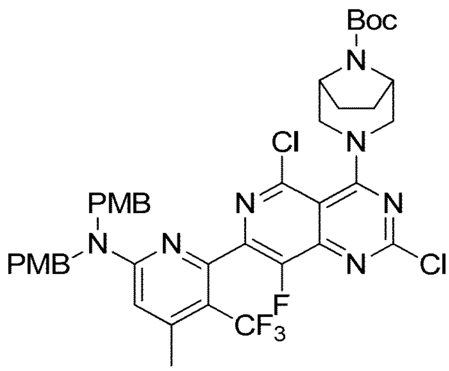

,

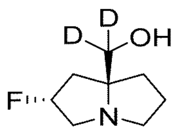

,

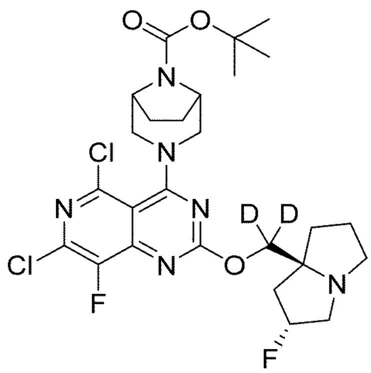

,

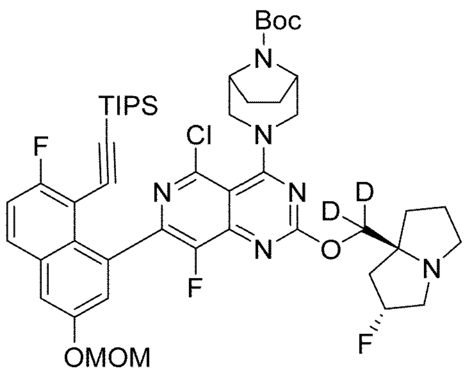

,

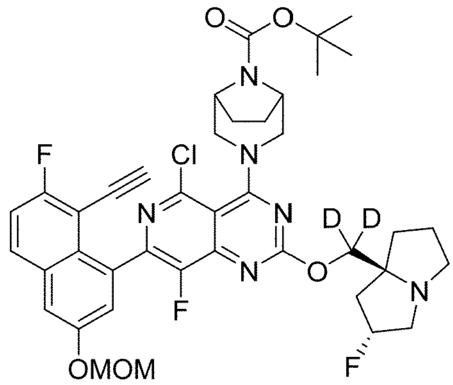

, or

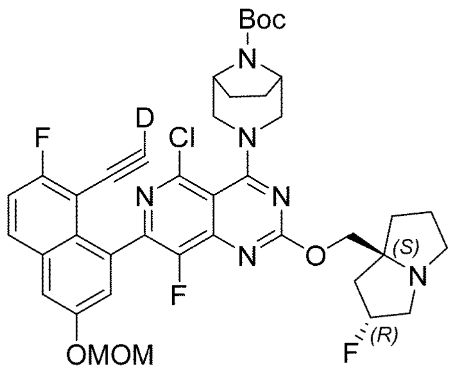

.

**16.** A pharmaceutical composition, which comprises a therapeutically effective amount of substance A and a pharmaceutical excipient; wherein the substance A is the nitrogen-containing heterocyclic compound of formula **I,** the stereoisomer thereof, or the pharmaceutically acceptable salt thereof according to any one of claims 1 to 13.

**17.** A use of substance A in the manufacture of a RAS inhibitor, wherein the substance A is the nitrogen-containing heterocyclic compound of formula I, the stereoisomer thereof, or the pharmaceutically acceptable salt thereof according to any one of claims 1 to 13;

the RAS inhibitor may be used in mammalian organisms *in vivo;* the RAS inhibitor may also be used *in vitro,* mainly for experimental purposes;
RAS may be KRAS or a KRAS mutation; for example, KRAS G12D, KRAS G12V.

**18.** A use of substance A in the manufacture of a medicament, wherein the substance A is the nitrogen-containing heterocyclic compound of formula I, the stereoisomer thereof, or the pharmaceutically acceptable salt thereof according to any one of claims 1 to 13;

the substance A is in a therapeutically effective amount;
the medicament may be used to treat and/or prevent a disease mediated by RAS; or, the medicament may be used to treat and/or prevent cancer.

**19.** The use according to claim 18, wherein

the RAS is KRAS or a KRAS mutation; for example, KRAS G12D, KRAS G12V;
the disease mediated by RAS is cancer;
the cancer may be selected from one or more than one of colon cancer, pancreatic cancer, breast cancer, prostate cancer, lung cancer, brain cancer, ovarian cancer, cervical cancer, testicular cancer, kidney cancer, head or neck cancer, bone cancer, skin cancer, rectal cancer, liver cancer, colorectal cancer, non-small cell lung cancer, small cell lung cancer, esophageal cancer, gastric cancer, thyroid cancer, bladder cancer, lymphoma, leukemia, and melanoma.

## INTERNATIONAL SEARCH REPORT

International application No. **PCT/CN2022/102497**

**A. CLASSIFICATION OF SUBJECT MATTER**

C07D 519/00(2006.01)i; C07F 7/18(2006.01)i; C07D 471/04(2006.01)i; C07C 53/18(2006.01)i; C07C 51/41(2006.01)i; A61K 31/519(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D; C07F; C07C; A61K; A61P

Documentation searched other than minimum documentation to the extent that such documents are included in the fields searched

Electronic data base consulted during the international search (name of data base and, where practicable, search terms used)

CNABS; CNKI; VEN; CNTXT; WPABS; ENTXT; STN-REGISTRY; STN-CAPLUS: 上海艾力斯医药科技股份有限公司, 罗会兵, 姜佳俊, 王家坡, 周华勇, 吡咯嗪嗪, 吡啶, 嘧啶, 癌症, 肿瘤, 根据式I的structural formula search, +Pyrrolizin+, +Pyrimidin+, +Pyridin+, RAS, KRAS, MRTX1133

**C. DOCUMENTS CONSIDERED TO BE RELEVANT**

| Category* | Citation of document, with indication, where appropriate, of the relevant passages | Relevant to claim No. |
|---|---|---|
| X | WO 2021041671 A1 (MIRATI THERAPEUTICS, INC. et al.) 04 March 2021 (2021-03-04) description, paragraphs [0167], [0196], [0197], and [0215-0224], embodiments 1-458, table 1 - table 3, and claims 1-61 | 1-19 |
| PX | WO 2022132200 A1 (MIRATI THERAPEUTICS, INC.) 23 June 2022 (2022-06-23) description, embodiments 1-514, table 2 - table 4, and claims 1-85 | 1-19 |
| PX | WO 2022042630 A1 (INVENTISBIO CO., LTD. et al.) 03 March 2022 (2022-03-03) description, paragraph [159], embodiments 15-82, table 1 - table 3, and claims 1-90 | 1-19 |
| PX | WO 2022015375 A1 (MIRATI THERAPEUTICS, INC. et al.) 20 January 2022 (2022-01-20) embodiments 1-466, table 2, and claims 1-43 | 1-19 |
| PX | WO 2022068921 A1 (SHANGHAI PHARMACEUTICALS HOLDING CO., LTD.) 07 April 2022 (2022-04-07) embodiments 1-70, claims 1-22, and table 16 | 1-19 |
| PX | CN 113999226 A (SHANGHAI KECHOW PHARMA, INC.) 01 February 2022 (2022-02-01) description, paragraphs [0664]-[0678], embodiments 1-938, and claims 1-23 | 1-19 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 August 2022** | **14 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/102497**

**C. DOCUMENTS CONSIDERED TO BE RELEVANT**

| Category* | Citation of document, with indication, where appropriate, of the relevant passages | Relevant to claim No. |
|---|---|---|
| PX | WO 2022061251 A1 (PLEXXIKON INC.) 24 March 2022 (2022-03-24)<br>embodiments 1-24, tables IA and table 2, and claims 1-32 | 1-19 |
| PX | WANG, X. L. et al. "Identification of MRTX1133, a Noncovalent, Potent, and Selective KRASG12D Inhibitor"<br>*Journal of Medicinal Chemistry,* Vol. 65, No. 4, 10 December 2021 (2021-12-10), ISSN: 0022-2623,<br>pages 3123-3133, table 1 – table 4, and solution 1 | 1-13, 15-19 |
| A | WO 2020146613 A1 (MIRATI THERAPEUTICS, INC. et al.) 16 July 2020 (2020-07-16)<br>entire document | 1-19 |

Form PCT/ISA/210 (second sheet) (January 2015)

**INTERNATIONAL SEARCH REPORT**
**Information on patent family members**

International application No.
**PCT/CN2022/102497**

| Patent document cited in search report | Publication date (day/month/year) | Patent family member(s) | Publication date (day/month/year) |
|---|---|---|---|
| WO 2021041671 A1 | 04 March 2021 | EP 4021444 A1 | 06 July 2022 |
| | | AU 2020337938 A1 | 17 March 2022 |
| | | CA 3148745 A1 | 04 March 2021 |
| | | TW 202122396 A | 16 June 2021 |
| | | KR 20220071193 A | 31 May 2022 |
| WO 2022132200 A1 | 23 June 2022 | None | |
| WO 2022042630 A1 | 03 March 2022 | None | |
| WO 2022015375 A1 | 20 January 2022 | None | |
| WO 2022068921 A1 | 07 April 2022 | None | |
| CN 113999226 A | 01 February 2022 | None | |
| WO 2022061251 A1 | 24 March 2022 | None | |
| WO 2020146613 A1 | 16 July 2020 | US 2020331911 A1 | 22 October 2020 |
| | | EP 3908283 A1 | 17 November 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110736463
- CN 202110959764
- CN 202210029060
- WO 2021041671 A
- WO 2021041671 A1


### Non-patent literature cited in the description


- **MOORE, A.R. et al.** *Nat Rev Drug Discov,* 2020, vol. 19, 533
- **BERGE et al.** Pharmaceutical Salts. *Journal of Pharmaceutical Science,* 1977, vol. 66, 1-19
- Handbook of Pharmaceutical Salts: Properties, Selection, and Use. Wiley-VCH, 2002
- Pharmacopoeia of the People's Republic of China. 2015
- **RAYMOND C ROWE.** Handbook of Pharmaceutical Excipients. 2009
- Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007